# EUROPEAN PATENT APPLICATION

(11) **EP 3 085 781 A1**
(43) Date of publication of application: **26.10.2016**
(21) Application number: 14871677.2
(22) Date of filing: 18.12.2014
(51) Int. Cl.: C12N 15/09, A61K 45/00, A61P 35/00, C07K 19/00, C12N 9/12, C12Q 1/68, G01N 33/574, C07K 16/40

(54) **NOVEL FUSION GENE AS FACTOR RESPONSIBLE FOR STOMACH CANCER**

(30) Priority: 19.12.2013 JP 2013263032
(71) Applicant: National Cancer Center, Tokyo 104-0045 (JP); LSIP, LLC, Chiyoda-ku Tokyo 100-0005 (JP)
(72) Inventor: SHIBATA, Tatsuhiro, Tokyo 104-0045 (JP); HOSODA, Fumie, Tokyo 104-0045 (JP)
(74) Representative: Cabinet Plasseraud
(86) International application number: PCT/JP2014/083526
(87) International publication number: WO 2015/093557

(57) **Abstract**

To identify mutations that can serve as indicators for predicting the effectiveness of drug treatments in cancers such as gastric cancer; to provide a means for detecting said mutations; and to provide a means for identifying, based on said mutations, patients with cancer or subjects with a risk of cancer, in whom drugs targeting genes having said mutations or proteins encoded by said genes show a therapeutic effect.

A method for detecting a gene fusion serving as a responsible mutation (driver mutation) for cancer, the method comprising the step of detecting any one of an ATG3-EPHB1 fusion polynucleotide, a TNIK-RNF123 fusion polynucleotide, or an SLC12A2-NRG2 fusion polynucleotide, or a polypeptide encoded thereby, in an isolated sample from a subject.

## Description

### TECHNICAL FIELD

The present invention relates mainly to a method for detecting gene fusions serving as responsible mutations for cancer, and a method for identifying patients with cancer or subjects with a risk of cancer, in whom substances suppressing the expression and/or activity of polypeptides encoded by fusion polynucleotides produced by said gene fusions show a therapeutic effect.

### BACKGROUND ART

Cancer is the first-ranked disease among causes of death in Japan. Cancer cells are known to become malignant due to genetic alterations. Identifying genetic alterations characteristic of cancer type is expected to contribute not only to the clarification of carcinogenic mechanisms but also to, for example, the diagnosis and prognosis prediction of cancers and the development and selection of a cancer therapy.

There have hitherto been reported many cancer-related genes.

For example, EPHB1 is a gene encoding a receptor tyrosine kinase involved in the development of the nervous system and other tissues. It was reported that loss or reduction of the expression of this protein is associated with invasion and metastasis in gastric cancers (Non Patent Literature 1), invasion in colorectal cancers (Non Patent Literature 2), and the like.

Also, it was reported that the TNIK (Traf2- and Nck-interacting kinase) gene is essential for colorectal cancer growth (Non Patent Literature 3) and may have an impact on breast cancer cell growth (Non Patent Literature 4).

The SLC12A2 gene, also called NKCC1 (Na-K-Cl cotransporter 1), was suggested to modulate the migration of glioma cells (Non Patent Literature 5).

NRG2 is a gene encoding a protein in the neuregulin family which controls cell growth and differentiation through interaction with ERBB family receptors. It was shown that high expression of this gene is related to the aggressiveness of tumors (Non Patent Literature 6).

In recent years, molecular targeted drugs against cancers have been developed, and there have been increasing examples of successful development of molecular targeted therapies against constituents (*e.g.*, kinases) of carcinogenesis-related signaling pathways. For example, it is reported that tyrosine kinase inhibitors (*e.g.*, crizotinib) targeting ALK protein are effective in the treatment of lung cancer patients bearing the EML4-ALK fusion gene, and that tyrosine kinase inhibitors (*e.g.*, imatinib) targeting the protein encoded by the BCR-ABL fusion gene are effective in the treatment of leukemia patients bearing this fusion gene.

According to a certain international comparison of morbidity rate, gastric cancer is known to be highly prevalent in East Asian countries including Japan as well as in South America. A comparison of cancer deaths in Japan by body part shows that gastric cancer is the second most frequent cause of cancer deaths in both men and women. In molecular therapies against gastric cancer, there are several known gene targets including HER2.

### CITATION LIST

### NON PATENT LITERATURES

[Non Patent Literature 1] Wang J. D., et al., Oncology, 2007, 73 (3-4), 238-45.
[Non Patent Literature 2] Sheng Z., et al., Pathobiology, 2008, 75 (5), 274-80.
[Non Patent Literature 3] Shitashige M., et al., Cancer Res, 2010, 70 (12), 5024-33.
[Non Patent Literature 4] Jiao X., et al., BMC Genomics, 2013, 14, 165.
[Non Patent Literature 5] Garzon-Muvdi T., et al., PLoS Biol, 2012, 10 (5), e1001320.
[Non Patent Literature 6] Revillion F., et al., Ann Oncol, 2008, 19 (1), 73-80.

### SUMMARY OF INVENTION

### TECHNICAL PROBLEM

Identification of mutations in various cancers including gastric cancer has not yet been made thoroughly, and there is still a demand for further identification of mutations that can serve as therapeutic targets and as indicators for predicting the effectiveness of drug treatments.

Accordingly, the objects of the present invention include but are not limited to the following: to identify mutations that can serve as an indicator for predicting the effectiveness of drug treatments in various cancers including gastric cancer; to provide a means for detecting said mutations; and to provide a means for identifying, based on said mutations, patients with cancers or subjects with a risk of cancers, in whom drugs targeting genes having said mutations or proteins encoded by said genes show a therapeutic effect.

### SOLUTION TO PROBLEM

As the result of conducting intensive studies with a view to achieving the above-mentioned objects, the present inventors identified kinase fusion genes (ATG3-EPHB1 and TNIK-RNF123) and a kinase-related molecule fusion gene (SLC12A2-NRG2) as novel genomic aberrations in gastric cancer. None of these fusion genes is expressed in normal gastric mucosal tissues, and besides, none of the known causative gene mutations for gastric cancer (*i.e*., KRAS, NRAS and BRAF mutations) was detected in case patients in whom any of the above-mentioned fusion genes was detected; thus, these fusion genes were considered as responsible mutations (driver mutations) for cancer.

The ATG3-EPHB1 and TNIK-RNF123 fusion genes were considered to lead to constitutive activation of EPHB1 and TNIK kinases, respectively, thereby contributing to malignant transformation of cancers. Therefore, it is considered that EPHB1 and TNIK kinase inhibitors, respectively, would produce a therapeutic effect on cancers positive for the above-mentioned gene fusions.

As regards the SLC12A2-NRG2 fusion gene, since the cell growth factor NRG2 is a ligand for HER2 which has already been known as a therapeutic target for gastric cancer, it was believed that this fusion gene contributes to the development of gastric cancer through a similar molecular mechanism to that of HER2 mutation. Therefore, an antibody drug against NRG2, or antibody drugs or kinase inhibitors against a group of HER proteins serving as NRG2 receptors could produce a therapeutic effect on cancer positive for this gene fusion.

The present inventors have made further studies based on these findings and completed the present invention.

More specifically, the present invention is defined as follows.
[1] A method for detecting a gene fusion serving as a responsible mutation (driver mutation) for cancer, the method comprising the step of detecting a fusion polynucleotide of any one of (a) to (c) below, or a polypeptide encoded thereby, in an isolated sample from a subject:
   (a) an ATG3-EPHB1 fusion polynucleotide which encodes a polypeptide comprising the kinase domain of EPHB1 and having kinase activity;
   (b) a TNIK-RNF123 fusion polynucleotide which encodes a polypeptide comprising the kinase domain of TNIK and the SPRY domain and RING finger domain of RNF123, and having kinase activity; and
   (c) an SLC 12A2-NRG2 fusion polynucleotide which encodes a polypeptide comprising the AA_permease_N super family domain of SLC12A2 and the neuregulin family conserved region of NRG2, and having intracellular signaling-enhancing activity.
[2] The method according to [1], wherein the fusion polynucleotide is any one of (a) to (c) below:
   (a) an ATG3-EPHB1 fusion polynucleotide encoding the polypeptide mentioned below:
      (i) a polypeptide consisting of the amino acid sequence of SEQ ID NO: 2,
      (ii) a polypeptide consisting of an amino acid sequence derived from the amino acid sequence of SEQ ID NO: 2 by deletion, substitution or addition of one or more amino acids, and the polypeptide having kinase activity, or
      (iii) a polypeptide consisting of an amino acid sequence having a sequence identity of at least 80% to the amino acid sequence of SEQ ID NO: 2, and the polypeptide having kinase activity;
   (b) a TNIK-RNF123 fusion polynucleotide encoding the polypeptide mentioned below:
      (i) a polypeptide consisting of the amino acid sequence of SEQ ID NO: 4, 6, 8, 10, 12, 14, 16 or 18,
      (ii) a polypeptide consisting of an amino acid sequence derived from the amino acid sequence of SEQ ID NO: 4, 6, 8, 10, 12, 14, 16 or 18 by deletion, substitution or addition of one or more amino acids, and the polypeptide having kinase activity, or
      (iii) a polypeptide consisting of an amino acid sequence having a sequence identity of at least 80% to the amino acid sequence of SEQ ID NO: 4, 6, 8, 10, 12, 14, 16 or 18, and the polypeptide having kinase activity; and
   (c) an SLC 12A2-NRG2 fusion polynucleotide encoding the polypeptide mentioned below:
      (i) a polypeptide consisting of the amino acid sequence of SEQ ID NO: 20, 22, 24, 26 or 28,
      (ii) a polypeptide consisting of an amino acid sequence derived from the amino acid sequence of SEQ ID NO: 20, 22, 24, 26 or 28 by deletion, substitution or addition of one or more amino acids, and the polypeptide having intracellular signaling-enhancing activity, or
      (iii) a polypeptide consisting of an amino acid sequence having a sequence identity of at least 80% to the amino acid sequence of SEQ ID NO: 20, 22, 24, 26 or 28, and the polypeptide having intracellular signaling-enhancing activity.
[3] The method according to [1] or [2], wherein the fusion polynucleotide is any one of (a) to (c) below:
   (a)
      (i) a polynucleotide consisting of the nucleotide sequence of SEQ ID NO: 1,
      (ii) a polynucleotide that hybridizes under stringent conditions with a polynucleotide consisting of a nucleotide sequence complementary to the polynucleotide consisting of the nucleotide sequence of SEQ ID NO: 1, and which encodes a polypeptide having kinase activity,
      (iii) a polynucleotide that consists of a nucleotide sequence derived from the nucleotide sequence of SEQ ID NO: 1 by deletion, substitution or addition of one or more nucleotides, and which encodes a polypeptide having kinase activity, or
      (iv) a polynucleotide that has a sequence identity of at least 80% to the polynucleotide consisting of the nucleotide sequence of SEQ ID NO: 1, and which encodes a polypeptide having kinase activity;
   (b)
      (i) a polynucleotide consisting of the nucleotide sequence of SEQ ID NO: 3,5,7, 9, 11, 13, 15 or 17,
      (ii) a polynucleotide that hybridizes under stringent conditions with a polynucleotide consisting of a nucleotide sequence complementary to the polynucleotide consisting of the nucleotide sequence of SEQ ID NO: 3, 5, 7, 9, 11, 13, 15 or 17, and which encodes a polypeptide having kinase activity,
      (iii) a polynucleotide that consists of a nucleotide sequence derived from the nucleotide sequence of SEQ ID NO: 3, 5, 7, 9, 11, 13, 15 or 17 by deletion, substitution or addition of one or more nucleotides, and which encodes a polypeptide having kinase activity, or
      (iv) a polynucleotide that has a sequence identity of at least 80% to the polynucleotide consisting of the nucleotide sequence of SEQ ID NO: 3, 5, 7, 9, 11, 13, 15 or 17, and which encodes a polypeptide having kinase activity; and
   (c)
      (i) a polynucleotide consisting of the nucleotide sequence of SEQ ID NO: 19, 21, 23, 25 or 27,
      (ii) a polynucleotide that hybridizes under stringent conditions with a polynucleotide consisting of a nucleotide sequence complementary to the polynucleotide consisting of the nucleotide sequence of SEQ ID NO: 19, 21, 23, 25 or 27, and which encodes a polypeptide having intracellular signaling-enhancing activity,
      (iii) a polynucleotide that consists of a nucleotide sequence derived from the nucleotide sequence of SEQ ID NO: 19, 21, 23, 25 or 27 by deletion, substitution or addition of one or more nucleotides, and which encodes a polypeptide having intracellular signaling-enhancing activity, or
      (iv) a polynucleotide that has a sequence identity of at least 80% to the polynucleotide consisting of the nucleotide sequence of SEQ ID NO: 19, 21, 23, 25 or 27, and which encodes a polypeptide having intracellular signaling-enhancing activity.
[4] The method according to any one of [1] to [3], wherein the cancer is gastric cancer.
[5] A method for identifying a patient with cancer or a subject with a risk of cancer, in whom a substance suppressing the expression and/or activity of a polypeptide encoded by a fusion polynucleotide produced by a gene fusion serving as a responsible mutation (driver mutation) for cancer shows a therapeutic effect, the method comprising the steps of:
   (1) detecting a fusion polynucleotide of any one of (a) to (c) below, or a polypeptide encoded thereby, in an isolated sample from the subject:
      (a) an ATG3-EPHB1 fusion polynucleotide which encodes a polypeptide comprising the kinase domain of EPHB1 and having kinase activity,
      (b) a TNIK-RNF123 fusion polynucleotide which encodes a polypeptide comprising the kinase domain of TNIK and the SPRY domain and RING finger domain of RNF123, and having kinase activity, and
      (c) an SLC12A2-NRG2 fusion polynucleotide which encodes a polypeptide comprising the AA_permease_N super family domain of SLC12A2 and the neuregulin family conserved region of NRG2, and having intracellular signaling-enhancing activity; and
   (2) determining that the substance suppressing the expression and/or activity of the polypeptide shows a therapeutic effect in the subject, in the case where the fusion polynucleotide of any one of (a) to (c) or the polypeptide encoded thereby is detected.
[6] A kit for detecting a gene fusion serving as a responsible mutation (driver mutation) for cancer, the kit comprising any or a combination of (A) to (C) below:
   (A) a polynucleotide that serves as a probe designed to specifically recognize an ATG3-EPHB1 fusion polynucleotide, a TNIK-RNF123 fusion polynucleotide, or an SLC12A2-NRG2 fusion polynucleotide;
   (B) polynucleotides that serve as a pair of primers designed to enable specific amplification of an ATG3-EPHB1 fusion polynucleotide, a TNIK-RNF123 fusion polynucleotide, or an SLC12A2-NRG2 fusion polynucleotide; and
   (C) an antibody that specifically recognizes an ATG3-EPHB1 fusion polypeptide, a TNIK-RNF123 fusion polypeptide, or an SLC12A2-NRG2 fusion polypeptide.
[7] An isolated ATG3-EPHB1 fusion polypeptide or a fragment thereof, which comprises the kinase domain of EPHB1 and has kinase activity.
[8] An isolated TNIK-RNF123 fusion polypeptide or a fragment thereof, which comprises the kinase domain of TNIK and the SPRY domain and RING finger domain of RNF123, and has kinase activity.
[9] An isolated SLC12A2-NRG2 fusion polypeptide or a fragment thereof, which comprises the AA_permease_N super family domain of SLC 12A2 and the neuregulin family conserved region of NRG2, and has intracellular signaling-enhancing activity.
[10] A polynucleotide encoding the fusion polypeptide or the fragment thereof according to any one of [7] to [9].
[11] A therapeutic agent for cancer positive for ATG3-EPHB1, TNIK-RNF123 or SLC12A2-NRG2 gene fusion.
[12] The therapeutic agent according to [11], comprising, as an active ingredient, a substance suppressing the expression and/or activity of: an ATG3-EPHB1 fusion polypeptide comprising the kinase domain of EPHB1 and having kinase activity; a TNIK-RNF123 fusion polypeptide comprising the kinase domain of TNIK and the SPRY domain and RING finger domain of RNF 123 and having kinase activity; or an SLC12A2-NRG2 fusion polypeptide comprising the AA_permease_N super family domain of SLC12A2 and the neuregulin family conserved region of NRG2, and having intracellular signaling-enhancing activity.
[13] The therapeutic agent according to [11] or [12], wherein the cancer is gastric cancer.
[14] A drug for use in the treatment of cancer, the drug comprising a substance suppressing the expression and/or activity of: an ATG3-EPHB1 fusion polypeptide comprising the kinase domain of EPHB1 and having kinase activity; a TNIK-RNF123 fusion polypeptide comprising the kinase domain of TNIK and the SPRY domain and RING finger domain of RNF123 and having kinase activity; or an SLC12A2-NRG2 fusion polypeptide comprising the AA_permease_N super family domain of SLC12A2 and the neuregulin family conserved region of NRG2, and having intracellular signaling-enhancing activity.
[15] The drug according to [14], wherein the cancer is cancer positive for ATG3-EPHB1, TNIK-RNF123 or SLC12A2-NRG2 gene fusion.
[16] The drug according to [14] or [15], wherein the cancer is gastric cancer.
[17] A method for treatment of cancer, comprising the step of administering to a subject a therapeutically effective amount of a substance suppressing the expression and/or activity of: an ATG3-EPHB1 fusion polypeptide comprising the kinase domain of EPHB1 and having kinase activity; a TNIK-RNF123 fusion polypeptide comprising the kinase domain of TNIK and the SPRY domain and RING finger domain of RNF123 and having kinase activity; or an SLC12A2-NRG2 fusion polypeptide comprising the AA_permease_N super family domain of SLC12A2 and the neuregulin family conserved region of NRG2, and having intracellular signaling-enhancing activity.
[18] The method according to [17], wherein the subject is a patient with cancer positive for ATG3-EPHB1, TNIK-RNF123 or SLC12A2-NRG2 gene fusion.
[19] The method according to [17], wherein the subject is a patient with gastric cancer.
[20] A method for screening a cancer therapeutic agent, the method comprising the steps of:
   (1) bringing a test substance into contact with a cell that expresses: an ATG3-EPHB1 fusion polypeptide comprising the kinase domain of EPHB1 and having kinase activity; a TNIK-RNF123 fusion polypeptide comprising the kinase domain of TNIK and the SPRY domain and RING finger domain of RNF123 and having kinase activity; or an SLC12A2-NRG2 fusion polypeptide comprising the AA_permease_N super family domain of SLC 12A2 and the neuregulin family conserved region of NRG2, and having intracellular signaling-enhancing activity;
   (2) determining whether the expression and/or activity of any of said fusion polypeptides is suppressed or not; and
   (3) selecting the substance determined to suppress the expression and/or activity of any of said fusion polypeptides, as a cancer therapeutic agent.

### ADVANTAGEOUS EFFECTS OF INVENTION

The present invention makes it possible to detect unknown responsible mutations for the particular cancer, which have been first identified according to the present invention; to identify, based on the presence of said responsible mutations, patients with said cancer or subjects with a risk of said cancer, in whom a cancer treatment takes effect; and to treat said patients. In other words, this invention provides stratification biomarkers for use in personalized cancer medicine as well as new targets for molecular targeted therapies against cancers.

### BRIEF DESCRIPTION OF DRAWINGS

Fig. 1 is a schematic drawing showing examples of the genome structures of ATG3 and EPHB1, and the domain structure of an ATG3-EPHB1 fusion polypeptide (SEQ ID NO: 2).
Fig. 2 is a schematic drawing showing examples of the genome structures of TNIK and RNF123, and the domain structure of a TNIK-RNF123 fusion polypeptide (SEQ ID NO: 4).
Fig. 3 is a schematic drawing showing examples of the genome structures of SLC 12A2 and NRG2, and the domain structure of an SLC12A2-NRG2 fusion polypeptide (SEQ ID NO: 28).
Fig. 4 is a schematic drawing showing examples of the genome structures of SLC 12A2 and NRG2, and the domain structure of an SLC12A2-NRG2 fusion polypeptide (SEQ ID NO: 20).

### DESCRIPTION OF EMBODIMENTS

As disclosed below in Examples, the present inventors first found, in cancer tissues, the following three types of gene fusions serving as responsible mutations (driver mutations) for cancer: ATG3-EPHB1 gene fusion, TNIK-RNF123 gene fusion, and SLC12A2-NRG2 gene fusion. On the basis of this finding, the present invention mainly provides a method for detecting said gene fusions; a method for identifying, based on the presence of said responsible mutations, patients with cancer or subjects with a risk of cancer, in whom cancer treatments take effect; a method for treatment of cancer; a cancer therapeutic agent; and a method for screening a cancer therapeutic agent.

For the purpose of the present invention, the term "responsible mutations for cancer" is a term used interchangeably with the term "driver mutations", and refers to mutations that are present in cancer tissues and which are capable of inducing carcinogenesis of cells. Typically speaking, if a mutation is found in a cancer tissue in which none of known oncogene mutations exists (in other words, if a mutation exists in a mutually exclusive manner with the known oncogene mutations), then the mutation can be determined to be a responsible mutation for cancer.

### <Specific responsible mutations for cancer>

Hereunder, the respective gene fusions will be explained. For the purpose of the present specification, the "point of fusion" in a fusion polynucleotide refers to a boundary that connects gene segments each extending toward the 5'- or 3'-end, in other words, a boundary between two nucleotide residues. The "point of fusion" in a fusion polypeptide refers to a boundary that connects polypeptides each extending toward the N- or C-terminus, in other words, a boundary between two amino acid residues, or, if a gene fusion occurs in one codon, one amino acid residue *per se* encoded by the codon.

### (1) ATG3-EPHB1 gene fusion

This gene fusion is a mutation that expresses a fusion protein between ATG3 protein and EPHB1 protein (hereinafter also referred to as the "ATG3-EPHB1 fusion polypeptide") and which is caused by an inversion (inv3) having breakpoints in regions 3q13.2 and 3q21-q23 of a human chromosome.

ATG3 protein is a protein encoded by the gene located on chromosome 3q13.2 in human, and is typified by a protein consisting of the amino acid sequence of SEQ ID NO: 30 (NCBI accession No. NP_071933.2 (29-Sep-2013)). ATG3 protein is an E2-like conjugation enzyme essential for autophagy (Tanida I., Tanida-Miyake E., Komatsu M., Ueno T., Kominami E., "Human Apg3p/Autlp homologue is an authentic E2 enzyme for multiple substrates, GATE-16, GABARAP, and MAP-LC3, and facilitates the conjugation of hApg12p to hApg5p.", JBiol Chem., 19 Apr. 2002; 277 (16): 13739-44).

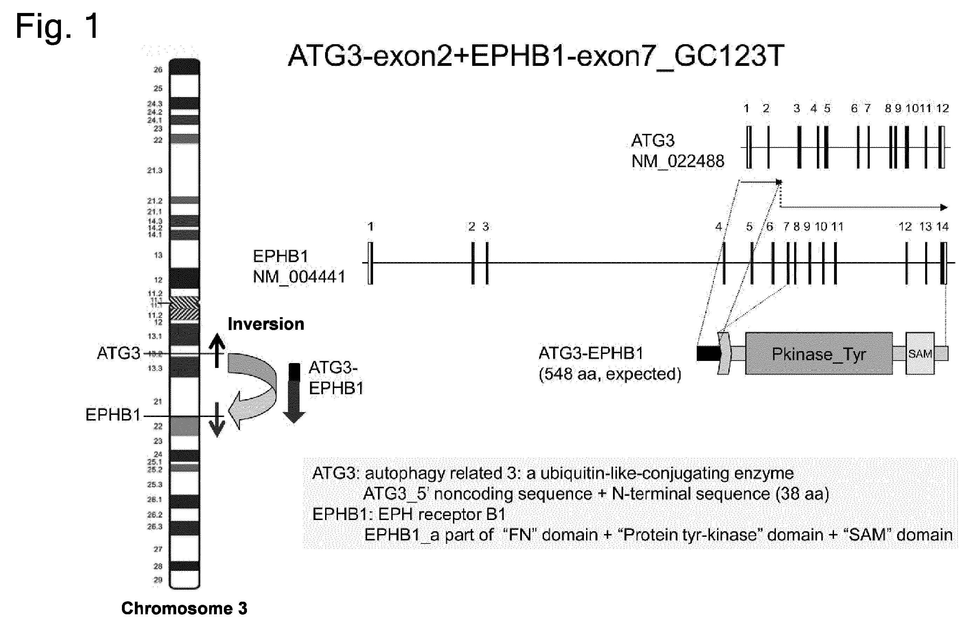

EPHB1 protein is a protein encoded by the gene located on chromosome 3q21-q23 in human, and is typified by a protein consisting of the amino acid sequence of SEQ ID NO: 32 (NCBI accession No. NP_004432.1 (25-Aug-2013)). EPHB1 protein is a transmembrane-ephrin receptor protein belonging to the receptor tyrosine kinase family (Vindis C., Cerretti D. P., Daniel T. O., Huynh-Do U., "EphBl recruits c-Src and p52Shc to activate MAPK/ERK and promote chemotaxis.", J Cell Biol., 18 Aug. 2003; 162 (4): 661-71). EPHB1 protein is characterized by having the kinase domain (Fig. 1), which corresponds to, for example, the amino acid sequence at positions 619 to 878 of the amino acid sequence of SEQ ID NO: 32 in humans.

In EPHB1 protein, a SAM domain (e.g., positions 908 to 975 of the amino acid sequence of SEQ ID NO: 32) is present in a region toward the C-terminus relative to the kinase domain.

The ATG3-EPHB1 fusion polypeptide is a polypeptide comprising the kinase domain of EPHB1 protein and having kinase activity.

The ATG3-EPHB1 fusion polypeptide can contain all of the kinase domain of EPHB1 protein, or can contain part of said kinase domain as long as the ATG3-EPHB1 fusion polypeptide has kinase activity.

The expression saying that the ATG3-EPHB1 fusion polypeptide "has kinase activity" means that said fusion polypeptide is active as an enzyme phosphorylating tyrosine due to the kinase domain derived from EPHB1 protein. The kinase activity of the ATG3-EPHB1 fusion polypeptide is determined by a conventional method, and is commonly determined by incubating the fusion polypeptide with a substrate (*e.g.*, synthetic peptide substrate) and ATP under appropriate conditions and then detecting phosphorylated tyrosine in the substrate. The kinase activity can also be determined using a commercially available measurement kit.

The ATG3 protein-derived sequence present in the ATG3-EPHB1 fusion polypeptide is not particularly limited.

In the present invention, the polynucleotide encoding the ATG3-EPHB1 fusion polypeptide (hereinafter also referred to as the "ATG3-EPHB1 fusion polynucleotide") is a polynucleotide that encodes a polypeptide comprising the kinase domain of EPHB1 protein and having kinase activity. The ATG3-EPHB1 fusion polynucleotide can be in the form of any of mRNA, cDNA and genomic DNA.

The ATG3-EPHB1 fusion polynucleotide according to the present invention can be, for example, an ATG3-EPHB1 fusion polynucleotide encoding the polypeptide mentioned below:
(i) a polypeptide consisting of the amino acid sequence of SEQ ID NO: 2,
(ii) a polypeptide consisting of an amino acid sequence derived from the amino acid sequence of SEQ ID NO: 2 by deletion, substitution or addition of one or more amino acids, and the polypeptide having kinase activity, or
(iii) a polypeptide consisting of an amino acid sequence having a sequence identity of at least 80% to the amino acid sequence of SEQ ID NO: 2, and the polypeptide having kinase activity.

As disclosed below in Examples, the amino acid sequence of SEQ ID NO: 2 is an amino acid sequence encoded by the ATG3-EPHB1 fusion polynucleotide which was found in a sample from a human cancer tissue.

As used above in (ii), the phrase "one or more amino acids" refers to generally 1 to 50 amino acids, preferably 1 to 30 amino acids, more preferably 1 to 10 amino acids, still more preferably one to several amino acids (for example, 1 to 5 amino acids, 1 to 4 amino acids, 1 to 3 amino acids, 1 or 2 amino acids, or one amino acid).

As used above in (iii), the **"sequence identity of at least 80%"** refers to a sequence identity of preferably at least 85%, more preferably at least 90% or at least 95%, still more preferably at least 97%, at least 98% or at least 99%. Amino acid sequence identity can be determined using the BLASTX or BLASTP program (Altschul S. F., et al., J. Mol. Biol., 1990, 215: 403) which is based on the BLAST algorithm developed by Karlin and Altschul (Proc. Natl. Acad. Sci. USA, 1990, 87: 2264-2268; and Proc. Natl. Acad. Sci. USA, 1993, 90: 5873). In the process of making amino acid sequence analysis using BLASTX, the parameter setting is typically made as follows: score = 50 and wordlength = 3. In the process of making amino acid sequence analysis using the BLAST and Gapped BLAST programs, the default parameters of these programs are used. The specific procedures for conducting these analyses are well known to those skilled in the art *(e.g.,* http://www.ncbi.nlm.nih.gov/).

Also, the ATG3-EPHB1 fusion polynucleotide according to the present invention can be, for example, any one of the polynucleotides mentioned below:
(i) a polynucleotide consisting of the nucleotide sequence of SEQ ID NO: 1,
(ii) a polynucleotide that hybridizes under stringent conditions with a polynucleotide consisting of a nucleotide sequence complementary to the polynucleotide consisting of the nucleotide sequence of SEQ ID NO: 1, and which encodes a polypeptide having kinase activity,
(iii) a polynucleotide that consists of a nucleotide sequence derived from the nucleotide sequence of SEQ ID NO: 1 by deletion, substitution or addition of one or more nucleotides, and which encodes a polypeptide having kinase activity, or
(iv) a polynucleotide that has a sequence identity of at least 80% to the polynucleotide consisting of the nucleotide sequence of SEQ ID NO: 1, and which encodes a polypeptide having kinase activity.

As disclosed below in Examples, the nucleotide sequence of SEQ ID NO: 1 is a nucleotide sequence of an ATG3-EPHB1 fusion polynucleotide which was found in a sample from a human cancer tissue.

As used above in (ii), the phrase **"under stringent conditions"** refers to moderately or highly stringent conditions, unless particularly specified.

The moderately stringent conditions can be easily designed by those skilled in the art on the basis of, for example, the length of a polynucleotide of interest. Basic conditions are described in Sambrook, et al., Molecular Cloning: A Laboratory Manual, 3rd ed., ch. 6-7, Cold Spring Harbor Laboratory Press, 2001. Typically, the moderately stringent conditions comprise: prewashing of a nitrocellulose filter in 5×SSC, 0.5% SDS, 1.0 mM EDTA (pH 8.0); hybridization in ca. 50% formamide, 2-6×SSC at about 40-50°C (or any other similar hybridization solution like a **Stark's solution** in ca. 50% formamide at about 42°C); and washing of the filter in 0.5-6×SSC, 0.1% SDS at about 40°C-60°C. The moderately stringent conditions preferably comprise hybridization in 6×SSC at about 50°C, and may comprise the prewashing and/or washing under the above-mentioned conditions.

The highly stringent conditions can also be easily designed by those skilled in the art on the basis of, for example, the length of a polynucleotide of interest. The highly stringent conditions involve a higher temperature and/or a lower salt concentration than the moderately stringent conditions. Typically, the highly stringent conditions comprise hybridization in 0.2-6×SSC, preferably 6×SSC, more preferably 2×SSC, still more preferably 0.2×SSC, at about 65°C. In any case, the highly stringent conditions preferably comprise washing in 0.2×SSC, 0.1% SDS at about 65-68°C.

In any case, as a buffer for use in hybridization, prewashing and washing, SSPE (1×SSPE: 0.15 M NaCl, 10 mM NaH₂PO₄, and 1.25 mM EDTA, pH 7.4) can be used in place of SSC (1×SSC: 0.15 M NaCl and 15 mM sodium citrate). In any case, washing can be done for about 15 minutes after the completion of hybridization.

As used above in (iii), the phrase "one or more nucleotides" refers to generally 1 to 50 nucleotides, preferably 1 to 30 nucleotides, more preferably 1 to 10 nucleotides, still more preferably one to several nucleotides (for example, 1 to 5 nucleotides, 1 to 4 nucleotides, 1 to 3 nucleotides, 1 or 2 nucleotides, or one nucleotide).

As used above in (iv), the "sequence identity of at least 80%" refers to a sequence identity of preferably at least 85%, more preferably at least 90% or at least 95%, still more preferably at least 97%, at least 98% or at least 99%. Nucleotide sequence identity can be determined using the BLASTN program (Altschul S. F., et al., J. Mol. Biol., 1990, 215: 403) which is based on the above-mentioned BLAST algorithm. In the process of making nucleotide sequence analysis using BLASTN, the parameter setting is typically made as follows: score = 100 and wordlength = 12.

### (2) TNIK-RNF123 gene fusion

This gene fusion is a mutation that expresses a fusion protein between TNIK protein and RNF123 protein (hereinafter also referred to as the **"TNIK-RNF123 fusion polypeptide")** and which is caused by an inversion (inv3) having breakpoints in regions 3q26.31 and 3p24.3 of a human chromosome.

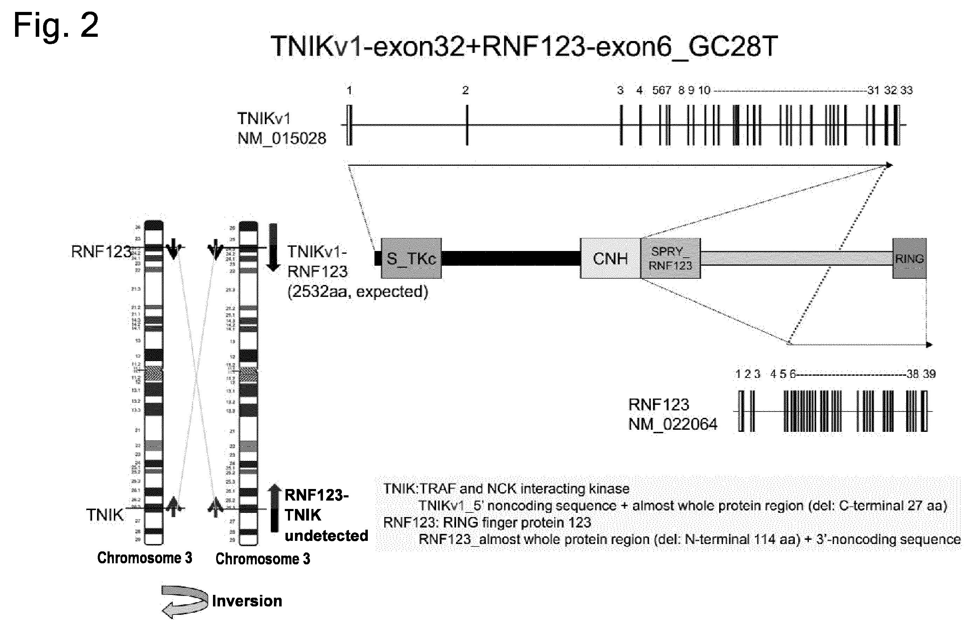

TNIK protein is a protein encoded by the gene located on chromosome 3q26.31 in human, and is typified by a protein consisting of the amino acid sequence of SEQ ID NO: 34, 36, 38, 40, 42, 44, 46 or 48 (NCBI accession No. NP_055843.1 (17-Apr-2013), NP_001155032.1 (17-Apr-2013), NP_001155033.1 (17-Apr-2013), NP_001155034.1 (17-Apr-2013), NP_001155035.1 (17-Apr-2013), NP_001155036.1 (17-Apr-2013), NP_001155037.1 (17-Apr-2013), or NP_001155038.1 (17-Apr-2013)). TNIK protein has the serine/threonine kinase domain at the N-terminus and the CNH (GCK) domain at the C-terminus (Fig. 2), and is known to be involved, for example, in cytoskeleton regulation (Fu C. A., Shen M., Huang B. C., Lasaga J., Payan D. G., Luo Y., "TNIK, a novel member of the germinal center kinase family that activates the c-Jun N-terminal kinase pathway and regulates the cytoskeleton.", JBiol Chem., 22 Oct. 1999; 274 (43): 30729-37) and in Wnt signaling activation (Mahmoudi T., Li V. S., Ng S. S., Taouatas N., Vries R. G., Mohammed S., Heck A. J., Clevers H., "The kinase TNIK is an essential activator of Wnt target genes.", EMBO J., 4 Nov. 2009; 28 (21): 3329-40). The kinase domain corresponds to, for example, the amino acid sequence at positions 25 to 289 of the amino acid sequence of SEQ ID NO: 34 in humans. The CNH domain corresponds to, for example, the amino acid sequence at positions 1042 to 1340 of the amino acid sequence of SEQ ID NO: 34 in humans.

RNF123 protein is a protein encoded by the gene located on chromosome 3p24.3 in human, and is typified by a protein consisting of the amino acid sequence of SEQ ID NO: 50 (NCBI accession No. NP_071347.2 (26-Aug-2013)). RNF123 protein is an E3 ubiquitin ligase enzyme which ubiquitinates cyclin-dependent kinase inhibitor 1B (CDKN1B = p27, Kip1) (Kamura T., Hara T., Matsumoto M., Ishida N., Okumura F., Hatakeyama S., Yoshida M., Nakayama K., Nakayama K. I., "Cytoplasmic ubiquitin ligase KPC regulates proteolysis of p27(Kipl) at G1 phase.", Nat Cell Biol., Dec. 2004; 6 (12): 1229-35). RNF123 protein is characterized by having the SPRY domain and the RING finger domain (Fig. 2). The SPRY domain corresponds to, for example, the amino acid sequence at positions 132 to 251 of the amino acid sequence of SEQ ID NO: 50 in humans. The RING finger domain corresponds to, for example, the amino acid sequence at positions 1254 to 1295 of the amino acid sequence of SEQ ID NO: 50 in humans.

The TNIK-RNF123 fusion polypeptide is a polypeptide comprising the kinase domain of TNIK protein and the SPRY domain and RING finger domain of RNF123 and having kinase activity.

The TNIK-RNF123 fusion polypeptide can contain all of the kinase domain of TNIK protein, or can contain part of said kinase domain as long as the TNIK-RNF123 fusion polypeptide has kinase activity.

The expression saying that the TNIK-RNF123 fusion polypeptide "has kinase activity" means that said fusion polypeptide is active as an enzyme phosphorylating serine or threonine due to the kinase domain derived from TNIK protein. The kinase activity of the TNIK-RNF123 fusion polypeptide is determined by a conventional method, and is commonly determined by incubating the fusion polypeptide with a substrate (e.g., synthetic peptide substrate) and ATP under appropriate conditions and then detecting phosphorylated serine or threonine in the substrate. The kinase activity can also be determined using a commercially available measurement kit.

The TNIK-RNF123 fusion polypeptide can contain all of the SPRY domain and the RING finger domain of RNF123 protein, or can contain part of the SPRY domain and the RING finger domain as long as their original functions in RNF 123 protein can be retained. The expression saying that part of the SPRY domain and the RING finger domain retains their original functions in RNF123 protein means that RNF123 protein containing part of the SPRY domain and RING finger domain has E3 activity. The E3 activity can be determined by, for example, a method described in Kamura, T., et al., Nature Cell Biology, 2004, 6 (12), 1229-35.

In the present invention, the polynucleotide encoding the TNIK-RNF123 fusion polypeptide (hereinafter also referred to as the "TNIK-RNF123 fusion polynucleotide") is a polynucleotide that encodes the polypeptide comprising the kinase domain of TNIK protein and the SPRY domain and RING finger domain of RNF123, and having kinase activity. The TNIK-RNF123 fusion polynucleotide can be in the form of any of mRNA, cDNA and genomic DNA.

The TNIK-RNF123 fusion polynucleotide according to the present invention can be, for example, a TNIK-RNF123 fusion polynucleotide encoding the polypeptide mentioned below:
(i) a polypeptide consisting of the amino acid sequence of SEQ ID NO: 4, 6, 8, 10, 12, 14, 16 or 18,
(ii) a polypeptide consisting of an amino acid sequence derived from the amino acid sequence of SEQ ID NO: 4, 6, 8, 10, 12, 14, 16 or 18 by deletion, substitution or addition of one or more amino acids, and the polypeptide having kinase activity, or
(iii) a polypeptide consisting of an amino acid sequence having a sequence identity of at least 80% to the amino acid sequence of SEQ ID NO: 4, 6, 8, 10, 12, 14, 16 or 18, and the polypeptide having kinase activity.

As disclosed below in Examples, the amino acid sequence of SEQ ID NO: 4, 6, 8, 10, 12, 14, 16 or 18 is an amino acid sequence encoded by the TNIK-RNF123 fusion polynucleotide comprising partial sequences, which was found in a sample from a human cancer tissue.

The phrases **"one or more amino acids"** and **"sequence identity of at least 80%"** as used above in (ii) and (iii), respectively, have the same meanings as described above in "(1) ATG3-EPHB1 gene fusion".

Also, the TNIK-RNF123 fusion polynucleotide according to the present invention can be, for example, any one of the polynucleotides mentioned below:
(i) a polynucleotide consisting of the nucleotide sequence of SEQ ID NO: 3, 5, 7, 9, 11, 13, 15 or 17,
(ii) a polynucleotide that hybridizes under stringent conditions with a polynucleotide consisting of a nucleotide sequence complementary to the polynucleotide consisting of the nucleotide sequence of SEQ ID NO: 3, 5, 7, 9, 11, 13, 15 or 17, and which encodes a polypeptide having kinase activity,
(iii) a polynucleotide that consists of a nucleotide sequence derived from the nucleotide sequence of SEQ ID NO: 3, 5, 7, 9, 11, 13, 15 or 17 by deletion, substitution or addition of one or more nucleotides, and which encodes a polypeptide having kinase activity, or
(iv) a polynucleotide that has a sequence identity of at least 80% to the polynucleotide consisting of the nucleotide sequence of SEQ ID NO: 3, 5, 7, 9, 11, 13, 15 or 17, and which encodes a polypeptide having kinase activity.

As disclosed below in Examples, the nucleotide sequence of SEQ ID NO: 3 is a nucleotide sequence of a TNIK-RNF123 fusion polynucleotide comprising partial sequences, which was found in a sample from a human cancer tissue.

The phrases **"under stringent conditions",** "one **or more nucleotides",** and **"sequence identity of at least 80%"** as used above in (ii), (iii) and (iv), respectively, have the same meanings as described above in "(1) ATG3-EPHB1 gene fusion".

### (3) SLC12A2-NRG2 gene fusion

This gene fusion is a mutation that expresses a fusion protein between SLC 12A2 protein and NRG2 protein (hereinafter also referred to as the "SLC12A2-NRG2 fusion polypeptide") and which is caused by an inversion (inv5) having breakpoints in regions 5q23.3 and 5q23-q33 of a human chromosome.

SLC12A2 protein is a protein encoded by the gene located on chromosome 5q23.3 in humans, and is typified by a protein consisting of the amino acid sequence of SEQ ID NO: 52 or 54 (NCBI accession No. NP_001037.1 (22-Sep-2013) or NP_001243390.1 (28-Sep-2013)). SLC12A2 protein is a membrane protein functioning as a Na-K-Cl cotransporter (Somasekharan S., Tanis J., Forbush B., "Loop diuretic and ion-binding residues revealed by scanning mutagenesis of transmembrane helix 3 (TM3) of Na-K-Cl cotransporter (NKCC1).", JBiol Chem., 18 May 2012; 287 (21): 17308-17), and having the AA_permease_N super family domain at the N-terminus (Fig. 1). This domain corresponds to, for example, the amino acid sequence at positions 210 to 267 of the amino acid sequence of SEQ ID NO: 52 in humans.

NRG2 protein is a protein encoded by the gene located on chromosome 5q23-q33 in humans, and is typified by a protein consisting of the amino acid sequence of SEQ ID NO: 56, 58, 60, 62 or 64 (NCBI accession No. NP_004874.1 (25-Aug-2013), NP_053584.1 (25-Aug-2013), NP_053585.1 (25-Aug-2013), NP_053586.1 (25-Aug-2013) or NP_001171864.1 (25-Aug-2013)). NRG2 protein is a protein belonging to the neuregulin family which controls cell growth and differentiation through interaction with ERBB family receptors (Chang H., Riese D. J. 2nd, Gilbert W., Stern D. F., McMahan U. J., "Ligands for ErbB-family receptors encoded by a neuregulin-like gene.", Nature, 29 May 1997; 387 (6632): 509-12; Carraway K. L. 3rd, Weber J. L., Unger M. J., Ledesma J., Yu N., Gassmann M., Lai C., "Neuregulin-2, a new ligand of ErbB3/ErbB4-receptor tyrosine kinases.", Nature, 29 May 1997; 387 (6632): 512-6; Hobbs S. S., Coffing S. L., Le A. T., Cameron E. M., Williams E. E., Andrew M., Blommel E. N., Hammer R. P., Chang H., Riese D. J. 2nd., "Neuregulin isoforms exhibit distinct patterns of ErbB family receptor activation.", Oncogene, 5 Dec 2002; 21 (55): 8442-52; and Nakano N., Higashiyama S., Ohmoto H., Ishiguro H., Taniguchi N., Wada Y., "The N-terminal region of NTAK/neuregulin-2 isoforms has an inhibitory activity on angiogenesis.", JBiol Chem., 19 Mar 2004; 279 (12): 11465-70.). NRG2 protein is characterized by having the neuregulin family conserved region (Fig. 1). This domain corresponds to, for example, the amino acid sequence at positions 398 to 718 of the amino acid sequence of SEQ ID NO: 56 in humans.

In NRG2 protein, the I-set domain (*e.g.*, positions 239 to 328 of the amino acid sequence of SEQ ID NO: 56) is present in a region toward the N-terminus relative to the neuregulin family conserved region. Further, there is another isoform having the EGF domain (*e.g.*, positions 353 to 381 of the amino acid sequence of SEQ ID NO: 56) between the I-set domain and the neuregulin family conserved region.

The SLC12A2-NRG2 fusion polypeptide is a polypeptide comprising the AA_permease_N super family domain of SLC12A2 protein and the neuregulin family conserved region of NRG2 protein, and having intracellular signaling-enhancing activity.

The SLC12A2-NRG2 fusion polypeptide can contain all of the AA_permease_N super family domain of SLC 12A2 protein, or can contain part of the AA_permease_N super family domain as long as the SLC12A2-NRG2 fusion polypeptide has intracellular signaling-enhancing activity.

The SLC12A2-NRG2 fusion polypeptide can contain all of the neuregulin family conserved region of NRG2 protein, or can contain part of the neuregulin family conserved region as long as the SLC12A2-NRG2 fusion polypeptide has intracellular signaling-enhancing activity.

The expression saying that the SLC12A2-NRG2 fusion polypeptide "has intracellular signaling-enhancing activity" means that said fusion polypeptide is active in enhancing intracellular signaling due to the neuregulin family conserved region derived from NRG2 protein. This activity is determined by the following method described in Wilson, T. R., et al., Cancer Cell, 2011, 20, 158-172.

A test substance is added to EFM-19 cells (DSMZ, No. ACC-231) cultured in a serum-starved condition, the cells are treated for 30 minutes and then lysed to extract protein. The phosphorylation of EGFR, ERBB2, ERBB3 or ERBB4 is analyzed by Western blotting. If phosphorylation is higher than in the case where no test substance is added, it is determined that the test substance has intracellular signaling-enhancing activity. As the test substance as referred to herein, the entire SLC12A2-NRG2 fusion polypeptide may be used, or a fragment thereof which lacks a transmembrane domain but contains the neuregulin family conserved region may be used in consideration of solubility or other factors.

The SLC12A2-NRG2 fusion polypeptide can contain all or part of the I-set domain of NRG2 protein as long as said fusion polypeptide has intracellular signaling-enhancing activity.

The SLC12A2-NRG2 fusion polypeptide can contain all or part of the EGF domain of NRG2 protein as long as said fusion polypeptide has intracellular signaling-enhancing activity.

In the present invention, the polynucleotide encoding the SLC12A2-NRG2 fusion polypeptide (hereinafter also referred to as the "SLC12A2-NRG2 fusion polynucleotide") is a polynucleotide that encodes the polypeptide comprising the AA_permease_N super family domain of SLC12A2 protein and the neuregulin family conserved region of NRG2 protein, and having intracellular signaling-enhancing activity. The SLC12A2-NRG2 fusion polynucleotide can be in the form of any of mRNA, cDNA and genomic DNA.

The SLC12A2-NRG2 fusion polynucleotide according to the present invention can be, for example, an SLC12A2-NRG2 fusion polynucleotide encoding the polypeptide mentioned below:
(i) a polypeptide consisting of the amino acid sequence of SEQ ID NO: 20, 22, 24, 26 or 28,
(ii) a polypeptide consisting of an amino acid sequence derived from the amino acid sequence of SEQ ID NO: 20, 22, 24, 26 or 28 by deletion, substitution or addition of one or more amino acids, and the polypeptide having intracellular signaling-enhancing activity, or
(iii) a polypeptide consisting of an amino acid sequence having a sequence identity of at least 80% to the amino acid sequence of SEQ ID NO: 20, 22, 24, 26 or 28, and the polypeptide having intracellular signaling-enhancing activity.

As disclosed below in Examples, the amino acid sequence of SEQ ID NO: 20, 22, 24, 26 or 28 is an amino acid sequence encoded by the SLC12A2-NRG2 fusion polynucleotide comprising partial sequences, which was found in a sample from a human cancer tissue.

The phrases "one or more amino acids" and "sequence identity of at least 80%" as used above in (ii) and (iii), respectively, have the same meanings as described above in "(1) ATG3-EPHB1 gene fusion".

Also, the SLC12A2-NRG2 fusion polynucleotide according to the present invention can be, for example, any one of the polynucleotides mentioned below:
(i) a polynucleotide consisting of the nucleotide sequence of SEQ ID NO: 19, 21, 23, 25 or 27,
(ii) a polynucleotide that hybridizes under stringent conditions with a polynucleotide consisting of a nucleotide sequence complementary to the polynucleotide consisting of the nucleotide sequence of SEQ ID NO: 19, 21, 23, 25 or 27, and which encodes a polypeptide having intracellular signaling-enhancing activity,
(iii) a polynucleotide that consists of a nucleotide sequence derived from the nucleotide sequence of SEQ ID NO: 19, 21, 23, 25 or 27 by deletion, substitution or addition of one or more nucleotides, and which encodes a polypeptide having intracellular signaling-enhancing activity, or
(iv) a polynucleotide that has a sequence identity of at least 80% to the polynucleotide consisting of the nucleotide sequence of SEQ ID NO: 19, 21, 23, 25 or 27, and which encodes a polypeptide having intracellular signaling-enhancing activity.

As disclosed below in Examples, the nucleotide sequence of SEQ ID NO: 19, 21, 23, 25 or 27 is a nucleotide sequence of an SLC12A2-NRG2 fusion polynucleotide comprising partial sequences, which was found in a sample from a human cancer tissue.

The phrases "under stringent conditions", "one or more nucleotides", and "sequence identity of at least 80%" as used above in (ii), (iii) and (iv), respectively, have the same meanings as described above in "(1) ATG3-EPHB1 gene fusion".

<Method for detecting gene fusions serving as responsible mutations (driver mutations) for cancer>

The present invention provides a method for detecting the above-mentioned three types of gene fusions (hereinafter also referred to as the "inventive detection method"). The inventive detection method comprises the step of detecting any one of the above-mentioned fusion polynucleotides, or a polypeptide encoded thereby, in an isolated sample from a subject.

In the inventive detection method, the subject is not particularly limited as long as it is a mammal. Examples of the mammal include: rodents such as mouse, rat, hamster, chipmunk and guinea pig; rabbit, pig, cow, goat, horse, sheep, mink, dog, cat; and primates such as human, monkey, cynomolgus monkey, rhesus monkey, marmoset, orangutan, and chimpanzee, with human being preferred.

The subject may be not only a subject affected by cancer, but also a subject suspected of being affected by cancer or a subject with a future risk of cancer. The "cancer" to which the inventive detection method is to be applied is not particularly limited as long as it is a cancer in which any of the above-mentioned three types of gene fusions can be detected, with gastric cancer being preferred, and special-type gastric cancer, undifferentiated gastric cancer, or moderately differentiated gastric cancer being more preferred.

The "isolated sample" from the subject encompasses not only biological samples (for example, cells, tissues, organs (e.g., small intestine, spleen, kidney, liver, stomach, lung, adrenal gland, heart, brain, pancreas, and aorta), body fluids *(e.g.,* blood, lymphs, bone marrow fluid), digestive juices, sputum, bronchoalveolar/bronchial lavage fluids, urine, and feces), but also nucleic acid extracts from these biological samples (e.g., genomic DNA extracts, mRNA extracts, and cDNA and cRNA preparations from mRNA extracts) and protein extracts. The genomic DNA, mRNA, cDNA or protein can be prepared by those skilled in the art through considering various factors including the type and state of the sample and selecting a known technique suitable therefor. The sample may also be the one that is fixed with formalin or alcohol, frozen, or embedded in paraffin.

Further, the "isolated sample" is preferably derived from an organ having or suspected of having the above-mentioned cancer, and more preferably derived from the stomach having or suspected of having the above-mentioned cancer. Examples of the "isolated sample" include, but are not limited to, a gastric tissue from a patient with gastric cancer (preferably, special-type gastric cancer, undifferentiated gastric cancer, or moderately differentiated gastric cancer) or a subject suspected of being affected by gastric cancer, as well as total RNA extracted from said gastric tissue.

In the inventive detection method, the detection of a fusion polynucleotide or a polypeptide encoded thereby can be made using a *per se* known technique.

If the object to be detected is a transcript from a genomic DNA (mRNA, or cDNA prepared from mRNA), a fusion polynucleotide in the form of mRNA or cDNA can be detected using, for example, RT-PCR, sequencing, TaqMan probe method, Northern blotting, dot blotting, or cDNA microarray analysis.

If the object to be detected is a genomic DNA, a fusion polynucleotide in the form of genomic DNA can be detected using, for example, *in situ* hybridization (ISH), genomic PCR, sequencing, TaqMan probe method, Southern blotting, or genome microarray analysis.

Any of the above-mentioned detection techniques can be used alone or in combination. For example, since the above-mentioned three types of gene fusions are believed to contribute to oncogenesis by expressing fusion polypeptides, it is also preferred that if a fusion polynucleotide in the form of genomic DNA is detected (*e.g.,* by *in situ* hybridization or the like), production of a transcript or a protein should be further confirmed *(e.g.,* by RT-PCR, immunostaining or the like).

If a fusion polynucleotide is detected by a hybridization technique (e.g., TaqMan probe method, Northern blotting, Southern blotting, dot blotting, microarray analysis, *in situ* hybridization (ISH)), detection can be made using a polynucleotide that serves as a probe designed to specifically recognize the fusion polynucleotide. As used herein, the phrase "specifically recognize the fusion polynucleotide" means that under stringent conditions, the probe distinguishes and recognizes the fusion polynucleotide from other polynucleotides, including wild-type genes from which to derive both segments of the fusion polynucleotide each extending from the point of fusion toward the 5'- or 3'-end.

Since biological samples (e.g., biopsy samples) obtained in the course of treatment or diagnosis are often fixed in formalin, it is preferred to use *in situ* hybridization in the inventive detection method, from the viewpoints that the genomic DNA to be detected is stable even when fixed in formalin and that this technique is high in detection sensitivity.

According to the *in situ* hybridization method, the genomic DNA (fusion polynucleotide) encoding a fusion polypeptide can be detected by hybridizing, to such a biological sample, the following polynucleotide (a) or (b) which has a chain length of at least 15 nucleotides and serves as a probe(s) designed to specifically recognize said fusion polynucleotide:
(a) a polynucleotide serving as at least one probe for each of the above-mentioned fusion genes, as selected from the group consisting of a probe that hybridizes to the nucleotide sequence of the fusion partner gene toward the 5'-end (ATG3, TNIK or SLC12A2 gene) and a probe that hybridizes to the nucleotide sequence of the fusion partner gene toward the 3'-end (EPHB1, RNF 123 or NRG2 gene); or
(b) a polynucleotide serving as a probe for each of the above-mentioned fusion genes, which hybridizes to a nucleotide sequence containing the point of fusion between the fusion partner gene toward the 5'-end and the fusion partner gene toward the 3'-end.

The ATG3 gene according to the present invention, as far as it is derived from humans, is typified by a gene consisting of the DNA sequence (encoded into complementary strand) from position 112251354 to position 112280810 in the genome sequence identified in Genbank accession No. NC_000003.11.

The TNIK gene according to the present invention, as far as it is derived from humans, is typified by a gene consisting of the DNA sequence (encoded into complementary strand) from position 170780292 to position 171178197 in the genome sequence identified in Genbank accession No. NC_000003.11.

The SLC12A2 gene according to the present invention, as far as it is derived from humans, is typified by a gene consisting of the DNA sequence from position 127419483 to position 127525380 in the genome sequence identified in Genbank accession No. NC_000005.9.

The EPHB1 gene according to the present invention, as far as it is derived from humans, is typified by a gene consisting of the DNA sequence from position 134514099 to position 134979309 in the genome sequence identified in Genbank accession No. NC_000003.11.

The RNF123 gene according to the present invention, as far as it is derived from humans, is typified by a gene consisting of the DNA sequence from position 49726950 to position 49758962 in the genome sequence identified in Genbank accession No. NC_000003.11.

The NRG2 gene according to the present invention, as far as it is derived from humans, is typified by a gene consisting of the DNA sequence (encoded into complementary strand) from position 139226364 to position 139422884 in the genome sequence identified in Genbank accession No. NC_000005.9.

However, the DNA sequences of genes can change in nature (*i.e*., in a non-artificial way) due to their mutations and the like. Thus, such native mutants can also be encompassed by the present invention (the same applies hereinafter).

The polynucleotide mentioned in (a) according to the present invention can be of any type as far as it is capable of detecting the presence of the genomic DNA encoding a fusion polypeptide in the above-mentioned biological sample by hybridizing to a nucleotide sequence(s) targeted by said polynucleotide, *i.e*., the nucleotide sequence of the fusion partner gene toward the 5'-end (ATG3, TNIK or SLC12A2 gene) and/or the nucleotide sequence of the fusion partner gene toward the 3'-end (EPHB1, RNF123 or NRG2 gene); preferably, the polynucleotide (a) is any of the polynucleotides mentioned below in (a1) to (a3):
(a1) a combination of a polynucleotide that hybridizes to the nucleotide sequence of that region of the fusion partner gene toward the 5'-end which extends upstream from its breakpoint toward the 5'-end (this polynucleotide is hereinafter also referred to as the "5' fusion partner gene probe 1"), and a polynucleotide that hybridizes to the nucleotide sequence of that region of the fusion partner gene toward the 3'-end which extends downstream from its breakpoint toward the 3'-end (this polynucleotide is hereinafter also referred to as the "3' fusion partner gene probe 1");
(a2) a combination of a polynucleotide that hybridizes to the nucleotide sequence of that region of the fusion partner gene toward the 5'-end which extends upstream from its breakpoint toward the 5'-end (this polynucleotide is hereinafter also referred to as the "5' fusion partner gene probe 1"), and a polynucleotide that hybridizes to the nucleotide sequence of that region of the fusion partner gene toward the 5'-end which extends downstream from its breakpoint toward the 3'-end (this polynucleotide is hereinafter also referred to as the "5' fusion partner gene probe 2"); and
(a3) a combination of a polynucleotide that hybridizes to the nucleotide sequence of that region of the fusion partner gene toward the 3'-end which extends upstream from its breakpoint toward the 5'-end (this polynucleotide is hereinafter also referred to as the "3' fusion partner gene probe 2"), and a polynucleotide that hybridizes to the nucleotide sequence of that region of the fusion partner gene toward the 3'-end which extends downstream from its breakpoint toward the 3'-end (this polynucleotide is hereinafter also referred to as the "3' fusion partner gene probe 1").

In the context of (a1) to (a3) above, when the fusion partner gene toward the 5'-end is the ATG3 gene, the nucleotide sequence of that region of the fusion partner gene toward the 5'-end which extends upstream from its breakpoint toward the 5'-end typically contains exon 2 (at positions 508 to 549 in the nucleotide sequence of SEQ ID NO: 29) of the ATG3 gene, and its upstream region.

In the context of (a1) to (a3) above, when the fusion partner gene toward the 3'-end is the EPHB1 gene, the nucleotide sequence of that region of the fusion partner gene toward the 3'-end which extends downstream from its breakpoint toward the 3'-end typically contains exon 7 (at positions 1798 to 1960 in the nucleotide sequence of SEQ ID NO: 31) of the EPHB1 gene, and its downstream region.

In the context of (a1) to (a3) above, when the fusion partner gene toward the 5'-end is the TNIK gene, the nucleotide sequence of that region of the fusion partner gene toward the 5'-end which extends upstream from its breakpoint toward the 5'-end typically contains exon 32 (at positions 4205 to 4344 in the nucleotide sequence of SEQ ID NO: 33) of the TNIK gene, and its upstream region.

In the context of (a1) to (a3) above, when the fusion partner gene toward the 3'-end is the RNF123 gene, the nucleotide sequence of that region of the fusion partner gene toward the 3'-end which extends downstream from its breakpoint toward the 3'-end typically contains exon 6 (at positions 469 to 523 in the nucleotide sequence of SEQ ID NO: 49) of the RNF123 gene, and its downstream region.

In the context of (a1) to (a3) above, when the fusion partner gene toward the 5'-end is the SLC12A2 gene, the nucleotide sequence of that region of the fusion partner gene toward the 5'-end which extends upstream from its breakpoint toward the 5'-end typically contains exon 4 (at positions 1117 to 1212 in the nucleotide sequence of SEQ ID NO: 51) of the SLC12A2 gene, and its upstream region.

In the context of (a1) to (a3) above, when the fusion partner gene toward the 3'-end is the NRG2 gene, the nucleotide sequence of that region of the fusion partner gene toward the 3'-end which extends downstream from its breakpoint toward the 3'-end typically contains exon 2 (at positions 931 to 1102 in the nucleotide sequence of SEQ ID NO: 55) of the NRG2 gene, and its downstream region.

The polynucleotides mentioned above in (a1) can be exemplified by the polynucleotide combinations mentioned below in (a1-1) to (a1-3):
(a1-1) a combination of a polynucleotide that hybridizes to exon 2 of the ATG3 gene, and a polynucleotide that hybridizes to exon 7 of the EPHB1 gene;
(a1-2) a combination of a polynucleotide that hybridizes to exon 32 of the TNIK gene, and a polynucleotide that hybridizes to exon 6 of the RNF123 gene; and
(a1-3) a combination of a polynucleotide that hybridizes to exon 4 of the SLC12A2 gene, and a polynucleotide that hybridizes to exon 2 of the NRG2 gene.

In the present invention, the region to which the polynucleotide for use for *in situ* hybridization as mentioned above in (a) is to hybridize (such a region is hereinafter referred to as the "target nucleotide sequence") is preferred to be located not more than 1000000 nucleotides away from the point of fusion between the fusion partner gene toward the 5'-end (ATG3, TNIK or SLC12A2 gene) and the fusion partner gene toward the 3'-end (EPHB1, RNF123 or NRG2 gene), from the viewpoints of specificity for the target nucleotide sequence and detection sensitivity.

In the present invention, the polynucleotide for use for *in situ* hybridization as mentioned above in (b) can be of any type as far as it is capable of detecting the presence of the genomic DNA encoding a fusion polypeptide in the above-mentioned biological sample by hybridizing to a nucleotide sequence targeted by said polynucleotide, *i.e*., a nucleotide sequence containing the point of fusion between the fusion partner gene toward the 5'-end and the fusion partner gene toward the 3'-end; and typical examples of the polynucleotide (b) are those which each hybridize to a nucleotide sequence containing the point of fusion in the nucleotide sequence of SEQ ID NO: 1, 3, 5, 7, 9, 11, 13, 15, 17, 19, 21, 23, 25 or 27.

Further, in the present invention, the polynucleotide for use for *in situ* hybridization as mentioned above in (a) or (b) is preferred to be a group consisting of multiple types of polynucleotides which can cover the entire target nucleotide sequence, from the viewpoints of specificity for the target nucleotide sequence and detection sensitivity. In such a case, each of the polynucleotides constituting the group has a length of at least 15 nucleotides, preferably from 100 to 1000 nucleotides.

The polynucleotide for use for *in situ* hybridization as mentioned above in (a) or (b) is preferably labeled with a fluorescent dye or the like for the purpose of detection. Examples of such a fluorescent dye include, but are not limited to, DEAC, FITC, R6G, TexRed, and Cy5. Aside from the fluorescent dye, the polynucleotide may also be labeled with a radioactive isotope (*e.g*., ¹²⁵I, ¹³¹I, ³H, ¹⁴C, ³³P, ³²P), an enzyme (*e.g*., β-galactosidase, β-glucosidase, alkaline phosphatase, peroxidase, malate dehydrogenase), or a luminescent substance (e.g., luminol, luminol derivative, luciferin, lucigenin, 3,3'-diaminobenzidine (DAB)).

When *in situ* hybridization is performed using a combination of 5' fusion partner gene probe 1 and 3' fusion partner gene probe 1, a combination of 5' fusion partner gene probe 1 and 5' fusion partner gene probe 2, or a combination of 3' fusion partner gene probe 2 and 3' fusion partner gene probe 1, the probes of each combination are preferably labeled with different dyes. If, as the result of *in situ* hybridization using such a combination of probes labeled with different dyes, an overlap is observed between the signal (*e.g.,* fluorescence) emitted from the label on 5' fusion partner gene probe 1 and the signal emitted from the label on 3' fusion partner gene probe 1, then it can be determined that a genomic DNA encoding a fusion polypeptide of interest has been detected successfully. Also, if a split is observed between the signal emitted from the label on 5' fusion partner gene probe 1 and the signal emitted from the label on 5' fusion partner gene probe 2, or between the signal emitted from the label on 3' fusion partner gene probe 2 and the signal emitted from the label on 3' fusion partner gene probe 1, then it can be determined that a genomic DNA encoding a fusion polypeptide of interest has been detected successfully.

Polynucleotide labeling can be effected by a known method. For example, polynucleotides can be labeled by nick translation or random priming, in which the polynucleotides are caused to incorporate substrate nucleotides labeled with a fluorescent dye or the like.

The conditions for hybridizing the polynucleotide mentioned above in (a) or (b) to the above-mentioned biological sample by *in situ* hybridization can vary with various factors including the length of said polynucleotide; and exemplary highly stringent hybridization conditions are 0.2×SSC at 65°C, and exemplary low stringent hybridization conditions are 2.0×SSC at 50°C. Those skilled in the art could realize comparable stringent hybridization conditions to those mentioned above, by appropriately selecting salt concentration (e.g., SSC dilution rate), temperature, and various other conditions including concentrations of surfactant (e.g., NP-40) and formamide, and pH.

Besides *in situ* hybridization, other examples of the method for detecting a genomic DNA encoding a fusion polypeptide of interest using the polynucleotide mentioned above in (a) or (b) include Southern blotting, Northern blotting and dot blotting. According to these methods, a fusion gene of interest is detected by hybridizing said polynucleotide (a) or (b) to a membrane in which a nucleic acid extract from the above-mentioned biological sample is transcribed. In the case of using said polynucleotide (a), if a polynucleotide that hybridizes to the nucleotide sequence of the fusion partner gene toward the 5'-end and a polynucleotide that hybridizes to the nucleotide sequence of a fusion partner gene toward the 3'-end both recognize the same band developed in a membrane, then it can be determined that a genomic DNA encoding a fusion polypeptide of interest has been detected successfully.

Additional examples of the method for detecting a genomic DNA encoding a fusion polypeptide of interest using said polynucleotide (b) include genome microarray analysis and DNA microarray analysis. According to these methods, the genomic DNA is detected by preparing an array in which said polynucleotide (b) is immobilized on a substrate and bringing the above-mentioned biological sample into contact with the polynucleotide immobilized on the array. The substrate is not particularly limited as long as it allows conversion of an oligo- or polynucleotide into a solid phase, and examples include glass plate, nylon membrane, microbeads, silicon chip, and capillary.

In the inventive detection method, it is also preferred to detect a fusion polynucleotide of interest using PCR.

PCR can be performed using polynucleotides serving as a pair of primers designed to specifically amplify a fusion polynucleotide using DNA (e.g., genomic DNA, cDNA) or RNA prepared from the above-mentioned biological sample as a template. As used herein, the phrase "specifically amplify a fusion polynucleotide" means that primers do not amplify wild-type genes from which to derive both segments of a fusion polynucleotide of interest each extending from the point of fusion toward the 5'- or 3'-end, but can amplify said fusion polynucleotide alone. It is acceptable to amplify all of the fusion polynucleotide or to amplify that part of the fusion polynucleotide which contains the point of fusion.

The "polynucleotides serving as a pair of primers" to be used for PCR or the like consist of a sense primer (forward primer) and an anti-sense primer (reverse primer) that specifically amplify a target fusion polynucleotide. The sense primer is designed from the nucleotide sequence of that region of said fusion polynucleotide which extends from the point of fusion toward the 5'-end. The anti-sense primer is designed from the nucleotide sequence of that region of said fusion polynucleotide which extends from the point of fusion toward the 3'-end. From the viewpoints of the accuracy and sensitivity of PCR detection, these primers are commonly designed such that amplified PCR products are not more than 5 kb in size. The primers can be designed as appropriate by a known method, for example, using the Primer Express® software (Applied Biosystems). The length of these polynucleotides is generally not less than 15 nucleotides (preferably not less than 16, 17, 18, 19 or 20 nucleotides, more preferably not less than 21 nucleotides) and not more than 100 nucleotides (preferably not more than 90, 80, 70, 60, 50 or 40 nucleotides, more preferably not more than 30 nucleotides).

Preferred examples of the "polynucleotides serving as a pair of primers" include: a primer set for the ATG3-EPHB1 fusion polynucleotide, which consists of a polynucleotide consisting of the nucleotide sequence of SEQ ID NO: 65 and a polynucleotide consisting of the nucleotide sequence of SEQ ID NO: 66; a primer set for the TNIK-RNF123 fusion polynucleotide, which consists of a polynucleotide consisting of the nucleotide sequence of SEQ ID NO: 67 and a polynucleotide consisting of the nucleotide sequence of SEQ ID NO: 68; and a primer set for the SLC12A2-NRG2 fusion polynucleotide, which consists of a polynucleotide consisting of the nucleotide sequence of SEQ ID NO: 69 and a polynucleotide consisting of the nucleotide sequence of SEQ ID NO: 70 (refer to Table 1 shown below).

In the process of detecting a fusion polynucleotide by PCR, direct sequencing is performed on PCR products to sequence a nucleotide sequence containing the point of fusion, whereby it can be confirmed that a gene segment toward the 5'-end and a gene segment toward the 3'-end are joined in-frame and/or that specified domains are contained in the fusion polynucleotide. Sequencing can be done by a known method -- it can be easily done by using a sequencer (e.g., ABI-PRISM 310 Genetic Analyzer (Applied Biosystems Inc.)) in accordance with its operating instructions.

Also, in the process of detecting a fusion polynucleotide by PCR, it can be confirmed by the TaqMan probe method that a gene segment toward the 5'-end and a gene segment toward the 3'-end are joined in-frame and/or that specified domains are contained in the fusion polynucleotide. The probe to be used in the TaqMan probe method can be exemplified by the polynucleotide mentioned above in (a) or (b). The probe is labeled with a reporter dye *(e.g.,* FAM, FITC, VIC) and a quencher *(e.g.,* TAMRA, Eclipse, DABCYL, MGB).

The above-mentioned primers and probes can be in the form of DNA, RNA, or DNA/RNA chimera, and is preferably in the form of DNA. Alternatively, the primers and probes may be the ones whose nucleotides are partially or wholly substituted with an artificial nucleic acid such as PNA (polyamide nucleic acid: a peptide nucleic acid), LNA™ (Locked Nucleic Acid; a bridged nucleic acid), ENA® (2'-O,4'-C-Ethylene-bridged Nucleic Acid), GNA (glycerol nucleic acid) or TNA (threose nucleic acid). Further, the primers and probes can be double- or single-stranded, and are preferably single-stranded.

As far as the primers and probes are capable of specifically hybridizing to a target sequence, they may contain one or more nucleotide mismatches, generally have at least 80% identity, preferably at least 90%, at least 91%, at least 92%, at least 93%, or at least 94% identity, more preferably at least 95%, at least 96%, at least 97%, at least 98%, or at least 99% identity, and most preferably 100% identity, to a sequence complementary to the target sequence.

The primers and probes can be synthesized, for example, according to a conventional method using an automatic DNA/RNA synthesizer on the basis of the information on the nucleotide sequences disclosed in the present specification.

In the inventive detection method, it is also acceptable to detect a fusion polynucleotide of interest by whole-transcriptome sequencing (RNA sequencing) or genome sequencing. These techniques can be carried out, for example, using a next-generation sequencer (e.g., Genome Analyzer IIx (Illumina), HiSeq sequencer (HiSeq 2000, Illumina), Genome Sequencer FLX System (Roche)), or the like according to the manufacturer's instructions. RNA sequencing can be done by, for example, preparing a cDNA library from a total RNA using a commercially available kit (e.g., mRNA-Seq sample preparation kit (Illumina)) according to the manufacturer's instructions and sequencing the prepared library using a next-generation sequencer.

In the inventive detection method, if the object to be detected is a translation product of a fusion polynucleotide (*i.e*., fusion polypeptide), the translation product can be detected using, for example, immunostaining, Western blotting, RIA, ELISA, flow cytometry, immunoprecipitation, or antibody array analysis. These techniques use an antibody that specifically recognizes a fusion polypeptide. As used herein, the phrase "specifically recognizes a fusion polypeptide" means that besides the fusion polypeptide, an antibody recognizes no other proteins, including wild-type proteins from which to derive both segments of said fusion polynucleotide each extending from the point of fusion toward the Nor C-terminus. The antibody that "specifically recognizes a fusion polypeptide", which is to be used in the inventive detection method, can be one antibody or a combination of two or more antibodies.

The "antibody that specifically recognizes a fusion polypeptide" can be exemplified by an antibody specific to a polypeptide containing the point of fusion of said fusion polypeptide (hereinafter referred to as the "fusion point-specific antibody"). As referred to herein, the "fusion point-specific antibody" means an antibody that specifically binds to the polypeptide containing the point of fusion but does not bind to wild-type proteins from which to derive the segments of the fusion polypeptide each extending toward the N- or C-terminus.

Also, the "antibody that specifically recognizes a fusion polypeptide" can be exemplified by a combination of an antibody binding to a polypeptide consisting of that region of the fusion polypeptide which extends from the point of fusion toward the N-terminus and an antibody binding to a polypeptide consisting of that region of the fusion polypeptide which extends from the point of fusion toward the C-terminus. The fusion polypeptide can be detected by performing sandwich ELISA, immunostaining, immunoprecipitation, Western blotting or the like using these two antibodies.

In the present invention, examples of the antibodies include, but are not limited to, natural antibodies such as polyclonal antibodies and monoclonal antibodies (mAb), and chimeric, humanized and single-stranded antibodies which can be prepared using genetic recombination techniques, and binding fragments thereof. The "binding fragments" refers to partial regions of the above-mentioned antibodies which have specific binding activity, and specific examples include Fab, Fab', F(ab')₂, Fv, and single-chain antibodies. The class of antibody is not particularly limited, and any isotype of antibody, such as IgG, IgM, IgA, IgD or IgE, is acceptable, with IgG being preferred in consideration of ease of purification or other factors.

The "antibody that specifically recognizes a fusion polypeptide" can be prepared by those skilled in the art through appropriate selection of a known technique. Examples of such a known technique include: a method in which a polypeptide containing the point of fusion of the fusion polypeptide, a polypeptide consisting of that region of the fusion polypeptide which extends from the point of fusion toward the N-terminus, or a polypeptide consisting of that region of the fusion polypeptide which extends from the point of fusion toward the C-terminus is inoculated into an immune animal, the immune system of the animal is activated, and then the serum (polyclonal antibody) of the animal is collected; as well as monoclonal antibody preparation methods such as hybridoma method, recombinant DNA method, and phage display method. Commercially available antibodies may also be used. If an antibody having a labeling agent attached thereto is used, the target protein can be detected directly by detecting this label. The labeling agent is not particularly limited as long as it is capable of binding to an antibody and is detectable, and examples include peroxidase, β-D-galactosidase, microperoxidase, horseradish peroxidase (HRP), fluorescein isothiocyanate (FITC), rhodamine isothiocyanate (RITC), alkaline phosphatase, biotin, and radioactive materials. Aside from the direct detection of the target protein using the antibody having a labeling agent attached thereto, the target protein can also be detected indirectly using a secondary antibody having a labeling agent attached thereto, Protein G or A, or the like.

### <Kit for detecting gene fusions serving as responsible mutations (driver mutations) for cancer>

As described above, fusion polynucleotides produced by gene fusions serving as responsible mutations for cancer, or polypeptides encoded thereby, can be detected using such a primer, probe, or antibody as mentioned above, or a combination thereof, and thereby the gene fusions can be detected. Thus, the present invention provides a kit for detecting a gene fusion serving as a responsible mutation for cancer, the kit comprising any or a combination of (A) to (C) below (hereinafter also referred to as the "inventive kit"):
(A) a polynucleotide that serves as a probe designed to specifically recognize an ATG3-EPHB1 fusion polynucleotide, a TNIK-RNF123 fusion polynucleotide, or an SLC12A2-NRG2 fusion polynucleotide;
(B) polynucleotides that serve as a pair of primers designed to enable specific amplification of an ATG3-EPHB1 fusion polynucleotide, a TNIK-RNF123 fusion polynucleotide, or an SLC12A2-NRG2 fusion polynucleotide; and
(C) an antibody that specifically recognizes an ATG3-EPHB1 fusion polypeptide, a TNIK-RNF123 fusion polypeptide, or an SLC12A2-NRG2 fusion polypeptide.

In addition to the above-mentioned polynucleotide(s) or antibody, the inventive kit can also contain an appropriate combination of other components, including: a substrate required for detecting a label attached to the polynucleotide(s) or the antibody; a positive control (e.g., ATG3-EPHB1 fusion polynucleotide, TNIK-RNF123 fusion polynucleotide, or SLC12A2-NRG2 fusion polynucleotide; or ATG3-EPHB1 fusion polypeptide, TNIK-RNF123 fusion polypeptide, or SLC12A2-NRG2 fusion polypeptide; or cells bearing the same); a negative control; a PCR reagent; a counterstaining reagent for use for *in situ* hybridization or the like *(e.g.,* DAPI); a molecule required for antibody detection (*e.g.,* secondary antibody, Protein G, Protein A); and a buffer solution for use in sample dilution or washing. The inventive kit can also contain instructions for use thereof. The above-mentioned inventive detection method can be easily carried out by using the inventive kit.

The inventive detection method and kit, which enable detection of the gene fusions newly discovered as responsible mutations for cancer, are very useful in identifying subjects positive for said gene fusion and applying personalized medicine to each of the subjects, as described below.

### <Method for identifying patients with cancer or subjects with a risk of cancer>

The above-mentioned three types of gene fusions, serving as responsible mutations for cancer, are each believed to lead to constitutive activation of EPHB1 kinase activity, constitutive activation of TNIK kinase activity, and enhancement of the function of NRG2 as a cell growth factor, thereby contributing to malignant transformation of cancers. Thus, cancer patients in whom such a gene fusion is detected are highly probable to be responsive to the treatment with a substance suppressing the expression and/or activity of a polypeptide encoded by a fusion polynucleotide produced by said gene fusion.

Thus, the present invention provides a method for identifying patients with cancer or subjects with a risk of cancer, in whom substances suppressing the expression and/or activity of polypeptides encoded by fusion polynucleotides produced by gene fusions serving as responsible mutations (driver mutations) for cancer show a therapeutic effect (hereinafter also referred to as the "inventive identification method").

The inventive identification method comprises the steps of:
(1) detecting a fusion polynucleotide of any one of (a) to (c) below, or a polypeptide encoded thereby, in an isolated sample from a subject:
   (a) an ATG3-EPHB1 fusion polynucleotide which encodes a polypeptide comprising the kinase domain of EPHB1 and having kinase activity;
   (b) a TNIK-RNF123 fusion polynucleotide which encodes a polypeptide comprising the kinase domain of TNIK and the SPRY domain and RING finger domain of RNF123, and having kinase activity; and
   (c) an SLC12A2-NRG2 fusion polynucleotide which encodes a polypeptide comprising the AA_permease_N super family domain of SLC12A2 and the neuregulin family conserved region of NRG2, and having intracellular signaling-enhancing activity; and
(2) determining that the substance suppressing the expression and/or activity of the polypeptide shows a therapeutic effect in the subject, in the case where the fusion polynucleotide of any one of (a) to (c) or the polypeptide encoded thereby is detected.

In the inventive identification method, the phrase "a patient with cancer or a subject with a risk of cancer" refers to a mammal, preferably human, which is, or is suspected of being, affected by cancer. The "cancer" to which the inventive identification method is to be applied is not particularly limited as long as it is a cancer in which any of the above-mentioned three types of gene fusions can be detected, with gastric cancer being preferred, and special-type gastric cancer, undifferentiated gastric cancer, or moderately differentiated gastric cancer being more preferred.

In the inventive identification method, the "therapeutic effect" is not particularly limited as long as it is a beneficial effect of cancer treatment for a patient, and examples include a tumor shrinkage effect, a progression-free survival prolongation effect, and a life lengthening effect.

In the inventive identification method, the "substance suppressing the expression and/or activity of a polypeptide encoded by a fusion polynucleotide produced by a gene fusion serving as a responsible mutation (driver mutation) for cancer", which is to be evaluated for its effectiveness in the treatment of cancer associated with the ATG3-EPHB1 gene fusion (this substance is hereinafter also referred to as the "substance to be evaluated in the inventive identification method"), is not particularly limited as long as it is a substance that directly or indirectly inhibits the expression and/or function of an ATG3-EPHB1 fusion polypeptide.

Examples of the substance inhibiting the expression of an ATG3-EPHB1 fusion polypeptide include: siRNAs (small interfering RNAs), shRNAs (short hairpin RNAs), miRNAs (micro RNAs), and antisense nucleic acids which suppress the expression of an ATG3-EPHB1 fusion polypeptide; expression vectors capable of expressing these polynucleotides; and low-molecular-weight compounds.

Examples of the substance inhibiting the function of an ATG3-EPHB1 fusion polypeptide include substances inhibiting EPHB1 kinase activity (e.g., low-molecular-weight compounds), and antibodies binding to an ATG3-EPHB1 fusion polypeptide.

These substances may be substances that specifically suppress the expression and/or activity of an ATG3-EPHB1 fusion polypeptide, or may be substances that suppress even the expression and/or activity of wild-type EPHB1 protein. Specific examples of such substances include, but are not limited to, dasatinib, PD-173955, foretinib, bosutinib, crizotinib, AST-487, staurosporine, vandetanib, MLN-8054, TAE 684, nilotinib, dorsomorphin, Cdk1/2 inhibitor III, IKK-2 inhibitor IV, GSK-3β inhibitor XII, and PP1 Analog II (*cf*., Ephrin receptor family: EPH receptor B1. Last modified on 26/03/2013. Accessed on 10/12/2013. IUPHAR database (IUPHAR-DB), http://www.iuphar-db.org/DATABASE/ObjectDisplayForward?objectId=1830).

These substances can be prepared by a *per se* known technique on the basis of the sequence information of an ATG3-EPHB1 fusion polynucleotide and/or an ATG3-EPHB1 fusion polypeptide which are disclosed in the present specification, or other data. Commercially available substances may also be used.

These substances are effective as cancer therapeutic agents for a subject, in the case where an ATG3-EPHB1 fusion polynucleotide or a polypeptide encoded thereby is detected in an isolated sample from said subject.

As regards TNIK-RNF123 gene fusion, the substance to be evaluated in the inventive identification method is not particularly limited as long as it is a substance that directly or indirectly inhibits the expression and/or function of a TNIK-RNF123 fusion polypeptide.

Examples of the substance inhibiting the expression of a TNIK-RNF123 fusion polypeptide include: siRNAs (small interfering RNAs), shRNAs (short hairpin RNAs), miRNAs (micro RNAs), and antisense nucleic acids which suppress the expression of a TNIK-RNF123 fusion polypeptide; expression vectors capable of expressing these polynucleotides; and low-molecular-weight compounds.

Examples of the substance inhibiting the function of a TNIK-RNF123 fusion polypeptide include substances inhibiting TNIK kinase activity (e.g., low-molecular-weight compounds), and antibodies binding to a TNIK-RNF123 fusion polypeptide.

These substances may be substances that specifically suppress the expression and/or activity of a TNIK-RNF123 fusion polypeptide, or may be substances that suppress even the expression and/or activity of wild-type TNIK protein. Specific examples of such substances include, but are not limited to, aminothiazole derivatives (refer to WO 2010/064111) and 4-phenyl-2-phenylaminopyridine-based compounds (refer to Ho K. K., et al., Bioorg Med Chem Lett, 2013, 23 (2): 569-73 (Epub 16 Nov 2012)).

These substances can be prepared by a *per se* known technique on the basis of the sequence information of a TNIK-RNF123 fusion polynucleotide and/or a TNIK-RNF123 fusion polypeptide which are disclosed in the present specification, or other data. Commercially available substances may also be used.

These substances are effective as cancer therapeutic agents for a subject, in the case where a TNIK-RNF123 fusion polynucleotide or a polypeptide encoded thereby is detected in an isolated sample from said subject.

As regards SLC12A2-NRG2 gene fusion, the substance to be evaluated in the inventive identification method is not particularly limited as long as it is a substance that directly or indirectly inhibits the expression and/or function of an SLC12A2-NRG2 fusion polypeptide.

Examples of the substance inhibiting the expression of an SLC12A2-NRG2 fusion polypeptide include: siRNAs (small interfering RNAs), shRNAs (short hairpin RNAs), miRNAs (micro RNAs), and antisense nucleic acids which suppress the expression of an SLC12A2-NRG2 fusion polypeptide; expression vectors capable of expressing these polynucleotides; and low-molecular-weight compounds.

Examples of the substance inhibiting the function of an SLC12A2-NRG2 fusion polypeptide include substances inhibiting the intracellular signaling-enhancing activity of NRG2 (e.g., low-molecular-weight compounds), and antibodies binding to an SLC12A2-NRG2 fusion polypeptide.

These substances may be substances that specifically suppress the expression and/or activity of an SLC 12A2-NRG2 fusion polypeptide, or may be substances that suppress even the expression and/or activity of wild-type NRG2 protein.

These substances can be prepared by a *per se* known technique on the basis of the sequence information of an SLC12A2-NRG2 fusion polynucleotide and/or an SLC12A2-NRG2 fusion polypeptide which are disclosed in the present specification, or other data. Commercially available substances may also be used.

Further, since wild-type NRG2 protein is believed to enhance intracellular signaling via a protein belonging to a group of HER proteins serving as receptors for the wild-type NRG2 protein, the substance to be evaluated in the inventive identification method can also be exemplified by substances that directly or indirectly inhibit the expression and/or function of HER proteins. As referred to herein, the group of HER proteins is a group of tyrosine kinase receptors which consists of the following four proteins: HER1 (ErbB1), HER2 (ErbB2), HER3 (ErbB3), and HER4 (ErbB4).

Examples of the substances inhibiting the expression of HER proteins include: siRNAs (small interfering RNAs), shRNAs (short hairpin RNAs), miRNAs (micro RNAs), and antisense nucleic acids which suppress the expression of HER proteins; expression vectors capable of expressing these polynucleotides; and low-molecular-weight compounds.

Examples of the substances inhibiting the function of HER proteins include substances inhibiting the kinase activity of HER proteins (e.g., low-molecular-weight compounds), and antibodies binding to HER proteins. Specific examples of such substances include rapatinib, afatinib, dacomitinib, and trastuzumab.

These substances can be prepared by a *per se* known technique on the basis of known sequence information or other data. Commercially available substances may also be used.

These substances are effective as cancer therapeutic agents for a subject, in the case where an SLC12A2-NRG2 fusion polynucleotide or a polypeptide encoded thereby is detected in an isolated sample from said subject.
Step (1) in the inventive identification method can be carried out in the same way as the step included in the inventive detection method mentioned above.
At step (2) in the inventive identification method, the substance to be evaluated in the inventive identification method is determined to show a therapeutic effect in a subject (*i.e*., a patient with cancer or a subject with a risk of cancer), in the case where a fusion polynucleotide of interest or a polypeptide encoded thereby is detected in an isolated sample from the subject at step (1); however, the substance to be evaluated in the inventive identification method is determined to be unlikely to show a therapeutic effect in the subject, in the case where neither the fusion polynucleotide of interest nor the polypeptide encoded thereby is detected.

According to the inventive identification method, it is possible to detect subjects positive for the gene fusions newly discovered as responsible mutations for cancer from among patients with cancer or subjects with a risk of cancer, and to identify patients with cancer or subjects with a risk of cancer, in whom substances suppressing the expression and/or activity of polypeptides encoded by fusion polynucleotides produced by said gene fusions show a therapeutic effect; thus, the present invention is useful in that it enables provision of suitable treatment for such subjects.

### <Method for treatment of cancer and cancer therapeutic agent>

As described above, the inventive identification method allows identification of patients with cancer, in whom substances suppressing the expression and/or activity of polypeptides encoded by fusion polynucleotides produced by any of the above-mentioned three types of gene fusions show a therapeutic effect. Thus, efficient cancer treatment can be performed by administering said substance selectively to a cancer patient who carries said fusion gene. Therefore, the present invention provides a method for treating cancer, comprising the step of administering said substance to a subject (hereinafter also referred to as the "inventive treatment method").

In the inventive treatment method, the subject is typified by a patient with cancer positive for ATG3-EPHB1, TNIK-RNF123 or SLC12A2-NRG2 gene fusion. The cancer is not particularly limited, and is preferably gastric cancer, more preferably special-type gastric cancer, undifferentiated gastric cancer, or moderately differentiated gastric cancer. As referred to herein, the cancer positive for ATG3-EPHB1, TNIK-RNF123 or SLC12A2-NRG2 gene fusion refers to a cancer in which a fusion polynucleotide or a polypeptide encoded thereby is detected, for any of these gene fusions, by the inventive detection method.

Also, the present invention provides a therapeutic agent for cancer positive for ATG3-EPHB1, TNIK-RNF123 or SLC12A2-NRG2 gene fusion (hereinafter also referred to as the "inventive cancer therapeutic agent"). For the cancers positive for the above-mentioned three types of gene fusions, no treatment targeting these gene fusions has been performed. Also, the presence of these gene fusions was totally unpredictable even in consideration of any prior art documents or other information. Therefore, the inventive cancer therapeutic agent provides novel therapeutic measures effective for the cancers positive for the above-mentioned three types of gene fusions.

In the inventive cancer therapeutic agent, the cancer is not particularly limited, and is preferably gastric cancer, more preferably special-type gastric cancer, undifferentiated gastric cancer, or moderately differentiated gastric cancer.

The inventive cancer therapeutic agent typically comprises, as an active ingredient, a substance suppressing the expression and/or activity of: an ATG3-EPHB1 fusion polypeptide comprising the kinase domain of EPHB1 and having kinase activity; a TNIK-RNF123 fusion polypeptide comprising the kinase domain of TNIK and the SPRY domain and RING finger domain of RNF 123 and having kinase activity; or an SLC12A2-NRG2 fusion polypeptide comprising the AA_permease_N super family domain of SLC12A2 and the neuregulin family conserved region of NRG2, and having intracellular signaling-enhancing activity.

In the context of the inventive treatment method and inventive cancer therapeutic agent, the substances suppressing the expression and/or activity of the above-mentioned three types of fusion polypeptides can be exemplified by the substances listed above in <Method for identifying patients with cancer or subjects with a risk of cancer>.

The inventive cancer therapeutic agent can be formulated into a pharmaceutical composition with the use of a pharmaceutically acceptable carrier, excipient and/or other additives which are commonly used in pharmaceutical manufacturing.

The method for administering the inventive cancer therapeutic agent is selected as appropriate depending on various factors including the type of this suppressing agent and the type of cancer, and exemplary modes of administration that can be adopted include oral, intravenous, intraperitoneal, transdermal, intramuscular, intratracheal (aerosol), rectal and intravaginal administrations.

The dose of the inventive cancer therapeutic agent can be determined as appropriate in consideration of various factors, including the activity and type of an active ingredient, the mode of administration (e.g., oral or parenteral administration), the severity of a disease, and the animal species, drug receptivity, body weight, and age of the subject to be treated with the inventive agent.

The treatment method and cancer therapeutic agent of the present invention are useful in that they allow treatment of patients having the particular responsible mutations for cancer which have been conventionally unknown and were first discovered according to this invention.

### <Method for screening cancer therapeutic agents>

The present invention provides a method for screening cancer therapeutic agents which show a therapeutic effect in cancer patients having any of the above-mentioned three types of gene fusions (hereinafter also referred to as the "inventive screening method"). According to the inventive screening method, substances suppressing the expression and/or activity of any of the above-mentioned three types of fusion polypeptides (*i.e*., ATG3-EPHB1 fusion polypeptide, TNIK-RNF123 fusion polypeptide, and SLC12A2-NRG2 fusion polypeptide) can be obtained as cancer therapeutic agents.

The test substance to be subjected to the inventive screening method can be any compound or composition, and can be exemplified by nucleic acids (e.g., nucleoside, oligonucleotide, polynucleotide), saccharides (e.g., monosaccharide, disaccharide, oligosaccharide, polysaccharide), fats (e.g., saturated or unsaturated, straight-chain, branched-chain and/or cyclic fatty acids), amino acids, proteins (e.g., oligopeptide, polypeptide), low-molecular-weight compounds, compound libraries, random peptide libraries, natural ingredients (e.g., ingredients derived from microbes, animals and plants, marine organisms, and others), foods, and the like.

The inventive screening method can be of any type as long as it enables evaluation of whether a test substance suppresses the expression and/or activity of any of the above-mentioned three types of fusion polypeptides. Typically, the inventive screening method comprises the following steps:
(1) bringing a cell expressing an ATG3-EPHB1 fusion polypeptide, a TNIK-RNF123 fusion polypeptide, or an SLC12A2-NRG2 fusion polypeptide into contact with a test substance;
(2) determining whether the expression and/or activity of any of said fusion polypeptides is suppressed or not; and
(3) selecting the substance determined to suppress the expression and/or activity of any of said fusion polypeptides, as a cancer therapeutic agent.

At step (1), the cell expressing any of the above-mentioned three types of fusion polypeptides is brought into contact with the test substance. A test substance-free solvent *(e.g.,* DMSO) can be used as a control. The contact can be effected in a medium. The medium is selected as appropriate depending on various factors including the type of the cell to be used, and examples include a minimum essential medium (MEM) supplemented with about 5-20% fetal bovine serum, a Dulbecco's modified eagle's medium (DMEM), RPMI1640 medium, and 199 medium. The culture conditions are also selected as appropriate depending on various factors including the type of the cell to be used, and for example, the pH of the medium is in the range of approximately from 6 to 8, the culture temperature is in the range of approximately from 30 to 40°C, and the culture time is in the range of approximately from 12 to 72 hours.

Examples of the cell expressing any of the above-mentioned three types of fusion polypeptides include, but are not limited to, cancer tissue-derived cells intrinsically expressing said fusion polypeptides, cell lines induced from said cells, and cell lines made by genetic engineering. Whether a cell expresses any of the above-mentioned three types of fusion polypeptides can also be confirmed using the inventive detection method described above. The cell is generally a mammalian cell, preferably a human cell.

At step (2), it is determined whether the test substance suppresses the expression and/or activity of any of said fusion polypeptides or not. The expression of fusion polypeptides can be measured by determining the mRNA or protein level in a cell using a known analysis technique such as Northern blotting, quantitative PCR, immunoblotting, or ELISA. Also, the activity of fusion polypeptides can be measured by a known analysis technique (e.g., kinase activity assay). A resulting measured value is compared with a value measured in a control cell not contacted with the test substance. The comparison of the measured values is made preferably based on the presence or absence of a significant difference. If the value measured in the cell contacted with the test substance is significantly lower than that measured in the control cell, it can be determined that the test substance suppresses the expression and/or activity of any of said fusion polypeptides.

Alternatively, given the fact that the cells expressing these types of fusion polypeptides show enhanced growth, the growth of said cells can be used as an indicator for the determination at this step. In this case, the growth of such a cell contacted with the test substance is measured as a first step. The cell growth measurement can be made by a *per se* known technique such as cell count, ³H-thymidine incorporation, or BRDU. Next, the growth of the cell contacted with the test substance is compared with that of a control cell not contacted with the test substance. The growth level comparison is made preferably based on the presence or absence of a significant difference. The value for the growth of the control cell not contacted with the test substance can be measured prior to, or at the same time as, the measurement of the growth of the cell contacted with the test substance, but is preferably measured at the same time from the viewpoints of the accuracy and reproducibility of the test. If the results of the comparison show that the growth of the cell contacted with the test substance is suppressed, it can be determined that the test substance suppresses the expression and/or activity of any of said fusion polypeptides.

At step (3), the test substance determined to suppress the expression and/or activity of any of said fusion polypeptides at step (2) is selected as a cancer therapeutic agent.

Hence, the inventive screening method makes it possible to obtain cancer therapeutic agents applicable to the treatment of patients with responsible mutations for cancer which have been conventionally unknown.

### <Isolated fusion polypeptides or fragments thereof, and polynucleotides encoding the same>

The present invention provides isolated fusion polypeptides (hereinafter also referred to as the "inventive fusion polypeptides") as mentioned below, or fragments thereof:
(1) an isolated ATG3-EPHB1 fusion polypeptide which comprises the kinase domain of EPHB1 protein and has kinase activity;
(2) an isolated TNIK-RNF123 fusion polypeptide which comprises the kinase domain of TNIK protein and the SPRY domain and RING finger domain of RNF123 protein and has kinase activity; and
(3) an isolated SLC12A2-NRG2 fusion polypeptide which comprises the AA_permease_N super family domain of SLC 12A2 protein and the neuregulin family conserved region of NRG2 protein, and has intracellular signaling-enhancing activity.

For the purpose of the present specification, the "isolated" substance refers to a substance substantially separated or purified from other substances (preferably, biological factors) found in an environment in which the substance naturally occurs *(e.g.,* in a cell of an organism) (for example, if the substance of interest is a nucleic acid, the "other substances" corresponds to other factors than nucleic acids as well as nucleic acids containing other nucleic acid sequences than that of the nucleic acid of interest; and if the substance of interest is a protein, the "other substances" corresponds to other factors than proteins as well as proteins containing other amino acid sequences than that of the protein of interest). For the purpose of the specification, the term "isolated" means that a substance has a purity of preferably at least 75% by weight, more preferably at least 85% by weight, still more preferably at least 95% by weight, and most preferably at least 96% by weight, at least 97% by weight, at least 98% by weight, at least 99% by weight, or 100%. Examples of the "isolated" polynucleotide and polypeptide include not only polynucleotides and polypeptides purified by standard purification techniques but also chemically synthesized polynucleotides and polypeptides.

Other terms used above in (1) to (3) have the same meanings as defined above in

### <Specific responsible mutations for cancer>.

The "fragments" refers to fragments of the inventive fusion polypeptides, which each consist of a consecutive partial sequence comprising sequences upstream and downstream from the point of fusion. The sequence upstream from the point of fusion, as contained in said partial sequence, can comprise at least one amino acid residue (for example, at least 2, 3, 4, 5, 6, 7, 8, 9, 10, 20, 30, 40, 50 or 100 amino acid residues) ranging from the point of fusion to the N-terminus of any of the inventive fusion polypeptides. The sequence downstream from the point of fusion, as contained in said partial sequence, can comprise at least one amino acid residue (for example, at least 2, 3, 4, 5, 6, 7, 8, 9, 10, 20, 30, 40, 50 or 100 amino acid residues) ranging from the point of fusion to the C-terminus of any of the inventive fusion polypeptides. The length of the fragments is not particularly limited, and is generally at least 8 amino acid residues (for example, at least 9, 10, 11, 12, 13, 14, 15, 20, 25, 50 or 100 amino acid residues).

Also, the inventive fusion polypeptides can be, for example, the isolated fusion polypeptides mentioned below:
(1) an ATG3-EPHB1 fusion polypeptide which is any one of (i) to (iii) below:
   (i) a polypeptide consisting of the amino acid sequence of SEQ ID NO: 2,
   (ii) a polypeptide consisting of an amino acid sequence derived from the amino acid sequence of SEQ ID NO: 2 by deletion, substitution or addition of one or more amino acids, and the polypeptide having kinase activity, or
   (iii) a polypeptide consisting of an amino acid sequence having a sequence identity of at least 80% to the amino acid sequence of SEQ ID NO: 2, and the polypeptide having kinase activity;
(2) a TNIK-RNF123 fusion polypeptide which is any one of (i) to (iii) below:
   (i) a polypeptide consisting of the amino acid sequence of SEQ ID NO: 4, 6, 8, 10, 12, 14, 16 or 18,
   (ii) a polypeptide consisting of an amino acid sequence derived from the amino acid sequence of SEQ ID NO: 4, 6, 8, 10, 12, 14, 16 or 18 by deletion, substitution or addition of one or more amino acids, and the polypeptide having kinase activity, or
   (iii) a polypeptide consisting of an amino acid sequence having a sequence identity of at least 80% to the amino acid sequence of SEQ ID NO: 4, 6, 8, 10, 12, 14, 16 or 18, and the polypeptide having kinase activity; and
(3) an SLC12A2-NRG2 fusion polypeptide which is any one of (i) to (iii) below:
   (i) a polypeptide consisting of the amino acid sequence of SEQ ID NO: 20, 22, 24, 26 or 28,
   (ii) a polypeptide consisting of an amino acid sequence derived from the amino acid sequence of SEQ ID NO: 20, 22, 24, 26 or 28 by deletion, substitution or addition of one or more amino acids, and the polypeptide having intracellular signaling-enhancing activity, or
   (iii) a polypeptide consisting of an amino acid sequence having a sequence identity of at least 80% to the amino acid sequence of SEQ ID NO: 20, 22, 24, 26 or 28, and the polypeptide having intracellular signaling-enhancing activity.

The terms used above in (i) to (iii) have the same meanings as defined above in

### <Specific responsible mutations for cancer>.

Further, the present invention provides isolated polynucleotides encoding the inventive fusion polypeptides or the fragments thereof as described above (hereinafter also referred to as the "inventive polynucleotides"). The inventive polynucleotides can be in the form of any of mRNA, cDNA and genomic DNA. Also, the polynucleotides may be double- or single-stranded.

A typical example of the cDNA encoding the ATG3-EPHB1 fusion polypeptide is a polynucleotide consisting of the nucleotide sequence of SEQ ID NO: 1.

Typical examples of the cDNA encoding the TNIK-RNF123 fusion polypeptide are polynucleotides each consisting of the nucleotide sequence of SEQ ID NO: 3, 5, 7, 9, 11, 13, 15 or 17.

Typical examples of the cDNA encoding the SLC12A2-NRG2 fusion polypeptide are polynucleotides each consisting of the nucleotide sequence of SEQ ID NO: 19, 21, 23, 25 or 27.

The inventive polynucleotides can be made by a *per se* known technique. For example, the inventive polynucleotides can be extracted using a known hybridization technique from a cDNA library or genomic library prepared from cancer tissues or the like harboring an ATG3-EPHB1 fusion polynucleotide, a TNIK-RNF123 fusion polynucleotide, or an SLC12A2-NRG2 fusion polynucleotide. The inventive polynucleotides can also be prepared by amplification utilizing a known gene amplification technique (PCR), with mRNA, cDNA or genomic DNA prepared from the cancer tissues or the like being used as a template. Alternatively, the polynucleotides can be prepared utilizing a known gene amplification or genetic recombination technique such as PCR, restriction enzyme treatment, or site-directed mutagenesis (Kramer, W. & Fritz, H. J., Methods Enzymol., 1987, 154, 350), using, as starting materials, the cDNAs of wild-type genes from which to derive those segments of each of the fusion polynucleotides which extend toward the 5'- or 3'-end.

The inventive fusion polypeptides or fragments thereof can also be made by a *per se* known technique. For example, after such a polynucleotide prepared as mentioned above is inserted into an appropriate expression vector, the vector is introduced into a cell-free protein synthesis system (e.g., reticulocyte extract, wheat germ extract) and the system is incubated, or alternatively the vector is introduced into appropriate cells (*e.g., E coli*., yeast, insect cells, animal cells) and the resulting transformant is cultured; in either of the above ways, the inventive fusion polypeptides can be prepared.

The inventive fusion polypeptides or fragments thereof can be used as a marker in the inventive detection method or the like, or can be used in other applications including preparation of antibodies against the inventive fusion polypeptides.

### EXAMPLES

Hereunder, the present invention will be more specifically described by way of working examples, but this invention is not limited to the examples given below.

### <Samples>

As samples for RNA sequencing, total RNAs were prepared by a conventional method from gastric tissues taken from 51 patients with gastric cancer (GC) (5 with moderately differentiated GC, 3 with special-type mucinous GC, and 43 with undifferentiated GC). As samples for RT-PCR and Sanger sequencing, total RNAs were prepared by a conventional method from gastric tissues taken from 149 patients with gastric cancer (27 with highly differentiated GC, 65 with moderately differentiated GC, 2 with special-type mucinous GC, and 55 with undifferentiated GC). The present study was conducted with the approval by the institutional review boards of the institutions involved in the study.

### <RNA sequencing>

RNA sequencing libraries were prepared using the TruSeq RNA Sample Prep Kit v2 produced by Illumina according to the manufacturer's standard protocol. These libraries were subjected to paired-end sequencing of 100 bp reads on the next-generation sequencer Hiseq2000 or GAIIx. The resulting paired-end reads were compared to the RefSeq (NCBI Reference Sequence) database to thereby detect fusion gene candidates.

### <RT-PCR, Sanger sequencing>

cDNAs were synthesized by a reverse transcriptase method from the total RNAs extracted from the gastric tissues taken from the gastric cancer patients. The resulting cDNAs were subjected to PCR amplification. The obtained PCR products were directly nucleotide-sequenced using the BigDye Terminator Kit and the ABI 3130x1 DNA Sequencer (Applied Biosystems). The primers used in this study are shown in Table 1.

**[Table 1]**

| Fusion gene | Sense primer | Anti-sense primer | RT-PCR product size (bp) |
|---|---|---|---|
| ATG3-EPHB1 | CACTGGAAGTGGCTGAGTACCTGA (SEQ ID NO:65) | GCCGCAGCCCATCAATCCTT (SEQ ID NO:66) | 150 |
| TNIK-RNF123 | GGTCAGTGGAAACAGGACATTTGG (SEQ ID NO:67) | ACGCCTTGCCATAATTCGTTGT (SEQ ID NO:68) | 318 |
| SLC12A2-NRG2 | GGTCAAGCTGGAATAGGTCTATCAG (SEQ ID NO:69) | TGCCTCACACTTCAGCGATT (SEQ ID NO:70) | 189 |

### (Example)

This example describes the identification of novel fusion transcripts in gastric tissues.

In order to identify novel fusion transcripts potentially serving as targets for treatment, the gastric tissues taken from 51 patients with gastric cancer (5 with moderately differentiated GC, 3 with special-type mucinous GC, and 43 with undifferentiated GC) were subjected to whole-transcriptome sequencing (RNA sequencing; refer to Meyerson, M., et al., Nat. Rev. Genet., 2010, 11, 685-696).

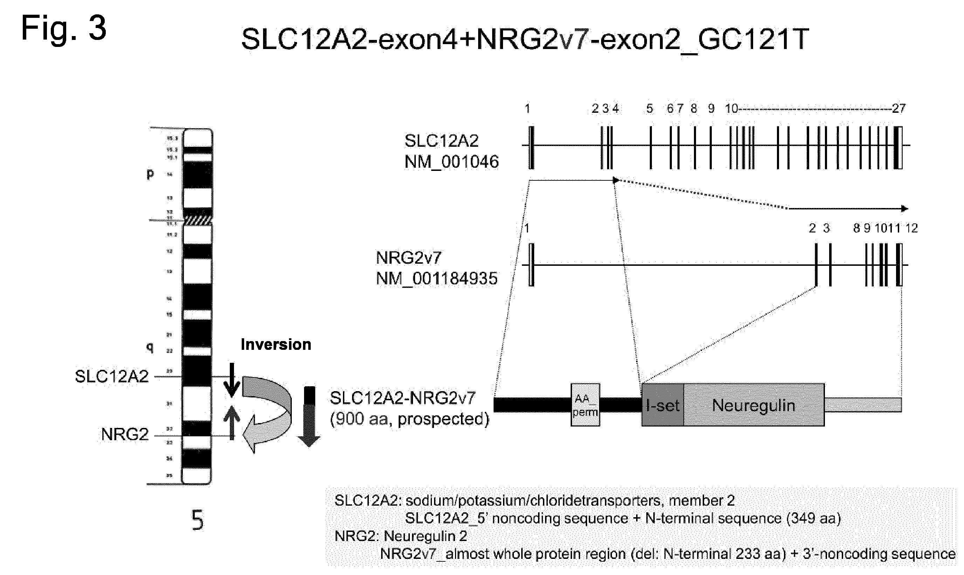

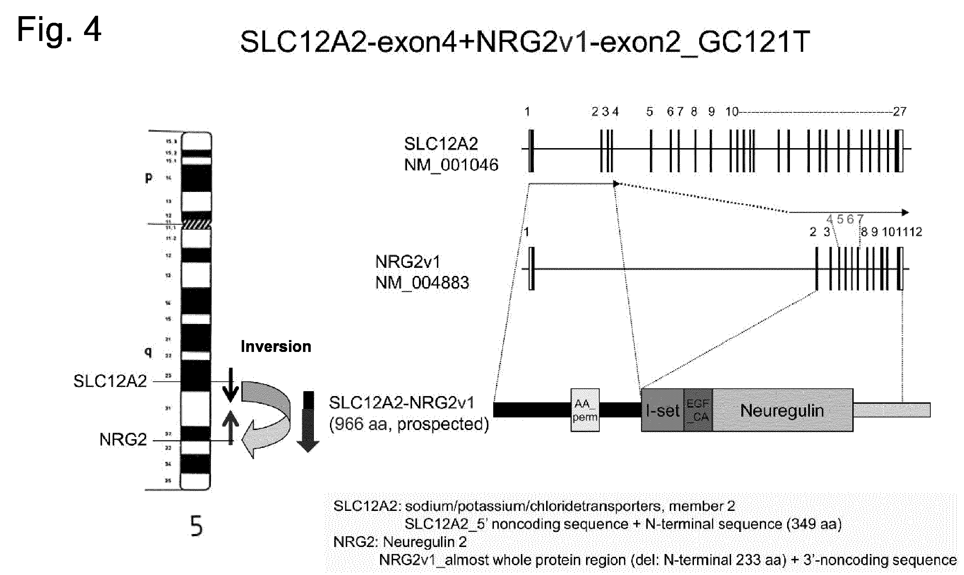

After overlapping clones generated by PCR were eliminated, at least 8.0×10⁷ paired nucleotide sequences were obtained for each case. These sequences were compared to existing gene databases; as a result, a candidate for fusion gene between the ATG3 gene and the EPHB1 gene was detected in one case (special-type mucinous GC patient), a candidate for fusion gene between the TNIK gene and the RNF123 gene was detected in one case (undifferentiated GC patient), and a candidate for fusion gene between the SLC 12A2 gene and the NRG2 gene was detected in one case (moderately differentiated GC patient). The structures of these fusion genes are shown in Table 2 and Figs. 1 to 4.

**[Table 2]**

| Fusion gene | Location of gene toward 5' -end | Location of gene toward 3'-end | Causative chromosomal aberration |
|---|---|---|---|
| ATG3-EPHB1 | 3q13.2 | 3q21-q23 | Chromosomal inversion (inv3) |
| TNTK-RNF123 | 3q26.31 | 3p24.3 | Chromosomal inversion (inv3) |
| SLC12A2-NRG2 | 5q23.3 | 5q23-q33 | Chromosomal inversion (inv5) |

Then, RT-PCR was performed using the primers shown in Table 1 on the specimens of the cases in which fusion genes were detected, and the obtained PCR products were subjected to sequence analysis to verify the expression of the above-mentioned three types of fusion genes. The sequences of the amplified PCR products are as follows. ATG3-EPHB1 (Uppercase and lowercase letters represent an ATG3 gene-derived sequence and an EPHB1 gene-derived sequence, respectively): 
CACTGGAAGTGGCTGAGTACCTGACCCCGGTCCTCAAGGAATCAAAGTTTAAGG
AAACAGGTGTAATTACCCCAGAAGAGgaacacaatgagttcaactcctccatggccaggagtcagaccaac
acagcaaggattgatgggctgcggc (SEQ ID NO: 71)
 TNIK-RNF123 (Uppercase and lowercase letters represent a TNIK gene-derived sequence and an RNF123 gene-derived sequence, respectively): 
GGTCAGTGGAAACAGGACATTTGGATGGAGTATTTATGCATAAGCGAGCTCAAA
GGTTAAAGTTTCTATGTGAAAGAAATGATAAGgtgattggacacagcaactttggcaccatccgctcta
ccacatgcgtgtacaaagggaaatggctctacgaggtcctcatctcctcccaggggctcatgcagatcggctggtgcaccatcagctg
ccgcttcaaccaggaggagggggttggagatacacacaactcctatgcctatgatggcaaccgcgtgcgcaagtggaatgtgacca
caacgaattatggcaaggcgt (SEQ ID NO: 72)
 SLC12A2-NRG2 fusion gene (Uppercase and lowercase letters represent an SLC12A2 gene-derived sequence and an NRG2 gene-derived sequence, respectively):

GGTCAAGCTGGAATAGGTCTATCAGTCCTTGTAATAATGATGGCCACTGTTGTGA
CAACTATCACAGGATTGTCTACTTCAGCAATAGCAACTAATGGATTTGTAAGAGG
AGccacccggcccaagttgaagaagatgaagagccagacgggacaggtgggtgagaagcaatcgctgaagtgtgaggca
(SEQ ID NO: 73)

ATG3-EPHB1 is a fusion gene generated by chromosomal inversion (inv3), in which exon 2 of the ATG3 gene is directly bound to exon 7 of the EPHB1 gene without inconsistency in the reading frames of these genes. The entire coding region of the ATG3-EPHB1 fusion gene was cloned and sequenced (SEQ ID NO: 1).

TNIK-RNF123 is a fusion gene generated by chromosomal inversion (inv3), in which exon 32 of the TNIK gene is directly bound to exon 6 of the RNF123 gene without inconsistency in the reading frames of these genes.

SLC12A2-NRG2 is a fusion gene generated by chromosomal inversion (inv5), in which exon 4 of the SLC 12A2 gene is directly bound to exon 2 of the NRG2 gene without inconsistency in the reading frames of these genes.

The above results demonstrated that the gene fusions found in Example can be detected by verifying the presence or absence of the amplification of specific bands by RT-PCR and sequencing the nucleotide sequences of the PCR products.

Further, RT-PCR was performed on normal gastric mucosal tissues using the primers shown in Table 1, and as a result, no amplification of a specific band was observed, which demonstrated that none of the above-mentioned three types of fusion genes was expressed. The results indicated that the above-mentioned three types of gene fusions were specific for cancer cells.

Furthermore, none of KRAS mutations (mutations at the 12th, 13rd or 61st amino acid residue from the N-terminus), NRAS mutations (mutations at the 12th, 13rd or 61st amino acid residue from the N-terminus), and BRAF mutations (mutations at the 600th amino acid residue from the N-terminus and its neighbors), which were known as causative gene mutations for gastric cancer, was detected in any of the three cases in which any of the above-mentioned three types of fusion genes was detected. Therefore, it was suggested that these fusion genes are highly likely to be responsible mutations for gastric cancer.

### INDUSTRIAL APPLICABILITY

The present invention makes it possible to detect gene fusions newly discovered as responsible mutations for cancer; to identify patients with cancer or subjects with a risk of cancer, in whom substances suppressing the expression and/or activity of polypeptides encoded by fusion polynucleotides produced by said gene fusions show a therapeutic effect; and to provide suitable treatment (personalized medicine) for such cancer patients.

### SEQUENCE LISTING FREE TEXT

SEQ ID NO: 1: ATG3-EPHB1 fusion polynucleotide
SEQ ID NO: 2: ATG3-EPHB1 fusion polypeptide
SEQ ID NOs: 3, 5, 7, 9, 11, 13, 15 and 17: TNIK-RNF123 fusion polynucleotides
SEQ ID NOs: 4, 6, 8, 10, 12, 14, 16 and 18: TNIK-RNF123 fusion polypeptides
SEQ ID NOs: 19, 21, 23, 25 and 27: SLC12A2-NRG2 fusion polynucleotides
SEQ ID NOs: 20, 22, 24, 26 and 28: SLC12A2-NRG2 fusion polypeptides
SEQ ID NO: 29: ATG3 cDNA
SEQ ID NO: 30: ATG3 polypeptide
SEQ ID NO: 31: EPHB1 cDNA
SEQ ID NO: 32: EPHB1 polypeptide
SEQ ID NOs: 33, 35, 37, 39, 41, 43, 45 and 47: TNIK cDNAs
SEQ ID NOs: 34, 36, 38, 40, 42, 44, 46 and 48: TNIK polypeptides
SEQ ID NO: 49: RNF 123 cDNA
SEQ ID NO: 50: RNF123 polypeptide
SEQ ID NOs: 51 and 53: SLC12A2 cDNAs
SEQ ID NOs: 52 and 54: SLC12A2 polypeptides
SEQ ID NOs: 55, 57, 59, 61 and 63: NRG2 cDNAs
SEQ ID NOs: 56, 58, 60, 62 and 64: NRG2 polypeptides
SEQ ID NO: 65: ATG3 sense primer
SEQ ID NO: 66: EPHB1 primer
SEQ ID NO: 67: TNIK sense primer
SEQ ID NO: 68: RNF 123 primer
SEQ ID NO: 69: SLC 12A2 sense primer
SEQ ID NO: 70: NRG2 primer
SEQ ID NO: 71: Partial sequence of ATG3-EPHB1 fusion polynucleotide
SEQ ID NO: 72: Partial sequence of TNIK-RNF123 fusion polynucleotide
SEQ ID NO: 73: Partial sequence of SLC12A2-NRG2 fusion polynucleotide

## Claims

1. A method for detecting a gene fusion serving as a responsible mutation (driver mutation) for cancer, the method comprising the step of detecting a fusion polynucleotide of any one of (a) to (c) below, or a polypeptide encoded thereby, in an isolated sample from a subject:
(a) an ATG3-EPHB1 fusion polynucleotide which encodes a polypeptide comprising the kinase domain of EPHB1 and having kinase activity;
(b) a TNIK-RNF123 fusion polynucleotide which encodes a polypeptide comprising the kinase domain of TNIK and the SPRY domain and RING finger domain of RNF123, and having kinase activity; and
(c) an SLC 12A2-NRG2 fusion polynucleotide which encodes a polypeptide comprising the AA_permease_N super family domain of SLC12A2 and the neuregulin family conserved region of NRG2, and having intracellular signaling-enhancing activity.

2. The method according to claim 1, wherein the fusion polynucleotide is any one of (a) to (c) below:
(a) an ATG3-EPHB1 fusion polynucleotide encoding the polypeptide mentioned below:
(i) a polypeptide consisting of the amino acid sequence of SEQ ID NO: 2,
(ii) a polypeptide consisting of an amino acid sequence derived from the amino acid sequence of SEQ ID NO: 2 by deletion, substitution or addition of one or more amino acids, and the polypeptide having kinase activity, or
(iii) a polypeptide consisting of an amino acid sequence having a sequence identity of at least 80% to the amino acid sequence of SEQ ID NO: 2, and the polypeptide having kinase activity;
(b) a TNIK-RNF123 fusion polynucleotide encoding the polypeptide mentioned below:
(i) a polypeptide consisting of the amino acid sequence of SEQ ID NO: 4, 6, 8, 10, 12, 14, 16 or 18,
(ii) a polypeptide consisting of an amino acid sequence derived from the amino acid sequence of SEQ ID NO: 4, 6, 8, 10, 12, 14, 16 or 18 by deletion, substitution or addition of one or more amino acids, and the polypeptide having kinase activity, or
(iii) a polypeptide consisting of an amino acid sequence having a sequence identity of at least 80% to the amino acid sequence of SEQ ID NO: 4, 6, 8, 10, 12, 14, 16 or 18, and the polypeptide having kinase activity; and
(c) an SLC 12A2-NRG2 fusion polynucleotide encoding the polypeptide mentioned below:
(i) a polypeptide consisting of the amino acid sequence of SEQ ID NO: 20, 22, 24, 26 or 28,
(ii) a polypeptide consisting of an amino acid sequence derived from the amino acid sequence of SEQ ID NO: 20, 22, 24, 26 or 28 by deletion, substitution or addition of one or more amino acids, and the polypeptide having intracellular signaling-enhancing activity, or
(iii) a polypeptide consisting of an amino acid sequence having a sequence identity of at least 80% to the amino acid sequence of SEQ ID NO: 20, 22, 24, 26 or 28, and the polypeptide having intracellular signaling-enhancing activity.

3. The method according to claim 1 or 2, wherein the fusion polynucleotide is any one of (a) to (c) below:
(a)
(i) a polynucleotide consisting of the nucleotide sequence of SEQ ID NO: 1,
(ii) a polynucleotide that hybridizes under stringent conditions with a polynucleotide consisting of a nucleotide sequence complementary to the polynucleotide consisting of the nucleotide sequence of SEQ ID NO: 1, and which encodes a polypeptide having kinase activity,
(iii) a polynucleotide that consists of a nucleotide sequence derived from the nucleotide sequence of SEQ ID NO: 1 by deletion, substitution or addition of one or more nucleotides, and which encodes a polypeptide having kinase activity, or
(iv) a polynucleotide that has a sequence identity of at least 80% to the polynucleotide consisting of the nucleotide sequence of SEQ ID NO: 1, and which encodes a polypeptide having kinase activity;
(b)
(i) a polynucleotide consisting of the nucleotide sequence of SEQ ID NO: 3,5,7, 9, 11, 13, 15 or 17,
(ii) a polynucleotide that hybridizes under stringent conditions with a polynucleotide consisting of a nucleotide sequence complementary to the polynucleotide consisting of the nucleotide sequence of SEQ ID NO: 3, 5, 7, 9, 11, 13, 15 or 17, and which encodes a polypeptide having kinase activity,
(iii) a polynucleotide that consists of a nucleotide sequence derived from the nucleotide sequence of SEQ ID NO: 3, 5, 7, 9, 11, 13, 15 or 17 by deletion, substitution or addition of one or more nucleotides, and which encodes a polypeptide having kinase activity, or
(iv) a polynucleotide that has a sequence identity of at least 80% to the polynucleotide consisting of the nucleotide sequence of SEQ ID NO: 3, 5, 7, 9, 11, 13, 15 or 17, and which encodes a polypeptide having kinase activity; and
(c)
(i) a polynucleotide consisting of the nucleotide sequence of SEQ ID NO: 19, 21, 23, 25 or 27,
(ii) a polynucleotide that hybridizes under stringent conditions with a polynucleotide consisting of a nucleotide sequence complementary to the polynucleotide consisting of the nucleotide sequence of SEQ ID NO: 19, 21, 23, 25 or 27, and which encodes a polypeptide having intracellular signaling-enhancing activity,
(iii) a polynucleotide that consists of a nucleotide sequence derived from the nucleotide sequence of SEQ ID NO: 19, 21, 23, 25 or 27 by deletion, substitution or addition of one or more nucleotides, and which encodes a polypeptide having intracellular signaling-enhancing activity, or
(iv) a polynucleotide that has a sequence identity of at least 80% to the polynucleotide consisting of the nucleotide sequence of SEQ ID NO: 19, 21, 23, 25 or 27, and which encodes a polypeptide having intracellular signaling-enhancing activity.

4. The method according to any one of claims 1 to 3, wherein the cancer is gastric cancer.

5. A method for identifying a patient with cancer or a subject with a risk of cancer, in whom a substance suppressing the expression and/or activity of a polypeptide encoded by a fusion polynucleotide produced by a gene fusion serving as a responsible mutation (driver mutation) for cancer shows a therapeutic effect, the method comprising the steps of:
(1) detecting a fusion polynucleotide of any one of (a) to (c) below, or a polypeptide encoded thereby, in an isolated sample from the subject:
(a) an ATG3-EPHB1 fusion polynucleotide which encodes a polypeptide comprising the kinase domain of EPHB1 and having kinase activity,
(b) a TNIK-RNF123 fusion polynucleotide which encodes a polypeptide comprising the kinase domain of TNIK and the SPRY domain and RING finger domain of RNF123, and having kinase activity, and
(c) an SLC12A2-NRG2 fusion polynucleotide which encodes a polypeptide comprising the AA_permease_N super family domain of SLC12A2 and the neuregulin family conserved region of NRG2, and having intracellular signaling-enhancing activity; and
(2) determining that the substance suppressing the expression and/or activity of the polypeptide shows a therapeutic effect in the subject, in the case where the fusion polynucleotide of any one of (a) to (c) or the polypeptide encoded thereby is detected.

6. A kit for detecting a gene fusion serving as a responsible mutation (driver mutation) for cancer, the kit comprising any or a combination of (A) to (C) below:
(A) a polynucleotide that serves as a probe designed to specifically recognize an ATG3-EPHB1 fusion polynucleotide, a TNIK-RNF123 fusion polynucleotide, or an SLC12A2-NRG2 fusion polynucleotide;
(B) polynucleotides that serve as a pair of primers designed to enable specific amplification of an ATG3-EPHB1 fusion polynucleotide, a TNIK-RNF123 fusion polynucleotide, or an SLC12A2-NRG2 fusion polynucleotide; and
(C) an antibody that specifically recognizes an ATG3-EPHB1 fusion polypeptide, a TNIK-RNF123 fusion polypeptide, or an SLC12A2-NRG2 fusion polypeptide.

7. An isolated ATG3-EPHB1 fusion polypeptide or a fragment thereof, which comprises the kinase domain of EPHB1 and has kinase activity.

8. An isolated TNIK-RNF123 fusion polypeptide or a fragment thereof, which comprises the kinase domain of TNIK and the SPRY domain and RING finger domain of RNF123, and has kinase activity.

9. An isolated SLC12A2-NRG2 fusion polypeptide or a fragment thereof, which comprises the AA_permease_N super family domain of SLC 12A2 and the neuregulin family conserved region of NRG2, and has intracellular signaling-enhancing activity.

10. A polynucleotide encoding the fusion polypeptide or the fragment thereof according to any one of claims 7 to 9.

11. A therapeutic agent for cancer positive for ATG3-EPHB1, TNIK-RNF123 or SLC12A2-NRG2 gene fusion.

12. The therapeutic agent according to claim 11, comprising, as an active ingredient, a substance suppressing the expression and/or activity of: an ATG3-EPHB1 fusion polypeptide comprising the kinase domain of EPHB1 and having kinase activity; a TNIK-RNF123 fusion polypeptide comprising the kinase domain of TNIK and the SPRY domain and RING finger domain of RNF 123 and having kinase activity; or an SLC12A2-NRG2 fusion polypeptide comprising the AA_permease_N super family domain of SLC12A2 and the neuregulin family conserved region of NRG2, and having intracellular signaling-enhancing activity.

13. The therapeutic agent according to claim 11 or 12, wherein the cancer is gastric cancer.

14. A method for screening a cancer therapeutic agent, the method comprising the steps of:
(1) bringing a test substance into contact with a cell that expresses: an ATG3-EPHB1 fusion polypeptide comprising the kinase domain of EPHB1 and having kinase activity; a TNIK-RNF123 fusion polypeptide comprising the kinase domain of TNIK and the SPRY domain and RING finger domain of RNF123 and having kinase activity; or an SLC12A2-NRG2 fusion polypeptide comprising the AA_permease_N super family domain of SLC 12A2 and the neuregulin family conserved region of NRG2, and having intracellular signaling-enhancing activity;
(2) determining whether the expression and/or activity of any of said fusion polypeptides is suppressed or not; and
(3) selecting the substance determined to suppress the expression and/or activity of any of said fusion polypeptides, as a cancer therapeutic agent.


SEQUENCE LISTING

<110>  National Cancer Center
       LSIP, LLC

<120>  Novel fusion genes responsible for stomach cancer

<130>  FA1575-14161

<150>  JP 2013-263032
<151>  2013-12-19

<160>  73

<170>  PatentIn version 3.5

<210>  1
<211>  1647
<212>  DNA
<213>  Homo sapiens

<220>
<221>  CDS
<222>  (1)..(1647)

<400>  1
atg cag aat gtg att aat act gtg aag gga aag gca ctg gaa gtg gct        48
Met Gln Asn Val Ile Asn Thr Val Lys Gly Lys Ala Leu Glu Val Ala
1               5                   10                  15

gag tac ctg acc ccg gtc ctc aag gaa tca aag ttt aag gaa aca ggt        96
Glu Tyr Leu Thr Pro Val Leu Lys Glu Ser Lys Phe Lys Glu Thr Gly
            20                  25                  30

gta att acc cca gaa gag gaa cac aat gag ttc aac tcc tcc atg gcc       144
Val Ile Thr Pro Glu Glu Glu His Asn Glu Phe Asn Ser Ser Met Ala
        35                  40                  45

agg agt cag acc aac aca gca agg att gat ggg ctg cgg cct ggc atg       192
Arg Ser Gln Thr Asn Thr Ala Arg Ile Asp Gly Leu Arg Pro Gly Met
    50                  55                  60

gta tat gtg gta cag gtg cgt gcc cgc act gtt gct ggc tac ggc aag       240
Val Tyr Val Val Gln Val Arg Ala Arg Thr Val Ala Gly Tyr Gly Lys
65                  70                  75                  80

ttc agt ggc aag atg tgc ttc cag act ctg act gac gat gat tac aag       288
Phe Ser Gly Lys Met Cys Phe Gln Thr Leu Thr Asp Asp Asp Tyr Lys
                85                  90                  95

tca gag ctg agg gag cag ctg ccc ctg att gct ggc tcg gca gcg gcc       336
Ser Glu Leu Arg Glu Gln Leu Pro Leu Ile Ala Gly Ser Ala Ala Ala
            100                 105                 110

ggg gtc gtg ttc gtt gtg tcc ttg gtg gcc atc tct atc gtc tgt agc       384
Gly Val Val Phe Val Val Ser Leu Val Ala Ile Ser Ile Val Cys Ser
        115                 120                 125

agg aaa cgg gct tat agc aaa gag gct gtg tac agc gat aag ctc cag       432
Arg Lys Arg Ala Tyr Ser Lys Glu Ala Val Tyr Ser Asp Lys Leu Gln
    130                 135                 140

cat tac agc aca ggc cga ggc tcc cca ggg atg aag atc tac att gac 480
His Tyr Ser Thr Gly Arg Gly Ser Pro Gly Met Lys Ile Tyr Ile Asp
145                 150                 155                 160

ccc ttc act tac gag gat ccc aac gaa gct gtc cgg gag ttt gcc aag 528
Pro Phe Thr Tyr Glu Asp Pro Asn Glu Ala Val Arg Glu Phe Ala Lys
                165                 170                 175

gag att gat gta tct ttt gtg aaa att gaa gag gtc atc gga gca ggg 576
Glu Ile Asp Val Ser Phe Val Lys Ile Glu Glu Val Ile Gly Ala Gly
            180                 185                 190

gag ttt gga gaa gtg tac aag ggg cgt ttg aaa ctg cca ggc aag agg 624
Glu Phe Gly Glu Val Tyr Lys Gly Arg Leu Lys Leu Pro Gly Lys Arg
        195                 200                 205

gaa atc tac gtg gcc atc aag acc ctg aag gca ggg tac tcg gag aag 672
Glu Ile Tyr Val Ala Ile Lys Thr Leu Lys Ala Gly Tyr Ser Glu Lys
    210                 215                 220

cag cgt cgg gac ttt ctg agt gag gcg agc atc atg ggc cag ttc gac 720
Gln Arg Arg Asp Phe Leu Ser Glu Ala Ser Ile Met Gly Gln Phe Asp
225                 230                 235                 240

cat cct aac atc att cgc ctg gag ggt gtg gtc acc aag agt cgg cct 768
His Pro Asn Ile Ile Arg Leu Glu Gly Val Val Thr Lys Ser Arg Pro
                245                 250                 255

gtc atg atc atc aca gag ttc atg gag aat ggt gca ttg gat tct ttc 816
Val Met Ile Ile Thr Glu Phe Met Glu Asn Gly Ala Leu Asp Ser Phe
            260                 265                 270

ctc agg caa aat gac ggg cag ttc acc gtg atc cag ctt gtg ggt atg 864
Leu Arg Gln Asn Asp Gly Gln Phe Thr Val Ile Gln Leu Val Gly Met
        275                 280                 285

ctc agg ggc atc gct gct ggc atg aag tac ctg gct gag atg aat tat 912
Leu Arg Gly Ile Ala Ala Gly Met Lys Tyr Leu Ala Glu Met Asn Tyr
    290                 295                 300

gtg cat cgg gac ctg gct gct agg aac att ctg gtc aac agt aac ctg 960
Val His Arg Asp Leu Ala Ala Arg Asn Ile Leu Val Asn Ser Asn Leu
305                 310                 315                 320

gtg tgc aag gtg tcc gac ttt ggc ctc tcc cgc tac ctc cag gat gac 1008
Val Cys Lys Val Ser Asp Phe Gly Leu Ser Arg Tyr Leu Gln Asp Asp
                325                 330                 335

acc tca gat ccc acc tac acc agc tcc ttg gga ggg aag atc cct gtg 1056
Thr Ser Asp Pro Thr Tyr Thr Ser Ser Leu Gly Gly Lys Ile Pro Val
            340                 345                 350

aga tgg aca gct cca gag gcc atc gcc tac cgc aag ttc act tca gcc 1104
Arg Trp Thr Ala Pro Glu Ala Ile Ala Tyr Arg Lys Phe Thr Ser Ala
        355                 360                 365

agc gac gtt tgg agc tat ggg atc gtc atg tgg gaa gtc atg tca ttt 1152
Ser Asp Val Trp Ser Tyr Gly Ile Val Met Trp Glu Val Met Ser Phe
    370                 375                 380

gga gag aga ccc tat tgg gat atg tcc aac caa gat gtc atc aat gcc 1200
Gly Glu Arg Pro Tyr Trp Asp Met Ser Asn Gln Asp Val Ile Asn Ala
385                 390                 395                 400

atc gag cag gac tac cgg ctg ccc cca ccc atg gac tgt cca gct gct     1248
Ile Glu Gln Asp Tyr Arg Leu Pro Pro Pro Met Asp Cys Pro Ala Ala
                405                 410                 415

cta cac cag ctc atg ctg gac tgt tgg cag aag gac cgg aac agc cgg     1296
Leu His Gln Leu Met Leu Asp Cys Trp Gln Lys Asp Arg Asn Ser Arg
            420                 425                 430

ccc cgg ttt gcg gag att gtc aac acc cta gat aag atg atc cgg aac     1344
Pro Arg Phe Ala Glu Ile Val Asn Thr Leu Asp Lys Met Ile Arg Asn
        435                 440                 445

ccg gca agt ctc aag act gtg gca acc atc acc gcc gtg cct tcc cag     1392
Pro Ala Ser Leu Lys Thr Val Ala Thr Ile Thr Ala Val Pro Ser Gln
    450                 455                 460

ccc ctg ctc gac cgc tcc atc cca gac ttc acg gcc ttt acc acc gtg     1440
Pro Leu Leu Asp Arg Ser Ile Pro Asp Phe Thr Ala Phe Thr Thr Val
465                 470                 475                 480

gat gac tgg ctc agc gcc atc aaa atg gtc cag tac agg gac agc ttc     1488
Asp Asp Trp Leu Ser Ala Ile Lys Met Val Gln Tyr Arg Asp Ser Phe
                485                 490                 495

ctc act gct ggc ttc acc tcc ctc cag ctg gtc acc cag atg aca tca     1536
Leu Thr Ala Gly Phe Thr Ser Leu Gln Leu Val Thr Gln Met Thr Ser
            500                 505                 510

gaa gac ctc ctg aga ata ggc atc acc ttg gca ggc cat cag aag aag     1584
Glu Asp Leu Leu Arg Ile Gly Ile Thr Leu Ala Gly His Gln Lys Lys
        515                 520                 525

atc ctg aac agc att cat tct atg agg gtc cag ata agt cag tca cca     1632
Ile Leu Asn Ser Ile His Ser Met Arg Val Gln Ile Ser Gln Ser Pro
    530                 535                 540

acg gca atg gca tga                                                 1647
Thr Ala Met Ala
545

<210>  2
<211>  548
<212>  PRT
<213>  Homo sapiens

<400>  2

Met Gln Asn Val Ile Asn Thr Val Lys Gly Lys Ala Leu Glu Val Ala
1               5                   10                  15

Glu Tyr Leu Thr Pro Val Leu Lys Glu Ser Lys Phe Lys Glu Thr Gly
            20                  25                  30

Val Ile Thr Pro Glu Glu Glu His Asn Glu Phe Asn Ser Ser Met Ala
        35                  40                  45

Arg Ser Gln Thr Asn Thr Ala Arg Ile Asp Gly Leu Arg Pro Gly Met
    50                  55                  60

Val Tyr Val Val Gln Val Arg Ala Arg Thr Val Ala Gly Tyr Gly Lys
65                  70                  75                  80

Phe Ser Gly Lys Met Cys Phe Gln Thr Leu Thr Asp Asp Asp Tyr Lys
                85                  90                  95

Ser Glu Leu Arg Glu Gln Leu Pro Leu Ile Ala Gly Ser Ala Ala Ala
            100                 105                 110

Gly Val Val Phe Val Val Ser Leu Val Ala Ile Ser Ile Val Cys Ser
        115                 120                 125

Arg Lys Arg Ala Tyr Ser Lys Glu Ala Val Tyr Ser Asp Lys Leu Gln
    130                 135                 140

His Tyr Ser Thr Gly Arg Gly Ser Pro Gly Met Lys Ile Tyr Ile Asp
145                 150                 155                 160

Pro Phe Thr Tyr Glu Asp Pro Asn Glu Ala Val Arg Glu Phe Ala Lys
                165                 170                 175

Glu Ile Asp Val Ser Phe Val Lys Ile Glu Glu Val Ile Gly Ala Gly
            180                 185                 190

Glu Phe Gly Glu Val Tyr Lys Gly Arg Leu Lys Leu Pro Gly Lys Arg
        195                 200                 205

Glu Ile Tyr Val Ala Ile Lys Thr Leu Lys Ala Gly Tyr Ser Glu Lys
    210                 215                 220

Gln Arg Arg Asp Phe Leu Ser Glu Ala Ser Ile Met Gly Gln Phe Asp
225                 230                 235                 240

His Pro Asn Ile Ile Arg Leu Glu Gly Val Val Thr Lys Ser Arg Pro
                245                 250                 255

Val Met Ile Ile Thr Glu Phe Met Glu Asn Gly Ala Leu Asp Ser Phe
            260                 265                 270

Leu Arg Gln Asn Asp Gly Gln Phe Thr Val Ile Gln Leu Val Gly Met
        275                 280                 285

Leu Arg Gly Ile Ala Ala Gly Met Lys Tyr Leu Ala Glu Met Asn Tyr
    290                 295                 300

Val His Arg Asp Leu Ala Ala Arg Asn Ile Leu Val Asn Ser Asn Leu

305                 310                 315                 320

Val Cys Lys Val Ser Asp Phe Gly Leu Ser Arg Tyr Leu Gln Asp Asp
                325                 330                 335

Thr Ser Asp Pro Thr Tyr Thr Ser Ser Leu Gly Gly Lys Ile Pro Val
            340                 345                 350

Arg Trp Thr Ala Pro Glu Ala Ile Ala Tyr Arg Lys Phe Thr Ser Ala
        355                 360                 365

Ser Asp Val Trp Ser Tyr Gly Ile Val Met Trp Glu Val Met Ser Phe
    370                 375                 380

Gly Glu Arg Pro Tyr Trp Asp Met Ser Asn Gln Asp Val Ile Asn Ala
385                 390                 395                 400

Ile Glu Gln Asp Tyr Arg Leu Pro Pro Pro Met Asp Cys Pro Ala Ala
                405                 410                 415

Leu His Gln Leu Met Leu Asp Cys Trp Gln Lys Asp Arg Asn Ser Arg
            420                 425                 430

Pro Arg Phe Ala Glu Ile Val Asn Thr Leu Asp Lys Met Ile Arg Asn
        435                 440                 445

Pro Ala Ser Leu Lys Thr Val Ala Thr Ile Thr Ala Val Pro Ser Gln
    450                 455                 460

Pro Leu Leu Asp Arg Ser Ile Pro Asp Phe Thr Ala Phe Thr Thr Val
465                 470                 475                 480

Asp Asp Trp Leu Ser Ala Ile Lys Met Val Gln Tyr Arg Asp Ser Phe
                485                 490                 495

Leu Thr Ala Gly Phe Thr Ser Leu Gln Leu Val Thr Gln Met Thr Ser
            500                 505                 510

Glu Asp Leu Leu Arg Ile Gly Ile Thr Leu Ala Gly His Gln Lys Lys
        515                 520                 525

Ile Leu Asn Ser Ile His Ser Met Arg Val Gln Ile Ser Gln Ser Pro
    530                 535                 540

Thr Ala Met Ala
545

<210>  3
<211>  7602
<212>  DNA
<213>  Homo sapiens

<220>
<221>  CDS
<222>  (1)..(7602)

<400>  3
atg gcg agc gac tcc ccg gct cga agc ctg gat gaa ata gat ctc tcg      48
Met Ala Ser Asp Ser Pro Ala Arg Ser Leu Asp Glu Ile Asp Leu Ser
1               5                   10                  15

gct ctg agg gac ccc gca ggg atc ttt gaa ttg gtg gaa ctt gtt gga      96
Ala Leu Arg Asp Pro Ala Gly Ile Phe Glu Leu Val Glu Leu Val Gly
            20                  25                  30

aat gga aca tac ggg caa gtt tat aag ggt cgt cat gtc aaa acg ggc      144
Asn Gly Thr Tyr Gly Gln Val Tyr Lys Gly Arg His Val Lys Thr Gly
        35                  40                  45

cag ctt gca gcc atc aag gtt atg gat gtc aca ggg gat gaa gag gaa      192
Gln Leu Ala Ala Ile Lys Val Met Asp Val Thr Gly Asp Glu Glu Glu
    50                  55                  60

gaa atc aaa caa gaa att aac atg ttg aag aaa tat tct cat cac cgg      240
Glu Ile Lys Gln Glu Ile Asn Met Leu Lys Lys Tyr Ser His His Arg
65                  70                  75                  80

aat att gct aca tac tat ggt gct ttt atc aaa aag aac cca cca ggc      288
Asn Ile Ala Thr Tyr Tyr Gly Ala Phe Ile Lys Lys Asn Pro Pro Gly
                85                  90                  95

atg gat gac caa ctt tgg ttg gtg atg gag ttt tgt ggt gct ggc tct      336
Met Asp Asp Gln Leu Trp Leu Val Met Glu Phe Cys Gly Ala Gly Ser
            100                 105                 110

gtc acc gac ctg atc aag aac aca aaa ggt aac acg ttg aaa gag gag      384
Val Thr Asp Leu Ile Lys Asn Thr Lys Gly Asn Thr Leu Lys Glu Glu
        115                 120                 125

tgg att gca tac atc tgc agg gaa atc tta cgg ggg ctg agt cac ctg      432
Trp Ile Ala Tyr Ile Cys Arg Glu Ile Leu Arg Gly Leu Ser His Leu
    130                 135                 140

cac cag cat aaa gtg att cat cga gat att aaa ggg caa aat gtc ttg      480
His Gln His Lys Val Ile His Arg Asp Ile Lys Gly Gln Asn Val Leu
145                 150                 155                 160

ctg act gaa aat gca gaa gtt aaa cta gtg gac ttt gga gtc agt gct      528
Leu Thr Glu Asn Ala Glu Val Lys Leu Val Asp Phe Gly Val Ser Ala
                165                 170                 175

cag ctt gat cga aca gtg ggc agg agg aat act ttc att gga act ccc      576
Gln Leu Asp Arg Thr Val Gly Arg Arg Asn Thr Phe Ile Gly Thr Pro
            180                 185                 190

tac tgg atg gca cca gaa gtt att gcc tgt gat gaa aac cca gat gcc      624
Tyr Trp Met Ala Pro Glu Val Ile Ala Cys Asp Glu Asn Pro Asp Ala
        195                 200                 205

aca tat gat ttc aag agt gac ttg tgg tct ttg ggt atc acc gcc att     672
Thr Tyr Asp Phe Lys Ser Asp Leu Trp Ser Leu Gly Ile Thr Ala Ile
    210                 215                 220

gaa atg gca gaa ggt gct ccc cct ctc tgt gac atg cac ccc atg aga     720
Glu Met Ala Glu Gly Ala Pro Pro Leu Cys Asp Met His Pro Met Arg
225                 230                 235                 240

gct ctc ttc ctc atc ccc cgg aac cca gcg cct cgg ctg aag tct aag     768
Ala Leu Phe Leu Ile Pro Arg Asn Pro Ala Pro Arg Leu Lys Ser Lys
                245                 250                 255

aag tgg tca aaa aaa ttc cag tca ttt att gag agc tgc ttg gta aag     816
Lys Trp Ser Lys Lys Phe Gln Ser Phe Ile Glu Ser Cys Leu Val Lys
            260                 265                 270

aat cac agc cag cga cca gca aca gaa caa ttg atg aag cat cca ttt     864
Asn His Ser Gln Arg Pro Ala Thr Glu Gln Leu Met Lys His Pro Phe
        275                 280                 285

ata cga gac caa cct aat gag cga cag gtc cgc att caa ctc aag gac     912
Ile Arg Asp Gln Pro Asn Glu Arg Gln Val Arg Ile Gln Leu Lys Asp
    290                 295                 300

cat att gat aga aca aag aag aag cga gga gaa aaa gat gag aca gag     960
His Ile Asp Arg Thr Lys Lys Lys Arg Gly Glu Lys Asp Glu Thr Glu
305                 310                 315                 320

tat gag tac agt gga agt gag gaa gaa gag gag gag aat gac tca gga     1008
Tyr Glu Tyr Ser Gly Ser Glu Glu Glu Glu Glu Glu Asn Asp Ser Gly
                325                 330                 335

gag ccc agc tcc atc ctg aat ctg cca ggg gag tcg acg ctg cgg agg     1056
Glu Pro Ser Ser Ile Leu Asn Leu Pro Gly Glu Ser Thr Leu Arg Arg
            340                 345                 350

gac ttt ctg agg ctg cag ctg gcc aac aag gag cgt tct gag gcc cta     1104
Asp Phe Leu Arg Leu Gln Leu Ala Asn Lys Glu Arg Ser Glu Ala Leu
        355                 360                 365

cgg agg cag cag ctg gag cag cag cag cgg gag aat gag gag cac aag     1152
Arg Arg Gln Gln Leu Glu Gln Gln Gln Arg Glu Asn Glu Glu His Lys
    370                 375                 380

cgg cag ctg ctg gcc gag cgt cag aag cgc atc gag gag cag aaa gag     1200
Arg Gln Leu Leu Ala Glu Arg Gln Lys Arg Ile Glu Glu Gln Lys Glu
385                 390                 395                 400

cag agg cgg cgg ctg gag gag caa caa agg cga gag aag gag ctg cgg     1248
Gln Arg Arg Arg Leu Glu Glu Gln Gln Arg Arg Glu Lys Glu Leu Arg
                405                 410                 415

aag cag cag gag agg gag cag cgc cgg cac tat gag gag cag atg cgc     1296
Lys Gln Gln Glu Arg Glu Gln Arg Arg His Tyr Glu Glu Gln Met Arg
            420                 425                 430

cgg gag gag gag agg agg cgt gcg gag cat gaa cag gaa tac atc agg     1344
Arg Glu Glu Glu Arg Arg Arg Ala Glu His Glu Gln Glu Tyr Ile Arg
        435                 440                 445

cga cag tta gag gag gag cag aga cag tta gag atc ttg cag cag cag     1392
Arg Gln Leu Glu Glu Glu Gln Arg Gln Leu Glu Ile Leu Gln Gln Gln
    450                 455                 460

cta ctg cat gaa caa gct cta ctt ctg gaa tat aag cgc aaa caa ttg 1440
Leu Leu His Glu Gln Ala Leu Leu Leu Glu Tyr Lys Arg Lys Gln Leu
465                 470                 475                 480

gaa gaa cag aga caa gca gaa aga ctg cag agg cag cta aag caa gaa 1488
Glu Glu Gln Arg Gln Ala Glu Arg Leu Gln Arg Gln Leu Lys Gln Glu
                485                 490                 495

aga gac tac tta gtt tcc ctt cag cat cag cgg cag gag cag agg cct 1536
Arg Asp Tyr Leu Val Ser Leu Gln His Gln Arg Gln Glu Gln Arg Pro
            500                 505                 510

gtg gag aag aag cca ctg tac cat tac aaa gaa gga atg agt cct agt 1584
Val Glu Lys Lys Pro Leu Tyr His Tyr Lys Glu Gly Met Ser Pro Ser
        515                 520                 525

gag aag cca gca tgg gcc aag gag gta gaa gaa cgg tca agg ctc aac 1632
Glu Lys Pro Ala Trp Ala Lys Glu Val Glu Glu Arg Ser Arg Leu Asn
    530                 535                 540

cgg caa agt tcc cct gcc atg cct cac aag gtt gcc aac agg ata tct 1680
Arg Gln Ser Ser Pro Ala Met Pro His Lys Val Ala Asn Arg Ile Ser
545                 550                 555                 560

gac ccc aac ctg ccc cca agg tcg gag tcc ttc agc att agt gga gtt 1728
Asp Pro Asn Leu Pro Pro Arg Ser Glu Ser Phe Ser Ile Ser Gly Val
                565                 570                 575

cag cct gct cga aca ccc ccc atg ctc aga cca gtc gat ccc cag atc 1776
Gln Pro Ala Arg Thr Pro Pro Met Leu Arg Pro Val Asp Pro Gln Ile
            580                 585                 590

cca cat ctg gta gct gta aaa tcc cag gga cct gcc ttg acc gcc tcc 1824
Pro His Leu Val Ala Val Lys Ser Gln Gly Pro Ala Leu Thr Ala Ser
        595                 600                 605

cag tca gtg cac gag cag ccc aca aag ggc ctc tct ggg ttt cag gag 1872
Gln Ser Val His Glu Gln Pro Thr Lys Gly Leu Ser Gly Phe Gln Glu
    610                 615                 620

gct ctg aac gtg acc tcc cac cgc gtg gag atg cca cgc cag aac tca 1920
Ala Leu Asn Val Thr Ser His Arg Val Glu Met Pro Arg Gln Asn Ser
625                 630                 635                 640

gat ccc acc tcg gaa aat cct cct ctc ccc act cgc att gaa aag ttt 1968
Asp Pro Thr Ser Glu Asn Pro Pro Leu Pro Thr Arg Ile Glu Lys Phe
                645                 650                 655

gac cga agc tct tgg tta cga cag gaa gaa gac att cca cca aag gtg 2016
Asp Arg Ser Ser Trp Leu Arg Gln Glu Glu Asp Ile Pro Pro Lys Val
            660                 665                 670

cct caa aga aca act tct ata tcc cca gca tta gcc aga aag aat tct 2064
Pro Gln Arg Thr Thr Ser Ile Ser Pro Ala Leu Ala Arg Lys Asn Ser
        675                 680                 685

cct ggg aat ggt agt gct ctg gga ccc aga cta gga tct caa ccc atc 2112
Pro Gly Asn Gly Ser Ala Leu Gly Pro Arg Leu Gly Ser Gln Pro Ile
    690                 695                 700

aga gca agc aac cct gat ctc cgg aga act gag ccc atc ttg gag agc 2160
Arg Ala Ser Asn Pro Asp Leu Arg Arg Thr Glu Pro Ile Leu Glu Ser

705                 710                 715                 720

ccc ttg cag agg acc agc agt ggc agt tcc tcc agc tcc agc acc cct      2208
Pro Leu Gln Arg Thr Ser Ser Gly Ser Ser Ser Ser Ser Ser Thr Pro
                725                 730                 735

agc tcc cag ccc agc tcc caa gga ggc tcc cag cct gga tca caa gca      2256
Ser Ser Gln Pro Ser Ser Gln Gly Gly Ser Gln Pro Gly Ser Gln Ala
            740                 745                 750

gga tcc agt gaa cgc acc aga gtt cga gcc aac agt aag tca gaa gga      2304
Gly Ser Ser Glu Arg Thr Arg Val Arg Ala Asn Ser Lys Ser Glu Gly
        755                 760                 765

tca cct gtg ctc ccc cat gag cct gcc aag gtg aaa cca gaa gaa tcc      2352
Ser Pro Val Leu Pro His Glu Pro Ala Lys Val Lys Pro Glu Glu Ser
    770                 775                 780

agg gac att acc cgg ccc agt cga cca gct agc tac aaa aaa gct ata      2400
Arg Asp Ile Thr Arg Pro Ser Arg Pro Ala Ser Tyr Lys Lys Ala Ile
785                 790                 795                 800

gat gag gat ctg acg gca tta gcc aaa gaa cta aga gaa ctc cgg att      2448
Asp Glu Asp Leu Thr Ala Leu Ala Lys Glu Leu Arg Glu Leu Arg Ile
                805                 810                 815

gaa gaa aca aac cgc cca atg aag aag gtg act gat tac tcc tcc tcc      2496
Glu Glu Thr Asn Arg Pro Met Lys Lys Val Thr Asp Tyr Ser Ser Ser
            820                 825                 830

agt gag gag tca gaa agt agc gag gaa gag gag gaa gat gga gag agc      2544
Ser Glu Glu Ser Glu Ser Ser Glu Glu Glu Glu Glu Asp Gly Glu Ser
        835                 840                 845

gag acc cat gat ggg aca gtg gct gtc agc gac ata ccc aga ctg ata      2592
Glu Thr His Asp Gly Thr Val Ala Val Ser Asp Ile Pro Arg Leu Ile
    850                 855                 860

cca aca gga gct cca ggc agc aac gag cag tac aat gtg gga atg gtg      2640
Pro Thr Gly Ala Pro Gly Ser Asn Glu Gln Tyr Asn Val Gly Met Val
865                 870                 875                 880

ggg acg cat ggg ctg gag acc tct cat gcg gac agt ttc agc ggc agt      2688
Gly Thr His Gly Leu Glu Thr Ser His Ala Asp Ser Phe Ser Gly Ser
                885                 890                 895

att tca aga gaa gga acc ttg atg att aga gag acg tct gga gag aag      2736
Ile Ser Arg Glu Gly Thr Leu Met Ile Arg Glu Thr Ser Gly Glu Lys
            900                 905                 910

aag cga tct ggc cac agt gac agc aat ggc ttt gct ggc cac atc aac      2784
Lys Arg Ser Gly His Ser Asp Ser Asn Gly Phe Ala Gly His Ile Asn
        915                 920                 925

ctc cct gac ctg gtg cag cag agc cat tct cca gct gga acc ccg act      2832
Leu Pro Asp Leu Val Gln Gln Ser His Ser Pro Ala Gly Thr Pro Thr
    930                 935                 940

gag gga ctg ggg cgc gtc tca acc cat tcc cag gag atg gac tct ggg      2880
Glu Gly Leu Gly Arg Val Ser Thr His Ser Gln Glu Met Asp Ser Gly
945                 950                 955                 960

act gaa tat ggc atg ggg agc agc acc aaa gcc tcc ttc acc ccc ttt      2928

Thr Glu Tyr Gly Met Gly Ser Ser Thr Lys Ala Ser Phe Thr Pro Phe
                965                 970                 975

gtg gac ccc aga gta tac cag acg tct ccc act gat gaa gat gaa gag      2976
Val Asp Pro Arg Val Tyr Gln Thr Ser Pro Thr Asp Glu Asp Glu Glu
            980                 985                 990

gat gag gaa tca tca gcc gca gct  ctg ttt act agc gaa  ctt ctt agg    3024
Asp Glu Glu Ser Ser Ala Ala Ala  Leu Phe Thr Ser Glu  Leu Leu Arg
        995                 1000                 1005

caa gaa  cag gcc aaa ctc aat  gaa gca aga aag att  tcg gtg gta    3069
Gln Glu  Gln Ala Lys Leu Asn  Glu Ala Arg Lys Ile  Ser Val Val
    1010                 1015                 1020

aat gta  aac cca acc aac att  cgg cct cat agc gac  aca cca gaa    3114
Asn Val  Asn Pro Thr Asn Ile  Arg Pro His Ser Asp  Thr Pro Glu
    1025                 1030                 1035

atc aga  aaa tac aag aaa cga  ttc aac tca gaa ata  ctt tgt gca    3159
Ile Arg  Lys Tyr Lys Lys Arg  Phe Asn Ser Glu Ile  Leu Cys Ala
    1040                 1045                 1050

gct ctg  tgg ggt gta aac ctt  ctg gtg ggg act gaa  aat ggc ctg    3204
Ala Leu  Trp Gly Val Asn Leu  Leu Val Gly Thr Glu  Asn Gly Leu
    1055                 1060                 1065

atg ctt  ttg gac cga agt ggg  caa ggc aaa gtc tat  aat ctg atc    3249
Met Leu  Leu Asp Arg Ser Gly  Gln Gly Lys Val Tyr  Asn Leu Ile
    1070                 1075                 1080

aac cgg  agg cga ttt cag cag  atg gat gtg cta gag  gga ctg aat    3294
Asn Arg  Arg Arg Phe Gln Gln  Met Asp Val Leu Glu  Gly Leu Asn
    1085                 1090                 1095

gtc ctt  gtg aca att tca gga  aag aag aat aag cta  cga gtt tac    3339
Val Leu  Val Thr Ile Ser Gly  Lys Lys Asn Lys Leu  Arg Val Tyr
    1100                 1105                 1110

tat ctt  tca tgg tta aga aac  aga ata cta cat aat  gac cca gaa    3384
Tyr Leu  Ser Trp Leu Arg Asn  Arg Ile Leu His Asn  Asp Pro Glu
    1115                 1120                 1125

gta gaa  aag aaa caa ggc tgg  atc act gtt ggg gac  ttg gaa ggc    3429
Val Glu  Lys Lys Gln Gly Trp  Ile Thr Val Gly Asp  Leu Glu Gly
    1130                 1135                 1140

tgt ata  cat tat aaa gtt gtt  aaa tat gaa agg atc  aaa ttt ttg    3474
Cys Ile  His Tyr Lys Val Val  Lys Tyr Glu Arg Ile  Lys Phe Leu
    1145                 1150                 1155

gtg att  gcc tta aag aat gct  gtg gaa ata tat gct  tgg gct cct    3519
Val Ile  Ala Leu Lys Asn Ala  Val Glu Ile Tyr Ala  Trp Ala Pro
    1160                 1165                 1170

aaa ccg  tat cat aaa ttc atg  gca ttt aag tct ttt  gca gat ctc    3564
Lys Pro  Tyr His Lys Phe Met  Ala Phe Lys Ser Phe  Ala Asp Leu
    1175                 1180                 1185

cag cac  aag cct ctg cta gtt  gat ctc acg gta gaa  gaa ggt caa    3609
Gln His  Lys Pro Leu Leu Val  Asp Leu Thr Val Glu  Glu Gly Gln
    1190                 1195                 1200

aga tta aag gtt att ttt ggt tca cac act ggt ttc cat gta att 3654
Arg Leu Lys Val Ile Phe Gly Ser His Thr Gly Phe His Val Ile
    1205                1210                1215

gat gtt gat tca gga aac tct tat gat atc tac ata cca tct cat 3699
Asp Val Asp Ser Gly Asn Ser Tyr Asp Ile Tyr Ile Pro Ser His
    1220                1225                1230

att cag ggc aat atc act cct cat gct att gtc atc ttg cct aaa 3744
Ile Gln Gly Asn Ile Thr Pro His Ala Ile Val Ile Leu Pro Lys
    1235                1240                1245

aca gat gga atg gaa atg ctt gtt tgc tat gag gat gag ggg gtg 3789
Thr Asp Gly Met Glu Met Leu Val Cys Tyr Glu Asp Glu Gly Val
    1250                1255                1260

tat gta aac acc tat ggc cgg ata act aag gat gtg gtg ctc caa 3834
Tyr Val Asn Thr Tyr Gly Arg Ile Thr Lys Asp Val Val Leu Gln
    1265                1270                1275

tgg gga gaa atg ccc acg tct gtg gcc tac att cat tcc aat cag 3879
Trp Gly Glu Met Pro Thr Ser Val Ala Tyr Ile His Ser Asn Gln
    1280                1285                1290

ata atg ggc tgg ggc gag aaa gct att gag atc cgg tca gtg gaa 3924
Ile Met Gly Trp Gly Glu Lys Ala Ile Glu Ile Arg Ser Val Glu
    1295                1300                1305

aca gga cat ttg gat gga gta ttt atg cat aag cga gct caa agg 3969
Thr Gly His Leu Asp Gly Val Phe Met His Lys Arg Ala Gln Arg
    1310                1315                1320

tta aag ttt cta tgt gaa aga aat gat aag gtg att gga cac agc 4014
Leu Lys Phe Leu Cys Glu Arg Asn Asp Lys Val Ile Gly His Ser
    1325                1330                1335

aac ttt ggc acc atc cgc tct acc aca tgc gtg tac aaa ggg aaa 4059
Asn Phe Gly Thr Ile Arg Ser Thr Thr Cys Val Tyr Lys Gly Lys
    1340                1345                1350

tgg ctc tac gag gtc ctc atc tcc tcc cag ggg ctc atg cag atc 4104
Trp Leu Tyr Glu Val Leu Ile Ser Ser Gln Gly Leu Met Gln Ile
    1355                1360                1365

ggc tgg tgc acc atc agc tgc cgc ttc aac cag gag gag ggg gtt 4149
Gly Trp Cys Thr Ile Ser Cys Arg Phe Asn Gln Glu Glu Gly Val
    1370                1375                1380

gga gat aca cac aac tcc tat gcc tat gat ggc aac cgc gtg cgc 4194
Gly Asp Thr His Asn Ser Tyr Ala Tyr Asp Gly Asn Arg Val Arg
    1385                1390                1395

aag tgg aat gtg acc aca acg aat tat ggc aag gcg tgg gca gcg 4239
Lys Trp Asn Val Thr Thr Thr Asn Tyr Gly Lys Ala Trp Ala Ala
    1400                1405                1410

ggg gac atc gtg agc tgc ctg att gac ctg gat gat ggc act ctg 4284
Gly Asp Ile Val Ser Cys Leu Ile Asp Leu Asp Asp Gly Thr Leu
    1415                1420                1425

tcc ttc tgc ctg aac ggt gta tca ctg ggc act gcc ttt gag aac 4329
Ser Phe Cys Leu Asn Gly Val Ser Leu Gly Thr Ala Phe Glu Asn
    1430                1435                1440

ctg tcc agg ggc ctg ggt atg gcc tac ttc cca gcc atc agc ctc 4374
Leu Ser Arg Gly Leu Gly Met Ala Tyr Phe Pro Ala Ile Ser Leu
    1445                1450                1455

tct ttc aag gag tcc gtg gcc ttc aac ttt ggc agc cgt cct ctg 4419
Ser Phe Lys Glu Ser Val Ala Phe Asn Phe Gly Ser Arg Pro Leu
    1460                1465                1470

cgc tac cca gtg gca ggc tac cgg ccc ctg cag gac cca ccg agt 4464
Arg Tyr Pro Val Ala Gly Tyr Arg Pro Leu Gln Asp Pro Pro Ser
    1475                1480                1485

gct gac ctg gtg cgg gca cag agg ttg ctg ggc tgc ttc cgg gca 4509
Ala Asp Leu Val Arg Ala Gln Arg Leu Leu Gly Cys Phe Arg Ala
    1490                1495                1500

gtg ctg agt gtg gag ctg gac cct gtg gag ggg cgg ctg ttg gac 4554
Val Leu Ser Val Glu Leu Asp Pro Val Glu Gly Arg Leu Leu Asp
    1505                1510                1515

aag gag agc tcc aag tgg cgg ttg cgg ggc cag ccc acc gtc ctc 4599
Lys Glu Ser Ser Lys Trp Arg Leu Arg Gly Gln Pro Thr Val Leu
    1520                1525                1530

ctc aca ctg gcc cac atc ttc cat cac ttt gca ccg ctt ctg cgc 4644
Leu Thr Leu Ala His Ile Phe His His Phe Ala Pro Leu Leu Arg
    1535                1540                1545

aag gtg tat ctg gtg gag gct gtg ctc atg agc ttc ttg ctg ggc 4689
Lys Val Tyr Leu Val Glu Ala Val Leu Met Ser Phe Leu Leu Gly
    1550                1555                1560

atc gtg gag aag ggc aca ccc aca cag gca cag tcc gtg gtg cac 4734
Ile Val Glu Lys Gly Thr Pro Thr Gln Ala Gln Ser Val Val His
    1565                1570                1575

cag gtc ctg gac ctc ttg tgg ctc ttc atg gag gac tac gag gta 4779
Gln Val Leu Asp Leu Leu Trp Leu Phe Met Glu Asp Tyr Glu Val
    1580                1585                1590

caa gat tgc ctc aag cag ttg atg atg tct ctg ctt cgg ctg tac 4824
Gln Asp Cys Leu Lys Gln Leu Met Met Ser Leu Leu Arg Leu Tyr
    1595                1600                1605

cga ttc tca ccc att gtc cca gac ctg ggc cta cag atc cat tac 4869
Arg Phe Ser Pro Ile Val Pro Asp Leu Gly Leu Gln Ile His Tyr
    1610                1615                1620

ctg cgg ctc act atc gcc atc ctg agg cat gag aag tcc cgc aag 4914
Leu Arg Leu Thr Ile Ala Ile Leu Arg His Glu Lys Ser Arg Lys
    1625                1630                1635

ttt ctg ctt agc aat gtc ctc ttc gac gtg ctc cgc tcc gtc gtc 4959
Phe Leu Leu Ser Asn Val Leu Phe Asp Val Leu Arg Ser Val Val
    1640                1645                1650

ttc ttt tac atc aag agc ccc ctg cgt gtg gag gag gcc ggc ctg 5004
Phe Phe Tyr Ile Lys Ser Pro Leu Arg Val Glu Glu Ala Gly Leu
    1655                1660                1665

cag gag ctc att ccc acc acc tgg tgg ccc cac tgc tcc agt agg 5049
Gln Glu Leu Ile Pro Thr Thr Trp Trp Pro His Cys Ser Ser Arg

1670                    1675                    1680

gag ggc aaa gag agc acg gag atg aag gag gag acc gca gag gag          5094
Glu Gly Lys Glu Ser Thr Glu Met Lys Glu Glu Thr Ala Glu Glu
    1685                    1690                    1695

cgg ctg cgg cgg cga gcc tac gaa cgg ggc tgt cag cgg ctc agg          5139
Arg Leu Arg Arg Arg Ala Tyr Glu Arg Gly Cys Gln Arg Leu Arg
    1700                    1705                    1710

aag cgc atc gaa gtg gtg gaa gaa cta cag gtc cag atc ctg aag          5184
Lys Arg Ile Glu Val Val Glu Glu Leu Gln Val Gln Ile Leu Lys
    1715                    1720                    1725

ctg ctg ctg gac aat aaa gat gac aat ggg ggt gaa gct tct agg          5229
Leu Leu Leu Asp Asn Lys Asp Asp Asn Gly Gly Glu Ala Ser Arg
    1730                    1735                    1740

tat atc ttc ctg acc aag ttt cgc aag ttt ctg cag gag aac gcc          5274
Tyr Ile Phe Leu Thr Lys Phe Arg Lys Phe Leu Gln Glu Asn Ala
    1745                    1750                    1755

agt ggc cgg ggg aac atg ccc atg ctc tgc ccc cct gag tac atg          5319
Ser Gly Arg Gly Asn Met Pro Met Leu Cys Pro Pro Glu Tyr Met
    1760                    1765                    1770

gtc tgc ttc tta cac cgg ctg atc tct gcc ctg cgc tac tat tgg          5364
Val Cys Phe Leu His Arg Leu Ile Ser Ala Leu Arg Tyr Tyr Trp
    1775                    1780                    1785

gat gaa tac aag gct tcc aat cct cat gct tcc ttc agt gag gag          5409
Asp Glu Tyr Lys Ala Ser Asn Pro His Ala Ser Phe Ser Glu Glu
    1790                    1795                    1800

gcc tac atc ccg ccc cag gtc ttc tat aat ggc aag gtg gac tac          5454
Ala Tyr Ile Pro Pro Gln Val Phe Tyr Asn Gly Lys Val Asp Tyr
    1805                    1810                    1815

ttt gac ctg cag cgc ctg ggg ggc ctc ctc tcg cac ctg cgg aag          5499
Phe Asp Leu Gln Arg Leu Gly Gly Leu Leu Ser His Leu Arg Lys
    1820                    1825                    1830

acc ctc aaa gat gac ctt gct tcc aaa gcc aac att gtg atc gac          5544
Thr Leu Lys Asp Asp Leu Ala Ser Lys Ala Asn Ile Val Ile Asp
    1835                    1840                    1845

cca ctg gag ctc cag tca acc gcc atg gat gac cta gat gag gat          5589
Pro Leu Glu Leu Gln Ser Thr Ala Met Asp Asp Leu Asp Glu Asp
    1850                    1855                    1860

gag gag cca gcc cca gct atg gcc cag cgc ccc atg cag gcc ctg          5634
Glu Glu Pro Ala Pro Ala Met Ala Gln Arg Pro Met Gln Ala Leu
    1865                    1870                    1875

gct gtt ggg ggg cca ctg ccc ctg ccc cgg ccc ggc tgg ctc agt          5679
Ala Val Gly Gly Pro Leu Pro Leu Pro Arg Pro Gly Trp Leu Ser
    1880                    1885                    1890

tct cca act ttg ggc cga gcc aac cgc ttc ctc agc aca gcg gct          5724
Ser Pro Thr Leu Gly Arg Ala Asn Arg Phe Leu Ser Thr Ala Ala
    1895                    1900                    1905

gtg agc ctc atg acc cca cgg cgg cct ctg agc acc tcg gag aaa          5769

Val Ser Leu Met Thr Pro Arg Arg Pro Leu Ser Thr Ser Glu Lys
    1910                1915                1920

gtg aag gtc cgc acg ctg agc gtg gag cag agg acc cgt gag gac      5814
Val Lys Val Arg Thr Leu Ser Val Glu Gln Arg Thr Arg Glu Asp
    1925                1930                1935

att gaa ggc agc cac tgg aat gag ggc ttg ctg ctg ggg cgg ccc      5859
Ile Glu Gly Ser His Trp Asn Glu Gly Leu Leu Leu Gly Arg Pro
    1940                1945                1950

ccc gag gag cct gag cag ccc ctc acc gag aac tcg ctg ctg gaa      5904
Pro Glu Glu Pro Glu Gln Pro Leu Thr Glu Asn Ser Leu Leu Glu
    1955                1960                1965

gtc ctg gat ggg gcg gtc atg atg tac aac ctc agc gta cac cag      5949
Val Leu Asp Gly Ala Val Met Met Tyr Asn Leu Ser Val His Gln
    1970                1975                1980

cag ctg ggc aag atg gtg ggt gtc tcc gat gat gtc aat gaa tac      5994
Gln Leu Gly Lys Met Val Gly Val Ser Asp Asp Val Asn Glu Tyr
    1985                1990                1995

gct atg gct ctg agg gac aca gag gac aag ctc cgc cgg tgc ccc      6039
Ala Met Ala Leu Arg Asp Thr Glu Asp Lys Leu Arg Arg Cys Pro
    2000                2005                2010

aag agg agg aag gac atc ctt gca gag ttg acc aag agc cag aag      6084
Lys Arg Arg Lys Asp Ile Leu Ala Glu Leu Thr Lys Ser Gln Lys
    2015                2020                2025

gtt ttc tca gaa aag ctg gac cac ctg agc cgc cgt ctt gcc tgg      6129
Val Phe Ser Glu Lys Leu Asp His Leu Ser Arg Arg Leu Ala Trp
    2030                2035                2040

gtc cat gcc act gtc tac tcc cag gag aag atg ctg gac atc tac      6174
Val His Ala Thr Val Tyr Ser Gln Glu Lys Met Leu Asp Ile Tyr
    2045                2050                2055

tgg ctg ctg cgc gtc tgc ctg cgg acc att gag cac ggt gat cgc      6219
Trp Leu Leu Arg Val Cys Leu Arg Thr Ile Glu His Gly Asp Arg
    2060                2065                2070

aca ggg tct ctc ttt gcc ttc atg ccc gag ttc tac ctg agc gtg      6264
Thr Gly Ser Leu Phe Ala Phe Met Pro Glu Phe Tyr Leu Ser Val
    2075                2080                2085

gcc atc aac agc tac agt gct ctc aag aat tac ttt ggt ccc gtg      6309
Ala Ile Asn Ser Tyr Ser Ala Leu Lys Asn Tyr Phe Gly Pro Val
    2090                2095                2100

cac agc atg gag gag ctc cca ggc tat gaa gag acc ctg acc cgc      6354
His Ser Met Glu Glu Leu Pro Gly Tyr Glu Glu Thr Leu Thr Arg
    2105                2110                2115

ctg gct gcc att ctc gcc aaa cac ttt gcc gac gca cgc att gtg      6399
Leu Ala Ala Ile Leu Ala Lys His Phe Ala Asp Ala Arg Ile Val
    2120                2125                2130

ggc act gac atc cga gac tca ctg atg cag gcc ctg gcc agc tac      6444
Gly Thr Asp Ile Arg Asp Ser Leu Met Gln Ala Leu Ala Ser Tyr
    2135                2140                2145

gtg tgc tac cca cac tcc ctg cgg gct gtg gag cga atc ccc gag 6489
Val Cys Tyr Pro His Ser Leu Arg Ala Val Glu Arg Ile Pro Glu
    2150                2155                2160

gag cag cgt atc gcc atg gtg agg aac ctc ctg gcg ccc tat gag 6534
Glu Gln Arg Ile Ala Met Val Arg Asn Leu Leu Ala Pro Tyr Glu
    2165                2170                2175

cag cgg ccc tgg gcc cag acc aac tgg atc ctg gtg cgg ctc tgg 6579
Gln Arg Pro Trp Ala Gln Thr Asn Trp Ile Leu Val Arg Leu Trp
    2180                2185                2190

agg ggc tgt ggc ttc ggg tac cgc tat aca cgg ctg cca cat ctg 6624
Arg Gly Cys Gly Phe Gly Tyr Arg Tyr Thr Arg Leu Pro His Leu
    2195                2200                2205

ctg aaa acc aaa ctt gag gac gcc aat ttg ccc agc ctc cag aag 6669
Leu Lys Thr Lys Leu Glu Asp Ala Asn Leu Pro Ser Leu Gln Lys
    2210                2215                2220

ccc tgc cct tcc acc ctg ctg cag cag cac atg gcg gac ctc cta 6714
Pro Cys Pro Ser Thr Leu Leu Gln Gln His Met Ala Asp Leu Leu
    2225                2230                2235

cag cag ggt cct gat gtg gca ccc agc ttc ctc aac agc gtc ctc 6759
Gln Gln Gly Pro Asp Val Ala Pro Ser Phe Leu Asn Ser Val Leu
    2240                2245                2250

aat cag ctc aac tgg gcc ttc tct gaa ttc att ggc atg atc caa 6804
Asn Gln Leu Asn Trp Ala Phe Ser Glu Phe Ile Gly Met Ile Gln
    2255                2260                2265

gag atc cag cag gct gct gag cgc ctg gag cgg aac ttt gtg gac 6849
Glu Ile Gln Gln Ala Ala Glu Arg Leu Glu Arg Asn Phe Val Asp
    2270                2275                2280

agc cgg cag ctc aag gta tgt gcc acc tgc ttt gac ctc tcg gtc 6894
Ser Arg Gln Leu Lys Val Cys Ala Thr Cys Phe Asp Leu Ser Val
    2285                2290                2295

agc ctg ctg cgt gtc ttg gag atg act atc aca ctg gtg cct gag 6939
Ser Leu Leu Arg Val Leu Glu Met Thr Ile Thr Leu Val Pro Glu
    2300                2305                2310

ata ttc ctt gac tgg acc cgg cct acc tct gag atg ctg ctg cgg 6984
Ile Phe Leu Asp Trp Thr Arg Pro Thr Ser Glu Met Leu Leu Arg
    2315                2320                2325

cgt ctt gca cag ctg cta aac cag gtg ctg aac cgg gtg aca gct 7029
Arg Leu Ala Gln Leu Leu Asn Gln Val Leu Asn Arg Val Thr Ala
    2330                2335                2340

gag agg aac ctg ttt gat cgt gtg gtc acc cta cgg ctg cct ggc 7074
Glu Arg Asn Leu Phe Asp Arg Val Val Thr Leu Arg Leu Pro Gly
    2345                2350                2355

cta gag agc gtg gac cac tat ccc att ctg gtg gca gtg acg ggc 7119
Leu Glu Ser Val Asp His Tyr Pro Ile Leu Val Ala Val Thr Gly
    2360                2365                2370

atc ctg gtg cag ctc ctg gtg cgt ggc cca gcc tca gag aga gag 7164
Ile Leu Val Gln Leu Leu Val Arg Gly Pro Ala Ser Glu Arg Glu
    2375                2380                2385

caa gcc aca tca gtg ctc ctg gca gat ccc tgc ttc cag cta cgc 7209
Gln Ala Thr Ser Val Leu Leu Ala Asp Pro Cys Phe Gln Leu Arg
    2390                2395                2400

tca ata tgc tat ctc ctg gga cag cca gag ccc cca gca cct ggc 7254
Ser Ile Cys Tyr Leu Leu Gly Gln Pro Glu Pro Pro Ala Pro Gly
    2405                2410                2415

act gct ctg cca gcc cct gac cgg aag cgc ttc tcc ctg cag agc 7299
Thr Ala Leu Pro Ala Pro Asp Arg Lys Arg Phe Ser Leu Gln Ser
    2420                2425                2430

tat gcg gat tat atc agt gcc gat gag ctg gcc caa gtg gaa cag 7344
Tyr Ala Asp Tyr Ile Ser Ala Asp Glu Leu Ala Gln Val Glu Gln
    2435                2440                2445

atg ctg gcg cac ctg acc tct gca tct gcc cag gca gca gct gcc 7389
Met Leu Ala His Leu Thr Ser Ala Ser Ala Gln Ala Ala Ala Ala
    2450                2455                2460

tcc ctg ccc acc agt gag gag gac ctc tgc ccc atc tgc tat gcc 7434
Ser Leu Pro Thr Ser Glu Glu Asp Leu Cys Pro Ile Cys Tyr Ala
    2465                2470                2475

cac ccc atc tct gct gtg ttc cag ccc tgt ggc cac aag tcc tgc 7479
His Pro Ile Ser Ala Val Phe Gln Pro Cys Gly His Lys Ser Cys
    2480                2485                2490

aaa gcc tgt atc aac cag cac ctg atg aac aac aag gac tgc ttc 7524
Lys Ala Cys Ile Asn Gln His Leu Met Asn Asn Lys Asp Cys Phe
    2495                2500                2505

ttc tgc aaa acc acc atc gtg tct gta gag gac tgg gag aag gga 7569
Phe Cys Lys Thr Thr Ile Val Ser Val Glu Asp Trp Glu Lys Gly
    2510                2515                2520

gcc aat acg agt act acc tcc tca gct gcc tag 7602
Ala Asn Thr Ser Thr Thr Ser Ser Ala Ala
    2525                2530

<210> 4
<211> 2533
<212> PRT
<213> Homo sapiens

<400> 4
   Met Ala Ser Asp Ser Pro Ala Arg Ser Leu Asp Glu Ile Asp Leu Ser
   1 5 10 15
   Ala Leu Arg Asp Pro Ala Gly Ile Phe Glu Leu Val Glu Leu Val Gly
   20 25 30
   Asn Gly Thr Tyr Gly Gln Val Tyr Lys Gly Arg His Val Lys Thr Gly
   35 40 45
   Gln Leu Ala Ala Ile Lys Val Met Asp Val Thr Gly Asp Glu Glu Glu
   50 55 60
   Glu Ile Lys Gln Glu Ile Asn Met Leu Lys Lys Tyr Ser His His Arg
   65                  70                  75                  80
   Asn Ile Ala Thr Tyr Tyr Gly Ala Phe Ile Lys Lys Asn Pro Pro Gly
                   85                  90                  95
   Met Asp Asp Gln Leu Trp Leu Val Met Glu Phe Cys Gly Ala Gly Ser
               100                 105                 110
   Val Thr Asp Leu Ile Lys Asn Thr Lys Gly Asn Thr Leu Lys Glu Glu
           115                 120                 125
   Trp Ile Ala Tyr Ile Cys Arg Glu Ile Leu Arg Gly Leu Ser His Leu
       130                 135                 140
   His Gln His Lys Val Ile His Arg Asp Ile Lys Gly Gln Asn Val Leu
   145                 150                 155                 160
   Leu Thr Glu Asn Ala Glu Val Lys Leu Val Asp Phe Gly Val Ser Ala
                   165                 170                 175
   Gln Leu Asp Arg Thr Val Gly Arg Arg Asn Thr Phe Ile Gly Thr Pro
               180                 185                 190
   Tyr Trp Met Ala Pro Glu Val Ile Ala Cys Asp Glu Asn Pro Asp Ala
           195                 200                 205
   Thr Tyr Asp Phe Lys Ser Asp Leu Trp Ser Leu Gly Ile Thr Ala Ile
       210                 215                 220
   Glu Met Ala Glu Gly Ala Pro Pro Leu Cys Asp Met His Pro Met Arg
   225                 230                 235                 240
   Ala Leu Phe Leu Ile Pro Arg Asn Pro Ala Pro Arg Leu Lys Ser Lys
                   245                 250                 255
   Lys Trp Ser Lys Lys Phe Gln Ser Phe Ile Glu Ser Cys Leu Val Lys
               260                 265                 270
   Asn His Ser Gln Arg Pro Ala Thr Glu Gln Leu Met Lys His Pro Phe
           275                 280                 285
   Ile Arg Asp Gln Pro Asn Glu Arg Gln Val Arg Ile Gln Leu Lys Asp
       290                 295                 300
   His Ile Asp Arg Thr Lys Lys Lys Arg Gly Glu Lys Asp Glu Thr Glu
   305                 310                 315                 320
   Tyr Glu Tyr Ser Gly Ser Glu Glu Glu Glu Glu Glu Asn Asp Ser Gly
                   325                 330                 335
   Glu Pro Ser Ser Ile Leu Asn Leu Pro Gly Glu Ser Thr Leu Arg Arg
               340                 345                 350
   Asp Phe Leu Arg Leu Gln Leu Ala Asn Lys Glu Arg Ser Glu Ala Leu
           355                 360                 365
   Arg Arg Gln Gln Leu Glu Gln Gln Gln Arg Glu Asn Glu Glu His Lys
       370                 375                 380
   Arg Gln Leu Leu Ala Glu Arg Gln Lys Arg Ile Glu Glu Gln Lys Glu
   385                 390                 395                 400
   Gln Arg Arg Arg Leu Glu Glu Gln Gln Arg Arg Glu Lys Glu Leu Arg
                   405                 410                 415
   Lys Gln Gln Glu Arg Glu Gln Arg Arg His Tyr Glu Glu Gln Met Arg
               420                 425                 430
   Arg Glu Glu Glu Arg Arg Arg Ala Glu His Glu Gln Glu Tyr Ile Arg
           435                 440                 445
   Arg Gln Leu Glu Glu Glu Gln Arg Gln Leu Glu Ile Leu Gln Gln Gln
       450                 455                 460
   Leu Leu His Glu Gln Ala Leu Leu Leu Glu Tyr Lys Arg Lys Gln Leu
   465                 470                 475                 480
   Glu Glu Gln Arg Gln Ala Glu Arg Leu Gln Arg Gln Leu Lys Gln Glu
                   485                 490                 495
   Arg Asp Tyr Leu Val Ser Leu Gln His Gln Arg Gln Glu Gln Arg Pro
               500                 505                 510
   Val Glu Lys Lys Pro Leu Tyr His Tyr Lys Glu Gly Met Ser Pro Ser
           515                 520                 525
   Glu Lys Pro Ala Trp Ala Lys Glu Val Glu Glu Arg Ser Arg Leu Asn
       530                 535                 540
   Arg Gln Ser Ser Pro Ala Met Pro His Lys Val Ala Asn Arg Ile Ser
   545                 550                 555                 560
   Asp Pro Asn Leu Pro Pro Arg Ser Glu Ser Phe Ser Ile Ser Gly Val
                   565                 570                 575
   Gln Pro Ala Arg Thr Pro Pro Met Leu Arg Pro Val Asp Pro Gln Ile
               580                 585                 590
   Pro His Leu Val Ala Val Lys Ser Gln Gly Pro Ala Leu Thr Ala Ser
           595                 600                 605
   Gln Ser Val His Glu Gln Pro Thr Lys Gly Leu Ser Gly Phe Gln Glu
       610                 615                 620
   Ala Leu Asn Val Thr Ser His Arg Val Glu Met Pro Arg Gln Asn Ser
   625                 630                 635                 640
   Asp Pro Thr Ser Glu Asn Pro Pro Leu Pro Thr Arg Ile Glu Lys Phe
                   645                 650                 655
   Asp Arg Ser Ser Trp Leu Arg Gln Glu Glu Asp Ile Pro Pro Lys Val
               660                 665                 670
   Pro Gln Arg Thr Thr Ser Ile Ser Pro Ala Leu Ala Arg Lys Asn Ser
           675                 680                 685
   Pro Gly Asn Gly Ser Ala Leu Gly Pro Arg Leu Gly Ser Gln Pro Ile
       690                 695                 700
   Arg Ala Ser Asn Pro Asp Leu Arg Arg Thr Glu Pro Ile Leu Glu Ser
   705                 710                 715                 720
   Pro Leu Gln Arg Thr Ser Ser Gly Ser Ser Ser Ser Ser Ser Thr Pro
                   725                 730                 735
   Ser Ser Gln Pro Ser Ser Gln Gly Gly Ser Gln Pro Gly Ser Gln Ala
               740                 745                 750
   Gly Ser Ser Glu Arg Thr Arg Val Arg Ala Asn Ser Lys Ser Glu Gly
           755                 760                 765
   Ser Pro Val Leu Pro His Glu Pro Ala Lys Val Lys Pro Glu Glu Ser
       770                 775                 780
   Arg Asp Ile Thr Arg Pro Ser Arg Pro Ala Ser Tyr Lys Lys Ala Ile
   785                 790                 795                 800
   Asp Glu Asp Leu Thr Ala Leu Ala Lys Glu Leu Arg Glu Leu Arg Ile
                   805                 810                 815
   Glu Glu Thr Asn Arg Pro Met Lys Lys Val Thr Asp Tyr Ser Ser Ser
               820                 825                 830
   Ser Glu Glu Ser Glu Ser Ser Glu Glu Glu Glu Glu Asp Gly Glu Ser
           835                 840                 845
   Glu Thr His Asp Gly Thr Val Ala Val Ser Asp Ile Pro Arg Leu Ile
       850                 855                 860
   Pro Thr Gly Ala Pro Gly Ser Asn Glu Gln Tyr Asn Val Gly Met Val
   865                 870                 875                 880
   Gly Thr His Gly Leu Glu Thr Ser His Ala Asp Ser Phe Ser Gly Ser
                   885                 890                 895
   Ile Ser Arg Glu Gly Thr Leu Met Ile Arg Glu Thr Ser Gly Glu Lys
               900                 905                 910
   Lys Arg Ser Gly His Ser Asp Ser Asn Gly Phe Ala Gly His Ile Asn
           915                 920                 925
   Leu Pro Asp Leu Val Gln Gln Ser His Ser Pro Ala Gly Thr Pro Thr
       930                 935                 940
   Glu Gly Leu Gly Arg Val Ser Thr His Ser Gln Glu Met Asp Ser Gly
   945                 950                 955                 960
   Thr Glu Tyr Gly Met Gly Ser Ser Thr Lys Ala Ser Phe Thr Pro Phe
                   965                 970                 975
   Val Asp Pro Arg Val Tyr Gln Thr Ser Pro Thr Asp Glu Asp Glu Glu
               980                 985                 990
   Asp Glu Glu Ser Ser Ala Ala Ala  Leu Phe Thr Ser Glu  Leu Leu Arg
           995                 1000                1005
   Gln Glu  Gln Ala Lys Leu Asn  Glu Ala Arg Lys Ile  Ser Val Val
       1010                1015                1020
   Asn Val  Asn Pro Thr Asn Ile  Arg Pro His Ser Asp  Thr Pro Glu
       1025                1030                1035
   Ile Arg  Lys Tyr Lys Lys Arg  Phe Asn Ser Glu Ile  Leu Cys Ala
       1040                1045                1050
   Ala Leu  Trp Gly Val Asn Leu  Leu Val Gly Thr Glu  Asn Gly Leu
       1055                1060                1065
   Met Leu Leu Asp Arg Ser Gly Gln Gly Lys Val Tyr Asn Leu Ile
   1070 1075 1080
   Asn Arg Arg Arg Phe Gln Gln Met Asp Val Leu Glu Gly Leu Asn
   1085 1090 1095
   Val Leu Val Thr Ile Ser Gly Lys Lys Asn Lys Leu Arg Val Tyr
   1100 1105 1110
   Tyr Leu Ser Trp Leu Arg Asn Arg Ile Leu His Asn Asp Pro Glu
   1115 1120 1125
   Val Glu Lys Lys Gln Gly Trp Ile Thr Val Gly Asp Leu Glu Gly
   1130 1135 1140
   Cys Ile His Tyr Lys Val Val Lys Tyr Glu Arg Ile Lys Phe Leu
   1145 1150 1155
   Val Ile Ala Leu Lys Asn Ala Val Glu Ile Tyr Ala Trp Ala Pro
   1160 1165 1170
   Lys Pro Tyr His Lys Phe Met Ala Phe Lys Ser Phe Ala Asp Leu
   1175 1180 1185
   Gln His Lys Pro Leu Leu Val Asp Leu Thr Val Glu Glu Gly Gln
   1190 1195 1200
   Arg Leu Lys Val Ile Phe Gly Ser His Thr Gly Phe His Val Ile
   1205 1210 1215
   Asp Val Asp Ser Gly Asn Ser Tyr Asp Ile Tyr Ile Pro Ser His
   1220 1225 1230
   Ile Gln Gly Asn Ile Thr Pro His Ala Ile Val Ile Leu Pro Lys
   1235 1240 1245
   Thr Asp Gly Met Glu Met Leu Val Cys Tyr Glu Asp Glu Gly Val
   1250 1255 1260
   Tyr Val Asn Thr Tyr Gly Arg Ile Thr Lys Asp Val Val Leu Gln
   1265 1270 1275
   Trp Gly Glu Met Pro Thr Ser Val Ala Tyr Ile His Ser Asn Gln
   1280 1285 1290
   Ile Met Gly Trp Gly Glu Lys Ala Ile Glu Ile Arg Ser Val Glu
   1295                1300                1305
   Thr Gly His Leu Asp Gly Val Phe Met His Lys Arg Ala Gln Arg
       1310                1315                1320
   Leu Lys Phe Leu Cys Glu Arg Asn Asp Lys Val Ile Gly His Ser
       1325                1330                1335
   Asn Phe Gly Thr Ile Arg Ser Thr Thr Cys Val Tyr Lys Gly Lys
       1340                1345                1350
   Trp Leu Tyr Glu Val Leu Ile Ser Ser Gln Gly Leu Met Gln Ile
       1355                1360                1365
   Gly Trp Cys Thr Ile Ser Cys Arg Phe Asn Gln Glu Glu Gly Val
       1370                1375                1380
   Gly Asp Thr His Asn Ser Tyr Ala Tyr Asp Gly Asn Arg Val Arg
       1385                1390                1395
   Lys Trp Asn Val Thr Thr Thr Asn Tyr Gly Lys Ala Trp Ala Ala
       1400                1405                1410
   Gly Asp Ile Val Ser Cys Leu Ile Asp Leu Asp Asp Gly Thr Leu
       1415                1420                1425
   Ser Phe Cys Leu Asn Gly Val Ser Leu Gly Thr Ala Phe Glu Asn
       1430                1435                1440
   Leu Ser Arg Gly Leu Gly Met Ala Tyr Phe Pro Ala Ile Ser Leu
       1445                1450                1455
   Ser Phe Lys Glu Ser Val Ala Phe Asn Phe Gly Ser Arg Pro Leu
       1460                1465                1470
   Arg Tyr Pro Val Ala Gly Tyr Arg Pro Leu Gln Asp Pro Pro Ser
       1475                1480                1485
   Ala Asp Leu Val Arg Ala Gln Arg Leu Leu Gly Cys Phe Arg Ala
       1490                1495                1500
   Val Leu Ser Val Glu Leu Asp Pro Val Glu Gly Arg Leu Leu Asp
       1505                1510                1515
   Lys Glu Ser Ser Lys Trp Arg Leu Arg Gly Gln Pro Thr Val Leu
       1520                1525                1530
   Leu Thr Leu Ala His Ile Phe His His Phe Ala Pro Leu Leu Arg
   1535 1540 1545
   Lys Val Tyr Leu Val Glu Ala Val Leu Met Ser Phe Leu Leu Gly
   1550 1555 1560
   Ile Val Glu Lys Gly Thr Pro Thr Gln Ala Gln Ser Val Val His
   1565 1570 1575
   Gln Val Leu Asp Leu Leu Trp Leu Phe Met Glu Asp Tyr Glu Val
   1580 1585 1590
   Gln Asp Cys Leu Lys Gln Leu Met Met Ser Leu Leu Arg Leu Tyr
   1595 1600 1605
   Arg Phe Ser Pro Ile Val Pro Asp Leu Gly Leu Gln Ile His Tyr
   1610 1615 1620
   Leu Arg Leu Thr Ile Ala Ile Leu Arg His Glu Lys Ser Arg Lys
   1625 1630 1635
   Phe Leu Leu Ser Asn Val Leu Phe Asp Val Leu Arg Ser Val Val
   1640 1645 1650
   Phe Phe Tyr Ile Lys Ser Pro Leu Arg Val Glu Glu Ala Gly Leu
   1655 1660 1665
   Gln Glu Leu Ile Pro Thr Thr Trp Trp Pro His Cys Ser Ser Arg
   1670 1675 1680
   Glu Gly Lys Glu Ser Thr Glu Met Lys Glu Glu Thr Ala Glu Glu
   1685 1690 1695
   Arg Leu Arg Arg Arg Ala Tyr Glu Arg Gly Cys Gln Arg Leu Arg
   1700 1705 1710
   Lys Arg Ile Glu Val Val Glu Glu Leu Gln Val Gln Ile Leu Lys
   1715 1720 1725
   Leu Leu Leu Asp Asn Lys Asp Asp Asn Gly Gly Glu Ala Ser Arg
   1730 1735 1740
   Tyr Ile Phe Leu Thr Lys Phe Arg Lys Phe Leu Gln Glu Asn Ala
   1745 1750 1755
   Ser Gly Arg Gly Asn Met Pro Met Leu Cys Pro Pro Glu Tyr Met
   1760 1765 1770
   Val Cys Phe Leu His Arg Leu Ile Ser Ala Leu Arg Tyr Tyr Trp
       1775                1780                1785
   Asp Glu Tyr Lys Ala Ser Asn Pro His Ala Ser Phe Ser Glu Glu
       1790                1795                1800
   Ala Tyr Ile Pro Pro Gln Val Phe Tyr Asn Gly Lys Val Asp Tyr
       1805                1810                1815
   Phe Asp Leu Gln Arg Leu Gly Gly Leu Leu Ser His Leu Arg Lys
       1820                1825                1830
   Thr Leu Lys Asp Asp Leu Ala Ser Lys Ala Asn Ile Val Ile Asp
       1835                1840                1845
   Pro Leu Glu Leu Gln Ser Thr Ala Met Asp Asp Leu Asp Glu Asp
       1850                1855                1860
   Glu Glu Pro Ala Pro Ala Met Ala Gln Arg Pro Met Gln Ala Leu
       1865                1870                1875
   Ala Val Gly Gly Pro Leu Pro Leu Pro Arg Pro Gly Trp Leu Ser
       1880                1885                1890
   Ser Pro Thr Leu Gly Arg Ala Asn Arg Phe Leu Ser Thr Ala Ala
       1895                1900                1905
   Val Ser Leu Met Thr Pro Arg Arg Pro Leu Ser Thr Ser Glu Lys
       1910                1915                1920
   Val Lys Val Arg Thr Leu Ser Val Glu Gln Arg Thr Arg Glu Asp
       1925                1930                1935
   Ile Glu Gly Ser His Trp Asn Glu Gly Leu Leu Leu Gly Arg Pro
       1940                1945                1950
   Pro Glu Glu Pro Glu Gln Pro Leu Thr Glu Asn Ser Leu Leu Glu
       1955                1960                1965
   Val Leu Asp Gly Ala Val Met Met Tyr Asn Leu Ser Val His Gln
       1970                1975                1980
   Gln Leu Gly Lys Met Val Gly Val Ser Asp Asp Val Asn Glu Tyr
       1985                1990                1995
   Ala Met Ala Leu Arg Asp Thr Glu Asp Lys Leu Arg Arg Cys Pro
       2000                2005                2010
   Lys Arg Arg Lys Asp Ile Leu Ala Glu Leu Thr Lys Ser Gln Lys
       2015                2020                2025
   Val Phe Ser Glu Lys Leu Asp His Leu Ser Arg Arg Leu Ala Trp
       2030                2035                2040
   Val His Ala Thr Val Tyr Ser Gln Glu Lys Met Leu Asp Ile Tyr
       2045                2050                2055
   Trp Leu Leu Arg Val Cys Leu Arg Thr Ile Glu His Gly Asp Arg
       2060                2065                2070
   Thr Gly Ser Leu Phe Ala Phe Met Pro Glu Phe Tyr Leu Ser Val
       2075                2080                2085
   Ala Ile Asn Ser Tyr Ser Ala Leu Lys Asn Tyr Phe Gly Pro Val
       2090                2095                2100
   His Ser Met Glu Glu Leu Pro Gly Tyr Glu Glu Thr Leu Thr Arg
       2105                2110                2115
   Leu Ala Ala Ile Leu Ala Lys His Phe Ala Asp Ala Arg Ile Val
       2120                2125                2130
   Gly Thr Asp Ile Arg Asp Ser Leu Met Gln Ala Leu Ala Ser Tyr
       2135                2140                2145
   Val Cys Tyr Pro His Ser Leu Arg Ala Val Glu Arg Ile Pro Glu
       2150                2155                2160
   Glu Gln Arg Ile Ala Met Val Arg Asn Leu Leu Ala Pro Tyr Glu
       2165                2170                2175
   Gln Arg Pro Trp Ala Gln Thr Asn Trp Ile Leu Val Arg Leu Trp
       2180                2185                2190
   Arg Gly Cys Gly Phe Gly Tyr Arg Tyr Thr Arg Leu Pro His Leu
       2195                2200                2205
   Leu Lys Thr Lys Leu Glu Asp Ala Asn Leu Pro Ser Leu Gln Lys
       2210                2215                2220
   Pro Cys Pro Ser Thr Leu Leu Gln Gln His Met Ala Asp Leu Leu
       2225                2230                2235
   Gln Gln Gly Pro Asp Val Ala Pro Ser Phe Leu Asn Ser Val Leu
   2240                2245                2250
   Asn Gln Leu Asn Trp Ala Phe Ser Glu Phe Ile Gly Met Ile Gln
       2255                2260                2265
   Glu Ile Gln Gln Ala Ala Glu Arg Leu Glu Arg Asn Phe Val Asp
       2270                2275                2280
   Ser Arg Gln Leu Lys Val Cys Ala Thr Cys Phe Asp Leu Ser Val
       2285                2290                2295
   Ser Leu Leu Arg Val Leu Glu Met Thr Ile Thr Leu Val Pro Glu
       2300                2305                2310
   Ile Phe Leu Asp Trp Thr Arg Pro Thr Ser Glu Met Leu Leu Arg
       2315                2320                2325
   Arg Leu Ala Gln Leu Leu Asn Gln Val Leu Asn Arg Val Thr Ala
       2330                2335                2340
   Glu Arg Asn Leu Phe Asp Arg Val Val Thr Leu Arg Leu Pro Gly
       2345                2350                2355
   Leu Glu Ser Val Asp His Tyr Pro Ile Leu Val Ala Val Thr Gly
       2360                2365                2370
   Ile Leu Val Gln Leu Leu Val Arg Gly Pro Ala Ser Glu Arg Glu
       2375                2380                2385
   Gln Ala Thr Ser Val Leu Leu Ala Asp Pro Cys Phe Gln Leu Arg
       2390                2395                2400
   Ser Ile Cys Tyr Leu Leu Gly Gln Pro Glu Pro Pro Ala Pro Gly
       2405                2410                2415
   Thr Ala Leu Pro Ala Pro Asp Arg Lys Arg Phe Ser Leu Gln Ser
       2420                2425                2430
   Tyr Ala Asp Tyr Ile Ser Ala Asp Glu Leu Ala Gln Val Glu Gln
       2435                2440                2445
   Met Leu Ala His Leu Thr Ser Ala Ser Ala Gln Ala Ala Ala Ala
       2450                2455                2460
   Ser Leu Pro Thr Ser Glu Glu Asp Leu Cys Pro Ile Cys Tyr Ala
       2465                2470                2475
   His Pro Ile Ser Ala Val Phe Gln Pro Cys Gly His Lys Ser Cys
       2480                2485                2490
   Lys Ala Cys Ile Asn Gln His Leu Met Asn Asn Lys Asp Cys Phe
       2495                2500                2505
   Phe Cys Lys Thr Thr Ile Val Ser Val Glu Asp Trp Glu Lys Gly
       2510                2515                2520
   Ala Asn Thr Ser Thr Thr Ser Ser Ala Ala
       2525                2530

<210> 5
<211> 7578
<212> DNA
<213> Homo sapiens

<220>
<221> CDS
<222> (1)..(7578)

<400> 5
atg gcg agc gac tcc ccg gct cga agc ctg gat gaa ata gat ctc tcg 48
Met Ala Ser Asp Ser Pro Ala Arg Ser Leu Asp Glu Ile Asp Leu Ser
1               5                   10                  15

gct ctg agg gac ccc gca ggg atc ttt gaa ttg gtg gaa ctt gtt gga 96
Ala Leu Arg Asp Pro Ala Gly Ile Phe Glu Leu Val Glu Leu Val Gly
            20                  25                  30

aat gga aca tac ggg caa gtt tat aag ggt cgt cat gtc aaa acg ggc 144
Asn Gly Thr Tyr Gly Gln Val Tyr Lys Gly Arg His Val Lys Thr Gly
        35                  40                  45

cag ctt gca gcc atc aag gtt atg gat gtc aca ggg gat gaa gag gaa 192
Gln Leu Ala Ala Ile Lys Val Met Asp Val Thr Gly Asp Glu Glu Glu
    50                  55                  60

gaa atc aaa caa gaa att aac atg ttg aag aaa tat tct cat cac cgg 240
Glu Ile Lys Gln Glu Ile Asn Met Leu Lys Lys Tyr Ser His His Arg
65                  70                  75                  80

aat att gct aca tac tat ggt gct ttt atc aaa aag aac cca cca ggc 288
Asn Ile Ala Thr Tyr Tyr Gly Ala Phe Ile Lys Lys Asn Pro Pro Gly
                85                  90                  95

atg gat gac caa ctt tgg ttg gtg atg gag ttt tgt ggt gct ggc tct 336
Met Asp Asp Gln Leu Trp Leu Val Met Glu Phe Cys Gly Ala Gly Ser
            100                 105                 110

gtc acc gac ctg atc aag aac aca aaa ggt aac acg ttg aaa gag gag 384
Val Thr Asp Leu Ile Lys Asn Thr Lys Gly Asn Thr Leu Lys Glu Glu
        115                 120                 125

tgg att gca tac atc tgc agg gaa atc tta cgg ggg ctg agt cac ctg 432
Trp Ile Ala Tyr Ile Cys Arg Glu Ile Leu Arg Gly Leu Ser His Leu
    130                 135                 140

cac cag cat aaa gtg att cat cga gat att aaa ggg caa aat gtc ttg 480
His Gln His Lys Val Ile His Arg Asp Ile Lys Gly Gln Asn Val Leu
145                 150                 155                 160

ctg act gaa aat gca gaa gtt aaa cta gtg gac ttt gga gtc agt gct 528
Leu Thr Glu Asn Ala Glu Val Lys Leu Val Asp Phe Gly Val Ser Ala
                165                 170                 175

cag ctt gat cga aca gtg ggc agg agg aat act ttc att gga act ccc 576
Gln Leu Asp Arg Thr Val Gly Arg Arg Asn Thr Phe Ile Gly Thr Pro
            180                 185                 190

tac tgg atg gca cca gaa gtt att gcc tgt gat gaa aac cca gat gcc 624
Tyr Trp Met Ala Pro Glu Val Ile Ala Cys Asp Glu Asn Pro Asp Ala
        195                 200                 205

aca tat gat ttc aag agt gac ttg tgg tct ttg ggt atc acc gcc att 672
Thr Tyr Asp Phe Lys Ser Asp Leu Trp Ser Leu Gly Ile Thr Ala Ile
    210                 215                 220

gaa atg gca gaa ggt gct ccc cct ctc tgt gac atg cac ccc atg aga 720
Glu Met Ala Glu Gly Ala Pro Pro Leu Cys Asp Met His Pro Met Arg
225                 230                 235                 240

gct ctc ttc ctc atc ccc cgg aac cca gcg cct cgg ctg aag tct aag 768
Ala Leu Phe Leu Ile Pro Arg Asn Pro Ala Pro Arg Leu Lys Ser Lys
                245                 250                 255

aag tgg tca aaa aaa ttc cag tca ttt att gag agc tgc ttg gta aag 816
Lys Trp Ser Lys Lys Phe Gln Ser Phe Ile Glu Ser Cys Leu Val Lys
            260                 265                 270

aat cac agc cag cga cca gca aca gaa caa ttg atg aag cat cca ttt 864
Asn His Ser Gln Arg Pro Ala Thr Glu Gln Leu Met Lys His Pro Phe
        275                 280                 285

ata cga gac caa cct aat gag cga cag gtc cgc att caa ctc aag gac 912
Ile Arg Asp Gln Pro Asn Glu Arg Gln Val Arg Ile Gln Leu Lys Asp
    290                 295                 300

cat att gat aga aca aag aag aag cga gga gaa aaa gat gag aca gag 960
His Ile Asp Arg Thr Lys Lys Lys Arg Gly Glu Lys Asp Glu Thr Glu
305                 310                 315                 320

tat gag tac agt gga agt gag gaa gaa gag gag gag aat gac tca gga 1008
Tyr Glu Tyr Ser Gly Ser Glu Glu Glu Glu Glu Glu Asn Asp Ser Gly
                325                 330                 335

gag ccc agc tcc atc ctg aat ctg cca ggg gag tcg acg ctg cgg agg 1056
Glu Pro Ser Ser Ile Leu Asn Leu Pro Gly Glu Ser Thr Leu Arg Arg
            340                 345                 350

gac ttt ctg agg ctg cag ctg gcc aac aag gag cgt tct gag gcc cta 1104
Asp Phe Leu Arg Leu Gln Leu Ala Asn Lys Glu Arg Ser Glu Ala Leu
        355                 360                 365

cgg agg cag cag ctg gag cag cag cag cgg gag aat gag gag cac aag 1152
Arg Arg Gln Gln Leu Glu Gln Gln Gln Arg Glu Asn Glu Glu His Lys
    370                 375                 380

cgg cag ctg ctg gcc gag cgt cag aag cgc atc gag gag cag aaa gag 1200
Arg Gln Leu Leu Ala Glu Arg Gln Lys Arg Ile Glu Glu Gln Lys Glu
385                 390                 395                 400

cag agg cgg cgg ctg gag gag caa caa agg cga gag aag gag ctg cgg     1248
Gln Arg Arg Arg Leu Glu Glu Gln Gln Arg Arg Glu Lys Glu Leu Arg
                405                 410                 415

aag cag cag gag agg gag cag cgc cgg cac tat gag gag cag atg cgc     1296
Lys Gln Gln Glu Arg Glu Gln Arg Arg His Tyr Glu Glu Gln Met Arg
            420                 425                 430

cgg gag gag gag agg agg cgt gcg gag cat gaa cag gaa tac atc agg     1344
Arg Glu Glu Glu Arg Arg Arg Ala Glu His Glu Gln Glu Tyr Ile Arg
        435                 440                 445

cga cag tta gag gag gag cag aga cag tta gag atc ttg cag cag cag     1392
Arg Gln Leu Glu Glu Glu Gln Arg Gln Leu Glu Ile Leu Gln Gln Gln
    450                 455                 460

cta ctg cat gaa caa gct cta ctt ctg gaa tat aag cgc aaa caa ttg     1440
Leu Leu His Glu Gln Ala Leu Leu Leu Glu Tyr Lys Arg Lys Gln Leu
465                 470                 475                 480

gaa gaa cag aga caa gca gaa aga ctg cag agg cag cta aag caa gaa     1488
Glu Glu Gln Arg Gln Ala Glu Arg Leu Gln Arg Gln Leu Lys Gln Glu
                485                 490                 495

aga gac tac tta gtt tcc ctt cag cat cag cgg cag gag cag agg cct     1536
Arg Asp Tyr Leu Val Ser Leu Gln His Gln Arg Gln Glu Gln Arg Pro
            500                 505                 510

gtg gag aag aag cca ctg tac cat tac aaa gaa gga atg agt cct agt     1584
Val Glu Lys Lys Pro Leu Tyr His Tyr Lys Glu Gly Met Ser Pro Ser
        515                 520                 525

gag aag cca gca tgg gcc aag gag gta gaa gaa cgg tca agg ctc aac     1632
Glu Lys Pro Ala Trp Ala Lys Glu Val Glu Glu Arg Ser Arg Leu Asn
    530                 535                 540

cgg caa agt tcc cct gcc atg cct cac aag gtt gcc aac agg ata tct     1680
Arg Gln Ser Ser Pro Ala Met Pro His Lys Val Ala Asn Arg Ile Ser
545                 550                 555                 560

gac ccc aac ctg ccc cca agg tcg gag tcc ttc agc att agt gga gtt     1728
Asp Pro Asn Leu Pro Pro Arg Ser Glu Ser Phe Ser Ile Ser Gly Val
                565                 570                 575

cag cct gct cga aca ccc ccc atg ctc aga cca gtc gat ccc cag atc     1776
Gln Pro Ala Arg Thr Pro Pro Met Leu Arg Pro Val Asp Pro Gln Ile
            580                 585                 590

cca cat ctg gta gct gta aaa tcc cag gga cct gcc ttg acc gcc tcc     1824
Pro His Leu Val Ala Val Lys Ser Gln Gly Pro Ala Leu Thr Ala Ser
        595                 600                 605

cag tca gtg cac gag cag ccc aca aag ggc ctc tct ggg ttt cag gag     1872
Gln Ser Val His Glu Gln Pro Thr Lys Gly Leu Ser Gly Phe Gln Glu
    610                 615                 620

gct ctg aac gtg acc tcc cac cgc gtg gag atg cca cgc cag aac tca     1920
Ala Leu Asn Val Thr Ser His Arg Val Glu Met Pro Arg Gln Asn Ser
625                 630                 635                 640

gat ccc acc tcg gaa aat cct cct ctc ccc act cgc att gaa aag ttt     1968
Asp Pro Thr Ser Glu Asn Pro Pro Leu Pro Thr Arg Ile Glu Lys Phe

645                 650                 655

gac cga agc tct tgg tta cga cag gaa gaa gac att cca cca aag gtg        2016
Asp Arg Ser Ser Trp Leu Arg Gln Glu Glu Asp Ile Pro Pro Lys Val
            660                 665                 670

cct caa aga aca act tct ata tcc cca gca tta gcc aga aag aat tct        2064
Pro Gln Arg Thr Thr Ser Ile Ser Pro Ala Leu Ala Arg Lys Asn Ser
        675                 680                 685

cct ggg aat ggt agt gct ctg gga ccc aga cta gga tct caa ccc atc        2112
Pro Gly Asn Gly Ser Ala Leu Gly Pro Arg Leu Gly Ser Gln Pro Ile
    690                 695                 700

aga gca agc aac cct gat ctc cgg aga act gag ccc atc ttg gag agc        2160
Arg Ala Ser Asn Pro Asp Leu Arg Arg Thr Glu Pro Ile Leu Glu Ser
705                 710                 715                 720

ccc ttg cag agg acc agc agt ggc agt tcc tcc agc tcc agc acc cct        2208
Pro Leu Gln Arg Thr Ser Ser Gly Ser Ser Ser Ser Ser Ser Thr Pro
                725                 730                 735

agc tcc cag ccc agc tcc caa gga ggc tcc cag cct gga tca caa gca        2256
Ser Ser Gln Pro Ser Ser Gln Gly Gly Ser Gln Pro Gly Ser Gln Ala
            740                 745                 750

gga tcc agt gaa cgc acc aga gtt cga gcc aac agt aag tca gaa gga        2304
Gly Ser Ser Glu Arg Thr Arg Val Arg Ala Asn Ser Lys Ser Glu Gly
        755                 760                 765

tca cct gtg ctc ccc cat gag cct gcc aag gtg aaa cca gaa gaa tcc        2352
Ser Pro Val Leu Pro His Glu Pro Ala Lys Val Lys Pro Glu Glu Ser
    770                 775                 780

agg gac att acc cgg ccc agt cga cca gct gat ctg acg gca tta gcc        2400
Arg Asp Ile Thr Arg Pro Ser Arg Pro Ala Asp Leu Thr Ala Leu Ala
785                 790                 795                 800

aaa gaa cta aga gaa ctc cgg att gaa gaa aca aac cgc cca atg aag        2448
Lys Glu Leu Arg Glu Leu Arg Ile Glu Glu Thr Asn Arg Pro Met Lys
                805                 810                 815

aag gtg act gat tac tcc tcc tcc agt gag gag tca gaa agt agc gag        2496
Lys Val Thr Asp Tyr Ser Ser Ser Ser Glu Glu Ser Glu Ser Ser Glu
            820                 825                 830

gaa gag gag gaa gat gga gag agc gag acc cat gat ggg aca gtg gct        2544
Glu Glu Glu Glu Asp Gly Glu Ser Glu Thr His Asp Gly Thr Val Ala
        835                 840                 845

gtc agc gac ata ccc aga ctg ata cca aca gga gct cca ggc agc aac        2592
Val Ser Asp Ile Pro Arg Leu Ile Pro Thr Gly Ala Pro Gly Ser Asn
    850                 855                 860

gag cag tac aat gtg gga atg gtg ggg acg cat ggg ctg gag acc tct        2640
Glu Gln Tyr Asn Val Gly Met Val Gly Thr His Gly Leu Glu Thr Ser
865                 870                 875                 880

cat gcg gac agt ttc agc ggc agt att tca aga gaa gga acc ttg atg        2688
His Ala Asp Ser Phe Ser Gly Ser Ile Ser Arg Glu Gly Thr Leu Met
                885                 890                 895

att aga gag acg tct gga gag aag aag cga tct ggc cac agt gac agc        2736

Ile Arg Glu Thr Ser Gly Glu Lys Lys Arg Ser Gly His Ser Asp Ser
            900                 905                 910

aat ggc ttt gct ggc cac atc aac ctc cct gac ctg gtg cag cag agc 2784
Asn Gly Phe Ala Gly His Ile Asn Leu Pro Asp Leu Val Gln Gln Ser
        915                 920                 925

cat tct cca gct gga acc ccg act gag gga ctg ggg cgc gtc tca acc 2832
His Ser Pro Ala Gly Thr Pro Thr Glu Gly Leu Gly Arg Val Ser Thr
    930                 935                 940

cat tcc cag gag atg gac tct ggg act gaa tat ggc atg ggg agc agc 2880
His Ser Gln Glu Met Asp Ser Gly Thr Glu Tyr Gly Met Gly Ser Ser
945                 950                 955                 960

acc aaa gcc tcc ttc acc ccc ttt gtg gac ccc aga gta tac cag acg 2928
Thr Lys Ala Ser Phe Thr Pro Phe Val Asp Pro Arg Val Tyr Gln Thr
                965                 970                 975

tct ccc act gat gaa gat gaa gag gat gag gaa tca tca gcc gca gct 2976
Ser Pro Thr Asp Glu Asp Glu Glu Asp Glu Glu Ser Ser Ala Ala Ala
            980                 985                 990

ctg ttt act agc gaa ctt ctt agg caa gaa cag gcc aaa ctc aat gaa 3024
Leu Phe Thr Ser Glu Leu Leu Arg Gln Glu Gln Ala Lys Leu Asn Glu
        995                 1000                1005

gca aga aag att tcg gtg gta aat gta aac cca acc aac att cgg 3069
Ala Arg Lys Ile Ser Val Val Asn Val Asn Pro Thr Asn Ile Arg
    1010                1015                1020

cct cat agc gac aca cca gaa atc aga aaa tac aag aaa cga ttc 3114
Pro His Ser Asp Thr Pro Glu Ile Arg Lys Tyr Lys Lys Arg Phe
    1025                1030                1035

aac tca gaa ata ctt tgt gca gct ctg tgg ggt gta aac ctt ctg 3159
Asn Ser Glu Ile Leu Cys Ala Ala Leu Trp Gly Val Asn Leu Leu
    1040                1045                1050

gtg ggg act gaa aat ggc ctg atg ctt ttg gac cga agt ggg caa 3204
Val Gly Thr Glu Asn Gly Leu Met Leu Leu Asp Arg Ser Gly Gln
    1055                1060                1065

ggc aaa gtc tat aat ctg atc aac cgg agg cga ttt cag cag atg 3249
Gly Lys Val Tyr Asn Leu Ile Asn Arg Arg Arg Phe Gln Gln Met
    1070                1075                1080

gat gtg cta gag gga ctg aat gtc ctt gtg aca att tca gga aag 3294
Asp Val Leu Glu Gly Leu Asn Val Leu Val Thr Ile Ser Gly Lys
    1085                1090                1095

aag aat aag cta cga gtt tac tat ctt tca tgg tta aga aac aga 3339
Lys Asn Lys Leu Arg Val Tyr Tyr Leu Ser Trp Leu Arg Asn Arg
    1100                1105                1110

ata cta cat aat gac cca gaa gta gaa aag aaa caa ggc tgg atc 3384
Ile Leu His Asn Asp Pro Glu Val Glu Lys Lys Gln Gly Trp Ile
    1115                1120                1125

act gtt ggg gac ttg gaa ggc tgt ata cat tat aaa gtt gtt aaa 3429
Thr Val Gly Asp Leu Glu Gly Cys Ile His Tyr Lys Val Val Lys
    1130                1135                1140

tat gaa agg atc aaa ttt ttg gtg att gcc tta aag aat gct gtg 3474
Tyr Glu Arg Ile Lys Phe Leu Val Ile Ala Leu Lys Asn Ala Val
    1145                1150                1155

gaa ata tat gct tgg gct cct aaa ccg tat cat aaa ttc atg gca 3519
Glu Ile Tyr Ala Trp Ala Pro Lys Pro Tyr His Lys Phe Met Ala
    1160                1165                1170

ttt aag tct ttt gca gat ctc cag cac aag cct ctg cta gtt gat 3564
Phe Lys Ser Phe Ala Asp Leu Gln His Lys Pro Leu Leu Val Asp
    1175                1180                1185

ctc acg gta gaa gaa ggt caa aga tta aag gtt att ttt ggt tca 3609
Leu Thr Val Glu Glu Gly Gln Arg Leu Lys Val Ile Phe Gly Ser
    1190                1195                1200

cac act ggt ttc cat gta att gat gtt gat tca gga aac tct tat 3654
His Thr Gly Phe His Val Ile Asp Val Asp Ser Gly Asn Ser Tyr
    1205                1210                1215

gat atc tac ata cca tct cat att cag ggc aat atc act cct cat 3699
Asp Ile Tyr Ile Pro Ser His Ile Gln Gly Asn Ile Thr Pro His
    1220                1225                1230

gct att gtc atc ttg cct aaa aca gat gga atg gaa atg ctt gtt 3744
Ala Ile Val Ile Leu Pro Lys Thr Asp Gly Met Glu Met Leu Val
    1235                1240                1245

tgc tat gag gat gag ggg gtg tat gta aac acc tat ggc cgg ata 3789
Cys Tyr Glu Asp Glu Gly Val Tyr Val Asn Thr Tyr Gly Arg Ile
    1250                1255                1260

act aag gat gtg gtg ctc caa tgg gga gaa atg ccc acg tct gtg 3834
Thr Lys Asp Val Val Leu Gln Trp Gly Glu Met Pro Thr Ser Val
    1265                1270                1275

gcc tac att cat tcc aat cag ata atg ggc tgg ggc gag aaa gct 3879
Ala Tyr Ile His Ser Asn Gln Ile Met Gly Trp Gly Glu Lys Ala
    1280                1285                1290

att gag atc cgg tca gtg gaa aca gga cat ttg gat gga gta ttt 3924
Ile Glu Ile Arg Ser Val Glu Thr Gly His Leu Asp Gly Val Phe
    1295                1300                1305

atg cat aag cga gct caa agg tta aag ttt cta tgt gaa aga aat 3969
Met His Lys Arg Ala Gln Arg Leu Lys Phe Leu Cys Glu Arg Asn
    1310                1315                1320

gat aag gtg att gga cac agc aac ttt ggc acc atc cgc tct acc 4014
Asp Lys Val Ile Gly His Ser Asn Phe Gly Thr Ile Arg Ser Thr
    1325                1330                1335

aca tgc gtg tac aaa ggg aaa tgg ctc tac gag gtc ctc atc tcc 4059
Thr Cys Val Tyr Lys Gly Lys Trp Leu Tyr Glu Val Leu Ile Ser
    1340                1345                1350

tcc cag ggg ctc atg cag atc ggc tgg tgc acc atc agc tgc cgc 4104
Ser Gln Gly Leu Met Gln Ile Gly Trp Cys Thr Ile Ser Cys Arg
    1355                1360                1365

ttc aac cag gag gag ggg gtt gga gat aca cac aac tcc tat gcc 4149
Phe Asn Gln Glu Glu Gly Val Gly Asp Thr His Asn Ser Tyr Ala
    1370                1375                1380

tat gat ggc aac cgc gtg cgc aag tgg aat gtg acc aca acg aat 4194
Tyr Asp Gly Asn Arg Val Arg Lys Trp Asn Val Thr Thr Thr Asn
    1385                1390                1395

tat ggc aag gcg tgg gca gcg ggg gac atc gtg agc tgc ctg att 4239
Tyr Gly Lys Ala Trp Ala Ala Gly Asp Ile Val Ser Cys Leu Ile
    1400                1405                1410

gac ctg gat gat ggc act ctg tcc ttc tgc ctg aac ggt gta tca 4284
Asp Leu Asp Asp Gly Thr Leu Ser Phe Cys Leu Asn Gly Val Ser
    1415                1420                1425

ctg ggc act gcc ttt gag aac ctg tcc agg ggc ctg ggt atg gcc 4329
Leu Gly Thr Ala Phe Glu Asn Leu Ser Arg Gly Leu Gly Met Ala
    1430                1435                1440

tac ttc cca gcc atc agc ctc tct ttc aag gag tcc gtg gcc ttc 4374
Tyr Phe Pro Ala Ile Ser Leu Ser Phe Lys Glu Ser Val Ala Phe
    1445                1450                1455

aac ttt ggc agc cgt cct ctg cgc tac cca gtg gca ggc tac cgg 4419
Asn Phe Gly Ser Arg Pro Leu Arg Tyr Pro Val Ala Gly Tyr Arg
    1460                1465                1470

ccc ctg cag gac cca ccg agt gct gac ctg gtg cgg gca cag agg 4464
Pro Leu Gln Asp Pro Pro Ser Ala Asp Leu Val Arg Ala Gln Arg
    1475                1480                1485

ttg ctg ggc tgc ttc cgg gca gtg ctg agt gtg gag ctg gac cct 4509
Leu Leu Gly Cys Phe Arg Ala Val Leu Ser Val Glu Leu Asp Pro
    1490                1495                1500

gtg gag ggg cgg ctg ttg gac aag gag agc tcc aag tgg cgg ttg 4554
Val Glu Gly Arg Leu Leu Asp Lys Glu Ser Ser Lys Trp Arg Leu
    1505                1510                1515

cgg ggc cag ccc acc gtc ctc ctc aca ctg gcc cac atc ttc cat 4599
Arg Gly Gln Pro Thr Val Leu Leu Thr Leu Ala His Ile Phe His
    1520                1525                1530

cac ttt gca ccg ctt ctg cgc aag gtg tat ctg gtg gag gct gtg 4644
His Phe Ala Pro Leu Leu Arg Lys Val Tyr Leu Val Glu Ala Val
    1535                1540                1545

ctc atg agc ttc ttg ctg ggc atc gtg gag aag ggc aca ccc aca 4689
Leu Met Ser Phe Leu Leu Gly Ile Val Glu Lys Gly Thr Pro Thr
    1550                1555                1560

cag gca cag tcc gtg gtg cac cag gtc ctg gac ctc ttg tgg ctc 4734
Gln Ala Gln Ser Val Val His Gln Val Leu Asp Leu Leu Trp Leu
    1565                1570                1575

ttc atg gag gac tac gag gta caa gat tgc ctc aag cag ttg atg 4779
Phe Met Glu Asp Tyr Glu Val Gln Asp Cys Leu Lys Gln Leu Met
    1580                1585                1590

atg tct ctg ctt cgg ctg tac cga ttc tca ccc att gtc cca gac 4824
Met Ser Leu Leu Arg Leu Tyr Arg Phe Ser Pro Ile Val Pro Asp
    1595                1600                1605

ctg ggc cta cag atc cat tac ctg cgg ctc act atc gcc atc ctg 4869
Leu Gly Leu Gln Ile His Tyr Leu Arg Leu Thr Ile Ala Ile Leu

1610                    1615                    1620

agg cat gag aag tcc cgc aag ttt ctg ctt agc aat gtc ctc ttc        4914
Arg His Glu Lys Ser Arg Lys Phe Leu Leu Ser Asn Val Leu Phe
    1625                    1630                    1635

gac gtg ctc cgc tcc gtc gtc ttc ttt tac atc aag agc ccc ctg        4959
Asp Val Leu Arg Ser Val Val Phe Phe Tyr Ile Lys Ser Pro Leu
    1640                    1645                    1650

cgt gtg gag gag gcc ggc ctg cag gag ctc att ccc acc acc tgg        5004
Arg Val Glu Glu Ala Gly Leu Gln Glu Leu Ile Pro Thr Thr Trp
    1655                    1660                    1665

tgg ccc cac tgc tcc agt agg gag ggc aaa gag agc acg gag atg        5049
Trp Pro His Cys Ser Ser Arg Glu Gly Lys Glu Ser Thr Glu Met
    1670                    1675                    1680

aag gag gag acc gca gag gag cgg ctg cgg cgg cga gcc tac gaa        5094
Lys Glu Glu Thr Ala Glu Glu Arg Leu Arg Arg Arg Ala Tyr Glu
    1685                    1690                    1695

cgg ggc tgt cag cgg ctc agg aag cgc atc gaa gtg gtg gaa gaa        5139
Arg Gly Cys Gln Arg Leu Arg Lys Arg Ile Glu Val Val Glu Glu
    1700                    1705                    1710

cta cag gtc cag atc ctg aag ctg ctg ctg gac aat aaa gat gac        5184
Leu Gln Val Gln Ile Leu Lys Leu Leu Leu Asp Asn Lys Asp Asp
    1715                    1720                    1725

aat ggg ggt gaa gct tct agg tat atc ttc ctg acc aag ttt cgc        5229
Asn Gly Gly Glu Ala Ser Arg Tyr Ile Phe Leu Thr Lys Phe Arg
    1730                    1735                    1740

aag ttt ctg cag gag aac gcc agt ggc cgg ggg aac atg ccc atg        5274
Lys Phe Leu Gln Glu Asn Ala Ser Gly Arg Gly Asn Met Pro Met
    1745                    1750                    1755

ctc tgc ccc cct gag tac atg gtc tgc ttc tta cac cgg ctg atc        5319
Leu Cys Pro Pro Glu Tyr Met Val Cys Phe Leu His Arg Leu Ile
    1760                    1765                    1770

tct gcc ctg cgc tac tat tgg gat gaa tac aag gct tcc aat cct        5364
Ser Ala Leu Arg Tyr Tyr Trp Asp Glu Tyr Lys Ala Ser Asn Pro
    1775                    1780                    1785

cat gct tcc ttc agt gag gag gcc tac atc ccg ccc cag gtc ttc        5409
His Ala Ser Phe Ser Glu Glu Ala Tyr Ile Pro Pro Gln Val Phe
    1790                    1795                    1800

tat aat ggc aag gtg gac tac ttt gac ctg cag cgc ctg ggg ggc        5454
Tyr Asn Gly Lys Val Asp Tyr Phe Asp Leu Gln Arg Leu Gly Gly
    1805                    1810                    1815

ctc ctc tcg cac ctg cgg aag acc ctc aaa gat gac ctt gct tcc        5499
Leu Leu Ser His Leu Arg Lys Thr Leu Lys Asp Asp Leu Ala Ser
    1820                    1825                    1830

aaa gcc aac att gtg atc gac cca ctg gag ctc cag tca acc gcc        5544
Lys Ala Asn Ile Val Ile Asp Pro Leu Glu Leu Gln Ser Thr Ala
    1835                    1840                    1845

atg gat gac cta gat gag gat gag gag cca gcc cca gct atg gcc        5589

Met Asp Asp Leu Asp Glu Asp Glu Glu Pro Ala Pro Ala Met Ala
    1850                1855                1860

cag cgc ccc atg cag gcc ctg gct gtt ggg ggg cca ctg ccc ctg 5634
Gln Arg Pro Met Gln Ala Leu Ala Val Gly Gly Pro Leu Pro Leu
    1865                1870                1875

ccc cgg ccc ggc tgg ctc agt tct cca act ttg ggc cga gcc aac 5679
Pro Arg Pro Gly Trp Leu Ser Ser Pro Thr Leu Gly Arg Ala Asn
    1880                1885                1890

cgc ttc ctc agc aca gcg gct gtg agc ctc atg acc cca cgg cgg 5724
Arg Phe Leu Ser Thr Ala Ala Val Ser Leu Met Thr Pro Arg Arg
    1895                1900                1905

cct ctg agc acc tcg gag aaa gtg aag gtc cgc acg ctg agc gtg 5769
Pro Leu Ser Thr Ser Glu Lys Val Lys Val Arg Thr Leu Ser Val
    1910                1915                1920

gag cag agg acc cgt gag gac att gaa ggc agc cac tgg aat gag 5814
Glu Gln Arg Thr Arg Glu Asp Ile Glu Gly Ser His Trp Asn Glu
    1925                1930                1935

ggc ttg ctg ctg ggg cgg ccc ccc gag gag cct gag cag ccc ctc 5859
Gly Leu Leu Leu Gly Arg Pro Pro Glu Glu Pro Glu Gln Pro Leu
    1940                1945                1950

acc gag aac tcg ctg ctg gaa gtc ctg gat ggg gcg gtc atg atg 5904
Thr Glu Asn Ser Leu Leu Glu Val Leu Asp Gly Ala Val Met Met
    1955                1960                1965

tac aac ctc agc gta cac cag cag ctg ggc aag atg gtg ggt gtc 5949
Tyr Asn Leu Ser Val His Gln Gln Leu Gly Lys Met Val Gly Val
    1970                1975                1980

tcc gat gat gtc aat gaa tac gct atg gct ctg agg gac aca gag 5994
Ser Asp Asp Val Asn Glu Tyr Ala Met Ala Leu Arg Asp Thr Glu
    1985                1990                1995

gac aag ctc cgc cgg tgc ccc aag agg agg aag gac atc ctt gca 6039
Asp Lys Leu Arg Arg Cys Pro Lys Arg Arg Lys Asp Ile Leu Ala
    2000                2005                2010

gag ttg acc aag agc cag aag gtt ttc tca gaa aag ctg gac cac 6084
Glu Leu Thr Lys Ser Gln Lys Val Phe Ser Glu Lys Leu Asp His
    2015                2020                2025

ctg agc cgc cgt ctt gcc tgg gtc cat gcc act gtc tac tcc cag 6129
Leu Ser Arg Arg Leu Ala Trp Val His Ala Thr Val Tyr Ser Gln
    2030                2035                2040

gag aag atg ctg gac atc tac tgg ctg ctg cgc gtc tgc ctg cgg 6174
Glu Lys Met Leu Asp Ile Tyr Trp Leu Leu Arg Val Cys Leu Arg
    2045                2050                2055

acc att gag cac ggt gat cgc aca ggg tct ctc ttt gcc ttc atg 6219
Thr Ile Glu His Gly Asp Arg Thr Gly Ser Leu Phe Ala Phe Met
    2060                2065                2070

ccc gag ttc tac ctg agc gtg gcc atc aac agc tac agt gct ctc 6264
Pro Glu Phe Tyr Leu Ser Val Ala Ile Asn Ser Tyr Ser Ala Leu
    2075                2080                2085

aag aat tac ttt ggt ccc gtg cac agc atg gag gag ctc cca ggc 6309
Lys Asn Tyr Phe Gly Pro Val His Ser Met Glu Glu Leu Pro Gly
    2090                2095                2100

tat gaa gag acc ctg acc cgc ctg gct gcc att ctc gcc aaa cac 6354
Tyr Glu Glu Thr Leu Thr Arg Leu Ala Ala Ile Leu Ala Lys His
    2105                2110                2115

ttt gcc gac gca cgc att gtg ggc act gac atc cga gac tca ctg 6399
Phe Ala Asp Ala Arg Ile Val Gly Thr Asp Ile Arg Asp Ser Leu
    2120                2125                2130

atg cag gcc ctg gcc agc tac gtg tgc tac cca cac tcc ctg cgg 6444
Met Gln Ala Leu Ala Ser Tyr Val Cys Tyr Pro His Ser Leu Arg
    2135                2140                2145

gct gtg gag cga atc ccc gag gag cag cgt atc gcc atg gtg agg 6489
Ala Val Glu Arg Ile Pro Glu Glu Gln Arg Ile Ala Met Val Arg
    2150                2155                2160

aac ctc ctg gcg ccc tat gag cag cgg ccc tgg gcc cag acc aac 6534
Asn Leu Leu Ala Pro Tyr Glu Gln Arg Pro Trp Ala Gln Thr Asn
    2165                2170                2175

tgg atc ctg gtg cgg ctc tgg agg ggc tgt ggc ttc ggg tac cgc 6579
Trp Ile Leu Val Arg Leu Trp Arg Gly Cys Gly Phe Gly Tyr Arg
    2180                2185                2190

tat aca cgg ctg cca cat ctg ctg aaa acc aaa ctt gag gac gcc 6624
Tyr Thr Arg Leu Pro His Leu Leu Lys Thr Lys Leu Glu Asp Ala
    2195                2200                2205

aat ttg ccc agc ctc cag aag ccc tgc cct tcc acc ctg ctg cag 6669
Asn Leu Pro Ser Leu Gln Lys Pro Cys Pro Ser Thr Leu Leu Gln
    2210                2215                2220

cag cac atg gcg gac ctc cta cag cag ggt cct gat gtg gca ccc 6714
Gln His Met Ala Asp Leu Leu Gln Gln Gly Pro Asp Val Ala Pro
    2225                2230                2235

agc ttc ctc aac agc gtc ctc aat cag ctc aac tgg gcc ttc tct 6759
Ser Phe Leu Asn Ser Val Leu Asn Gln Leu Asn Trp Ala Phe Ser
    2240                2245                2250

gaa ttc att ggc atg atc caa gag atc cag cag gct gct gag cgc 6804
Glu Phe Ile Gly Met Ile Gln Glu Ile Gln Gln Ala Ala Glu Arg
    2255                2260                2265

ctg gag cgg aac ttt gtg gac agc cgg cag ctc aag gta tgt gcc 6849
Leu Glu Arg Asn Phe Val Asp Ser Arg Gln Leu Lys Val Cys Ala
    2270                2275                2280

acc tgc ttt gac ctc tcg gtc agc ctg ctg cgt gtc ttg gag atg 6894
Thr Cys Phe Asp Leu Ser Val Ser Leu Leu Arg Val Leu Glu Met
    2285                2290                2295

act atc aca ctg gtg cct gag ata ttc ctt gac tgg acc cgg cct 6939
Thr Ile Thr Leu Val Pro Glu Ile Phe Leu Asp Trp Thr Arg Pro
    2300                2305                2310

acc tct gag atg ctg ctg cgg cgt ctt gca cag ctg cta aac cag 6984
Thr Ser Glu Met Leu Leu Arg Arg Leu Ala Gln Leu Leu Asn Gln
    2315                2320                2325

gtg ctg aac cgg gtg aca gct gag agg aac ctg ttt gat cgt gtg 7029
Val Leu Asn Arg Val Thr Ala Glu Arg Asn Leu Phe Asp Arg Val
    2330                2335                2340

gtc acc cta cgg ctg cct ggc cta gag agc gtg gac cac tat ccc 7074
Val Thr Leu Arg Leu Pro Gly Leu Glu Ser Val Asp His Tyr Pro
    2345                2350                2355

att ctg gtg gca gtg acg ggc atc ctg gtg cag ctc ctg gtg cgt 7119
Ile Leu Val Ala Val Thr Gly Ile Leu Val Gln Leu Leu Val Arg
    2360                2365                2370

ggc cca gcc tca gag aga gag caa gcc aca tca gtg ctc ctg gca 7164
Gly Pro Ala Ser Glu Arg Glu Gln Ala Thr Ser Val Leu Leu Ala
    2375                2380                2385

gat ccc tgc ttc cag cta cgc tca ata tgc tat ctc ctg gga cag 7209
Asp Pro Cys Phe Gln Leu Arg Ser Ile Cys Tyr Leu Leu Gly Gln
    2390                2395                2400

cca gag ccc cca gca cct ggc act gct ctg cca gcc cct gac cgg 7254
Pro Glu Pro Pro Ala Pro Gly Thr Ala Leu Pro Ala Pro Asp Arg
    2405                2410                2415

aag cgc ttc tcc ctg cag agc tat gcg gat tat atc agt gcc gat 7299
Lys Arg Phe Ser Leu Gln Ser Tyr Ala Asp Tyr Ile Ser Ala Asp
    2420                2425                2430

gag ctg gcc caa gtg gaa cag atg ctg gcg cac ctg acc tct gca 7344
Glu Leu Ala Gln Val Glu Gln Met Leu Ala His Leu Thr Ser Ala
    2435                2440                2445

tct gcc cag gca gca gct gcc tcc ctg ccc acc agt gag gag gac 7389
Ser Ala Gln Ala Ala Ala Ala Ser Leu Pro Thr Ser Glu Glu Asp
    2450                2455                2460

ctc tgc ccc atc tgc tat gcc cac ccc atc tct gct gtg ttc cag 7434
Leu Cys Pro Ile Cys Tyr Ala His Pro Ile Ser Ala Val Phe Gln
    2465                2470                2475

ccc tgt ggc cac aag tcc tgc aaa gcc tgt atc aac cag cac ctg 7479
Pro Cys Gly His Lys Ser Cys Lys Ala Cys Ile Asn Gln His Leu
    2480                2485                2490

atg aac aac aag gac tgc ttc ttc tgc aaa acc acc atc gtg tct 7524
Met Asn Asn Lys Asp Cys Phe Phe Cys Lys Thr Thr Ile Val Ser
    2495                2500                2505

gta gag gac tgg gag aag gga gcc aat acg agt act acc tcc tca 7569
Val Glu Asp Trp Glu Lys Gly Ala Asn Thr Ser Thr Thr Ser Ser
    2510                2515                2520

gct gcc tag 7578
Ala Ala
    2525

<210> 6
<211> 2525
<212> PRT
<213> Homo sapiens

<400> 6

Met Ala Ser Asp Ser Pro Ala Arg Ser Leu Asp Glu Ile Asp Leu Ser
1               5                   10                  15

Ala Leu Arg Asp Pro Ala Gly Ile Phe Glu Leu Val Glu Leu Val Gly
            20                  25                  30

Asn Gly Thr Tyr Gly Gln Val Tyr Lys Gly Arg His Val Lys Thr Gly
        35                  40                  45

Gln Leu Ala Ala Ile Lys Val Met Asp Val Thr Gly Asp Glu Glu Glu
    50                  55                  60

Glu Ile Lys Gln Glu Ile Asn Met Leu Lys Lys Tyr Ser His His Arg
65                  70                  75                  80

Asn Ile Ala Thr Tyr Tyr Gly Ala Phe Ile Lys Lys Asn Pro Pro Gly
                85                  90                  95

Met Asp Asp Gln Leu Trp Leu Val Met Glu Phe Cys Gly Ala Gly Ser
            100                 105                 110

Val Thr Asp Leu Ile Lys Asn Thr Lys Gly Asn Thr Leu Lys Glu Glu
        115                 120                 125

Trp Ile Ala Tyr Ile Cys Arg Glu Ile Leu Arg Gly Leu Ser His Leu
    130                 135                 140

His Gln His Lys Val Ile His Arg Asp Ile Lys Gly Gln Asn Val Leu
145                 150                 155                 160

Leu Thr Glu Asn Ala Glu Val Lys Leu Val Asp Phe Gly Val Ser Ala
                165                 170                 175

Gln Leu Asp Arg Thr Val Gly Arg Arg Asn Thr Phe Ile Gly Thr Pro
            180                 185                 190

Tyr Trp Met Ala Pro Glu Val Ile Ala Cys Asp Glu Asn Pro Asp Ala
        195                 200                 205

Thr Tyr Asp Phe Lys Ser Asp Leu Trp Ser Leu Gly Ile Thr Ala Ile
    210                 215                 220

Glu Met Ala Glu Gly Ala Pro Pro Leu Cys Asp Met His Pro Met Arg
225                 230                 235                 240

Ala Leu Phe Leu Ile Pro Arg Asn Pro Ala Pro Arg Leu Lys Ser Lys

245                 250                 255

Lys Trp Ser Lys Lys Phe Gln Ser Phe Ile Glu Ser Cys Leu Val Lys
            260                 265                 270

Asn His Ser Gln Arg Pro Ala Thr Glu Gln Leu Met Lys His Pro Phe
        275                 280                 285

Ile Arg Asp Gln Pro Asn Glu Arg Gln Val Arg Ile Gln Leu Lys Asp
    290                 295                 300

His Ile Asp Arg Thr Lys Lys Lys Arg Gly Glu Lys Asp Glu Thr Glu
305                 310                 315                 320

Tyr Glu Tyr Ser Gly Ser Glu Glu Glu Glu Glu Glu Asn Asp Ser Gly
                325                 330                 335

Glu Pro Ser Ser Ile Leu Asn Leu Pro Gly Glu Ser Thr Leu Arg Arg
            340                 345                 350

Asp Phe Leu Arg Leu Gln Leu Ala Asn Lys Glu Arg Ser Glu Ala Leu
        355                 360                 365

Arg Arg Gln Gln Leu Glu Gln Gln Gln Arg Glu Asn Glu Glu His Lys
    370                 375                 380

Arg Gln Leu Leu Ala Glu Arg Gln Lys Arg Ile Glu Glu Gln Lys Glu
385                 390                 395                 400

Gln Arg Arg Arg Leu Glu Glu Gln Gln Arg Arg Glu Lys Glu Leu Arg
                405                 410                 415

Lys Gln Gln Glu Arg Glu Gln Arg Arg His Tyr Glu Glu Gln Met Arg
            420                 425                 430

Arg Glu Glu Glu Arg Arg Arg Ala Glu His Glu Gln Glu Tyr Ile Arg
        435                 440                 445

Arg Gln Leu Glu Glu Glu Gln Arg Gln Leu Glu Ile Leu Gln Gln Gln
    450                 455                 460

Leu Leu His Glu Gln Ala Leu Leu Leu Glu Tyr Lys Arg Lys Gln Leu
465                 470                 475                 480

Glu Glu Gln Arg Gln Ala Glu Arg Leu Gln Arg Gln Leu Lys Gln Glu
                485                 490                 495

Arg Asp Tyr Leu Val Ser Leu Gln His Gln Arg Gln Glu Gln Arg Pro
            500                 505                 510

Val Glu Lys Lys Pro Leu Tyr His Tyr Lys Glu Gly Met Ser Pro Ser
        515                 520                 525

Glu Lys Pro Ala Trp Ala Lys Glu Val Glu Glu Arg Ser Arg Leu Asn
    530                 535                 540

Arg Gln Ser Ser Pro Ala Met Pro His Lys Val Ala Asn Arg Ile Ser
545                 550                 555                 560

Asp Pro Asn Leu Pro Pro Arg Ser Glu Ser Phe Ser Ile Ser Gly Val
                565                 570                 575

Gln Pro Ala Arg Thr Pro Pro Met Leu Arg Pro Val Asp Pro Gln Ile
            580                 585                 590

Pro His Leu Val Ala Val Lys Ser Gln Gly Pro Ala Leu Thr Ala Ser
        595                 600                 605

Gln Ser Val His Glu Gln Pro Thr Lys Gly Leu Ser Gly Phe Gln Glu
    610                 615                 620

Ala Leu Asn Val Thr Ser His Arg Val Glu Met Pro Arg Gln Asn Ser
625                 630                 635                 640

Asp Pro Thr Ser Glu Asn Pro Pro Leu Pro Thr Arg Ile Glu Lys Phe
                645                 650                 655

Asp Arg Ser Ser Trp Leu Arg Gln Glu Glu Asp Ile Pro Pro Lys Val
            660                 665                 670

Pro Gln Arg Thr Thr Ser Ile Ser Pro Ala Leu Ala Arg Lys Asn Ser
        675                 680                 685

Pro Gly Asn Gly Ser Ala Leu Gly Pro Arg Leu Gly Ser Gln Pro Ile
    690                 695                 700

Arg Ala Ser Asn Pro Asp Leu Arg Arg Thr Glu Pro Ile Leu Glu Ser
705                 710                 715                 720

Pro Leu Gln Arg Thr Ser Ser Gly Ser Ser Ser Ser Ser Thr Pro
                725                 730                 735

Ser Ser Gln Pro Ser Ser Gln Gly Gly Ser Gln Pro Gly Ser Gln Ala
            740                 745                 750

Gly Ser Ser Glu Arg Thr Arg Val Arg Ala Asn Ser Lys Ser Glu Gly
            755                 760                 765

Ser Pro Val Leu Pro His Glu Pro Ala Lys Val Lys Pro Glu Glu Ser
        770                 775                 780

Arg Asp Ile Thr Arg Pro Ser Arg Pro Ala Asp Leu Thr Ala Leu Ala
785 790 795 800

Lys Glu Leu Arg Glu Leu Arg Ile Glu Glu Thr Asn Arg Pro Met Lys
805 810 815

Lys Val Thr Asp Tyr Ser Ser Ser Ser Glu Glu Ser Glu Ser Ser Glu
                820                 825                 830

Glu Glu Glu Glu Asp Gly Glu Ser Glu Thr His Asp Gly Thr Val Ala
            835                 840                 845

Val Ser Asp Ile Pro Arg Leu Ile Pro Thr Gly Ala Pro Gly Ser Asn
        850                 855                 860

Glu Gln Tyr Asn Val Gly Met Val Gly Thr His Gly Leu Glu Thr Ser
865 870 875 880

His Ala Asp Ser Phe Ser Gly Ser Ile Ser Arg Glu Gly Thr Leu Met
885 890 895

Ile Arg Glu Thr Ser Gly Glu Lys Lys Arg Ser Gly His Ser Asp Ser
                900                 905                 910

Asn Gly Phe Ala Gly His Ile Asn Leu Pro Asp Leu Val Gln Gln Ser
            915                 920                 925

His Ser Pro Ala Gly Thr Pro Thr Glu Gly Leu Gly Arg Val Ser Thr
        930                 935                 940

His Ser Gln Glu Met Asp Ser Gly Thr Glu Tyr Gly Met Gly Ser Ser
945 950 955 960

Thr Lys Ala Ser Phe Thr Pro Phe Val Asp Pro Arg Val Tyr Gln Thr
965 970 975

Ser Pro Thr Asp Glu Asp Glu Glu Asp Glu Glu Ser Ser Ala Ala Ala
                980                 985                 990

Leu Phe Thr Ser Glu Leu Leu Arg Gln Glu Gln Ala Lys Leu Asn Glu
            995                 1000                1005

Ala Arg Lys Ile Ser Val Val Asn Val Asn Pro Thr Asn Ile Arg
    1010                1015                1020

Pro His Ser Asp Thr Pro Glu Ile Arg Lys Tyr Lys Lys Arg Phe
    1025                1030                1035

Asn Ser Glu Ile Leu Cys Ala Ala Leu Trp Gly Val Asn Leu Leu
    1040                1045                1050

Val Gly Thr Glu Asn Gly Leu Met Leu Leu Asp Arg Ser Gly Gln
    1055                1060                1065

Gly Lys Val Tyr Asn Leu Ile Asn Arg Arg Arg Phe Gln Gln Met
    1070                1075                1080

Asp Val Leu Glu Gly Leu Asn Val Leu Val Thr Ile Ser Gly Lys
    1085                1090                1095

Lys Asn Lys Leu Arg Val Tyr Tyr Leu Ser Trp Leu Arg Asn Arg
    1100                1105                1110

Ile Leu His Asn Asp Pro Glu Val Glu Lys Lys Gln Gly Trp Ile
    1115                1120                1125

Thr Val Gly Asp Leu Glu Gly Cys Ile His Tyr Lys Val Val Lys
    1130                1135                1140

Tyr Glu Arg Ile Lys Phe Leu Val Ile Ala Leu Lys Asn Ala Val
    1145                1150                1155

Glu Ile Tyr Ala Trp Ala Pro Lys Pro Tyr His Lys Phe Met Ala
    1160                1165                1170

Phe Lys Ser Phe Ala Asp Leu Gln His Lys Pro Leu Leu Val Asp
    1175                1180                1185

Leu Thr Val Glu Glu Gly Gln Arg Leu Lys Val Ile Phe Gly Ser
    1190                1195                1200

His Thr Gly Phe His Val Ile Asp Val Asp Ser Gly Asn Ser Tyr
    1205                1210                1215

Asp Ile Tyr Ile Pro Ser His Ile Gln Gly Asn Ile Thr Pro His
    1220                1225                1230

Ala Ile Val Ile Leu Pro Lys Thr Asp Gly Met Glu Met Leu Val

1235                1240                1245

Cys Tyr Glu Asp Glu Gly Val Tyr Val Asn Thr Tyr Gly Arg Ile
    1250                1255                1260

Thr Lys Asp Val Val Leu Gln Trp Gly Glu Met Pro Thr Ser Val
    1265                1270                1275

Ala Tyr Ile His Ser Asn Gln Ile Met Gly Trp Gly Glu Lys Ala
    1280                1285                1290

Ile Glu Ile Arg Ser Val Glu Thr Gly His Leu Asp Gly Val Phe
    1295                1300                1305

Met His Lys Arg Ala Gln Arg Leu Lys Phe Leu Cys Glu Arg Asn
    1310                1315                1320

Asp Lys Val Ile Gly His Ser Asn Phe Gly Thr Ile Arg Ser Thr
    1325                1330                1335

Thr Cys Val Tyr Lys Gly Lys Trp Leu Tyr Glu Val Leu Ile Ser
    1340                1345                1350

Ser Gln Gly Leu Met Gln Ile Gly Trp Cys Thr Ile Ser Cys Arg
    1355                1360                1365

Phe Asn Gln Glu Glu Gly Val Gly Asp Thr His Asn Ser Tyr Ala
    1370                1375                1380

Tyr Asp Gly Asn Arg Val Arg Lys Trp Asn Val Thr Thr Thr Asn
    1385                1390                1395

Tyr Gly Lys Ala Trp Ala Ala Gly Asp Ile Val Ser Cys Leu Ile
    1400                1405                1410

Asp Leu Asp Asp Gly Thr Leu Ser Phe Cys Leu Asn Gly Val Ser
    1415                1420                1425

Leu Gly Thr Ala Phe Glu Asn Leu Ser Arg Gly Leu Gly Met Ala
    1430                1435                1440

Tyr Phe Pro Ala Ile Ser Leu Ser Phe Lys Glu Ser Val Ala Phe
    1445                1450                1455

Asn Phe Gly Ser Arg Pro Leu Arg Tyr Pro Val Ala Gly Tyr Arg
    1460                1465                1470

Pro Leu Gln Asp Pro Pro Ser Ala Asp Leu Val Arg Ala Gln Arg
        1475                1480                1485

Leu Leu Gly Cys Phe Arg Ala Val Leu Ser Val Glu Leu Asp Pro
        1490                1495                1500

Val Glu Gly Arg Leu Leu Asp Lys Glu Ser Ser Lys Trp Arg Leu
        1505                1510                1515

Arg Gly Gln Pro Thr Val Leu Leu Thr Leu Ala His Ile Phe His
        1520                1525                1530

His Phe Ala Pro Leu Leu Arg Lys Val Tyr Leu Val Glu Ala Val
        1535                1540                1545

Leu Met Ser Phe Leu Leu Gly Ile Val Glu Lys Gly Thr Pro Thr
        1550                1555                1560

Gln Ala Gln Ser Val Val His Gln Val Leu Asp Leu Leu Trp Leu
        1565                1570                1575

Phe Met Glu Asp Tyr Glu Val Gln Asp Cys Leu Lys Gln Leu Met
        1580                1585                1590

Met Ser Leu Leu Arg Leu Tyr Arg Phe Ser Pro Ile Val Pro Asp
        1595                1600                1605

Leu Gly Leu Gln Ile His Tyr Leu Arg Leu Thr Ile Ala Ile Leu
        1610                1615                1620

Arg His Glu Lys Ser Arg Lys Phe Leu Leu Ser Asn Val Leu Phe
        1625                1630                1635

Asp Val Leu Arg Ser Val Val Phe Phe Tyr Ile Lys Ser Pro Leu
        1640                1645                1650

Arg Val Glu Glu Ala Gly Leu Gln Glu Leu Ile Pro Thr Thr Trp
        1655                1660                1665

Trp Pro His Cys Ser Ser Arg Glu Gly Lys Glu Ser Thr Glu Met
        1670                1675                1680

Lys Glu Glu Thr Ala Glu Glu Arg Leu Arg Arg Arg Ala Tyr Glu
        1685                1690                1695

Arg Gly Cys Gln Arg Leu Arg Lys Arg Ile Glu Val Val Glu Glu
        1700                1705                1710

Leu Gln Val Gln Ile Leu Lys Leu Leu Leu Asp Asn Lys Asp Asp
        1715                1720                1725

Asn Gly Gly Glu Ala Ser Arg Tyr Ile Phe Leu Thr Lys Phe Arg
        1730                1735                1740

Lys Phe Leu Gln Glu Asn Ala Ser Gly Arg Gly Asn Met Pro Met
        1745                1750                1755

Leu Cys Pro Pro Glu Tyr Met Val Cys Phe Leu His Arg Leu Ile
        1760                1765                1770

Ser Ala Leu Arg Tyr Tyr Trp Asp Glu Tyr Lys Ala Ser Asn Pro
        1775                1780                1785

His Ala Ser Phe Ser Glu Glu Ala Tyr Ile Pro Pro Gln Val Phe
        1790                1795                1800

Tyr Asn Gly Lys Val Asp Tyr Phe Asp Leu Gln Arg Leu Gly Gly
        1805                1810                1815

Leu Leu Ser His Leu Arg Lys Thr Leu Lys Asp Asp Leu Ala Ser
        1820                1825                1830

Lys Ala Asn Ile Val Ile Asp Pro Leu Glu Leu Gln Ser Thr Ala
        1835                1840                1845

Met Asp Asp Leu Asp Glu Asp Glu Glu Pro Ala Pro Ala Met Ala
        1850                1855                1860

Gln Arg Pro Met Gln Ala Leu Ala Val Gly Gly Pro Leu Pro Leu
        1865                1870                1875

Pro Arg Pro Gly Trp Leu Ser Ser Pro Thr Leu Gly Arg Ala Asn
        1880                1885                1890

Arg Phe Leu Ser Thr Ala Ala Val Ser Leu Met Thr Pro Arg Arg
        1895                1900                1905

Pro Leu Ser Thr Ser Glu Lys Val Lys Val Arg Thr Leu Ser Val
        1910                1915                1920

Glu Gln Arg Thr Arg Glu Asp Ile Glu Gly Ser His Trp Asn Glu
        1925                1930                1935

Gly Leu Leu Leu Gly Arg Pro Pro Glu Glu Pro Glu Gln Pro Leu
        1940                1945                1950

Thr Glu Asn Ser Leu Leu Glu Val Leu Asp Gly Ala Val Met Met
    1955                1960                1965

Tyr Asn Leu Ser Val His Gln Gln Leu Gly Lys Met Val Gly Val
    1970                1975                1980

Ser Asp Asp Val Asn Glu Tyr Ala Met Ala Leu Arg Asp Thr Glu
    1985                1990                1995

Asp Lys Leu Arg Arg Cys Pro Lys Arg Arg Lys Asp Ile Leu Ala
    2000                2005                2010

Glu Leu Thr Lys Ser Gln Lys Val Phe Ser Glu Lys Leu Asp His
    2015                2020                2025

Leu Ser Arg Arg Leu Ala Trp Val His Ala Thr Val Tyr Ser Gln
    2030                2035                2040

Glu Lys Met Leu Asp Ile Tyr Trp Leu Leu Arg Val Cys Leu Arg
    2045                2050                2055

Thr Ile Glu His Gly Asp Arg Thr Gly Ser Leu Phe Ala Phe Met
    2060                2065                2070

Pro Glu Phe Tyr Leu Ser Val Ala Ile Asn Ser Tyr Ser Ala Leu
    2075                2080                2085

Lys Asn Tyr Phe Gly Pro Val His Ser Met Glu Glu Leu Pro Gly
    2090                2095                2100

Tyr Glu Glu Thr Leu Thr Arg Leu Ala Ala Ile Leu Ala Lys His
    2105                2110                2115

Phe Ala Asp Ala Arg Ile Val Gly Thr Asp Ile Arg Asp Ser Leu
    2120                2125                2130

Met Gln Ala Leu Ala Ser Tyr Val Cys Tyr Pro His Ser Leu Arg
    2135                2140                2145

Ala Val Glu Arg Ile Pro Glu Glu Gln Arg Ile Ala Met Val Arg
    2150                2155                2160

Asn Leu Leu Ala Pro Tyr Glu Gln Arg Pro Trp Ala Gln Thr Asn
    2165                2170                2175

Trp Ile Leu Val Arg Leu Trp Arg Gly Cys Gly Phe Gly Tyr Arg

2180                2185                2190

Tyr Thr Arg Leu Pro His Leu Leu Lys Thr Lys Leu Glu Asp Ala
    2195                2200                2205

Asn Leu Pro Ser Leu Gln Lys Pro Cys Pro Ser Thr Leu Leu Gln
    2210                2215                2220

Gln His Met Ala Asp Leu Leu Gln Gln Gly Pro Asp Val Ala Pro
    2225                2230                2235

Ser Phe Leu Asn Ser Val Leu Asn Gln Leu Asn Trp Ala Phe Ser
    2240                2245                2250

Glu Phe Ile Gly Met Ile Gln Glu Ile Gln Gln Ala Ala Glu Arg
    2255                2260                2265

Leu Glu Arg Asn Phe Val Asp Ser Arg Gln Leu Lys Val Cys Ala
    2270                2275                2280

Thr Cys Phe Asp Leu Ser Val Ser Leu Leu Arg Val Leu Glu Met
    2285                2290                2295

Thr Ile Thr Leu Val Pro Glu Ile Phe Leu Asp Trp Thr Arg Pro
    2300                2305                2310

Thr Ser Glu Met Leu Leu Arg Arg Leu Ala Gln Leu Leu Asn Gln
    2315                2320                2325

Val Leu Asn Arg Val Thr Ala Glu Arg Asn Leu Phe Asp Arg Val
    2330                2335                2340

Val Thr Leu Arg Leu Pro Gly Leu Glu Ser Val Asp His Tyr Pro
    2345                2350                2355

Ile Leu Val Ala Val Thr Gly Ile Leu Val Gln Leu Leu Val Arg
    2360                2365                2370

Gly Pro Ala Ser Glu Arg Glu Gln Ala Thr Ser Val Leu Leu Ala
    2375                2380                2385

Asp Pro Cys Phe Gln Leu Arg Ser Ile Cys Tyr Leu Leu Gly Gln
    2390                2395                2400

Pro Glu Pro Pro Ala Pro Gly Thr Ala Leu Pro Ala Pro Asp Arg
    2405                2410                2415

Lys Arg Phe Ser Leu Gln Ser Tyr Ala Asp Tyr Ile Ser Ala Asp
    2420                2425                2430

Glu Leu Ala Gln Val Glu Gln Met Leu Ala His Leu Thr Ser Ala
    2435                2440                2445

Ser Ala Gln Ala Ala Ala Ala Ser Leu Pro Thr Ser Glu Glu Asp
    2450                2455                2460

Leu Cys Pro Ile Cys Tyr Ala His Pro Ile Ser Ala Val Phe Gln
    2465                2470                2475

Pro Cys Gly His Lys Ser Cys Lys Ala Cys Ile Asn Gln His Leu
    2480                2485                2490


    2495                2500                2505


    2510                2515                2520


    2525

<210>  7
<211>  7515
<212>  DNA
<213>  Homo sapiens

<220>
<221>  CDS
<222>  (1)..(7515)

<400>  7
atg gcg agc gac tcc ccg gct cga agc ctg gat gaa ata gat ctc tcg        48
Met Ala Ser Asp Ser Pro Ala Arg Ser Leu Asp Glu Ile Asp Leu Ser
1               5                   10                  15

gct ctg agg gac ccc gca ggg atc ttt gaa ttg gtg gaa ctt gtt gga        96
Ala Leu Arg Asp Pro Ala Gly Ile Phe Glu Leu Val Glu Leu Val Gly
            20                  25                  30

aat gga aca tac ggg caa gtt tat aag ggt cgt cat gtc aaa acg ggc       144
Asn Gly Thr Tyr Gly Gln Val Tyr Lys Gly Arg His Val Lys Thr Gly
        35                  40                  45

cag ctt gca gcc atc aag gtt atg gat gtc aca ggg gat gaa gag gaa       192
Gln Leu Ala Ala Ile Lys Val Met Asp Val Thr Gly Asp Glu Glu Glu
    50                  55                  60

gaa atc aaa caa gaa att aac atg ttg aag aaa tat tct cat cac cgg       240
Glu Ile Lys Gln Glu Ile Asn Met Leu Lys Lys Tyr Ser His His Arg
65                  70                  75                  80

aat att gct aca tac tat ggt gct ttt atc aaa aag aac cca cca ggc 288
Asn Ile Ala Thr Tyr Tyr Gly Ala Phe Ile Lys Lys Asn Pro Pro Gly
                85                  90                  95

atg gat gac caa ctt tgg ttg gtg atg gag ttt tgt ggt gct ggc tct 336
Met Asp Asp Gln Leu Trp Leu Val Met Glu Phe Cys Gly Ala Gly Ser
            100                 105                 110

gtc acc gac ctg atc aag aac aca aaa ggt aac acg ttg aaa gag gag 384
Val Thr Asp Leu Ile Lys Asn Thr Lys Gly Asn Thr Leu Lys Glu Glu
        115                 120                 125

tgg att gca tac atc tgc agg gaa atc tta cgg ggg ctg agt cac ctg 432
Trp Ile Ala Tyr Ile Cys Arg Glu Ile Leu Arg Gly Leu Ser His Leu
    130                 135                 140

cac cag cat aaa gtg att cat cga gat att aaa ggg caa aat gtc ttg 480
His Gln His Lys Val Ile His Arg Asp Ile Lys Gly Gln Asn Val Leu
145                 150                 155                 160

ctg act gaa aat gca gaa gtt aaa cta gtg gac ttt gga gtc agt gct 528
Leu Thr Glu Asn Ala Glu Val Lys Leu Val Asp Phe Gly Val Ser Ala
                165                 170                 175

cag ctt gat cga aca gtg ggc agg agg aat act ttc att gga act ccc 576
Gln Leu Asp Arg Thr Val Gly Arg Arg Asn Thr Phe Ile Gly Thr Pro
            180                 185                 190

tac tgg atg gca cca gaa gtt att gcc tgt gat gaa aac cca gat gcc 624
Tyr Trp Met Ala Pro Glu Val Ile Ala Cys Asp Glu Asn Pro Asp Ala
        195                 200                 205

aca tat gat ttc aag agt gac ttg tgg tct ttg ggt atc acc gcc att 672
Thr Tyr Asp Phe Lys Ser Asp Leu Trp Ser Leu Gly Ile Thr Ala Ile
    210                 215                 220

gaa atg gca gaa ggt gct ccc cct ctc tgt gac atg cac ccc atg aga 720
Glu Met Ala Glu Gly Ala Pro Pro Leu Cys Asp Met His Pro Met Arg
225                 230                 235                 240

gct ctc ttc ctc atc ccc cgg aac cca gcg cct cgg ctg aag tct aag 768
Ala Leu Phe Leu Ile Pro Arg Asn Pro Ala Pro Arg Leu Lys Ser Lys
                245                 250                 255

aag tgg tca aaa aaa ttc cag tca ttt att gag agc tgc ttg gta aag 816
Lys Trp Ser Lys Lys Phe Gln Ser Phe Ile Glu Ser Cys Leu Val Lys
            260                 265                 270

aat cac agc cag cga cca gca aca gaa caa ttg atg aag cat cca ttt 864
Asn His Ser Gln Arg Pro Ala Thr Glu Gln Leu Met Lys His Pro Phe
        275                 280                 285

ata cga gac caa cct aat gag cga cag gtc cgc att caa ctc aag gac 912
Ile Arg Asp Gln Pro Asn Glu Arg Gln Val Arg Ile Gln Leu Lys Asp
    290                 295                 300

cat att gat aga aca aag aag aag cga gga gaa aaa gat gag aca gag 960
His Ile Asp Arg Thr Lys Lys Lys Arg Gly Glu Lys Asp Glu Thr Glu
305                 310                 315                 320

tat gag tac agt gga agt gag gaa gaa gag gag gag aat gac tca gga 1008
Tyr Glu Tyr Ser Gly Ser Glu Glu Glu Glu Glu Glu Asn Asp Ser Gly
                325                 330                 335

gag ccc agc tcc atc ctg aat ctg cca ggg gag tcg acg ctg cgg agg 1056
Glu Pro Ser Ser Ile Leu Asn Leu Pro Gly Glu Ser Thr Leu Arg Arg
            340                 345                 350

gac ttt ctg agg ctg cag ctg gcc aac aag gag cgt tct gag gcc cta 1104
Asp Phe Leu Arg Leu Gln Leu Ala Asn Lys Glu Arg Ser Glu Ala Leu
        355                 360                 365

cgg agg cag cag ctg gag cag cag cag cgg gag aat gag gag cac aag 1152
Arg Arg Gln Gln Leu Glu Gln Gln Gln Arg Glu Asn Glu Glu His Lys
    370                 375                 380

cgg cag ctg ctg gcc gag cgt cag aag cgc atc gag gag cag aaa gag 1200
Arg Gln Leu Leu Ala Glu Arg Gln Lys Arg Ile Glu Glu Gln Lys Glu
385                 390                 395                 400

cag agg cgg cgg ctg gag gag caa caa agg cga gag aag gag ctg cgg 1248
Gln Arg Arg Arg Leu Glu Glu Gln Gln Arg Arg Glu Lys Glu Leu Arg
                405                 410                 415

aag cag cag gag agg gag cag cgc cgg cac tat gag gag cag atg cgc 1296
Lys Gln Gln Glu Arg Glu Gln Arg Arg His Tyr Glu Glu Gln Met Arg
            420                 425                 430

cgg gag gag gag agg agg cgt gcg gag cat gaa cag gaa tat aag cgc 1344
Arg Glu Glu Glu Arg Arg Arg Ala Glu His Glu Gln Glu Tyr Lys Arg
        435                 440                 445

aaa caa ttg gaa gaa cag aga caa gca gaa aga ctg cag agg cag cta 1392
Lys Gln Leu Glu Glu Gln Arg Gln Ala Glu Arg Leu Gln Arg Gln Leu
    450                 455                 460

aag caa gaa aga gac tac tta gtt tcc ctt cag cat cag cgg cag gag 1440
Lys Gln Glu Arg Asp Tyr Leu Val Ser Leu Gln His Gln Arg Gln Glu
465                 470                 475                 480

cag agg cct gtg gag aag aag cca ctg tac cat tac aaa gaa gga atg 1488
Gln Arg Pro Val Glu Lys Lys Pro Leu Tyr His Tyr Lys Glu Gly Met
                485                 490                 495

agt cct agt gag aag cca gca tgg gcc aag gag gta gaa gaa cgg tca 1536
Ser Pro Ser Glu Lys Pro Ala Trp Ala Lys Glu Val Glu Glu Arg Ser
            500                 505                 510

agg ctc aac cgg caa agt tcc cct gcc atg cct cac aag gtt gcc aac 1584
Arg Leu Asn Arg Gln Ser Ser Pro Ala Met Pro His Lys Val Ala Asn
        515                 520                 525

agg ata tct gac ccc aac ctg ccc cca agg tcg gag tcc ttc agc att 1632
Arg Ile Ser Asp Pro Asn Leu Pro Pro Arg Ser Glu Ser Phe Ser Ile
    530                 535                 540

agt gga gtt cag cct gct cga aca ccc ccc atg ctc aga cca gtc gat 1680
Ser Gly Val Gln Pro Ala Arg Thr Pro Pro Met Leu Arg Pro Val Asp
545                 550                 555                 560

ccc cag atc cca cat ctg gta gct gta aaa tcc cag gga cct gcc ttg 1728
Pro Gln Ile Pro His Leu Val Ala Val Lys Ser Gln Gly Pro Ala Leu
                565                 570                 575

acc gcc tcc cag tca gtg cac gag cag ccc aca aag ggc ctc tct ggg 1776
Thr Ala Ser Gln Ser Val His Glu Gln Pro Thr Lys Gly Leu Ser Gly

580                 585                 590

ttt cag gag gct ctg aac gtg acc tcc cac cgc gtg gag atg cca cgc       1824
Phe Gln Glu Ala Leu Asn Val Thr Ser His Arg Val Glu Met Pro Arg
        595                 600                 605

cag aac tca gat ccc acc tcg gaa aat cct cct ctc ccc act cgc att       1872
Gln Asn Ser Asp Pro Thr Ser Glu Asn Pro Pro Leu Pro Thr Arg Ile
    610                 615                 620

gaa aag ttt gac cga agc tct tgg tta cga cag gaa gaa gac att cca       1920
Glu Lys Phe Asp Arg Ser Ser Trp Leu Arg Gln Glu Glu Asp Ile Pro
625                 630                 635                 640

cca aag gtg cct caa aga aca act tct ata tcc cca gca tta gcc aga       1968
Pro Lys Val Pro Gln Arg Thr Thr Ser Ile Ser Pro Ala Leu Ala Arg
                645                 650                 655

aag aat tct cct ggg aat ggt agt gct ctg gga ccc aga cta gga tct       2016
Lys Asn Ser Pro Gly Asn Gly Ser Ala Leu Gly Pro Arg Leu Gly Ser
            660                 665                 670

caa ccc atc aga gca agc aac cct gat ctc cgg aga act gag ccc atc       2064
Gln Pro Ile Arg Ala Ser Asn Pro Asp Leu Arg Arg Thr Glu Pro Ile
        675                 680                 685

ttg gag agc ccc ttg cag agg acc agc agt ggc agt tcc tcc agc tcc       2112
Leu Glu Ser Pro Leu Gln Arg Thr Ser Ser Gly Ser Ser Ser Ser Ser
    690                 695                 700

agc acc cct agc tcc cag ccc agc tcc caa gga ggc tcc cag cct gga       2160
Ser Thr Pro Ser Ser Gln Pro Ser Ser Gln Gly Gly Ser Gln Pro Gly
705                 710                 715                 720

tca caa gca gga tcc agt gaa cgc acc aga gtt cga gcc aac agt aag       2208
Ser Gln Ala Gly Ser Ser Glu Arg Thr Arg Val Arg Ala Asn Ser Lys
                725                 730                 735

tca gaa gga tca cct gtg ctc ccc cat gag cct gcc aag gtg aaa cca       2256
Ser Glu Gly Ser Pro Val Leu Pro His Glu Pro Ala Lys Val Lys Pro
            740                 745                 750

gaa gaa tcc agg gac att acc cgg ccc agt cga cca gct agc tac aaa       2304
Glu Glu Ser Arg Asp Ile Thr Arg Pro Ser Arg Pro Ala Ser Tyr Lys
        755                 760                 765

aaa gct ata gat gag gat ctg acg gca tta gcc aaa gaa cta aga gaa       2352
Lys Ala Ile Asp Glu Asp Leu Thr Ala Leu Ala Lys Glu Leu Arg Glu
    770                 775                 780

ctc cgg att gaa gaa aca aac cgc cca atg aag aag gtg act gat tac       2400
Leu Arg Ile Glu Glu Thr Asn Arg Pro Met Lys Lys Val Thr Asp Tyr
785                 790                 795                 800

tcc tcc tcc agt gag gag tca gaa agt agc gag gaa gag gag gaa gat       2448
Ser Ser Ser Ser Glu Glu Ser Glu Ser Ser Glu Glu Glu Glu Glu Asp
                805                 810                 815

gga gag agc gag acc cat gat ggg aca gtg gct gtc agc gac ata ccc       2496
Gly Glu Ser Glu Thr His Asp Gly Thr Val Ala Val Ser Asp Ile Pro
            820                 825                 830

aga ctg ata cca aca gga gct cca ggc agc aac gag cag tac aat gtg       2544

Arg Leu Ile Pro Thr Gly Ala Pro Gly Ser Asn Glu Gln Tyr Asn Val
        835                 840                 845

gga atg gtg ggg acg cat ggg ctg gag acc tct cat gcg gac agt ttc      2592
Gly Met Val Gly Thr His Gly Leu Glu Thr Ser His Ala Asp Ser Phe
    850                 855                 860

agc ggc agt att tca aga gaa gga acc ttg atg att aga gag acg tct      2640
Ser Gly Ser Ile Ser Arg Glu Gly Thr Leu Met Ile Arg Glu Thr Ser
865                 870                 875                 880

gga gag aag aag cga tct ggc cac agt gac agc aat ggc ttt gct ggc      2688
Gly Glu Lys Lys Arg Ser Gly His Ser Asp Ser Asn Gly Phe Ala Gly
                885                 890                 895

cac atc aac ctc cct gac ctg gtg cag cag agc cat tct cca gct gga      2736
His Ile Asn Leu Pro Asp Leu Val Gln Gln Ser His Ser Pro Ala Gly
            900                 905                 910

acc ccg act gag gga ctg ggg cgc gtc tca acc cat tcc cag gag atg      2784
Thr Pro Thr Glu Gly Leu Gly Arg Val Ser Thr His Ser Gln Glu Met
        915                 920                 925

gac tct ggg act gaa tat ggc atg ggg agc agc acc aaa gcc tcc ttc      2832
Asp Ser Gly Thr Glu Tyr Gly Met Gly Ser Ser Thr Lys Ala Ser Phe
    930                 935                 940

acc ccc ttt gtg gac ccc aga gta tac cag acg tct ccc act gat gaa      2880
Thr Pro Phe Val Asp Pro Arg Val Tyr Gln Thr Ser Pro Thr Asp Glu
945                 950                 955                 960

gat gaa gag gat gag gaa tca tca gcc gca gct ctg ttt act agc gaa      2928
Asp Glu Glu Asp Glu Glu Ser Ser Ala Ala Ala Leu Phe Thr Ser Glu
                965                 970                 975

ctt ctt agg caa gaa cag gcc aaa ctc aat gaa gca aga aag att tcg      2976
Leu Leu Arg Gln Glu Gln Ala Lys Leu Asn Glu Ala Arg Lys Ile Ser
            980                 985                 990

gtg gta aat gta aac cca acc aac  att cgg cct cat agc  gac aca cca      3024
Val Val Asn Val Asn Pro Thr Asn  Ile Arg Pro His Ser  Asp Thr Pro
        995                 1000                1005

gaa atc  aga aaa tac aag aaa  cga ttc aac tca gaa  ata ctt tgt      3069
Glu Ile  Arg Lys Tyr Lys Lys  Arg Phe Asn Ser Glu  Ile Leu Cys
    1010                1015                1020

gca gct  ctg tgg ggt gta aac  ctt ctg gtg ggg act  gaa aat ggc      3114
Ala Ala  Leu Trp Gly Val Asn  Leu Leu Val Gly Thr  Glu Asn Gly
    1025                1030                1035

ctg atg  ctt ttg gac cga agt  ggg caa ggc aaa gtc  tat aat ctg      3159
Leu Met  Leu Leu Asp Arg Ser  Gly Gln Gly Lys Val  Tyr Asn Leu
    1040                1045                1050

atc aac  cgg agg cga ttt cag  cag atg gat gtg cta  gag gga ctg      3204
Ile Asn  Arg Arg Arg Phe Gln  Gln Met Asp Val Leu  Glu Gly Leu
    1055                1060                1065

aat gtc  ctt gtg aca att tca  gga aag aag aat aag  cta cga gtt      3249
Asn Val  Leu Val Thr Ile Ser  Gly Lys Lys Asn Lys  Leu Arg Val
    1070                1075                1080

tac tat ctt tca tgg tta aga aac aga ata cta cat aat gac cca 3294
Tyr Tyr Leu Ser Trp Leu Arg Asn Arg Ile Leu His Asn Asp Pro
    1085                1090                1095

gaa gta gaa aag aaa caa ggc tgg atc act gtt ggg gac ttg gaa 3339
Glu Val Glu Lys Lys Gln Gly Trp Ile Thr Val Gly Asp Leu Glu
    1100                1105                1110

ggc tgt ata cat tat aaa gtt gtt aaa tat gaa agg atc aaa ttt 3384
Gly Cys Ile His Tyr Lys Val Val Lys Tyr Glu Arg Ile Lys Phe
    1115                1120                1125

ttg gtg att gcc tta aag aat gct gtg gaa ata tat gct tgg gct 3429
Leu Val Ile Ala Leu Lys Asn Ala Val Glu Ile Tyr Ala Trp Ala
    1130                1135                1140

cct aaa ccg tat cat aaa ttc atg gca ttt aag tct ttt gca gat 3474
Pro Lys Pro Tyr His Lys Phe Met Ala Phe Lys Ser Phe Ala Asp
    1145                1150                1155

ctc cag cac aag cct ctg cta gtt gat ctc acg gta gaa gaa ggt 3519
Leu Gln His Lys Pro Leu Leu Val Asp Leu Thr Val Glu Glu Gly
    1160                1165                1170

caa aga tta aag gtt att ttt ggt tca cac act ggt ttc cat gta 3564
Gln Arg Leu Lys Val Ile Phe Gly Ser His Thr Gly Phe His Val
    1175                1180                1185

att gat gtt gat tca gga aac tct tat gat atc tac ata cca tct 3609
Ile Asp Val Asp Ser Gly Asn Ser Tyr Asp Ile Tyr Ile Pro Ser
    1190                1195                1200

cat att cag ggc aat atc act cct cat gct att gtc atc ttg cct 3654
His Ile Gln Gly Asn Ile Thr Pro His Ala Ile Val Ile Leu Pro
    1205                1210                1215

aaa aca gat gga atg gaa atg ctt gtt tgc tat gag gat gag ggg 3699
Lys Thr Asp Gly Met Glu Met Leu Val Cys Tyr Glu Asp Glu Gly
    1220                1225                1230

gtg tat gta aac acc tat ggc cgg ata act aag gat gtg gtg ctc 3744
Val Tyr Val Asn Thr Tyr Gly Arg Ile Thr Lys Asp Val Val Leu
    1235                1240                1245

caa tgg gga gaa atg ccc acg tct gtg gcc tac att cat tcc aat 3789
Gln Trp Gly Glu Met Pro Thr Ser Val Ala Tyr Ile His Ser Asn
    1250                1255                1260

cag ata atg ggc tgg ggc gag aaa gct att gag atc cgg tca gtg 3834
Gln Ile Met Gly Trp Gly Glu Lys Ala Ile Glu Ile Arg Ser Val
    1265                1270                1275

gaa aca gga cat ttg gat gga gta ttt atg cat aag cga gct caa 3879
Glu Thr Gly His Leu Asp Gly Val Phe Met His Lys Arg Ala Gln
    1280                1285                1290

agg tta aag ttt cta tgt gaa aga aat gat aag gtg att gga cac 3924
Arg Leu Lys Phe Leu Cys Glu Arg Asn Asp Lys Val Ile Gly His
    1295                1300                1305

agc aac ttt ggc acc atc cgc tct acc aca tgc gtg tac aaa ggg 3969
Ser Asn Phe Gly Thr Ile Arg Ser Thr Thr Cys Val Tyr Lys Gly
    1310                1315                1320

aaa tgg ctc tac gag gtc ctc atc tcc tcc cag ggg ctc atg cag 4014
Lys Trp Leu Tyr Glu Val Leu Ile Ser Ser Gln Gly Leu Met Gln
    1325                1330                1335

atc ggc tgg tgc acc atc agc tgc cgc ttc aac cag gag gag ggg 4059
Ile Gly Trp Cys Thr Ile Ser Cys Arg Phe Asn Gln Glu Glu Gly
    1340                1345                1350

gtt gga gat aca cac aac tcc tat gcc tat gat ggc aac cgc gtg 4104
Val Gly Asp Thr His Asn Ser Tyr Ala Tyr Asp Gly Asn Arg Val
    1355                1360                1365

cgc aag tgg aat gtg acc aca acg aat tat ggc aag gcg tgg gca 4149
Arg Lys Trp Asn Val Thr Thr Thr Asn Tyr Gly Lys Ala Trp Ala
    1370                1375                1380

gcg ggg gac atc gtg agc tgc ctg att gac ctg gat gat ggc act 4194
Ala Gly Asp Ile Val Ser Cys Leu Ile Asp Leu Asp Asp Gly Thr
    1385                1390                1395

ctg tcc ttc tgc ctg aac ggt gta tca ctg ggc act gcc ttt gag 4239
Leu Ser Phe Cys Leu Asn Gly Val Ser Leu Gly Thr Ala Phe Glu
    1400                1405                1410

aac ctg tcc agg ggc ctg ggt atg gcc tac ttc cca gcc atc agc 4284
Asn Leu Ser Arg Gly Leu Gly Met Ala Tyr Phe Pro Ala Ile Ser
    1415                1420                1425

ctc tct ttc aag gag tcc gtg gcc ttc aac ttt ggc agc cgt cct 4329
Leu Ser Phe Lys Glu Ser Val Ala Phe Asn Phe Gly Ser Arg Pro
    1430                1435                1440

ctg cgc tac cca gtg gca ggc tac cgg ccc ctg cag gac cca ccg 4374
Leu Arg Tyr Pro Val Ala Gly Tyr Arg Pro Leu Gln Asp Pro Pro
    1445                1450                1455

agt gct gac ctg gtg cgg gca cag agg ttg ctg ggc tgc ttc cgg 4419
Ser Ala Asp Leu Val Arg Ala Gln Arg Leu Leu Gly Cys Phe Arg
    1460                1465                1470

gca gtg ctg agt gtg gag ctg gac cct gtg gag ggg cgg ctg ttg 4464
Ala Val Leu Ser Val Glu Leu Asp Pro Val Glu Gly Arg Leu Leu
    1475                1480                1485

gac aag gag agc tcc aag tgg cgg ttg cgg ggc cag ccc acc gtc 4509
Asp Lys Glu Ser Ser Lys Trp Arg Leu Arg Gly Gln Pro Thr Val
    1490                1495                1500

ctc ctc aca ctg gcc cac atc ttc cat cac ttt gca ccg ctt ctg 4554
Leu Leu Thr Leu Ala His Ile Phe His His Phe Ala Pro Leu Leu
    1505                1510                1515

cgc aag gtg tat ctg gtg gag gct gtg ctc atg agc ttc ttg ctg 4599
Arg Lys Val Tyr Leu Val Glu Ala Val Leu Met Ser Phe Leu Leu
    1520                1525                1530

ggc atc gtg gag aag ggc aca ccc aca cag gca cag tcc gtg gtg 4644
Gly Ile Val Glu Lys Gly Thr Pro Thr Gln Ala Gln Ser Val Val
    1535                1540                1545

cac cag gtc ctg gac ctc ttg tgg ctc ttc atg gag gac tac gag 4689
His Gln Val Leu Asp Leu Leu Trp Leu Phe Met Glu Asp Tyr Glu

1550                1555                1560

gta caa gat tgc ctc aag cag ttg atg atg tct ctg ctt cgg ctg        4734
Val Gln Asp Cys Leu Lys Gln Leu Met Met Ser Leu Leu Arg Leu
    1565                1570                1575

tac cga ttc tca ccc att gtc cca gac ctg ggc cta cag atc cat        4779
Tyr Arg Phe Ser Pro Ile Val Pro Asp Leu Gly Leu Gln Ile His
    1580                1585                1590

tac ctg cgg ctc act atc gcc atc ctg agg cat gag aag tcc cgc        4824
Tyr Leu Arg Leu Thr Ile Ala Ile Leu Arg His Glu Lys Ser Arg
    1595                1600                1605

aag ttt ctg ctt agc aat gtc ctc ttc gac gtg ctc cgc tcc gtc        4869
Lys Phe Leu Leu Ser Asn Val Leu Phe Asp Val Leu Arg Ser Val
    1610                1615                1620

gtc ttc ttt tac atc aag agc ccc ctg cgt gtg gag gag gcc ggc        4914
Val Phe Phe Tyr Ile Lys Ser Pro Leu Arg Val Glu Glu Ala Gly
    1625                1630                1635

ctg cag gag ctc att ccc acc acc tgg tgg ccc cac tgc tcc agt        4959
Leu Gln Glu Leu Ile Pro Thr Thr Trp Trp Pro His Cys Ser Ser
    1640                1645                1650

agg gag ggc aaa gag agc acg gag atg aag gag gag acc gca gag        5004
Arg Glu Gly Lys Glu Ser Thr Glu Met Lys Glu Glu Thr Ala Glu
    1655                1660                1665

gag cgg ctg cgg cgg cga gcc tac gaa cgg ggc tgt cag cgg ctc        5049
Glu Arg Leu Arg Arg Arg Ala Tyr Glu Arg Gly Cys Gln Arg Leu
    1670                1675                1680

agg aag cgc atc gaa gtg gtg gaa gaa cta cag gtc cag atc ctg        5094
Arg Lys Arg Ile Glu Val Val Glu Glu Leu Gln Val Gln Ile Leu
    1685                1690                1695

aag ctg ctg ctg gac aat aaa gat gac aat ggg ggt gaa gct tct        5139
Lys Leu Leu Leu Asp Asn Lys Asp Asp Asn Gly Gly Glu Ala Ser
    1700                1705                1710

agg tat atc ttc ctg acc aag ttt cgc aag ttt ctg cag gag aac        5184
Arg Tyr Ile Phe Leu Thr Lys Phe Arg Lys Phe Leu Gln Glu Asn
    1715                1720                1725

gcc agt ggc cgg ggg aac atg ccc atg ctc tgc ccc cct gag tac        5229
Ala Ser Gly Arg Gly Asn Met Pro Met Leu Cys Pro Pro Glu Tyr
    1730                1735                1740

atg gtc tgc ttc tta cac cgg ctg atc tct gcc ctg cgc tac tat        5274
Met Val Cys Phe Leu His Arg Leu Ile Ser Ala Leu Arg Tyr Tyr
    1745                1750                1755

tgg gat gaa tac aag gct tcc aat cct cat gct tcc ttc agt gag        5319
Trp Asp Glu Tyr Lys Ala Ser Asn Pro His Ala Ser Phe Ser Glu
    1760                1765                1770

gag gcc tac atc ccg ccc cag gtc ttc tat aat ggc aag gtg gac        5364
Glu Ala Tyr Ile Pro Pro Gln Val Phe Tyr Asn Gly Lys Val Asp
    1775                1780                1785

tac ttt gac ctg cag cgc ctg ggg ggc ctc ctc tcg cac ctg cgg        5409

Tyr Phe Asp Leu Gln Arg Leu Gly Gly Leu Leu Ser His Leu Arg
    1790                1795                1800

aag acc ctc aaa gat gac ctt gct tcc aaa gcc aac att gtg atc 5454
Lys Thr Leu Lys Asp Asp Leu Ala Ser Lys Ala Asn Ile Val Ile
    1805                1810                1815

gac cca ctg gag ctc cag tca acc gcc atg gat gac cta gat gag 5499
Asp Pro Leu Glu Leu Gln Ser Thr Ala Met Asp Asp Leu Asp Glu
    1820                1825                1830

gat gag gag cca gcc cca gct atg gcc cag cgc ccc atg cag gcc 5544
Asp Glu Glu Pro Ala Pro Ala Met Ala Gln Arg Pro Met Gln Ala
    1835                1840                1845

ctg gct gtt ggg ggg cca ctg ccc ctg ccc cgg ccc ggc tgg ctc 5589
Leu Ala Val Gly Gly Pro Leu Pro Leu Pro Arg Pro Gly Trp Leu
    1850                1855                1860

agt tct cca act ttg ggc cga gcc aac cgc ttc ctc agc aca gcg 5634
Ser Ser Pro Thr Leu Gly Arg Ala Asn Arg Phe Leu Ser Thr Ala
    1865                1870                1875

gct gtg agc ctc atg acc cca cgg cgg cct ctg agc acc tcg gag 5679
Ala Val Ser Leu Met Thr Pro Arg Arg Pro Leu Ser Thr Ser Glu
    1880                1885                1890

aaa gtg aag gtc cgc acg ctg agc gtg gag cag agg acc cgt gag 5724
Lys Val Lys Val Arg Thr Leu Ser Val Glu Gln Arg Thr Arg Glu
    1895                1900                1905

gac att gaa ggc agc cac tgg aat gag ggc ttg ctg ctg ggg cgg 5769
Asp Ile Glu Gly Ser His Trp Asn Glu Gly Leu Leu Leu Gly Arg
    1910                1915                1920

ccc ccc gag gag cct gag cag ccc ctc acc gag aac tcg ctg ctg 5814
Pro Pro Glu Glu Pro Glu Gln Pro Leu Thr Glu Asn Ser Leu Leu
    1925                1930                1935

gaa gtc ctg gat ggg gcg gtc atg atg tac aac ctc agc gta cac 5859
Glu Val Leu Asp Gly Ala Val Met Met Tyr Asn Leu Ser Val His
    1940                1945                1950

cag cag ctg ggc aag atg gtg ggt gtc tcc gat gat gtc aat gaa 5904
Gln Gln Leu Gly Lys Met Val Gly Val Ser Asp Asp Val Asn Glu
    1955                1960                1965

tac gct atg gct ctg agg gac aca gag gac aag ctc cgc cgg tgc 5949
Tyr Ala Met Ala Leu Arg Asp Thr Glu Asp Lys Leu Arg Arg Cys
    1970                1975                1980

ccc aag agg agg aag gac atc ctt gca gag ttg acc aag agc cag 5994
Pro Lys Arg Arg Lys Asp Ile Leu Ala Glu Leu Thr Lys Ser Gln
    1985                1990                1995

aag gtt ttc tca gaa aag ctg gac cac ctg agc cgc cgt ctt gcc 6039
Lys Val Phe Ser Glu Lys Leu Asp His Leu Ser Arg Arg Leu Ala
    2000                2005                2010

tgg gtc cat gcc act gtc tac tcc cag gag aag atg ctg gac atc 6084
Trp Val His Ala Thr Val Tyr Ser Gln Glu Lys Met Leu Asp Ile
    2015                2020                2025

tac tgg ctg ctg cgc gtc tgc ctg cgg acc att gag cac ggt gat 6129
Tyr Trp Leu Leu Arg Val Cys Leu Arg Thr Ile Glu His Gly Asp
    2030                2035                2040

cgc aca ggg tct ctc ttt gcc ttc atg ccc gag ttc tac ctg agc 6174
Arg Thr Gly Ser Leu Phe Ala Phe Met Pro Glu Phe Tyr Leu Ser
    2045                2050                2055

gtg gcc atc aac agc tac agt gct ctc aag aat tac ttt ggt ccc 6219
Val Ala Ile Asn Ser Tyr Ser Ala Leu Lys Asn Tyr Phe Gly Pro
    2060                2065                2070

gtg cac agc atg gag gag ctc cca ggc tat gaa gag acc ctg acc 6264
Val His Ser Met Glu Glu Leu Pro Gly Tyr Glu Glu Thr Leu Thr
    2075                2080                2085

cgc ctg gct gcc att ctc gcc aaa cac ttt gcc gac gca cgc att 6309
Arg Leu Ala Ala Ile Leu Ala Lys His Phe Ala Asp Ala Arg Ile
    2090                2095                2100

gtg ggc act gac atc cga gac tca ctg atg cag gcc ctg gcc agc 6354
Val Gly Thr Asp Ile Arg Asp Ser Leu Met Gln Ala Leu Ala Ser
    2105                2110                2115

tac gtg tgc tac cca cac tcc ctg cgg gct gtg gag cga atc ccc 6399
Tyr Val Cys Tyr Pro His Ser Leu Arg Ala Val Glu Arg Ile Pro
    2120                2125                2130

gag gag cag cgt atc gcc atg gtg agg aac ctc ctg gcg ccc tat 6444
Glu Glu Gln Arg Ile Ala Met Val Arg Asn Leu Leu Ala Pro Tyr
    2135                2140                2145

gag cag cgg ccc tgg gcc cag acc aac tgg atc ctg gtg cgg ctc 6489
Glu Gln Arg Pro Trp Ala Gln Thr Asn Trp Ile Leu Val Arg Leu
    2150                2155                2160

tgg agg ggc tgt ggc ttc ggg tac cgc tat aca cgg ctg cca cat 6534
Trp Arg Gly Cys Gly Phe Gly Tyr Arg Tyr Thr Arg Leu Pro His
    2165                2170                2175

ctg ctg aaa acc aaa ctt gag gac gcc aat ttg ccc agc ctc cag 6579
Leu Leu Lys Thr Lys Leu Glu Asp Ala Asn Leu Pro Ser Leu Gln
    2180                2185                2190

aag ccc tgc cct tcc acc ctg ctg cag cag cac atg gcg gac ctc 6624
Lys Pro Cys Pro Ser Thr Leu Leu Gln Gln His Met Ala Asp Leu
    2195                2200                2205

cta cag cag ggt cct gat gtg gca ccc agc ttc ctc aac agc gtc 6669
Leu Gln Gln Gly Pro Asp Val Ala Pro Ser Phe Leu Asn Ser Val
    2210                2215                2220

ctc aat cag ctc aac tgg gcc ttc tct gaa ttc att ggc atg atc 6714
Leu Asn Gln Leu Asn Trp Ala Phe Ser Glu Phe Ile Gly Met Ile
    2225                2230                2235

caa gag atc cag cag gct gct gag cgc ctg gag cgg aac ttt gtg 6759
Gln Glu Ile Gln Gln Ala Ala Glu Arg Leu Glu Arg Asn Phe Val
    2240                2245                2250

gac agc cgg cag ctc aag gta tgt gcc acc tgc ttt gac ctc tcg 6804
Asp Ser Arg Gln Leu Lys Val Cys Ala Thr Cys Phe Asp Leu Ser
    2255                2260                2265

gtc agc ctg ctg cgt gtc ttg gag atg act atc aca ctg gtg cct 6849
Val Ser Leu Leu Arg Val Leu Glu Met Thr Ile Thr Leu Val Pro
    2270                2275                2280

gag ata ttc ctt gac tgg acc cgg cct acc tct gag atg ctg ctg 6894
Glu Ile Phe Leu Asp Trp Thr Arg Pro Thr Ser Glu Met Leu Leu
    2285                2290                2295

cgg cgt ctt gca cag ctg cta aac cag gtg ctg aac cgg gtg aca 6939
Arg Arg Leu Ala Gln Leu Leu Asn Gln Val Leu Asn Arg Val Thr
    2300                2305                2310

gct gag agg aac ctg ttt gat cgt gtg gtc acc cta cgg ctg cct 6984
Ala Glu Arg Asn Leu Phe Asp Arg Val Val Thr Leu Arg Leu Pro
    2315                2320                2325

ggc cta gag agc gtg gac cac tat ccc att ctg gtg gca gtg acg 7029
Gly Leu Glu Ser Val Asp His Tyr Pro Ile Leu Val Ala Val Thr
    2330                2335                2340

ggc atc ctg gtg cag ctc ctg gtg cgt ggc cca gcc tca gag aga 7074
Gly Ile Leu Val Gln Leu Leu Val Arg Gly Pro Ala Ser Glu Arg
    2345                2350                2355

gag caa gcc aca tca gtg ctc ctg gca gat ccc tgc ttc cag cta 7119
Glu Gln Ala Thr Ser Val Leu Leu Ala Asp Pro Cys Phe Gln Leu
    2360                2365                2370

cgc tca ata tgc tat ctc ctg gga cag cca gag ccc cca gca cct 7164
Arg Ser Ile Cys Tyr Leu Leu Gly Gln Pro Glu Pro Pro Ala Pro
    2375                2380                2385

ggc act gct ctg cca gcc cct gac cgg aag cgc ttc tcc ctg cag 7209
Gly Thr Ala Leu Pro Ala Pro Asp Arg Lys Arg Phe Ser Leu Gln
    2390                2395                2400

agc tat gcg gat tat atc agt gcc gat gag ctg gcc caa gtg gaa 7254
Ser Tyr Ala Asp Tyr Ile Ser Ala Asp Glu Leu Ala Gln Val Glu
    2405                2410                2415

cag atg ctg gcg cac ctg acc tct gca tct gcc cag gca gca gct 7299
Gln Met Leu Ala His Leu Thr Ser Ala Ser Ala Gln Ala Ala Ala
    2420                2425                2430

gcc tcc ctg ccc acc agt gag gag gac ctc tgc ccc atc tgc tat 7344
Ala Ser Leu Pro Thr Ser Glu Glu Asp Leu Cys Pro Ile Cys Tyr
    2435                2440                2445

gcc cac ccc atc tct gct gtg ttc cag ccc tgt ggc cac aag tcc 7389
Ala His Pro Ile Ser Ala Val Phe Gln Pro Cys Gly His Lys Ser
    2450                2455                2460

tgc aaa gcc tgt atc aac cag cac ctg atg aac aac aag gac tgc 7434
Cys Lys Ala Cys Ile Asn Gln His Leu Met Asn Asn Lys Asp Cys
    2465                2470                2475

ttc ttc tgc aaa acc acc atc gtg tct gta gag gac tgg gag aag 7479
Phe Phe Cys Lys Thr Thr Ile Val Ser Val Glu Asp Trp Glu Lys
    2480                2485                2490

gga gcc aat acg agt act acc tcc tca gct gcc tag 7515
Gly Ala Asn Thr Ser Thr Thr Ser Ser Ala Ala

2495                2500


<210>  8
<211>  2504
<212>  PRT
<213>  Homo sapiens

<400>  8
   Met Ala Ser Asp Ser Pro Ala Arg Ser Leu Asp Glu Ile Asp Leu Ser
   1               5                   10                  15
   Ala Leu Arg Asp Pro Ala Gly Ile Phe Glu Leu Val Glu Leu Val Gly
               20                  25                  30
   Asn Gly Thr Tyr Gly Gln Val Tyr Lys Gly Arg His Val Lys Thr Gly
           35                  40                  45
   Gln Leu Ala Ala Ile Lys Val Met Asp Val Thr Gly Asp Glu Glu Glu
       50                  55                  60
   Glu Ile Lys Gln Glu Ile Asn Met Leu Lys Lys Tyr Ser His His Arg
   65                  70                  75                  80
   Asn Ile Ala Thr Tyr Tyr Gly Ala Phe Ile Lys Lys Asn Pro Pro Gly
                   85                  90                  95
   Met Asp Asp Gln Leu Trp Leu Val Met Glu Phe Cys Gly Ala Gly Ser
               100                 105                 110
   Val Thr Asp Leu Ile Lys Asn Thr Lys Gly Asn Thr Leu Lys Glu Glu
           115                 120                 125
   Trp Ile Ala Tyr Ile Cys Arg Glu Ile Leu Arg Gly Leu Ser His Leu
       130                 135                 140
   His Gln His Lys Val Ile His Arg Asp Ile Lys Gly Gln Asn Val Leu
   145                 150                 155                 160
   Leu Thr Glu Asn Ala Glu Val Lys Leu Val Asp Phe Gly Val Ser Ala
                   165                 170                 175
   Gln Leu Asp Arg Thr Val Gly Arg Arg Asn Thr Phe Ile Gly Thr Pro
               180                 185                 190
   Tyr Trp Met Ala Pro Glu Val Ile Ala Cys Asp Glu Asn Pro Asp Ala
           195                 200                 205
   Thr Tyr Asp Phe Lys Ser Asp Leu Trp Ser Leu Gly Ile Thr Ala Ile
   210 215 220
   Glu Met Ala Glu Gly Ala Pro Pro Leu Cys Asp Met His Pro Met Arg
   225 230 235 240
   Ala Leu Phe Leu Ile Pro Arg Asn Pro Ala Pro Arg Leu Lys Ser Lys
   245 250 255
   Lys Trp Ser Lys Lys Phe Gln Ser Phe Ile Glu Ser Cys Leu Val Lys
   260 265 270
   Asn His Ser Gln Arg Pro Ala Thr Glu Gln Leu Met Lys His Pro Phe
   275 280 285
   Ile Arg Asp Gln Pro Asn Glu Arg Gln Val Arg Ile Gln Leu Lys Asp
   290 295 300
   His Ile Asp Arg Thr Lys Lys Lys Arg Gly Glu Lys Asp Glu Thr Glu
   305 310 315 320
   Tyr Glu Tyr Ser Gly Ser Glu Glu Glu Glu Glu Glu Asn Asp Ser Gly
   325 330 335
   Glu Pro Ser Ser Ile Leu Asn Leu Pro Gly Glu Ser Thr Leu Arg Arg
   340 345 350
   Asp Phe Leu Arg Leu Gln Leu Ala Asn Lys Glu Arg Ser Glu Ala Leu
   355 360 365
   Arg Arg Gln Gln Leu Glu Gln Gln Gln Arg Glu Asn Glu Glu His Lys
   370 375 380
   Arg Gln Leu Leu Ala Glu Arg Gln Lys Arg Ile Glu Glu Gln Lys Glu
   385 390 395 400
   Gln Arg Arg Arg Leu Glu Glu Gln Gln Arg Arg Glu Lys Glu Leu Arg
   405 410 415
   Lys Gln Gln Glu Arg Glu Gln Arg Arg His Tyr Glu Glu Gln Met Arg
   420 425 430
   Arg Glu Glu Glu Arg Arg Arg Ala Glu His Glu Gln Glu Tyr Lys Arg
   435 440 445
   Lys Gln Leu Glu Glu Gln Arg Gln Ala Glu Arg Leu Gln Arg Gln Leu
   450 455 460
   Lys Gln Glu Arg Asp Tyr Leu Val Ser Leu Gln His Gln Arg Gln Glu
   465                 470                 475                 480
   Gln Arg Pro Val Glu Lys Lys Pro Leu Tyr His Tyr Lys Glu Gly Met
                   485                 490                 495
   Ser Pro Ser Glu Lys Pro Ala Trp Ala Lys Glu Val Glu Glu Arg Ser
               500                 505                 510
   Arg Leu Asn Arg Gln Ser Ser Pro Ala Met Pro His Lys Val Ala Asn
           515                 520                 525
   Arg Ile Ser Asp Pro Asn Leu Pro Pro Arg Ser Glu Ser Phe Ser Ile
       530                 535                 540
   Ser Gly Val Gln Pro Ala Arg Thr Pro Pro Met Leu Arg Pro Val Asp
   545                 550                 555                 560
   Pro Gln Ile Pro His Leu Val Ala Val Lys Ser Gln Gly Pro Ala Leu
                   565                 570                 575
   Thr Ala Ser Gln Ser Val His Glu Gln Pro Thr Lys Gly Leu Ser Gly
               580                 585                 590
   Phe Gln Glu Ala Leu Asn Val Thr Ser His Arg Val Glu Met Pro Arg
           595                 600                 605
   Gln Asn Ser Asp Pro Thr Ser Glu Asn Pro Pro Leu Pro Thr Arg Ile
       610                 615                 620
   Glu Lys Phe Asp Arg Ser Ser Trp Leu Arg Gln Glu Glu Asp Ile Pro
   625                 630                 635                 640
   Pro Lys Val Pro Gln Arg Thr Thr Ser Ile Ser Pro Ala Leu Ala Arg
                   645                 650                 655
   Lys Asn Ser Pro Gly Asn Gly Ser Ala Leu Gly Pro Arg Leu Gly Ser
               660                 665                 670
   Gln Pro Ile Arg Ala Ser Asn Pro Asp Leu Arg Arg Thr Glu Pro Ile
           675                 680                 685
   Leu Glu Ser Pro Leu Gln Arg Thr Ser Ser Gly Ser Ser Ser Ser Ser
       690                 695                 700
   Ser Thr Pro Ser Ser Gln Pro Ser Ser Gln Gly Gly Ser Gln Pro Gly
   705                 710                 715                 720
   Ser Gln Ala Gly Ser Ser Glu Arg Thr Arg Val Arg Ala Asn Ser Lys
                   725                 730                 735
   Ser Glu Gly Ser Pro Val Leu Pro His Glu Pro Ala Lys Val Lys Pro
               740                 745                 750
   Glu Glu Ser Arg Asp Ile Thr Arg Pro Ser Arg Pro Ala Ser Tyr Lys
           755                 760                 765
   Lys Ala Ile Asp Glu Asp Leu Thr Ala Leu Ala Lys Glu Leu Arg Glu
       770                 775                 780
   Leu Arg Ile Glu Glu Thr Asn Arg Pro Met Lys Lys Val Thr Asp Tyr
   785                 790                 795                 800
   Ser Ser Ser Ser Glu Glu Ser Glu Ser Ser Glu Glu Glu Glu Glu Asp
                   805                 810                 815
   Gly Glu Ser Glu Thr His Asp Gly Thr Val Ala Val Ser Asp Ile Pro
               820                 825                 830
   Arg Leu Ile Pro Thr Gly Ala Pro Gly Ser Asn Glu Gln Tyr Asn Val
           835                 840                 845
   Gly Met Val Gly Thr His Gly Leu Glu Thr Ser His Ala Asp Ser Phe
       850                 855                 860
   Ser Gly Ser Ile Ser Arg Glu Gly Thr Leu Met Ile Arg Glu Thr Ser
   865                 870                 875                 880
   Gly Glu Lys Lys Arg Ser Gly His Ser Asp Ser Asn Gly Phe Ala Gly
                   885                 890                 895
   His Ile Asn Leu Pro Asp Leu Val Gln Gln Ser His Ser Pro Ala Gly
               900                 905                 910
   Thr Pro Thr Glu Gly Leu Gly Arg Val Ser Thr His Ser Gln Glu Met
           915                 920                 925
   Asp Ser Gly Thr Glu Tyr Gly Met Gly Ser Ser Thr Lys Ala Ser Phe
       930                 935                 940
   Thr Pro Phe Val Asp Pro Arg Val Tyr Gln Thr Ser Pro Thr Asp Glu
   945                 950                 955                 960
   Asp Glu Glu Asp Glu Glu Ser Ser Ala Ala Ala Leu Phe Thr Ser Glu
                   965                 970                 975
   Leu Leu Arg Gln Glu Gln Ala Lys Leu Asn Glu Ala Arg Lys Ile Ser
               980                 985                 990
   Val Val Asn Val Asn Pro Thr Asn Ile Arg Pro His Ser Asp Thr Pro
           995                 1000                1005
   Glu Ile Arg Lys Tyr Lys Lys Arg Phe Asn Ser Glu Ile Leu Cys
       1010                1015                1020
   Ala Ala Leu Trp Gly Val Asn Leu Leu Val Gly Thr Glu Asn Gly
       1025                1030                1035
   Leu Met Leu Leu Asp Arg Ser Gly Gln Gly Lys Val Tyr Asn Leu
       1040                1045                1050
   Ile Asn Arg Arg Arg Phe Gln Gln Met Asp Val Leu Glu Gly Leu
       1055                1060                1065
   Asn Val Leu Val Thr Ile Ser Gly Lys Lys Asn Lys Leu Arg Val
       1070                1075                1080
   Tyr Tyr Leu Ser Trp Leu Arg Asn Arg Ile Leu His Asn Asp Pro
       1085                1090                1095
   Glu Val Glu Lys Lys Gln Gly Trp Ile Thr Val Gly Asp Leu Glu
       1100                1105                1110
   Gly Cys Ile His Tyr Lys Val Val Lys Tyr Glu Arg Ile Lys Phe
       1115                1120                1125
   Leu Val Ile Ala Leu Lys Asn Ala Val Glu Ile Tyr Ala Trp Ala
       1130                1135                1140
   Pro Lys Pro Tyr His Lys Phe Met Ala Phe Lys Ser Phe Ala Asp
       1145                1150                1155
   Leu Gln His Lys Pro Leu Leu Val Asp Leu Thr Val Glu Glu Gly
       1160                1165                1170
   Gln Arg Leu Lys Val Ile Phe Gly Ser His Thr Gly Phe His Val
       1175                1180                1185
   Ile Asp Val Asp Ser Gly Asn Ser Tyr Asp Ile Tyr Ile Pro Ser
       1190                1195                1200
   His Ile Gln Gly Asn Ile Thr Pro His Ala Ile Val Ile Leu Pro
   1205                1210                1215
   Lys Thr Asp Gly Met Glu Met Leu Val Cys Tyr Glu Asp Glu Gly
       1220                1225                1230
   Val Tyr Val Asn Thr Tyr Gly Arg Ile Thr Lys Asp Val Val Leu
       1235                1240                1245
   Gln Trp Gly Glu Met Pro Thr Ser Val Ala Tyr Ile His Ser Asn
       1250                1255                1260
   Gln Ile Met Gly Trp Gly Glu Lys Ala Ile Glu Ile Arg Ser Val
       1265                1270                1275
   Glu Thr Gly His Leu Asp Gly Val Phe Met His Lys Arg Ala Gln
       1280                1285                1290
   Arg Leu Lys Phe Leu Cys Glu Arg Asn Asp Lys Val Ile Gly His
       1295                1300                1305
   Ser Asn Phe Gly Thr Ile Arg Ser Thr Thr Cys Val Tyr Lys Gly
       1310                1315                1320
   Lys Trp Leu Tyr Glu Val Leu Ile Ser Ser Gln Gly Leu Met Gln
       1325                1330                1335
   Ile Gly Trp Cys Thr Ile Ser Cys Arg Phe Asn Gln Glu Glu Gly
       1340                1345                1350
   Val Gly Asp Thr His Asn Ser Tyr Ala Tyr Asp Gly Asn Arg Val
       1355                1360                1365
   Arg Lys Trp Asn Val Thr Thr Thr Asn Tyr Gly Lys Ala Trp Ala
       1370                1375                1380
   Ala Gly Asp Ile Val Ser Cys Leu Ile Asp Leu Asp Asp Gly Thr
       1385                1390                1395
   Leu Ser Phe Cys Leu Asn Gly Val Ser Leu Gly Thr Ala Phe Glu
       1400                1405                1410
   Asn Leu Ser Arg Gly Leu Gly Met Ala Tyr Phe Pro Ala Ile Ser
       1415                1420                1425
   Leu Ser Phe Lys Glu Ser Val Ala Phe Asn Phe Gly Ser Arg Pro
       1430                1435                1440
   Leu Arg Tyr Pro Val Ala Gly Tyr Arg Pro Leu Gln Asp Pro Pro
   1445 1450 1455
   Ser Ala Asp Leu Val Arg Ala Gln Arg Leu Leu Gly Cys Phe Arg
   1460 1465 1470
   Ala Val Leu Ser Val Glu Leu Asp Pro Val Glu Gly Arg Leu Leu
   1475 1480 1485
   Asp Lys Glu Ser Ser Lys Trp Arg Leu Arg Gly Gln Pro Thr Val
   1490 1495 1500
   Leu Leu Thr Leu Ala His Ile Phe His His Phe Ala Pro Leu Leu
   1505 1510 1515
   Arg Lys Val Tyr Leu Val Glu Ala Val Leu Met Ser Phe Leu Leu
   1520 1525 1530
   Gly Ile Val Glu Lys Gly Thr Pro Thr Gln Ala Gln Ser Val Val
   1535 1540 1545
   His Gln Val Leu Asp Leu Leu Trp Leu Phe Met Glu Asp Tyr Glu
   1550 1555 1560
   Val Gln Asp Cys Leu Lys Gln Leu Met Met Ser Leu Leu Arg Leu
   1565 1570 1575
   Tyr Arg Phe Ser Pro Ile Val Pro Asp Leu Gly Leu Gln Ile His
   1580 1585 1590
   Tyr Leu Arg Leu Thr Ile Ala Ile Leu Arg His Glu Lys Ser Arg
   1595 1600 1605
   Lys Phe Leu Leu Ser Asn Val Leu Phe Asp Val Leu Arg Ser Val
   1610 1615 1620
   Val Phe Phe Tyr Ile Lys Ser Pro Leu Arg Val Glu Glu Ala Gly
   1625 1630 1635
   Leu Gln Glu Leu Ile Pro Thr Thr Trp Trp Pro His Cys Ser Ser
   1640 1645 1650
   Arg Glu Gly Lys Glu Ser Thr Glu Met Lys Glu Glu Thr Ala Glu
   1655 1660 1665
   Glu Arg Leu Arg Arg Arg Ala Tyr Glu Arg Gly Cys Gln Arg Leu
   1670 1675 1680
   Arg Lys Arg Ile Glu Val Val Glu Glu Leu Gln Val Gln Ile Leu
   1685 1690 1695
   Lys Leu Leu Leu Asp Asn Lys Asp Asp Asn Gly Gly Glu Ala Ser
   1700 1705 1710
   Arg Tyr Ile Phe Leu Thr Lys Phe Arg Lys Phe Leu Gln Glu Asn
   1715 1720 1725
   Ala Ser Gly Arg Gly Asn Met Pro Met Leu Cys Pro Pro Glu Tyr
   1730 1735 1740
   Met Val Cys Phe Leu His Arg Leu Ile Ser Ala Leu Arg Tyr Tyr
   1745 1750 1755
   Trp Asp Glu Tyr Lys Ala Ser Asn Pro His Ala Ser Phe Ser Glu
   1760 1765 1770
   Glu Ala Tyr Ile Pro Pro Gln Val Phe Tyr Asn Gly Lys Val Asp
   1775 1780 1785
   Tyr Phe Asp Leu Gln Arg Leu Gly Gly Leu Leu Ser His Leu Arg
   1790 1795 1800
   Lys Thr Leu Lys Asp Asp Leu Ala Ser Lys Ala Asn Ile Val Ile
   1805 1810 1815
   Asp Pro Leu Glu Leu Gln Ser Thr Ala Met Asp Asp Leu Asp Glu
   1820 1825 1830
   Asp Glu Glu Pro Ala Pro Ala Met Ala Gln Arg Pro Met Gln Ala
   1835 1840 1845
   Leu Ala Val Gly Gly Pro Leu Pro Leu Pro Arg Pro Gly Trp Leu
   1850 1855 1860
   Ser Ser Pro Thr Leu Gly Arg Ala Asn Arg Phe Leu Ser Thr Ala
   1865 1870 1875
   Ala Val Ser Leu Met Thr Pro Arg Arg Pro Leu Ser Thr Ser Glu
   1880 1885 1890
   Lys Val Lys Val Arg Thr Leu Ser Val Glu Gln Arg Thr Arg Glu
   1895 1900 1905
   Asp Ile Glu Gly Ser His Trp Asn Glu Gly Leu Leu Leu Gly Arg
   1910 1915 1920
   Pro Pro Glu Glu Pro Glu Gln Pro Leu Thr Glu Asn Ser Leu Leu
       1925                1930                1935
   Glu Val Leu Asp Gly Ala Val Met Met Tyr Asn Leu Ser Val His
       1940                1945                1950
   Gln Gln Leu Gly Lys Met Val Gly Val Ser Asp Asp Val Asn Glu
       1955                1960                1965
   Tyr Ala Met Ala Leu Arg Asp Thr Glu Asp Lys Leu Arg Arg Cys
       1970                1975                1980
   Pro Lys Arg Arg Lys Asp Ile Leu Ala Glu Leu Thr Lys Ser Gln
       1985                1990                1995
   Lys Val Phe Ser Glu Lys Leu Asp His Leu Ser Arg Arg Leu Ala
       2000                2005                2010
   Trp Val His Ala Thr Val Tyr Ser Gln Glu Lys Met Leu Asp Ile
       2015                2020                2025
   Tyr Trp Leu Leu Arg Val Cys Leu Arg Thr Ile Glu His Gly Asp
       2030                2035                2040
   Arg Thr Gly Ser Leu Phe Ala Phe Met Pro Glu Phe Tyr Leu Ser
       2045                2050                2055
   Val Ala Ile Asn Ser Tyr Ser Ala Leu Lys Asn Tyr Phe Gly Pro
       2060                2065                2070
   Val His Ser Met Glu Glu Leu Pro Gly Tyr Glu Glu Thr Leu Thr
       2075                2080                2085
   Arg Leu Ala Ala Ile Leu Ala Lys His Phe Ala Asp Ala Arg Ile
       2090                2095                2100
   Val Gly Thr Asp Ile Arg Asp Ser Leu Met Gln Ala Leu Ala Ser
       2105                2110                2115
   Tyr Val Cys Tyr Pro His Ser Leu Arg Ala Val Glu Arg Ile Pro
       2120                2125                2130
   Glu Glu Gln Arg Ile Ala Met Val Arg Asn Leu Leu Ala Pro Tyr
       2135                2140                2145
   Glu Gln Arg Pro Trp Ala Gln Thr Asn Trp Ile Leu Val Arg Leu
   2150                2155                2160
   Trp Arg Gly Cys Gly Phe Gly Tyr Arg Tyr Thr Arg Leu Pro His
       2165                2170                2175
   Leu Leu Lys Thr Lys Leu Glu Asp Ala Asn Leu Pro Ser Leu Gln
       2180                2185                2190
   Lys Pro Cys Pro Ser Thr Leu Leu Gln Gln His Met Ala Asp Leu
       2195                2200                2205
   Leu Gln Gln Gly Pro Asp Val Ala Pro Ser Phe Leu Asn Ser Val
       2210                2215                2220
   Leu Asn Gln Leu Asn Trp Ala Phe Ser Glu Phe Ile Gly Met Ile
       2225                2230                2235
   Gln Glu Ile Gln Gln Ala Ala Glu Arg Leu Glu Arg Asn Phe Val
       2240                2245                2250
   Asp Ser Arg Gln Leu Lys Val Cys Ala Thr Cys Phe Asp Leu Ser
       2255                2260                2265
   Val Ser Leu Leu Arg Val Leu Glu Met Thr Ile Thr Leu Val Pro
       2270                2275                2280
   Glu Ile Phe Leu Asp Trp Thr Arg Pro Thr Ser Glu Met Leu Leu
       2285                2290                2295
   Arg Arg Leu Ala Gln Leu Leu Asn Gln Val Leu Asn Arg Val Thr
       2300                2305                2310
   Ala Glu Arg Asn Leu Phe Asp Arg Val Val Thr Leu Arg Leu Pro
       2315                2320                2325
   Gly Leu Glu Ser Val Asp His Tyr Pro Ile Leu Val Ala Val Thr
       2330                2335                2340
   Gly Ile Leu Val Gln Leu Leu Val Arg Gly Pro Ala Ser Glu Arg
       2345                2350                2355
   Glu Gln Ala Thr Ser Val Leu Leu Ala Asp Pro Cys Phe Gln Leu
       2360                2365                2370
   Arg Ser Ile Cys Tyr Leu Leu Gly Gln Pro Glu Pro Pro Ala Pro
       2375                2380                2385
   Gly Thr Ala Leu Pro Ala Pro Asp Arg Lys Arg Phe Ser Leu Gln
       2390                2395                2400
   Ser Tyr Ala Asp Tyr Ile Ser Ala Asp Glu Leu Ala Gln Val Glu
       2405                2410                2415
   Gln Met Leu Ala His Leu Thr Ser Ala Ser Ala Gln Ala Ala Ala
       2420                2425                2430
   Ala Ser Leu Pro Thr Ser Glu Glu Asp Leu Cys Pro Ile Cys Tyr
       2435                2440                2445
   Ala His Pro Ile Ser Ala Val Phe Gln Pro Cys Gly His Lys Ser
       2450                2455                2460
   Cys Lys Ala Cys Ile Asn Gln His Leu Met Asn Asn Lys Asp Cys
       2465                2470                2475
   Phe Phe Cys Lys Thr Thr Ile Val Ser Val Glu Asp Trp Glu Lys
       2480                2485                2490
   Gly Ala Asn Thr Ser Thr Thr Ser Ser Ala Ala
       2495                2500

<210> 9
<211> 7491
<212> DNA
<213> Homo sapiens

<220>
<221> CDS
<222> (1)..(7491)

<400> 9
atg gcg agc gac tcc ccg gct cga agc ctg gat gaa ata gat ctc tcg 48
Met Ala Ser Asp Ser Pro Ala Arg Ser Leu Asp Glu Ile Asp Leu Ser
1               5                   10                  15

gct ctg agg gac ccc gca ggg atc ttt gaa ttg gtg gaa ctt gtt gga 96
Ala Leu Arg Asp Pro Ala Gly Ile Phe Glu Leu Val Glu Leu Val Gly
            20                  25                  30

aat gga aca tac ggg caa gtt tat aag ggt cgt cat gtc aaa acg ggc 144
Asn Gly Thr Tyr Gly Gln Val Tyr Lys Gly Arg His Val Lys Thr Gly
        35                  40                  45

cag ctt gca gcc atc aag gtt atg gat gtc aca ggg gat gaa gag gaa 192
Gln Leu Ala Ala Ile Lys Val Met Asp Val Thr Gly Asp Glu Glu Glu
    50                  55                  60

gaa atc aaa caa gaa att aac atg ttg aag aaa tat tct cat cac cgg 240
Glu Ile Lys Gln Glu Ile Asn Met Leu Lys Lys Tyr Ser His His Arg
65                  70                  75                  80

aat att gct aca tac tat ggt gct ttt atc aaa aag aac cca cca ggc 288
Asn Ile Ala Thr Tyr Tyr Gly Ala Phe Ile Lys Lys Asn Pro Pro Gly
                85                  90                  95

atg gat gac caa ctt tgg ttg gtg atg gag ttt tgt ggt gct ggc tct 336
Met Asp Asp Gln Leu Trp Leu Val Met Glu Phe Cys Gly Ala Gly Ser
            100                 105                 110

gtc acc gac ctg atc aag aac aca aaa ggt aac acg ttg aaa gag gag 384
Val Thr Asp Leu Ile Lys Asn Thr Lys Gly Asn Thr Leu Lys Glu Glu
        115                 120                 125

tgg att gca tac atc tgc agg gaa atc tta cgg ggg ctg agt cac ctg 432
Trp Ile Ala Tyr Ile Cys Arg Glu Ile Leu Arg Gly Leu Ser His Leu
    130                 135                 140

cac cag cat aaa gtg att cat cga gat att aaa ggg caa aat gtc ttg 480
His Gln His Lys Val Ile His Arg Asp Ile Lys Gly Gln Asn Val Leu
145                 150                 155                 160

ctg act gaa aat gca gaa gtt aaa cta gtg gac ttt gga gtc agt gct 528
Leu Thr Glu Asn Ala Glu Val Lys Leu Val Asp Phe Gly Val Ser Ala
                165                 170                 175

cag ctt gat cga aca gtg ggc agg agg aat act ttc att gga act ccc 576
Gln Leu Asp Arg Thr Val Gly Arg Arg Asn Thr Phe Ile Gly Thr Pro
            180                 185                 190

tac tgg atg gca cca gaa gtt att gcc tgt gat gaa aac cca gat gcc 624
Tyr Trp Met Ala Pro Glu Val Ile Ala Cys Asp Glu Asn Pro Asp Ala
        195                 200                 205

aca tat gat ttc aag agt gac ttg tgg tct ttg ggt atc acc gcc att 672
Thr Tyr Asp Phe Lys Ser Asp Leu Trp Ser Leu Gly Ile Thr Ala Ile
    210                 215                 220

gaa atg gca gaa ggt gct ccc cct ctc tgt gac atg cac ccc atg aga 720
Glu Met Ala Glu Gly Ala Pro Pro Leu Cys Asp Met His Pro Met Arg
225                 230                 235                 240

gct ctc ttc ctc atc ccc cgg aac cca gcg cct cgg ctg aag tct aag 768
Ala Leu Phe Leu Ile Pro Arg Asn Pro Ala Pro Arg Leu Lys Ser Lys
                245                 250                 255

aag tgg tca aaa aaa ttc cag tca ttt att gag agc tgc ttg gta aag 816
Lys Trp Ser Lys Lys Phe Gln Ser Phe Ile Glu Ser Cys Leu Val Lys
            260                 265                 270

aat cac agc cag cga cca gca aca gaa caa ttg atg aag cat cca ttt 864
Asn His Ser Gln Arg Pro Ala Thr Glu Gln Leu Met Lys His Pro Phe
        275                 280                 285

ata cga gac caa cct aat gag cga cag gtc cgc att caa ctc aag gac 912
Ile Arg Asp Gln Pro Asn Glu Arg Gln Val Arg Ile Gln Leu Lys Asp
    290                 295                 300

cat att gat aga aca aag aag aag cga gga gaa aaa gat gag aca gag 960
His Ile Asp Arg Thr Lys Lys Lys Arg Gly Glu Lys Asp Glu Thr Glu
305                 310                 315                 320

tat gag tac agt gga agt gag gaa gaa gag gag gag aat gac tca gga 1008
Tyr Glu Tyr Ser Gly Ser Glu Glu Glu Glu Glu Glu Asn Asp Ser Gly
                325                 330                 335

gag ccc agc tcc atc ctg aat ctg cca ggg gag tcg acg ctg cgg agg 1056
Glu Pro Ser Ser Ile Leu Asn Leu Pro Gly Glu Ser Thr Leu Arg Arg
            340                 345                 350

gac ttt ctg agg ctg cag ctg gcc aac aag gag cgt tct gag gcc cta 1104
Asp Phe Leu Arg Leu Gln Leu Ala Asn Lys Glu Arg Ser Glu Ala Leu
        355                 360                 365

cgg agg cag cag ctg gag cag cag cag cgg gag aat gag gag cac aag 1152
Arg Arg Gln Gln Leu Glu Gln Gln Gln Arg Glu Asn Glu Glu His Lys
    370                 375                 380

cgg cag ctg ctg gcc gag cgt cag aag cgc atc gag gag cag aaa gag 1200
Arg Gln Leu Leu Ala Glu Arg Gln Lys Arg Ile Glu Glu Gln Lys Glu
385                 390                 395                 400

cag agg cgg cgg ctg gag gag caa caa agg cga gag aag gag ctg cgg 1248
Gln Arg Arg Arg Leu Glu Glu Gln Gln Arg Arg Glu Lys Glu Leu Arg
                405                 410                 415

aag cag cag gag agg gag cag cgc cgg cac tat gag gag cag atg cgc 1296
Lys Gln Gln Glu Arg Glu Gln Arg Arg His Tyr Glu Glu Gln Met Arg
            420                 425                 430

cgg gag gag gag agg agg cgt gcg gag cat gaa cag gaa tat aag cgc 1344
Arg Glu Glu Glu Arg Arg Arg Ala Glu His Glu Gln Glu Tyr Lys Arg
        435                 440                 445

aaa caa ttg gaa gaa cag aga caa gca gaa aga ctg cag agg cag cta 1392
Lys Gln Leu Glu Glu Gln Arg Gln Ala Glu Arg Leu Gln Arg Gln Leu
    450                 455                 460

aag caa gaa aga gac tac tta gtt tcc ctt cag cat cag cgg cag gag 1440
Lys Gln Glu Arg Asp Tyr Leu Val Ser Leu Gln His Gln Arg Gln Glu
465                 470                 475                 480

cag agg cct gtg gag aag aag cca ctg tac cat tac aaa gaa gga atg 1488
Gln Arg Pro Val Glu Lys Lys Pro Leu Tyr His Tyr Lys Glu Gly Met
                485                 490                 495

agt cct agt gag aag cca gca tgg gcc aag gag gta gaa gaa cgg tca 1536
Ser Pro Ser Glu Lys Pro Ala Trp Ala Lys Glu Val Glu Glu Arg Ser
            500                 505                 510

agg ctc aac cgg caa agt tcc cct gcc atg cct cac aag gtt gcc aac 1584
Arg Leu Asn Arg Gln Ser Ser Pro Ala Met Pro His Lys Val Ala Asn
        515                 520                 525

agg ata tct gac ccc aac ctg ccc cca agg tcg gag tcc ttc agc att 1632
Arg Ile Ser Asp Pro Asn Leu Pro Pro Arg Ser Glu Ser Phe Ser Ile
    530                 535                 540

agt gga gtt cag cct gct cga aca ccc ccc atg ctc aga cca gtc gat 1680
Ser Gly Val Gln Pro Ala Arg Thr Pro Pro Met Leu Arg Pro Val Asp
545                 550                 555                 560

ccc cag atc cca cat ctg gta gct gta aaa tcc cag gga cct gcc ttg 1728
Pro Gln Ile Pro His Leu Val Ala Val Lys Ser Gln Gly Pro Ala Leu
                565                 570                 575

acc gcc tcc cag tca gtg cac gag cag ccc aca aag ggc ctc tct ggg 1776
Thr Ala Ser Gln Ser Val His Glu Gln Pro Thr Lys Gly Leu Ser Gly

580                 585                 590

ttt cag gag gct ctg aac gtg acc tcc cac cgc gtg gag atg cca cgc      1824
Phe Gln Glu Ala Leu Asn Val Thr Ser His Arg Val Glu Met Pro Arg
        595                 600                 605

cag aac tca gat ccc acc tcg gaa aat cct cct ctc ccc act cgc att      1872
Gln Asn Ser Asp Pro Thr Ser Glu Asn Pro Pro Leu Pro Thr Arg Ile
    610                 615                 620

gaa aag ttt gac cga agc tct tgg tta cga cag gaa gaa gac att cca      1920
Glu Lys Phe Asp Arg Ser Ser Trp Leu Arg Gln Glu Glu Asp Ile Pro
625                 630                 635                 640

cca aag gtg cct caa aga aca act tct ata tcc cca gca tta gcc aga      1968
Pro Lys Val Pro Gln Arg Thr Thr Ser Ile Ser Pro Ala Leu Ala Arg
                645                 650                 655

aag aat tct cct ggg aat ggt agt gct ctg gga ccc aga cta gga tct      2016
Lys Asn Ser Pro Gly Asn Gly Ser Ala Leu Gly Pro Arg Leu Gly Ser
            660                 665                 670

caa ccc atc aga gca agc aac cct gat ctc cgg aga act gag ccc atc      2064
Gln Pro Ile Arg Ala Ser Asn Pro Asp Leu Arg Arg Thr Glu Pro Ile
        675                 680                 685

ttg gag agc ccc ttg cag agg acc agc agt ggc agt tcc tcc agc tcc      2112
Leu Glu Ser Pro Leu Gln Arg Thr Ser Ser Gly Ser Ser Ser Ser Ser
    690                 695                 700

agc acc cct agc tcc cag ccc agc tcc caa gga ggc tcc cag cct gga      2160
Ser Thr Pro Ser Ser Gln Pro Ser Ser Gln Gly Gly Ser Gln Pro Gly
705                 710                 715                 720

tca caa gca gga tcc agt gaa cgc acc aga gtt cga gcc aac agt aag      2208
Ser Gln Ala Gly Ser Ser Glu Arg Thr Arg Val Arg Ala Asn Ser Lys
                725                 730                 735

tca gaa gga tca cct gtg ctc ccc cat gag cct gcc aag gtg aaa cca      2256
Ser Glu Gly Ser Pro Val Leu Pro His Glu Pro Ala Lys Val Lys Pro
            740                 745                 750

gaa gaa tcc agg gac att acc cgg ccc agt cga cca gct gat ctg acg      2304
Glu Glu Ser Arg Asp Ile Thr Arg Pro Ser Arg Pro Ala Asp Leu Thr
        755                 760                 765

gca tta gcc aaa gaa cta aga gaa ctc cgg att gaa gaa aca aac cgc      2352
Ala Leu Ala Lys Glu Leu Arg Glu Leu Arg Ile Glu Glu Thr Asn Arg
    770                 775                 780

cca atg aag aag gtg act gat tac tcc tcc tcc agt gag gag tca gaa      2400
Pro Met Lys Lys Val Thr Asp Tyr Ser Ser Ser Ser Glu Glu Ser Glu
785                 790                 795                 800

agt agc gag gaa gag gag gaa gat gga gag agc gag acc cat gat ggg      2448
Ser Ser Glu Glu Glu Glu Glu Asp Gly Glu Ser Glu Thr His Asp Gly
                805                 810                 815

aca gtg gct gtc agc gac ata ccc aga ctg ata cca aca gga gct cca      2496
Thr Val Ala Val Ser Asp Ile Pro Arg Leu Ile Pro Thr Gly Ala Pro
            820                 825                 830

ggc agc aac gag cag tac aat gtg gga atg gtg ggg acg cat ggg ctg      2544

Gly Ser Asn Glu Gln Tyr Asn Val Gly Met Val Gly Thr His Gly Leu
        835                 840                 845

gag acc tct cat gcg gac agt ttc agc ggc agt att tca aga gaa gga 2592
Glu Thr Ser His Ala Asp Ser Phe Ser Gly Ser Ile Ser Arg Glu Gly
    850                 855                 860

acc ttg atg att aga gag acg tct gga gag aag aag cga tct ggc cac 2640
Thr Leu Met Ile Arg Glu Thr Ser Gly Glu Lys Lys Arg Ser Gly His
865                 870                 875                 880

agt gac agc aat ggc ttt gct ggc cac atc aac ctc cct gac ctg gtg 2688
Ser Asp Ser Asn Gly Phe Ala Gly His Ile Asn Leu Pro Asp Leu Val
                885                 890                 895

cag cag agc cat tct cca gct gga acc ccg act gag gga ctg ggg cgc 2736
Gln Gln Ser His Ser Pro Ala Gly Thr Pro Thr Glu Gly Leu Gly Arg
            900                 905                 910

gtc tca acc cat tcc cag gag atg gac tct ggg act gaa tat ggc atg 2784
Val Ser Thr His Ser Gln Glu Met Asp Ser Gly Thr Glu Tyr Gly Met
        915                 920                 925

ggg agc agc acc aaa gcc tcc ttc acc ccc ttt gtg gac ccc aga gta 2832
Gly Ser Ser Thr Lys Ala Ser Phe Thr Pro Phe Val Asp Pro Arg Val
    930                 935                 940

tac cag acg tct ccc act gat gaa gat gaa gag gat gag gaa tca tca 2880
Tyr Gln Thr Ser Pro Thr Asp Glu Asp Glu Glu Asp Glu Glu Ser Ser
945                 950                 955                 960

gcc gca gct ctg ttt act agc gaa ctt ctt agg caa gaa cag gcc aaa 2928
Ala Ala Ala Leu Phe Thr Ser Glu Leu Leu Arg Gln Glu Gln Ala Lys
                965                 970                 975

ctc aat gaa gca aga aag att tcg gtg gta aat gta aac cca acc aac 2976
Leu Asn Glu Ala Arg Lys Ile Ser Val Val Asn Val Asn Pro Thr Asn
            980                 985                 990

att cgg cct cat agc gac aca cca gaa atc aga aaa tac aag aaa cga 3024
Ile Arg Pro His Ser Asp Thr Pro Glu Ile Arg Lys Tyr Lys Lys Arg
        995                 1000                1005

ttc aac tca gaa ata ctt tgt gca gct ctg tgg ggt gta aac ctt 3069
Phe Asn Ser Glu Ile Leu Cys Ala Ala Leu Trp Gly Val Asn Leu
    1010                1015                1020

ctg gtg ggg act gaa aat ggc ctg atg ctt ttg gac cga agt ggg 3114
Leu Val Gly Thr Glu Asn Gly Leu Met Leu Leu Asp Arg Ser Gly
    1025                1030                1035

caa ggc aaa gtc tat aat ctg atc aac cgg agg cga ttt cag cag 3159
Gln Gly Lys Val Tyr Asn Leu Ile Asn Arg Arg Arg Phe Gln Gln
    1040                1045                1050

atg gat gtg cta gag gga ctg aat gtc ctt gtg aca att tca gga 3204
Met Asp Val Leu Glu Gly Leu Asn Val Leu Val Thr Ile Ser Gly
    1055                1060                1065

aag aag aat aag cta cga gtt tac tat ctt tca tgg tta aga aac 3249
Lys Lys Asn Lys Leu Arg Val Tyr Tyr Leu Ser Trp Leu Arg Asn
    1070                1075                1080

aga ata cta cat aat gac cca gaa gta gaa aag aaa caa ggc tgg 3294
Arg Ile Leu His Asn Asp Pro Glu Val Glu Lys Lys Gln Gly Trp
    1085                1090                1095

atc act gtt ggg gac ttg gaa ggc tgt ata cat tat aaa gtt gtt 3339
Ile Thr Val Gly Asp Leu Glu Gly Cys Ile His Tyr Lys Val Val
    1100                1105                1110

aaa tat gaa agg atc aaa ttt ttg gtg att gcc tta aag aat gct 3384
Lys Tyr Glu Arg Ile Lys Phe Leu Val Ile Ala Leu Lys Asn Ala
    1115                1120                1125

gtg gaa ata tat gct tgg gct cct aaa ccg tat cat aaa ttc atg 3429
Val Glu Ile Tyr Ala Trp Ala Pro Lys Pro Tyr His Lys Phe Met
    1130                1135                1140

gca ttt aag tct ttt gca gat ctc cag cac aag cct ctg cta gtt 3474
Ala Phe Lys Ser Phe Ala Asp Leu Gln His Lys Pro Leu Leu Val
    1145                1150                1155

gat ctc acg gta gaa gaa ggt caa aga tta aag gtt att ttt ggt 3519
Asp Leu Thr Val Glu Glu Gly Gln Arg Leu Lys Val Ile Phe Gly
    1160                1165                1170

tca cac act ggt ttc cat gta att gat gtt gat tca gga aac tct 3564
Ser His Thr Gly Phe His Val Ile Asp Val Asp Ser Gly Asn Ser
    1175                1180                1185

tat gat atc tac ata cca tct cat att cag ggc aat atc act cct 3609
Tyr Asp Ile Tyr Ile Pro Ser His Ile Gln Gly Asn Ile Thr Pro
    1190                1195                1200

cat gct att gtc atc ttg cct aaa aca gat gga atg gaa atg ctt 3654
His Ala Ile Val Ile Leu Pro Lys Thr Asp Gly Met Glu Met Leu
    1205                1210                1215

gtt tgc tat gag gat gag ggg gtg tat gta aac acc tat ggc cgg 3699
Val Cys Tyr Glu Asp Glu Gly Val Tyr Val Asn Thr Tyr Gly Arg
    1220                1225                1230

ata act aag gat gtg gtg ctc caa tgg gga gaa atg ccc acg tct 3744
Ile Thr Lys Asp Val Val Leu Gln Trp Gly Glu Met Pro Thr Ser
    1235                1240                1245

gtg gcc tac att cat tcc aat cag ata atg ggc tgg ggc gag aaa 3789
Val Ala Tyr Ile His Ser Asn Gln Ile Met Gly Trp Gly Glu Lys
    1250                1255                1260

gct att gag atc cgg tca gtg gaa aca gga cat ttg gat gga gta 3834
Ala Ile Glu Ile Arg Ser Val Glu Thr Gly His Leu Asp Gly Val
    1265                1270                1275

ttt atg cat aag cga gct caa agg tta aag ttt cta tgt gaa aga 3879
Phe Met His Lys Arg Ala Gln Arg Leu Lys Phe Leu Cys Glu Arg
    1280                1285                1290

aat gat aag gtg att gga cac agc aac ttt ggc acc atc cgc tct 3924
Asn Asp Lys Val Ile Gly His Ser Asn Phe Gly Thr Ile Arg Ser
    1295                1300                1305

acc aca tgc gtg tac aaa ggg aaa tgg ctc tac gag gtc ctc atc 3969
Thr Thr Cys Val Tyr Lys Gly Lys Trp Leu Tyr Glu Val Leu Ile
    1310                1315                1320

tcc tcc cag ggg ctc atg cag atc ggc tgg tgc acc atc agc tgc 4014
Ser Ser Gln Gly Leu Met Gln Ile Gly Trp Cys Thr Ile Ser Cys
    1325                1330                1335

cgc ttc aac cag gag gag ggg gtt gga gat aca cac aac tcc tat 4059
Arg Phe Asn Gln Glu Glu Gly Val Gly Asp Thr His Asn Ser Tyr
    1340                1345                1350

gcc tat gat ggc aac cgc gtg cgc aag tgg aat gtg acc aca acg 4104
Ala Tyr Asp Gly Asn Arg Val Arg Lys Trp Asn Val Thr Thr Thr
    1355                1360                1365

aat tat ggc aag gcg tgg gca gcg ggg gac atc gtg agc tgc ctg 4149
Asn Tyr Gly Lys Ala Trp Ala Ala Gly Asp Ile Val Ser Cys Leu
    1370                1375                1380

att gac ctg gat gat ggc act ctg tcc ttc tgc ctg aac ggt gta 4194
Ile Asp Leu Asp Asp Gly Thr Leu Ser Phe Cys Leu Asn Gly Val
    1385                1390                1395

tca ctg ggc act gcc ttt gag aac ctg tcc agg ggc ctg ggt atg 4239
Ser Leu Gly Thr Ala Phe Glu Asn Leu Ser Arg Gly Leu Gly Met
    1400                1405                1410

gcc tac ttc cca gcc atc agc ctc tct ttc aag gag tcc gtg gcc 4284
Ala Tyr Phe Pro Ala Ile Ser Leu Ser Phe Lys Glu Ser Val Ala
    1415                1420                1425

ttc aac ttt ggc agc cgt cct ctg cgc tac cca gtg gca ggc tac 4329
Phe Asn Phe Gly Ser Arg Pro Leu Arg Tyr Pro Val Ala Gly Tyr
    1430                1435                1440

cgg ccc ctg cag gac cca ccg agt gct gac ctg gtg cgg gca cag 4374
Arg Pro Leu Gln Asp Pro Pro Ser Ala Asp Leu Val Arg Ala Gln
    1445                1450                1455

agg ttg ctg ggc tgc ttc cgg gca gtg ctg agt gtg gag ctg gac 4419
Arg Leu Leu Gly Cys Phe Arg Ala Val Leu Ser Val Glu Leu Asp
    1460                1465                1470

cct gtg gag ggg cgg ctg ttg gac aag gag agc tcc aag tgg cgg 4464
Pro Val Glu Gly Arg Leu Leu Asp Lys Glu Ser Ser Lys Trp Arg
    1475                1480                1485

ttg cgg ggc cag ccc acc gtc ctc ctc aca ctg gcc cac atc ttc 4509
Leu Arg Gly Gln Pro Thr Val Leu Leu Thr Leu Ala His Ile Phe
    1490                1495                1500

cat cac ttt gca ccg ctt ctg cgc aag gtg tat ctg gtg gag gct 4554
His His Phe Ala Pro Leu Leu Arg Lys Val Tyr Leu Val Glu Ala
    1505                1510                1515

gtg ctc atg agc ttc ttg ctg ggc atc gtg gag aag ggc aca ccc 4599
Val Leu Met Ser Phe Leu Leu Gly Ile Val Glu Lys Gly Thr Pro
    1520                1525                1530

aca cag gca cag tcc gtg gtg cac cag gtc ctg gac ctc ttg tgg 4644
Thr Gln Ala Gln Ser Val Val His Gln Val Leu Asp Leu Leu Trp
    1535                1540                1545

ctc ttc atg gag gac tac gag gta caa gat tgc ctc aag cag ttg 4689
Leu Phe Met Glu Asp Tyr Glu Val Gln Asp Cys Leu Lys Gln Leu

1550                    1555                    1560

atg atg  tct ctg ctt cgg ctg  tac cga ttc tca ccc  att gtc cca      4734
Met Met  Ser Leu Leu Arg Leu  Tyr Arg Phe Ser Pro  Ile Val Pro
    1565                    1570                    1575

gac ctg  ggc cta cag atc cat  tac ctg cgg ctc act  atc gcc atc      4779
Asp Leu  Gly Leu Gln Ile His  Tyr Leu Arg Leu Thr  Ile Ala Ile
    1580                    1585                    1590

ctg agg  cat gag aag tcc cgc  aag ttt ctg ctt agc  aat gtc ctc      4824
Leu Arg  His Glu Lys Ser Arg  Lys Phe Leu Leu Ser  Asn Val Leu
    1595                    1600                    1605

ttc gac  gtg ctc cgc tcc gtc  gtc ttc ttt tac atc  aag agc ccc      4869
Phe Asp  Val Leu Arg Ser Val  Val Phe Phe Tyr Ile  Lys Ser Pro
    1610                    1615                    1620

ctg cgt  gtg gag gag gcc ggc  ctg cag gag ctc att  ccc acc acc      4914
Leu Arg  Val Glu Glu Ala Gly  Leu Gln Glu Leu Ile  Pro Thr Thr
    1625                    1630                    1635

tgg tgg  ccc cac tgc tcc agt  agg gag ggc aaa gag  agc acg gag      4959
Trp Trp  Pro His Cys Ser Ser  Arg Glu Gly Lys Glu  Ser Thr Glu
    1640                    1645                    1650

atg aag  gag gag acc gca gag  gag cgg ctg cgg cgg  cga gcc tac      5004
Met Lys  Glu Glu Thr Ala Glu  Glu Arg Leu Arg Arg  Arg Ala Tyr
    1655                    1660                    1665

gaa cgg  ggc tgt cag cgg ctc  agg aag cgc atc gaa  gtg gtg gaa      5049
Glu Arg  Gly Cys Gln Arg Leu  Arg Lys Arg Ile Glu  Val Val Glu
    1670                    1675                    1680

gaa cta  cag gtc cag atc ctg  aag ctg ctg ctg gac  aat aaa gat      5094
Glu Leu  Gln Val Gln Ile Leu  Lys Leu Leu Leu Asp  Asn Lys Asp
    1685                    1690                    1695

gac aat  ggg ggt gaa gct tct  agg tat atc ttc ctg  acc aag ttt      5139
Asp Asn  Gly Gly Glu Ala Ser  Arg Tyr Ile Phe Leu  Thr Lys Phe
    1700                    1705                    1710

cgc aag  ttt ctg cag gag aac  gcc agt ggc cgg ggg  aac atg ccc      5184
Arg Lys  Phe Leu Gln Glu Asn  Ala Ser Gly Arg Gly  Asn Met Pro
    1715                    1720                    1725

atg ctc  tgc ccc cct gag tac  atg gtc tgc ttc tta  cac cgg ctg      5229
Met Leu  Cys Pro Pro Glu Tyr  Met Val Cys Phe Leu  His Arg Leu
    1730                    1735                    1740

atc tct  gcc ctg cgc tac tat  tgg gat gaa tac aag  gct tcc aat      5274
Ile Ser  Ala Leu Arg Tyr Tyr  Trp Asp Glu Tyr Lys  Ala Ser Asn
    1745                    1750                    1755

cct cat  gct tcc ttc agt gag  gag gcc tac atc ccg  ccc cag gtc      5319
Pro His  Ala Ser Phe Ser Glu  Glu Ala Tyr Ile Pro  Pro Gln Val
    1760                    1765                    1770

ttc tat  aat ggc aag gtg gac  tac ttt gac ctg cag  cgc ctg ggg      5364
Phe Tyr  Asn Gly Lys Val Asp  Tyr Phe Asp Leu Gln  Arg Leu Gly
    1775                    1780                    1785

ggc ctc  ctc tcg cac ctg cgg  aag acc ctc aaa gat  gac ctt gct      5409

Gly Leu Leu Ser His Leu Arg Lys Thr Leu Lys Asp Asp Leu Ala
    1790                1795                1800

tcc aaa gcc aac att gtg atc gac cca ctg gag ctc cag tca acc 5454
Ser Lys Ala Asn Ile Val Ile Asp Pro Leu Glu Leu Gln Ser Thr
    1805                1810                1815

gcc atg gat gac cta gat gag gat gag gag cca gcc cca gct atg 5499
Ala Met Asp Asp Leu Asp Glu Asp Glu Glu Pro Ala Pro Ala Met
    1820                1825                1830

gcc cag cgc ccc atg cag gcc ctg gct gtt ggg ggg cca ctg ccc 5544
Ala Gln Arg Pro Met Gln Ala Leu Ala Val Gly Gly Pro Leu Pro
    1835                1840                1845

ctg ccc cgg ccc ggc tgg ctc agt tct cca act ttg ggc cga gcc 5589
Leu Pro Arg Pro Gly Trp Leu Ser Ser Pro Thr Leu Gly Arg Ala
    1850                1855                1860

aac cgc ttc ctc agc aca gcg gct gtg agc ctc atg acc cca cgg 5634
Asn Arg Phe Leu Ser Thr Ala Ala Val Ser Leu Met Thr Pro Arg
    1865                1870                1875

cgg cct ctg agc acc tcg gag aaa gtg aag gtc cgc acg ctg agc 5679
Arg Pro Leu Ser Thr Ser Glu Lys Val Lys Val Arg Thr Leu Ser
    1880                1885                1890

gtg gag cag agg acc cgt gag gac att gaa ggc agc cac tgg aat 5724
Val Glu Gln Arg Thr Arg Glu Asp Ile Glu Gly Ser His Trp Asn
    1895                1900                1905

gag ggc ttg ctg ctg ggg cgg ccc ccc gag gag cct gag cag ccc 5769
Glu Gly Leu Leu Leu Gly Arg Pro Pro Glu Glu Pro Glu Gln Pro
    1910                1915                1920

ctc acc gag aac tcg ctg ctg gaa gtc ctg gat ggg gcg gtc atg 5814
Leu Thr Glu Asn Ser Leu Leu Glu Val Leu Asp Gly Ala Val Met
    1925                1930                1935

atg tac aac ctc agc gta cac cag cag ctg ggc aag atg gtg ggt 5859
Met Tyr Asn Leu Ser Val His Gln Gln Leu Gly Lys Met Val Gly
    1940                1945                1950

gtc tcc gat gat gtc aat gaa tac gct atg gct ctg agg gac aca 5904
Val Ser Asp Asp Val Asn Glu Tyr Ala Met Ala Leu Arg Asp Thr
    1955                1960                1965

gag gac aag ctc cgc cgg tgc ccc aag agg agg aag gac atc ctt 5949
Glu Asp Lys Leu Arg Arg Cys Pro Lys Arg Arg Lys Asp Ile Leu
    1970                1975                1980

gca gag ttg acc aag agc cag aag gtt ttc tca gaa aag ctg gac 5994
Ala Glu Leu Thr Lys Ser Gln Lys Val Phe Ser Glu Lys Leu Asp
    1985                1990                1995

cac ctg agc cgc cgt ctt gcc tgg gtc cat gcc act gtc tac tcc 6039
His Leu Ser Arg Arg Leu Ala Trp Val His Ala Thr Val Tyr Ser
    2000                2005                2010

cag gag aag atg ctg gac atc tac tgg ctg ctg cgc gtc tgc ctg 6084
Gln Glu Lys Met Leu Asp Ile Tyr Trp Leu Leu Arg Val Cys Leu
    2015                2020                2025

cgg acc att gag cac ggt gat cgc aca ggg tct ctc ttt gcc ttc 6129
Arg Thr Ile Glu His Gly Asp Arg Thr Gly Ser Leu Phe Ala Phe
    2030                2035                2040

atg ccc gag ttc tac ctg agc gtg gcc atc aac agc tac agt gct 6174
Met Pro Glu Phe Tyr Leu Ser Val Ala Ile Asn Ser Tyr Ser Ala
    2045                2050                2055

ctc aag aat tac ttt ggt ccc gtg cac agc atg gag gag ctc cca 6219
Leu Lys Asn Tyr Phe Gly Pro Val His Ser Met Glu Glu Leu Pro
    2060                2065                2070

ggc tat gaa gag acc ctg acc cgc ctg gct gcc att ctc gcc aaa 6264
Gly Tyr Glu Glu Thr Leu Thr Arg Leu Ala Ala Ile Leu Ala Lys
    2075                2080                2085

cac ttt gcc gac gca cgc att gtg ggc act gac atc cga gac tca 6309
His Phe Ala Asp Ala Arg Ile Val Gly Thr Asp Ile Arg Asp Ser
    2090                2095                2100

ctg atg cag gcc ctg gcc agc tac gtg tgc tac cca cac tcc ctg 6354
Leu Met Gln Ala Leu Ala Ser Tyr Val Cys Tyr Pro His Ser Leu
    2105                2110                2115

cgg gct gtg gag cga atc ccc gag gag cag cgt atc gcc atg gtg 6399
Arg Ala Val Glu Arg Ile Pro Glu Glu Gln Arg Ile Ala Met Val
    2120                2125                2130

agg aac ctc ctg gcg ccc tat gag cag cgg ccc tgg gcc cag acc 6444
Arg Asn Leu Leu Ala Pro Tyr Glu Gln Arg Pro Trp Ala Gln Thr
    2135                2140                2145

aac tgg atc ctg gtg cgg ctc tgg agg ggc tgt ggc ttc ggg tac 6489
Asn Trp Ile Leu Val Arg Leu Trp Arg Gly Cys Gly Phe Gly Tyr
    2150                2155                2160

cgc tat aca cgg ctg cca cat ctg ctg aaa acc aaa ctt gag gac 6534
Arg Tyr Thr Arg Leu Pro His Leu Leu Lys Thr Lys Leu Glu Asp
    2165                2170                2175

gcc aat ttg ccc agc ctc cag aag ccc tgc cct tcc acc ctg ctg 6579
Ala Asn Leu Pro Ser Leu Gln Lys Pro Cys Pro Ser Thr Leu Leu
    2180                2185                2190

cag cag cac atg gcg gac ctc cta cag cag ggt cct gat gtg gca 6624
Gln Gln His Met Ala Asp Leu Leu Gln Gln Gly Pro Asp Val Ala
    2195                2200                2205

ccc agc ttc ctc aac agc gtc ctc aat cag ctc aac tgg gcc ttc 6669
Pro Ser Phe Leu Asn Ser Val Leu Asn Gln Leu Asn Trp Ala Phe
    2210                2215                2220

tct gaa ttc att ggc atg atc caa gag atc cag cag gct gct gag 6714
Ser Glu Phe Ile Gly Met Ile Gln Glu Ile Gln Gln Ala Ala Glu
    2225                2230                2235

cgc ctg gag cgg aac ttt gtg gac agc cgg cag ctc aag gta tgt 6759
Arg Leu Glu Arg Asn Phe Val Asp Ser Arg Gln Leu Lys Val Cys
    2240                2245                2250

gcc acc tgc ttt gac ctc tcg gtc agc ctg ctg cgt gtc ttg gag 6804
Ala Thr Cys Phe Asp Leu Ser Val Ser Leu Leu Arg Val Leu Glu
    2255                2260                2265

atg act atc aca ctg gtg cct gag ata ttc ctt gac tgg acc cgg 6849
Met Thr Ile Thr Leu Val Pro Glu Ile Phe Leu Asp Trp Thr Arg
    2270                2275                2280

cct acc tct gag atg ctg ctg cgg cgt ctt gca cag ctg cta aac 6894
Pro Thr Ser Glu Met Leu Leu Arg Arg Leu Ala Gln Leu Leu Asn
    2285                2290                2295

cag gtg ctg aac cgg gtg aca gct gag agg aac ctg ttt gat cgt 6939
Gln Val Leu Asn Arg Val Thr Ala Glu Arg Asn Leu Phe Asp Arg
    2300                2305                2310

gtg gtc acc cta cgg ctg cct ggc cta gag agc gtg gac cac tat 6984
Val Val Thr Leu Arg Leu Pro Gly Leu Glu Ser Val Asp His Tyr
    2315                2320                2325

ccc att ctg gtg gca gtg acg ggc atc ctg gtg cag ctc ctg gtg 7029
Pro Ile Leu Val Ala Val Thr Gly Ile Leu Val Gln Leu Leu Val
    2330                2335                2340

cgt ggc cca gcc tca gag aga gag caa gcc aca tca gtg ctc ctg 7074
Arg Gly Pro Ala Ser Glu Arg Glu Gln Ala Thr Ser Val Leu Leu
    2345                2350                2355

gca gat ccc tgc ttc cag cta cgc tca ata tgc tat ctc ctg gga 7119
Ala Asp Pro Cys Phe Gln Leu Arg Ser Ile Cys Tyr Leu Leu Gly
    2360                2365                2370

cag cca gag ccc cca gca cct ggc act gct ctg cca gcc cct gac 7164
Gln Pro Glu Pro Pro Ala Pro Gly Thr Ala Leu Pro Ala Pro Asp
    2375                2380                2385

cgg aag cgc ttc tcc ctg cag agc tat gcg gat tat atc agt gcc 7209
Arg Lys Arg Phe Ser Leu Gln Ser Tyr Ala Asp Tyr Ile Ser Ala
    2390                2395                2400

gat gag ctg gcc caa gtg gaa cag atg ctg gcg cac ctg acc tct 7254
Asp Glu Leu Ala Gln Val Glu Gln Met Leu Ala His Leu Thr Ser
    2405                2410                2415

gca tct gcc cag gca gca gct gcc tcc ctg ccc acc agt gag gag 7299
Ala Ser Ala Gln Ala Ala Ala Ala Ser Leu Pro Thr Ser Glu Glu
    2420                2425                2430

gac ctc tgc ccc atc tgc tat gcc cac ccc atc tct gct gtg ttc 7344
Asp Leu Cys Pro Ile Cys Tyr Ala His Pro Ile Ser Ala Val Phe
    2435                2440                2445

cag ccc tgt ggc cac aag tcc tgc aaa gcc tgt atc aac cag cac 7389
Gln Pro Cys Gly His Lys Ser Cys Lys Ala Cys Ile Asn Gln His
    2450                2455                2460

ctg atg aac aac aag gac tgc ttc ttc tgc aaa acc acc atc gtg 7434
Leu Met Asn Asn Lys Asp Cys Phe Phe Cys Lys Thr Thr Ile Val
    2465                2470                2475

tct gta gag gac tgg gag aag gga gcc aat acg agt act acc tcc 7479
Ser Val Glu Asp Trp Glu Lys Gly Ala Asn Thr Ser Thr Thr Ser
    2480                2485                2490

tca gct gcc tag 7491
Ser Ala Ala

    2495

<210>  10
<211>  2496
<212>  PRT
<213>  Homo sapiens

<400>  10
   Met Ala Ser Asp Ser Pro Ala Arg Ser Leu Asp Glu Ile Asp Leu Ser
   1               5                   10                  15
   Ala Leu Arg Asp Pro Ala Gly Ile Phe Glu Leu Val Glu Leu Val Gly
               20                  25                  30
   Asn Gly Thr Tyr Gly Gln Val Tyr Lys Gly Arg His Val Lys Thr Gly
           35                  40                  45
   Gln Leu Ala Ala Ile Lys Val Met Asp Val Thr Gly Asp Glu Glu Glu
       50                  55                  60
   Glu Ile Lys Gln Glu Ile Asn Met Leu Lys Lys Tyr Ser His His Arg
   65                  70                  75                  80
   Asn Ile Ala Thr Tyr Tyr Gly Ala Phe Ile Lys Lys Asn Pro Pro Gly
                   85                  90                  95
   Met Asp Asp Gln Leu Trp Leu Val Met Glu Phe Cys Gly Ala Gly Ser
               100                 105                 110
   Val Thr Asp Leu Ile Lys Asn Thr Lys Gly Asn Thr Leu Lys Glu Glu
           115                 120                 125
   Trp Ile Ala Tyr Ile Cys Arg Glu Ile Leu Arg Gly Leu Ser His Leu
       130                 135                 140
   His Gln His Lys Val Ile His Arg Asp Ile Lys Gly Gln Asn Val Leu
   145                 150                 155                 160
   Leu Thr Glu Asn Ala Glu Val Lys Leu Val Asp Phe Gly Val Ser Ala
                   165                 170                 175
   Gln Leu Asp Arg Thr Val Gly Arg Arg Asn Thr Phe Ile Gly Thr Pro
               180                 185                 190
   Tyr Trp Met Ala Pro Glu Val Ile Ala Cys Asp Glu Asn Pro Asp Ala
           195                 200                 205
   Thr Tyr Asp Phe Lys Ser Asp Leu Trp Ser Leu Gly Ile Thr Ala Ile
   210 215 220
   Glu Met Ala Glu Gly Ala Pro Pro Leu Cys Asp Met His Pro Met Arg
   225 230 235 240
   Ala Leu Phe Leu Ile Pro Arg Asn Pro Ala Pro Arg Leu Lys Ser Lys
   245 250 255
   Lys Trp Ser Lys Lys Phe Gln Ser Phe Ile Glu Ser Cys Leu Val Lys
   260 265 270
   Asn His Ser Gln Arg Pro Ala Thr Glu Gln Leu Met Lys His Pro Phe
   275 280 285
   Ile Arg Asp Gln Pro Asn Glu Arg Gln Val Arg Ile Gln Leu Lys Asp
   290 295 300
   His Ile Asp Arg Thr Lys Lys Lys Arg Gly Glu Lys Asp Glu Thr Glu
   305 310 315 320
   Tyr Glu Tyr Ser Gly Ser Glu Glu Glu Glu Glu Glu Asn Asp Ser Gly
   325 330 335
   Glu Pro Ser Ser Ile Leu Asn Leu Pro Gly Glu Ser Thr Leu Arg Arg
   340 345 350
   Asp Phe Leu Arg Leu Gln Leu Ala Asn Lys Glu Arg Ser Glu Ala Leu
   355 360 365
   Arg Arg Gln Gln Leu Glu Gln Gln Gln Arg Glu Asn Glu Glu His Lys
   370 375 380
   Arg Gln Leu Leu Ala Glu Arg Gln Lys Arg Ile Glu Glu Gln Lys Glu
   385 390 395 400
   Gln Arg Arg Arg Leu Glu Glu Gln Gln Arg Arg Glu Lys Glu Leu Arg
   405 410 415
   Lys Gln Gln Glu Arg Glu Gln Arg Arg His Tyr Glu Glu Gln Met Arg
   420 425 430
   Arg Glu Glu Glu Arg Arg Arg Ala Glu His Glu Gln Glu Tyr Lys Arg
   435 440 445
   Lys Gln Leu Glu Glu Gln Arg Gln Ala Glu Arg Leu Gln Arg Gln Leu
   450 455 460
   Lys Gln Glu Arg Asp Tyr Leu Val Ser Leu Gln His Gln Arg Gln Glu
   465                 470                 475                 480
   Gln Arg Pro Val Glu Lys Lys Pro Leu Tyr His Tyr Lys Glu Gly Met
                   485                 490                 495
   Ser Pro Ser Glu Lys Pro Ala Trp Ala Lys Glu Val Glu Glu Arg Ser
               500                 505                 510
   Arg Leu Asn Arg Gln Ser Ser Pro Ala Met Pro His Lys Val Ala Asn
           515                 520                 525
   Arg Ile Ser Asp Pro Asn Leu Pro Pro Arg Ser Glu Ser Phe Ser Ile
       530                 535                 540
   Ser Gly Val Gln Pro Ala Arg Thr Pro Pro Met Leu Arg Pro Val Asp
   545                 550                 555                 560
   Pro Gln Ile Pro His Leu Val Ala Val Lys Ser Gln Gly Pro Ala Leu
                   565                 570                 575
   Thr Ala Ser Gln Ser Val His Glu Gln Pro Thr Lys Gly Leu Ser Gly
               580                 585                 590
   Phe Gln Glu Ala Leu Asn Val Thr Ser His Arg Val Glu Met Pro Arg
           595                 600                 605
   Gln Asn Ser Asp Pro Thr Ser Glu Asn Pro Pro Leu Pro Thr Arg Ile
       610                 615                 620
   Glu Lys Phe Asp Arg Ser Ser Trp Leu Arg Gln Glu Glu Asp Ile Pro
   625                 630                 635                 640
   Pro Lys Val Pro Gln Arg Thr Thr Ser Ile Ser Pro Ala Leu Ala Arg
                   645                 650                 655
   Lys Asn Ser Pro Gly Asn Gly Ser Ala Leu Gly Pro Arg Leu Gly Ser
               660                 665                 670
   Gln Pro Ile Arg Ala Ser Asn Pro Asp Leu Arg Arg Thr Glu Pro Ile
           675                 680                 685
   Leu Glu Ser Pro Leu Gln Arg Thr Ser Ser Gly Ser Ser Ser Ser Ser
       690                 695                 700
   Ser Thr Pro Ser Ser Gln Pro Ser Ser Gln Gly Gly Ser Gln Pro Gly
   705                 710                 715                 720
   Ser Gln Ala Gly Ser Ser Glu Arg Thr Arg Val Arg Ala Asn Ser Lys
                   725                 730                 735
   Ser Glu Gly Ser Pro Val Leu Pro His Glu Pro Ala Lys Val Lys Pro
               740                 745                 750
   Glu Glu Ser Arg Asp Ile Thr Arg Pro Ser Arg Pro Ala Asp Leu Thr
           755                 760                 765
   Ala Leu Ala Lys Glu Leu Arg Glu Leu Arg Ile Glu Glu Thr Asn Arg
       770                 775                 780
   Pro Met Lys Lys Val Thr Asp Tyr Ser Ser Ser Ser Glu Glu Ser Glu
   785                 790                 795                 800
   Ser Ser Glu Glu Glu Glu Glu Asp Gly Glu Ser Glu Thr His Asp Gly
                   805                 810                 815
   Thr Val Ala Val Ser Asp Ile Pro Arg Leu Ile Pro Thr Gly Ala Pro
               820                 825                 830
   Gly Ser Asn Glu Gln Tyr Asn Val Gly Met Val Gly Thr His Gly Leu
           835                 840                 845
   Glu Thr Ser His Ala Asp Ser Phe Ser Gly Ser Ile Ser Arg Glu Gly
       850                 855                 860
   Thr Leu Met Ile Arg Glu Thr Ser Gly Glu Lys Lys Arg Ser Gly His
   865                 870                 875                 880
   Ser Asp Ser Asn Gly Phe Ala Gly His Ile Asn Leu Pro Asp Leu Val
                   885                 890                 895
   Gln Gln Ser His Ser Pro Ala Gly Thr Pro Thr Glu Gly Leu Gly Arg
               900                 905                 910
   Val Ser Thr His Ser Gln Glu Met Asp Ser Gly Thr Glu Tyr Gly Met
           915                 920                 925
   Gly Ser Ser Thr Lys Ala Ser Phe Thr Pro Phe Val Asp Pro Arg Val
       930                 935                 940
   Tyr Gln Thr Ser Pro Thr Asp Glu Asp Glu Glu Asp Glu Glu Ser Ser
   945                 950                 955                 960
   Ala Ala Ala Leu Phe Thr Ser Glu Leu Leu Arg Gln Glu Gln Ala Lys
                   965                 970                 975
   Leu Asn Glu Ala Arg Lys Ile Ser Val Val Asn Val Asn Pro Thr Asn
               980                 985                 990
   Ile Arg Pro His Ser Asp Thr Pro Glu Ile Arg Lys Tyr Lys Lys Arg
           995                 1000                1005
   Phe Asn Ser Glu Ile Leu Cys Ala Ala Leu Trp Gly Val Asn Leu
       1010                1015                1020
   Leu Val Gly Thr Glu Asn Gly Leu Met Leu Leu Asp Arg Ser Gly
       1025                1030                1035
   Gln Gly Lys Val Tyr Asn Leu Ile Asn Arg Arg Arg Phe Gln Gln
       1040                1045                1050
   Met Asp Val Leu Glu Gly Leu Asn Val Leu Val Thr Ile Ser Gly
       1055                1060                1065
   Lys Lys Asn Lys Leu Arg Val Tyr Tyr Leu Ser Trp Leu Arg Asn
       1070                1075                1080
   Arg Ile Leu His Asn Asp Pro Glu Val Glu Lys Lys Gln Gly Trp
       1085                1090                1095
   Ile Thr Val Gly Asp Leu Glu Gly Cys Ile His Tyr Lys Val Val
       1100                1105                1110
   Lys Tyr Glu Arg Ile Lys Phe Leu Val Ile Ala Leu Lys Asn Ala
       1115                1120                1125
   Val Glu Ile Tyr Ala Trp Ala Pro Lys Pro Tyr His Lys Phe Met
       1130                1135                1140
   Ala Phe Lys Ser Phe Ala Asp Leu Gln His Lys Pro Leu Leu Val
       1145                1150                1155
   Asp Leu Thr Val Glu Glu Gly Gln Arg Leu Lys Val Ile Phe Gly
       1160                1165                1170
   Ser His Thr Gly Phe His Val Ile Asp Val Asp Ser Gly Asn Ser
       1175                1180                1185
   Tyr Asp Ile Tyr Ile Pro Ser His Ile Gln Gly Asn Ile Thr Pro
       1190                1195                1200
   His Ala Ile Val Ile Leu Pro Lys Thr Asp Gly Met Glu Met Leu
   1205                1210                1215
   Val Cys Tyr Glu Asp Glu Gly Val Tyr Val Asn Thr Tyr Gly Arg
       1220                1225                1230
   Ile Thr Lys Asp Val Val Leu Gln Trp Gly Glu Met Pro Thr Ser
       1235                1240                1245
   Val Ala Tyr Ile His Ser Asn Gln Ile Met Gly Trp Gly Glu Lys
       1250                1255                1260
   Ala Ile Glu Ile Arg Ser Val Glu Thr Gly His Leu Asp Gly Val
       1265                1270                1275
   Phe Met His Lys Arg Ala Gln Arg Leu Lys Phe Leu Cys Glu Arg
       1280                1285                1290
   Asn Asp Lys Val Ile Gly His Ser Asn Phe Gly Thr Ile Arg Ser
       1295                1300                1305
   Thr Thr Cys Val Tyr Lys Gly Lys Trp Leu Tyr Glu Val Leu Ile
       1310                1315                1320
   Ser Ser Gln Gly Leu Met Gln Ile Gly Trp Cys Thr Ile Ser Cys
       1325                1330                1335
   Arg Phe Asn Gln Glu Glu Gly Val Gly Asp Thr His Asn Ser Tyr
       1340                1345                1350
   Ala Tyr Asp Gly Asn Arg Val Arg Lys Trp Asn Val Thr Thr Thr
       1355                1360                1365
   Asn Tyr Gly Lys Ala Trp Ala Ala Gly Asp Ile Val Ser Cys Leu
       1370                1375                1380
   Ile Asp Leu Asp Asp Gly Thr Leu Ser Phe Cys Leu Asn Gly Val
       1385                1390                1395
   Ser Leu Gly Thr Ala Phe Glu Asn Leu Ser Arg Gly Leu Gly Met
       1400                1405                1410
   Ala Tyr Phe Pro Ala Ile Ser Leu Ser Phe Lys Glu Ser Val Ala
       1415                1420                1425
   Phe Asn Phe Gly Ser Arg Pro Leu Arg Tyr Pro Val Ala Gly Tyr
       1430                1435                1440
   Arg Pro Leu Gln Asp Pro Pro Ser Ala Asp Leu Val Arg Ala Gln
   1445 1450 1455
   Arg Leu Leu Gly Cys Phe Arg Ala Val Leu Ser Val Glu Leu Asp
   1460 1465 1470
   Pro Val Glu Gly Arg Leu Leu Asp Lys Glu Ser Ser Lys Trp Arg
   1475 1480 1485
   Leu Arg Gly Gln Pro Thr Val Leu Leu Thr Leu Ala His Ile Phe
   1490 1495 1500
   His His Phe Ala Pro Leu Leu Arg Lys Val Tyr Leu Val Glu Ala
   1505 1510 1515
   Val Leu Met Ser Phe Leu Leu Gly Ile Val Glu Lys Gly Thr Pro
   1520 1525 1530
   Thr Gln Ala Gln Ser Val Val His Gln Val Leu Asp Leu Leu Trp
   1535 1540 1545
   Leu Phe Met Glu Asp Tyr Glu Val Gln Asp Cys Leu Lys Gln Leu
   1550 1555 1560
   Met Met Ser Leu Leu Arg Leu Tyr Arg Phe Ser Pro Ile Val Pro
   1565 1570 1575
   Asp Leu Gly Leu Gln Ile His Tyr Leu Arg Leu Thr Ile Ala Ile
   1580 1585 1590
   Leu Arg His Glu Lys Ser Arg Lys Phe Leu Leu Ser Asn Val Leu
   1595 1600 1605
   Phe Asp Val Leu Arg Ser Val Val Phe Phe Tyr Ile Lys Ser Pro
   1610 1615 1620
   Leu Arg Val Glu Glu Ala Gly Leu Gln Glu Leu Ile Pro Thr Thr
   1625 1630 1635
   Trp Trp Pro His Cys Ser Ser Arg Glu Gly Lys Glu Ser Thr Glu
   1640 1645 1650
   Met Lys Glu Glu Thr Ala Glu Glu Arg Leu Arg Arg Arg Ala Tyr
   1655 1660 1665
   Glu Arg Gly Cys Gln Arg Leu Arg Lys Arg Ile Glu Val Val Glu
   1670 1675 1680
   Glu Leu Gln Val Gln Ile Leu Lys Leu Leu Leu Asp Asn Lys Asp
   1685 1690 1695
   Asp Asn Gly Gly Glu Ala Ser Arg Tyr Ile Phe Leu Thr Lys Phe
   1700 1705 1710
   Arg Lys Phe Leu Gln Glu Asn Ala Ser Gly Arg Gly Asn Met Pro
   1715 1720 1725
   Met Leu Cys Pro Pro Glu Tyr Met Val Cys Phe Leu His Arg Leu
   1730 1735 1740
   Ile Ser Ala Leu Arg Tyr Tyr Trp Asp Glu Tyr Lys Ala Ser Asn
   1745 1750 1755
   Pro His Ala Ser Phe Ser Glu Glu Ala Tyr Ile Pro Pro Gln Val
   1760 1765 1770
   Phe Tyr Asn Gly Lys Val Asp Tyr Phe Asp Leu Gln Arg Leu Gly
   1775 1780 1785
   Gly Leu Leu Ser His Leu Arg Lys Thr Leu Lys Asp Asp Leu Ala
   1790 1795 1800
   Ser Lys Ala Asn Ile Val Ile Asp Pro Leu Glu Leu Gln Ser Thr
   1805 1810 1815
   Ala Met Asp Asp Leu Asp Glu Asp Glu Glu Pro Ala Pro Ala Met
   1820 1825 1830
   Ala Gln Arg Pro Met Gln Ala Leu Ala Val Gly Gly Pro Leu Pro
   1835 1840 1845
   Leu Pro Arg Pro Gly Trp Leu Ser Ser Pro Thr Leu Gly Arg Ala
   1850 1855 1860
   Asn Arg Phe Leu Ser Thr Ala Ala Val Ser Leu Met Thr Pro Arg
   1865 1870 1875
   Arg Pro Leu Ser Thr Ser Glu Lys Val Lys Val Arg Thr Leu Ser
   1880 1885 1890
   Val Glu Gln Arg Thr Arg Glu Asp Ile Glu Gly Ser His Trp Asn
   1895 1900 1905
   Glu Gly Leu Leu Leu Gly Arg Pro Pro Glu Glu Pro Glu Gln Pro
   1910 1915 1920
   Leu Thr Glu Asn Ser Leu Leu Glu Val Leu Asp Gly Ala Val Met
       1925                1930                1935
   Met Tyr Asn Leu Ser Val His Gln Gln Leu Gly Lys Met Val Gly
       1940                1945                1950
   Val Ser Asp Asp Val Asn Glu Tyr Ala Met Ala Leu Arg Asp Thr
       1955                1960                1965
   Glu Asp Lys Leu Arg Arg Cys Pro Lys Arg Arg Lys Asp Ile Leu
       1970                1975                1980
   Ala Glu Leu Thr Lys Ser Gln Lys Val Phe Ser Glu Lys Leu Asp
       1985                1990                1995
   His Leu Ser Arg Arg Leu Ala Trp Val His Ala Thr Val Tyr Ser
       2000                2005                2010
   Gln Glu Lys Met Leu Asp Ile Tyr Trp Leu Leu Arg Val Cys Leu
       2015                2020                2025
   Arg Thr Ile Glu His Gly Asp Arg Thr Gly Ser Leu Phe Ala Phe
       2030                2035                2040
   Met Pro Glu Phe Tyr Leu Ser Val Ala Ile Asn Ser Tyr Ser Ala
       2045                2050                2055
   Leu Lys Asn Tyr Phe Gly Pro Val His Ser Met Glu Glu Leu Pro
       2060                2065                2070
   Gly Tyr Glu Glu Thr Leu Thr Arg Leu Ala Ala Ile Leu Ala Lys
       2075                2080                2085
   His Phe Ala Asp Ala Arg Ile Val Gly Thr Asp Ile Arg Asp Ser
       2090                2095                2100
   Leu Met Gln Ala Leu Ala Ser Tyr Val Cys Tyr Pro His Ser Leu
       2105                2110                2115
   Arg Ala Val Glu Arg Ile Pro Glu Glu Gln Arg Ile Ala Met Val
       2120                2125                2130
   Arg Asn Leu Leu Ala Pro Tyr Glu Gln Arg Pro Trp Ala Gln Thr
       2135                2140                2145
   Asn Trp Ile Leu Val Arg Leu Trp Arg Gly Cys Gly Phe Gly Tyr
   2150                2155                2160
   Arg Tyr Thr Arg Leu Pro His Leu Leu Lys Thr Lys Leu Glu Asp
       2165                2170                2175
   Ala Asn Leu Pro Ser Leu Gln Lys Pro Cys Pro Ser Thr Leu Leu
       2180                2185                2190
   Gln Gln His Met Ala Asp Leu Leu Gln Gln Gly Pro Asp Val Ala
       2195                2200                2205
   Pro Ser Phe Leu Asn Ser Val Leu Asn Gln Leu Asn Trp Ala Phe
       2210                2215                2220
   Ser Glu Phe Ile Gly Met Ile Gln Glu Ile Gln Gln Ala Ala Glu
       2225                2230                2235
   Arg Leu Glu Arg Asn Phe Val Asp Ser Arg Gln Leu Lys Val Cys
       2240                2245                2250
   Ala Thr Cys Phe Asp Leu Ser Val Ser Leu Leu Arg Val Leu Glu
       2255                2260                2265
   Met Thr Ile Thr Leu Val Pro Glu Ile Phe Leu Asp Trp Thr Arg
       2270                2275                2280
   Pro Thr Ser Glu Met Leu Leu Arg Arg Leu Ala Gln Leu Leu Asn
       2285                2290                2295
   Gln Val Leu Asn Arg Val Thr Ala Glu Arg Asn Leu Phe Asp Arg
       2300                2305                2310
   Val Val Thr Leu Arg Leu Pro Gly Leu Glu Ser Val Asp His Tyr
       2315                2320                2325
   Pro Ile Leu Val Ala Val Thr Gly Ile Leu Val Gln Leu Leu Val
       2330                2335                2340
   Arg Gly Pro Ala Ser Glu Arg Glu Gln Ala Thr Ser Val Leu Leu
       2345                2350                2355
   Ala Asp Pro Cys Phe Gln Leu Arg Ser Ile Cys Tyr Leu Leu Gly
       2360                2365                2370
   Gln Pro Glu Pro Pro Ala Pro Gly Thr Ala Leu Pro Ala Pro Asp
       2375                2380                2385
   Arg Lys Arg Phe Ser Leu Gln Ser Tyr Ala Asp Tyr Ile Ser Ala
       2390                2395                2400
   Asp Glu Leu Ala Gln Val Glu Gln Met Leu Ala His Leu Thr Ser
       2405                2410                2415
   Ala Ser Ala Gln Ala Ala Ala Ala Ser Leu Pro Thr Ser Glu Glu
       2420                2425                2430
   Asp Leu Cys Pro Ile Cys Tyr Ala His Pro Ile Ser Ala Val Phe
       2435                2440                2445
   Gln Pro Cys Gly His Lys Ser Cys Lys Ala Cys Ile Asn Gln His
       2450                2455                2460
   Leu Met Asn Asn Lys Asp Cys Phe Phe Cys Lys Thr Thr Ile Val
       2465                2470                2475
   Ser Val Glu Asp Trp Glu Lys Gly Ala Asn Thr Ser Thr Thr Ser
       2480                2485                2490
   Ser Ala Ala
       2495

<210>  11
<211>  7437
<212>  DNA
<213>  Homo sapiens

<220>
<221>  CDS
<222>  (1)..(7437)

<400>  11
atg gcg agc gac tcc ccg gct cga agc ctg gat gaa ata gat ctc tcg      48
Met Ala Ser Asp Ser Pro Ala Arg Ser Leu Asp Glu Ile Asp Leu Ser
1               5                   10                  15

gct ctg agg gac ccc gca ggg atc ttt gaa ttg gtg gaa ctt gtt gga      96
Ala Leu Arg Asp Pro Ala Gly Ile Phe Glu Leu Val Glu Leu Val Gly
            20                  25                  30

aat gga aca tac ggg caa gtt tat aag ggt cgt cat gtc aaa acg ggc      144
Asn Gly Thr Tyr Gly Gln Val Tyr Lys Gly Arg His Val Lys Thr Gly
        35                  40                  45

cag ctt gca gcc atc aag gtt atg gat gtc aca ggg gat gaa gag gaa      192
Gln Leu Ala Ala Ile Lys Val Met Asp Val Thr Gly Asp Glu Glu Glu
    50                  55                  60

gaa atc aaa caa gaa att aac atg ttg aag aaa tat tct cat cac cgg      240
Glu Ile Lys Gln Glu Ile Asn Met Leu Lys Lys Tyr Ser His His Arg
65                  70                  75                  80

aat att gct aca tac tat ggt gct ttt atc aaa aag aac cca cca ggc 288
Asn Ile Ala Thr Tyr Tyr Gly Ala Phe Ile Lys Lys Asn Pro Pro Gly
                85                  90                  95

atg gat gac caa ctt tgg ttg gtg atg gag ttt tgt ggt gct ggc tct 336
Met Asp Asp Gln Leu Trp Leu Val Met Glu Phe Cys Gly Ala Gly Ser
            100                 105                 110

gtc acc gac ctg atc aag aac aca aaa ggt aac acg ttg aaa gag gag 384
Val Thr Asp Leu Ile Lys Asn Thr Lys Gly Asn Thr Leu Lys Glu Glu
        115                 120                 125

tgg att gca tac atc tgc agg gaa atc tta cgg ggg ctg agt cac ctg 432
Trp Ile Ala Tyr Ile Cys Arg Glu Ile Leu Arg Gly Leu Ser His Leu
    130                 135                 140

cac cag cat aaa gtg att cat cga gat att aaa ggg caa aat gtc ttg 480
His Gln His Lys Val Ile His Arg Asp Ile Lys Gly Gln Asn Val Leu
145                 150                 155                 160

ctg act gaa aat gca gaa gtt aaa cta gtg gac ttt gga gtc agt gct 528
Leu Thr Glu Asn Ala Glu Val Lys Leu Val Asp Phe Gly Val Ser Ala
                165                 170                 175

cag ctt gat cga aca gtg ggc agg agg aat act ttc att gga act ccc 576
Gln Leu Asp Arg Thr Val Gly Arg Arg Asn Thr Phe Ile Gly Thr Pro
            180                 185                 190

tac tgg atg gca cca gaa gtt att gcc tgt gat gaa aac cca gat gcc 624
Tyr Trp Met Ala Pro Glu Val Ile Ala Cys Asp Glu Asn Pro Asp Ala
        195                 200                 205

aca tat gat ttc aag agt gac ttg tgg tct ttg ggt atc acc gcc att 672
Thr Tyr Asp Phe Lys Ser Asp Leu Trp Ser Leu Gly Ile Thr Ala Ile
    210                 215                 220

gaa atg gca gaa ggt gct ccc cct ctc tgt gac atg cac ccc atg aga 720
Glu Met Ala Glu Gly Ala Pro Pro Leu Cys Asp Met His Pro Met Arg
225                 230                 235                 240

gct ctc ttc ctc atc ccc cgg aac cca gcg cct cgg ctg aag tct aag 768
Ala Leu Phe Leu Ile Pro Arg Asn Pro Ala Pro Arg Leu Lys Ser Lys
                245                 250                 255

aag tgg tca aaa aaa ttc cag tca ttt att gag agc tgc ttg gta aag 816
Lys Trp Ser Lys Lys Phe Gln Ser Phe Ile Glu Ser Cys Leu Val Lys
            260                 265                 270

aat cac agc cag cga cca gca aca gaa caa ttg atg aag cat cca ttt 864
Asn His Ser Gln Arg Pro Ala Thr Glu Gln Leu Met Lys His Pro Phe
        275                 280                 285

ata cga gac caa cct aat gag cga cag gtc cgc att caa ctc aag gac 912
Ile Arg Asp Gln Pro Asn Glu Arg Gln Val Arg Ile Gln Leu Lys Asp
    290                 295                 300

cat att gat aga aca aag aag aag cga gga gaa aaa gat gag aca gag 960
His Ile Asp Arg Thr Lys Lys Lys Arg Gly Glu Lys Asp Glu Thr Glu
305                 310                 315                 320

tat gag tac agt gga agt gag gaa gaa gag gag gag aat gac tca gga 1008
Tyr Glu Tyr Ser Gly Ser Glu Glu Glu Glu Glu Glu Asn Asp Ser Gly
                325                 330                 335

gag ccc agc tcc atc ctg aat ctg cca ggg gag tcg acg ctg cgg agg 1056
Glu Pro Ser Ser Ile Leu Asn Leu Pro Gly Glu Ser Thr Leu Arg Arg
            340                 345                 350

gac ttt ctg agg ctg cag ctg gcc aac aag gag cgt tct gag gcc cta 1104
Asp Phe Leu Arg Leu Gln Leu Ala Asn Lys Glu Arg Ser Glu Ala Leu
        355                 360                 365

cgg agg cag cag ctg gag cag cag cag cgg gag aat gag gag cac aag 1152
Arg Arg Gln Gln Leu Glu Gln Gln Gln Arg Glu Asn Glu Glu His Lys
    370                 375                 380

cgg cag ctg ctg gcc gag cgt cag aag cgc atc gag gag cag aaa gag 1200
Arg Gln Leu Leu Ala Glu Arg Gln Lys Arg Ile Glu Glu Gln Lys Glu
385                 390                 395                 400

cag agg cgg cgg ctg gag gag caa caa agg cga gag aag gag ctg cgg 1248
Gln Arg Arg Arg Leu Glu Glu Gln Gln Arg Arg Glu Lys Glu Leu Arg
                405                 410                 415

aag cag cag gag agg gag cag cgc cgg cac tat gag gag cag atg cgc 1296
Lys Gln Gln Glu Arg Glu Gln Arg Arg His Tyr Glu Glu Gln Met Arg
            420                 425                 430

cgg gag gag gag agg agg cgt gcg gag cat gaa cag gaa tac atc agg 1344
Arg Glu Glu Glu Arg Arg Arg Ala Glu His Glu Gln Glu Tyr Ile Arg
        435                 440                 445

cga cag tta gag gag gag cag aga cag tta gag atc ttg cag cag cag 1392
Arg Gln Leu Glu Glu Glu Gln Arg Gln Leu Glu Ile Leu Gln Gln Gln
    450                 455                 460

cta ctg cat gaa caa gct cta ctt ctg gaa tat aag cgc aaa caa ttg 1440
Leu Leu His Glu Gln Ala Leu Leu Leu Glu Tyr Lys Arg Lys Gln Leu
465                 470                 475                 480

gaa gaa cag aga caa gca gaa aga ctg cag agg cag cta aag caa gaa 1488
Glu Glu Gln Arg Gln Ala Glu Arg Leu Gln Arg Gln Leu Lys Gln Glu
                485                 490                 495

aga gac tac tta gtt tcc ctt cag cat cag cgg cag gag cag agg cct 1536
Arg Asp Tyr Leu Val Ser Leu Gln His Gln Arg Gln Glu Gln Arg Pro
            500                 505                 510

gtg gag aag aag cca ctg tac cat tac aaa gaa gga atg agt cct agt 1584
Val Glu Lys Lys Pro Leu Tyr His Tyr Lys Glu Gly Met Ser Pro Ser
        515                 520                 525

gag aag cca gca tgg gcc aag gag atc cca cat ctg gta gct gta aaa 1632
Glu Lys Pro Ala Trp Ala Lys Glu Ile Pro His Leu Val Ala Val Lys
    530                 535                 540

tcc cag gga cct gcc ttg acc gcc tcc cag tca gtg cac gag cag ccc 1680
Ser Gln Gly Pro Ala Leu Thr Ala Ser Gln Ser Val His Glu Gln Pro
545                 550                 555                 560

aca aag ggc ctc tct ggg ttt cag gag gct ctg aac gtg acc tcc cac 1728
Thr Lys Gly Leu Ser Gly Phe Gln Glu Ala Leu Asn Val Thr Ser His
                565                 570                 575

cgc gtg gag atg cca cgc cag aac tca gat ccc acc tcg gaa aat cct 1776
Arg Val Glu Met Pro Arg Gln Asn Ser Asp Pro Thr Ser Glu Asn Pro

580                 585                 590

cct ctc ccc act cgc att gaa aag ttt gac cga agc tct tgg tta cga        1824
Pro Leu Pro Thr Arg Ile Glu Lys Phe Asp Arg Ser Ser Trp Leu Arg
        595                 600                 605

cag gaa gaa gac att cca cca aag gtg cct caa aga aca act tct ata        1872
Gln Glu Glu Asp Ile Pro Pro Lys Val Pro Gln Arg Thr Thr Ser Ile
    610                 615                 620

tcc cca gca tta gcc aga aag aat tct cct ggg aat ggt agt gct ctg        1920
Ser Pro Ala Leu Ala Arg Lys Asn Ser Pro Gly Asn Gly Ser Ala Leu
625                 630                 635                 640

gga ccc aga cta gga tct caa ccc atc aga gca agc aac cct gat ctc        1968
Gly Pro Arg Leu Gly Ser Gln Pro Ile Arg Ala Ser Asn Pro Asp Leu
                645                 650                 655

cgg aga act gag ccc atc ttg gag agc ccc ttg cag agg acc agc agt        2016
Arg Arg Thr Glu Pro Ile Leu Glu Ser Pro Leu Gln Arg Thr Ser Ser
            660                 665                 670

ggc agt tcc tcc agc tcc agc acc cct agc tcc cag ccc agc tcc caa        2064
Gly Ser Ser Ser Ser Ser Ser Thr Pro Ser Ser Gln Pro Ser Ser Gln
        675                 680                 685

gga ggc tcc cag cct gga tca caa gca gga tcc agt gaa cgc acc aga        2112
Gly Gly Ser Gln Pro Gly Ser Gln Ala Gly Ser Ser Glu Arg Thr Arg
    690                 695                 700

gtt cga gcc aac agt aag tca gaa gga tca cct gtg ctc ccc cat gag        2160
Val Arg Ala Asn Ser Lys Ser Glu Gly Ser Pro Val Leu Pro His Glu
705                 710                 715                 720

cct gcc aag gtg aaa cca gaa gaa tcc agg gac att acc cgg ccc agt        2208
Pro Ala Lys Val Lys Pro Glu Glu Ser Arg Asp Ile Thr Arg Pro Ser
                725                 730                 735

cga cca gct agc tac aaa aaa gct ata gat gag gat ctg acg gca tta        2256
Arg Pro Ala Ser Tyr Lys Lys Ala Ile Asp Glu Asp Leu Thr Ala Leu
            740                 745                 750

gcc aaa gaa cta aga gaa ctc cgg att gaa gaa aca aac cgc cca atg        2304
Ala Lys Glu Leu Arg Glu Leu Arg Ile Glu Glu Thr Asn Arg Pro Met
        755                 760                 765

aag aag gtg act gat tac tcc tcc tcc agt gag gag tca gaa agt agc        2352
Lys Lys Val Thr Asp Tyr Ser Ser Ser Ser Glu Glu Ser Glu Ser Ser
    770                 775                 780

gag gaa gag gag gaa gat gga gag agc gag acc cat gat ggg aca gtg        2400
Glu Glu Glu Glu Glu Asp Gly Glu Ser Glu Thr His Asp Gly Thr Val
785                 790                 795                 800

gct gtc agc gac ata ccc aga ctg ata cca aca gga gct cca ggc agc        2448
Ala Val Ser Asp Ile Pro Arg Leu Ile Pro Thr Gly Ala Pro Gly Ser
                805                 810                 815

aac gag cag tac aat gtg gga atg gtg ggg acg cat ggg ctg gag acc        2496
Asn Glu Gln Tyr Asn Val Gly Met Val Gly Thr His Gly Leu Glu Thr
            820                 825                 830

tct cat gcg gac agt ttc agc ggc agt att tca aga gaa gga acc ttg        2544

Ser His Ala Asp Ser Phe Ser Gly Ser Ile Ser Arg Glu Gly Thr Leu
        835                 840                 845

atg att aga gag acg tct gga gag aag aag cga tct ggc cac agt gac        2592
Met Ile Arg Glu Thr Ser Gly Glu Lys Lys Arg Ser Gly His Ser Asp
    850                 855                 860

agc aat ggc ttt gct ggc cac atc aac ctc cct gac ctg gtg cag cag        2640
Ser Asn Gly Phe Ala Gly His Ile Asn Leu Pro Asp Leu Val Gln Gln
865                 870                 875                 880

agc cat tct cca gct gga acc ccg act gag gga ctg ggg cgc gtc tca        2688
Ser His Ser Pro Ala Gly Thr Pro Thr Glu Gly Leu Gly Arg Val Ser
                885                 890                 895

acc cat tcc cag gag atg gac tct ggg act gaa tat ggc atg ggg agc        2736
Thr His Ser Gln Glu Met Asp Ser Gly Thr Glu Tyr Gly Met Gly Ser
            900                 905                 910

agc acc aaa gcc tcc ttc acc ccc ttt gtg gac ccc aga gta tac cag        2784
Ser Thr Lys Ala Ser Phe Thr Pro Phe Val Asp Pro Arg Val Tyr Gln
        915                 920                 925

acg tct ccc act gat gaa gat gaa gag gat gag gaa tca tca gcc gca        2832
Thr Ser Pro Thr Asp Glu Asp Glu Glu Asp Glu Glu Ser Ser Ala Ala
    930                 935                 940

gct ctg ttt act agc gaa ctt ctt agg caa gaa cag gcc aaa ctc aat        2880
Ala Leu Phe Thr Ser Glu Leu Leu Arg Gln Glu Gln Ala Lys Leu Asn
945                 950                 955                 960

gaa gca aga aag att tcg gtg gta aat gta aac cca acc aac att cgg        2928
Glu Ala Arg Lys Ile Ser Val Val Asn Val Asn Pro Thr Asn Ile Arg
                965                 970                 975

cct cat agc gac aca cca gaa atc aga aaa tac aag aaa cga ttc aac        2976
Pro His Ser Asp Thr Pro Glu Ile Arg Lys Tyr Lys Lys Arg Phe Asn
            980                 985                 990

tca gaa ata ctt tgt gca gct ctg  tgg ggt gta aac ctt  ctg gtg ggg        3024
Ser Glu Ile Leu Cys Ala Ala Leu  Trp Gly Val Asn Leu  Leu Val Gly
        995                 1000                1005

act gaa  aat ggc ctg atg ctt  ttg gac cga agt ggg  caa ggc aaa        3069
Thr Glu  Asn Gly Leu Met Leu  Leu Asp Arg Ser Gly  Gln Gly Lys
    1010                1015                1020

gtc tat  aat ctg atc aac cgg  agg cga ttt cag cag  atg gat gtg        3114
Val Tyr  Asn Leu Ile Asn Arg  Arg Arg Phe Gln Gln  Met Asp Val
    1025                1030                1035

cta gag  gga ctg aat gtc ctt  gtg aca att tca gga  aag aag aat        3159
Leu Glu  Gly Leu Asn Val Leu  Val Thr Ile Ser Gly  Lys Lys Asn
    1040                1045                1050

aag cta  cga gtt tac tat ctt  tca tgg tta aga aac  aga ata cta        3204
Lys Leu  Arg Val Tyr Tyr Leu  Ser Trp Leu Arg Asn  Arg Ile Leu
    1055                1060                1065

cat aat  gac cca gaa gta gaa  aag aaa caa ggc tgg  atc act gtt        3249
His Asn  Asp Pro Glu Val Glu  Lys Lys Gln Gly Trp  Ile Thr Val
    1070                1075                1080

ggg gac ttg gaa ggc tgt ata cat tat aaa gtt gtt aaa tat gaa 3294
Gly Asp Leu Glu Gly Cys Ile His Tyr Lys Val Val Lys Tyr Glu
    1085                1090                1095

agg atc aaa ttt ttg gtg att gcc tta aag aat gct gtg gaa ata 3339
Arg Ile Lys Phe Leu Val Ile Ala Leu Lys Asn Ala Val Glu Ile
    1100                1105                1110

tat gct tgg gct cct aaa ccg tat cat aaa ttc atg gca ttt aag 3384
Tyr Ala Trp Ala Pro Lys Pro Tyr His Lys Phe Met Ala Phe Lys
    1115                1120                1125

tct ttt gca gat ctc cag cac aag cct ctg cta gtt gat ctc acg 3429
Ser Phe Ala Asp Leu Gln His Lys Pro Leu Leu Val Asp Leu Thr
    1130                1135                1140

gta gaa gaa ggt caa aga tta aag gtt att ttt ggt tca cac act 3474
Val Glu Glu Gly Gln Arg Leu Lys Val Ile Phe Gly Ser His Thr
    1145                1150                1155

ggt ttc cat gta att gat gtt gat tca gga aac tct tat gat atc 3519
Gly Phe His Val Ile Asp Val Asp Ser Gly Asn Ser Tyr Asp Ile
    1160                1165                1170

tac ata cca tct cat att cag ggc aat atc act cct cat gct att 3564
Tyr Ile Pro Ser His Ile Gln Gly Asn Ile Thr Pro His Ala Ile
    1175                1180                1185

gtc atc ttg cct aaa aca gat gga atg gaa atg ctt gtt tgc tat 3609
Val Ile Leu Pro Lys Thr Asp Gly Met Glu Met Leu Val Cys Tyr
    1190                1195                1200

gag gat gag ggg gtg tat gta aac acc tat ggc cgg ata act aag 3654
Glu Asp Glu Gly Val Tyr Val Asn Thr Tyr Gly Arg Ile Thr Lys
    1205                1210                1215

gat gtg gtg ctc caa tgg gga gaa atg ccc acg tct gtg gcc tac 3699
Asp Val Val Leu Gln Trp Gly Glu Met Pro Thr Ser Val Ala Tyr
    1220                1225                1230

att cat tcc aat cag ata atg ggc tgg ggc gag aaa gct att gag 3744
Ile His Ser Asn Gln Ile Met Gly Trp Gly Glu Lys Ala Ile Glu
    1235                1240                1245

atc cgg tca gtg gaa aca gga cat ttg gat gga gta ttt atg cat 3789
Ile Arg Ser Val Glu Thr Gly His Leu Asp Gly Val Phe Met His
    1250                1255                1260

aag cga gct caa agg tta aag ttt cta tgt gaa aga aat gat aag 3834
Lys Arg Ala Gln Arg Leu Lys Phe Leu Cys Glu Arg Asn Asp Lys
    1265                1270                1275

gtg att gga cac agc aac ttt ggc acc atc cgc tct acc aca tgc 3879
Val Ile Gly His Ser Asn Phe Gly Thr Ile Arg Ser Thr Thr Cys
    1280                1285                1290

gtg tac aaa ggg aaa tgg ctc tac gag gtc ctc atc tcc tcc cag 3924
Val Tyr Lys Gly Lys Trp Leu Tyr Glu Val Leu Ile Ser Ser Gln
    1295                1300                1305

ggg ctc atg cag atc ggc tgg tgc acc atc agc tgc cgc ttc aac 3969
Gly Leu Met Gln Ile Gly Trp Cys Thr Ile Ser Cys Arg Phe Asn
    1310                1315                1320

cag gag gag ggg gtt gga gat aca cac aac tcc tat gcc tat gat 4014
Gln Glu Glu Gly Val Gly Asp Thr His Asn Ser Tyr Ala Tyr Asp
    1325                1330                1335

ggc aac cgc gtg cgc aag tgg aat gtg acc aca acg aat tat ggc 4059
Gly Asn Arg Val Arg Lys Trp Asn Val Thr Thr Thr Asn Tyr Gly
    1340                1345                1350

aag gcg tgg gca gcg ggg gac atc gtg agc tgc ctg att gac ctg 4104
Lys Ala Trp Ala Ala Gly Asp Ile Val Ser Cys Leu Ile Asp Leu
    1355                1360                1365

gat gat ggc act ctg tcc ttc tgc ctg aac ggt gta tca ctg ggc 4149
Asp Asp Gly Thr Leu Ser Phe Cys Leu Asn Gly Val Ser Leu Gly
    1370                1375                1380

act gcc ttt gag aac ctg tcc agg ggc ctg ggt atg gcc tac ttc 4194
Thr Ala Phe Glu Asn Leu Ser Arg Gly Leu Gly Met Ala Tyr Phe
    1385                1390                1395

cca gcc atc agc ctc tct ttc aag gag tcc gtg gcc ttc aac ttt 4239
Pro Ala Ile Ser Leu Ser Phe Lys Glu Ser Val Ala Phe Asn Phe
    1400                1405                1410

ggc agc cgt cct ctg cgc tac cca gtg gca ggc tac cgg ccc ctg 4284
Gly Ser Arg Pro Leu Arg Tyr Pro Val Ala Gly Tyr Arg Pro Leu
    1415                1420                1425

cag gac cca ccg agt gct gac ctg gtg cgg gca cag agg ttg ctg 4329
Gln Asp Pro Pro Ser Ala Asp Leu Val Arg Ala Gln Arg Leu Leu
    1430                1435                1440

ggc tgc ttc cgg gca gtg ctg agt gtg gag ctg gac cct gtg gag 4374
Gly Cys Phe Arg Ala Val Leu Ser Val Glu Leu Asp Pro Val Glu
    1445                1450                1455

ggg cgg ctg ttg gac aag gag agc tcc aag tgg cgg ttg cgg ggc 4419
Gly Arg Leu Leu Asp Lys Glu Ser Ser Lys Trp Arg Leu Arg Gly
    1460                1465                1470

cag ccc acc gtc ctc ctc aca ctg gcc cac atc ttc cat cac ttt 4464
Gln Pro Thr Val Leu Leu Thr Leu Ala His Ile Phe His His Phe
    1475                1480                1485

gca ccg ctt ctg cgc aag gtg tat ctg gtg gag gct gtg ctc atg 4509
Ala Pro Leu Leu Arg Lys Val Tyr Leu Val Glu Ala Val Leu Met
    1490                1495                1500

agc ttc ttg ctg ggc atc gtg gag aag ggc aca ccc aca cag gca 4554
Ser Phe Leu Leu Gly Ile Val Glu Lys Gly Thr Pro Thr Gln Ala
    1505                1510                1515

cag tcc gtg gtg cac cag gtc ctg gac ctc ttg tgg ctc ttc atg 4599
Gln Ser Val Val His Gln Val Leu Asp Leu Leu Trp Leu Phe Met
    1520                1525                1530

gag gac tac gag gta caa gat tgc ctc aag cag ttg atg atg tct 4644
Glu Asp Tyr Glu Val Gln Asp Cys Leu Lys Gln Leu Met Met Ser
    1535                1540                1545

ctg ctt cgg ctg tac cga ttc tca ccc att gtc cca gac ctg ggc 4689
Leu Leu Arg Leu Tyr Arg Phe Ser Pro Ile Val Pro Asp Leu Gly

1550                    1555                    1560

cta cag atc cat tac ctg cgg ctc act atc gcc atc ctg agg cat          4734
Leu Gln Ile His Tyr Leu Arg Leu Thr Ile Ala Ile Leu Arg His
1565                    1570                    1575

gag aag tcc cgc aag ttt ctg ctt agc aat gtc ctc ttc gac gtg          4779
Glu Lys Ser Arg Lys Phe Leu Leu Ser Asn Val Leu Phe Asp Val
1580                    1585                    1590

ctc cgc tcc gtc gtc ttc ttt tac atc aag agc ccc ctg cgt gtg          4824
Leu Arg Ser Val Val Phe Phe Tyr Ile Lys Ser Pro Leu Arg Val
1595                    1600                    1605

gag gag gcc ggc ctg cag gag ctc att ccc acc acc tgg tgg ccc          4869
Glu Glu Ala Gly Leu Gln Glu Leu Ile Pro Thr Thr Trp Trp Pro
1610                    1615                    1620

cac tgc tcc agt agg gag ggc aaa gag agc acg gag atg aag gag          4914
His Cys Ser Ser Arg Glu Gly Lys Glu Ser Thr Glu Met Lys Glu
1625                    1630                    1635

gag acc gca gag gag cgg ctg cgg cgg cga gcc tac gaa cgg ggc          4959
Glu Thr Ala Glu Glu Arg Leu Arg Arg Arg Ala Tyr Glu Arg Gly
1640                    1645                    1650

tgt cag cgg ctc agg aag cgc atc gaa gtg gtg gaa gaa cta cag          5004
Cys Gln Arg Leu Arg Lys Arg Ile Glu Val Val Glu Glu Leu Gln
1655                    1660                    1665

gtc cag atc ctg aag ctg ctg ctg gac aat aaa gat gac aat ggg          5049
Val Gln Ile Leu Lys Leu Leu Leu Asp Asn Lys Asp Asp Asn Gly
1670                    1675                    1680

ggt gaa gct tct agg tat atc ttc ctg acc aag ttt cgc aag ttt          5094
Gly Glu Ala Ser Arg Tyr Ile Phe Leu Thr Lys Phe Arg Lys Phe
1685                    1690                    1695

ctg cag gag aac gcc agt ggc cgg ggg aac atg ccc atg ctc tgc          5139
Leu Gln Glu Asn Ala Ser Gly Arg Gly Asn Met Pro Met Leu Cys
1700                    1705                    1710

ccc cct gag tac atg gtc tgc ttc tta cac cgg ctg atc tct gcc          5184
Pro Pro Glu Tyr Met Val Cys Phe Leu His Arg Leu Ile Ser Ala
1715                    1720                    1725

ctg cgc tac tat tgg gat gaa tac aag gct tcc aat cct cat gct          5229
Leu Arg Tyr Tyr Trp Asp Glu Tyr Lys Ala Ser Asn Pro His Ala
1730                    1735                    1740

tcc ttc agt gag gag gcc tac atc ccg ccc cag gtc ttc tat aat          5274
Ser Phe Ser Glu Glu Ala Tyr Ile Pro Pro Gln Val Phe Tyr Asn
1745                    1750                    1755

ggc aag gtg gac tac ttt gac ctg cag cgc ctg ggg ggc ctc ctc          5319
Gly Lys Val Asp Tyr Phe Asp Leu Gln Arg Leu Gly Gly Leu Leu
1760                    1765                    1770

tcg cac ctg cgg aag acc ctc aaa gat gac ctt gct tcc aaa gcc          5364
Ser His Leu Arg Lys Thr Leu Lys Asp Asp Leu Ala Ser Lys Ala
1775                    1780                    1785

aac att gtg atc gac cca ctg gag ctc cag tca acc gcc atg gat          5409

Asn Ile Val Ile Asp Pro Leu Glu Leu Gln Ser Thr Ala Met Asp
    1790                1795                1800

gac cta gat gag gat gag gag cca gcc cca gct atg gcc cag cgc 5454
Asp Leu Asp Glu Asp Glu Glu Pro Ala Pro Ala Met Ala Gln Arg
    1805                1810                1815

ccc atg cag gcc ctg gct gtt ggg ggg cca ctg ccc ctg ccc cgg 5499
Pro Met Gln Ala Leu Ala Val Gly Gly Pro Leu Pro Leu Pro Arg
    1820                1825                1830

ccc ggc tgg ctc agt tct cca act ttg ggc cga gcc aac cgc ttc 5544
Pro Gly Trp Leu Ser Ser Pro Thr Leu Gly Arg Ala Asn Arg Phe
    1835                1840                1845

ctc agc aca gcg gct gtg agc ctc atg acc cca cgg cgg cct ctg 5589
Leu Ser Thr Ala Ala Val Ser Leu Met Thr Pro Arg Arg Pro Leu
    1850                1855                1860

agc acc tcg gag aaa gtg aag gtc cgc acg ctg agc gtg gag cag 5634
Ser Thr Ser Glu Lys Val Lys Val Arg Thr Leu Ser Val Glu Gln
    1865                1870                1875

agg acc cgt gag gac att gaa ggc agc cac tgg aat gag ggc ttg 5679
Arg Thr Arg Glu Asp Ile Glu Gly Ser His Trp Asn Glu Gly Leu
    1880                1885                1890

ctg ctg ggg cgg ccc ccc gag gag cct gag cag ccc ctc acc gag 5724
Leu Leu Gly Arg Pro Pro Glu Glu Pro Glu Gln Pro Leu Thr Glu
    1895                1900                1905

aac tcg ctg ctg gaa gtc ctg gat ggg gcg gtc atg atg tac aac 5769
Asn Ser Leu Leu Glu Val Leu Asp Gly Ala Val Met Met Tyr Asn
    1910                1915                1920

ctc agc gta cac cag cag ctg ggc aag atg gtg ggt gtc tcc gat 5814
Leu Ser Val His Gln Gln Leu Gly Lys Met Val Gly Val Ser Asp
    1925                1930                1935

gat gtc aat gaa tac gct atg gct ctg agg gac aca gag gac aag 5859
Asp Val Asn Glu Tyr Ala Met Ala Leu Arg Asp Thr Glu Asp Lys
    1940                1945                1950

ctc cgc cgg tgc ccc aag agg agg aag gac atc ctt gca gag ttg 5904
Leu Arg Arg Cys Pro Lys Arg Arg Lys Asp Ile Leu Ala Glu Leu
    1955                1960                1965

acc aag agc cag aag gtt ttc tca gaa aag ctg gac cac ctg agc 5949
Thr Lys Ser Gln Lys Val Phe Ser Glu Lys Leu Asp His Leu Ser
    1970                1975                1980

cgc cgt ctt gcc tgg gtc cat gcc act gtc tac tcc cag gag aag 5994
Arg Arg Leu Ala Trp Val His Ala Thr Val Tyr Ser Gln Glu Lys
    1985                1990                1995

atg ctg gac atc tac tgg ctg ctg cgc gtc tgc ctg cgg acc att 6039
Met Leu Asp Ile Tyr Trp Leu Leu Arg Val Cys Leu Arg Thr Ile
    2000                2005                2010

gag cac ggt gat cgc aca ggg tct ctc ttt gcc ttc atg ccc gag 6084
Glu His Gly Asp Arg Thr Gly Ser Leu Phe Ala Phe Met Pro Glu
    2015                2020                2025

ttc tac ctg agc gtg gcc atc aac agc tac agt gct ctc aag aat 6129
Phe Tyr Leu Ser Val Ala Ile Asn Ser Tyr Ser Ala Leu Lys Asn
    2030                2035                2040

tac ttt ggt ccc gtg cac agc atg gag gag ctc cca ggc tat gaa 6174
Tyr Phe Gly Pro Val His Ser Met Glu Glu Leu Pro Gly Tyr Glu
    2045                2050                2055

gag acc ctg acc cgc ctg gct gcc att ctc gcc aaa cac ttt gcc 6219
Glu Thr Leu Thr Arg Leu Ala Ala Ile Leu Ala Lys His Phe Ala
    2060                2065                2070

gac gca cgc att gtg ggc act gac atc cga gac tca ctg atg cag 6264
Asp Ala Arg Ile Val Gly Thr Asp Ile Arg Asp Ser Leu Met Gln
    2075                2080                2085

gcc ctg gcc agc tac gtg tgc tac cca cac tcc ctg cgg gct gtg 6309
Ala Leu Ala Ser Tyr Val Cys Tyr Pro His Ser Leu Arg Ala Val
    2090                2095                2100

gag cga atc ccc gag gag cag cgt atc gcc atg gtg agg aac ctc 6354
Glu Arg Ile Pro Glu Glu Gln Arg Ile Ala Met Val Arg Asn Leu
    2105                2110                2115

ctg gcg ccc tat gag cag cgg ccc tgg gcc cag acc aac tgg atc 6399
Leu Ala Pro Tyr Glu Gln Arg Pro Trp Ala Gln Thr Asn Trp Ile
    2120                2125                2130

ctg gtg cgg ctc tgg agg ggc tgt ggc ttc ggg tac cgc tat aca 6444
Leu Val Arg Leu Trp Arg Gly Cys Gly Phe Gly Tyr Arg Tyr Thr
    2135                2140                2145

cgg ctg cca cat ctg ctg aaa acc aaa ctt gag gac gcc aat ttg 6489
Arg Leu Pro His Leu Leu Lys Thr Lys Leu Glu Asp Ala Asn Leu
    2150                2155                2160

ccc agc ctc cag aag ccc tgc cct tcc acc ctg ctg cag cag cac 6534
Pro Ser Leu Gln Lys Pro Cys Pro Ser Thr Leu Leu Gln Gln His
    2165                2170                2175

atg gcg gac ctc cta cag cag ggt cct gat gtg gca ccc agc ttc 6579
Met Ala Asp Leu Leu Gln Gln Gly Pro Asp Val Ala Pro Ser Phe
    2180                2185                2190

ctc aac agc gtc ctc aat cag ctc aac tgg gcc ttc tct gaa ttc 6624
Leu Asn Ser Val Leu Asn Gln Leu Asn Trp Ala Phe Ser Glu Phe
    2195                2200                2205

att ggc atg atc caa gag atc cag cag gct gct gag cgc ctg gag 6669
Ile Gly Met Ile Gln Glu Ile Gln Gln Ala Ala Glu Arg Leu Glu
    2210                2215                2220

cgg aac ttt gtg gac agc cgg cag ctc aag gta tgt gcc acc tgc 6714
Arg Asn Phe Val Asp Ser Arg Gln Leu Lys Val Cys Ala Thr Cys
    2225                2230                2235

ttt gac ctc tcg gtc agc ctg ctg cgt gtc ttg gag atg act atc 6759
Phe Asp Leu Ser Val Ser Leu Leu Arg Val Leu Glu Met Thr Ile
    2240                2245                2250

aca ctg gtg cct gag ata ttc ctt gac tgg acc cgg cct acc tct 6804
Thr Leu Val Pro Glu Ile Phe Leu Asp Trp Thr Arg Pro Thr Ser
    2255                2260                2265

gag atg ctg ctg cgg cgt ctt gca cag ctg cta aac cag gtg ctg 6849
Glu Met Leu Leu Arg Arg Leu Ala Gln Leu Leu Asn Gln Val Leu
    2270                2275                2280

aac cgg gtg aca gct gag agg aac ctg ttt gat cgt gtg gtc acc 6894
Asn Arg Val Thr Ala Glu Arg Asn Leu Phe Asp Arg Val Val Thr
    2285                2290                2295

cta cgg ctg cct ggc cta gag agc gtg gac cac tat ccc att ctg 6939
Leu Arg Leu Pro Gly Leu Glu Ser Val Asp His Tyr Pro Ile Leu
    2300                2305                2310

gtg gca gtg acg ggc atc ctg gtg cag ctc ctg gtg cgt ggc cca 6984
Val Ala Val Thr Gly Ile Leu Val Gln Leu Leu Val Arg Gly Pro
    2315                2320                2325

gcc tca gag aga gag caa gcc aca tca gtg ctc ctg gca gat ccc 7029
Ala Ser Glu Arg Glu Gln Ala Thr Ser Val Leu Leu Ala Asp Pro
    2330                2335                2340

tgc ttc cag cta cgc tca ata tgc tat ctc ctg gga cag cca gag 7074
Cys Phe Gln Leu Arg Ser Ile Cys Tyr Leu Leu Gly Gln Pro Glu
    2345                2350                2355

ccc cca gca cct ggc act gct ctg cca gcc cct gac cgg aag cgc 7119
Pro Pro Ala Pro Gly Thr Ala Leu Pro Ala Pro Asp Arg Lys Arg
    2360                2365                2370

ttc tcc ctg cag agc tat gcg gat tat atc agt gcc gat gag ctg 7164
Phe Ser Leu Gln Ser Tyr Ala Asp Tyr Ile Ser Ala Asp Glu Leu
    2375                2380                2385

gcc caa gtg gaa cag atg ctg gcg cac ctg acc tct gca tct gcc 7209
Ala Gln Val Glu Gln Met Leu Ala His Leu Thr Ser Ala Ser Ala
    2390                2395                2400

cag gca gca gct gcc tcc ctg ccc acc agt gag gag gac ctc tgc 7254
Gln Ala Ala Ala Ala Ser Leu Pro Thr Ser Glu Glu Asp Leu Cys
    2405                2410                2415

ccc atc tgc tat gcc cac ccc atc tct gct gtg ttc cag ccc tgt 7299
Pro Ile Cys Tyr Ala His Pro Ile Ser Ala Val Phe Gln Pro Cys
    2420                2425                2430

ggc cac aag tcc tgc aaa gcc tgt atc aac cag cac ctg atg aac 7344
Gly His Lys Ser Cys Lys Ala Cys Ile Asn Gln His Leu Met Asn
    2435                2440                2445

aac aag gac tgc ttc ttc tgc aaa acc acc atc gtg tct gta gag 7389
Asn Lys Asp Cys Phe Phe Cys Lys Thr Thr Ile Val Ser Val Glu
    2450                2455                2460

gac tgg gag aag gga gcc aat acg agt act acc tcc tca gct gcc 7434
Asp Trp Glu Lys Gly Ala Asn Thr Ser Thr Thr Ser Ser Ala Ala
    2465                2470                2475

tag 7437

<210> 12
<211> 2478
<212> PRT

<213>  Homo sapiens

<400>  12

Met Ala Ser Asp Ser Pro Ala Arg Ser Leu Asp Glu Ile Asp Leu Ser
1               5                   10                  15

Ala Leu Arg Asp Pro Ala Gly Ile Phe Glu Leu Val Glu Leu Val Gly
            20                  25                  30

Asn Gly Thr Tyr Gly Gln Val Tyr Lys Gly Arg His Val Lys Thr Gly
        35                  40                  45

Gln Leu Ala Ala Ile Lys Val Met Asp Val Thr Gly Asp Glu Glu Glu
    50                  55                  60

Glu Ile Lys Gln Glu Ile Asn Met Leu Lys Lys Tyr Ser His His Arg
65                  70                  75                  80

Asn Ile Ala Thr Tyr Tyr Gly Ala Phe Ile Lys Lys Asn Pro Pro Gly
                85                  90                  95

Met Asp Asp Gln Leu Trp Leu Val Met Glu Phe Cys Gly Ala Gly Ser
            100                 105                 110

Val Thr Asp Leu Ile Lys Asn Thr Lys Gly Asn Thr Leu Lys Glu Glu
        115                 120                 125

Trp Ile Ala Tyr Ile Cys Arg Glu Ile Leu Arg Gly Leu Ser His Leu
    130                 135                 140

His Gln His Lys Val Ile His Arg Asp Ile Lys Gly Gln Asn Val Leu
145                 150                 155                 160

Leu Thr Glu Asn Ala Glu Val Lys Leu Val Asp Phe Gly Val Ser Ala
                165                 170                 175

Gln Leu Asp Arg Thr Val Gly Arg Arg Asn Thr Phe Ile Gly Thr Pro
            180                 185                 190

Tyr Trp Met Ala Pro Glu Val Ile Ala Cys Asp Glu Asn Pro Asp Ala
        195                 200                 205

Thr Tyr Asp Phe Lys Ser Asp Leu Trp Ser Leu Gly Ile Thr Ala Ile
    210                 215                 220

Glu Met Ala Glu Gly Ala Pro Pro Leu Cys Asp Met His Pro Met Arg
225                 230                 235                 240

Ala Leu Phe Leu Ile Pro Arg Asn Pro Ala Pro Arg Leu Lys Ser Lys
                245                 250                 255

Lys Trp Ser Lys Lys Phe Gln Ser Phe Ile Glu Ser Cys Leu Val Lys
            260                 265                 270

Asn His Ser Gln Arg Pro Ala Thr Glu Gln Leu Met Lys His Pro Phe
        275                 280                 285

Ile Arg Asp Gln Pro Asn Glu Arg Gln Val Arg Ile Gln Leu Lys Asp
    290                 295                 300

His Ile Asp Arg Thr Lys Lys Lys Arg Gly Glu Lys Asp Glu Thr Glu
305                 310                 315                 320

Tyr Glu Tyr Ser Gly Ser Glu Glu Glu Glu Glu Glu Asn Asp Ser Gly
                325                 330                 335

Glu Pro Ser Ser Ile Leu Asn Leu Pro Gly Glu Ser Thr Leu Arg Arg
            340                 345                 350

Asp Phe Leu Arg Leu Gln Leu Ala Asn Lys Glu Arg Ser Glu Ala Leu
        355                 360                 365

Arg Arg Gln Gln Leu Glu Gln Gln Gln Arg Glu Asn Glu Glu His Lys
    370                 375                 380

Arg Gln Leu Leu Ala Glu Arg Gln Lys Arg Ile Glu Glu Gln Lys Glu
385                 390                 395                 400

Gln Arg Arg Arg Leu Glu Glu Gln Gln Arg Arg Glu Lys Glu Leu Arg
                405                 410                 415

Lys Gln Gln Glu Arg Glu Gln Arg Arg His Tyr Glu Glu Gln Met Arg
            420                 425                 430

Arg Glu Glu Glu Arg Arg Arg Ala Glu His Glu Gln Glu Tyr Ile Arg
        435                 440                 445

Arg Gln Leu Glu Glu Glu Gln Arg Gln Leu Glu Ile Leu Gln Gln Gln
    450                 455                 460

Leu Leu His Glu Gln Ala Leu Leu Leu Glu Tyr Lys Arg Lys Gln Leu
465                 470                 475                 480

Glu Glu Gln Arg Gln Ala Glu Arg Leu Gln Arg Gln Leu Lys Gln Glu
                485                 490                 495

Arg Asp Tyr Leu Val Ser Leu Gln His Gln Arg Gln Glu Gln Arg Pro
            500                 505                 510

Val Glu Lys Lys Pro Leu Tyr His Tyr Lys Glu Gly Met Ser Pro Ser
        515                 520                 525

Glu Lys Pro Ala Trp Ala Lys Glu Ile Pro His Leu Val Ala Val Lys
    530                 535                 540

Ser Gln Gly Pro Ala Leu Thr Ala Ser Gln Ser Val His Glu Gln Pro
545                 550                 555                 560

Thr Lys Gly Leu Ser Gly Phe Gln Glu Ala Leu Asn Val Thr Ser His
                565                 570                 575

Arg Val Glu Met Pro Arg Gln Asn Ser Asp Pro Thr Ser Glu Asn Pro
            580                 585                 590

Pro Leu Pro Thr Arg Ile Glu Lys Phe Asp Arg Ser Ser Trp Leu Arg
        595                 600                 605

Gln Glu Glu Asp Ile Pro Pro Lys Val Pro Gln Arg Thr Thr Ser Ile
    610                 615                 620

Ser Pro Ala Leu Ala Arg Lys Asn Ser Pro Gly Asn Gly Ser Ala Leu
625                 630                 635                 640

Gly Pro Arg Leu Gly Ser Gln Pro Ile Arg Ala Ser Asn Pro Asp Leu
                645                 650                 655

Arg Arg Thr Glu Pro Ile Leu Glu Ser Pro Leu Gln Arg Thr Ser Ser
            660                 665                 670

Gly Ser Ser Ser Ser Ser Ser Thr Pro Ser Ser Gln Pro Ser Ser Gln
        675                 680                 685

Gly Gly Ser Gln Pro Gly Ser Gln Ala Gly Ser Ser Glu Arg Thr Arg
    690                 695                 700

Val Arg Ala Asn Ser Lys Ser Glu Gly Ser Pro Val Leu Pro His Glu
705                 710                 715                 720

Pro Ala Lys Val Lys Pro Glu Glu Ser Arg Asp Ile Thr Arg Pro Ser
                725                 730                 735

Arg Pro Ala Ser Tyr Lys Lys Ala Ile Asp Glu Asp Leu Thr Ala Leu

740                 745                 750

Ala Lys Glu Leu Arg Glu Leu Arg Ile Glu Glu Thr Asn Arg Pro Met
        755                 760                 765

Lys Lys Val Thr Asp Tyr Ser Ser Ser Ser Glu Glu Ser Glu Ser Ser
    770                 775                 780

Glu Glu Glu Glu Glu Asp Gly Glu Ser Glu Thr His Asp Gly Thr Val
785                 790                 795                 800

Ala Val Ser Asp Ile Pro Arg Leu Ile Pro Thr Gly Ala Pro Gly Ser
                805                 810                 815

Asn Glu Gln Tyr Asn Val Gly Met Val Gly Thr His Gly Leu Glu Thr
            820                 825                 830

Ser His Ala Asp Ser Phe Ser Gly Ser Ile Ser Arg Glu Gly Thr Leu
        835                 840                 845

Met Ile Arg Glu Thr Ser Gly Glu Lys Lys Arg Ser Gly His Ser Asp
    850                 855                 860

Ser Asn Gly Phe Ala Gly His Ile Asn Leu Pro Asp Leu Val Gln Gln
865                 870                 875                 880

Ser His Ser Pro Ala Gly Thr Pro Thr Glu Gly Leu Gly Arg Val Ser
                885                 890                 895

Thr His Ser Gln Glu Met Asp Ser Gly Thr Glu Tyr Gly Met Gly Ser
            900                 905                 910

Ser Thr Lys Ala Ser Phe Thr Pro Phe Val Asp Pro Arg Val Tyr Gln
        915                 920                 925

Thr Ser Pro Thr Asp Glu Asp Glu Glu Asp Glu Glu Ser Ser Ala Ala
    930                 935                 940

Ala Leu Phe Thr Ser Glu Leu Leu Arg Gln Glu Gln Ala Lys Leu Asn
945                 950                 955                 960

Glu Ala Arg Lys Ile Ser Val Val Asn Val Asn Pro Thr Asn Ile Arg
                965                 970                 975

Pro His Ser Asp Thr Pro Glu Ile Arg Lys Tyr Lys Lys Arg Phe Asn
            980                 985                 990

Ser Glu Ile Leu Cys Ala Ala Leu  Trp Gly Val Asn Leu  Leu Val Gly
        995                 1000                1005

Thr Glu  Asn Gly Leu Met Leu  Leu Asp Arg Ser Gly  Gln Gly Lys
    1010                 1015                 1020

Val Tyr  Asn Leu Ile Asn Arg  Arg Arg Phe Gln Gln  Met Asp Val
    1025                 1030                 1035

Leu Glu  Gly Leu Asn Val Leu  Val Thr Ile Ser Gly  Lys Lys Asn
    1040                 1045                 1050

Lys Leu  Arg Val Tyr Tyr Leu  Ser Trp Leu Arg Asn  Arg Ile Leu
    1055                 1060                 1065

His Asn  Asp Pro Glu Val Glu  Lys Lys Gln Gly Trp  Ile Thr Val
    1070                 1075                 1080

Gly Asp  Leu Glu Gly Cys Ile  His Tyr Lys Val Val  Lys Tyr Glu
    1085                 1090                 1095

Arg Ile  Lys Phe Leu Val Ile  Ala Leu Lys Asn Ala  Val Glu Ile
    1100                 1105                 1110

Tyr Ala  Trp Ala Pro Lys Pro  Tyr His Lys Phe Met  Ala Phe Lys
    1115                 1120                 1125

Ser Phe  Ala Asp Leu Gln His  Lys Pro Leu Leu Val  Asp Leu Thr
    1130                 1135                 1140

Val Glu  Glu Gly Gln Arg Leu  Lys Val Ile Phe Gly  Ser His Thr
    1145                 1150                 1155

Gly Phe  His Val Ile Asp Val  Asp Ser Gly Asn Ser  Tyr Asp Ile
    1160                 1165                 1170

Tyr Ile  Pro Ser His Ile Gln  Gly Asn Ile Thr Pro  His Ala Ile
    1175                 1180                 1185

Val Ile  Leu Pro Lys Thr Asp  Gly Met Glu Met Leu  Val Cys Tyr
    1190                 1195                 1200

Glu Asp  Glu Gly Val Tyr Val  Asn Thr Tyr Gly Arg  Ile Thr Lys
    1205                 1210                 1215

Asp Val  Val Leu Gln Trp Gly  Glu Met Pro Thr Ser  Val Ala Tyr
    1220                 1225                 1230

Ile His Ser Asn Gln Ile Met Gly Trp Gly Glu Lys Ala Ile Glu
    1235                1240                1245

Ile Arg Ser Val Glu Thr Gly His Leu Asp Gly Val Phe Met His
    1250                1255                1260

Lys Arg Ala Gln Arg Leu Lys Phe Leu Cys Glu Arg Asn Asp Lys
    1265                1270                1275

Val Ile Gly His Ser Asn Phe Gly Thr Ile Arg Ser Thr Thr Cys
    1280                1285                1290

Val Tyr Lys Gly Lys Trp Leu Tyr Glu Val Leu Ile Ser Ser Gln
    1295                1300                1305

Gly Leu Met Gln Ile Gly Trp Cys Thr Ile Ser Cys Arg Phe Asn
    1310                1315                1320

Gln Glu Glu Gly Val Gly Asp Thr His Asn Ser Tyr Ala Tyr Asp
    1325                1330                1335

Gly Asn Arg Val Arg Lys Trp Asn Val Thr Thr Thr Asn Tyr Gly
    1340                1345                1350

Lys Ala Trp Ala Ala Gly Asp Ile Val Ser Cys Leu Ile Asp Leu
    1355                1360                1365

Asp Asp Gly Thr Leu Ser Phe Cys Leu Asn Gly Val Ser Leu Gly
    1370                1375                1380

Thr Ala Phe Glu Asn Leu Ser Arg Gly Leu Gly Met Ala Tyr Phe
    1385                1390                1395

Pro Ala Ile Ser Leu Ser Phe Lys Glu Ser Val Ala Phe Asn Phe
    1400                1405                1410

Gly Ser Arg Pro Leu Arg Tyr Pro Val Ala Gly Tyr Arg Pro Leu
    1415                1420                1425

Gln Asp Pro Pro Ser Ala Asp Leu Val Arg Ala Gln Arg Leu Leu
    1430                1435                1440

Gly Cys Phe Arg Ala Val Leu Ser Val Glu Leu Asp Pro Val Glu
    1445                1450                1455

Gly Arg Leu Leu Asp Lys Glu Ser Ser Lys Trp Arg Leu Arg Gly
    1460                1465                1470

Gln Pro Thr Val Leu Leu Thr Leu Ala His Ile Phe His His Phe
1475                1480                1485

Ala Pro Leu Leu Arg Lys Val Tyr Leu Val Glu Ala Val Leu Met
1490                1495                1500

Ser Phe Leu Leu Gly Ile Val Glu Lys Gly Thr Pro Thr Gln Ala
1505                1510                1515

Gln Ser Val Val His Gln Val Leu Asp Leu Leu Trp Leu Phe Met
1520                1525                1530

Glu Asp Tyr Glu Val Gln Asp Cys Leu Lys Gln Leu Met Met Ser
1535                1540                1545

Leu Leu Arg Leu Tyr Arg Phe Ser Pro Ile Val Pro Asp Leu Gly
1550                1555                1560

Leu Gln Ile His Tyr Leu Arg Leu Thr Ile Ala Ile Leu Arg His
1565                1570                1575

Glu Lys Ser Arg Lys Phe Leu Leu Ser Asn Val Leu Phe Asp Val
1580                1585                1590

Leu Arg Ser Val Val Phe Phe Tyr Ile Lys Ser Pro Leu Arg Val
1595                1600                1605

Glu Glu Ala Gly Leu Gln Glu Leu Ile Pro Thr Thr Trp Trp Pro
1610                1615                1620

His Cys Ser Ser Arg Glu Gly Lys Glu Ser Thr Glu Met Lys Glu
1625                1630                1635

Glu Thr Ala Glu Glu Arg Leu Arg Arg Arg Ala Tyr Glu Arg Gly
1640                1645                1650

Cys Gln Arg Leu Arg Lys Arg Ile Glu Val Val Glu Glu Leu Gln
1655                1660                1665

Val Gln Ile Leu Lys Leu Leu Leu Asp Asn Lys Asp Asp Asn Gly
1670                1675                1680

Gly Glu Ala Ser Arg Tyr Ile Phe Leu Thr Lys Phe Arg Lys Phe
1685                1690                1695

Leu Gln Glu Asn Ala Ser Gly Arg Gly Asn Met Pro Met Leu Cys

1700                1705                1710

Pro Pro Glu Tyr Met Val Cys Phe Leu His Arg Leu Ile Ser Ala
    1715                1720                1725

Leu Arg Tyr Tyr Trp Asp Glu Tyr Lys Ala Ser Asn Pro His Ala
    1730                1735                1740

Ser Phe Ser Glu Glu Ala Tyr Ile Pro Pro Gln Val Phe Tyr Asn
    1745                1750                1755

Gly Lys Val Asp Tyr Phe Asp Leu Gln Arg Leu Gly Gly Leu Leu
    1760                1765                1770

Ser His Leu Arg Lys Thr Leu Lys Asp Asp Leu Ala Ser Lys Ala
    1775                1780                1785

Asn Ile Val Ile Asp Pro Leu Glu Leu Gln Ser Thr Ala Met Asp
    1790                1795                1800

Asp Leu Asp Glu Asp Glu Glu Pro Ala Pro Ala Met Ala Gln Arg
    1805                1810                1815

Pro Met Gln Ala Leu Ala Val Gly Gly Pro Leu Pro Leu Pro Arg
    1820                1825                1830

Pro Gly Trp Leu Ser Ser Pro Thr Leu Gly Arg Ala Asn Arg Phe
    1835                1840                1845

Leu Ser Thr Ala Ala Val Ser Leu Met Thr Pro Arg Arg Pro Leu
    1850                1855                1860

Ser Thr Ser Glu Lys Val Lys Val Arg Thr Leu Ser Val Glu Gln
    1865                1870                1875

Arg Thr Arg Glu Asp Ile Glu Gly Ser His Trp Asn Glu Gly Leu
    1880                1885                1890

Leu Leu Gly Arg Pro Pro Glu Glu Pro Glu Gln Pro Leu Thr Glu
    1895                1900                1905

Asn Ser Leu Leu Glu Val Leu Asp Gly Ala Val Met Met Tyr Asn
    1910                1915                1920

Leu Ser Val His Gln Gln Leu Gly Lys Met Val Gly Val Ser Asp
    1925                1930                1935

Asp Val Asn Glu Tyr Ala Met Ala Leu Arg Asp Thr Glu Asp Lys
    1940                1945                1950

Leu Arg Arg Cys Pro Lys Arg Arg Lys Asp Ile Leu Ala Glu Leu
    1955                1960                1965

Thr Lys Ser Gln Lys Val Phe Ser Glu Lys Leu Asp His Leu Ser
    1970                1975                1980

Arg Arg Leu Ala Trp Val His Ala Thr Val Tyr Ser Gln Glu Lys
    1985                1990                1995

Met Leu Asp Ile Tyr Trp Leu Leu Arg Val Cys Leu Arg Thr Ile
    2000                2005                2010

Glu His Gly Asp Arg Thr Gly Ser Leu Phe Ala Phe Met Pro Glu
    2015                2020                2025

Phe Tyr Leu Ser Val Ala Ile Asn Ser Tyr Ser Ala Leu Lys Asn
    2030                2035                2040

Tyr Phe Gly Pro Val His Ser Met Glu Glu Leu Pro Gly Tyr Glu
    2045                2050                2055

Glu Thr Leu Thr Arg Leu Ala Ala Ile Leu Ala Lys His Phe Ala
    2060                2065                2070

Asp Ala Arg Ile Val Gly Thr Asp Ile Arg Asp Ser Leu Met Gln
    2075                2080                2085

Ala Leu Ala Ser Tyr Val Cys Tyr Pro His Ser Leu Arg Ala Val
    2090                2095                2100

Glu Arg Ile Pro Glu Glu Gln Arg Ile Ala Met Val Arg Asn Leu
    2105                2110                2115

Leu Ala Pro Tyr Glu Gln Arg Pro Trp Ala Gln Thr Asn Trp Ile
    2120                2125                2130

Leu Val Arg Leu Trp Arg Gly Cys Gly Phe Gly Tyr Arg Tyr Thr
    2135                2140                2145

Arg Leu Pro His Leu Leu Lys Thr Lys Leu Glu Asp Ala Asn Leu
    2150                2155                2160

Pro Ser Leu Gln Lys Pro Cys Pro Ser Thr Leu Leu Gln Gln His
    2165                2170                2175

Met Ala Asp Leu Leu Gln Gln Gly Pro Asp Val Ala Pro Ser Phe
    2180                2185                2190

Leu Asn Ser Val Leu Asn Gln Leu Asn Trp Ala Phe Ser Glu Phe
    2195                2200                2205

Ile Gly Met Ile Gln Glu Ile Gln Gln Ala Ala Glu Arg Leu Glu
    2210                2215                2220

Arg Asn Phe Val Asp Ser Arg Gln Leu Lys Val Cys Ala Thr Cys
    2225                2230                2235

Phe Asp Leu Ser Val Ser Leu Leu Arg Val Leu Glu Met Thr Ile
    2240                2245                2250

Thr Leu Val Pro Glu Ile Phe Leu Asp Trp Thr Arg Pro Thr Ser
    2255                2260                2265

Glu Met Leu Leu Arg Arg Leu Ala Gln Leu Leu Asn Gln Val Leu
    2270                2275                2280

Asn Arg Val Thr Ala Glu Arg Asn Leu Phe Asp Arg Val Val Thr
    2285                2290                2295

Leu Arg Leu Pro Gly Leu Glu Ser Val Asp His Tyr Pro Ile Leu
    2300                2305                2310

Val Ala Val Thr Gly Ile Leu Val Gln Leu Leu Val Arg Gly Pro
    2315                2320                2325

Ala Ser Glu Arg Glu Gln Ala Thr Ser Val Leu Leu Ala Asp Pro
    2330                2335                2340

Cys Phe Gln Leu Arg Ser Ile Cys Tyr Leu Leu Gly Gln Pro Glu
    2345                2350                2355

Pro Pro Ala Pro Gly Thr Ala Leu Pro Ala Pro Asp Arg Lys Arg
    2360                2365                2370

Phe Ser Leu Gln Ser Tyr Ala Asp Tyr Ile Ser Ala Asp Glu Leu
    2375                2380                2385

Ala Gln Val Glu Gln Met Leu Ala His Leu Thr Ser Ala Ser Ala
    2390                2395                2400

Gln Ala Ala Ala Ala Ser Leu Pro Thr Ser Glu Glu Asp Leu Cys
    2405                2410                2415

Pro Ile Cys Tyr Ala His Pro Ile Ser Ala Val Phe Gln Pro Cys
    2420                2425                2430

Gly His Lys Ser Cys Lys Ala Cys Ile Asn Gln His Leu Met Asn
    2435                2440                2445

Asn Lys Asp Cys Phe Phe Cys Lys Thr Thr Ile Val Ser Val Glu
    2450                2455                2460

Asp Trp Glu Lys Gly Ala Asn Thr Ser Thr Thr Ser Ser Ala Ala
    2465                2470                2475

<210> 13
<211> 7413
<212> DNA
<213> Homo sapiens

<220>
<221> CDS
<222> (1)..(7413)

<400> 13
atg gcg agc gac tcc ccg gct cga agc ctg gat gaa ata gat ctc tcg 48
Met Ala Ser Asp Ser Pro Ala Arg Ser Leu Asp Glu Ile Asp Leu Ser
1               5                   10                  15

gct ctg agg gac ccc gca ggg atc ttt gaa ttg gtg gaa ctt gtt gga 96
Ala Leu Arg Asp Pro Ala Gly Ile Phe Glu Leu Val Glu Leu Val Gly
            20                  25                  30

aat gga aca tac ggg caa gtt tat aag ggt cgt cat gtc aaa acg ggc 144
Asn Gly Thr Tyr Gly Gln Val Tyr Lys Gly Arg His Val Lys Thr Gly
        35                  40                  45

cag ctt gca gcc atc aag gtt atg gat gtc aca ggg gat gaa gag gaa 192
Gln Leu Ala Ala Ile Lys Val Met Asp Val Thr Gly Asp Glu Glu Glu
    50                  55                  60

gaa atc aaa caa gaa att aac atg ttg aag aaa tat tct cat cac cgg 240
Glu Ile Lys Gln Glu Ile Asn Met Leu Lys Lys Tyr Ser His His Arg
65                  70                  75                  80

aat att gct aca tac tat ggt gct ttt atc aaa aag aac cca cca ggc 288
Asn Ile Ala Thr Tyr Tyr Gly Ala Phe Ile Lys Lys Asn Pro Pro Gly
                85                  90                  95

atg gat gac caa ctt tgg ttg gtg atg gag ttt tgt ggt gct ggc tct 336
Met Asp Asp Gln Leu Trp Leu Val Met Glu Phe Cys Gly Ala Gly Ser
            100                 105                 110

gtc acc gac ctg atc aag aac aca aaa ggt aac acg ttg aaa gag gag 384
Val Thr Asp Leu Ile Lys Asn Thr Lys Gly Asn Thr Leu Lys Glu Glu
        115                 120                 125

tgg att gca tac atc tgc agg gaa atc tta cgg ggg ctg agt cac ctg 432
Trp Ile Ala Tyr Ile Cys Arg Glu Ile Leu Arg Gly Leu Ser His Leu

130                 135                 140

cac cag cat aaa gtg att cat cga gat att aaa ggg caa aat gtc ttg         480
His Gln His Lys Val Ile His Arg Asp Ile Lys Gly Gln Asn Val Leu
145                 150                 155                 160

ctg act gaa aat gca gaa gtt aaa cta gtg gac ttt gga gtc agt gct         528
Leu Thr Glu Asn Ala Glu Val Lys Leu Val Asp Phe Gly Val Ser Ala
                165                 170                 175

cag ctt gat cga aca gtg ggc agg agg aat act ttc att gga act ccc         576
Gln Leu Asp Arg Thr Val Gly Arg Arg Asn Thr Phe Ile Gly Thr Pro
            180                 185                 190

tac tgg atg gca cca gaa gtt att gcc tgt gat gaa aac cca gat gcc         624
Tyr Trp Met Ala Pro Glu Val Ile Ala Cys Asp Glu Asn Pro Asp Ala
        195                 200                 205

aca tat gat ttc aag agt gac ttg tgg tct ttg ggt atc acc gcc att         672
Thr Tyr Asp Phe Lys Ser Asp Leu Trp Ser Leu Gly Ile Thr Ala Ile
    210                 215                 220

gaa atg gca gaa ggt gct ccc cct ctc tgt gac atg cac ccc atg aga         720
Glu Met Ala Glu Gly Ala Pro Pro Leu Cys Asp Met His Pro Met Arg
225                 230                 235                 240

gct ctc ttc ctc atc ccc cgg aac cca gcg cct cgg ctg aag tct aag         768
Ala Leu Phe Leu Ile Pro Arg Asn Pro Ala Pro Arg Leu Lys Ser Lys
                245                 250                 255

aag tgg tca aaa aaa ttc cag tca ttt att gag agc tgc ttg gta aag         816
Lys Trp Ser Lys Lys Phe Gln Ser Phe Ile Glu Ser Cys Leu Val Lys
            260                 265                 270

aat cac agc cag cga cca gca aca gaa caa ttg atg aag cat cca ttt         864
Asn His Ser Gln Arg Pro Ala Thr Glu Gln Leu Met Lys His Pro Phe
        275                 280                 285

ata cga gac caa cct aat gag cga cag gtc cgc att caa ctc aag gac         912
Ile Arg Asp Gln Pro Asn Glu Arg Gln Val Arg Ile Gln Leu Lys Asp
    290                 295                 300

cat att gat aga aca aag aag aag cga gga gaa aaa gat gag aca gag         960
His Ile Asp Arg Thr Lys Lys Lys Arg Gly Glu Lys Asp Glu Thr Glu
305                 310                 315                 320

tat gag tac agt gga agt gag gaa gaa gag gag gag aat gac tca gga        1008
Tyr Glu Tyr Ser Gly Ser Glu Glu Glu Glu Glu Glu Asn Asp Ser Gly
                325                 330                 335

gag ccc agc tcc atc ctg aat ctg cca ggg gag tcg acg ctg cgg agg        1056
Glu Pro Ser Ser Ile Leu Asn Leu Pro Gly Glu Ser Thr Leu Arg Arg
            340                 345                 350

gac ttt ctg agg ctg cag ctg gcc aac aag gag cgt tct gag gcc cta        1104
Asp Phe Leu Arg Leu Gln Leu Ala Asn Lys Glu Arg Ser Glu Ala Leu
        355                 360                 365

cgg agg cag cag ctg gag cag cag cag cgg gag aat gag gag cac aag        1152
Arg Arg Gln Gln Leu Glu Gln Gln Gln Arg Glu Asn Glu Glu His Lys
    370                 375                 380

cgg cag ctg ctg gcc gag cgt cag aag cgc atc gag gag cag aaa gag        1200

Arg Gln Leu Leu Ala Glu Arg Gln Lys Arg Ile Glu Glu Gln Lys Glu
385                 390                 395                 400

cag agg cgg cgg ctg gag gag caa caa agg cga gag aag gag ctg cgg      1248
Gln Arg Arg Arg Leu Glu Glu Gln Gln Arg Arg Glu Lys Glu Leu Arg
                405                 410                 415

aag cag cag gag agg gag cag cgc cgg cac tat gag gag cag atg cgc      1296
Lys Gln Gln Glu Arg Glu Gln Arg Arg His Tyr Glu Glu Gln Met Arg
            420                 425                 430

cgg gag gag gag agg agg cgt gcg gag cat gaa cag gaa tac atc agg      1344
Arg Glu Glu Glu Arg Arg Arg Ala Glu His Glu Gln Glu Tyr Ile Arg
        435                 440                 445

cga cag tta gag gag gag cag aga cag tta gag atc ttg cag cag cag      1392
Arg Gln Leu Glu Glu Glu Gln Arg Gln Leu Glu Ile Leu Gln Gln Gln
    450                 455                 460

cta ctg cat gaa caa gct cta ctt ctg gaa tat aag cgc aaa caa ttg      1440
Leu Leu His Glu Gln Ala Leu Leu Leu Glu Tyr Lys Arg Lys Gln Leu
465                 470                 475                 480

gaa gaa cag aga caa gca gaa aga ctg cag agg cag cta aag caa gaa      1488
Glu Glu Gln Arg Gln Ala Glu Arg Leu Gln Arg Gln Leu Lys Gln Glu
                485                 490                 495

aga gac tac tta gtt tcc ctt cag cat cag cgg cag gag cag agg cct      1536
Arg Asp Tyr Leu Val Ser Leu Gln His Gln Arg Gln Glu Gln Arg Pro
            500                 505                 510

gtg gag aag aag cca ctg tac cat tac aaa gaa gga atg agt cct agt      1584
Val Glu Lys Lys Pro Leu Tyr His Tyr Lys Glu Gly Met Ser Pro Ser
        515                 520                 525

gag aag cca gca tgg gcc aag gag atc cca cat ctg gta gct gta aaa      1632
Glu Lys Pro Ala Trp Ala Lys Glu Ile Pro His Leu Val Ala Val Lys
    530                 535                 540

tcc cag gga cct gcc ttg acc gcc tcc cag tca gtg cac gag cag ccc      1680
Ser Gln Gly Pro Ala Leu Thr Ala Ser Gln Ser Val His Glu Gln Pro
545                 550                 555                 560

aca aag ggc ctc tct ggg ttt cag gag gct ctg aac gtg acc tcc cac      1728
Thr Lys Gly Leu Ser Gly Phe Gln Glu Ala Leu Asn Val Thr Ser His
                565                 570                 575

cgc gtg gag atg cca cgc cag aac tca gat ccc acc tcg gaa aat cct      1776
Arg Val Glu Met Pro Arg Gln Asn Ser Asp Pro Thr Ser Glu Asn Pro
            580                 585                 590

cct ctc ccc act cgc att gaa aag ttt gac cga agc tct tgg tta cga      1824
Pro Leu Pro Thr Arg Ile Glu Lys Phe Asp Arg Ser Ser Trp Leu Arg
        595                 600                 605

cag gaa gaa gac att cca cca aag gtg cct caa aga aca act tct ata      1872
Gln Glu Glu Asp Ile Pro Pro Lys Val Pro Gln Arg Thr Thr Ser Ile
    610                 615                 620

tcc cca gca tta gcc aga aag aat tct cct ggg aat ggt agt gct ctg      1920
Ser Pro Ala Leu Ala Arg Lys Asn Ser Pro Gly Asn Gly Ser Ala Leu
625                 630                 635                 640

gga ccc aga cta gga tct caa ccc atc aga gca agc aac cct gat ctc 1968
Gly Pro Arg Leu Gly Ser Gln Pro Ile Arg Ala Ser Asn Pro Asp Leu
                645                 650                 655

cgg aga act gag ccc atc ttg gag agc ccc ttg cag agg acc agc agt 2016
Arg Arg Thr Glu Pro Ile Leu Glu Ser Pro Leu Gln Arg Thr Ser Ser
            660                 665                 670

ggc agt tcc tcc agc tcc agc acc cct agc tcc cag ccc agc tcc caa 2064
Gly Ser Ser Ser Ser Ser Ser Thr Pro Ser Ser Gln Pro Ser Ser Gln
        675                 680                 685

gga ggc tcc cag cct gga tca caa gca gga tcc agt gaa cgc acc aga 2112
Gly Gly Ser Gln Pro Gly Ser Gln Ala Gly Ser Ser Glu Arg Thr Arg
    690                 695                 700

gtt cga gcc aac agt aag tca gaa gga tca cct gtg ctc ccc cat gag 2160
Val Arg Ala Asn Ser Lys Ser Glu Gly Ser Pro Val Leu Pro His Glu
705                 710                 715                 720

cct gcc aag gtg aaa cca gaa gaa tcc agg gac att acc cgg ccc agt 2208
Pro Ala Lys Val Lys Pro Glu Glu Ser Arg Asp Ile Thr Arg Pro Ser
                725                 730                 735

cga cca gct gat ctg acg gca tta gcc aaa gaa cta aga gaa ctc cgg 2256
Arg Pro Ala Asp Leu Thr Ala Leu Ala Lys Glu Leu Arg Glu Leu Arg
            740                 745                 750

att gaa gaa aca aac cgc cca atg aag aag gtg act gat tac tcc tcc 2304
Ile Glu Glu Thr Asn Arg Pro Met Lys Lys Val Thr Asp Tyr Ser Ser
        755                 760                 765

tcc agt gag gag tca gaa agt agc gag gaa gag gag gaa gat gga gag 2352
Ser Ser Glu Glu Ser Glu Ser Ser Glu Glu Glu Glu Glu Asp Gly Glu
    770                 775                 780

agc gag acc cat gat ggg aca gtg gct gtc agc gac ata ccc aga ctg 2400
Ser Glu Thr His Asp Gly Thr Val Ala Val Ser Asp Ile Pro Arg Leu
785                 790                 795                 800

ata cca aca gga gct cca ggc agc aac gag cag tac aat gtg gga atg 2448
Ile Pro Thr Gly Ala Pro Gly Ser Asn Glu Gln Tyr Asn Val Gly Met
                805                 810                 815

gtg ggg acg cat ggg ctg gag acc tct cat gcg gac agt ttc agc ggc 2496
Val Gly Thr His Gly Leu Glu Thr Ser His Ala Asp Ser Phe Ser Gly
            820                 825                 830

agt att tca aga gaa gga acc ttg atg att aga gag acg tct gga gag 2544
Ser Ile Ser Arg Glu Gly Thr Leu Met Ile Arg Glu Thr Ser Gly Glu
        835                 840                 845

aag aag cga tct ggc cac agt gac agc aat ggc ttt gct ggc cac atc 2592
Lys Lys Arg Ser Gly His Ser Asp Ser Asn Gly Phe Ala Gly His Ile
    850                 855                 860

aac ctc cct gac ctg gtg cag cag agc cat tct cca gct gga acc ccg 2640
Asn Leu Pro Asp Leu Val Gln Gln Ser His Ser Pro Ala Gly Thr Pro
865                 870                 875                 880

act gag gga ctg ggg cgc gtc tca acc cat tcc cag gag atg gac tct 2688
Thr Glu Gly Leu Gly Arg Val Ser Thr His Ser Gln Glu Met Asp Ser
                885                 890                 895

ggg act gaa tat ggc atg ggg agc agc acc aaa gcc tcc ttc acc ccc 2736
Gly Thr Glu Tyr Gly Met Gly Ser Ser Thr Lys Ala Ser Phe Thr Pro
            900                 905                 910

ttt gtg gac ccc aga gta tac cag acg tct ccc act gat gaa gat gaa 2784
Phe Val Asp Pro Arg Val Tyr Gln Thr Ser Pro Thr Asp Glu Asp Glu
        915                 920                 925

gag gat gag gaa tca tca gcc gca gct ctg ttt act agc gaa ctt ctt 2832
Glu Asp Glu Glu Ser Ser Ala Ala Ala Leu Phe Thr Ser Glu Leu Leu
    930                 935                 940

agg caa gaa cag gcc aaa ctc aat gaa gca aga aag att tcg gtg gta 2880
Arg Gln Glu Gln Ala Lys Leu Asn Glu Ala Arg Lys Ile Ser Val Val
945                 950                 955                 960

aat gta aac cca acc aac att cgg cct cat agc gac aca cca gaa atc 2928
Asn Val Asn Pro Thr Asn Ile Arg Pro His Ser Asp Thr Pro Glu Ile
                965                 970                 975

aga aaa tac aag aaa cga ttc aac tca gaa ata ctt tgt gca gct ctg 2976
Arg Lys Tyr Lys Lys Arg Phe Asn Ser Glu Ile Leu Cys Ala Ala Leu
            980                 985                 990

tgg ggt gta aac ctt ctg gtg ggg act gaa aat ggc ctg atg ctt ttg 3024
Trp Gly Val Asn Leu Leu Val Gly Thr Glu Asn Gly Leu Met Leu Leu
        995                 1000                1005

gac cga agt ggg caa ggc aaa gtc tat aat ctg atc aac cgg agg 3069
Asp Arg Ser Gly Gln Gly Lys Val Tyr Asn Leu Ile Asn Arg Arg
    1010                1015                1020

cga ttt cag cag atg gat gtg cta gag gga ctg aat gtc ctt gtg 3114
Arg Phe Gln Gln Met Asp Val Leu Glu Gly Leu Asn Val Leu Val
    1025                1030                1035

aca att tca gga aag aag aat aag cta cga gtt tac tat ctt tca 3159
Thr Ile Ser Gly Lys Lys Asn Lys Leu Arg Val Tyr Tyr Leu Ser
    1040                1045                1050

tgg tta aga aac aga ata cta cat aat gac cca gaa gta gaa aag 3204
Trp Leu Arg Asn Arg Ile Leu His Asn Asp Pro Glu Val Glu Lys
    1055                1060                1065

aaa caa ggc tgg atc act gtt ggg gac ttg gaa ggc tgt ata cat 3249
Lys Gln Gly Trp Ile Thr Val Gly Asp Leu Glu Gly Cys Ile His
    1070                1075                1080

tat aaa gtt gtt aaa tat gaa agg atc aaa ttt ttg gtg att gcc 3294
Tyr Lys Val Val Lys Tyr Glu Arg Ile Lys Phe Leu Val Ile Ala
    1085                1090                1095

tta aag aat gct gtg gaa ata tat gct tgg gct cct aaa ccg tat 3339
Leu Lys Asn Ala Val Glu Ile Tyr Ala Trp Ala Pro Lys Pro Tyr
    1100                1105                1110

cat aaa ttc atg gca ttt aag tct ttt gca gat ctc cag cac aag 3384
His Lys Phe Met Ala Phe Lys Ser Phe Ala Asp Leu Gln His Lys
    1115                1120                1125

cct ctg cta gtt gat ctc acg gta gaa gaa ggt caa aga tta aag 3429
Pro Leu Leu Val Asp Leu Thr Val Glu Glu Gly Gln Arg Leu Lys

1130                1135                1140

gtt att ttt ggt tca cac act ggt ttc cat gta att gat gtt gat        3474
Val Ile Phe Gly Ser His Thr Gly Phe His Val Ile Asp Val Asp
    1145                1150                1155

tca gga aac tct tat gat atc tac ata cca tct cat att cag ggc        3519
Ser Gly Asn Ser Tyr Asp Ile Tyr Ile Pro Ser His Ile Gln Gly
    1160                1165                1170

aat atc act cct cat gct att gtc atc ttg cct aaa aca gat gga        3564
Asn Ile Thr Pro His Ala Ile Val Ile Leu Pro Lys Thr Asp Gly
    1175                1180                1185

atg gaa atg ctt gtt tgc tat gag gat gag ggg gtg tat gta aac        3609
Met Glu Met Leu Val Cys Tyr Glu Asp Glu Gly Val Tyr Val Asn
    1190                1195                1200

acc tat ggc cgg ata act aag gat gtg gtg ctc caa tgg gga gaa        3654
Thr Tyr Gly Arg Ile Thr Lys Asp Val Val Leu Gln Trp Gly Glu
    1205                1210                1215

atg ccc acg tct gtg gcc tac att cat tcc aat cag ata atg ggc        3699
Met Pro Thr Ser Val Ala Tyr Ile His Ser Asn Gln Ile Met Gly
    1220                1225                1230

tgg ggc gag aaa gct att gag atc cgg tca gtg gaa aca gga cat        3744
Trp Gly Glu Lys Ala Ile Glu Ile Arg Ser Val Glu Thr Gly His
    1235                1240                1245

ttg gat gga gta ttt atg cat aag cga gct caa agg tta aag ttt        3789
Leu Asp Gly Val Phe Met His Lys Arg Ala Gln Arg Leu Lys Phe
    1250                1255                1260

cta tgt gaa aga aat gat aag gtg att gga cac agc aac ttt ggc        3834
Leu Cys Glu Arg Asn Asp Lys Val Ile Gly His Ser Asn Phe Gly
    1265                1270                1275

acc atc cgc tct acc aca tgc gtg tac aaa ggg aaa tgg ctc tac        3879
Thr Ile Arg Ser Thr Thr Cys Val Tyr Lys Gly Lys Trp Leu Tyr
    1280                1285                1290

gag gtc ctc atc tcc tcc cag ggg ctc atg cag atc ggc tgg tgc        3924
Glu Val Leu Ile Ser Ser Gln Gly Leu Met Gln Ile Gly Trp Cys
    1295                1300                1305

acc atc agc tgc cgc ttc aac cag gag gag ggg gtt gga gat aca        3969
Thr Ile Ser Cys Arg Phe Asn Gln Glu Glu Gly Val Gly Asp Thr
    1310                1315                1320

cac aac tcc tat gcc tat gat ggc aac cgc gtg cgc aag tgg aat        4014
His Asn Ser Tyr Ala Tyr Asp Gly Asn Arg Val Arg Lys Trp Asn
    1325                1330                1335

gtg acc aca acg aat tat ggc aag gcg tgg gca gcg ggg gac atc        4059
Val Thr Thr Thr Asn Tyr Gly Lys Ala Trp Ala Ala Gly Asp Ile
    1340                1345                1350

gtg agc tgc ctg att gac ctg gat gat ggc act ctg tcc ttc tgc        4104
Val Ser Cys Leu Ile Asp Leu Asp Asp Gly Thr Leu Ser Phe Cys
    1355                1360                1365

ctg aac ggt gta tca ctg ggc act gcc ttt gag aac ctg tcc agg        4149

Leu Asn Gly Val Ser Leu Gly Thr Ala Phe Glu Asn Leu Ser Arg
    1370                1375                1380

ggc ctg ggt atg gcc tac ttc cca gcc atc agc ctc tct ttc aag 4194
Gly Leu Gly Met Ala Tyr Phe Pro Ala Ile Ser Leu Ser Phe Lys
    1385                1390                1395

gag tcc gtg gcc ttc aac ttt ggc agc cgt cct ctg cgc tac cca 4239
Glu Ser Val Ala Phe Asn Phe Gly Ser Arg Pro Leu Arg Tyr Pro
    1400                1405                1410

gtg gca ggc tac cgg ccc ctg cag gac cca ccg agt gct gac ctg 4284
Val Ala Gly Tyr Arg Pro Leu Gln Asp Pro Pro Ser Ala Asp Leu
    1415                1420                1425

gtg cgg gca cag agg ttg ctg ggc tgc ttc cgg gca gtg ctg agt 4329
Val Arg Ala Gln Arg Leu Leu Gly Cys Phe Arg Ala Val Leu Ser
    1430                1435                1440

gtg gag ctg gac cct gtg gag ggg cgg ctg ttg gac aag gag agc 4374
Val Glu Leu Asp Pro Val Glu Gly Arg Leu Leu Asp Lys Glu Ser
    1445                1450                1455

tcc aag tgg cgg ttg cgg ggc cag ccc acc gtc ctc ctc aca ctg 4419
Ser Lys Trp Arg Leu Arg Gly Gln Pro Thr Val Leu Leu Thr Leu
    1460                1465                1470

gcc cac atc ttc cat cac ttt gca ccg ctt ctg cgc aag gtg tat 4464
Ala His Ile Phe His His Phe Ala Pro Leu Leu Arg Lys Val Tyr
    1475                1480                1485

ctg gtg gag gct gtg ctc atg agc ttc ttg ctg ggc atc gtg gag 4509
Leu Val Glu Ala Val Leu Met Ser Phe Leu Leu Gly Ile Val Glu
    1490                1495                1500

aag ggc aca ccc aca cag gca cag tcc gtg gtg cac cag gtc ctg 4554
Lys Gly Thr Pro Thr Gln Ala Gln Ser Val Val His Gln Val Leu
    1505                1510                1515

gac ctc ttg tgg ctc ttc atg gag gac tac gag gta caa gat tgc 4599
Asp Leu Leu Trp Leu Phe Met Glu Asp Tyr Glu Val Gln Asp Cys
    1520                1525                1530

ctc aag cag ttg atg atg tct ctg ctt cgg ctg tac cga ttc tca 4644
Leu Lys Gln Leu Met Met Ser Leu Leu Arg Leu Tyr Arg Phe Ser
    1535                1540                1545

ccc att gtc cca gac ctg ggc cta cag atc cat tac ctg cgg ctc 4689
Pro Ile Val Pro Asp Leu Gly Leu Gln Ile His Tyr Leu Arg Leu
    1550                1555                1560

act atc gcc atc ctg agg cat gag aag tcc cgc aag ttt ctg ctt 4734
Thr Ile Ala Ile Leu Arg His Glu Lys Ser Arg Lys Phe Leu Leu
    1565                1570                1575

agc aat gtc ctc ttc gac gtg ctc cgc tcc gtc gtc ttc ttt tac 4779
Ser Asn Val Leu Phe Asp Val Leu Arg Ser Val Val Phe Phe Tyr
    1580                1585                1590

atc aag agc ccc ctg cgt gtg gag gag gcc ggc ctg cag gag ctc 4824
Ile Lys Ser Pro Leu Arg Val Glu Glu Ala Gly Leu Gln Glu Leu
    1595                1600                1605

att ccc acc acc tgg tgg ccc cac tgc tcc agt agg gag ggc aaa 4869
Ile Pro Thr Thr Trp Trp Pro His Cys Ser Ser Arg Glu Gly Lys
    1610                1615                1620

gag agc acg gag atg aag gag gag acc gca gag gag cgg ctg cgg 4914
Glu Ser Thr Glu Met Lys Glu Glu Thr Ala Glu Glu Arg Leu Arg
    1625                1630                1635

cgg cga gcc tac gaa cgg ggc tgt cag cgg ctc agg aag cgc atc 4959
Arg Arg Ala Tyr Glu Arg Gly Cys Gln Arg Leu Arg Lys Arg Ile
    1640                1645                1650

gaa gtg gtg gaa gaa cta cag gtc cag atc ctg aag ctg ctg ctg 5004
Glu Val Val Glu Glu Leu Gln Val Gln Ile Leu Lys Leu Leu Leu
    1655                1660                1665

gac aat aaa gat gac aat ggg ggt gaa gct tct agg tat atc ttc 5049
Asp Asn Lys Asp Asp Asn Gly Gly Glu Ala Ser Arg Tyr Ile Phe
    1670                1675                1680

ctg acc aag ttt cgc aag ttt ctg cag gag aac gcc agt ggc cgg 5094
Leu Thr Lys Phe Arg Lys Phe Leu Gln Glu Asn Ala Ser Gly Arg
    1685                1690                1695

ggg aac atg ccc atg ctc tgc ccc cct gag tac atg gtc tgc ttc 5139
Gly Asn Met Pro Met Leu Cys Pro Pro Glu Tyr Met Val Cys Phe
    1700                1705                1710

tta cac cgg ctg atc tct gcc ctg cgc tac tat tgg gat gaa tac 5184
Leu His Arg Leu Ile Ser Ala Leu Arg Tyr Tyr Trp Asp Glu Tyr
    1715                1720                1725

aag gct tcc aat cct cat gct tcc ttc agt gag gag gcc tac atc 5229
Lys Ala Ser Asn Pro His Ala Ser Phe Ser Glu Glu Ala Tyr Ile
    1730                1735                1740

ccg ccc cag gtc ttc tat aat ggc aag gtg gac tac ttt gac ctg 5274
Pro Pro Gln Val Phe Tyr Asn Gly Lys Val Asp Tyr Phe Asp Leu
    1745                1750                1755

cag cgc ctg ggg ggc ctc ctc tcg cac ctg cgg aag acc ctc aaa 5319
Gln Arg Leu Gly Gly Leu Leu Ser His Leu Arg Lys Thr Leu Lys
    1760                1765                1770

gat gac ctt gct tcc aaa gcc aac att gtg atc gac cca ctg gag 5364
Asp Asp Leu Ala Ser Lys Ala Asn Ile Val Ile Asp Pro Leu Glu
    1775                1780                1785

ctc cag tca acc gcc atg gat gac cta gat gag gat gag gag cca 5409
Leu Gln Ser Thr Ala Met Asp Asp Leu Asp Glu Asp Glu Glu Pro
    1790                1795                1800

gcc cca gct atg gcc cag cgc ccc atg cag gcc ctg gct gtt ggg 5454
Ala Pro Ala Met Ala Gln Arg Pro Met Gln Ala Leu Ala Val Gly
    1805                1810                1815

ggg cca ctg ccc ctg ccc cgg ccc ggc tgg ctc agt tct cca act 5499
Gly Pro Leu Pro Leu Pro Arg Pro Gly Trp Leu Ser Ser Pro Thr
    1820                1825                1830

ttg ggc cga gcc aac cgc ttc ctc agc aca gcg gct gtg agc ctc 5544
Leu Gly Arg Ala Asn Arg Phe Leu Ser Thr Ala Ala Val Ser Leu
    1835                1840                1845

atg acc cca cgg cgg cct ctg agc acc tcg gag aaa gtg aag gtc 5589
Met Thr Pro Arg Arg Pro Leu Ser Thr Ser Glu Lys Val Lys Val
    1850                1855                1860

cgc acg ctg agc gtg gag cag agg acc cgt gag gac att gaa ggc 5634
Arg Thr Leu Ser Val Glu Gln Arg Thr Arg Glu Asp Ile Glu Gly
    1865                1870                1875

agc cac tgg aat gag ggc ttg ctg ctg ggg cgg ccc ccc gag gag 5679
Ser His Trp Asn Glu Gly Leu Leu Leu Gly Arg Pro Pro Glu Glu
    1880                1885                1890

cct gag cag ccc ctc acc gag aac tcg ctg ctg gaa gtc ctg gat 5724
Pro Glu Gln Pro Leu Thr Glu Asn Ser Leu Leu Glu Val Leu Asp
    1895                1900                1905

ggg gcg gtc atg atg tac aac ctc agc gta cac cag cag ctg ggc 5769
Gly Ala Val Met Met Tyr Asn Leu Ser Val His Gln Gln Leu Gly
    1910                1915                1920

aag atg gtg ggt gtc tcc gat gat gtc aat gaa tac gct atg gct 5814
Lys Met Val Gly Val Ser Asp Asp Val Asn Glu Tyr Ala Met Ala
    1925                1930                1935

ctg agg gac aca gag gac aag ctc cgc cgg tgc ccc aag agg agg 5859
Leu Arg Asp Thr Glu Asp Lys Leu Arg Arg Cys Pro Lys Arg Arg
    1940                1945                1950

aag gac atc ctt gca gag ttg acc aag agc cag aag gtt ttc tca 5904
Lys Asp Ile Leu Ala Glu Leu Thr Lys Ser Gln Lys Val Phe Ser
    1955                1960                1965

gaa aag ctg gac cac ctg agc cgc cgt ctt gcc tgg gtc cat gcc 5949
Glu Lys Leu Asp His Leu Ser Arg Arg Leu Ala Trp Val His Ala
    1970                1975                1980

act gtc tac tcc cag gag aag atg ctg gac atc tac tgg ctg ctg 5994
Thr Val Tyr Ser Gln Glu Lys Met Leu Asp Ile Tyr Trp Leu Leu
    1985                1990                1995

cgc gtc tgc ctg cgg acc att gag cac ggt gat cgc aca ggg tct 6039
Arg Val Cys Leu Arg Thr Ile Glu His Gly Asp Arg Thr Gly Ser
    2000                2005                2010

ctc ttt gcc ttc atg ccc gag ttc tac ctg agc gtg gcc atc aac 6084
Leu Phe Ala Phe Met Pro Glu Phe Tyr Leu Ser Val Ala Ile Asn
    2015                2020                2025

agc tac agt gct ctc aag aat tac ttt ggt ccc gtg cac agc atg 6129
Ser Tyr Ser Ala Leu Lys Asn Tyr Phe Gly Pro Val His Ser Met
    2030                2035                2040

gag gag ctc cca ggc tat gaa gag acc ctg acc cgc ctg gct gcc 6174
Glu Glu Leu Pro Gly Tyr Glu Glu Thr Leu Thr Arg Leu Ala Ala
    2045                2050                2055

att ctc gcc aaa cac ttt gcc gac gca cgc att gtg ggc act gac 6219
Ile Leu Ala Lys His Phe Ala Asp Ala Arg Ile Val Gly Thr Asp
    2060                2065                2070

atc cga gac tca ctg atg cag gcc ctg gcc agc tac gtg tgc tac 6264
Ile Arg Asp Ser Leu Met Gln Ala Leu Ala Ser Tyr Val Cys Tyr

2075                    2080                    2085

cca cac tcc ctg cgg gct gtg gag cga atc ccc gag gag cag cgt      6309
Pro His Ser Leu Arg Ala Val Glu Arg Ile Pro Glu Glu Gln Arg
    2090                2095                2100

atc gcc atg gtg agg aac ctc ctg gcg ccc tat gag cag cgg ccc      6354
Ile Ala Met Val Arg Asn Leu Leu Ala Pro Tyr Glu Gln Arg Pro
    2105                2110                2115

tgg gcc cag acc aac tgg atc ctg gtg cgg ctc tgg agg ggc tgt      6399
Trp Ala Gln Thr Asn Trp Ile Leu Val Arg Leu Trp Arg Gly Cys
    2120                2125                2130

ggc ttc ggg tac cgc tat aca cgg ctg cca cat ctg ctg aaa acc      6444
Gly Phe Gly Tyr Arg Tyr Thr Arg Leu Pro His Leu Leu Lys Thr
    2135                2140                2145

aaa ctt gag gac gcc aat ttg ccc agc ctc cag aag ccc tgc cct      6489
Lys Leu Glu Asp Ala Asn Leu Pro Ser Leu Gln Lys Pro Cys Pro
    2150                2155                2160

tcc acc ctg ctg cag cag cac atg gcg gac ctc cta cag cag ggt      6534
Ser Thr Leu Leu Gln Gln His Met Ala Asp Leu Leu Gln Gln Gly
    2165                2170                2175

cct gat gtg gca ccc agc ttc ctc aac agc gtc ctc aat cag ctc      6579
Pro Asp Val Ala Pro Ser Phe Leu Asn Ser Val Leu Asn Gln Leu
    2180                2185                2190

aac tgg gcc ttc tct gaa ttc att ggc atg atc caa gag atc cag      6624
Asn Trp Ala Phe Ser Glu Phe Ile Gly Met Ile Gln Glu Ile Gln
    2195                2200                2205

cag gct gct gag cgc ctg gag cgg aac ttt gtg gac agc cgg cag      6669
Gln Ala Ala Glu Arg Leu Glu Arg Asn Phe Val Asp Ser Arg Gln
    2210                2215                2220

ctc aag gta tgt gcc acc tgc ttt gac ctc tcg gtc agc ctg ctg      6714
Leu Lys Val Cys Ala Thr Cys Phe Asp Leu Ser Val Ser Leu Leu
    2225                2230                2235

cgt gtc ttg gag atg act atc aca ctg gtg cct gag ata ttc ctt      6759
Arg Val Leu Glu Met Thr Ile Thr Leu Val Pro Glu Ile Phe Leu
    2240                2245                2250

gac tgg acc cgg cct acc tct gag atg ctg ctg cgg cgt ctt gca      6804
Asp Trp Thr Arg Pro Thr Ser Glu Met Leu Leu Arg Arg Leu Ala
    2255                2260                2265

cag ctg cta aac cag gtg ctg aac cgg gtg aca gct gag agg aac      6849
Gln Leu Leu Asn Gln Val Leu Asn Arg Val Thr Ala Glu Arg Asn
    2270                2275                2280

ctg ttt gat cgt gtg gtc acc cta cgg ctg cct ggc cta gag agc      6894
Leu Phe Asp Arg Val Val Thr Leu Arg Leu Pro Gly Leu Glu Ser
    2285                2290                2295

gtg gac cac tat ccc att ctg gtg gca gtg acg ggc atc ctg gtg      6939
Val Asp His Tyr Pro Ile Leu Val Ala Val Thr Gly Ile Leu Val
    2300                2305                2310

cag ctc ctg gtg cgt ggc cca gcc tca gag aga gag caa gcc aca      6984

Gln Leu Leu Val Arg Gly Pro Ala Ser Glu Arg Glu Gln Ala Thr
    2315                2320                2325

tca gtg ctc ctg gca gat ccc tgc ttc cag cta cgc tca ata tgc      7029
Ser Val Leu Leu Ala Asp Pro Cys Phe Gln Leu Arg Ser Ile Cys
    2330                2335                2340

tat ctc ctg gga cag cca gag ccc cca gca cct ggc act gct ctg      7074
Tyr Leu Leu Gly Gln Pro Glu Pro Pro Ala Pro Gly Thr Ala Leu
    2345                2350                2355

cca gcc cct gac cgg aag cgc ttc tcc ctg cag agc tat gcg gat      7119
Pro Ala Pro Asp Arg Lys Arg Phe Ser Leu Gln Ser Tyr Ala Asp
    2360                2365                2370

tat atc agt gcc gat gag ctg gcc caa gtg gaa cag atg ctg gcg      7164
Tyr Ile Ser Ala Asp Glu Leu Ala Gln Val Glu Gln Met Leu Ala
    2375                2380                2385

cac ctg acc tct gca tct gcc cag gca gca gct gcc tcc ctg ccc      7209
His Leu Thr Ser Ala Ser Ala Gln Ala Ala Ala Ala Ser Leu Pro
    2390                2395                2400

acc agt gag gag gac ctc tgc ccc atc tgc tat gcc cac ccc atc      7254
Thr Ser Glu Glu Asp Leu Cys Pro Ile Cys Tyr Ala His Pro Ile
    2405                2410                2415

tct gct gtg ttc cag ccc tgt ggc cac aag tcc tgc aaa gcc tgt      7299
Ser Ala Val Phe Gln Pro Cys Gly His Lys Ser Cys Lys Ala Cys
    2420                2425                2430

atc aac cag cac ctg atg aac aac aag gac tgc ttc ttc tgc aaa      7344
Ile Asn Gln His Leu Met Asn Asn Lys Asp Cys Phe Phe Cys Lys
    2435                2440                2445

acc acc atc gtg tct gta gag gac tgg gag aag gga gcc aat acg      7389
Thr Thr Ile Val Ser Val Glu Asp Trp Glu Lys Gly Ala Asn Thr
    2450                2455                2460

agt act acc tcc tca gct gcc tag                                  7413
Ser Thr Thr Ser Ser Ala Ala
    2465                2470


<210>  14
<211>  2470
<212>  PRT
<213>  Homo sapiens

<400>  14

Met Ala Ser Asp Ser Pro Ala Arg Ser Leu Asp Glu Ile Asp Leu Ser
1               5                   10                  15


Ala Leu Arg Asp Pro Ala Gly Ile Phe Glu Leu Val Glu Leu Val Gly
            20                  25                  30


Asn Gly Thr Tyr Gly Gln Val Tyr Lys Gly Arg His Val Lys Thr Gly
        35                  40                  45

Gln Leu Ala Ala Ile Lys Val Met Asp Val Thr Gly Asp Glu Glu Glu
    50                  55                  60

Glu Ile Lys Gln Glu Ile Asn Met Leu Lys Lys Tyr Ser His His Arg
65                  70                  75                  80

Asn Ile Ala Thr Tyr Tyr Gly Ala Phe Ile Lys Lys Asn Pro Pro Gly
                85                  90                  95

Met Asp Asp Gln Leu Trp Leu Val Met Glu Phe Cys Gly Ala Gly Ser
            100                 105                 110

Val Thr Asp Leu Ile Lys Asn Thr Lys Gly Asn Thr Leu Lys Glu Glu
        115                 120                 125

Trp Ile Ala Tyr Ile Cys Arg Glu Ile Leu Arg Gly Leu Ser His Leu
    130                 135                 140

His Gln His Lys Val Ile His Arg Asp Ile Lys Gly Gln Asn Val Leu
145                 150                 155                 160

Leu Thr Glu Asn Ala Glu Val Lys Leu Val Asp Phe Gly Val Ser Ala
                165                 170                 175

Gln Leu Asp Arg Thr Val Gly Arg Arg Asn Thr Phe Ile Gly Thr Pro
            180                 185                 190

Tyr Trp Met Ala Pro Glu Val Ile Ala Cys Asp Glu Asn Pro Asp Ala
        195                 200                 205

Thr Tyr Asp Phe Lys Ser Asp Leu Trp Ser Leu Gly Ile Thr Ala Ile
    210                 215                 220

Glu Met Ala Glu Gly Ala Pro Pro Leu Cys Asp Met His Pro Met Arg
225                 230                 235                 240

Ala Leu Phe Leu Ile Pro Arg Asn Pro Ala Pro Arg Leu Lys Ser Lys
                245                 250                 255

Lys Trp Ser Lys Lys Phe Gln Ser Phe Ile Glu Ser Cys Leu Val Lys
            260                 265                 270

Asn His Ser Gln Arg Pro Ala Thr Glu Gln Leu Met Lys His Pro Phe
        275                 280                 285

Ile Arg Asp Gln Pro Asn Glu Arg Gln Val Arg Ile Gln Leu Lys Asp
    290                 295                 300

His Ile Asp Arg Thr Lys Lys Lys Arg Gly Glu Lys Asp Glu Thr Glu
305                 310                 315                 320

Tyr Glu Tyr Ser Gly Ser Glu Glu Glu Glu Glu Glu Asn Asp Ser Gly
                325                 330                 335

Glu Pro Ser Ser Ile Leu Asn Leu Pro Gly Glu Ser Thr Leu Arg Arg
            340                 345                 350

Asp Phe Leu Arg Leu Gln Leu Ala Asn Lys Glu Arg Ser Glu Ala Leu
        355                 360                 365

Arg Arg Gln Gln Leu Glu Gln Gln Gln Arg Glu Asn Glu Glu His Lys
    370                 375                 380

Arg Gln Leu Leu Ala Glu Arg Gln Lys Arg Ile Glu Glu Gln Lys Glu
385                 390                 395                 400

Gln Arg Arg Arg Leu Glu Glu Gln Gln Arg Arg Glu Lys Glu Leu Arg
                405                 410                 415

Lys Gln Gln Glu Arg Glu Gln Arg Arg His Tyr Glu Glu Gln Met Arg
            420                 425                 430

Arg Glu Glu Glu Arg Arg Arg Ala Glu His Glu Gln Glu Tyr Ile Arg
        435                 440                 445

Arg Gln Leu Glu Glu Glu Gln Arg Gln Leu Glu Ile Leu Gln Gln Gln
    450                 455                 460

Leu Leu His Glu Gln Ala Leu Leu Leu Glu Tyr Lys Arg Lys Gln Leu
465                 470                 475                 480

Glu Glu Gln Arg Gln Ala Glu Arg Leu Gln Arg Gln Leu Lys Gln Glu
                485                 490                 495

Arg Asp Tyr Leu Val Ser Leu Gln His Gln Arg Gln Glu Gln Arg Pro
            500                 505                 510

Val Glu Lys Lys Pro Leu Tyr His Tyr Lys Glu Gly Met Ser Pro Ser
        515                 520                 525

Glu Lys Pro Ala Trp Ala Lys Glu Ile Pro His Leu Val Ala Val Lys
    530                 535                 540

Ser Gln Gly Pro Ala Leu Thr Ala Ser Gln Ser Val His Glu Gln Pro
545                 550                 555                 560

Thr Lys Gly Leu Ser Gly Phe Gln Glu Ala Leu Asn Val Thr Ser His
                565                 570                 575

Arg Val Glu Met Pro Arg Gln Asn Ser Asp Pro Thr Ser Glu Asn Pro
            580                 585                 590

Pro Leu Pro Thr Arg Ile Glu Lys Phe Asp Arg Ser Ser Trp Leu Arg
        595                 600                 605

Gln Glu Glu Asp Ile Pro Pro Lys Val Pro Gln Arg Thr Thr Ser Ile
    610                 615                 620

Ser Pro Ala Leu Ala Arg Lys Asn Ser Pro Gly Asn Gly Ser Ala Leu
625                 630                 635                 640

Gly Pro Arg Leu Gly Ser Gln Pro Ile Arg Ala Ser Asn Pro Asp Leu
                645                 650                 655

Arg Arg Thr Glu Pro Ile Leu Glu Ser Pro Leu Gln Arg Thr Ser Ser
            660                 665                 670

Gly Ser Ser Ser Ser Ser Ser Thr Pro Ser Ser Gln Pro Ser Ser Gln
        675                 680                 685

Gly Gly Ser Gln Pro Gly Ser Gln Ala Gly Ser Ser Glu Arg Thr Arg
    690                 695                 700

Val Arg Ala Asn Ser Lys Ser Glu Gly Ser Pro Val Leu Pro His Glu
705                 710                 715                 720

Pro Ala Lys Val Lys Pro Glu Glu Ser Arg Asp Ile Thr Arg Pro Ser
                725                 730                 735

Arg Pro Ala Asp Leu Thr Ala Leu Ala Lys Glu Leu Arg Glu Leu Arg
            740                 745                 750

Ile Glu Glu Thr Asn Arg Pro Met Lys Lys Val Thr Asp Tyr Ser Ser
        755                 760                 765

Ser Ser Glu Glu Ser Glu Ser Ser Glu Glu Glu Glu Glu Asp Gly Glu
    770                 775                 780

Ser Glu Thr His Asp Gly Thr Val Ala Val Ser Asp Ile Pro Arg Leu
785                 790                 795                 800

Ile Pro Thr Gly Ala Pro Gly Ser Asn Glu Gln Tyr Asn Val Gly Met

                805                 810                 815

Val Gly Thr His Gly Leu Glu Thr Ser His Ala Asp Ser Phe Ser Gly
            820                 825                 830

Ser Ile Ser Arg Glu Gly Thr Leu Met Ile Arg Glu Thr Ser Gly Glu
        835                 840                 845

Lys Lys Arg Ser Gly His Ser Asp Ser Asn Gly Phe Ala Gly His Ile
    850                 855                 860

Asn Leu Pro Asp Leu Val Gln Gln Ser His Ser Pro Ala Gly Thr Pro
865                 870                 875                 880

Thr Glu Gly Leu Gly Arg Val Ser Thr His Ser Gln Glu Met Asp Ser
                885                 890                 895

Gly Thr Glu Tyr Gly Met Gly Ser Ser Thr Lys Ala Ser Phe Thr Pro
            900                 905                 910

Phe Val Asp Pro Arg Val Tyr Gln Thr Ser Pro Thr Asp Glu Asp Glu
        915                 920                 925

Glu Asp Glu Glu Ser Ser Ala Ala Ala Leu Phe Thr Ser Glu Leu Leu
    930                 935                 940

Arg Gln Glu Gln Ala Lys Leu Asn Glu Ala Arg Lys Ile Ser Val Val
945                 950                 955                 960

Asn Val Asn Pro Thr Asn Ile Arg Pro His Ser Asp Thr Pro Glu Ile
                965                 970                 975

Arg Lys Tyr Lys Lys Arg Phe Asn Ser Glu Ile Leu Cys Ala Ala Leu
            980                 985                 990

Trp Gly Val Asn Leu Leu Val Gly Thr Glu Asn Gly Leu Met Leu Leu
        995                 1000                1005

Asp Arg Ser Gly Gln Gly Lys Val Tyr Asn Leu Ile Asn Arg Arg
    1010                1015                1020

Arg Phe Gln Gln Met Asp Val Leu Glu Gly Leu Asn Val Leu Val
    1025                1030                1035

Thr Ile Ser Gly Lys Lys Asn Lys Leu Arg Val Tyr Tyr Leu Ser
    1040                1045                1050

Trp Leu Arg Asn Arg Ile Leu His Asn Asp Pro Glu Val Glu Lys
    1055                1060                1065

Lys Gln Gly Trp Ile Thr Val Gly Asp Leu Glu Gly Cys Ile His
    1070                1075                1080

Tyr Lys Val Val Lys Tyr Glu Arg Ile Lys Phe Leu Val Ile Ala
    1085                1090                1095

Leu Lys Asn Ala Val Glu Ile Tyr Ala Trp Ala Pro Lys Pro Tyr
    1100                1105                1110

His Lys Phe Met Ala Phe Lys Ser Phe Ala Asp Leu Gln His Lys
    1115                1120                1125

Pro Leu Leu Val Asp Leu Thr Val Glu Glu Gly Gln Arg Leu Lys
    1130                1135                1140

Val Ile Phe Gly Ser His Thr Gly Phe His Val Ile Asp Val Asp
    1145                1150                1155

Ser Gly Asn Ser Tyr Asp Ile Tyr Ile Pro Ser His Ile Gln Gly
    1160                1165                1170

Asn Ile Thr Pro His Ala Ile Val Ile Leu Pro Lys Thr Asp Gly
    1175                1180                1185

Met Glu Met Leu Val Cys Tyr Glu Asp Glu Gly Val Tyr Val Asn
    1190                1195                1200

Thr Tyr Gly Arg Ile Thr Lys Asp Val Val Leu Gln Trp Gly Glu
    1205                1210                1215

Met Pro Thr Ser Val Ala Tyr Ile His Ser Asn Gln Ile Met Gly
    1220                1225                1230

Trp Gly Glu Lys Ala Ile Glu Ile Arg Ser Val Glu Thr Gly His
    1235                1240                1245

Leu Asp Gly Val Phe Met His Lys Arg Ala Gln Arg Leu Lys Phe
    1250                1255                1260

Leu Cys Glu Arg Asn Asp Lys Val Ile Gly His Ser Asn Phe Gly
    1265                1270                1275

Thr Ile Arg Ser Thr Thr Cys Val Tyr Lys Gly Lys Trp Leu Tyr
    1280                1285                1290

Glu Val Leu Ile Ser Ser Gln Gly Leu Met Gln Ile Gly Trp Cys
    1295                1300                1305

Thr Ile Ser Cys Arg Phe Asn Gln Glu Glu Gly Val Gly Asp Thr
    1310                1315                1320

His Asn Ser Tyr Ala Tyr Asp Gly Asn Arg Val Arg Lys Trp Asn
    1325                1330                1335

Val Thr Thr Thr Asn Tyr Gly Lys Ala Trp Ala Ala Gly Asp Ile
    1340                1345                1350

Val Ser Cys Leu Ile Asp Leu Asp Asp Gly Thr Leu Ser Phe Cys
    1355                1360                1365

Leu Asn Gly Val Ser Leu Gly Thr Ala Phe Glu Asn Leu Ser Arg
    1370                1375                1380

Gly Leu Gly Met Ala Tyr Phe Pro Ala Ile Ser Leu Ser Phe Lys
    1385                1390                1395

Glu Ser Val Ala Phe Asn Phe Gly Ser Arg Pro Leu Arg Tyr Pro
    1400                1405                1410

Val Ala Gly Tyr Arg Pro Leu Gln Asp Pro Pro Ser Ala Asp Leu
    1415                1420                1425

Val Arg Ala Gln Arg Leu Leu Gly Cys Phe Arg Ala Val Leu Ser
    1430                1435                1440

Val Glu Leu Asp Pro Val Glu Gly Arg Leu Leu Asp Lys Glu Ser
    1445                1450                1455

Ser Lys Trp Arg Leu Arg Gly Gln Pro Thr Val Leu Leu Thr Leu
    1460                1465                1470

Ala His Ile Phe His His Phe Ala Pro Leu Leu Arg Lys Val Tyr
    1475                1480                1485

Leu Val Glu Ala Val Leu Met Ser Phe Leu Leu Gly Ile Val Glu
    1490                1495                1500

Lys Gly Thr Pro Thr Gln Ala Gln Ser Val Val His Gln Val Leu
    1505                1510                1515

Asp Leu Leu Trp Leu Phe Met Glu Asp Tyr Glu Val Gln Asp Cys
    1520                1525                1530

Leu Lys Gln Leu Met Met Ser Leu Leu Arg Leu Tyr Arg Phe Ser
    1535                1540                1545

Pro Ile Val Pro Asp Leu Gly Leu Gln Ile His Tyr Leu Arg Leu
    1550                1555                1560

Thr Ile Ala Ile Leu Arg His Glu Lys Ser Arg Lys Phe Leu Leu
    1565                1570                1575

Ser Asn Val Leu Phe Asp Val Leu Arg Ser Val Val Phe Phe Tyr
    1580                1585                1590

Ile Lys Ser Pro Leu Arg Val Glu Glu Ala Gly Leu Gln Glu Leu
    1595                1600                1605

Ile Pro Thr Thr Trp Trp Pro His Cys Ser Ser Arg Glu Gly Lys
    1610                1615                1620

Glu Ser Thr Glu Met Lys Glu Glu Thr Ala Glu Glu Arg Leu Arg
    1625                1630                1635

Arg Arg Ala Tyr Glu Arg Gly Cys Gln Arg Leu Arg Lys Arg Ile
    1640                1645                1650

Glu Val Val Glu Glu Leu Gln Val Gln Ile Leu Lys Leu Leu Leu
    1655                1660                1665

Asp Asn Lys Asp Asp Asn Gly Gly Glu Ala Ser Arg Tyr Ile Phe
    1670                1675                1680

Leu Thr Lys Phe Arg Lys Phe Leu Gln Glu Asn Ala Ser Gly Arg
    1685                1690                1695

Gly Asn Met Pro Met Leu Cys Pro Pro Glu Tyr Met Val Cys Phe
    1700                1705                1710

Leu His Arg Leu Ile Ser Ala Leu Arg Tyr Tyr Trp Asp Glu Tyr
    1715                1720                1725

Lys Ala Ser Asn Pro His Ala Ser Phe Ser Glu Glu Ala Tyr Ile
    1730                1735                1740

Pro Pro Gln Val Phe Tyr Asn Gly Lys Val Asp Tyr Phe Asp Leu
    1745                1750                1755

Gln Arg Leu Gly Gly Leu Leu Ser His Leu Arg Lys Thr Leu Lys

1760                1765                1770

Asp Asp  Leu Ala Ser Lys Ala  Asn Ile Val Ile Asp  Pro Leu Glu
    1775                1780                1785

Leu Gln  Ser Thr Ala Met Asp  Asp Leu Asp Glu Asp  Glu Glu Pro
    1790                1795                1800

Ala Pro  Ala Met Ala Gln Arg  Pro Met Gln Ala Leu  Ala Val Gly
    1805                1810                1815

Gly Pro  Leu Pro Leu Pro Arg  Pro Gly Trp Leu Ser  Ser Pro Thr
    1820                1825                1830

Leu Gly  Arg Ala Asn Arg Phe  Leu Ser Thr Ala Ala  Val Ser Leu
    1835                1840                1845

Met Thr  Pro Arg Arg Pro Leu  Ser Thr Ser Glu Lys  Val Lys Val
    1850                1855                1860

Arg Thr  Leu Ser Val Glu Gln  Arg Thr Arg Glu Asp  Ile Glu Gly
    1865                1870                1875

Ser His  Trp Asn Glu Gly Leu  Leu Leu Gly Arg Pro  Pro Glu Glu
    1880                1885                1890

Pro Glu  Gln Pro Leu Thr Glu  Asn Ser Leu Leu Glu  Val Leu Asp
    1895                1900                1905

Gly Ala  Val Met Met Tyr Asn  Leu Ser Val His Gln  Gln Leu Gly
    1910                1915                1920

Lys Met  Val Gly Val Ser Asp  Asp Val Asn Glu Tyr  Ala Met Ala
    1925                1930                1935

Leu Arg  Asp Thr Glu Asp Lys  Leu Arg Arg Cys Pro  Lys Arg Arg
    1940                1945                1950

Lys Asp  Ile Leu Ala Glu Leu  Thr Lys Ser Gln Lys  Val Phe Ser
    1955                1960                1965

Glu Lys  Leu Asp His Leu Ser  Arg Arg Leu Ala Trp  Val His Ala
    1970                1975                1980

Thr Val  Tyr Ser Gln Glu Lys  Met Leu Asp Ile Tyr  Trp Leu Leu
    1985                1990                1995

Arg Val Cys Leu Arg Thr Ile Glu His Gly Asp Arg Thr Gly Ser
    2000                2005                2010

Leu Phe Ala Phe Met Pro Glu Phe Tyr Leu Ser Val Ala Ile Asn
    2015                2020                2025

Ser Tyr Ser Ala Leu Lys Asn Tyr Phe Gly Pro Val His Ser Met
    2030                2035                2040

Glu Glu Leu Pro Gly Tyr Glu Glu Thr Leu Thr Arg Leu Ala Ala
    2045                2050                2055

Ile Leu Ala Lys His Phe Ala Asp Ala Arg Ile Val Gly Thr Asp
    2060                2065                2070

Ile Arg Asp Ser Leu Met Gln Ala Leu Ala Ser Tyr Val Cys Tyr
    2075                2080                2085

Pro His Ser Leu Arg Ala Val Glu Arg Ile Pro Glu Glu Gln Arg
    2090                2095                2100

Ile Ala Met Val Arg Asn Leu Leu Ala Pro Tyr Glu Gln Arg Pro
    2105                2110                2115

Trp Ala Gln Thr Asn Trp Ile Leu Val Arg Leu Trp Arg Gly Cys
    2120                2125                2130

Gly Phe Gly Tyr Arg Tyr Thr Arg Leu Pro His Leu Leu Lys Thr
    2135                2140                2145

Lys Leu Glu Asp Ala Asn Leu Pro Ser Leu Gln Lys Pro Cys Pro
    2150                2155                2160

Ser Thr Leu Leu Gln Gln His Met Ala Asp Leu Leu Gln Gln Gly
    2165                2170                2175

Pro Asp Val Ala Pro Ser Phe Leu Asn Ser Val Leu Asn Gln Leu
    2180                2185                2190

Asn Trp Ala Phe Ser Glu Phe Ile Gly Met Ile Gln Glu Ile Gln
    2195                2200                2205

Gln Ala Ala Glu Arg Leu Glu Arg Asn Phe Val Asp Ser Arg Gln
    2210                2215                2220

Leu Lys Val Cys Ala Thr Cys Phe Asp Leu Ser Val Ser Leu Leu
    2225                2230                2235

Arg Val Leu Glu Met Thr Ile Thr Leu Val Pro Glu Ile Phe Leu
    2240                2245                2250

Asp Trp Thr Arg Pro Thr Ser Glu Met Leu Leu Arg Arg Leu Ala
    2255                2260                2265

Gln Leu Leu Asn Gln Val Leu Asn Arg Val Thr Ala Glu Arg Asn
    2270                2275                2280

Leu Phe Asp Arg Val Val Thr Leu Arg Leu Pro Gly Leu Glu Ser
    2285                2290                2295

Val Asp His Tyr Pro Ile Leu Val Ala Val Thr Gly Ile Leu Val
    2300                2305                2310

Gln Leu Leu Val Arg Gly Pro Ala Ser Glu Arg Glu Gln Ala Thr
    2315                2320                2325

Ser Val Leu Leu Ala Asp Pro Cys Phe Gln Leu Arg Ser Ile Cys
    2330                2335                2340

Tyr Leu Leu Gly Gln Pro Glu Pro Pro Ala Pro Gly Thr Ala Leu
    2345                2350                2355

Pro Ala Pro Asp Arg Lys Arg Phe Ser Leu Gln Ser Tyr Ala Asp
    2360                2365                2370

Tyr Ile Ser Ala Asp Glu Leu Ala Gln Val Glu Gln Met Leu Ala
    2375                2380                2385

His Leu Thr Ser Ala Ser Ala Gln Ala Ala Ala Ala Ser Leu Pro
    2390                2395                2400

Thr Ser Glu Glu Asp Leu Cys Pro Ile Cys Tyr Ala His Pro Ile
    2405                2410                2415

Ser Ala Val Phe Gln Pro Cys Gly His Lys Ser Cys Lys Ala Cys
    2420                2425                2430

Ile Asn Gln His Leu Met Asn Asn Lys Asp Cys Phe Phe Cys Lys
    2435                2440                2445

Thr Thr Ile Val Ser Val Glu Asp Trp Glu Lys Gly Ala Asn Thr
    2450                2455                2460

Ser Thr Thr Ser Ser Ala Ala
    2465                2470

<210>  15
<211>  7350
<212>  DNA
<213>  Homo sapiens

<220>
<221>  CDS
<222>  (1)..(7350)

<400>  15
atg gcg agc gac tcc ccg gct cga agc ctg gat gaa ata gat ctc tcg      48
Met Ala Ser Asp Ser Pro Ala Arg Ser Leu Asp Glu Ile Asp Leu Ser
1               5                   10                  15

gct ctg agg gac ccc gca ggg atc ttt gaa ttg gtg gaa ctt gtt gga      96
Ala Leu Arg Asp Pro Ala Gly Ile Phe Glu Leu Val Glu Leu Val Gly
            20                  25                  30

aat gga aca tac ggg caa gtt tat aag ggt cgt cat gtc aaa acg ggc      144
Asn Gly Thr Tyr Gly Gln Val Tyr Lys Gly Arg His Val Lys Thr Gly
        35                  40                  45

cag ctt gca gcc atc aag gtt atg gat gtc aca ggg gat gaa gag gaa      192
Gln Leu Ala Ala Ile Lys Val Met Asp Val Thr Gly Asp Glu Glu Glu
    50                  55                  60

gaa atc aaa caa gaa att aac atg ttg aag aaa tat tct cat cac cgg      240
Glu Ile Lys Gln Glu Ile Asn Met Leu Lys Lys Tyr Ser His His Arg
65                  70                  75                  80

aat att gct aca tac tat ggt gct ttt atc aaa aag aac cca cca ggc      288
Asn Ile Ala Thr Tyr Tyr Gly Ala Phe Ile Lys Lys Asn Pro Pro Gly
                85                  90                  95

atg gat gac caa ctt tgg ttg gtg atg gag ttt tgt ggt gct ggc tct      336
Met Asp Asp Gln Leu Trp Leu Val Met Glu Phe Cys Gly Ala Gly Ser
            100                 105                 110

gtc acc gac ctg atc aag aac aca aaa ggt aac acg ttg aaa gag gag      384
Val Thr Asp Leu Ile Lys Asn Thr Lys Gly Asn Thr Leu Lys Glu Glu
        115                 120                 125

tgg att gca tac atc tgc agg gaa atc tta cgg ggg ctg agt cac ctg      432
Trp Ile Ala Tyr Ile Cys Arg Glu Ile Leu Arg Gly Leu Ser His Leu
    130                 135                 140

cac cag cat aaa gtg att cat cga gat att aaa ggg caa aat gtc ttg      480
His Gln His Lys Val Ile His Arg Asp Ile Lys Gly Gln Asn Val Leu
145                 150                 155                 160

ctg act gaa aat gca gaa gtt aaa cta gtg gac ttt gga gtc agt gct      528
Leu Thr Glu Asn Ala Glu Val Lys Leu Val Asp Phe Gly Val Ser Ala
                165                 170                 175

cag ctt gat cga aca gtg ggc agg agg aat act ttc att gga act ccc      576
Gln Leu Asp Arg Thr Val Gly Arg Arg Asn Thr Phe Ile Gly Thr Pro
            180                 185                 190

tac tgg atg gca cca gaa gtt att gcc tgt gat gaa aac cca gat gcc      624
Tyr Trp Met Ala Pro Glu Val Ile Ala Cys Asp Glu Asn Pro Asp Ala

195                 200                 205

aca tat gat ttc aag agt gac ttg tgg tct ttg ggt atc acc gcc att      672
Thr Tyr Asp Phe Lys Ser Asp Leu Trp Ser Leu Gly Ile Thr Ala Ile
    210                 215                 220

gaa atg gca gaa ggt gct ccc cct ctc tgt gac atg cac ccc atg aga      720
Glu Met Ala Glu Gly Ala Pro Pro Leu Cys Asp Met His Pro Met Arg
225                 230                 235                 240

gct ctc ttc ctc atc ccc cgg aac cca gcg cct cgg ctg aag tct aag      768
Ala Leu Phe Leu Ile Pro Arg Asn Pro Ala Pro Arg Leu Lys Ser Lys
                245                 250                 255

aag tgg tca aaa aaa ttc cag tca ttt att gag agc tgc ttg gta aag      816
Lys Trp Ser Lys Lys Phe Gln Ser Phe Ile Glu Ser Cys Leu Val Lys
            260                 265                 270

aat cac agc cag cga cca gca aca gaa caa ttg atg aag cat cca ttt      864
Asn His Ser Gln Arg Pro Ala Thr Glu Gln Leu Met Lys His Pro Phe
        275                 280                 285

ata cga gac caa cct aat gag cga cag gtc cgc att caa ctc aag gac      912
Ile Arg Asp Gln Pro Asn Glu Arg Gln Val Arg Ile Gln Leu Lys Asp
    290                 295                 300

cat att gat aga aca aag aag aag cga gga gaa aaa gat gag aca gag      960
His Ile Asp Arg Thr Lys Lys Lys Arg Gly Glu Lys Asp Glu Thr Glu
305                 310                 315                 320

tat gag tac agt gga agt gag gaa gaa gag gag gag aat gac tca gga      1008
Tyr Glu Tyr Ser Gly Ser Glu Glu Glu Glu Glu Glu Asn Asp Ser Gly
                325                 330                 335

gag ccc agc tcc atc ctg aat ctg cca ggg gag tcg acg ctg cgg agg      1056
Glu Pro Ser Ser Ile Leu Asn Leu Pro Gly Glu Ser Thr Leu Arg Arg
            340                 345                 350

gac ttt ctg agg ctg cag ctg gcc aac aag gag cgt tct gag gcc cta      1104
Asp Phe Leu Arg Leu Gln Leu Ala Asn Lys Glu Arg Ser Glu Ala Leu
        355                 360                 365

cgg agg cag cag ctg gag cag cag cag cgg gag aat gag gag cac aag      1152
Arg Arg Gln Gln Leu Glu Gln Gln Gln Arg Glu Asn Glu Glu His Lys
    370                 375                 380

cgg cag ctg ctg gcc gag cgt cag aag cgc atc gag gag cag aaa gag      1200
Arg Gln Leu Leu Ala Glu Arg Gln Lys Arg Ile Glu Glu Gln Lys Glu
385                 390                 395                 400

cag agg cgg cgg ctg gag gag caa caa agg cga gag aag gag ctg cgg      1248
Gln Arg Arg Arg Leu Glu Glu Gln Gln Arg Arg Glu Lys Glu Leu Arg
                405                 410                 415

aag cag cag gag agg gag cag cgc cgg cac tat gag gag cag atg cgc      1296
Lys Gln Gln Glu Arg Glu Gln Arg Arg His Tyr Glu Glu Gln Met Arg
            420                 425                 430

cgg gag gag gag agg agg cgt gcg gag cat gaa cag gaa tat aag cgc      1344
Arg Glu Glu Glu Arg Arg Arg Ala Glu His Glu Gln Glu Tyr Lys Arg
        435                 440                 445

aaa caa ttg gaa gaa cag aga caa gca gaa aga ctg cag agg cag cta      1392

Lys Gln Leu Glu Glu Gln Arg Gln Ala Glu Arg Leu Gln Arg Gln Leu
    450                 455                 460

aag caa gaa aga gac tac tta gtt tcc ctt cag cat cag cgg cag gag 1440
Lys Gln Glu Arg Asp Tyr Leu Val Ser Leu Gln His Gln Arg Gln Glu
465                 470                 475                 480

cag agg cct gtg gag aag aag cca ctg tac cat tac aaa gaa gga atg 1488
Gln Arg Pro Val Glu Lys Lys Pro Leu Tyr His Tyr Lys Glu Gly Met
                485                 490                 495

agt cct agt gag aag cca gca tgg gcc aag gag atc cca cat ctg gta 1536
Ser Pro Ser Glu Lys Pro Ala Trp Ala Lys Glu Ile Pro His Leu Val
            500                 505                 510

gct gta aaa tcc cag gga cct gcc ttg acc gcc tcc cag tca gtg cac 1584
Ala Val Lys Ser Gln Gly Pro Ala Leu Thr Ala Ser Gln Ser Val His
        515                 520                 525

gag cag ccc aca aag ggc ctc tct ggg ttt cag gag gct ctg aac gtg 1632
Glu Gln Pro Thr Lys Gly Leu Ser Gly Phe Gln Glu Ala Leu Asn Val
    530                 535                 540

acc tcc cac cgc gtg gag atg cca cgc cag aac tca gat ccc acc tcg 1680
Thr Ser His Arg Val Glu Met Pro Arg Gln Asn Ser Asp Pro Thr Ser
545                 550                 555                 560

gaa aat cct cct ctc ccc act cgc att gaa aag ttt gac cga agc tct 1728
Glu Asn Pro Pro Leu Pro Thr Arg Ile Glu Lys Phe Asp Arg Ser Ser
                565                 570                 575

tgg tta cga cag gaa gaa gac att cca cca aag gtg cct caa aga aca 1776
Trp Leu Arg Gln Glu Glu Asp Ile Pro Pro Lys Val Pro Gln Arg Thr
            580                 585                 590

act tct ata tcc cca gca tta gcc aga aag aat tct cct ggg aat ggt 1824
Thr Ser Ile Ser Pro Ala Leu Ala Arg Lys Asn Ser Pro Gly Asn Gly
        595                 600                 605

agt gct ctg gga ccc aga cta gga tct caa ccc atc aga gca agc aac 1872
Ser Ala Leu Gly Pro Arg Leu Gly Ser Gln Pro Ile Arg Ala Ser Asn
    610                 615                 620

cct gat ctc cgg aga act gag ccc atc ttg gag agc ccc ttg cag agg 1920
Pro Asp Leu Arg Arg Thr Glu Pro Ile Leu Glu Ser Pro Leu Gln Arg
625                 630                 635                 640

acc agc agt ggc agt tcc tcc agc tcc agc acc cct agc tcc cag ccc 1968
Thr Ser Ser Gly Ser Ser Ser Ser Ser Ser Thr Pro Ser Ser Gln Pro
                645                 650                 655

agc tcc caa gga ggc tcc cag cct gga tca caa gca gga tcc agt gaa 2016
Ser Ser Gln Gly Gly Ser Gln Pro Gly Ser Gln Ala Gly Ser Ser Glu
            660                 665                 670

cgc acc aga gtt cga gcc aac agt aag tca gaa gga tca cct gtg ctc 2064
Arg Thr Arg Val Arg Ala Asn Ser Lys Ser Glu Gly Ser Pro Val Leu
        675                 680                 685

ccc cat gag cct gcc aag gtg aaa cca gaa gaa tcc agg gac att acc 2112
Pro His Glu Pro Ala Lys Val Lys Pro Glu Glu Ser Arg Asp Ile Thr
    690                 695                 700

cgg ccc agt cga cca gct agc tac aaa aaa gct ata gat gag gat ctg 2160
Arg Pro Ser Arg Pro Ala Ser Tyr Lys Lys Ala Ile Asp Glu Asp Leu
705                 710                 715                 720

acg gca tta gcc aaa gaa cta aga gaa ctc cgg att gaa gaa aca aac 2208
Thr Ala Leu Ala Lys Glu Leu Arg Glu Leu Arg Ile Glu Glu Thr Asn
                725                 730                 735

cgc cca atg aag aag gtg act gat tac tcc tcc tcc agt gag gag tca 2256
Arg Pro Met Lys Lys Val Thr Asp Tyr Ser Ser Ser Ser Glu Glu Ser
            740                 745                 750

gaa agt agc gag gaa gag gag gaa gat gga gag agc gag acc cat gat 2304
Glu Ser Ser Glu Glu Glu Glu Glu Asp Gly Glu Ser Glu Thr His Asp
        755                 760                 765

ggg aca gtg gct gtc agc gac ata ccc aga ctg ata cca aca gga gct 2352
Gly Thr Val Ala Val Ser Asp Ile Pro Arg Leu Ile Pro Thr Gly Ala
    770                 775                 780

cca ggc agc aac gag cag tac aat gtg gga atg gtg ggg acg cat ggg 2400
Pro Gly Ser Asn Glu Gln Tyr Asn Val Gly Met Val Gly Thr His Gly
785                 790                 795                 800

ctg gag acc tct cat gcg gac agt ttc agc ggc agt att tca aga gaa 2448
Leu Glu Thr Ser His Ala Asp Ser Phe Ser Gly Ser Ile Ser Arg Glu
                805                 810                 815

gga acc ttg atg att aga gag acg tct gga gag aag aag cga tct ggc 2496
Gly Thr Leu Met Ile Arg Glu Thr Ser Gly Glu Lys Lys Arg Ser Gly
            820                 825                 830

cac agt gac agc aat ggc ttt gct ggc cac atc aac ctc cct gac ctg 2544
His Ser Asp Ser Asn Gly Phe Ala Gly His Ile Asn Leu Pro Asp Leu
        835                 840                 845

gtg cag cag agc cat tct cca gct gga acc ccg act gag gga ctg ggg 2592
Val Gln Gln Ser His Ser Pro Ala Gly Thr Pro Thr Glu Gly Leu Gly
    850                 855                 860

cgc gtc tca acc cat tcc cag gag atg gac tct ggg act gaa tat ggc 2640
Arg Val Ser Thr His Ser Gln Glu Met Asp Ser Gly Thr Glu Tyr Gly
865                 870                 875                 880

atg ggg agc agc acc aaa gcc tcc ttc acc ccc ttt gtg gac ccc aga 2688
Met Gly Ser Ser Thr Lys Ala Ser Phe Thr Pro Phe Val Asp Pro Arg
                885                 890                 895

gta tac cag acg tct ccc act gat gaa gat gaa gag gat gag gaa tca 2736
Val Tyr Gln Thr Ser Pro Thr Asp Glu Asp Glu Glu Asp Glu Glu Ser
            900                 905                 910

tca gcc gca gct ctg ttt act agc gaa ctt ctt agg caa gaa cag gcc 2784
Ser Ala Ala Ala Leu Phe Thr Ser Glu Leu Leu Arg Gln Glu Gln Ala
        915                 920                 925

aaa ctc aat gaa gca aga aag att tcg gtg gta aat gta aac cca acc 2832
Lys Leu Asn Glu Ala Arg Lys Ile Ser Val Val Asn Val Asn Pro Thr
    930                 935                 940

aac att cgg cct cat agc gac aca cca gaa atc aga aaa tac aag aaa 2880
Asn Ile Arg Pro His Ser Asp Thr Pro Glu Ile Arg Lys Tyr Lys Lys
945                 950                 955                 960

cga ttc aac tca gaa ata ctt tgt gca gct ctg tgg ggt gta aac ctt 2928
Arg Phe Asn Ser Glu Ile Leu Cys Ala Ala Leu Trp Gly Val Asn Leu
                965                 970                 975

ctg gtg ggg act gaa aat ggc ctg atg ctt ttg gac cga agt ggg caa 2976
Leu Val Gly Thr Glu Asn Gly Leu Met Leu Leu Asp Arg Ser Gly Gln
            980                 985                 990

ggc aaa gtc tat aat ctg atc aac cgg agg cga ttt cag cag atg gat 3024
Gly Lys Val Tyr Asn Leu Ile Asn Arg Arg Arg Phe Gln Gln Met Asp
        995                 1000                1005

gtg cta gag gga ctg aat gtc ctt gtg aca att tca gga aag aag 3069
Val Leu Glu Gly Leu Asn Val Leu Val Thr Ile Ser Gly Lys Lys
    1010                1015                1020

aat aag cta cga gtt tac tat ctt tca tgg tta aga aac aga ata 3114
Asn Lys Leu Arg Val Tyr Tyr Leu Ser Trp Leu Arg Asn Arg Ile
    1025                1030                1035

cta cat aat gac cca gaa gta gaa aag aaa caa ggc tgg atc act 3159
Leu His Asn Asp Pro Glu Val Glu Lys Lys Gln Gly Trp Ile Thr
    1040                1045                1050

gtt ggg gac ttg gaa ggc tgt ata cat tat aaa gtt gtt aaa tat 3204
Val Gly Asp Leu Glu Gly Cys Ile His Tyr Lys Val Val Lys Tyr
    1055                1060                1065

gaa agg atc aaa ttt ttg gtg att gcc tta aag aat gct gtg gaa 3249
Glu Arg Ile Lys Phe Leu Val Ile Ala Leu Lys Asn Ala Val Glu
    1070                1075                1080

ata tat gct tgg gct cct aaa ccg tat cat aaa ttc atg gca ttt 3294
Ile Tyr Ala Trp Ala Pro Lys Pro Tyr His Lys Phe Met Ala Phe
    1085                1090                1095

aag tct ttt gca gat ctc cag cac aag cct ctg cta gtt gat ctc 3339
Lys Ser Phe Ala Asp Leu Gln His Lys Pro Leu Leu Val Asp Leu
    1100                1105                1110

acg gta gaa gaa ggt caa aga tta aag gtt att ttt ggt tca cac 3384
Thr Val Glu Glu Gly Gln Arg Leu Lys Val Ile Phe Gly Ser His
    1115                1120                1125

act ggt ttc cat gta att gat gtt gat tca gga aac tct tat gat 3429
Thr Gly Phe His Val Ile Asp Val Asp Ser Gly Asn Ser Tyr Asp
    1130                1135                1140

atc tac ata cca tct cat att cag ggc aat atc act cct cat gct 3474
Ile Tyr Ile Pro Ser His Ile Gln Gly Asn Ile Thr Pro His Ala
    1145                1150                1155

att gtc atc ttg cct aaa aca gat gga atg gaa atg ctt gtt tgc 3519
Ile Val Ile Leu Pro Lys Thr Asp Gly Met Glu Met Leu Val Cys
    1160                1165                1170

tat gag gat gag ggg gtg tat gta aac acc tat ggc cgg ata act 3564
Tyr Glu Asp Glu Gly Val Tyr Val Asn Thr Tyr Gly Arg Ile Thr
    1175                1180                1185

aag gat gtg gtg ctc caa tgg gga gaa atg ccc acg tct gtg gcc 3609
Lys Asp Val Val Leu Gln Trp Gly Glu Met Pro Thr Ser Val Ala

1190                1195                1200

tac att cat tcc aat cag ata atg ggc tgg ggc gag aaa gct att        3654
Tyr Ile His Ser Asn Gln Ile Met Gly Trp Gly Glu Lys Ala Ile
    1205                1210                1215

gag atc cgg tca gtg gaa aca gga cat ttg gat gga gta ttt atg        3699
Glu Ile Arg Ser Val Glu Thr Gly His Leu Asp Gly Val Phe Met
    1220                1225                1230

cat aag cga gct caa agg tta aag ttt cta tgt gaa aga aat gat        3744
His Lys Arg Ala Gln Arg Leu Lys Phe Leu Cys Glu Arg Asn Asp
    1235                1240                1245

aag gtg att gga cac agc aac ttt ggc acc atc cgc tct acc aca        3789
Lys Val Ile Gly His Ser Asn Phe Gly Thr Ile Arg Ser Thr Thr
    1250                1255                1260

tgc gtg tac aaa ggg aaa tgg ctc tac gag gtc ctc atc tcc tcc        3834
Cys Val Tyr Lys Gly Lys Trp Leu Tyr Glu Val Leu Ile Ser Ser
    1265                1270                1275

cag ggg ctc atg cag atc ggc tgg tgc acc atc agc tgc cgc ttc        3879
Gln Gly Leu Met Gln Ile Gly Trp Cys Thr Ile Ser Cys Arg Phe
    1280                1285                1290

aac cag gag gag ggg gtt gga gat aca cac aac tcc tat gcc tat        3924
Asn Gln Glu Glu Gly Val Gly Asp Thr His Asn Ser Tyr Ala Tyr
    1295                1300                1305

gat ggc aac cgc gtg cgc aag tgg aat gtg acc aca acg aat tat        3969
Asp Gly Asn Arg Val Arg Lys Trp Asn Val Thr Thr Thr Asn Tyr
    1310                1315                1320

ggc aag gcg tgg gca gcg ggg gac atc gtg agc tgc ctg att gac        4014
Gly Lys Ala Trp Ala Ala Gly Asp Ile Val Ser Cys Leu Ile Asp
    1325                1330                1335

ctg gat gat ggc act ctg tcc ttc tgc ctg aac ggt gta tca ctg        4059
Leu Asp Asp Gly Thr Leu Ser Phe Cys Leu Asn Gly Val Ser Leu
    1340                1345                1350

ggc act gcc ttt gag aac ctg tcc agg ggc ctg ggt atg gcc tac        4104
Gly Thr Ala Phe Glu Asn Leu Ser Arg Gly Leu Gly Met Ala Tyr
    1355                1360                1365

ttc cca gcc atc agc ctc tct ttc aag gag tcc gtg gcc ttc aac        4149
Phe Pro Ala Ile Ser Leu Ser Phe Lys Glu Ser Val Ala Phe Asn
    1370                1375                1380

ttt ggc agc cgt cct ctg cgc tac cca gtg gca ggc tac cgg ccc        4194
Phe Gly Ser Arg Pro Leu Arg Tyr Pro Val Ala Gly Tyr Arg Pro
    1385                1390                1395

ctg cag gac cca ccg agt gct gac ctg gtg cgg gca cag agg ttg        4239
Leu Gln Asp Pro Pro Ser Ala Asp Leu Val Arg Ala Gln Arg Leu
    1400                1405                1410

ctg ggc tgc ttc cgg gca gtg ctg agt gtg gag ctg gac cct gtg        4284
Leu Gly Cys Phe Arg Ala Val Leu Ser Val Glu Leu Asp Pro Val
    1415                1420                1425

gag ggg cgg ctg ttg gac aag gag agc tcc aag tgg cgg ttg cgg        4329

Glu Gly Arg Leu Leu Asp Lys Glu Ser Ser Lys Trp Arg Leu Arg
    1430                1435                1440

ggc cag ccc acc gtc ctc ctc aca ctg gcc cac atc ttc cat cac 4374
Gly Gln Pro Thr Val Leu Leu Thr Leu Ala His Ile Phe His His
    1445                1450                1455

ttt gca ccg ctt ctg cgc aag gtg tat ctg gtg gag gct gtg ctc 4419
Phe Ala Pro Leu Leu Arg Lys Val Tyr Leu Val Glu Ala Val Leu
    1460                1465                1470

atg agc ttc ttg ctg ggc atc gtg gag aag ggc aca ccc aca cag 4464
Met Ser Phe Leu Leu Gly Ile Val Glu Lys Gly Thr Pro Thr Gln
    1475                1480                1485

gca cag tcc gtg gtg cac cag gtc ctg gac ctc ttg tgg ctc ttc 4509
Ala Gln Ser Val Val His Gln Val Leu Asp Leu Leu Trp Leu Phe
    1490                1495                1500

atg gag gac tac gag gta caa gat tgc ctc aag cag ttg atg atg 4554
Met Glu Asp Tyr Glu Val Gln Asp Cys Leu Lys Gln Leu Met Met
    1505                1510                1515

tct ctg ctt cgg ctg tac cga ttc tca ccc att gtc cca gac ctg 4599
Ser Leu Leu Arg Leu Tyr Arg Phe Ser Pro Ile Val Pro Asp Leu
    1520                1525                1530

ggc cta cag atc cat tac ctg cgg ctc act atc gcc atc ctg agg 4644
Gly Leu Gln Ile His Tyr Leu Arg Leu Thr Ile Ala Ile Leu Arg
    1535                1540                1545

cat gag aag tcc cgc aag ttt ctg ctt agc aat gtc ctc ttc gac 4689
His Glu Lys Ser Arg Lys Phe Leu Leu Ser Asn Val Leu Phe Asp
    1550                1555                1560

gtg ctc cgc tcc gtc gtc ttc ttt tac atc aag agc ccc ctg cgt 4734
Val Leu Arg Ser Val Val Phe Phe Tyr Ile Lys Ser Pro Leu Arg
    1565                1570                1575

gtg gag gag gcc ggc ctg cag gag ctc att ccc acc acc tgg tgg 4779
Val Glu Glu Ala Gly Leu Gln Glu Leu Ile Pro Thr Thr Trp Trp
    1580                1585                1590

ccc cac tgc tcc agt agg gag ggc aaa gag agc acg gag atg aag 4824
Pro His Cys Ser Ser Arg Glu Gly Lys Glu Ser Thr Glu Met Lys
    1595                1600                1605

gag gag acc gca gag gag cgg ctg cgg cgg cga gcc tac gaa cgg 4869
Glu Glu Thr Ala Glu Glu Arg Leu Arg Arg Arg Ala Tyr Glu Arg
    1610                1615                1620

ggc tgt cag cgg ctc agg aag cgc atc gaa gtg gtg gaa gaa cta 4914
Gly Cys Gln Arg Leu Arg Lys Arg Ile Glu Val Val Glu Glu Leu
    1625                1630                1635

cag gtc cag atc ctg aag ctg ctg ctg gac aat aaa gat gac aat 4959
Gln Val Gln Ile Leu Lys Leu Leu Leu Asp Asn Lys Asp Asp Asn
    1640                1645                1650

ggg ggt gaa gct tct agg tat atc ttc ctg acc aag ttt cgc aag 5004
Gly Gly Glu Ala Ser Arg Tyr Ile Phe Leu Thr Lys Phe Arg Lys
    1655                1660                1665

ttt ctg cag gag aac gcc agt ggc cgg ggg aac atg ccc atg ctc 5049
Phe Leu Gln Glu Asn Ala Ser Gly Arg Gly Asn Met Pro Met Leu
    1670                1675                1680

tgc ccc cct gag tac atg gtc tgc ttc tta cac cgg ctg atc tct 5094
Cys Pro Pro Glu Tyr Met Val Cys Phe Leu His Arg Leu Ile Ser
    1685                1690                1695

gcc ctg cgc tac tat tgg gat gaa tac aag gct tcc aat cct cat 5139
Ala Leu Arg Tyr Tyr Trp Asp Glu Tyr Lys Ala Ser Asn Pro His
    1700                1705                1710

gct tcc ttc agt gag gag gcc tac atc ccg ccc cag gtc ttc tat 5184
Ala Ser Phe Ser Glu Glu Ala Tyr Ile Pro Pro Gln Val Phe Tyr
    1715                1720                1725

aat ggc aag gtg gac tac ttt gac ctg cag cgc ctg ggg ggc ctc 5229
Asn Gly Lys Val Asp Tyr Phe Asp Leu Gln Arg Leu Gly Gly Leu
    1730                1735                1740

ctc tcg cac ctg cgg aag acc ctc aaa gat gac ctt gct tcc aaa 5274
Leu Ser His Leu Arg Lys Thr Leu Lys Asp Asp Leu Ala Ser Lys
    1745                1750                1755

gcc aac att gtg atc gac cca ctg gag ctc cag tca acc gcc atg 5319
Ala Asn Ile Val Ile Asp Pro Leu Glu Leu Gln Ser Thr Ala Met
    1760                1765                1770

gat gac cta gat gag gat gag gag cca gcc cca gct atg gcc cag 5364
Asp Asp Leu Asp Glu Asp Glu Glu Pro Ala Pro Ala Met Ala Gln
    1775                1780                1785

cgc ccc atg cag gcc ctg gct gtt ggg ggg cca ctg ccc ctg ccc 5409
Arg Pro Met Gln Ala Leu Ala Val Gly Gly Pro Leu Pro Leu Pro
    1790                1795                1800

cgg ccc ggc tgg ctc agt tct cca act ttg ggc cga gcc aac cgc 5454
Arg Pro Gly Trp Leu Ser Ser Pro Thr Leu Gly Arg Ala Asn Arg
    1805                1810                1815

ttc ctc agc aca gcg gct gtg agc ctc atg acc cca cgg cgg cct 5499
Phe Leu Ser Thr Ala Ala Val Ser Leu Met Thr Pro Arg Arg Pro
    1820                1825                1830

ctg agc acc tcg gag aaa gtg aag gtc cgc acg ctg agc gtg gag 5544
Leu Ser Thr Ser Glu Lys Val Lys Val Arg Thr Leu Ser Val Glu
    1835                1840                1845

cag agg acc cgt gag gac att gaa ggc agc cac tgg aat gag ggc 5589
Gln Arg Thr Arg Glu Asp Ile Glu Gly Ser His Trp Asn Glu Gly
    1850                1855                1860

ttg ctg ctg ggg cgg ccc ccc gag gag cct gag cag ccc ctc acc 5634
Leu Leu Leu Gly Arg Pro Pro Glu Glu Pro Glu Gln Pro Leu Thr
    1865                1870                1875

gag aac tcg ctg ctg gaa gtc ctg gat ggg gcg gtc atg atg tac 5679
Glu Asn Ser Leu Leu Glu Val Leu Asp Gly Ala Val Met Met Tyr
    1880                1885                1890

aac ctc agc gta cac cag cag ctg ggc aag atg gtg ggt gtc tcc 5724
Asn Leu Ser Val His Gln Gln Leu Gly Lys Met Val Gly Val Ser
    1895                1900                1905

gat gat gtc aat gaa tac gct atg gct ctg agg gac aca gag gac 5769
Asp Asp Val Asn Glu Tyr Ala Met Ala Leu Arg Asp Thr Glu Asp
    1910                1915                1920

aag ctc cgc cgg tgc ccc aag agg agg aag gac atc ctt gca gag 5814
Lys Leu Arg Arg Cys Pro Lys Arg Arg Lys Asp Ile Leu Ala Glu
    1925                1930                1935

ttg acc aag agc cag aag gtt ttc tca gaa aag ctg gac cac ctg 5859
Leu Thr Lys Ser Gln Lys Val Phe Ser Glu Lys Leu Asp His Leu
    1940                1945                1950

agc cgc cgt ctt gcc tgg gtc cat gcc act gtc tac tcc cag gag 5904
Ser Arg Arg Leu Ala Trp Val His Ala Thr Val Tyr Ser Gln Glu
    1955                1960                1965

aag atg ctg gac atc tac tgg ctg ctg cgc gtc tgc ctg cgg acc 5949
Lys Met Leu Asp Ile Tyr Trp Leu Leu Arg Val Cys Leu Arg Thr
    1970                1975                1980

att gag cac ggt gat cgc aca ggg tct ctc ttt gcc ttc atg ccc 5994
Ile Glu His Gly Asp Arg Thr Gly Ser Leu Phe Ala Phe Met Pro
    1985                1990                1995

gag ttc tac ctg agc gtg gcc atc aac agc tac agt gct ctc aag 6039
Glu Phe Tyr Leu Ser Val Ala Ile Asn Ser Tyr Ser Ala Leu Lys
    2000                2005                2010

aat tac ttt ggt ccc gtg cac agc atg gag gag ctc cca ggc tat 6084
Asn Tyr Phe Gly Pro Val His Ser Met Glu Glu Leu Pro Gly Tyr
    2015                2020                2025

gaa gag acc ctg acc cgc ctg gct gcc att ctc gcc aaa cac ttt 6129
Glu Glu Thr Leu Thr Arg Leu Ala Ala Ile Leu Ala Lys His Phe
    2030                2035                2040

gcc gac gca cgc att gtg ggc act gac atc cga gac tca ctg atg 6174
Ala Asp Ala Arg Ile Val Gly Thr Asp Ile Arg Asp Ser Leu Met
    2045                2050                2055

cag gcc ctg gcc agc tac gtg tgc tac cca cac tcc ctg cgg gct 6219
Gln Ala Leu Ala Ser Tyr Val Cys Tyr Pro His Ser Leu Arg Ala
    2060                2065                2070

gtg gag cga atc ccc gag gag cag cgt atc gcc atg gtg agg aac 6264
Val Glu Arg Ile Pro Glu Glu Gln Arg Ile Ala Met Val Arg Asn
    2075                2080                2085

ctc ctg gcg ccc tat gag cag cgg ccc tgg gcc cag acc aac tgg 6309
Leu Leu Ala Pro Tyr Glu Gln Arg Pro Trp Ala Gln Thr Asn Trp
    2090                2095                2100

atc ctg gtg cgg ctc tgg agg ggc tgt ggc ttc ggg tac cgc tat 6354
Ile Leu Val Arg Leu Trp Arg Gly Cys Gly Phe Gly Tyr Arg Tyr
    2105                2110                2115

aca cgg ctg cca cat ctg ctg aaa acc aaa ctt gag gac gcc aat 6399
Thr Arg Leu Pro His Leu Leu Lys Thr Lys Leu Glu Asp Ala Asn
    2120                2125                2130

ttg ccc agc ctc cag aag ccc tgc cct tcc acc ctg ctg cag cag 6444
Leu Pro Ser Leu Gln Lys Pro Cys Pro Ser Thr Leu Leu Gln Gln

2135                    2140                    2145

cac atg gcg gac ctc cta cag cag ggt cct gat gtg gca ccc agc          6489
His Met Ala Asp Leu Leu Gln Gln Gly Pro Asp Val Ala Pro Ser
    2150                    2155                    2160

ttc ctc aac agc gtc ctc aat cag ctc aac tgg gcc ttc tct gaa          6534
Phe Leu Asn Ser Val Leu Asn Gln Leu Asn Trp Ala Phe Ser Glu
    2165                    2170                    2175

ttc att ggc atg atc caa gag atc cag cag gct gct gag cgc ctg          6579
Phe Ile Gly Met Ile Gln Glu Ile Gln Gln Ala Ala Glu Arg Leu
    2180                    2185                    2190

gag cgg aac ttt gtg gac agc cgg cag ctc aag gta tgt gcc acc          6624
Glu Arg Asn Phe Val Asp Ser Arg Gln Leu Lys Val Cys Ala Thr
    2195                    2200                    2205

tgc ttt gac ctc tcg gtc agc ctg ctg cgt gtc ttg gag atg act          6669
Cys Phe Asp Leu Ser Val Ser Leu Leu Arg Val Leu Glu Met Thr
    2210                    2215                    2220

atc aca ctg gtg cct gag ata ttc ctt gac tgg acc cgg cct acc          6714
Ile Thr Leu Val Pro Glu Ile Phe Leu Asp Trp Thr Arg Pro Thr
    2225                    2230                    2235

tct gag atg ctg ctg cgg cgt ctt gca cag ctg cta aac cag gtg          6759
Ser Glu Met Leu Leu Arg Arg Leu Ala Gln Leu Leu Asn Gln Val
    2240                    2245                    2250

ctg aac cgg gtg aca gct gag agg aac ctg ttt gat cgt gtg gtc          6804
Leu Asn Arg Val Thr Ala Glu Arg Asn Leu Phe Asp Arg Val Val
    2255                    2260                    2265

acc cta cgg ctg cct ggc cta gag agc gtg gac cac tat ccc att          6849
Thr Leu Arg Leu Pro Gly Leu Glu Ser Val Asp His Tyr Pro Ile
    2270                    2275                    2280

ctg gtg gca gtg acg ggc atc ctg gtg cag ctc ctg gtg cgt ggc          6894
Leu Val Ala Val Thr Gly Ile Leu Val Gln Leu Leu Val Arg Gly
    2285                    2290                    2295

cca gcc tca gag aga gag caa gcc aca tca gtg ctc ctg gca gat          6939
Pro Ala Ser Glu Arg Glu Gln Ala Thr Ser Val Leu Leu Ala Asp
    2300                    2305                    2310

ccc tgc ttc cag cta cgc tca ata tgc tat ctc ctg gga cag cca          6984
Pro Cys Phe Gln Leu Arg Ser Ile Cys Tyr Leu Leu Gly Gln Pro
    2315                    2320                    2325

gag ccc cca gca cct ggc act gct ctg cca gcc cct gac cgg aag          7029
Glu Pro Pro Ala Pro Gly Thr Ala Leu Pro Ala Pro Asp Arg Lys
    2330                    2335                    2340

cgc ttc tcc ctg cag agc tat gcg gat tat atc agt gcc gat gag          7074
Arg Phe Ser Leu Gln Ser Tyr Ala Asp Tyr Ile Ser Ala Asp Glu
    2345                    2350                    2355

ctg gcc caa gtg gaa cag atg ctg gcg cac ctg acc tct gca tct          7119
Leu Ala Gln Val Glu Gln Met Leu Ala His Leu Thr Ser Ala Ser
    2360                    2365                    2370

gcc cag gca gca gct gcc tcc ctg ccc acc agt gag gag gac ctc          7164

Ala Gln Ala Ala Ala Ala Ser Leu Pro Thr Ser Glu Glu Asp Leu
    2375                2380                2385

tgc ccc atc tgc tat gcc cac ccc atc tct gct gtg ttc cag ccc      7209
Cys Pro Ile Cys Tyr Ala His Pro Ile Ser Ala Val Phe Gln Pro
    2390                2395                2400

tgt ggc cac aag tcc tgc aaa gcc tgt atc aac cag cac ctg atg      7254
Cys Gly His Lys Ser Cys Lys Ala Cys Ile Asn Gln His Leu Met
    2405                2410                2415

aac aac aag gac tgc ttc ttc tgc aaa acc acc atc gtg tct gta      7299
Asn Asn Lys Asp Cys Phe Phe Cys Lys Thr Thr Ile Val Ser Val
    2420                2425                2430

gag gac tgg gag aag gga gcc aat acg agt act acc tcc tca gct      7344
Glu Asp Trp Glu Lys Gly Ala Asn Thr Ser Thr Thr Ser Ser Ala
    2435                2440                2445

gcc tag                                                          7350
Ala

<210>  16
<211>  2449
<212>  PRT
<213>  Homo sapiens

<400>  16
   Met Ala Ser Asp Ser Pro Ala Arg Ser Leu Asp Glu Ile Asp Leu Ser
   1               5                   10                  15
   Ala Leu Arg Asp Pro Ala Gly Ile Phe Glu Leu Val Glu Leu Val Gly
               20                  25                  30
   Asn Gly Thr Tyr Gly Gln Val Tyr Lys Gly Arg His Val Lys Thr Gly
           35                  40                  45
   Gln Leu Ala Ala Ile Lys Val Met Asp Val Thr Gly Asp Glu Glu Glu
       50                  55                  60
   Glu Ile Lys Gln Glu Ile Asn Met Leu Lys Lys Tyr Ser His His Arg
   65                  70                  75                  80
   Asn Ile Ala Thr Tyr Tyr Gly Ala Phe Ile Lys Lys Asn Pro Pro Gly
                   85                  90                  95
   Met Asp Asp Gln Leu Trp Leu Val Met Glu Phe Cys Gly Ala Gly Ser
               100                 105                 110
   Val Thr Asp Leu Ile Lys Asn Thr Lys Gly Asn Thr Leu Lys Glu Glu
           115                 120                 125
   Trp Ile Ala Tyr Ile Cys Arg Glu Ile Leu Arg Gly Leu Ser His Leu
       130                 135                 140
   His Gln His Lys Val Ile His Arg Asp Ile Lys Gly Gln Asn Val Leu
   145                 150                 155                 160
   Leu Thr Glu Asn Ala Glu Val Lys Leu Val Asp Phe Gly Val Ser Ala
                   165                 170                 175
   Gln Leu Asp Arg Thr Val Gly Arg Arg Asn Thr Phe Ile Gly Thr Pro
               180                 185                 190
   Tyr Trp Met Ala Pro Glu Val Ile Ala Cys Asp Glu Asn Pro Asp Ala
           195                 200                 205
   Thr Tyr Asp Phe Lys Ser Asp Leu Trp Ser Leu Gly Ile Thr Ala Ile
       210                 215                 220
   Glu Met Ala Glu Gly Ala Pro Pro Leu Cys Asp Met His Pro Met Arg
   225                 230                 235                 240
   Ala Leu Phe Leu Ile Pro Arg Asn Pro Ala Pro Arg Leu Lys Ser Lys
                   245                 250                 255
   Lys Trp Ser Lys Lys Phe Gln Ser Phe Ile Glu Ser Cys Leu Val Lys
               260                 265                 270
   Asn His Ser Gln Arg Pro Ala Thr Glu Gln Leu Met Lys His Pro Phe
           275                 280                 285
   Ile Arg Asp Gln Pro Asn Glu Arg Gln Val Arg Ile Gln Leu Lys Asp
       290                 295                 300
   His Ile Asp Arg Thr Lys Lys Lys Arg Gly Glu Lys Asp Glu Thr Glu
   305                 310                 315                 320
   Tyr Glu Tyr Ser Gly Ser Glu Glu Glu Glu Glu Glu Asn Asp Ser Gly
                   325                 330                 335
   Glu Pro Ser Ser Ile Leu Asn Leu Pro Gly Glu Ser Thr Leu Arg Arg
               340                 345                 350
   Asp Phe Leu Arg Leu Gln Leu Ala Asn Lys Glu Arg Ser Glu Ala Leu
           355                 360                 365
   Arg Arg Gln Gln Leu Glu Gln Gln Gln Arg Glu Asn Glu Glu His Lys
       370                 375                 380
   Arg Gln Leu Leu Ala Glu Arg Gln Lys Arg Ile Glu Glu Gln Lys Glu
   385                 390                 395                 400
   Gln Arg Arg Arg Leu Glu Glu Gln Gln Arg Arg Glu Lys Glu Leu Arg
                   405                 410                 415
   Lys Gln Gln Glu Arg Glu Gln Arg Arg His Tyr Glu Glu Gln Met Arg
               420                 425                 430
   Arg Glu Glu Glu Arg Arg Arg Ala Glu His Glu Gln Glu Tyr Lys Arg
           435                 440                 445
   Lys Gln Leu Glu Glu Gln Arg Gln Ala Glu Arg Leu Gln Arg Gln Leu
       450                 455                 460
   Lys Gln Glu Arg Asp Tyr Leu Val Ser Leu Gln His Gln Arg Gln Glu
   465                 470                 475                 480
   Gln Arg Pro Val Glu Lys Lys Pro Leu Tyr His Tyr Lys Glu Gly Met
                   485                 490                 495
   Ser Pro Ser Glu Lys Pro Ala Trp Ala Lys Glu Ile Pro His Leu Val
               500                 505                 510
   Ala Val Lys Ser Gln Gly Pro Ala Leu Thr Ala Ser Gln Ser Val His
           515                 520                 525
   Glu Gln Pro Thr Lys Gly Leu Ser Gly Phe Gln Glu Ala Leu Asn Val
       530                 535                 540
   Thr Ser His Arg Val Glu Met Pro Arg Gln Asn Ser Asp Pro Thr Ser
   545                 550                 555                 560
   Glu Asn Pro Pro Leu Pro Thr Arg Ile Glu Lys Phe Asp Arg Ser Ser
                   565                 570                 575
   Trp Leu Arg Gln Glu Glu Asp Ile Pro Pro Lys Val Pro Gln Arg Thr
               580                 585                 590
   Thr Ser Ile Ser Pro Ala Leu Ala Arg Lys Asn Ser Pro Gly Asn Gly
           595                 600                 605
   Ser Ala Leu Gly Pro Arg Leu Gly Ser Gln Pro Ile Arg Ala Ser Asn
       610                 615                 620
   Pro Asp Leu Arg Arg Thr Glu Pro Ile Leu Glu Ser Pro Leu Gln Arg
   625                 630                 635                 640
   Thr Ser Ser Gly Ser Ser Ser Ser Ser Ser Thr Pro Ser Ser Gln Pro
                   645                 650                 655
   Ser Ser Gln Gly Gly Ser Gln Pro Gly Ser Gln Ala Gly Ser Ser Glu
               660                 665                 670
   Arg Thr Arg Val Arg Ala Asn Ser Lys Ser Glu Gly Ser Pro Val Leu
           675                 680                 685
   Pro His Glu Pro Ala Lys Val Lys Pro Glu Glu Ser Arg Asp Ile Thr
       690                 695                 700
   Arg Pro Ser Arg Pro Ala Ser Tyr Lys Lys Ala Ile Asp Glu Asp Leu
   705                 710                 715                 720
   Thr Ala Leu Ala Lys Glu Leu Arg Glu Leu Arg Ile Glu Glu Thr Asn
                   725                 730                 735
   Arg Pro Met Lys Lys Val Thr Asp Tyr Ser Ser Ser Ser Glu Glu Ser
               740                 745                 750
   Glu Ser Ser Glu Glu Glu Glu Glu Asp Gly Glu Ser Glu Thr His Asp
           755                 760                 765
   Gly Thr Val Ala Val Ser Asp Ile Pro Arg Leu Ile Pro Thr Gly Ala
       770                 775                 780
   Pro Gly Ser Asn Glu Gln Tyr Asn Val Gly Met Val Gly Thr His Gly
   785                 790                 795                 800
   Leu Glu Thr Ser His Ala Asp Ser Phe Ser Gly Ser Ile Ser Arg Glu
                   805                 810                 815
   Gly Thr Leu Met Ile Arg Glu Thr Ser Gly Glu Lys Lys Arg Ser Gly
               820                 825                 830
   His Ser Asp Ser Asn Gly Phe Ala Gly His Ile Asn Leu Pro Asp Leu
           835                 840                 845
   Val Gln Gln Ser His Ser Pro Ala Gly Thr Pro Thr Glu Gly Leu Gly
       850                 855                 860
   Arg Val Ser Thr His Ser Gln Glu Met Asp Ser Gly Thr Glu Tyr Gly
   865                 870                 875                 880
   Met Gly Ser Ser Thr Lys Ala Ser Phe Thr Pro Phe Val Asp Pro Arg
   885 890 895
   Val Tyr Gln Thr Ser Pro Thr Asp Glu Asp Glu Glu Asp Glu Glu Ser
   900 905 910
   Ser Ala Ala Ala Leu Phe Thr Ser Glu Leu Leu Arg Gln Glu Gln Ala
   915 920 925
   Lys Leu Asn Glu Ala Arg Lys Ile Ser Val Val Asn Val Asn Pro Thr
   930 935 940
   Asn Ile Arg Pro His Ser Asp Thr Pro Glu Ile Arg Lys Tyr Lys Lys
   945 950 955 960
   Arg Phe Asn Ser Glu Ile Leu Cys Ala Ala Leu Trp Gly Val Asn Leu
   965 970 975
   Leu Val Gly Thr Glu Asn Gly Leu Met Leu Leu Asp Arg Ser Gly Gln
   980 985 990
   Gly Lys Val Tyr Asn Leu Ile Asn Arg Arg Arg Phe Gln Gln Met Asp
   995 1000 1005
   Val Leu Glu Gly Leu Asn Val Leu Val Thr Ile Ser Gly Lys Lys
   1010 1015 1020
   Asn Lys Leu Arg Val Tyr Tyr Leu Ser Trp Leu Arg Asn Arg Ile
   1025 1030 1035
   Leu His Asn Asp Pro Glu Val Glu Lys Lys Gln Gly Trp Ile Thr
   1040 1045 1050
   Val Gly Asp Leu Glu Gly Cys Ile His Tyr Lys Val Val Lys Tyr
   1055 1060 1065
   Glu Arg Ile Lys Phe Leu Val Ile Ala Leu Lys Asn Ala Val Glu
   1070 1075 1080
   Ile Tyr Ala Trp Ala Pro Lys Pro Tyr His Lys Phe Met Ala Phe
   1085 1090 1095
   Lys Ser Phe Ala Asp Leu Gln His Lys Pro Leu Leu Val Asp Leu
   1100 1105 1110
   Thr Val Glu Glu Gly Gln Arg Leu Lys Val Ile Phe Gly Ser His
   1115 1120 1125
   Thr Gly Phe His Val Ile Asp Val Asp Ser Gly Asn Ser Tyr Asp
       1130                1135                1140
   Ile Tyr Ile Pro Ser His Ile Gln Gly Asn Ile Thr Pro His Ala
       1145                1150                1155
   Ile Val Ile Leu Pro Lys Thr Asp Gly Met Glu Met Leu Val Cys
       1160                1165                1170
   Tyr Glu Asp Glu Gly Val Tyr Val Asn Thr Tyr Gly Arg Ile Thr
       1175                1180                1185
   Lys Asp Val Val Leu Gln Trp Gly Glu Met Pro Thr Ser Val Ala
       1190                1195                1200
   Tyr Ile His Ser Asn Gln Ile Met Gly Trp Gly Glu Lys Ala Ile
       1205                1210                1215
   Glu Ile Arg Ser Val Glu Thr Gly His Leu Asp Gly Val Phe Met
       1220                1225                1230
   His Lys Arg Ala Gln Arg Leu Lys Phe Leu Cys Glu Arg Asn Asp
       1235                1240                1245
   Lys Val Ile Gly His Ser Asn Phe Gly Thr Ile Arg Ser Thr Thr
       1250                1255                1260
   Cys Val Tyr Lys Gly Lys Trp Leu Tyr Glu Val Leu Ile Ser Ser
       1265                1270                1275
   Gln Gly Leu Met Gln Ile Gly Trp Cys Thr Ile Ser Cys Arg Phe
       1280                1285                1290
   Asn Gln Glu Glu Gly Val Gly Asp Thr His Asn Ser Tyr Ala Tyr
       1295                1300                1305
   Asp Gly Asn Arg Val Arg Lys Trp Asn Val Thr Thr Thr Asn Tyr
       1310                1315                1320
   Gly Lys Ala Trp Ala Ala Gly Asp Ile Val Ser Cys Leu Ile Asp
       1325                1330                1335
   Leu Asp Asp Gly Thr Leu Ser Phe Cys Leu Asn Gly Val Ser Leu
       1340                1345                1350
   Gly Thr Ala Phe Glu Asn Leu Ser Arg Gly Leu Gly Met Ala Tyr
       1355                1360                1365
   Phe Pro Ala Ile Ser Leu Ser Phe Lys Glu Ser Val Ala Phe Asn
   1370 1375 1380
   Phe Gly Ser Arg Pro Leu Arg Tyr Pro Val Ala Gly Tyr Arg Pro
   1385 1390 1395
   Leu Gln Asp Pro Pro Ser Ala Asp Leu Val Arg Ala Gln Arg Leu
   1400 1405 1410
   Leu Gly Cys Phe Arg Ala Val Leu Ser Val Glu Leu Asp Pro Val
   1415 1420 1425
   Glu Gly Arg Leu Leu Asp Lys Glu Ser Ser Lys Trp Arg Leu Arg
   1430 1435 1440
   Gly Gln Pro Thr Val Leu Leu Thr Leu Ala His Ile Phe His His
   1445 1450 1455
   Phe Ala Pro Leu Leu Arg Lys Val Tyr Leu Val Glu Ala Val Leu
   1460 1465 1470
   Met Ser Phe Leu Leu Gly Ile Val Glu Lys Gly Thr Pro Thr Gln
   1475 1480 1485
   Ala Gln Ser Val Val His Gln Val Leu Asp Leu Leu Trp Leu Phe
   1490 1495 1500
   Met Glu Asp Tyr Glu Val Gln Asp Cys Leu Lys Gln Leu Met Met
   1505 1510 1515
   Ser Leu Leu Arg Leu Tyr Arg Phe Ser Pro Ile Val Pro Asp Leu
   1520 1525 1530
   Gly Leu Gln Ile His Tyr Leu Arg Leu Thr Ile Ala Ile Leu Arg
   1535 1540 1545
   His Glu Lys Ser Arg Lys Phe Leu Leu Ser Asn Val Leu Phe Asp
   1550 1555 1560
   Val Leu Arg Ser Val Val Phe Phe Tyr Ile Lys Ser Pro Leu Arg
   1565 1570 1575
   Val Glu Glu Ala Gly Leu Gln Glu Leu Ile Pro Thr Thr Trp Trp
   1580 1585 1590
   Pro His Cys Ser Ser Arg Glu Gly Lys Glu Ser Thr Glu Met Lys
   1595 1600 1605
   Glu Glu Thr Ala Glu Glu Arg Leu Arg Arg Arg Ala Tyr Glu Arg
       1610                1615                1620
   Gly Cys Gln Arg Leu Arg Lys Arg Ile Glu Val Val Glu Glu Leu
       1625                1630                1635
   Gln Val Gln Ile Leu Lys Leu Leu Leu Asp Asn Lys Asp Asp Asn
       1640                1645                1650
   Gly Gly Glu Ala Ser Arg Tyr Ile Phe Leu Thr Lys Phe Arg Lys
       1655                1660                1665
   Phe Leu Gln Glu Asn Ala Ser Gly Arg Gly Asn Met Pro Met Leu
       1670                1675                1680
   Cys Pro Pro Glu Tyr Met Val Cys Phe Leu His Arg Leu Ile Ser
       1685                1690                1695
   Ala Leu Arg Tyr Tyr Trp Asp Glu Tyr Lys Ala Ser Asn Pro His
       1700                1705                1710
   Ala Ser Phe Ser Glu Glu Ala Tyr Ile Pro Pro Gln Val Phe Tyr
       1715                1720                1725
   Asn Gly Lys Val Asp Tyr Phe Asp Leu Gln Arg Leu Gly Gly Leu
       1730                1735                1740
   Leu Ser His Leu Arg Lys Thr Leu Lys Asp Asp Leu Ala Ser Lys
       1745                1750                1755
   Ala Asn Ile Val Ile Asp Pro Leu Glu Leu Gln Ser Thr Ala Met
       1760                1765                1770
   Asp Asp Leu Asp Glu Asp Glu Glu Pro Ala Pro Ala Met Ala Gln
       1775                1780                1785
   Arg Pro Met Gln Ala Leu Ala Val Gly Gly Pro Leu Pro Leu Pro
       1790                1795                1800
   Arg Pro Gly Trp Leu Ser Ser Pro Thr Leu Gly Arg Ala Asn Arg
       1805                1810                1815
   Phe Leu Ser Thr Ala Ala Val Ser Leu Met Thr Pro Arg Arg Pro
       1820                1825                1830
   Leu Ser Thr Ser Glu Lys Val Lys Val Arg Thr Leu Ser Val Glu
   1835                1840                1845
   Gln Arg Thr Arg Glu Asp Ile Glu Gly Ser His Trp Asn Glu Gly
       1850                1855                1860
   Leu Leu Leu Gly Arg Pro Pro Glu Glu Pro Glu Gln Pro Leu Thr
       1865                1870                1875
   Glu Asn Ser Leu Leu Glu Val Leu Asp Gly Ala Val Met Met Tyr
       1880                1885                1890
   Asn Leu Ser Val His Gln Gln Leu Gly Lys Met Val Gly Val Ser
       1895                1900                1905
   Asp Asp Val Asn Glu Tyr Ala Met Ala Leu Arg Asp Thr Glu Asp
       1910                1915                1920
   Lys Leu Arg Arg Cys Pro Lys Arg Arg Lys Asp Ile Leu Ala Glu
       1925                1930                1935
   Leu Thr Lys Ser Gln Lys Val Phe Ser Glu Lys Leu Asp His Leu
       1940                1945                1950
   Ser Arg Arg Leu Ala Trp Val His Ala Thr Val Tyr Ser Gln Glu
       1955                1960                1965
   Lys Met Leu Asp Ile Tyr Trp Leu Leu Arg Val Cys Leu Arg Thr
       1970                1975                1980
   Ile Glu His Gly Asp Arg Thr Gly Ser Leu Phe Ala Phe Met Pro
       1985                1990                1995
   Glu Phe Tyr Leu Ser Val Ala Ile Asn Ser Tyr Ser Ala Leu Lys
       2000                2005                2010
   Asn Tyr Phe Gly Pro Val His Ser Met Glu Glu Leu Pro Gly Tyr
       2015                2020                2025
   Glu Glu Thr Leu Thr Arg Leu Ala Ala Ile Leu Ala Lys His Phe
       2030                2035                2040
   Ala Asp Ala Arg Ile Val Gly Thr Asp Ile Arg Asp Ser Leu Met
       2045                2050                2055
   Gln Ala Leu Ala Ser Tyr Val Cys Tyr Pro His Ser Leu Arg Ala
       2060                2065                2070
   Val Glu Arg Ile Pro Glu Glu Gln Arg Ile Ala Met Val Arg Asn
       2075                2080                2085
   Leu Leu Ala Pro Tyr Glu Gln Arg Pro Trp Ala Gln Thr Asn Trp
       2090                2095                2100
   Ile Leu Val Arg Leu Trp Arg Gly Cys Gly Phe Gly Tyr Arg Tyr
       2105                2110                2115
   Thr Arg Leu Pro His Leu Leu Lys Thr Lys Leu Glu Asp Ala Asn
       2120                2125                2130
   Leu Pro Ser Leu Gln Lys Pro Cys Pro Ser Thr Leu Leu Gln Gln
       2135                2140                2145
   His Met Ala Asp Leu Leu Gln Gln Gly Pro Asp Val Ala Pro Ser
       2150                2155                2160
   Phe Leu Asn Ser Val Leu Asn Gln Leu Asn Trp Ala Phe Ser Glu
       2165                2170                2175
   Phe Ile Gly Met Ile Gln Glu Ile Gln Gln Ala Ala Glu Arg Leu
       2180                2185                2190
   Glu Arg Asn Phe Val Asp Ser Arg Gln Leu Lys Val Cys Ala Thr
       2195                2200                2205
   Cys Phe Asp Leu Ser Val Ser Leu Leu Arg Val Leu Glu Met Thr
       2210                2215                2220
   Ile Thr Leu Val Pro Glu Ile Phe Leu Asp Trp Thr Arg Pro Thr
       2225                2230                2235
   Ser Glu Met Leu Leu Arg Arg Leu Ala Gln Leu Leu Asn Gln Val
       2240                2245                2250
   Leu Asn Arg Val Thr Ala Glu Arg Asn Leu Phe Asp Arg Val Val
       2255                2260                2265
   Thr Leu Arg Leu Pro Gly Leu Glu Ser Val Asp His Tyr Pro Ile
       2270                2275                2280
   Leu Val Ala Val Thr Gly Ile Leu Val Gln Leu Leu Val Arg Gly
       2285                2290                2295
   Pro Ala Ser Glu Arg Glu Gln Ala Thr Ser Val Leu Leu Ala Asp
       2300                2305                2310
   Pro Cys Phe Gln Leu Arg Ser Ile Cys Tyr Leu Leu Gly Gln Pro
       2315                2320                2325
   Glu Pro Pro Ala Pro Gly Thr Ala Leu Pro Ala Pro Asp Arg Lys
       2330                2335                2340
   Arg Phe Ser Leu Gln Ser Tyr Ala Asp Tyr Ile Ser Ala Asp Glu
       2345                2350                2355
   Leu Ala Gln Val Glu Gln Met Leu Ala His Leu Thr Ser Ala Ser
       2360                2365                2370
   Ala Gln Ala Ala Ala Ala Ser Leu Pro Thr Ser Glu Glu Asp Leu
       2375                2380                2385
   Cys Pro Ile Cys Tyr Ala His Pro Ile Ser Ala Val Phe Gln Pro
       2390                2395                2400
   Cys Gly His Lys Ser Cys Lys Ala Cys Ile Asn Gln His Leu Met
       2405                2410                2415
   Asn Asn Lys Asp Cys Phe Phe Cys Lys Thr Thr Ile Val Ser Val
       2420                2425                2430
   Glu Asp Trp Glu Lys Gly Ala Asn Thr Ser Thr Thr Ser Ser Ala
       2435                2440                2445
   Ala

<210> 17
<211> 7326
<212> DNA
<213> Homo sapiens

<220>
<221> CDS
<222> (1)..(7326)

<400> 17
atg gcg agc gac tcc ccg gct cga agc ctg gat gaa ata gat ctc tcg      48
Met Ala Ser Asp Ser Pro Ala Arg Ser Leu Asp Glu Ile Asp Leu Ser
1               5                   10                  15

gct ctg agg gac ccc gca ggg atc ttt gaa ttg gtg gaa ctt gtt gga      96
Ala Leu Arg Asp Pro Ala Gly Ile Phe Glu Leu Val Glu Leu Val Gly
            20                  25                  30

aat gga aca tac ggg caa gtt tat aag ggt cgt cat gtc aaa acg ggc      144
Asn Gly Thr Tyr Gly Gln Val Tyr Lys Gly Arg His Val Lys Thr Gly
        35                  40                  45

cag ctt gca gcc atc aag gtt atg gat gtc aca ggg gat gaa gag gaa 192
Gln Leu Ala Ala Ile Lys Val Met Asp Val Thr Gly Asp Glu Glu Glu
    50                  55                  60

gaa atc aaa caa gaa att aac atg ttg aag aaa tat tct cat cac cgg 240
Glu Ile Lys Gln Glu Ile Asn Met Leu Lys Lys Tyr Ser His His Arg
65                  70                  75                  80

aat att gct aca tac tat ggt gct ttt atc aaa aag aac cca cca ggc 288
Asn Ile Ala Thr Tyr Tyr Gly Ala Phe Ile Lys Lys Asn Pro Pro Gly
                85                  90                  95

atg gat gac caa ctt tgg ttg gtg atg gag ttt tgt ggt gct ggc tct 336
Met Asp Asp Gln Leu Trp Leu Val Met Glu Phe Cys Gly Ala Gly Ser
            100                 105                 110

gtc acc gac ctg atc aag aac aca aaa ggt aac acg ttg aaa gag gag 384
Val Thr Asp Leu Ile Lys Asn Thr Lys Gly Asn Thr Leu Lys Glu Glu
        115                 120                 125

tgg att gca tac atc tgc agg gaa atc tta cgg ggg ctg agt cac ctg 432
Trp Ile Ala Tyr Ile Cys Arg Glu Ile Leu Arg Gly Leu Ser His Leu
    130                 135                 140

cac cag cat aaa gtg att cat cga gat att aaa ggg caa aat gtc ttg 480
His Gln His Lys Val Ile His Arg Asp Ile Lys Gly Gln Asn Val Leu
145                 150                 155                 160

ctg act gaa aat gca gaa gtt aaa cta gtg gac ttt gga gtc agt gct 528
Leu Thr Glu Asn Ala Glu Val Lys Leu Val Asp Phe Gly Val Ser Ala
                165                 170                 175

cag ctt gat cga aca gtg ggc agg agg aat act ttc att gga act ccc 576
Gln Leu Asp Arg Thr Val Gly Arg Arg Asn Thr Phe Ile Gly Thr Pro
            180                 185                 190

tac tgg atg gca cca gaa gtt att gcc tgt gat gaa aac cca gat gcc 624
Tyr Trp Met Ala Pro Glu Val Ile Ala Cys Asp Glu Asn Pro Asp Ala
        195                 200                 205

aca tat gat ttc aag agt gac ttg tgg tct ttg ggt atc acc gcc att 672
Thr Tyr Asp Phe Lys Ser Asp Leu Trp Ser Leu Gly Ile Thr Ala Ile
    210                 215                 220

gaa atg gca gaa ggt gct ccc cct ctc tgt gac atg cac ccc atg aga 720
Glu Met Ala Glu Gly Ala Pro Pro Leu Cys Asp Met His Pro Met Arg
225                 230                 235                 240

gct ctc ttc ctc atc ccc cgg aac cca gcg cct cgg ctg aag tct aag 768
Ala Leu Phe Leu Ile Pro Arg Asn Pro Ala Pro Arg Leu Lys Ser Lys
                245                 250                 255

aag tgg tca aaa aaa ttc cag tca ttt att gag agc tgc ttg gta aag 816
Lys Trp Ser Lys Lys Phe Gln Ser Phe Ile Glu Ser Cys Leu Val Lys
            260                 265                 270

aat cac agc cag cga cca gca aca gaa caa ttg atg aag cat cca ttt 864
Asn His Ser Gln Arg Pro Ala Thr Glu Gln Leu Met Lys His Pro Phe
        275                 280                 285

ata cga gac caa cct aat gag cga cag gtc cgc att caa ctc aag gac 912
Ile Arg Asp Gln Pro Asn Glu Arg Gln Val Arg Ile Gln Leu Lys Asp

290                 295                 300

cat att gat aga aca aag aag aag cga gga gaa aaa gat gag aca gag        960
His Ile Asp Arg Thr Lys Lys Lys Arg Gly Glu Lys Asp Glu Thr Glu
305                 310                 315                 320

tat gag tac agt gga agt gag gaa gaa gag gag gag aat gac tca gga       1008
Tyr Glu Tyr Ser Gly Ser Glu Glu Glu Glu Glu Glu Asn Asp Ser Gly
                325                 330                 335

gag ccc agc tcc atc ctg aat ctg cca ggg gag tcg acg ctg cgg agg       1056
Glu Pro Ser Ser Ile Leu Asn Leu Pro Gly Glu Ser Thr Leu Arg Arg
            340                 345                 350

gac ttt ctg agg ctg cag ctg gcc aac aag gag cgt tct gag gcc cta       1104
Asp Phe Leu Arg Leu Gln Leu Ala Asn Lys Glu Arg Ser Glu Ala Leu
        355                 360                 365

cgg agg cag cag ctg gag cag cag cag cgg gag aat gag gag cac aag       1152
Arg Arg Gln Gln Leu Glu Gln Gln Gln Arg Glu Asn Glu Glu His Lys
    370                 375                 380

cgg cag ctg ctg gcc gag cgt cag aag cgc atc gag gag cag aaa gag       1200
Arg Gln Leu Leu Ala Glu Arg Gln Lys Arg Ile Glu Glu Gln Lys Glu
385                 390                 395                 400

cag agg cgg cgg ctg gag gag caa caa agg cga gag aag gag ctg cgg       1248
Gln Arg Arg Arg Leu Glu Glu Gln Gln Arg Arg Glu Lys Glu Leu Arg
                405                 410                 415

aag cag cag gag agg gag cag cgc cgg cac tat gag gag cag atg cgc       1296
Lys Gln Gln Glu Arg Glu Gln Arg Arg His Tyr Glu Glu Gln Met Arg
            420                 425                 430

cgg gag gag gag agg agg cgt gcg gag cat gaa cag gaa tat aag cgc       1344
Arg Glu Glu Glu Arg Arg Arg Ala Glu His Glu Gln Glu Tyr Lys Arg
        435                 440                 445

aaa caa ttg gaa gaa cag aga caa gca gaa aga ctg cag agg cag cta       1392
Lys Gln Leu Glu Glu Gln Arg Gln Ala Glu Arg Leu Gln Arg Gln Leu
    450                 455                 460

aag caa gaa aga gac tac tta gtt tcc ctt cag cat cag cgg cag gag       1440
Lys Gln Glu Arg Asp Tyr Leu Val Ser Leu Gln His Gln Arg Gln Glu
465                 470                 475                 480

cag agg cct gtg gag aag aag cca ctg tac cat tac aaa gaa gga atg       1488
Gln Arg Pro Val Glu Lys Lys Pro Leu Tyr His Tyr Lys Glu Gly Met
                485                 490                 495

agt cct agt gag aag cca gca tgg gcc aag gag atc cca cat ctg gta       1536
Ser Pro Ser Glu Lys Pro Ala Trp Ala Lys Glu Ile Pro His Leu Val
            500                 505                 510

gct gta aaa tcc cag gga cct gcc ttg acc gcc tcc cag tca gtg cac       1584
Ala Val Lys Ser Gln Gly Pro Ala Leu Thr Ala Ser Gln Ser Val His
        515                 520                 525

gag cag ccc aca aag ggc ctc tct ggg ttt cag gag gct ctg aac gtg       1632
Glu Gln Pro Thr Lys Gly Leu Ser Gly Phe Gln Glu Ala Leu Asn Val
    530                 535                 540

acc tcc cac cgc gtg gag atg cca cgc cag aac tca gat ccc acc tcg       1680

Thr Ser His Arg Val Glu Met Pro Arg Gln Asn Ser Asp Pro Thr Ser
545                 550                 555                 560

gaa aat cct cct ctc ccc act cgc att gaa aag ttt gac cga agc tct      1728
Glu Asn Pro Pro Leu Pro Thr Arg Ile Glu Lys Phe Asp Arg Ser Ser
                565                 570                 575

tgg tta cga cag gaa gaa gac att cca cca aag gtg cct caa aga aca      1776
Trp Leu Arg Gln Glu Glu Asp Ile Pro Pro Lys Val Pro Gln Arg Thr
            580                 585                 590

act tct ata tcc cca gca tta gcc aga aag aat tct cct ggg aat ggt      1824
Thr Ser Ile Ser Pro Ala Leu Ala Arg Lys Asn Ser Pro Gly Asn Gly
        595                 600                 605

agt gct ctg gga ccc aga cta gga tct caa ccc atc aga gca agc aac      1872
Ser Ala Leu Gly Pro Arg Leu Gly Ser Gln Pro Ile Arg Ala Ser Asn
    610                 615                 620

cct gat ctc cgg aga act gag ccc atc ttg gag agc ccc ttg cag agg      1920
Pro Asp Leu Arg Arg Thr Glu Pro Ile Leu Glu Ser Pro Leu Gln Arg
625                 630                 635                 640

acc agc agt ggc agt tcc tcc agc tcc agc acc cct agc tcc cag ccc      1968
Thr Ser Ser Gly Ser Ser Ser Ser Ser Ser Thr Pro Ser Ser Gln Pro
                645                 650                 655

agc tcc caa gga ggc tcc cag cct gga tca caa gca gga tcc agt gaa      2016
Ser Ser Gln Gly Gly Ser Gln Pro Gly Ser Gln Ala Gly Ser Ser Glu
            660                 665                 670

cgc acc aga gtt cga gcc aac agt aag tca gaa gga tca cct gtg ctc      2064
Arg Thr Arg Val Arg Ala Asn Ser Lys Ser Glu Gly Ser Pro Val Leu
        675                 680                 685

ccc cat gag cct gcc aag gtg aaa cca gaa gaa tcc agg gac att acc      2112
Pro His Glu Pro Ala Lys Val Lys Pro Glu Glu Ser Arg Asp Ile Thr
    690                 695                 700

cgg ccc agt cga cca gct gat ctg acg gca tta gcc aaa gaa cta aga      2160
Arg Pro Ser Arg Pro Ala Asp Leu Thr Ala Leu Ala Lys Glu Leu Arg
705                 710                 715                 720

gaa ctc cgg att gaa gaa aca aac cgc cca atg aag aag gtg act gat      2208
Glu Leu Arg Ile Glu Glu Thr Asn Arg Pro Met Lys Lys Val Thr Asp
                725                 730                 735

tac tcc tcc tcc agt gag gag tca gaa agt agc gag gaa gag gag gaa      2256
Tyr Ser Ser Ser Ser Glu Glu Ser Glu Ser Ser Glu Glu Glu Glu Glu
            740                 745                 750

gat gga gag agc gag acc cat gat ggg aca gtg gct gtc agc gac ata      2304
Asp Gly Glu Ser Glu Thr His Asp Gly Thr Val Ala Val Ser Asp Ile
        755                 760                 765

ccc aga ctg ata cca aca gga gct cca ggc agc aac gag cag tac aat      2352
Pro Arg Leu Ile Pro Thr Gly Ala Pro Gly Ser Asn Glu Gln Tyr Asn
    770                 775                 780

gtg gga atg gtg ggg acg cat ggg ctg gag acc tct cat gcg gac agt      2400
Val Gly Met Val Gly Thr His Gly Leu Glu Thr Ser His Ala Asp Ser
785                 790                 795                 800

ttc agc ggc agt att tca aga gaa gga acc ttg atg att aga gag acg 2448
Phe Ser Gly Ser Ile Ser Arg Glu Gly Thr Leu Met Ile Arg Glu Thr
                805                 810                 815

tct gga gag aag aag cga tct ggc cac agt gac agc aat ggc ttt gct 2496
Ser Gly Glu Lys Lys Arg Ser Gly His Ser Asp Ser Asn Gly Phe Ala
            820                 825                 830

ggc cac atc aac ctc cct gac ctg gtg cag cag agc cat tct cca gct 2544
Gly His Ile Asn Leu Pro Asp Leu Val Gln Gln Ser His Ser Pro Ala
        835                 840                 845

gga acc ccg act gag gga ctg ggg cgc gtc tca acc cat tcc cag gag 2592
Gly Thr Pro Thr Glu Gly Leu Gly Arg Val Ser Thr His Ser Gln Glu
    850                 855                 860

atg gac tct ggg act gaa tat ggc atg ggg agc agc acc aaa gcc tcc 2640
Met Asp Ser Gly Thr Glu Tyr Gly Met Gly Ser Ser Thr Lys Ala Ser
865                 870                 875                 880

ttc acc ccc ttt gtg gac ccc aga gta tac cag acg tct ccc act gat 2688
Phe Thr Pro Phe Val Asp Pro Arg Val Tyr Gln Thr Ser Pro Thr Asp
                885                 890                 895

gaa gat gaa gag gat gag gaa tca tca gcc gca gct ctg ttt act agc 2736
Glu Asp Glu Glu Asp Glu Glu Ser Ser Ala Ala Ala Leu Phe Thr Ser
            900                 905                 910

gaa ctt ctt agg caa gaa cag gcc aaa ctc aat gaa gca aga aag att 2784
Glu Leu Leu Arg Gln Glu Gln Ala Lys Leu Asn Glu Ala Arg Lys Ile
        915                 920                 925

tcg gtg gta aat gta aac cca acc aac att cgg cct cat agc gac aca 2832
Ser Val Val Asn Val Asn Pro Thr Asn Ile Arg Pro His Ser Asp Thr
    930                 935                 940

cca gaa atc aga aaa tac aag aaa cga ttc aac tca gaa ata ctt tgt 2880
Pro Glu Ile Arg Lys Tyr Lys Lys Arg Phe Asn Ser Glu Ile Leu Cys
945                 950                 955                 960

gca gct ctg tgg ggt gta aac ctt ctg gtg ggg act gaa aat ggc ctg 2928
Ala Ala Leu Trp Gly Val Asn Leu Leu Val Gly Thr Glu Asn Gly Leu
                965                 970                 975

atg ctt ttg gac cga agt ggg caa ggc aaa gtc tat aat ctg atc aac 2976
Met Leu Leu Asp Arg Ser Gly Gln Gly Lys Val Tyr Asn Leu Ile Asn
            980                 985                 990

cgg agg cga ttt cag cag atg gat gtg cta gag gga ctg aat gtc ctt 3024
Arg Arg Arg Phe Gln Gln Met Asp Val Leu Glu Gly Leu Asn Val Leu
        995                 1000                1005

gtg aca att tca gga aag aag aat aag cta cga gtt tac tat ctt 3069
Val Thr Ile Ser Gly Lys Lys Asn Lys Leu Arg Val Tyr Tyr Leu
    1010                1015                1020

tca tgg tta aga aac aga ata cta cat aat gac cca gaa gta gaa 3114
Ser Trp Leu Arg Asn Arg Ile Leu His Asn Asp Pro Glu Val Glu
    1025                1030                1035

aag aaa caa ggc tgg atc act gtt ggg gac ttg gaa ggc tgt ata 3159
Lys Lys Gln Gly Trp Ile Thr Val Gly Asp Leu Glu Gly Cys Ile
    1040                1045                1050

cat tat aaa gtt gtt aaa tat gaa agg atc aaa ttt ttg gtg att 3204
His Tyr Lys Val Val Lys Tyr Glu Arg Ile Lys Phe Leu Val Ile
    1055                1060                1065

gcc tta aag aat gct gtg gaa ata tat gct tgg gct cct aaa ccg 3249
Ala Leu Lys Asn Ala Val Glu Ile Tyr Ala Trp Ala Pro Lys Pro
    1070                1075                1080

tat cat aaa ttc atg gca ttt aag tct ttt gca gat ctc cag cac 3294
Tyr His Lys Phe Met Ala Phe Lys Ser Phe Ala Asp Leu Gln His
    1085                1090                1095

aag cct ctg cta gtt gat ctc acg gta gaa gaa ggt caa aga tta 3339
Lys Pro Leu Leu Val Asp Leu Thr Val Glu Glu Gly Gln Arg Leu
    1100                1105                1110

aag gtt att ttt ggt tca cac act ggt ttc cat gta att gat gtt 3384
Lys Val Ile Phe Gly Ser His Thr Gly Phe His Val Ile Asp Val
    1115                1120                1125

gat tca gga aac tct tat gat atc tac ata cca tct cat att cag 3429
Asp Ser Gly Asn Ser Tyr Asp Ile Tyr Ile Pro Ser His Ile Gln
    1130                1135                1140

ggc aat atc act cct cat gct att gtc atc ttg cct aaa aca gat 3474
Gly Asn Ile Thr Pro His Ala Ile Val Ile Leu Pro Lys Thr Asp
    1145                1150                1155

gga atg gaa atg ctt gtt tgc tat gag gat gag ggg gtg tat gta 3519
Gly Met Glu Met Leu Val Cys Tyr Glu Asp Glu Gly Val Tyr Val
    1160                1165                1170

aac acc tat ggc cgg ata act aag gat gtg gtg ctc caa tgg gga 3564
Asn Thr Tyr Gly Arg Ile Thr Lys Asp Val Val Leu Gln Trp Gly
    1175                1180                1185

gaa atg ccc acg tct gtg gcc tac att cat tcc aat cag ata atg 3609
Glu Met Pro Thr Ser Val Ala Tyr Ile His Ser Asn Gln Ile Met
    1190                1195                1200

ggc tgg ggc gag aaa gct att gag atc cgg tca gtg gaa aca gga 3654
Gly Trp Gly Glu Lys Ala Ile Glu Ile Arg Ser Val Glu Thr Gly
    1205                1210                1215

cat ttg gat gga gta ttt atg cat aag cga gct caa agg tta aag 3699
His Leu Asp Gly Val Phe Met His Lys Arg Ala Gln Arg Leu Lys
    1220                1225                1230

ttt cta tgt gaa aga aat gat aag gtg att gga cac agc aac ttt 3744
Phe Leu Cys Glu Arg Asn Asp Lys Val Ile Gly His Ser Asn Phe
    1235                1240                1245

ggc acc atc cgc tct acc aca tgc gtg tac aaa ggg aaa tgg ctc 3789
Gly Thr Ile Arg Ser Thr Thr Cys Val Tyr Lys Gly Lys Trp Leu
    1250                1255                1260

tac gag gtc ctc atc tcc tcc cag ggg ctc atg cag atc ggc tgg 3834
Tyr Glu Val Leu Ile Ser Ser Gln Gly Leu Met Gln Ile Gly Trp
    1265                1270                1275

tgc acc atc agc tgc cgc ttc aac cag gag gag ggg gtt gga gat 3879
Cys Thr Ile Ser Cys Arg Phe Asn Gln Glu Glu Gly Val Gly Asp

1280                    1285                    1290

aca cac aac tcc tat gcc tat gat ggc aac cgc gtg cgc aag tgg          3924
Thr His Asn Ser Tyr Ala Tyr Asp Gly Asn Arg Val Arg Lys Trp
    1295                    1300                    1305

aat gtg acc aca acg aat tat ggc aag gcg tgg gca gcg ggg gac          3969
Asn Val Thr Thr Thr Asn Tyr Gly Lys Ala Trp Ala Ala Gly Asp
    1310                    1315                    1320

atc gtg agc tgc ctg att gac ctg gat gat ggc act ctg tcc ttc          4014
Ile Val Ser Cys Leu Ile Asp Leu Asp Asp Gly Thr Leu Ser Phe
    1325                    1330                    1335

tgc ctg aac ggt gta tca ctg ggc act gcc ttt gag aac ctg tcc          4059
Cys Leu Asn Gly Val Ser Leu Gly Thr Ala Phe Glu Asn Leu Ser
    1340                    1345                    1350

agg ggc ctg ggt atg gcc tac ttc cca gcc atc agc ctc tct ttc          4104
Arg Gly Leu Gly Met Ala Tyr Phe Pro Ala Ile Ser Leu Ser Phe
    1355                    1360                    1365

aag gag tcc gtg gcc ttc aac ttt ggc agc cgt cct ctg cgc tac          4149
Lys Glu Ser Val Ala Phe Asn Phe Gly Ser Arg Pro Leu Arg Tyr
    1370                    1375                    1380

cca gtg gca ggc tac cgg ccc ctg cag gac cca ccg agt gct gac          4194
Pro Val Ala Gly Tyr Arg Pro Leu Gln Asp Pro Pro Ser Ala Asp
    1385                    1390                    1395

ctg gtg cgg gca cag agg ttg ctg ggc tgc ttc cgg gca gtg ctg          4239
Leu Val Arg Ala Gln Arg Leu Leu Gly Cys Phe Arg Ala Val Leu
    1400                    1405                    1410

agt gtg gag ctg gac cct gtg gag ggg cgg ctg ttg gac aag gag          4284
Ser Val Glu Leu Asp Pro Val Glu Gly Arg Leu Leu Asp Lys Glu
    1415                    1420                    1425

agc tcc aag tgg cgg ttg cgg ggc cag ccc acc gtc ctc ctc aca          4329
Ser Ser Lys Trp Arg Leu Arg Gly Gln Pro Thr Val Leu Leu Thr
    1430                    1435                    1440

ctg gcc cac atc ttc cat cac ttt gca ccg ctt ctg cgc aag gtg          4374
Leu Ala His Ile Phe His His Phe Ala Pro Leu Leu Arg Lys Val
    1445                    1450                    1455

tat ctg gtg gag gct gtg ctc atg agc ttc ttg ctg ggc atc gtg          4419
Tyr Leu Val Glu Ala Val Leu Met Ser Phe Leu Leu Gly Ile Val
    1460                    1465                    1470

gag aag ggc aca ccc aca cag gca cag tcc gtg gtg cac cag gtc          4464
Glu Lys Gly Thr Pro Thr Gln Ala Gln Ser Val Val His Gln Val
    1475                    1480                    1485

ctg gac ctc ttg tgg ctc ttc atg gag gac tac gag gta caa gat          4509
Leu Asp Leu Leu Trp Leu Phe Met Glu Asp Tyr Glu Val Gln Asp
    1490                    1495                    1500

tgc ctc aag cag ttg atg atg tct ctg ctt cgg ctg tac cga ttc          4554
Cys Leu Lys Gln Leu Met Met Ser Leu Leu Arg Leu Tyr Arg Phe
    1505                    1510                    1515

tca ccc att gtc cca gac ctg ggc cta cag atc cat tac ctg cgg          4599

Ser Pro Ile Val Pro Asp Leu Gly Leu Gln Ile His Tyr Leu Arg
    1520                1525                1530

ctc act atc gcc atc ctg agg cat gag aag tcc cgc aag ttt ctg 4644
Leu Thr Ile Ala Ile Leu Arg His Glu Lys Ser Arg Lys Phe Leu
    1535                1540                1545

ctt agc aat gtc ctc ttc gac gtg ctc cgc tcc gtc gtc ttc ttt 4689
Leu Ser Asn Val Leu Phe Asp Val Leu Arg Ser Val Val Phe Phe
    1550                1555                1560

tac atc aag agc ccc ctg cgt gtg gag gag gcc ggc ctg cag gag 4734
Tyr Ile Lys Ser Pro Leu Arg Val Glu Glu Ala Gly Leu Gln Glu
    1565                1570                1575

ctc att ccc acc acc tgg tgg ccc cac tgc tcc agt agg gag ggc 4779
Leu Ile Pro Thr Thr Trp Trp Pro His Cys Ser Ser Arg Glu Gly
    1580                1585                1590

aaa gag agc acg gag atg aag gag gag acc gca gag gag cgg ctg 4824
Lys Glu Ser Thr Glu Met Lys Glu Glu Thr Ala Glu Glu Arg Leu
    1595                1600                1605

cgg cgg cga gcc tac gaa cgg ggc tgt cag cgg ctc agg aag cgc 4869
Arg Arg Arg Ala Tyr Glu Arg Gly Cys Gln Arg Leu Arg Lys Arg
    1610                1615                1620

atc gaa gtg gtg gaa gaa cta cag gtc cag atc ctg aag ctg ctg 4914
Ile Glu Val Val Glu Glu Leu Gln Val Gln Ile Leu Lys Leu Leu
    1625                1630                1635

ctg gac aat aaa gat gac aat ggg ggt gaa gct tct agg tat atc 4959
Leu Asp Asn Lys Asp Asp Asn Gly Gly Glu Ala Ser Arg Tyr Ile
    1640                1645                1650

ttc ctg acc aag ttt cgc aag ttt ctg cag gag aac gcc agt ggc 5004
Phe Leu Thr Lys Phe Arg Lys Phe Leu Gln Glu Asn Ala Ser Gly
    1655                1660                1665

cgg ggg aac atg ccc atg ctc tgc ccc cct gag tac atg gtc tgc 5049
Arg Gly Asn Met Pro Met Leu Cys Pro Pro Glu Tyr Met Val Cys
    1670                1675                1680

ttc tta cac cgg ctg atc tct gcc ctg cgc tac tat tgg gat gaa 5094
Phe Leu His Arg Leu Ile Ser Ala Leu Arg Tyr Tyr Trp Asp Glu
    1685                1690                1695

tac aag gct tcc aat cct cat gct tcc ttc agt gag gag gcc tac 5139
Tyr Lys Ala Ser Asn Pro His Ala Ser Phe Ser Glu Glu Ala Tyr
    1700                1705                1710

atc ccg ccc cag gtc ttc tat aat ggc aag gtg gac tac ttt gac 5184
Ile Pro Pro Gln Val Phe Tyr Asn Gly Lys Val Asp Tyr Phe Asp
    1715                1720                1725

ctg cag cgc ctg ggg ggc ctc ctc tcg cac ctg cgg aag acc ctc 5229
Leu Gln Arg Leu Gly Gly Leu Leu Ser His Leu Arg Lys Thr Leu
    1730                1735                1740

aaa gat gac ctt gct tcc aaa gcc aac att gtg atc gac cca ctg 5274
Lys Asp Asp Leu Ala Ser Lys Ala Asn Ile Val Ile Asp Pro Leu
    1745                1750                1755

gag ctc cag tca acc gcc atg gat gac cta gat gag gat gag gag 5319
Glu Leu Gln Ser Thr Ala Met Asp Asp Leu Asp Glu Asp Glu Glu
    1760                1765                1770

cca gcc cca gct atg gcc cag cgc ccc atg cag gcc ctg gct gtt 5364
Pro Ala Pro Ala Met Ala Gln Arg Pro Met Gln Ala Leu Ala Val
    1775                1780                1785

ggg ggg cca ctg ccc ctg ccc cgg ccc ggc tgg ctc agt tct cca 5409
Gly Gly Pro Leu Pro Leu Pro Arg Pro Gly Trp Leu Ser Ser Pro
    1790                1795                1800

act ttg ggc cga gcc aac cgc ttc ctc agc aca gcg gct gtg agc 5454
Thr Leu Gly Arg Ala Asn Arg Phe Leu Ser Thr Ala Ala Val Ser
    1805                1810                1815

ctc atg acc cca cgg cgg cct ctg agc acc tcg gag aaa gtg aag 5499
Leu Met Thr Pro Arg Arg Pro Leu Ser Thr Ser Glu Lys Val Lys
    1820                1825                1830

gtc cgc acg ctg agc gtg gag cag agg acc cgt gag gac att gaa 5544
Val Arg Thr Leu Ser Val Glu Gln Arg Thr Arg Glu Asp Ile Glu
    1835                1840                1845

ggc agc cac tgg aat gag ggc ttg ctg ctg ggg cgg ccc ccc gag 5589
Gly Ser His Trp Asn Glu Gly Leu Leu Leu Gly Arg Pro Pro Glu
    1850                1855                1860

gag cct gag cag ccc ctc acc gag aac tcg ctg ctg gaa gtc ctg 5634
Glu Pro Glu Gln Pro Leu Thr Glu Asn Ser Leu Leu Glu Val Leu
    1865                1870                1875

gat ggg gcg gtc atg atg tac aac ctc agc gta cac cag cag ctg 5679
Asp Gly Ala Val Met Met Tyr Asn Leu Ser Val His Gln Gln Leu
    1880                1885                1890

ggc aag atg gtg ggt gtc tcc gat gat gtc aat gaa tac gct atg 5724
Gly Lys Met Val Gly Val Ser Asp Asp Val Asn Glu Tyr Ala Met
    1895                1900                1905

gct ctg agg gac aca gag gac aag ctc cgc cgg tgc ccc aag agg 5769
Ala Leu Arg Asp Thr Glu Asp Lys Leu Arg Arg Cys Pro Lys Arg
    1910                1915                1920

agg aag gac atc ctt gca gag ttg acc aag agc cag aag gtt ttc 5814
Arg Lys Asp Ile Leu Ala Glu Leu Thr Lys Ser Gln Lys Val Phe
    1925                1930                1935

tca gaa aag ctg gac cac ctg agc cgc cgt ctt gcc tgg gtc cat 5859
Ser Glu Lys Leu Asp His Leu Ser Arg Arg Leu Ala Trp Val His
    1940                1945                1950

gcc act gtc tac tcc cag gag aag atg ctg gac atc tac tgg ctg 5904
Ala Thr Val Tyr Ser Gln Glu Lys Met Leu Asp Ile Tyr Trp Leu
    1955                1960                1965

ctg cgc gtc tgc ctg cgg acc att gag cac ggt gat cgc aca ggg 5949
Leu Arg Val Cys Leu Arg Thr Ile Glu His Gly Asp Arg Thr Gly
    1970                1975                1980

tct ctc ttt gcc ttc atg ccc gag ttc tac ctg agc gtg gcc atc 5994
Ser Leu Phe Ala Phe Met Pro Glu Phe Tyr Leu Ser Val Ala Ile
    1985                1990                1995

aac agc tac agt gct ctc aag aat tac ttt ggt ccc gtg cac agc 6039
Asn Ser Tyr Ser Ala Leu Lys Asn Tyr Phe Gly Pro Val His Ser
    2000                2005                2010

atg gag gag ctc cca ggc tat gaa gag acc ctg acc cgc ctg gct 6084
Met Glu Glu Leu Pro Gly Tyr Glu Glu Thr Leu Thr Arg Leu Ala
    2015                2020                2025

gcc att ctc gcc aaa cac ttt gcc gac gca cgc att gtg ggc act 6129
Ala Ile Leu Ala Lys His Phe Ala Asp Ala Arg Ile Val Gly Thr
    2030                2035                2040

gac atc cga gac tca ctg atg cag gcc ctg gcc agc tac gtg tgc 6174
Asp Ile Arg Asp Ser Leu Met Gln Ala Leu Ala Ser Tyr Val Cys
    2045                2050                2055

tac cca cac tcc ctg cgg gct gtg gag cga atc ccc gag gag cag 6219
Tyr Pro His Ser Leu Arg Ala Val Glu Arg Ile Pro Glu Glu Gln
    2060                2065                2070

cgt atc gcc atg gtg agg aac ctc ctg gcg ccc tat gag cag cgg 6264
Arg Ile Ala Met Val Arg Asn Leu Leu Ala Pro Tyr Glu Gln Arg
    2075                2080                2085

ccc tgg gcc cag acc aac tgg atc ctg gtg cgg ctc tgg agg ggc 6309
Pro Trp Ala Gln Thr Asn Trp Ile Leu Val Arg Leu Trp Arg Gly
    2090                2095                2100

tgt ggc ttc ggg tac cgc tat aca cgg ctg cca cat ctg ctg aaa 6354
Cys Gly Phe Gly Tyr Arg Tyr Thr Arg Leu Pro His Leu Leu Lys
    2105                2110                2115

acc aaa ctt gag gac gcc aat ttg ccc agc ctc cag aag ccc tgc 6399
Thr Lys Leu Glu Asp Ala Asn Leu Pro Ser Leu Gln Lys Pro Cys
    2120                2125                2130

cct tcc acc ctg ctg cag cag cac atg gcg gac ctc cta cag cag 6444
Pro Ser Thr Leu Leu Gln Gln His Met Ala Asp Leu Leu Gln Gln
    2135                2140                2145

ggt cct gat gtg gca ccc agc ttc ctc aac agc gtc ctc aat cag 6489
Gly Pro Asp Val Ala Pro Ser Phe Leu Asn Ser Val Leu Asn Gln
    2150                2155                2160

ctc aac tgg gcc ttc tct gaa ttc att ggc atg atc caa gag atc 6534
Leu Asn Trp Ala Phe Ser Glu Phe Ile Gly Met Ile Gln Glu Ile
    2165                2170                2175

cag cag gct gct gag cgc ctg gag cgg aac ttt gtg gac agc cgg 6579
Gln Gln Ala Ala Glu Arg Leu Glu Arg Asn Phe Val Asp Ser Arg
    2180                2185                2190

cag ctc aag gta tgt gcc acc tgc ttt gac ctc tcg gtc agc ctg 6624
Gln Leu Lys Val Cys Ala Thr Cys Phe Asp Leu Ser Val Ser Leu
    2195                2200                2205

ctg cgt gtc ttg gag atg act atc aca ctg gtg cct gag ata ttc 6669
Leu Arg Val Leu Glu Met Thr Ile Thr Leu Val Pro Glu Ile Phe
    2210                2215                2220

ctt gac tgg acc cgg cct acc tct gag atg ctg ctg cgg cgt ctt 6714
Leu Asp Trp Thr Arg Pro Thr Ser Glu Met Leu Leu Arg Arg Leu

2225                    2230                    2235

gca cag  ctg cta aac cag gtg  ctg aac cgg gtg aca  gct gag agg        6759
Ala Gln  Leu Leu Asn Gln Val  Leu Asn Arg Val Thr  Ala Glu Arg
    2240                    2245                    2250

aac ctg  ttt gat cgt gtg gtc  acc cta cgg ctg cct  ggc cta gag        6804
Asn Leu  Phe Asp Arg Val Val  Thr Leu Arg Leu Pro  Gly Leu Glu
    2255                    2260                    2265

agc gtg  gac cac tat ccc att  ctg gtg gca gtg acg  ggc atc ctg        6849
Ser Val  Asp His Tyr Pro Ile  Leu Val Ala Val Thr  Gly Ile Leu
    2270                    2275                    2280

gtg cag  ctc ctg gtg cgt ggc  cca gcc tca gag aga  gag caa gcc        6894
Val Gln  Leu Leu Val Arg Gly  Pro Ala Ser Glu Arg  Glu Gln Ala
    2285                    2290                    2295

aca tca  gtg ctc ctg gca gat  ccc tgc ttc cag cta  cgc tca ata        6939
Thr Ser  Val Leu Leu Ala Asp  Pro Cys Phe Gln Leu  Arg Ser Ile
    2300                    2305                    2310

tgc tat  ctc ctg gga cag cca  gag ccc cca gca cct  ggc act gct        6984
Cys Tyr  Leu Leu Gly Gln Pro  Glu Pro Pro Ala Pro  Gly Thr Ala
    2315                    2320                    2325

ctg cca  gcc cct gac cgg aag  cgc ttc tcc ctg cag  agc tat gcg        7029
Leu Pro  Ala Pro Asp Arg Lys  Arg Phe Ser Leu Gln  Ser Tyr Ala
    2330                    2335                    2340

gat tat  atc agt gcc gat gag  ctg gcc caa gtg gaa  cag atg ctg        7074
Asp Tyr  Ile Ser Ala Asp Glu  Leu Ala Gln Val Glu  Gln Met Leu
    2345                    2350                    2355

gcg cac  ctg acc tct gca tct  gcc cag gca gca gct  gcc tcc ctg        7119
Ala His  Leu Thr Ser Ala Ser  Ala Gln Ala Ala Ala  Ala Ser Leu
    2360                    2365                    2370

ccc acc  agt gag gag gac ctc  tgc ccc atc tgc tat  gcc cac ccc        7164
Pro Thr  Ser Glu Glu Asp Leu  Cys Pro Ile Cys Tyr  Ala His Pro
    2375                    2380                    2385

atc tct  gct gtg ttc cag ccc  tgt ggc cac aag tcc  tgc aaa gcc        7209
Ile Ser  Ala Val Phe Gln Pro  Cys Gly His Lys Ser  Cys Lys Ala
    2390                    2395                    2400

tgt atc  aac cag cac ctg atg  aac aac aag gac tgc  ttc ttc tgc        7254
Cys Ile  Asn Gln His Leu Met  Asn Asn Lys Asp Cys  Phe Phe Cys
    2405                    2410                    2415

aaa acc  acc atc gtg tct gta  gag gac tgg gag aag  gga gcc aat        7299
Lys Thr  Thr Ile Val Ser Val  Glu Asp Trp Glu Lys  Gly Ala Asn
    2420                    2425                    2430

acg agt  act acc tcc tca gct  gcc tag                                 7326
Thr Ser  Thr Thr Ser Ser Ala  Ala
    2435                    2440

<210>  18
<211>  2441
<212>  PRT
<213>  Homo sapiens

<400>  18

Met Ala Ser Asp Ser Pro Ala Arg Ser Leu Asp Glu Ile Asp Leu Ser
1               5                   10                  15

Ala Leu Arg Asp Pro Ala Gly Ile Phe Glu Leu Val Glu Leu Val Gly
            20                  25                  30

Asn Gly Thr Tyr Gly Gln Val Tyr Lys Gly Arg His Val Lys Thr Gly
        35                  40                  45

Gln Leu Ala Ala Ile Lys Val Met Asp Val Thr Gly Asp Glu Glu Glu
    50                  55                  60

Glu Ile Lys Gln Glu Ile Asn Met Leu Lys Lys Tyr Ser His His Arg
65                  70                  75                  80

Asn Ile Ala Thr Tyr Tyr Gly Ala Phe Ile Lys Lys Asn Pro Pro Gly
                85                  90                  95

Met Asp Asp Gln Leu Trp Leu Val Met Glu Phe Cys Gly Ala Gly Ser
            100                 105                 110

Val Thr Asp Leu Ile Lys Asn Thr Lys Gly Asn Thr Leu Lys Glu Glu
        115                 120                 125

Trp Ile Ala Tyr Ile Cys Arg Glu Ile Leu Arg Gly Leu Ser His Leu
    130                 135                 140

His Gln His Lys Val Ile His Arg Asp Ile Lys Gly Gln Asn Val Leu
145                 150                 155                 160

Leu Thr Glu Asn Ala Glu Val Lys Leu Val Asp Phe Gly Val Ser Ala
                165                 170                 175

Gln Leu Asp Arg Thr Val Gly Arg Arg Asn Thr Phe Ile Gly Thr Pro
            180                 185                 190

Tyr Trp Met Ala Pro Glu Val Ile Ala Cys Asp Glu Asn Pro Asp Ala
        195                 200                 205

Thr Tyr Asp Phe Lys Ser Asp Leu Trp Ser Leu Gly Ile Thr Ala Ile
    210                 215                 220

Glu Met Ala Glu Gly Ala Pro Pro Leu Cys Asp Met His Pro Met Arg
225                 230                 235                 240

Ala Leu Phe Leu Ile Pro Arg Asn Pro Ala Pro Arg Leu Lys Ser Lys
                245                 250                 255

Lys Trp Ser Lys Lys Phe Gln Ser Phe Ile Glu Ser Cys Leu Val Lys
            260                 265                 270

Asn His Ser Gln Arg Pro Ala Thr Glu Gln Leu Met Lys His Pro Phe
        275                 280                 285

Ile Arg Asp Gln Pro Asn Glu Arg Gln Val Arg Ile Gln Leu Lys Asp
    290                 295                 300

His Ile Asp Arg Thr Lys Lys Lys Arg Gly Glu Lys Asp Glu Thr Glu
305                 310                 315                 320

Tyr Glu Tyr Ser Gly Ser Glu Glu Glu Glu Glu Glu Asn Asp Ser Gly
                325                 330                 335

Glu Pro Ser Ser Ile Leu Asn Leu Pro Gly Glu Ser Thr Leu Arg Arg
            340                 345                 350

Asp Phe Leu Arg Leu Gln Leu Ala Asn Lys Glu Arg Ser Glu Ala Leu
        355                 360                 365

Arg Arg Gln Gln Leu Glu Gln Gln Gln Arg Glu Asn Glu Glu His Lys
    370                 375                 380

Arg Gln Leu Leu Ala Glu Arg Gln Lys Arg Ile Glu Glu Gln Lys Glu
385                 390                 395                 400

Gln Arg Arg Arg Leu Glu Glu Gln Gln Arg Arg Glu Lys Glu Leu Arg
                405                 410                 415

Lys Gln Gln Glu Arg Glu Gln Arg Arg His Tyr Glu Glu Gln Met Arg
            420                 425                 430

Arg Glu Glu Glu Arg Arg Arg Ala Glu His Glu Gln Glu Tyr Lys Arg
        435                 440                 445

Lys Gln Leu Glu Glu Gln Arg Gln Ala Glu Arg Leu Gln Arg Gln Leu
    450                 455                 460

Lys Gln Glu Arg Asp Tyr Leu Val Ser Leu Gln His Gln Arg Gln Glu
465                 470                 475                 480

Gln Arg Pro Val Glu Lys Lys Pro Leu Tyr His Tyr Lys Glu Gly Met
                485                 490                 495

Ser Pro Ser Glu Lys Pro Ala Trp Ala Lys Glu Ile Pro His Leu Val
        500                 505                 510

Ala Val Lys Ser Gln Gly Pro Ala Leu Thr Ala Ser Gln Ser Val His
    515                 520                 525

Glu Gln Pro Thr Lys Gly Leu Ser Gly Phe Gln Glu Ala Leu Asn Val
    530                 535                 540

Thr Ser His Arg Val Glu Met Pro Arg Gln Asn Ser Asp Pro Thr Ser
545                 550                 555                 560

Glu Asn Pro Pro Leu Pro Thr Arg Ile Glu Lys Phe Asp Arg Ser Ser
                565                 570                 575

Trp Leu Arg Gln Glu Glu Asp Ile Pro Pro Lys Val Pro Gln Arg Thr
            580                 585                 590

Thr Ser Ile Ser Pro Ala Leu Ala Arg Lys Asn Ser Pro Gly Asn Gly
        595                 600                 605

Ser Ala Leu Gly Pro Arg Leu Gly Ser Gln Pro Ile Arg Ala Ser Asn
    610                 615                 620

Pro Asp Leu Arg Arg Thr Glu Pro Ile Leu Glu Ser Pro Leu Gln Arg
625                 630                 635                 640

Thr Ser Ser Gly Ser Ser Ser Ser Ser Ser Thr Pro Ser Ser Gln Pro
                645                 650                 655

Ser Ser Gln Gly Gly Ser Gln Pro Gly Ser Gln Ala Gly Ser Ser Glu
            660                 665                 670

Arg Thr Arg Val Arg Ala Asn Ser Lys Ser Glu Gly Ser Pro Val Leu
        675                 680                 685

Pro His Glu Pro Ala Lys Val Lys Pro Glu Glu Ser Arg Asp Ile Thr
    690                 695                 700

Arg Pro Ser Arg Pro Ala Asp Leu Thr Ala Leu Ala Lys Glu Leu Arg
705                 710                 715                 720

Glu Leu Arg Ile Glu Glu Thr Asn Arg Pro Met Lys Lys Val Thr Asp
                725                 730                 735

Tyr Ser Ser Ser Ser Glu Glu Ser Glu Ser Ser Glu Glu Glu Glu Glu
            740                 745                 750

Asp Gly Glu Ser Glu Thr His Asp Gly Thr Val Ala Val Ser Asp Ile
        755                 760                 765

Pro Arg Leu Ile Pro Thr Gly Ala Pro Gly Ser Asn Glu Gln Tyr Asn
    770                 775                 780

Val Gly Met Val Gly Thr His Gly Leu Glu Thr Ser His Ala Asp Ser
785                 790                 795                 800

Phe Ser Gly Ser Ile Ser Arg Glu Gly Thr Leu Met Ile Arg Glu Thr
                805                 810                 815

Ser Gly Glu Lys Lys Arg Ser Gly His Ser Asp Ser Asn Gly Phe Ala
            820                 825                 830

Gly His Ile Asn Leu Pro Asp Leu Val Gln Gln Ser His Ser Pro Ala
        835                 840                 845

Gly Thr Pro Thr Glu Gly Leu Gly Arg Val Ser Thr His Ser Gln Glu
    850                 855                 860

Met Asp Ser Gly Thr Glu Tyr Gly Met Gly Ser Ser Thr Lys Ala Ser
865                 870                 875                 880

Phe Thr Pro Phe Val Asp Pro Arg Val Tyr Gln Thr Ser Pro Thr Asp
                885                 890                 895

Glu Asp Glu Glu Asp Glu Glu Ser Ser Ala Ala Ala Leu Phe Thr Ser
            900                 905                 910

Glu Leu Leu Arg Gln Glu Gln Ala Lys Leu Asn Glu Ala Arg Lys Ile
        915                 920                 925

Ser Val Val Asn Val Asn Pro Thr Asn Ile Arg Pro His Ser Asp Thr
    930                 935                 940

Pro Glu Ile Arg Lys Tyr Lys Lys Arg Phe Asn Ser Glu Ile Leu Cys
945                 950                 955                 960

Ala Ala Leu Trp Gly Val Asn Leu Leu Val Gly Thr Glu Asn Gly Leu
                965                 970                 975

Met Leu Leu Asp Arg Ser Gly Gln Gly Lys Val Tyr Asn Leu Ile Asn
            980                 985                 990

Arg Arg Arg Phe Gln Gln Met Asp  Val Leu Glu Gly Leu  Asn Val Leu

        995                 1000                 1005

Val Thr Ile Ser Gly Lys Lys Asn Lys Leu Arg Val Tyr Tyr Leu
    1010                1015                1020

Ser Trp Leu Arg Asn Arg Ile Leu His Asn Asp Pro Glu Val Glu
    1025                1030                1035

Lys Lys Gln Gly Trp Ile Thr Val Gly Asp Leu Glu Gly Cys Ile
    1040                1045                1050

His Tyr Lys Val Val Lys Tyr Glu Arg Ile Lys Phe Leu Val Ile
    1055                1060                1065

Ala Leu Lys Asn Ala Val Glu Ile Tyr Ala Trp Ala Pro Lys Pro
    1070                1075                1080

Tyr His Lys Phe Met Ala Phe Lys Ser Phe Ala Asp Leu Gln His
    1085                1090                1095

Lys Pro Leu Leu Val Asp Leu Thr Val Glu Glu Gly Gln Arg Leu
    1100                1105                1110

Lys Val Ile Phe Gly Ser His Thr Gly Phe His Val Ile Asp Val
    1115                1120                1125

Asp Ser Gly Asn Ser Tyr Asp Ile Tyr Ile Pro Ser His Ile Gln
    1130                1135                1140

Gly Asn Ile Thr Pro His Ala Ile Val Ile Leu Pro Lys Thr Asp
    1145                1150                1155

Gly Met Glu Met Leu Val Cys Tyr Glu Asp Glu Gly Val Tyr Val
    1160                1165                1170

Asn Thr Tyr Gly Arg Ile Thr Lys Asp Val Val Leu Gln Trp Gly
    1175                1180                1185

Glu Met Pro Thr Ser Val Ala Tyr Ile His Ser Asn Gln Ile Met
    1190                1195                1200

Gly Trp Gly Glu Lys Ala Ile Glu Ile Arg Ser Val Glu Thr Gly
    1205                1210                1215

His Leu Asp Gly Val Phe Met His Lys Arg Ala Gln Arg Leu Lys
    1220                1225                1230

Phe Leu Cys Glu Arg Asn Asp Lys Val Ile Gly His Ser Asn Phe
    1235                1240                1245

Gly Thr Ile Arg Ser Thr Thr Cys Val Tyr Lys Gly Lys Trp Leu
    1250                1255                1260

Tyr Glu Val Leu Ile Ser Ser Gln Gly Leu Met Gln Ile Gly Trp
    1265                1270                1275

Cys Thr Ile Ser Cys Arg Phe Asn Gln Glu Glu Gly Val Gly Asp
    1280                1285                1290

Thr His Asn Ser Tyr Ala Tyr Asp Gly Asn Arg Val Arg Lys Trp
    1295                1300                1305

Asn Val Thr Thr Thr Asn Tyr Gly Lys Ala Trp Ala Ala Gly Asp
    1310                1315                1320

Ile Val Ser Cys Leu Ile Asp Leu Asp Asp Gly Thr Leu Ser Phe
    1325                1330                1335

Cys Leu Asn Gly Val Ser Leu Gly Thr Ala Phe Glu Asn Leu Ser
    1340                1345                1350

Arg Gly Leu Gly Met Ala Tyr Phe Pro Ala Ile Ser Leu Ser Phe
    1355                1360                1365

Lys Glu Ser Val Ala Phe Asn Phe Gly Ser Arg Pro Leu Arg Tyr
    1370                1375                1380

Pro Val Ala Gly Tyr Arg Pro Leu Gln Asp Pro Pro Ser Ala Asp
    1385                1390                1395

Leu Val Arg Ala Gln Arg Leu Leu Gly Cys Phe Arg Ala Val Leu
    1400                1405                1410

Ser Val Glu Leu Asp Pro Val Glu Gly Arg Leu Leu Asp Lys Glu
    1415                1420                1425

Ser Ser Lys Trp Arg Leu Arg Gly Gln Pro Thr Val Leu Leu Thr
    1430                1435                1440

Leu Ala His Ile Phe His His Phe Ala Pro Leu Leu Arg Lys Val
    1445                1450                1455

Tyr Leu Val Glu Ala Val Leu Met Ser Phe Leu Leu Gly Ile Val
    1460                1465                1470

Glu Lys Gly Thr Pro Thr Gln Ala Gln Ser Val Val His Gln Val
    1475                1480                1485

Leu Asp Leu Leu Trp Leu Phe Met Glu Asp Tyr Glu Val Gln Asp
    1490                1495                1500

Cys Leu Lys Gln Leu Met Met Ser Leu Leu Arg Leu Tyr Arg Phe
    1505                1510                1515

Ser Pro Ile Val Pro Asp Leu Gly Leu Gln Ile His Tyr Leu Arg
    1520                1525                1530

Leu Thr Ile Ala Ile Leu Arg His Glu Lys Ser Arg Lys Phe Leu
    1535                1540                1545

Leu Ser Asn Val Leu Phe Asp Val Leu Arg Ser Val Val Phe Phe
    1550                1555                1560

Tyr Ile Lys Ser Pro Leu Arg Val Glu Glu Ala Gly Leu Gln Glu
    1565                1570                1575

Leu Ile Pro Thr Thr Trp Trp Pro His Cys Ser Ser Arg Glu Gly
    1580                1585                1590

Lys Glu Ser Thr Glu Met Lys Glu Glu Thr Ala Glu Glu Arg Leu
    1595                1600                1605

Arg Arg Arg Ala Tyr Glu Arg Gly Cys Gln Arg Leu Arg Lys Arg
    1610                1615                1620

Ile Glu Val Val Glu Glu Leu Gln Val Gln Ile Leu Lys Leu Leu
    1625                1630                1635

Leu Asp Asn Lys Asp Asp Asn Gly Gly Glu Ala Ser Arg Tyr Ile
    1640                1645                1650

Phe Leu Thr Lys Phe Arg Lys Phe Leu Gln Glu Asn Ala Ser Gly
    1655                1660                1665

Arg Gly Asn Met Pro Met Leu Cys Pro Pro Glu Tyr Met Val Cys
    1670                1675                1680

Phe Leu His Arg Leu Ile Ser Ala Leu Arg Tyr Tyr Trp Asp Glu
    1685                1690                1695

Tyr Lys Ala Ser Asn Pro His Ala Ser Phe Ser Glu Glu Ala Tyr
    1700                1705                1710

Ile Pro Pro Gln Val Phe Tyr Asn Gly Lys Val Asp Tyr Phe Asp
    1715                1720                1725

Leu Gln Arg Leu Gly Gly Leu Leu Ser His Leu Arg Lys Thr Leu
    1730                1735                1740

Lys Asp Asp Leu Ala Ser Lys Ala Asn Ile Val Ile Asp Pro Leu
    1745                1750                1755

Glu Leu Gln Ser Thr Ala Met Asp Asp Leu Asp Glu Asp Glu Glu
    1760                1765                1770

Pro Ala Pro Ala Met Ala Gln Arg Pro Met Gln Ala Leu Ala Val
    1775                1780                1785

Gly Gly Pro Leu Pro Leu Pro Arg Pro Gly Trp Leu Ser Ser Pro
    1790                1795                1800

Thr Leu Gly Arg Ala Asn Arg Phe Leu Ser Thr Ala Ala Val Ser
    1805                1810                1815

Leu Met Thr Pro Arg Arg Pro Leu Ser Thr Ser Glu Lys Val Lys
    1820                1825                1830

Val Arg Thr Leu Ser Val Glu Gln Arg Thr Arg Glu Asp Ile Glu
    1835                1840                1845

Gly Ser His Trp Asn Glu Gly Leu Leu Leu Gly Arg Pro Pro Glu
    1850                1855                1860

Glu Pro Glu Gln Pro Leu Thr Glu Asn Ser Leu Leu Glu Val Leu
    1865                1870                1875

Asp Gly Ala Val Met Met Tyr Asn Leu Ser Val His Gln Gln Leu
    1880                1885                1890

Gly Lys Met Val Gly Val Ser Asp Asp Val Asn Glu Tyr Ala Met
    1895                1900                1905

Ala Leu Arg Asp Thr Glu Asp Lys Leu Arg Arg Cys Pro Lys Arg
    1910                1915                1920

Arg Lys Asp Ile Leu Ala Glu Leu Thr Lys Ser Gln Lys Val Phe
    1925                1930                1935

Ser Glu Lys Leu Asp His Leu Ser Arg Arg Leu Ala Trp Val His

1940                1945                1950

Ala Thr Val Tyr Ser Gln Glu Lys Met Leu Asp Ile Tyr Trp Leu
    1955                1960                1965

Leu Arg Val Cys Leu Arg Thr Ile Glu His Gly Asp Arg Thr Gly
    1970                1975                1980

Ser Leu Phe Ala Phe Met Pro Glu Phe Tyr Leu Ser Val Ala Ile
    1985                1990                1995

Asn Ser Tyr Ser Ala Leu Lys Asn Tyr Phe Gly Pro Val His Ser
    2000                2005                2010

Met Glu Glu Leu Pro Gly Tyr Glu Glu Thr Leu Thr Arg Leu Ala
    2015                2020                2025

Ala Ile Leu Ala Lys His Phe Ala Asp Ala Arg Ile Val Gly Thr
    2030                2035                2040

Asp Ile Arg Asp Ser Leu Met Gln Ala Leu Ala Ser Tyr Val Cys
    2045                2050                2055

Tyr Pro His Ser Leu Arg Ala Val Glu Arg Ile Pro Glu Glu Gln
    2060                2065                2070

Arg Ile Ala Met Val Arg Asn Leu Leu Ala Pro Tyr Glu Gln Arg
    2075                2080                2085

Pro Trp Ala Gln Thr Asn Trp Ile Leu Val Arg Leu Trp Arg Gly
    2090                2095                2100

Cys Gly Phe Gly Tyr Arg Tyr Thr Arg Leu Pro His Leu Leu Lys
    2105                2110                2115

Thr Lys Leu Glu Asp Ala Asn Leu Pro Ser Leu Gln Lys Pro Cys
    2120                2125                2130

Pro Ser Thr Leu Leu Gln Gln His Met Ala Asp Leu Leu Gln Gln
    2135                2140                2145

Gly Pro Asp Val Ala Pro Ser Phe Leu Asn Ser Val Leu Asn Gln
    2150                2155                2160

Leu Asn Trp Ala Phe Ser Glu Phe Ile Gly Met Ile Gln Glu Ile
    2165                2170                2175

Gln Gln Ala Ala Glu Arg Leu Glu Arg Asn Phe Val Asp Ser Arg
    2180                2185                2190

Gln Leu Lys Val Cys Ala Thr Cys Phe Asp Leu Ser Val Ser Leu
    2195                2200                2205

Leu Arg Val Leu Glu Met Thr Ile Thr Leu Val Pro Glu Ile Phe
    2210                2215                2220

Leu Asp Trp Thr Arg Pro Thr Ser Glu Met Leu Leu Arg Arg Leu
    2225                2230                2235

Ala Gln Leu Leu Asn Gln Val Leu Asn Arg Val Thr Ala Glu Arg
    2240                2245                2250

Asn Leu Phe Asp Arg Val Val Thr Leu Arg Leu Pro Gly Leu Glu
    2255                2260                2265

Ser Val Asp His Tyr Pro Ile Leu Val Ala Val Thr Gly Ile Leu
    2270                2275                2280

Val Gln Leu Leu Val Arg Gly Pro Ala Ser Glu Arg Glu Gln Ala
    2285                2290                2295

Thr Ser Val Leu Leu Ala Asp Pro Cys Phe Gln Leu Arg Ser Ile
    2300                2305                2310

Cys Tyr Leu Leu Gly Gln Pro Glu Pro Pro Ala Pro Gly Thr Ala
    2315                2320                2325

Leu Pro Ala Pro Asp Arg Lys Arg Phe Ser Leu Gln Ser Tyr Ala
    2330                2335                2340

Asp Tyr Ile Ser Ala Asp Glu Leu Ala Gln Val Glu Gln Met Leu
    2345                2350                2355

Ala His Leu Thr Ser Ala Ser Ala Gln Ala Ala Ala Ala Ser Leu
    2360                2365                2370

Pro Thr Ser Glu Glu Asp Leu Cys Pro Ile Cys Tyr Ala His Pro
    2375                2380                2385

Ile Ser Ala Val Phe Gln Pro Cys Gly His Lys Ser Cys Lys Ala
    2390                2395                2400

Cys Ile Asn Gln His Leu Met Asn Asn Lys Asp Cys Phe Phe Cys
    2405                2410                2415

Lys Thr Thr Ile Val Ser Val Glu Asp Trp Glu Lys Gly Ala Asn
    2420                2425                2430

Thr Ser Thr Thr Ser Ser Ala Ala
    2435                2440

<210> 19
<211> 2901
<212> DNA
<213> Homo sapiens

<220>
<221> CDS
<222> (1)..(2901)

<400> 19
atg gag ccg cgg ccc acg gcg ccc tcc tcc ggc gcc ccg gga ctg gcc      48
Met Glu Pro Arg Pro Thr Ala Pro Ser Ser Gly Ala Pro Gly Leu Ala
1               5                   10                  15

ggg gtc ggg gag acg ccg tca gcc gct gcg ctg gcc gca gcc agg gtg      96
Gly Val Gly Glu Thr Pro Ser Ala Ala Ala Leu Ala Ala Ala Arg Val
            20                  25                  30

gaa ctg ccc ggc acg gct gtg ccc tcg gtg ccg gag gat gct gcg ccc      144
Glu Leu Pro Gly Thr Ala Val Pro Ser Val Pro Glu Asp Ala Ala Pro
        35                  40                  45

gcg agc cgg gac ggc ggc ggg gtc cgc gat gag ggc ccc gcg gcg gcc      192
Ala Ser Arg Asp Gly Gly Gly Val Arg Asp Glu Gly Pro Ala Ala Ala
    50                  55                  60

ggg gac ggg ctg ggc aga ccc ttg ggg ccc acc ccg agc cag agc cgt      240
Gly Asp Gly Leu Gly Arg Pro Leu Gly Pro Thr Pro Ser Gln Ser Arg
65                  70                  75                  80

ttc cag gtg gac ctg gtt tcc gag aac gcc ggg cgg gcc gct gct gcg      288
Phe Gln Val Asp Leu Val Ser Glu Asn Ala Gly Arg Ala Ala Ala Ala
                85                  90                  95

gcg gcg gcg gcg gcg gcg gca gcg gcg gcg gct ggt gct ggg gcg ggg      336
Ala Ala Ala Ala Ala Ala Ala Ala Ala Ala Ala Gly Ala Gly Ala Gly
            100                 105                 110

gcc aag cag acc ccc gcg gac ggg gaa gcc agc ggc gag agc gag ccg      384
Ala Lys Gln Thr Pro Ala Asp Gly Glu Ala Ser Gly Glu Ser Glu Pro
        115                 120                 125

gct aaa ggc agc gag gaa gcc aag ggc cgc ttc cgc gtg aac ttc gtg      432
Ala Lys Gly Ser Glu Glu Ala Lys Gly Arg Phe Arg Val Asn Phe Val
    130                 135                 140

gac cca gct gcc tcc tcg tcg gct gaa gac agc ctg tca gat gct gcc      480
Asp Pro Ala Ala Ser Ser Ser Ala Glu Asp Ser Leu Ser Asp Ala Ala
145                 150                 155                 160

ggg gtc gga gtc gac ggg ccc aac gtg agc ttc cag aac ggc ggg gac      528
Gly Val Gly Val Asp Gly Pro Asn Val Ser Phe Gln Asn Gly Gly Asp
                165                 170                 175

acg gtg ctg agc gag ggc agc agc ctg cac tcc ggc ggc ggc ggc 576
Thr Val Leu Ser Glu Gly Ser Ser Leu His Ser Gly Gly Gly Gly
            180                 185                 190

agt ggg cac cac cag cac tac tat tat gat acc cac acc aac acc tac 624
Ser Gly His His Gln His Tyr Tyr Tyr Asp Thr His Thr Asn Thr Tyr
            195                 200                 205

tac ctg cgc acc ttc ggc cac aac acc atg gac gct gtg ccc agg atc 672
Tyr Leu Arg Thr Phe Gly His Asn Thr Met Asp Ala Val Pro Arg Ile
        210                 215                 220

gat cac tac cgg cac aca gcc gcg cag ctg ggc gag aag ctg ctc cgg 720
Asp His Tyr Arg His Thr Ala Ala Gln Leu Gly Glu Lys Leu Leu Arg
225 230 235 240

cct agc ctg gcg gag ctc cac gac gag ctg gaa aag gaa cct ttt gag 768
Pro Ser Leu Ala Glu Leu His Asp Glu Leu Glu Lys Glu Pro Phe Glu
245 250 255

gat ggc ttt gca aat ggg gaa gaa agt act cca acc aga gat gct gtg 816
Asp Gly Phe Ala Asn Gly Glu Glu Ser Thr Pro Thr Arg Asp Ala Val
                260                 265                 270

gtc acg tat act gca gaa agt aaa gga gtc gtg aag ttt ggc tgg atc 864
Val Thr Tyr Thr Ala Glu Ser Lys Gly Val Val Lys Phe Gly Trp Ile
            275                 280                 285

aag ggt gta tta gta cgt tgt atg tta aac att tgg ggt gtg atg ctt 912
Lys Gly Val Leu Val Arg Cys Met Leu Asn Ile Trp Gly Val Met Leu
        290                 295                 300

ttc att aga ttg tca tgg att gtg ggt caa gct gga ata ggt cta tca 960
Phe Ile Arg Leu Ser Trp Ile Val Gly Gln Ala Gly Ile Gly Leu Ser
305 310 315 320

gtc ctt gta ata atg atg gcc act gtt gtg aca act atc aca gga ttg 1008
Val Leu Val Ile Met Met Ala Thr Val Val Thr Thr Ile Thr Gly Leu
325 330 335

tct act tca gca ata gca act aat gga ttt gta aga gga gcc acc cgg 1056
Ser Thr Ser Ala Ile Ala Thr Asn Gly Phe Val Arg Gly Ala Thr Arg
                340                 345                 350

ccc aag ttg aag aag atg aag agc cag acg gga cag gtg ggt gag aag 1104
Pro Lys Leu Lys Lys Met Lys Ser Gln Thr Gly Gln Val Gly Glu Lys
            355                 360                 365

caa tcg ctg aag tgt gag gca gca gcc ggt aat ccc cag cct tcc tac 1152
Gln Ser Leu Lys Cys Glu Ala Ala Ala Gly Asn Pro Gln Pro Ser Tyr
        370                 375                 380

cgt tgg ttc aag gat ggc aag gag ctc aac cgc agc cga gac att cgc 1200
Arg Trp Phe Lys Asp Gly Lys Glu Leu Asn Arg Ser Arg Asp Ile Arg
385 390 395 400

atc aaa tat ggc aac ggc aga aag aac tca cga cta cag ttc aac aag 1248
Ile Lys Tyr Gly Asn Gly Arg Lys Asn Ser Arg Leu Gln Phe Asn Lys
405 410 415

gtg aag gtg gag gac gct ggg gag tat gtc tgc gag gcc gag aac atc 1296
Val Lys Val Glu Asp Ala Gly Glu Tyr Val Cys Glu Ala Glu Asn Ile

420                 425                 430

ctg ggg aag gac acc gtc cgg ggc cgg ctt tac gtc aac agc gtg agc      1344
Leu Gly Lys Asp Thr Val Arg Gly Arg Leu Tyr Val Asn Ser Val Ser
        435                 440                 445

acc acc ctg tca tcc tgg tcg ggg cac gcc cgg aag tgc aac gag aca      1392
Thr Thr Leu Ser Ser Trp Ser Gly His Ala Arg Lys Cys Asn Glu Thr
    450                 455                 460

gcc aag tcc tat tgc gtc aat gga ggc gtc tgc tac tac atc gag ggc      1440
Ala Lys Ser Tyr Cys Val Asn Gly Gly Val Cys Tyr Tyr Ile Glu Gly
465                 470                 475                 480

atc aac cag ctc tcc tgc aaa tgt cca aat gga ttc ttc gga cag aga      1488
Ile Asn Gln Leu Ser Cys Lys Cys Pro Asn Gly Phe Phe Gly Gln Arg
                485                 490                 495

tgt ttg gag aaa ctg cct ttg cga ttg tac atg cca gat cct aag caa      1536
Cys Leu Glu Lys Leu Pro Leu Arg Leu Tyr Met Pro Asp Pro Lys Gln
            500                 505                 510

aaa gcc gag gag ctg tac cag aag agg gtc ctg acc atc acg ggc atc      1584
Lys Ala Glu Glu Leu Tyr Gln Lys Arg Val Leu Thr Ile Thr Gly Ile
        515                 520                 525

tgc gtg gct ctg ctg gtc gtg ggc atc gtc tgt gtg gtg gcc tac tgc      1632
Cys Val Ala Leu Leu Val Val Gly Ile Val Cys Val Val Ala Tyr Cys
    530                 535                 540

aag acc aaa aaa cag cgg aag cag atg cac aac cac ctc cgg cag aac      1680
Lys Thr Lys Lys Gln Arg Lys Gln Met His Asn His Leu Arg Gln Asn
545                 550                 555                 560

atg tgc ccg gcc cat cag aac cgg agc ttg gcc aat ggg ccc agc cac      1728
Met Cys Pro Ala His Gln Asn Arg Ser Leu Ala Asn Gly Pro Ser His
                565                 570                 575

ccc cgg ctg gac cca gag gag atc cag atg gca gat tat att tcc aag      1776
Pro Arg Leu Asp Pro Glu Glu Ile Gln Met Ala Asp Tyr Ile Ser Lys
            580                 585                 590

aac gtg cca gcc aca gac cat gtc atc agg aga gaa act gag acc acc      1824
Asn Val Pro Ala Thr Asp His Val Ile Arg Arg Glu Thr Glu Thr Thr
        595                 600                 605

ttc tct ggg agc cac tcc tgt tct cct tct cac cac tgc tcc aca gcc      1872
Phe Ser Gly Ser His Ser Cys Ser Pro Ser His His Cys Ser Thr Ala
    610                 615                 620

aca ccc acc tcc agc cac aga cac gag agc cac acg tgg agc ctg gaa      1920
Thr Pro Thr Ser Ser His Arg His Glu Ser His Thr Trp Ser Leu Glu
625                 630                 635                 640

cgt tct gag agc ctg act tct gac tcc cag tcg ggg atc atg cta tca      1968
Arg Ser Glu Ser Leu Thr Ser Asp Ser Gln Ser Gly Ile Met Leu Ser
                645                 650                 655

tca gtg ggt acc agc aaa tgc aac agc cca gca tgt gtg gag gcc cgg      2016
Ser Val Gly Thr Ser Lys Cys Asn Ser Pro Ala Cys Val Glu Ala Arg
            660                 665                 670

gca agg cgg gca gca gcc tac aac ctg gag gag cgg cgc agg gcc acc      2064

Ala Arg Arg Ala Ala Ala Tyr Asn Leu Glu Glu Arg Arg Arg Ala Thr
        675                 680                 685

gcg cca ccc tat cac gat tcc gtg gac tcc ctt cgc gac tcc cca cac        2112
Ala Pro Pro Tyr His Asp Ser Val Asp Ser Leu Arg Asp Ser Pro His
    690                 695                 700

agc gag agg tac gtg tcg gcc ctg acc acg ccc gcg cgc ctc tcg ccc        2160
Ser Glu Arg Tyr Val Ser Ala Leu Thr Thr Pro Ala Arg Leu Ser Pro
705                 710                 715                 720

gtg gac ttc cac tac tcg ctg gcc acg cag gtg cca act ttc gag atc        2208
Val Asp Phe His Tyr Ser Leu Ala Thr Gln Val Pro Thr Phe Glu Ile
                725                 730                 735

acg tcc ccc aac tcg gcg cac gcc gtg tcg ctg ccg ccg gcg gcg ccc        2256
Thr Ser Pro Asn Ser Ala His Ala Val Ser Leu Pro Pro Ala Ala Pro
            740                 745                 750

atc agt tac cgc ctg gcc gag cag cag ccg tta ctg cgg cac ccg gcg        2304
Ile Ser Tyr Arg Leu Ala Glu Gln Gln Pro Leu Leu Arg His Pro Ala
        755                 760                 765

ccc ccc ggc ccg gga ccc gga ccc ggg ccc ggg ccc ggg ccc ggc gca        2352
Pro Pro Gly Pro Gly Pro Gly Pro Gly Pro Gly Pro Gly Pro Gly Ala
    770                 775                 780

gac atg cag cgc agc tat gac agc tac tat tac ccc gcg gcg ggg ccc        2400
Asp Met Gln Arg Ser Tyr Asp Ser Tyr Tyr Tyr Pro Ala Ala Gly Pro
785                 790                 795                 800

gga ccg cgg cgc ggg acc tgc gcg ctc ggc ggc agc ctg ggc agc ctg        2448
Gly Pro Arg Arg Gly Thr Cys Ala Leu Gly Gly Ser Leu Gly Ser Leu
                805                 810                 815

cct gcc agc ccc ttc cgc atc ccc gag gac gac gag tac gag acc acg        2496
Pro Ala Ser Pro Phe Arg Ile Pro Glu Asp Asp Glu Tyr Glu Thr Thr
            820                 825                 830

cag gag tgc gcg ccc ccg ccg ccg ccg cgg ccg cgc gcg cgc ggt gcg        2544
Gln Glu Cys Ala Pro Pro Pro Pro Pro Arg Pro Arg Ala Arg Gly Ala
        835                 840                 845

tcc cgc agg acg tcg gcg ggg ccc cgg cgc tgg cgc cgc tcg cgc ctc        2592
Ser Arg Arg Thr Ser Ala Gly Pro Arg Arg Trp Arg Arg Ser Arg Leu
    850                 855                 860

aac ggg ctg gcg gcg cag cgc gca cgg gcg gcg agg gac tcg ctg tcg        2640
Asn Gly Leu Ala Ala Gln Arg Ala Arg Ala Ala Arg Asp Ser Leu Ser
865                 870                 875                 880

ctg agc agc ggc tcg ggc ggc ggc tca gcc tcg gcg tcg gac gac gac        2688
Leu Ser Ser Gly Ser Gly Gly Gly Ser Ala Ser Ala Ser Asp Asp Asp
                885                 890                 895

gcg gac gac gcg gac ggg gcg ctg gcg gcc gag agc aca cct ttc ctg        2736
Ala Asp Asp Ala Asp Gly Ala Leu Ala Ala Glu Ser Thr Pro Phe Leu
            900                 905                 910

ggc ctg cgt ggg gcg cac gac gcg ctg cgc tcg gac tcg ccg cca ctg        2784
Gly Leu Arg Gly Ala His Asp Ala Leu Arg Ser Asp Ser Pro Pro Leu
        915                 920                 925

tgc ccg gcg gcc gac agc agg act tac tac tca ctg gac agc cac agc     2832
Cys Pro Ala Ala Asp Ser Arg Thr Tyr Tyr Ser Leu Asp Ser His Ser
    930                 935                 940

acg cgg gcc agc agc aga cac agc cgc ggg ccg ccc ccg cgg gcc aag     2880
Thr Arg Ala Ser Ser Arg His Ser Arg Gly Pro Pro Pro Arg Ala Lys
945                 950                 955                 960

cag gac tcg gcg cca ctc tag                                         2901
Gln Asp Ser Ala Pro Leu
                965

<210>  20
<211>  966
<212>  PRT
<213>  Homo sapiens

<400>  20

Met Glu Pro Arg Pro Thr Ala Pro Ser Ser Gly Ala Pro Gly Leu Ala
1               5                   10                  15

Gly Val Gly Glu Thr Pro Ser Ala Ala Ala Leu Ala Ala Ala Arg Val
            20                  25                  30

Glu Leu Pro Gly Thr Ala Val Pro Ser Val Pro Glu Asp Ala Ala Pro
        35                  40                  45

Ala Ser Arg Asp Gly Gly Gly Val Arg Asp Glu Gly Pro Ala Ala Ala
    50                  55                  60

Gly Asp Gly Leu Gly Arg Pro Leu Gly Pro Thr Pro Ser Gln Ser Arg
65                  70                  75                  80

Phe Gln Val Asp Leu Val Ser Glu Asn Ala Gly Arg Ala Ala Ala Ala
                85                  90                  95

Ala Ala Ala Ala Ala Ala Ala Ala Ala Ala Ala Gly Ala Gly Ala Gly
            100                 105                 110

Ala Lys Gln Thr Pro Ala Asp Gly Glu Ala Ser Gly Glu Ser Glu Pro
        115                 120                 125

Ala Lys Gly Ser Glu Glu Ala Lys Gly Arg Phe Arg Val Asn Phe Val
    130                 135                 140

Asp Pro Ala Ala Ser Ser Ser Ala Glu Asp Ser Leu Ser Asp Ala Ala
145                 150                 155                 160

Gly Val Gly Val Asp Gly Pro Asn Val Ser Phe Gln Asn Gly Gly Asp
                165                 170                 175

Thr Val Leu Ser Glu Gly Ser Ser Leu His Ser Gly Gly Gly Gly Gly
            180                 185                 190

Ser Gly His His Gln His Tyr Tyr Tyr Asp Thr His Thr Asn Thr Tyr
        195                 200                 205

Tyr Leu Arg Thr Phe Gly His Asn Thr Met Asp Ala Val Pro Arg Ile
    210                 215                 220

Asp His Tyr Arg His Thr Ala Ala Gln Leu Gly Glu Lys Leu Leu Arg
225                 230                 235                 240

Pro Ser Leu Ala Glu Leu His Asp Glu Leu Glu Lys Glu Pro Phe Glu
                245                 250                 255

Asp Gly Phe Ala Asn Gly Glu Glu Ser Thr Pro Thr Arg Asp Ala Val
            260                 265                 270

Val Thr Tyr Thr Ala Glu Ser Lys Gly Val Val Lys Phe Gly Trp Ile
        275                 280                 285

Lys Gly Val Leu Val Arg Cys Met Leu Asn Ile Trp Gly Val Met Leu
    290                 295                 300

Phe Ile Arg Leu Ser Trp Ile Val Gly Gln Ala Gly Ile Gly Leu Ser
305                 310                 315                 320

Val Leu Val Ile Met Met Ala Thr Val Val Thr Thr Ile Thr Gly Leu
                325                 330                 335

Ser Thr Ser Ala Ile Ala Thr Asn Gly Phe Val Arg Gly Ala Thr Arg
            340                 345                 350

Pro Lys Leu Lys Lys Met Lys Ser Gln Thr Gly Gln Val Gly Glu Lys
        355                 360                 365

Gln Ser Leu Lys Cys Glu Ala Ala Ala Gly Asn Pro Gln Pro Ser Tyr
    370                 375                 380

Arg Trp Phe Lys Asp Gly Lys Glu Leu Asn Arg Ser Arg Asp Ile Arg
385                 390                 395                 400

Ile Lys Tyr Gly Asn Gly Arg Lys Asn Ser Arg Leu Gln Phe Asn Lys
                405                 410                 415

Val Lys Val Glu Asp Ala Gly Glu Tyr Val Cys Glu Ala Glu Asn Ile
            420                 425                 430

Leu Gly Lys Asp Thr Val Arg Gly Arg Leu Tyr Val Asn Ser Val Ser
        435                 440                 445

Thr Thr Leu Ser Ser Trp Ser Gly His Ala Arg Lys Cys Asn Glu Thr
    450                 455                 460

Ala Lys Ser Tyr Cys Val Asn Gly Gly Val Cys Tyr Tyr Ile Glu Gly
465                 470                 475                 480

Ile Asn Gln Leu Ser Cys Lys Cys Pro Asn Gly Phe Phe Gly Gln Arg
                485                 490                 495

Cys Leu Glu Lys Leu Pro Leu Arg Leu Tyr Met Pro Asp Pro Lys Gln
            500                 505                 510

Lys Ala Glu Glu Leu Tyr Gln Lys Arg Val Leu Thr Ile Thr Gly Ile
        515                 520                 525

Cys Val Ala Leu Leu Val Val Gly Ile Val Cys Val Val Ala Tyr Cys
    530                 535                 540

Lys Thr Lys Lys Gln Arg Lys Gln Met His Asn His Leu Arg Gln Asn
545                 550                 555                 560

Met Cys Pro Ala His Gln Asn Arg Ser Leu Ala Asn Gly Pro Ser His
                565                 570                 575

Pro Arg Leu Asp Pro Glu Glu Ile Gln Met Ala Asp Tyr Ile Ser Lys
            580                 585                 590

Asn Val Pro Ala Thr Asp His Val Ile Arg Arg Glu Thr Glu Thr Thr
        595                 600                 605

Phe Ser Gly Ser His Ser Cys Ser Pro Ser His His Cys Ser Thr Ala
    610                 615                 620

Thr Pro Thr Ser Ser His Arg His Glu Ser His Thr Trp Ser Leu Glu
625                 630                 635                 640

Arg Ser Glu Ser Leu Thr Ser Asp Ser Gln Ser Gly Ile Met Leu Ser
                645                 650                 655

Ser Val Gly Thr Ser Lys Cys Asn Ser Pro Ala Cys Val Glu Ala Arg
            660                 665                 670

Ala Arg Arg Ala Ala Ala Tyr Asn Leu Glu Glu Arg Arg Arg Ala Thr

        675                 680                 685

Ala Pro Pro Tyr His Asp Ser Val Asp Ser Leu Arg Asp Ser Pro His
    690                 695                 700

Ser Glu Arg Tyr Val Ser Ala Leu Thr Thr Pro Ala Arg Leu Ser Pro
705                 710                 715                 720

Val Asp Phe His Tyr Ser Leu Ala Thr Gln Val Pro Thr Phe Glu Ile
                725                 730                 735

Thr Ser Pro Asn Ser Ala His Ala Val Ser Leu Pro Pro Ala Ala Pro
            740                 745                 750

Ile Ser Tyr Arg Leu Ala Glu Gln Gln Pro Leu Leu Arg His Pro Ala
        755                 760                 765

Pro Pro Gly Pro Gly Pro Gly Pro Gly Pro Gly Pro Gly Pro Gly Ala
    770                 775                 780

Asp Met Gln Arg Ser Tyr Asp Ser Tyr Tyr Tyr Pro Ala Ala Gly Pro
785                 790                 795                 800

Gly Pro Arg Arg Gly Thr Cys Ala Leu Gly Gly Ser Leu Gly Ser Leu
                805                 810                 815

Pro Ala Ser Pro Phe Arg Ile Pro Glu Asp Asp Glu Tyr Glu Thr Thr
            820                 825                 830

Gln Glu Cys Ala Pro Pro Pro Pro Pro Arg Pro Arg Ala Arg Gly Ala
        835                 840                 845

Ser Arg Arg Thr Ser Ala Gly Pro Arg Arg Trp Arg Arg Ser Arg Leu
    850                 855                 860

Asn Gly Leu Ala Ala Gln Arg Ala Arg Ala Ala Arg Asp Ser Leu Ser
865                 870                 875                 880

Leu Ser Ser Gly Ser Gly Gly Gly Ser Ala Ser Ala Ser Asp Asp Asp
                885                 890                 895

Ala Asp Asp Ala Asp Gly Ala Leu Ala Ala Glu Ser Thr Pro Phe Leu
            900                 905                 910

Gly Leu Arg Gly Ala His Asp Ala Leu Arg Ser Asp Ser Pro Pro Leu
        915                 920                 925

Cys Pro Ala Ala Asp Ser Arg Thr Tyr Tyr Ser Leu Asp Ser His Ser
    930                 935                 940

Thr Arg Ala Ser Ser Arg His Ser Arg Gly Pro Pro Pro Arg Ala Lys
945                 950                 955                 960

Gln Asp Ser Ala Pro Leu
                965

<210>  21
<211>  2883
<212>  DNA
<213>  Homo sapiens

<220>
<221>  CDS
<222>  (1)..(2883)

<400>  21
atg gag ccg cgg ccc acg gcg ccc tcc tcc ggc gcc ccg gga ctg gcc      48
Met Glu Pro Arg Pro Thr Ala Pro Ser Ser Gly Ala Pro Gly Leu Ala
1               5                   10                  15

ggg gtc ggg gag acg ccg tca gcc gct gcg ctg gcc gca gcc agg gtg      96
Gly Val Gly Glu Thr Pro Ser Ala Ala Ala Leu Ala Ala Ala Arg Val
            20                  25                  30

gaa ctg ccc ggc acg gct gtg ccc tcg gtg ccg gag gat gct gcg ccc      144
Glu Leu Pro Gly Thr Ala Val Pro Ser Val Pro Glu Asp Ala Ala Pro
        35                  40                  45

gcg agc cgg gac ggc ggc ggg gtc cgc gat gag ggc ccc gcg gcg gcc      192
Ala Ser Arg Asp Gly Gly Gly Val Arg Asp Glu Gly Pro Ala Ala Ala
    50                  55                  60

ggg gac ggg ctg ggc aga ccc ttg ggg ccc acc ccg agc cag agc cgt      240
Gly Asp Gly Leu Gly Arg Pro Leu Gly Pro Thr Pro Ser Gln Ser Arg
65                  70                  75                  80

ttc cag gtg gac ctg gtt tcc gag aac gcc ggg cgg gcc gct gct gcg      288
Phe Gln Val Asp Leu Val Ser Glu Asn Ala Gly Arg Ala Ala Ala Ala
                85                  90                  95

gcg gcg gcg gcg gcg gcg gca gcg gcg gcg gct ggt gct ggg gcg ggg      336
Ala Ala Ala Ala Ala Ala Ala Ala Ala Ala Ala Gly Ala Gly Ala Gly
            100                 105                 110

gcc aag cag acc ccc gcg gac ggg gaa gcc agc ggc gag agc gag ccg      384
Ala Lys Gln Thr Pro Ala Asp Gly Glu Ala Ser Gly Glu Ser Glu Pro
        115                 120                 125

gct aaa ggc agc gag gaa gcc aag ggc cgc ttc cgc gtg aac ttc gtg      432
Ala Lys Gly Ser Glu Glu Ala Lys Gly Arg Phe Arg Val Asn Phe Val
    130                 135                 140

gac cca gct gcc tcc tcg tcg gct gaa gac agc ctg tca gat gct gcc      480
Asp Pro Ala Ala Ser Ser Ser Ala Glu Asp Ser Leu Ser Asp Ala Ala
145                 150                 155                 160

ggg gtc gga gtc gac ggg ccc aac gtg agc ttc cag aac ggc ggg gac 528
Gly Val Gly Val Asp Gly Pro Asn Val Ser Phe Gln Asn Gly Gly Asp
                165                 170                 175

acg gtg ctg agc gag ggc agc agc ctg cac tcc ggc ggc ggc ggc ggc 576
Thr Val Leu Ser Glu Gly Ser Ser Leu His Ser Gly Gly Gly Gly Gly
            180                 185                 190

agt ggg cac cac cag cac tac tat tat gat acc cac acc aac acc tac 624
Ser Gly His His Gln His Tyr Tyr Tyr Asp Thr His Thr Asn Thr Tyr
        195                 200                 205

tac ctg cgc acc ttc ggc cac aac acc atg gac gct gtg ccc agg atc 672
Tyr Leu Arg Thr Phe Gly His Asn Thr Met Asp Ala Val Pro Arg Ile
    210                 215                 220

gat cac tac cgg cac aca gcc gcg cag ctg ggc gag aag ctg ctc cgg 720
Asp His Tyr Arg His Thr Ala Ala Gln Leu Gly Glu Lys Leu Leu Arg
225                 230                 235                 240

cct agc ctg gcg gag ctc cac gac gag ctg gaa aag gaa cct ttt gag 768
Pro Ser Leu Ala Glu Leu His Asp Glu Leu Glu Lys Glu Pro Phe Glu
                245                 250                 255

gat ggc ttt gca aat ggg gaa gaa agt act cca acc aga gat gct gtg 816
Asp Gly Phe Ala Asn Gly Glu Glu Ser Thr Pro Thr Arg Asp Ala Val
            260                 265                 270

gtc acg tat act gca gaa agt aaa gga gtc gtg aag ttt ggc tgg atc 864
Val Thr Tyr Thr Ala Glu Ser Lys Gly Val Val Lys Phe Gly Trp Ile
        275                 280                 285

aag ggt gta tta gta cgt tgt atg tta aac att tgg ggt gtg atg ctt 912
Lys Gly Val Leu Val Arg Cys Met Leu Asn Ile Trp Gly Val Met Leu
    290                 295                 300

ttc att aga ttg tca tgg att gtg ggt caa gct gga ata ggt cta tca 960
Phe Ile Arg Leu Ser Trp Ile Val Gly Gln Ala Gly Ile Gly Leu Ser
305                 310                 315                 320

gtc ctt gta ata atg atg gcc act gtt gtg aca act atc aca gga ttg 1008
Val Leu Val Ile Met Met Ala Thr Val Val Thr Thr Ile Thr Gly Leu
                325                 330                 335

tct act tca gca ata gca act aat gga ttt gta aga gga gcc acc cgg 1056
Ser Thr Ser Ala Ile Ala Thr Asn Gly Phe Val Arg Gly Ala Thr Arg
            340                 345                 350

ccc aag ttg aag aag atg aag agc cag acg gga cag gtg ggt gag aag 1104
Pro Lys Leu Lys Lys Met Lys Ser Gln Thr Gly Gln Val Gly Glu Lys
        355                 360                 365

caa tcg ctg aag tgt gag gca gca gcc ggt aat ccc cag cct tcc tac 1152
Gln Ser Leu Lys Cys Glu Ala Ala Ala Gly Asn Pro Gln Pro Ser Tyr
    370                 375                 380

cgt tgg ttc aag gat ggc aag gag ctc aac cgc agc cga gac att cgc 1200
Arg Trp Phe Lys Asp Gly Lys Glu Leu Asn Arg Ser Arg Asp Ile Arg
385                 390                 395                 400

atc aaa tat ggc aac ggc aga aag aac tca cga cta cag ttc aac aag 1248
Ile Lys Tyr Gly Asn Gly Arg Lys Asn Ser Arg Leu Gln Phe Asn Lys
                405                 410                 415

gtg aag gtg gag gac gct ggg gag tat gtc tgc gag gcc gag aac atc 1296
Val Lys Val Glu Asp Ala Gly Glu Tyr Val Cys Glu Ala Glu Asn Ile
            420                 425                 430

ctg ggg aag gac acc gtc cgg ggc cgg ctt tac gtc aac agc gtg agc 1344
Leu Gly Lys Asp Thr Val Arg Gly Arg Leu Tyr Val Asn Ser Val Ser
        435                 440                 445

acc acc ctg tca tcc tgg tcg ggg cac gcc cgg aag tgc aac gag aca 1392
Thr Thr Leu Ser Ser Trp Ser Gly His Ala Arg Lys Cys Asn Glu Thr
    450                 455                 460

gcc aag tcc tat tgc gtc aat gga ggc gtc tgc tac tac atc gag ggc 1440
Ala Lys Ser Tyr Cys Val Asn Gly Gly Val Cys Tyr Tyr Ile Glu Gly
465                 470                 475                 480

atc aac cag ctc tcc tgc aag tgt cct gtg gga tac acc ggg gac agg 1488
Ile Asn Gln Leu Ser Cys Lys Cys Pro Val Gly Tyr Thr Gly Asp Arg
                485                 490                 495

tgt cag cag ttc gca atg gtc aac ttc tcc aaa gcc gag gag ctg tac 1536
Cys Gln Gln Phe Ala Met Val Asn Phe Ser Lys Ala Glu Glu Leu Tyr
            500                 505                 510

cag aag agg gtc ctg acc atc acg ggc atc tgc gtg gct ctg ctg gtc 1584
Gln Lys Arg Val Leu Thr Ile Thr Gly Ile Cys Val Ala Leu Leu Val
        515                 520                 525

gtg ggc atc gtc tgt gtg gtg gcc tac tgc aag acc aaa aaa cag cgg 1632
Val Gly Ile Val Cys Val Val Ala Tyr Cys Lys Thr Lys Lys Gln Arg
    530                 535                 540

aag cag atg cac aac cac ctc cgg cag aac atg tgc ccg gcc cat cag 1680
Lys Gln Met His Asn His Leu Arg Gln Asn Met Cys Pro Ala His Gln
545                 550                 555                 560

aac cgg agc ttg gcc aat ggg ccc agc cac ccc cgg ctg gac cca gag 1728
Asn Arg Ser Leu Ala Asn Gly Pro Ser His Pro Arg Leu Asp Pro Glu
                565                 570                 575

gag atc cag atg gca gat tat att tcc aag aac gtg cca gcc aca gac 1776
Glu Ile Gln Met Ala Asp Tyr Ile Ser Lys Asn Val Pro Ala Thr Asp
            580                 585                 590

cat gtc atc agg aga gaa act gag acc acc ttc tct ggg agc cac tcc 1824
His Val Ile Arg Arg Glu Thr Glu Thr Thr Phe Ser Gly Ser His Ser
        595                 600                 605

tgt tct cct tct cac cac tgc tcc aca gcc aca ccc acc tcc agc cac 1872
Cys Ser Pro Ser His His Cys Ser Thr Ala Thr Pro Thr Ser Ser His
    610                 615                 620

aga cac gag agc cac acg tgg agc ctg gaa cgt tct gag agc ctg act 1920
Arg His Glu Ser His Thr Trp Ser Leu Glu Arg Ser Glu Ser Leu Thr
625                 630                 635                 640

tct gac tcc cag tcg ggg atc atg cta tca tca gtg ggt acc agc aaa 1968
Ser Asp Ser Gln Ser Gly Ile Met Leu Ser Ser Val Gly Thr Ser Lys
                645                 650                 655

tgc aac agc cca gca tgt gtg gag gcc cgg gca agg cgg gca gca gcc 2016
Cys Asn Ser Pro Ala Cys Val Glu Ala Arg Ala Arg Arg Ala Ala Ala

660                 665                 670

tac aac ctg gag gag cgg cgc agg gcc acc gcg cca ccc tat cac gat        2064
Tyr Asn Leu Glu Glu Arg Arg Arg Ala Thr Ala Pro Pro Tyr His Asp
        675                 680                 685

tcc gtg gac tcc ctt cgc gac tcc cca cac agc gag agg tac gtg tcg        2112
Ser Val Asp Ser Leu Arg Asp Ser Pro His Ser Glu Arg Tyr Val Ser
    690                 695                 700

gcc ctg acc acg ccc gcg cgc ctc tcg ccc gtg gac ttc cac tac tcg        2160
Ala Leu Thr Thr Pro Ala Arg Leu Ser Pro Val Asp Phe His Tyr Ser
705                 710                 715                 720

ctg gcc acg cag gtg cca act ttc gag atc acg tcc ccc aac tcg gcg        2208
Leu Ala Thr Gln Val Pro Thr Phe Glu Ile Thr Ser Pro Asn Ser Ala
                725                 730                 735

cac gcc gtg tcg ctg ccg ccg gcg gcg ccc atc agt tac cgc ctg gcc        2256
His Ala Val Ser Leu Pro Pro Ala Ala Pro Ile Ser Tyr Arg Leu Ala
            740                 745                 750

gag cag cag ccg tta ctg cgg cac ccg gcg ccc ccc ggc ccg gga ccc        2304
Glu Gln Gln Pro Leu Leu Arg His Pro Ala Pro Pro Gly Pro Gly Pro
        755                 760                 765

gga ccc ggg ccc ggg ccc ggg ccc ggc gca gac atg cag cgc agc tat        2352
Gly Pro Gly Pro Gly Pro Gly Pro Gly Ala Asp Met Gln Arg Ser Tyr
    770                 775                 780

gac agc tac tat tac ccc gcg gcg ggg ccc gga ccg cgg cgc ggg acc        2400
Asp Ser Tyr Tyr Tyr Pro Ala Ala Gly Pro Gly Pro Arg Arg Gly Thr
785                 790                 795                 800

tgc gcg ctc ggc ggc agc ctg ggc agc ctg cct gcc agc ccc ttc cgc        2448
Cys Ala Leu Gly Gly Ser Leu Gly Ser Leu Pro Ala Ser Pro Phe Arg
                805                 810                 815

atc ccc gag gac gac gag tac gag acc acg cag gag tgc gcg ccc ccg        2496
Ile Pro Glu Asp Asp Glu Tyr Glu Thr Thr Gln Glu Cys Ala Pro Pro
            820                 825                 830

ccg ccg ccg cgg ccg cgc gcg cgc ggt gcg tcc cgc agg acg tcg gcg        2544
Pro Pro Pro Arg Pro Arg Ala Arg Gly Ala Ser Arg Arg Thr Ser Ala
        835                 840                 845

ggg ccc cgg cgc tgg cgc cgc tcg cgc ctc aac ggg ctg gcg gcg cag        2592
Gly Pro Arg Arg Trp Arg Arg Ser Arg Leu Asn Gly Leu Ala Ala Gln
    850                 855                 860

cgc gca cgg gcg gcg agg gac tcg ctg tcg ctg agc agc ggc tcg ggc        2640
Arg Ala Arg Ala Ala Arg Asp Ser Leu Ser Leu Ser Ser Gly Ser Gly
865                 870                 875                 880

ggc ggc tca gcc tcg gcg tcg gac gac gac gcg gac gac gcg gac ggg        2688
Gly Gly Ser Ala Ser Ala Ser Asp Asp Asp Ala Asp Asp Ala Asp Gly
                885                 890                 895

gcg ctg gcg gcc gag agc aca cct ttc ctg ggc ctg cgt ggg gcg cac        2736
Ala Leu Ala Ala Glu Ser Thr Pro Phe Leu Gly Leu Arg Gly Ala His
            900                 905                 910

gac gcg ctg cgc tcg gac tcg ccg cca ctg tgc ccg gcg gcc gac agc        2784

Asp Ala Leu Arg Ser Asp Ser Pro Pro Leu Cys Pro Ala Ala Asp Ser
        915                 920                 925

agg act tac tac tca ctg gac agc cac agc acg cgg gcc agc agc aga        2832
Arg Thr Tyr Tyr Ser Leu Asp Ser His Ser Thr Arg Ala Ser Ser Arg
    930                 935                 940

cac agc cgc ggg ccg ccc ccg cgg gcc aag cag gac tcg gcg cca ctc        2880
His Ser Arg Gly Pro Pro Pro Arg Ala Lys Gln Asp Ser Ala Pro Leu
945                 950                 955                 960

tag                                                                    2883

<210>  22
<211>  960
<212>  PRT
<213>  Homo sapiens

<400>  22

Met Glu Pro Arg Pro Thr Ala Pro Ser Ser Gly Ala Pro Gly Leu Ala
1               5                   10                  15

Gly Val Gly Glu Thr Pro Ser Ala Ala Ala Leu Ala Ala Ala Arg Val
            20                  25                  30

Glu Leu Pro Gly Thr Ala Val Pro Ser Val Pro Glu Asp Ala Ala Pro
        35                  40                  45

Ala Ser Arg Asp Gly Gly Gly Val Arg Asp Glu Gly Pro Ala Ala Ala
    50                  55                  60

Gly Asp Gly Leu Gly Arg Pro Leu Gly Pro Thr Pro Ser Gln Ser Arg
65                  70                  75                  80

Phe Gln Val Asp Leu Val Ser Glu Asn Ala Gly Arg Ala Ala Ala Ala
                85                  90                  95

Ala Ala Ala Ala Ala Ala Ala Ala Ala Ala Ala Gly Ala Gly Ala Gly
            100                 105                 110

Ala Lys Gln Thr Pro Ala Asp Gly Glu Ala Ser Gly Glu Ser Glu Pro
        115                 120                 125

Ala Lys Gly Ser Glu Glu Ala Lys Gly Arg Phe Arg Val Asn Phe Val
    130                 135                 140

Asp Pro Ala Ala Ser Ser Ser Ala Glu Asp Ser Leu Ser Asp Ala Ala
145                 150                 155                 160

Gly Val Gly Val Asp Gly Pro Asn Val Ser Phe Gln Asn Gly Gly Asp
                165                 170                 175

Thr Val Leu Ser Glu Gly Ser Ser Leu His Ser Gly Gly Gly Gly Gly
                180                 185                 190

Ser Gly His His Gln His Tyr Tyr Tyr Asp Thr His Thr Asn Thr Tyr
        195                 200                 205

Tyr Leu Arg Thr Phe Gly His Asn Thr Met Asp Ala Val Pro Arg Ile
    210                 215                 220

Asp His Tyr Arg His Thr Ala Ala Gln Leu Gly Glu Lys Leu Leu Arg
225                 230                 235                 240

Pro Ser Leu Ala Glu Leu His Asp Glu Leu Glu Lys Glu Pro Phe Glu
                245                 250                 255

Asp Gly Phe Ala Asn Gly Glu Glu Ser Thr Pro Thr Arg Asp Ala Val
                260                 265                 270

Val Thr Tyr Thr Ala Glu Ser Lys Gly Val Val Lys Phe Gly Trp Ile
        275                 280                 285

Lys Gly Val Leu Val Arg Cys Met Leu Asn Ile Trp Gly Val Met Leu
    290                 295                 300

Phe Ile Arg Leu Ser Trp Ile Val Gly Gln Ala Gly Ile Gly Leu Ser
305                 310                 315                 320

Val Leu Val Ile Met Met Ala Thr Val Val Thr Thr Ile Thr Gly Leu
                325                 330                 335

Ser Thr Ser Ala Ile Ala Thr Asn Gly Phe Val Arg Gly Ala Thr Arg
                340                 345                 350

Pro Lys Leu Lys Lys Met Lys Ser Gln Thr Gly Gln Val Gly Glu Lys
        355                 360                 365

Gln Ser Leu Lys Cys Glu Ala Ala Ala Gly Asn Pro Gln Pro Ser Tyr
    370                 375                 380

Arg Trp Phe Lys Asp Gly Lys Glu Leu Asn Arg Ser Arg Asp Ile Arg
385                 390                 395                 400

Ile Lys Tyr Gly Asn Gly Arg Lys Asn Ser Arg Leu Gln Phe Asn Lys
                405                 410                 415

Val Lys Val Glu Asp Ala Gly Glu Tyr Val Cys Glu Ala Glu Asn Ile

            420                 425                 430

Leu Gly Lys Asp Thr Val Arg Gly Arg Leu Tyr Val Asn Ser Val Ser
        435                 440                 445

Thr Thr Leu Ser Ser Trp Ser Gly His Ala Arg Lys Cys Asn Glu Thr
    450                 455                 460

Ala Lys Ser Tyr Cys Val Asn Gly Gly Val Cys Tyr Tyr Ile Glu Gly
465                 470                 475                 480

Ile Asn Gln Leu Ser Cys Lys Cys Pro Val Gly Tyr Thr Gly Asp Arg
                485                 490                 495

Cys Gln Gln Phe Ala Met Val Asn Phe Ser Lys Ala Glu Glu Leu Tyr
            500                 505                 510

Gln Lys Arg Val Leu Thr Ile Thr Gly Ile Cys Val Ala Leu Leu Val
        515                 520                 525

Val Gly Ile Val Cys Val Val Ala Tyr Cys Lys Thr Lys Lys Gln Arg
    530                 535                 540

Lys Gln Met His Asn His Leu Arg Gln Asn Met Cys Pro Ala His Gln
545                 550                 555                 560

Asn Arg Ser Leu Ala Asn Gly Pro Ser His Pro Arg Leu Asp Pro Glu
                565                 570                 575

Glu Ile Gln Met Ala Asp Tyr Ile Ser Lys Asn Val Pro Ala Thr Asp
            580                 585                 590

His Val Ile Arg Arg Glu Thr Glu Thr Thr Phe Ser Gly Ser His Ser
        595                 600                 605

Cys Ser Pro Ser His His Cys Ser Thr Ala Thr Pro Thr Ser Ser His
    610                 615                 620

Arg His Glu Ser His Thr Trp Ser Leu Glu Arg Ser Glu Ser Leu Thr
625                 630                 635                 640

Ser Asp Ser Gln Ser Gly Ile Met Leu Ser Ser Val Gly Thr Ser Lys
                645                 650                 655

Cys Asn Ser Pro Ala Cys Val Glu Ala Arg Ala Arg Arg Ala Ala Ala
            660                 665                 670

Tyr Asn Leu Glu Glu Arg Arg Arg Ala Thr Ala Pro Pro Tyr His Asp
        675                 680                 685

Ser Val Asp Ser Leu Arg Asp Ser Pro His Ser Glu Arg Tyr Val Ser
    690                 695                 700

Ala Leu Thr Thr Pro Ala Arg Leu Ser Pro Val Asp Phe His Tyr Ser
705                 710                 715                 720

Leu Ala Thr Gln Val Pro Thr Phe Glu Ile Thr Ser Pro Asn Ser Ala
                725                 730                 735

His Ala Val Ser Leu Pro Pro Ala Ala Pro Ile Ser Tyr Arg Leu Ala
            740                 745                 750

Glu Gln Gln Pro Leu Leu Arg His Pro Ala Pro Pro Gly Pro Gly Pro
        755                 760                 765

Gly Pro Gly Pro Gly Pro Gly Pro Gly Ala Asp Met Gln Arg Ser Tyr
    770                 775                 780

Asp Ser Tyr Tyr Tyr Pro Ala Ala Gly Pro Gly Pro Arg Arg Gly Thr
785                 790                 795                 800

Cys Ala Leu Gly Gly Ser Leu Gly Ser Leu Pro Ala Ser Pro Phe Arg
                805                 810                 815

Ile Pro Glu Asp Asp Glu Tyr Glu Thr Thr Gln Glu Cys Ala Pro Pro
            820                 825                 830

Pro Pro Pro Arg Pro Arg Ala Arg Gly Ala Ser Arg Arg Thr Ser Ala
        835                 840                 845

Gly Pro Arg Arg Trp Arg Arg Ser Arg Leu Asn Gly Leu Ala Ala Gln
    850                 855                 860

Arg Ala Arg Ala Ala Arg Asp Ser Leu Ser Leu Ser Ser Gly Ser Gly
865                 870                 875                 880

Gly Gly Ser Ala Ser Ala Ser Asp Asp Asp Ala Asp Asp Ala Asp Gly
                885                 890                 895

Ala Leu Ala Ala Glu Ser Thr Pro Phe Leu Gly Leu Arg Gly Ala His
            900                 905                 910

Asp Ala Leu Arg Ser Asp Ser Pro Pro Leu Cys Pro Ala Ala Asp Ser
        915                 920                 925

Arg Thr Tyr Tyr Ser Leu Asp Ser His Ser Thr Arg Ala Ser Ser Arg
    930                 935                 940

His Ser Arg Gly Pro Pro Pro Arg Ala Lys Gln Asp Ser Ala Pro Leu
945                 950                 955                 960

<210>  23
<211>  2925
<212>  DNA
<213>  Homo sapiens

<220>
<221>  CDS
<222>  (1)..(2925)

<400>  23
atg gag ccg cgg ccc acg gcg ccc tcc tcc ggc gcc ccg gga ctg gcc      48
Met Glu Pro Arg Pro Thr Ala Pro Ser Ser Gly Ala Pro Gly Leu Ala
1               5                   10                  15

ggg gtc ggg gag acg ccg tca gcc gct gcg ctg gcc gca gcc agg gtg      96
Gly Val Gly Glu Thr Pro Ser Ala Ala Ala Leu Ala Ala Ala Arg Val
            20                  25                  30

gaa ctg ccc ggc acg gct gtg ccc tcg gtg ccg gag gat gct gcg ccc      144
Glu Leu Pro Gly Thr Ala Val Pro Ser Val Pro Glu Asp Ala Ala Pro
        35                  40                  45

gcg agc cgg gac ggc ggc ggg gtc cgc gat gag ggc ccc gcg gcg gcc      192
Ala Ser Arg Asp Gly Gly Gly Val Arg Asp Glu Gly Pro Ala Ala Ala
    50                  55                  60

ggg gac ggg ctg ggc aga ccc ttg ggg ccc acc ccg agc cag agc cgt      240
Gly Asp Gly Leu Gly Arg Pro Leu Gly Pro Thr Pro Ser Gln Ser Arg
65                  70                  75                  80

ttc cag gtg gac ctg gtt tcc gag aac gcc ggg cgg gcc gct gct gcg      288
Phe Gln Val Asp Leu Val Ser Glu Asn Ala Gly Arg Ala Ala Ala Ala
                85                  90                  95

gcg gcg gcg gcg gcg gcg gca gcg gcg gcg gct ggt gct ggg gcg ggg      336
Ala Ala Ala Ala Ala Ala Ala Ala Ala Ala Ala Gly Ala Gly Ala Gly
            100                 105                 110

gcc aag cag acc ccc gcg gac ggg gaa gcc agc ggc gag agc gag ccg      384
Ala Lys Gln Thr Pro Ala Asp Gly Glu Ala Ser Gly Glu Ser Glu Pro
        115                 120                 125

gct aaa ggc agc gag gaa gcc aag ggc cgc ttc cgc gtg aac ttc gtg      432
Ala Lys Gly Ser Glu Glu Ala Lys Gly Arg Phe Arg Val Asn Phe Val
    130                 135                 140

gac cca gct gcc tcc tcg tcg gct gaa gac agc ctg tca gat gct gcc      480
Asp Pro Ala Ala Ser Ser Ser Ala Glu Asp Ser Leu Ser Asp Ala Ala
145                 150                 155                 160

ggg gtc gga gtc gac ggg ccc aac gtg agc ttc cag aac ggc ggg gac      528
Gly Val Gly Val Asp Gly Pro Asn Val Ser Phe Gln Asn Gly Gly Asp
                165                 170                 175

acg gtg ctg agc gag ggc agc agc ctg cac tcc ggc ggc ggc ggc 576
Thr Val Leu Ser Glu Gly Ser Ser Leu His Ser Gly Gly Gly Gly
            180                 185                 190

agt ggg cac cac cag cac tac tat tat gat acc cac acc aac acc tac 624
Ser Gly His His Gln His Tyr Tyr Tyr Asp Thr His Thr Asn Thr Tyr
            195                 200                 205

tac ctg cgc acc ttc ggc cac aac acc atg gac gct gtg ccc agg atc 672
Tyr Leu Arg Thr Phe Gly His Asn Thr Met Asp Ala Val Pro Arg Ile
        210                 215                 220

gat cac tac cgg cac aca gcc gcg cag ctg ggc gag aag ctg ctc cgg 720
Asp His Tyr Arg His Thr Ala Ala Gln Leu Gly Glu Lys Leu Leu Arg
225 230 235 240

cct agc ctg gcg gag ctc cac gac gag ctg gaa aag gaa cct ttt gag 768
Pro Ser Leu Ala Glu Leu His Asp Glu Leu Glu Lys Glu Pro Phe Glu
245 250 255

gat ggc ttt gca aat ggg gaa gaa agt act cca acc aga gat gct gtg 816
Asp Gly Phe Ala Asn Gly Glu Glu Ser Thr Pro Thr Arg Asp Ala Val
                260                 265                 270

gtc acg tat act gca gaa agt aaa gga gtc gtg aag ttt ggc tgg atc 864
Val Thr Tyr Thr Ala Glu Ser Lys Gly Val Val Lys Phe Gly Trp Ile
            275                 280                 285

aag ggt gta tta gta cgt tgt atg tta aac att tgg ggt gtg atg ctt 912
Lys Gly Val Leu Val Arg Cys Met Leu Asn Ile Trp Gly Val Met Leu
        290                 295                 300

ttc att aga ttg tca tgg att gtg ggt caa gct gga ata ggt cta tca 960
Phe Ile Arg Leu Ser Trp Ile Val Gly Gln Ala Gly Ile Gly Leu Ser
305 310 315 320

gtc ctt gta ata atg atg gcc act gtt gtg aca act atc aca gga ttg 1008
Val Leu Val Ile Met Met Ala Thr Val Val Thr Thr Ile Thr Gly Leu
325 330 335

tct act tca gca ata gca act aat gga ttt gta aga gga gcc acc cgg 1056
Ser Thr Ser Ala Ile Ala Thr Asn Gly Phe Val Arg Gly Ala Thr Arg
                340                 345                 350

ccc aag ttg aag aag atg aag agc cag acg gga cag gtg ggt gag aag 1104
Pro Lys Leu Lys Lys Met Lys Ser Gln Thr Gly Gln Val Gly Glu Lys
            355                 360                 365

caa tcg ctg aag tgt gag gca gca gcc ggt aat ccc cag cct tcc tac 1152
Gln Ser Leu Lys Cys Glu Ala Ala Ala Gly Asn Pro Gln Pro Ser Tyr
        370                 375                 380

cgt tgg ttc aag gat ggc aag gag ctc aac cgc agc cga gac att cgc 1200
Arg Trp Phe Lys Asp Gly Lys Glu Leu Asn Arg Ser Arg Asp Ile Arg
385 390 395 400

atc aaa tat ggc aac ggc aga aag aac tca cga cta cag ttc aac aag 1248
Ile Lys Tyr Gly Asn Gly Arg Lys Asn Ser Arg Leu Gln Phe Asn Lys
405 410 415

gtg aag gtg gag gac gct ggg gag tat gtc tgc gag gcc gag aac atc 1296
Val Lys Val Glu Asp Ala Gly Glu Tyr Val Cys Glu Ala Glu Asn Ile

420                 425                 430

ctg ggg aag gac acc gtc cgg ggc cgg ctt tac gtc aac agc gtg agc        1344
Leu Gly Lys Asp Thr Val Arg Gly Arg Leu Tyr Val Asn Ser Val Ser
        435                 440                 445

acc acc ctg tca tcc tgg tcg ggg cac gcc cgg aag tgc aac gag aca        1392
Thr Thr Leu Ser Ser Trp Ser Gly His Ala Arg Lys Cys Asn Glu Thr
    450                 455                 460

gcc aag tcc tat tgc gtc aat gga ggc gtc tgc tac tac atc gag ggc        1440
Ala Lys Ser Tyr Cys Val Asn Gly Gly Val Cys Tyr Tyr Ile Glu Gly
465                 470                 475                 480

atc aac cag ctc tcc tgc aaa tgt cca aat gga ttc ttc gga cag aga        1488
Ile Asn Gln Leu Ser Cys Lys Cys Pro Asn Gly Phe Phe Gly Gln Arg
                485                 490                 495

tgt ttg gag aaa ctg cct ttg cga ttg tac atg cca gat cct aag caa        1536
Cys Leu Glu Lys Leu Pro Leu Arg Leu Tyr Met Pro Asp Pro Lys Gln
            500                 505                 510

aag cac ctt gga ttt gaa tta aag gaa gcc gag gag ctg tac cag aag        1584
Lys His Leu Gly Phe Glu Leu Lys Glu Ala Glu Glu Leu Tyr Gln Lys
        515                 520                 525

agg gtc ctg acc atc acg ggc atc tgc gtg gct ctg ctg gtc gtg ggc        1632
Arg Val Leu Thr Ile Thr Gly Ile Cys Val Ala Leu Leu Val Val Gly
    530                 535                 540

atc gtc tgt gtg gtg gcc tac tgc aag acc aaa aaa cag cgg aag cag        1680
Ile Val Cys Val Val Ala Tyr Cys Lys Thr Lys Lys Gln Arg Lys Gln
545                 550                 555                 560

atg cac aac cac ctc cgg cag aac atg tgc ccg gcc cat cag aac cgg        1728
Met His Asn His Leu Arg Gln Asn Met Cys Pro Ala His Gln Asn Arg
                565                 570                 575

agc ttg gcc aat ggg ccc agc cac ccc cgg ctg gac cca gag gag atc        1776
Ser Leu Ala Asn Gly Pro Ser His Pro Arg Leu Asp Pro Glu Glu Ile
            580                 585                 590

cag atg gca gat tat att tcc aag aac gtg cca gcc aca gac cat gtc        1824
Gln Met Ala Asp Tyr Ile Ser Lys Asn Val Pro Ala Thr Asp His Val
        595                 600                 605

atc agg aga gaa act gag acc acc ttc tct ggg agc cac tcc tgt tct        1872
Ile Arg Arg Glu Thr Glu Thr Thr Phe Ser Gly Ser His Ser Cys Ser
    610                 615                 620

cct tct cac cac tgc tcc aca gcc aca ccc acc tcc agc cac aga cac        1920
Pro Ser His His Cys Ser Thr Ala Thr Pro Thr Ser Ser His Arg His
625                 630                 635                 640

gag agc cac acg tgg agc ctg gaa cgt tct gag agc ctg act tct gac        1968
Glu Ser His Thr Trp Ser Leu Glu Arg Ser Glu Ser Leu Thr Ser Asp
                645                 650                 655

tcc cag tcg ggg atc atg cta tca tca gtg ggt acc agc aaa tgc aac        2016
Ser Gln Ser Gly Ile Met Leu Ser Ser Val Gly Thr Ser Lys Cys Asn
            660                 665                 670

agc cca gca tgt gtg gag gcc cgg gca agg cgg gca gca gcc tac aac        2064

Ser Pro Ala Cys Val Glu Ala Arg Ala Arg Arg Ala Ala Ala Tyr Asn
        675                 680                 685

ctg gag gag cgg cgc agg gcc acc gcg cca ccc tat cac gat tcc gtg      2112
Leu Glu Glu Arg Arg Arg Ala Thr Ala Pro Pro Tyr His Asp Ser Val
    690                 695                 700

gac tcc ctt cgc gac tcc cca cac agc gag agg tac gtg tcg gcc ctg      2160
Asp Ser Leu Arg Asp Ser Pro His Ser Glu Arg Tyr Val Ser Ala Leu
705                 710                 715                 720

acc acg ccc gcg cgc ctc tcg ccc gtg gac ttc cac tac tcg ctg gcc      2208
Thr Thr Pro Ala Arg Leu Ser Pro Val Asp Phe His Tyr Ser Leu Ala
                725                 730                 735

acg cag gtg cca act ttc gag atc acg tcc ccc aac tcg gcg cac gcc      2256
Thr Gln Val Pro Thr Phe Glu Ile Thr Ser Pro Asn Ser Ala His Ala
            740                 745                 750

gtg tcg ctg ccg ccg gcg gcg ccc atc agt tac cgc ctg gcc gag cag      2304
Val Ser Leu Pro Pro Ala Ala Pro Ile Ser Tyr Arg Leu Ala Glu Gln
        755                 760                 765

cag ccg tta ctg cgg cac ccg gcg ccc ccc ggc ccg gga ccc gga ccc      2352
Gln Pro Leu Leu Arg His Pro Ala Pro Pro Gly Pro Gly Pro Gly Pro
    770                 775                 780

ggg ccc ggg ccc ggg ccc ggc gca gac atg cag cgc agc tat gac agc      2400
Gly Pro Gly Pro Gly Pro Gly Ala Asp Met Gln Arg Ser Tyr Asp Ser
785                 790                 795                 800

tac tat tac ccc gcg gcg ggg ccc gga ccg cgg cgc ggg acc tgc gcg      2448
Tyr Tyr Tyr Pro Ala Ala Gly Pro Gly Pro Arg Arg Gly Thr Cys Ala
                805                 810                 815

ctc ggc ggc agc ctg ggc agc ctg cct gcc agc ccc ttc cgc atc ccc      2496
Leu Gly Gly Ser Leu Gly Ser Leu Pro Ala Ser Pro Phe Arg Ile Pro
            820                 825                 830

gag gac gac gag tac gag acc acg cag gag tgc gcg ccc ccg ccg ccg      2544
Glu Asp Asp Glu Tyr Glu Thr Thr Gln Glu Cys Ala Pro Pro Pro Pro
        835                 840                 845

ccg cgg ccg cgc gcg cgc ggt gcg tcc cgc agg acg tcg gcg ggg ccc      2592
Pro Arg Pro Arg Ala Arg Gly Ala Ser Arg Arg Thr Ser Ala Gly Pro
    850                 855                 860

cgg cgc tgg cgc cgc tcg cgc ctc aac ggg ctg gcg gcg cag cgc gca      2640
Arg Arg Trp Arg Arg Ser Arg Leu Asn Gly Leu Ala Ala Gln Arg Ala
865                 870                 875                 880

cgg gcg gcg agg gac tcg ctg tcg ctg agc agc ggc tcg ggc ggc ggc      2688
Arg Ala Ala Arg Asp Ser Leu Ser Leu Ser Ser Gly Ser Gly Gly Gly
                885                 890                 895

tca gcc tcg gcg tcg gac gac gac gcg gac gac gcg gac ggg gcg ctg      2736
Ser Ala Ser Ala Ser Asp Asp Asp Ala Asp Asp Ala Asp Gly Ala Leu
            900                 905                 910

gcg gcc gag agc aca cct ttc ctg ggc ctg cgt ggg gcg cac gac gcg      2784
Ala Ala Glu Ser Thr Pro Phe Leu Gly Leu Arg Gly Ala His Asp Ala
        915                 920                 925

ctg cgc tcg gac tcg ccg cca ctg tgc ccg gcg gcc gac agc agg act 2832
Leu Arg Ser Asp Ser Pro Pro Leu Cys Pro Ala Ala Asp Ser Arg Thr
        930                 935                 940

tac tac tca ctg gac agc cac agc acg cgg gcc agc agc aga cac agc 2880
Tyr Tyr Ser Leu Asp Ser His Ser Thr Arg Ala Ser Ser Arg His Ser
945 950 955 960

cgc ggg ccg ccc ccg cgg gcc aag cag gac tcg gcg cca ctc tag 2925
Arg Gly Pro Pro Pro Arg Ala Lys Gln Asp Ser Ala Pro Leu
965 970

<210> 24
<211> 974
<212> PRT
<213> Homo sapiens

<400> 24

Met Glu Pro Arg Pro Thr Ala Pro Ser Ser Gly Ala Pro Gly Leu Ala
1               5                   10                  15

Gly Val Gly Glu Thr Pro Ser Ala Ala Ala Leu Ala Ala Ala Arg Val
            20                  25                  30

Glu Leu Pro Gly Thr Ala Val Pro Ser Val Pro Glu Asp Ala Ala Pro
        35                  40                  45

Ala Ser Arg Asp Gly Gly Gly Val Arg Asp Glu Gly Pro Ala Ala Ala
    50                  55                  60

Gly Asp Gly Leu Gly Arg Pro Leu Gly Pro Thr Pro Ser Gln Ser Arg
65                  70                  75                  80

Phe Gln Val Asp Leu Val Ser Glu Asn Ala Gly Arg Ala Ala Ala Ala
                85                  90                  95

Ala Ala Ala Ala Ala Ala Ala Ala Ala Ala Ala Gly Ala Gly Ala Gly
            100                 105                 110

Ala Lys Gln Thr Pro Ala Asp Gly Glu Ala Ser Gly Glu Ser Glu Pro
        115                 120                 125

Ala Lys Gly Ser Glu Glu Ala Lys Gly Arg Phe Arg Val Asn Phe Val
    130                 135                 140

Asp Pro Ala Ala Ser Ser Ser Ala Glu Asp Ser Leu Ser Asp Ala Ala
145                 150                 155                 160

Gly Val Gly Val Asp Gly Pro Asn Val Ser Phe Gln Asn Gly Gly Asp
                165                 170                 175

Thr Val Leu Ser Glu Gly Ser Ser Leu His Ser Gly Gly Gly Gly Gly
            180                 185                 190

Ser Gly His His Gln His Tyr Tyr Tyr Asp Thr His Thr Asn Thr Tyr
        195                 200                 205

Tyr Leu Arg Thr Phe Gly His Asn Thr Met Asp Ala Val Pro Arg Ile
    210                 215                 220

Asp His Tyr Arg His Thr Ala Ala Gln Leu Gly Glu Lys Leu Leu Arg
225                 230                 235                 240

Pro Ser Leu Ala Glu Leu His Asp Glu Leu Glu Lys Glu Pro Phe Glu
                245                 250                 255

Asp Gly Phe Ala Asn Gly Glu Glu Ser Thr Pro Thr Arg Asp Ala Val
            260                 265                 270

Val Thr Tyr Thr Ala Glu Ser Lys Gly Val Val Lys Phe Gly Trp Ile
        275                 280                 285

Lys Gly Val Leu Val Arg Cys Met Leu Asn Ile Trp Gly Val Met Leu
    290                 295                 300

Phe Ile Arg Leu Ser Trp Ile Val Gly Gln Ala Gly Ile Gly Leu Ser
305                 310                 315                 320

Val Leu Val Ile Met Met Ala Thr Val Val Thr Thr Ile Thr Gly Leu
                325                 330                 335

Ser Thr Ser Ala Ile Ala Thr Asn Gly Phe Val Arg Gly Ala Thr Arg
            340                 345                 350

Pro Lys Leu Lys Lys Met Lys Ser Gln Thr Gly Gln Val Gly Glu Lys
        355                 360                 365

Gln Ser Leu Lys Cys Glu Ala Ala Ala Gly Asn Pro Gln Pro Ser Tyr
    370                 375                 380

Arg Trp Phe Lys Asp Gly Lys Glu Leu Asn Arg Ser Arg Asp Ile Arg
385                 390                 395                 400

Ile Lys Tyr Gly Asn Gly Arg Lys Asn Ser Arg Leu Gln Phe Asn Lys
                405                 410                 415

Val Lys Val Glu Asp Ala Gly Glu Tyr Val Cys Glu Ala Glu Asn Ile
            420                 425                 430

Leu Gly Lys Asp Thr Val Arg Gly Arg Leu Tyr Val Asn Ser Val Ser
        435                 440                 445

Thr Thr Leu Ser Ser Trp Ser Gly His Ala Arg Lys Cys Asn Glu Thr
    450                 455                 460

Ala Lys Ser Tyr Cys Val Asn Gly Gly Val Cys Tyr Tyr Ile Glu Gly
465                 470                 475                 480

Ile Asn Gln Leu Ser Cys Lys Cys Pro Asn Gly Phe Phe Gly Gln Arg
                485                 490                 495

Cys Leu Glu Lys Leu Pro Leu Arg Leu Tyr Met Pro Asp Pro Lys Gln
            500                 505                 510

Lys His Leu Gly Phe Glu Leu Lys Glu Ala Glu Glu Leu Tyr Gln Lys
        515                 520                 525

Arg Val Leu Thr Ile Thr Gly Ile Cys Val Ala Leu Leu Val Val Gly
    530                 535                 540

Ile Val Cys Val Val Ala Tyr Cys Lys Thr Lys Lys Gln Arg Lys Gln
545                 550                 555                 560

Met His Asn His Leu Arg Gln Asn Met Cys Pro Ala His Gln Asn Arg
                565                 570                 575

Ser Leu Ala Asn Gly Pro Ser His Pro Arg Leu Asp Pro Glu Glu Ile
            580                 585                 590

Gln Met Ala Asp Tyr Ile Ser Lys Asn Val Pro Ala Thr Asp His Val
        595                 600                 605

Ile Arg Arg Glu Thr Glu Thr Thr Phe Ser Gly Ser His Ser Cys Ser
    610                 615                 620

Pro Ser His His Cys Ser Thr Ala Thr Pro Thr Ser Ser His Arg His
625                 630                 635                 640

Glu Ser His Thr Trp Ser Leu Glu Arg Ser Glu Ser Leu Thr Ser Asp
                645                 650                 655

Ser Gln Ser Gly Ile Met Leu Ser Ser Val Gly Thr Ser Lys Cys Asn
            660                 665                 670

Ser Pro Ala Cys Val Glu Ala Arg Ala Arg Arg Ala Ala Ala Tyr Asn

675                 680                 685

Leu Glu Glu Arg Arg Arg Ala Thr Ala Pro Pro Tyr His Asp Ser Val
    690                 695                 700

Asp Ser Leu Arg Asp Ser Pro His Ser Glu Arg Tyr Val Ser Ala Leu
705                 710                 715                 720

Thr Thr Pro Ala Arg Leu Ser Pro Val Asp Phe His Tyr Ser Leu Ala
                725                 730                 735

Thr Gln Val Pro Thr Phe Glu Ile Thr Ser Pro Asn Ser Ala His Ala
            740                 745                 750

Val Ser Leu Pro Pro Ala Ala Pro Ile Ser Tyr Arg Leu Ala Glu Gln
        755                 760                 765

Gln Pro Leu Leu Arg His Pro Ala Pro Pro Gly Pro Gly Pro Gly Pro
    770                 775                 780

Gly Pro Gly Pro Gly Pro Gly Ala Asp Met Gln Arg Ser Tyr Asp Ser
785                 790                 795                 800

Tyr Tyr Tyr Pro Ala Ala Gly Pro Gly Pro Arg Arg Gly Thr Cys Ala
                805                 810                 815

Leu Gly Gly Ser Leu Gly Ser Leu Pro Ala Ser Pro Phe Arg Ile Pro
            820                 825                 830

Glu Asp Asp Glu Tyr Glu Thr Thr Gln Glu Cys Ala Pro Pro Pro Pro
        835                 840                 845

Pro Arg Pro Arg Ala Arg Gly Ala Ser Arg Arg Thr Ser Ala Gly Pro
    850                 855                 860

Arg Arg Trp Arg Arg Ser Arg Leu Asn Gly Leu Ala Ala Gln Arg Ala
865                 870                 875                 880

Arg Ala Ala Arg Asp Ser Leu Ser Leu Ser Ser Gly Ser Gly Gly Gly
                885                 890                 895

Ser Ala Ser Ala Ser Asp Asp Asp Ala Asp Asp Ala Asp Gly Ala Leu
            900                 905                 910

Ala Ala Glu Ser Thr Pro Phe Leu Gly Leu Arg Gly Ala His Asp Ala
        915                 920                 925

Leu Arg Ser Asp Ser Pro Pro Leu Cys Pro Ala Ala Asp Ser Arg Thr
    930                 935                 940

Tyr Tyr Ser Leu Asp Ser His Ser Thr Arg Ala Ser Ser Arg His Ser
945                 950                 955                 960

Arg Gly Pro Pro Pro Arg Ala Lys Gln Asp Ser Ala Pro Leu
                965                 970

<210>  25
<211>  2907
<212>  DNA
<213>  Homo sapiens

<220>
<221>  CDS
<222>  (1)..(2907)

<400>  25
atg gag ccg cgg ccc acg gcg ccc tcc tcc ggc gcc ccg gga ctg gcc       48
Met Glu Pro Arg Pro Thr Ala Pro Ser Ser Gly Ala Pro Gly Leu Ala
1               5                   10                  15

ggg gtc ggg gag acg ccg tca gcc gct gcg ctg gcc gca gcc agg gtg       96
Gly Val Gly Glu Thr Pro Ser Ala Ala Ala Leu Ala Ala Ala Arg Val
            20                  25                  30

gaa ctg ccc ggc acg gct gtg ccc tcg gtg ccg gag gat gct gcg ccc      144
Glu Leu Pro Gly Thr Ala Val Pro Ser Val Pro Glu Asp Ala Ala Pro
        35                  40                  45

gcg agc cgg gac ggc ggc ggg gtc cgc gat gag ggc ccc gcg gcg gcc      192
Ala Ser Arg Asp Gly Gly Gly Val Arg Asp Glu Gly Pro Ala Ala Ala
    50                  55                  60

ggg gac ggg ctg ggc aga ccc ttg ggg ccc acc ccg agc cag agc cgt      240
Gly Asp Gly Leu Gly Arg Pro Leu Gly Pro Thr Pro Ser Gln Ser Arg
65                  70                  75                  80

ttc cag gtg gac ctg gtt tcc gag aac gcc ggg cgg gcc gct gct gcg      288
Phe Gln Val Asp Leu Val Ser Glu Asn Ala Gly Arg Ala Ala Ala Ala
                85                  90                  95

gcg gcg gcg gcg gcg gcg gca gcg gcg gcg gct ggt gct ggg gcg ggg      336
Ala Ala Ala Ala Ala Ala Ala Ala Ala Ala Ala Gly Ala Gly Ala Gly
            100                 105                 110

gcc aag cag acc ccc gcg gac ggg gaa gcc agc ggc gag agc gag ccg      384
Ala Lys Gln Thr Pro Ala Asp Gly Glu Ala Ser Gly Glu Ser Glu Pro
        115                 120                 125

gct aaa ggc agc gag gaa gcc aag ggc cgc ttc cgc gtg aac ttc gtg      432
Ala Lys Gly Ser Glu Glu Ala Lys Gly Arg Phe Arg Val Asn Phe Val
    130                 135                 140

gac cca gct gcc tcc tcg tcg gct gaa gac agc ctg tca gat gct gcc      480
Asp Pro Ala Ala Ser Ser Ser Ala Glu Asp Ser Leu Ser Asp Ala Ala
145                 150                 155                 160

ggg gtc gga gtc gac ggg ccc aac gtg agc ttc cag aac ggc ggg gac 528
Gly Val Gly Val Asp Gly Pro Asn Val Ser Phe Gln Asn Gly Gly Asp
                165                 170                 175

acg gtg ctg agc gag ggc agc agc ctg cac tcc ggc ggc ggc ggc ggc 576
Thr Val Leu Ser Glu Gly Ser Ser Leu His Ser Gly Gly Gly Gly Gly
            180                 185                 190

agt ggg cac cac cag cac tac tat tat gat acc cac acc aac acc tac 624
Ser Gly His His Gln His Tyr Tyr Tyr Asp Thr His Thr Asn Thr Tyr
        195                 200                 205

tac ctg cgc acc ttc ggc cac aac acc atg gac gct gtg ccc agg atc 672
Tyr Leu Arg Thr Phe Gly His Asn Thr Met Asp Ala Val Pro Arg Ile
    210                 215                 220

gat cac tac cgg cac aca gcc gcg cag ctg ggc gag aag ctg ctc cgg 720
Asp His Tyr Arg His Thr Ala Ala Gln Leu Gly Glu Lys Leu Leu Arg
225                 230                 235                 240

cct agc ctg gcg gag ctc cac gac gag ctg gaa aag gaa cct ttt gag 768
Pro Ser Leu Ala Glu Leu His Asp Glu Leu Glu Lys Glu Pro Phe Glu
                245                 250                 255

gat ggc ttt gca aat ggg gaa gaa agt act cca acc aga gat gct gtg 816
Asp Gly Phe Ala Asn Gly Glu Glu Ser Thr Pro Thr Arg Asp Ala Val
            260                 265                 270

gtc acg tat act gca gaa agt aaa gga gtc gtg aag ttt ggc tgg atc 864
Val Thr Tyr Thr Ala Glu Ser Lys Gly Val Val Lys Phe Gly Trp Ile
        275                 280                 285

aag ggt gta tta gta cgt tgt atg tta aac att tgg ggt gtg atg ctt 912
Lys Gly Val Leu Val Arg Cys Met Leu Asn Ile Trp Gly Val Met Leu
    290                 295                 300

ttc att aga ttg tca tgg att gtg ggt caa gct gga ata ggt cta tca 960
Phe Ile Arg Leu Ser Trp Ile Val Gly Gln Ala Gly Ile Gly Leu Ser
305                 310                 315                 320

gtc ctt gta ata atg atg gcc act gtt gtg aca act atc aca gga ttg 1008
Val Leu Val Ile Met Met Ala Thr Val Val Thr Thr Ile Thr Gly Leu
                325                 330                 335

tct act tca gca ata gca act aat gga ttt gta aga gga gcc acc cgg 1056
Ser Thr Ser Ala Ile Ala Thr Asn Gly Phe Val Arg Gly Ala Thr Arg
            340                 345                 350

ccc aag ttg aag aag atg aag agc cag acg gga cag gtg ggt gag aag 1104
Pro Lys Leu Lys Lys Met Lys Ser Gln Thr Gly Gln Val Gly Glu Lys
        355                 360                 365

caa tcg ctg aag tgt gag gca gca gcc ggt aat ccc cag cct tcc tac 1152
Gln Ser Leu Lys Cys Glu Ala Ala Ala Gly Asn Pro Gln Pro Ser Tyr
    370                 375                 380

cgt tgg ttc aag gat ggc aag gag ctc aac cgc agc cga gac att cgc 1200
Arg Trp Phe Lys Asp Gly Lys Glu Leu Asn Arg Ser Arg Asp Ile Arg
385                 390                 395                 400

atc aaa tat ggc aac ggc aga aag aac tca cga cta cag ttc aac aag 1248
Ile Lys Tyr Gly Asn Gly Arg Lys Asn Ser Arg Leu Gln Phe Asn Lys
                405                 410                 415

gtg aag gtg gag gac gct ggg gag tat gtc tgc gag gcc gag aac atc 1296
Val Lys Val Glu Asp Ala Gly Glu Tyr Val Cys Glu Ala Glu Asn Ile
            420                 425                 430

ctg ggg aag gac acc gtc cgg ggc cgg ctt tac gtc aac agc gtg agc 1344
Leu Gly Lys Asp Thr Val Arg Gly Arg Leu Tyr Val Asn Ser Val Ser
        435                 440                 445

acc acc ctg tca tcc tgg tcg ggg cac gcc cgg aag tgc aac gag aca 1392
Thr Thr Leu Ser Ser Trp Ser Gly His Ala Arg Lys Cys Asn Glu Thr
    450                 455                 460

gcc aag tcc tat tgc gtc aat gga ggc gtc tgc tac tac atc gag ggc 1440
Ala Lys Ser Tyr Cys Val Asn Gly Gly Val Cys Tyr Tyr Ile Glu Gly
465                 470                 475                 480

atc aac cag ctc tcc tgc aag tgt cct gtg gga tac acc ggg gac agg 1488
Ile Asn Gln Leu Ser Cys Lys Cys Pro Val Gly Tyr Thr Gly Asp Arg
                485                 490                 495

tgt cag cag ttc gca atg gtc aac ttc tcc aag cac ctt gga ttt gaa 1536
Cys Gln Gln Phe Ala Met Val Asn Phe Ser Lys His Leu Gly Phe Glu
            500                 505                 510

tta aag gaa gcc gag gag ctg tac cag aag agg gtc ctg acc atc acg 1584
Leu Lys Glu Ala Glu Glu Leu Tyr Gln Lys Arg Val Leu Thr Ile Thr
        515                 520                 525

ggc atc tgc gtg gct ctg ctg gtc gtg ggc atc gtc tgt gtg gtg gcc 1632
Gly Ile Cys Val Ala Leu Leu Val Val Gly Ile Val Cys Val Val Ala
    530                 535                 540

tac tgc aag acc aaa aaa cag cgg aag cag atg cac aac cac ctc cgg 1680
Tyr Cys Lys Thr Lys Lys Gln Arg Lys Gln Met His Asn His Leu Arg
545                 550                 555                 560

cag aac atg tgc ccg gcc cat cag aac cgg agc ttg gcc aat ggg ccc 1728
Gln Asn Met Cys Pro Ala His Gln Asn Arg Ser Leu Ala Asn Gly Pro
                565                 570                 575

agc cac ccc cgg ctg gac cca gag gag atc cag atg gca gat tat att 1776
Ser His Pro Arg Leu Asp Pro Glu Glu Ile Gln Met Ala Asp Tyr Ile
            580                 585                 590

tcc aag aac gtg cca gcc aca gac cat gtc atc agg aga gaa act gag 1824
Ser Lys Asn Val Pro Ala Thr Asp His Val Ile Arg Arg Glu Thr Glu
        595                 600                 605

acc acc ttc tct ggg agc cac tcc tgt tct cct tct cac cac tgc tcc 1872
Thr Thr Phe Ser Gly Ser His Ser Cys Ser Pro Ser His His Cys Ser
    610                 615                 620

aca gcc aca ccc acc tcc agc cac aga cac gag agc cac acg tgg agc 1920
Thr Ala Thr Pro Thr Ser Ser His Arg His Glu Ser His Thr Trp Ser
625                 630                 635                 640

ctg gaa cgt tct gag agc ctg act tct gac tcc cag tcg ggg atc atg 1968
Leu Glu Arg Ser Glu Ser Leu Thr Ser Asp Ser Gln Ser Gly Ile Met
                645                 650                 655

cta tca tca gtg ggt acc agc aaa tgc aac agc cca gca tgt gtg gag 2016
Leu Ser Ser Val Gly Thr Ser Lys Cys Asn Ser Pro Ala Cys Val Glu

            660                 665                 670

gcc cgg gca agg cgg gca gca gcc tac aac ctg gag gag cgg cgc agg 2064
Ala Arg Ala Arg Arg Ala Ala Ala Tyr Asn Leu Glu Glu Arg Arg Arg
        675                 680                 685

gcc acc gcg cca ccc tat cac gat tcc gtg gac tcc ctt cgc gac tcc 2112
Ala Thr Ala Pro Pro Tyr His Asp Ser Val Asp Ser Leu Arg Asp Ser
    690                 695                 700

cca cac agc gag agg tac gtg tcg gcc ctg acc acg ccc gcg cgc ctc 2160
Pro His Ser Glu Arg Tyr Val Ser Ala Leu Thr Thr Pro Ala Arg Leu
705                 710                 715                 720

tcg ccc gtg gac ttc cac tac tcg ctg gcc acg cag gtg cca act ttc 2208
Ser Pro Val Asp Phe His Tyr Ser Leu Ala Thr Gln Val Pro Thr Phe
                725                 730                 735

gag atc acg tcc ccc aac tcg gcg cac gcc gtg tcg ctg ccg ccg gcg 2256
Glu Ile Thr Ser Pro Asn Ser Ala His Ala Val Ser Leu Pro Pro Ala
            740                 745                 750

gcg ccc atc agt tac cgc ctg gcc gag cag cag ccg tta ctg cgg cac 2304
Ala Pro Ile Ser Tyr Arg Leu Ala Glu Gln Gln Pro Leu Leu Arg His
        755                 760                 765

ccg gcg ccc ccc ggc ccg gga ccc gga ccc ggg ccc ggg ccc ggg ccc 2352
Pro Ala Pro Pro Gly Pro Gly Pro Gly Pro Gly Pro Gly Pro Gly Pro
    770                 775                 780

ggc gca gac atg cag cgc agc tat gac agc tac tat tac ccc gcg gcg 2400
Gly Ala Asp Met Gln Arg Ser Tyr Asp Ser Tyr Tyr Tyr Pro Ala Ala
785                 790                 795                 800

ggg ccc gga ccg cgg cgc ggg acc tgc gcg ctc ggc ggc agc ctg ggc 2448
Gly Pro Gly Pro Arg Arg Gly Thr Cys Ala Leu Gly Gly Ser Leu Gly
                805                 810                 815

agc ctg cct gcc agc ccc ttc cgc atc ccc gag gac gac gag tac gag 2496
Ser Leu Pro Ala Ser Pro Phe Arg Ile Pro Glu Asp Asp Glu Tyr Glu
            820                 825                 830

acc acg cag gag tgc gcg ccc ccg ccg ccg ccg cgg ccg cgc gcg cgc 2544
Thr Thr Gln Glu Cys Ala Pro Pro Pro Pro Pro Arg Pro Arg Ala Arg
        835                 840                 845

ggt gcg tcc cgc agg acg tcg gcg ggg ccc cgg cgc tgg cgc cgc tcg 2592
Gly Ala Ser Arg Arg Thr Ser Ala Gly Pro Arg Arg Trp Arg Arg Ser
    850                 855                 860

cgc ctc aac ggg ctg gcg gcg cag cgc gca cgg gcg gcg agg gac tcg 2640
Arg Leu Asn Gly Leu Ala Ala Gln Arg Ala Arg Ala Ala Arg Asp Ser
865                 870                 875                 880

ctg tcg ctg agc agc ggc tcg ggc ggc ggc tca gcc tcg gcg tcg gac 2688
Leu Ser Leu Ser Ser Gly Ser Gly Gly Gly Ser Ala Ser Ala Ser Asp
                885                 890                 895

gac gac gcg gac gac gcg gac ggg gcg ctg gcg gcc gag agc aca cct 2736
Asp Asp Ala Asp Asp Ala Asp Gly Ala Leu Ala Ala Glu Ser Thr Pro
            900                 905                 910

ttc ctg ggc ctg cgt ggg gcg cac gac gcg ctg cgc tcg gac tcg ccg 2784

Phe Leu Gly Leu Arg Gly Ala His Asp Ala Leu Arg Ser Asp Ser Pro
        915                 920                 925

cca ctg tgc ccg gcg gcc gac agc agg act tac tac tca ctg gac agc      2832
Pro Leu Cys Pro Ala Ala Asp Ser Arg Thr Tyr Tyr Ser Leu Asp Ser
    930                 935                 940

cac agc acg cgg gcc agc agc aga cac agc cgc ggg ccg ccc ccg cgg      2880
His Ser Thr Arg Ala Ser Ser Arg His Ser Arg Gly Pro Pro Pro Arg
945                 950                 955                 960

gcc aag cag gac tcg gcg cca ctc tag                                  2907
Ala Lys Gln Asp Ser Ala Pro Leu
                965

<210>  26
<211>  968
<212>  PRT
<213>  Homo sapiens

<400>  26

Met Glu Pro Arg Pro Thr Ala Pro Ser Ser Gly Ala Pro Gly Leu Ala
1               5                   10                  15

Gly Val Gly Glu Thr Pro Ser Ala Ala Ala Leu Ala Ala Ala Arg Val
            20                  25                  30

Glu Leu Pro Gly Thr Ala Val Pro Ser Val Pro Glu Asp Ala Ala Pro
        35                  40                  45

Ala Ser Arg Asp Gly Gly Gly Val Arg Asp Glu Gly Pro Ala Ala Ala
    50                  55                  60

Gly Asp Gly Leu Gly Arg Pro Leu Gly Pro Thr Pro Ser Gln Ser Arg
65                  70                  75                  80

Phe Gln Val Asp Leu Val Ser Glu Asn Ala Gly Arg Ala Ala Ala Ala
                85                  90                  95

Ala Ala Ala Ala Ala Ala Ala Ala Ala Ala Ala Gly Ala Gly Ala Gly
            100                 105                 110

Ala Lys Gln Thr Pro Ala Asp Gly Glu Ala Ser Gly Glu Ser Glu Pro
        115                 120                 125

Ala Lys Gly Ser Glu Glu Ala Lys Gly Arg Phe Arg Val Asn Phe Val
    130                 135                 140

Asp Pro Ala Ala Ser Ser Ser Ala Glu Asp Ser Leu Ser Asp Ala Ala
145                 150                 155                 160

Gly Val Gly Val Asp Gly Pro Asn Val Ser Phe Gln Asn Gly Gly Asp
                165                 170                 175

Thr Val Leu Ser Glu Gly Ser Ser Leu His Ser Gly Gly Gly Gly Gly
            180                 185                 190

Ser Gly His His Gln His Tyr Tyr Tyr Asp Thr His Thr Asn Thr Tyr
        195                 200                 205

Tyr Leu Arg Thr Phe Gly His Asn Thr Met Asp Ala Val Pro Arg Ile
    210                 215                 220

Asp His Tyr Arg His Thr Ala Ala Gln Leu Gly Glu Lys Leu Leu Arg
225                 230                 235                 240

Pro Ser Leu Ala Glu Leu His Asp Glu Leu Glu Lys Glu Pro Phe Glu
                245                 250                 255

Asp Gly Phe Ala Asn Gly Glu Glu Ser Thr Pro Thr Arg Asp Ala Val
            260                 265                 270

Val Thr Tyr Thr Ala Glu Ser Lys Gly Val Val Lys Phe Gly Trp Ile
        275                 280                 285

Lys Gly Val Leu Val Arg Cys Met Leu Asn Ile Trp Gly Val Met Leu
    290                 295                 300

Phe Ile Arg Leu Ser Trp Ile Val Gly Gln Ala Gly Ile Gly Leu Ser
305                 310                 315                 320

Val Leu Val Ile Met Met Ala Thr Val Val Thr Thr Ile Thr Gly Leu
                325                 330                 335

Ser Thr Ser Ala Ile Ala Thr Asn Gly Phe Val Arg Gly Ala Thr Arg
            340                 345                 350

Pro Lys Leu Lys Lys Met Lys Ser Gln Thr Gly Gln Val Gly Glu Lys
        355                 360                 365

Gln Ser Leu Lys Cys Glu Ala Ala Ala Gly Asn Pro Gln Pro Ser Tyr
    370                 375                 380

Arg Trp Phe Lys Asp Gly Lys Glu Leu Asn Arg Ser Arg Asp Ile Arg
385                 390                 395                 400

Ile Lys Tyr Gly Asn Gly Arg Lys Asn Ser Arg Leu Gln Phe Asn Lys
                405                 410                 415

Val Lys Val Glu Asp Ala Gly Glu Tyr Val Cys Glu Ala Glu Asn Ile
            420                 425                 430

Leu Gly Lys Asp Thr Val Arg Gly Arg Leu Tyr Val Asn Ser Val Ser
        435                 440                 445

Thr Thr Leu Ser Ser Trp Ser Gly His Ala Arg Lys Cys Asn Glu Thr
    450                 455                 460

Ala Lys Ser Tyr Cys Val Asn Gly Gly Val Cys Tyr Tyr Ile Glu Gly
465                 470                 475                 480

Ile Asn Gln Leu Ser Cys Lys Cys Pro Val Gly Tyr Thr Gly Asp Arg
                485                 490                 495

Cys Gln Gln Phe Ala Met Val Asn Phe Ser Lys His Leu Gly Phe Glu
            500                 505                 510

Leu Lys Glu Ala Glu Glu Leu Tyr Gln Lys Arg Val Leu Thr Ile Thr
        515                 520                 525

Gly Ile Cys Val Ala Leu Leu Val Val Gly Ile Val Cys Val Val Ala
    530                 535                 540

Tyr Cys Lys Thr Lys Lys Gln Arg Lys Gln Met His Asn His Leu Arg
545                 550                 555                 560

Gln Asn Met Cys Pro Ala His Gln Asn Arg Ser Leu Ala Asn Gly Pro
                565                 570                 575

Ser His Pro Arg Leu Asp Pro Glu Glu Ile Gln Met Ala Asp Tyr Ile
            580                 585                 590

Ser Lys Asn Val Pro Ala Thr Asp His Val Ile Arg Arg Glu Thr Glu
        595                 600                 605

Thr Thr Phe Ser Gly Ser His Ser Cys Ser Pro Ser His His Cys Ser
    610                 615                 620

Thr Ala Thr Pro Thr Ser Ser His Arg His Glu Ser His Thr Trp Ser
625                 630                 635                 640

Leu Glu Arg Ser Glu Ser Leu Thr Ser Asp Ser Gln Ser Gly Ile Met
                645                 650                 655

Leu Ser Ser Val Gly Thr Ser Lys Cys Asn Ser Pro Ala Cys Val Glu
            660                 665                 670

Ala Arg Ala Arg Arg Ala Ala Ala Tyr Asn Leu Glu Glu Arg Arg Arg
        675                 680                 685

Ala Thr Ala Pro Pro Tyr His Asp Ser Val Asp Ser Leu Arg Asp Ser
    690                 695                 700

Pro His Ser Glu Arg Tyr Val Ser Ala Leu Thr Thr Pro Ala Arg Leu
705                 710                 715                 720

Ser Pro Val Asp Phe His Tyr Ser Leu Ala Thr Gln Val Pro Thr Phe
                725                 730                 735

Glu Ile Thr Ser Pro Asn Ser Ala His Ala Val Ser Leu Pro Pro Ala
            740                 745                 750

Ala Pro Ile Ser Tyr Arg Leu Ala Glu Gln Gln Pro Leu Leu Arg His
        755                 760                 765

Pro Ala Pro Pro Gly Pro Gly Pro Gly Pro Gly Pro Gly Pro Gly Pro
    770                 775                 780

Gly Ala Asp Met Gln Arg Ser Tyr Asp Ser Tyr Tyr Tyr Pro Ala Ala
785                 790                 795                 800

Gly Pro Gly Pro Arg Arg Gly Thr Cys Ala Leu Gly Gly Ser Leu Gly
                805                 810                 815

Ser Leu Pro Ala Ser Pro Phe Arg Ile Pro Glu Asp Asp Glu Tyr Glu
            820                 825                 830

Thr Thr Gln Glu Cys Ala Pro Pro Pro Pro Pro Arg Pro Arg Ala Arg
        835                 840                 845

Gly Ala Ser Arg Arg Thr Ser Ala Gly Pro Arg Arg Trp Arg Arg Ser
    850                 855                 860

Arg Leu Asn Gly Leu Ala Ala Gln Arg Ala Arg Ala Ala Arg Asp Ser
865                 870                 875                 880

Leu Ser Leu Ser Ser Gly Ser Gly Gly Gly Ser Ala Ser Ala Ser Asp
                885                 890                 895

Asp Asp Ala Asp Asp Ala Asp Gly Ala Leu Ala Ala Glu Ser Thr Pro
            900                 905                 910

Phe Leu Gly Leu Arg Gly Ala His Asp Ala Leu Arg Ser Asp Ser Pro

915                 920                 925

Pro Leu Cys Pro Ala Ala Asp Ser Arg Thr Tyr Tyr Ser Leu Asp Ser
    930                 935                 940

His Ser Thr Arg Ala Ser Ser Arg His Ser Arg Gly Pro Pro Pro Arg
945                 950                 955                 960

Ala Lys Gln Asp Ser Ala Pro Leu
                965

<210>  27
<211>  2703
<212>  DNA
<213>  Homo sapiens

<220>
<221>  CDS
<222>  (1)..(2703)

<400>  27
atg gag ccg cgg ccc acg gcg ccc tcc tcc ggc gcc ccg gga ctg gcc       48
Met Glu Pro Arg Pro Thr Ala Pro Ser Ser Gly Ala Pro Gly Leu Ala
1               5                   10                  15

ggg gtc ggg gag acg ccg tca gcc gct gcg ctg gcc gca gcc agg gtg       96
Gly Val Gly Glu Thr Pro Ser Ala Ala Ala Leu Ala Ala Ala Arg Val
            20                  25                  30

gaa ctg ccc ggc acg gct gtg ccc tcg gtg ccg gag gat gct gcg ccc      144
Glu Leu Pro Gly Thr Ala Val Pro Ser Val Pro Glu Asp Ala Ala Pro
        35                  40                  45

gcg agc cgg gac ggc ggc ggg gtc cgc gat gag ggc ccc gcg gcg gcc      192
Ala Ser Arg Asp Gly Gly Gly Val Arg Asp Glu Gly Pro Ala Ala Ala
    50                  55                  60

ggg gac ggg ctg ggc aga ccc ttg ggg ccc acc ccg agc cag agc cgt      240
Gly Asp Gly Leu Gly Arg Pro Leu Gly Pro Thr Pro Ser Gln Ser Arg
65                  70                  75                  80

ttc cag gtg gac ctg gtt tcc gag aac gcc ggg cgg gcc gct gct gcg      288
Phe Gln Val Asp Leu Val Ser Glu Asn Ala Gly Arg Ala Ala Ala Ala
                85                  90                  95

gcg gcg gcg gcg gcg gcg gca gcg gcg gcg gct ggt gct ggg gcg ggg      336
Ala Ala Ala Ala Ala Ala Ala Ala Ala Ala Ala Gly Ala Gly Ala Gly
            100                 105                 110

gcc aag cag acc ccc gcg gac ggg gaa gcc agc ggc gag agc gag ccg      384
Ala Lys Gln Thr Pro Ala Asp Gly Glu Ala Ser Gly Glu Ser Glu Pro
        115                 120                 125

gct aaa ggc agc gag gaa gcc aag ggc cgc ttc cgc gtg aac ttc gtg      432
Ala Lys Gly Ser Glu Glu Ala Lys Gly Arg Phe Arg Val Asn Phe Val
    130                 135                 140

gac cca gct gcc tcc tcg tcg gct gaa gac agc ctg tca gat gct gcc      480

Asp Pro Ala Ala Ser Ser Ser Ala Glu Asp Ser Leu Ser Asp Ala Ala
145                 150                 155                 160

ggg gtc gga gtc gac ggg ccc aac gtg agc ttc cag aac ggc ggg gac     528
Gly Val Gly Val Asp Gly Pro Asn Val Ser Phe Gln Asn Gly Gly Asp
                165                 170                 175

acg gtg ctg agc gag ggc agc agc ctg cac tcc ggc ggc ggc ggc ggc     576
Thr Val Leu Ser Glu Gly Ser Ser Leu His Ser Gly Gly Gly Gly Gly
            180                 185                 190

agt ggg cac cac cag cac tac tat tat gat acc cac acc aac acc tac     624
Ser Gly His His Gln His Tyr Tyr Tyr Asp Thr His Thr Asn Thr Tyr
        195                 200                 205

tac ctg cgc acc ttc ggc cac aac acc atg gac gct gtg ccc agg atc     672
Tyr Leu Arg Thr Phe Gly His Asn Thr Met Asp Ala Val Pro Arg Ile
    210                 215                 220

gat cac tac cgg cac aca gcc gcg cag ctg ggc gag aag ctg ctc cgg     720
Asp His Tyr Arg His Thr Ala Ala Gln Leu Gly Glu Lys Leu Leu Arg
225                 230                 235                 240

cct agc ctg gcg gag ctc cac gac gag ctg gaa aag gaa cct ttt gag     768
Pro Ser Leu Ala Glu Leu His Asp Glu Leu Glu Lys Glu Pro Phe Glu
                245                 250                 255

gat ggc ttt gca aat ggg gaa gaa agt act cca acc aga gat gct gtg     816
Asp Gly Phe Ala Asn Gly Glu Glu Ser Thr Pro Thr Arg Asp Ala Val
            260                 265                 270

gtc acg tat act gca gaa agt aaa gga gtc gtg aag ttt ggc tgg atc     864
Val Thr Tyr Thr Ala Glu Ser Lys Gly Val Val Lys Phe Gly Trp Ile
        275                 280                 285

aag ggt gta tta gta cgt tgt atg tta aac att tgg ggt gtg atg ctt     912
Lys Gly Val Leu Val Arg Cys Met Leu Asn Ile Trp Gly Val Met Leu
    290                 295                 300

ttc att aga ttg tca tgg att gtg ggt caa gct gga ata ggt cta tca     960
Phe Ile Arg Leu Ser Trp Ile Val Gly Gln Ala Gly Ile Gly Leu Ser
305                 310                 315                 320

gtc ctt gta ata atg atg gcc act gtt gtg aca act atc aca gga ttg     1008
Val Leu Val Ile Met Met Ala Thr Val Val Thr Thr Ile Thr Gly Leu
                325                 330                 335

tct act tca gca ata gca act aat gga ttt gta aga gga gcc acc cgg     1056
Ser Thr Ser Ala Ile Ala Thr Asn Gly Phe Val Arg Gly Ala Thr Arg
            340                 345                 350

ccc aag ttg aag aag atg aag agc cag acg gga cag gtg ggt gag aag     1104
Pro Lys Leu Lys Lys Met Lys Ser Gln Thr Gly Gln Val Gly Glu Lys
        355                 360                 365

caa tcg ctg aag tgt gag gca gca gcc ggt aat ccc cag cct tcc tac     1152
Gln Ser Leu Lys Cys Glu Ala Ala Ala Gly Asn Pro Gln Pro Ser Tyr
    370                 375                 380

cgt tgg ttc aag gat ggc aag gag ctc aac cgc agc cga gac att cgc     1200
Arg Trp Phe Lys Asp Gly Lys Glu Leu Asn Arg Ser Arg Asp Ile Arg
385                 390                 395                 400

atc aaa tat ggc aac ggc aga aag aac tca cga cta cag ttc aac aag 1248
Ile Lys Tyr Gly Asn Gly Arg Lys Asn Ser Arg Leu Gln Phe Asn Lys
                405                 410                 415

gtg aag gtg gag gac gct ggg gag tat gtc tgc gag gcc gag aac atc 1296
Val Lys Val Glu Asp Ala Gly Glu Tyr Val Cys Glu Ala Glu Asn Ile
            420                 425                 430

ctg ggg aag gac acc gtc cgg ggc cgg ctt tac gtc aac agc gaa gcc 1344
Leu Gly Lys Asp Thr Val Arg Gly Arg Leu Tyr Val Asn Ser Glu Ala
        435                 440                 445

gag gag ctg tac cag aag agg gtc ctg acc atc acg ggc atc tgc gtg 1392
Glu Glu Leu Tyr Gln Lys Arg Val Leu Thr Ile Thr Gly Ile Cys Val
    450                 455                 460

gct ctg ctg gtc gtg ggc atc gtc tgt gtg gtg gcc tac tgc aag acc 1440
Ala Leu Leu Val Val Gly Ile Val Cys Val Val Ala Tyr Cys Lys Thr
465                 470                 475                 480

aaa aaa cag cgg aag cag atg cac aac cac ctc cgg cag aac atg tgc 1488
Lys Lys Gln Arg Lys Gln Met His Asn His Leu Arg Gln Asn Met Cys
                485                 490                 495

ccg gcc cat cag aac cgg agc ttg gcc aat ggg ccc agc cac ccc cgg 1536
Pro Ala His Gln Asn Arg Ser Leu Ala Asn Gly Pro Ser His Pro Arg
            500                 505                 510

ctg gac cca gag gag atc cag atg gca gat tat att tcc aag aac gtg 1584
Leu Asp Pro Glu Glu Ile Gln Met Ala Asp Tyr Ile Ser Lys Asn Val
        515                 520                 525

cca gcc aca gac cat gtc atc agg aga gaa act gag acc acc ttc tct 1632
Pro Ala Thr Asp His Val Ile Arg Arg Glu Thr Glu Thr Thr Phe Ser
    530                 535                 540

ggg agc cac tcc tgt tct cct tct cac cac tgc tcc aca gcc aca ccc 1680
Gly Ser His Ser Cys Ser Pro Ser His His Cys Ser Thr Ala Thr Pro
545                 550                 555                 560

acc tcc agc cac aga cac gag agc cac acg tgg agc ctg gaa cgt tct 1728
Thr Ser Ser His Arg His Glu Ser His Thr Trp Ser Leu Glu Arg Ser
                565                 570                 575

gag agc ctg act tct gac tcc cag tcg ggg atc atg cta tca tca gtg 1776
Glu Ser Leu Thr Ser Asp Ser Gln Ser Gly Ile Met Leu Ser Ser Val
            580                 585                 590

ggt acc agc aaa tgc aac agc cca gca tgt gtg gag gcc cgg gca agg 1824
Gly Thr Ser Lys Cys Asn Ser Pro Ala Cys Val Glu Ala Arg Ala Arg
        595                 600                 605

cgg gca gca gcc tac aac ctg gag gag cgg cgc agg gcc acc gcg cca 1872
Arg Ala Ala Ala Tyr Asn Leu Glu Glu Arg Arg Arg Ala Thr Ala Pro
    610                 615                 620

ccc tat cac gat tcc gtg gac tcc ctt cgc gac tcc cca cac agc gag 1920
Pro Tyr His Asp Ser Val Asp Ser Leu Arg Asp Ser Pro His Ser Glu
625                 630                 635                 640

agg tac gtg tcg gcc ctg acc acg ccc gcg cgc ctc tcg ccc gtg gac 1968
Arg Tyr Val Ser Ala Leu Thr Thr Pro Ala Arg Leu Ser Pro Val Asp
                645                 650                 655

ttc cac tac tcg ctg gcc acg cag gtg cca act ttc gag atc acg tcc     2016
Phe His Tyr Ser Leu Ala Thr Gln Val Pro Thr Phe Glu Ile Thr Ser
            660                 665                 670

ccc aac tcg gcg cac gcc gtg tcg ctg ccg ccg gcg gcg ccc atc agt     2064
Pro Asn Ser Ala His Ala Val Ser Leu Pro Pro Ala Ala Pro Ile Ser
        675                 680                 685

tac cgc ctg gcc gag cag cag ccg tta ctg cgg cac ccg gcg ccc ccc     2112
Tyr Arg Leu Ala Glu Gln Gln Pro Leu Leu Arg His Pro Ala Pro Pro
    690                 695                 700

ggc ccg gga ccc gga ccc ggg ccc ggg ccc ggg ccc ggc gca gac atg     2160
Gly Pro Gly Pro Gly Pro Gly Pro Gly Pro Gly Pro Gly Ala Asp Met
705                 710                 715                 720

cag cgc agc tat gac agc tac tat tac ccc gcg gcg ggg ccc gga ccg     2208
Gln Arg Ser Tyr Asp Ser Tyr Tyr Tyr Pro Ala Ala Gly Pro Gly Pro
                725                 730                 735

cgg cgc ggg acc tgc gcg ctc ggc ggc agc ctg ggc agc ctg cct gcc     2256
Arg Arg Gly Thr Cys Ala Leu Gly Gly Ser Leu Gly Ser Leu Pro Ala
            740                 745                 750

agc ccc ttc cgc atc ccc gag gac gac gag tac gag acc acg cag gag     2304
Ser Pro Phe Arg Ile Pro Glu Asp Asp Glu Tyr Glu Thr Thr Gln Glu
        755                 760                 765

tgc gcg ccc ccg ccg ccg ccg cgg ccg cgc gcg cgc ggt gcg tcc cgc     2352
Cys Ala Pro Pro Pro Pro Pro Arg Pro Arg Ala Arg Gly Ala Ser Arg
    770                 775                 780

agg acg tcg gcg ggg ccc cgg cgc tgg cgc cgc tcg cgc ctc aac ggg     2400
Arg Thr Ser Ala Gly Pro Arg Arg Trp Arg Arg Ser Arg Leu Asn Gly
785                 790                 795                 800

ctg gcg gcg cag cgc gca cgg gcg gcg agg gac tcg ctg tcg ctg agc     2448
Leu Ala Ala Gln Arg Ala Arg Ala Ala Arg Asp Ser Leu Ser Leu Ser
                805                 810                 815

agc ggc tcg ggc ggc ggc tca gcc tcg gcg tcg gac gac gac gcg gac     2496
Ser Gly Ser Gly Gly Gly Ser Ala Ser Ala Ser Asp Asp Asp Ala Asp
            820                 825                 830

gac gcg gac ggg gcg ctg gcg gcc gag agc aca cct ttc ctg ggc ctg     2544
Asp Ala Asp Gly Ala Leu Ala Ala Glu Ser Thr Pro Phe Leu Gly Leu
        835                 840                 845

cgt ggg gcg cac gac gcg ctg cgc tcg gac tcg ccg cca ctg tgc ccg     2592
Arg Gly Ala His Asp Ala Leu Arg Ser Asp Ser Pro Pro Leu Cys Pro
    850                 855                 860

gcg gcc gac agc agg act tac tac tca ctg gac agc cac agc acg cgg     2640
Ala Ala Asp Ser Arg Thr Tyr Tyr Ser Leu Asp Ser His Ser Thr Arg
865                 870                 875                 880

gcc agc agc aga cac agc cgc ggg ccg ccc ccg cgg gcc aag cag gac     2688
Ala Ser Ser Arg His Ser Arg Gly Pro Pro Pro Arg Ala Lys Gln Asp
                885                 890                 895

tcg gcg cca ctc tag                                                 2703
Ser Ala Pro Leu

            900

<210>  28
<211>  900
<212>  PRT
<213>  Homo sapiens

<400>  28

Met Glu Pro Arg Pro Thr Ala Pro Ser Ser Gly Ala Pro Gly Leu Ala
1               5                   10                  15

Gly Val Gly Glu Thr Pro Ser Ala Ala Ala Leu Ala Ala Ala Arg Val
            20                  25                  30

Glu Leu Pro Gly Thr Ala Val Pro Ser Val Pro Glu Asp Ala Ala Pro
        35                  40                  45

Ala Ser Arg Asp Gly Gly Gly Val Arg Asp Glu Gly Pro Ala Ala Ala
    50                  55                  60

Gly Asp Gly Leu Gly Arg Pro Leu Gly Pro Thr Pro Ser Gln Ser Arg
65                  70                  75                  80

Phe Gln Val Asp Leu Val Ser Glu Asn Ala Gly Arg Ala Ala Ala Ala
                85                  90                  95

Ala Ala Ala Ala Ala Ala Ala Ala Ala Ala Ala Gly Ala Gly Ala Gly
            100                 105                 110

Ala Lys Gln Thr Pro Ala Asp Gly Glu Ala Ser Gly Glu Ser Glu Pro
        115                 120                 125

Ala Lys Gly Ser Glu Glu Ala Lys Gly Arg Phe Arg Val Asn Phe Val
    130                 135                 140

Asp Pro Ala Ala Ser Ser Ser Ala Glu Asp Ser Leu Ser Asp Ala Ala
145                 150                 155                 160

Gly Val Gly Val Asp Gly Pro Asn Val Ser Phe Gln Asn Gly Gly Asp
                165                 170                 175

Thr Val Leu Ser Glu Gly Ser Ser Leu His Ser Gly Gly Gly Gly Gly
            180                 185                 190

Ser Gly His His Gln His Tyr Tyr Tyr Asp Thr His Thr Asn Thr Tyr
        195                 200                 205

Tyr Leu Arg Thr Phe Gly His Asn Thr Met Asp Ala Val Pro Arg Ile

210                 215                 220

Asp His Tyr Arg His Thr Ala Ala Gln Leu Gly Glu Lys Leu Leu Arg
225                 230                 235                 240

Pro Ser Leu Ala Glu Leu His Asp Glu Leu Glu Lys Glu Pro Phe Glu
                245                 250                 255

Asp Gly Phe Ala Asn Gly Glu Glu Ser Thr Pro Thr Arg Asp Ala Val
            260                 265                 270

Val Thr Tyr Thr Ala Glu Ser Lys Gly Val Val Lys Phe Gly Trp Ile
        275                 280                 285

Lys Gly Val Leu Val Arg Cys Met Leu Asn Ile Trp Gly Val Met Leu
    290                 295                 300

Phe Ile Arg Leu Ser Trp Ile Val Gly Gln Ala Gly Ile Gly Leu Ser
305                 310                 315                 320

Val Leu Val Ile Met Met Ala Thr Val Val Thr Thr Ile Thr Gly Leu
                325                 330                 335

Ser Thr Ser Ala Ile Ala Thr Asn Gly Phe Val Arg Gly Ala Thr Arg
            340                 345                 350

Pro Lys Leu Lys Lys Met Lys Ser Gln Thr Gly Gln Val Gly Glu Lys
        355                 360                 365

Gln Ser Leu Lys Cys Glu Ala Ala Ala Gly Asn Pro Gln Pro Ser Tyr
    370                 375                 380

Arg Trp Phe Lys Asp Gly Lys Glu Leu Asn Arg Ser Arg Asp Ile Arg
385                 390                 395                 400

Ile Lys Tyr Gly Asn Gly Arg Lys Asn Ser Arg Leu Gln Phe Asn Lys
                405                 410                 415

Val Lys Val Glu Asp Ala Gly Glu Tyr Val Cys Glu Ala Glu Asn Ile
            420                 425                 430

Leu Gly Lys Asp Thr Val Arg Gly Arg Leu Tyr Val Asn Ser Glu Ala
        435                 440                 445

Glu Glu Leu Tyr Gln Lys Arg Val Leu Thr Ile Thr Gly Ile Cys Val
    450                 455                 460

Ala Leu Leu Val Val Gly Ile Val Cys Val Val Ala Tyr Cys Lys Thr
465                 470                 475                 480

Lys Lys Gln Arg Lys Gln Met His Asn His Leu Arg Gln Asn Met Cys
                485                 490                 495

Pro Ala His Gln Asn Arg Ser Leu Ala Asn Gly Pro Ser His Pro Arg
            500                 505                 510

Leu Asp Pro Glu Glu Ile Gln Met Ala Asp Tyr Ile Ser Lys Asn Val
        515                 520                 525

Pro Ala Thr Asp His Val Ile Arg Arg Glu Thr Glu Thr Thr Phe Ser
    530                 535                 540

Gly Ser His Ser Cys Ser Pro Ser His His Cys Ser Thr Ala Thr Pro
545                 550                 555                 560

Thr Ser Ser His Arg His Glu Ser His Thr Trp Ser Leu Glu Arg Ser
                565                 570                 575

Glu Ser Leu Thr Ser Asp Ser Gln Ser Gly Ile Met Leu Ser Ser Val
            580                 585                 590

Gly Thr Ser Lys Cys Asn Ser Pro Ala Cys Val Glu Ala Arg Ala Arg
        595                 600                 605

Arg Ala Ala Ala Tyr Asn Leu Glu Glu Arg Arg Arg Ala Thr Ala Pro
    610                 615                 620

Pro Tyr His Asp Ser Val Asp Ser Leu Arg Asp Ser Pro His Ser Glu
625                 630                 635                 640

Arg Tyr Val Ser Ala Leu Thr Thr Pro Ala Arg Leu Ser Pro Val Asp
                645                 650                 655

Phe His Tyr Ser Leu Ala Thr Gln Val Pro Thr Phe Glu Ile Thr Ser
            660                 665                 670

Pro Asn Ser Ala His Ala Val Ser Leu Pro Pro Ala Ala Pro Ile Ser
        675                 680                 685

Tyr Arg Leu Ala Glu Gln Gln Pro Leu Leu Arg His Pro Ala Pro Pro
    690                 695                 700

Gly Pro Gly Pro Gly Pro Gly Pro Gly Pro Gly Pro Gly Ala Asp Met
705                 710                 715                 720

Gln Arg Ser Tyr Asp Ser Tyr Tyr Tyr Pro Ala Ala Gly Pro Gly Pro
                725                 730                 735

Arg Arg Gly Thr Cys Ala Leu Gly Gly Ser Leu Gly Ser Leu Pro Ala
            740                 745                 750

Ser Pro Phe Arg Ile Pro Glu Asp Asp Glu Tyr Glu Thr Thr Gln Glu
        755                 760                 765

Cys Ala Pro Pro Pro Pro Pro Arg Pro Arg Ala Arg Gly Ala Ser Arg
    770                 775                 780

Arg Thr Ser Ala Gly Pro Arg Arg Trp Arg Arg Ser Arg Leu Asn Gly
785                 790                 795                 800

Leu Ala Ala Gln Arg Ala Arg Ala Ala Arg Asp Ser Leu Ser Leu Ser
                805                 810                 815

Ser Gly Ser Gly Gly Gly Ser Ala Ser Ala Ser Asp Asp Asp Ala Asp
            820                 825                 830

Asp Ala Asp Gly Ala Leu Ala Ala Glu Ser Thr Pro Phe Leu Gly Leu
        835                 840                 845

Arg Gly Ala His Asp Ala Leu Arg Ser Asp Ser Pro Pro Leu Cys Pro
    850                 855                 860

Ala Ala Asp Ser Arg Thr Tyr Tyr Ser Leu Asp Ser His Ser Thr Arg
865                 870                 875                 880

Ala Ser Ser Arg His Ser Arg Gly Pro Pro Pro Arg Ala Lys Gln Asp
                885                 890                 895

Ser Ala Pro Leu
            900

<210>  29
<211>  1572
<212>  DNA
<213>  Homo sapiens

<220>
<221>  CDS
<222>  (436)..(1380)

<400>  29
tagtgtcacg tgaggccccg gtggcggcgc agctacggca agagagtgag aaggaaggga      60

agccggaagg ggcgcgagtg aagcaaagcg aggacagaca gctcgcagag ggcgaggggt     120

gcgtgtgcgt ccgcttctca cctcaggtct cccttcggcc ccgctgccct ccctcgcggc 180

tgggtgacag ctgggtccgg tccgtcgcgg gctgcctggg gtgcgaggat cgcgcacccc 240

gtcttcgcgc gctgtgcctg ccgccccgcc ccctcgtccc gcccgtcccg tcgcgtcgcg 300

tcccgtcccc tcgggtgctg ccagccgggt gctgatgcga gtcggtggca gcgaggacat 360

tttctgactc cctggcccct gacacggctg cactttccat cccgtcgcgg ggccggccgc 420

tactccggcc ccagg atg cag aat gtg att aat act gtg aag gga aag gca 471
Met Gln Asn Val Ile Asn Thr Val Lys Gly Lys Ala
1               5                   10

ctg gaa gtg gct gag tac ctg acc ccg gtc ctc aag gaa tca aag ttt 519
Leu Glu Val Ala Glu Tyr Leu Thr Pro Val Leu Lys Glu Ser Lys Phe
        15                  20                  25

aag gaa aca ggt gta att acc cca gaa gag ttt gtg gca gct gga gat 567
Lys Glu Thr Gly Val Ile Thr Pro Glu Glu Phe Val Ala Ala Gly Asp
    30                  35                  40

cac cta gtc cac cac tgt cca aca tgg caa tgg gct aca ggg gaa gaa 615
His Leu Val His His Cys Pro Thr Trp Gln Trp Ala Thr Gly Glu Glu
45                  50                  55                  60

ttg aaa gtg aag gca tac cta cca aca ggc aaa caa ttt ttg gta acc 663
Leu Lys Val Lys Ala Tyr Leu Pro Thr Gly Lys Gln Phe Leu Val Thr
                65                  70                  75

aaa aat gtg ccg tgc tat aag cgg tgc aaa cag atg gaa tat tca gat 711
Lys Asn Val Pro Cys Tyr Lys Arg Cys Lys Gln Met Glu Tyr Ser Asp
            80                  85                  90

gaa ttg gaa gct atc att gaa gaa gat gat ggt gat ggc gga tgg gta 759
Glu Leu Glu Ala Ile Ile Glu Glu Asp Asp Gly Asp Gly Gly Trp Val
        95                  100                 105

gat aca tat cac aac aca ggt att aca gga ata acg gaa gcc gtt aaa 807
Asp Thr Tyr His Asn Thr Gly Ile Thr Gly Ile Thr Glu Ala Val Lys
    110                 115                 120

gag atc aca ctg gaa aat aag gac aat ata agg ctt caa gat tgc tca 855
Glu Ile Thr Leu Glu Asn Lys Asp Asn Ile Arg Leu Gln Asp Cys Ser
125                 130                 135                 140

gca cta tgt gaa gag gaa gaa gat gaa gat gaa gga gaa gct gca gat 903
Ala Leu Cys Glu Glu Glu Glu Asp Glu Asp Glu Gly Glu Ala Ala Asp
                145                 150                 155

atg gaa gaa tat gaa gag agt gga ttg ttg gaa aca gat gag gct acc 951
Met Glu Glu Tyr Glu Glu Ser Gly Leu Leu Glu Thr Asp Glu Ala Thr
            160                 165                 170

cta gat aca agg aaa ata gta gaa gct tgt aaa gcc aaa act gat gct 999
Leu Asp Thr Arg Lys Ile Val Glu Ala Cys Lys Ala Lys Thr Asp Ala
        175                 180                 185

ggc ggt gaa gat gct att ttg caa acc aga act tat gac ctt tac atc 1047
Gly Gly Glu Asp Ala Ile Leu Gln Thr Arg Thr Tyr Asp Leu Tyr Ile
    190                 195                 200

act tat gat aaa tat tac cag act cca cga tta tgg ttg ttt ggc tat 1095
Thr Tyr Asp Lys Tyr Tyr Gln Thr Pro Arg Leu Trp Leu Phe Gly Tyr
205                 210                 215                 220

gat gag caa cgg cag cct tta aca gtt gag cac atg tat gaa gac atc 1143
Asp Glu Gln Arg Gln Pro Leu Thr Val Glu His Met Tyr Glu Asp Ile
                225                 230                 235

agt cag gat cat gtg aag aaa aca gtg acc att gaa aat cac cct cat 1191
Ser Gln Asp His Val Lys Lys Thr Val Thr Ile Glu Asn His Pro His
            240                 245                 250

ctg cca cca cct ccc atg tgt tca gtt cac cca tgc agg cat gct gag 1239
Leu Pro Pro Pro Pro Met Cys Ser Val His Pro Cys Arg His Ala Glu
        255                 260                 265

gtg atg aag aaa atc att gag act gtt gca gaa gga ggg gga gaa ctt 1287
Val Met Lys Lys Ile Ile Glu Thr Val Ala Glu Gly Gly Gly Glu Leu
    270                 275                 280

gga gtt cat atg tat ctt ctt att ttc ttg aaa ttt gta caa gct gtc 1335
Gly Val His Met Tyr Leu Leu Ile Phe Leu Lys Phe Val Gln Ala Val
285                 290                 295                 300

att cca aca ata gaa tat gac tac aca aga cac ttc aca atg taa 1380
Ile Pro Thr Ile Glu Tyr Asp Tyr Thr Arg His Phe Thr Met
                305                 310

tgaagagagc ataaaatcta tcctaattat tggttctgat ttttaaagaa ttaacccata 1440

gatgtgacca ttgaccatat tcatcaatat atacagtttc tctaataagg gacttatatg 1500

tttatgcatt aaataaaaat atgttccact accagcctta cttgtttaat aaaaatcagt 1560

gcaaagagag tt 1572

<210> 30
<211> 314
<212> PRT
<213> Homo sapiens

<400> 30

Met Gln Asn Val Ile Asn Thr Val Lys Gly Lys Ala Leu Glu Val Ala
1               5                   10                  15

Glu Tyr Leu Thr Pro Val Leu Lys Glu Ser Lys Phe Lys Glu Thr Gly
            20                  25                  30

Val Ile Thr Pro Glu Glu Phe Val Ala Ala Gly Asp His Leu Val His
        35                  40                  45

His Cys Pro Thr Trp Gln Trp Ala Thr Gly Glu Glu Leu Lys Val Lys
    50                  55                  60

Ala Tyr Leu Pro Thr Gly Lys Gln Phe Leu Val Thr Lys Asn Val Pro
65                  70                  75                  80

Cys Tyr Lys Arg Cys Lys Gln Met Glu Tyr Ser Asp Glu Leu Glu Ala
                85                  90                  95

Ile Ile Glu Glu Asp Asp Gly Asp Gly Gly Trp Val Asp Thr Tyr His
            100                 105                 110

Asn Thr Gly Ile Thr Gly Ile Thr Glu Ala Val Lys Glu Ile Thr Leu
        115                 120                 125

Glu Asn Lys Asp Asn Ile Arg Leu Gln Asp Cys Ser Ala Leu Cys Glu
    130                 135                 140

Glu Glu Glu Asp Glu Asp Glu Gly Glu Ala Ala Asp Met Glu Glu Tyr
145                 150                 155                 160

Glu Glu Ser Gly Leu Leu Glu Thr Asp Glu Ala Thr Leu Asp Thr Arg
                165                 170                 175

Lys Ile Val Glu Ala Cys Lys Ala Lys Thr Asp Ala Gly Gly Glu Asp
            180                 185                 190

Ala Ile Leu Gln Thr Arg Thr Tyr Asp Leu Tyr Ile Thr Tyr Asp Lys
        195                 200                 205

Tyr Tyr Gln Thr Pro Arg Leu Trp Leu Phe Gly Tyr Asp Glu Gln Arg
    210                 215                 220

Gln Pro Leu Thr Val Glu His Met Tyr Glu Asp Ile Ser Gln Asp His
225                 230                 235                 240

Val Lys Lys Thr Val Thr Ile Glu Asn His Pro His Leu Pro Pro Pro
                245                 250                 255

Pro Met Cys Ser Val His Pro Cys Arg His Ala Glu Val Met Lys Lys
            260                 265                 270

Ile Ile Glu Thr Val Ala Glu Gly Gly Gly Glu Leu Gly Val His Met
        275                 280                 285

Tyr Leu Leu Ile Phe Leu Lys Phe Val Gln Ala Val Ile Pro Thr Ile
    290                 295                 300

Glu Tyr Asp Tyr Thr Arg His Phe Thr Met
305                 310

<210>  31
<211>  4691

<212>  DNA
<213>  Homo sapiens

<220>
<221>  CDS
<222>  (376)..(3330)

<400>  31
gtcagtctgg ccggctccgt cctcccgtag gctccgctgt agctagcaat gtgacaccag      60

gacgcactcg ctctcgcgcg ctctcccagg ctcgttctcc ctcgccctct ctctctcaca     120

cacgcacgca cacacccacc tctcccataa acacacacac acacatgcac acccacaccc     180

acgcgcgccc gcaccgcccc acgcgcacac actcctgccc acgcccacgc agcgctccgg     240

gaagtccggt ccgggcgaga gcgcgaaagg ataccgagaa gccacccgcg gagagcgcag     300

cggcgccctg ggacgcggcg ctctcccggc gctgctgcct cggcttggtc tcggcctgcg     360

ggccgtcggc cggcg atg gcc ctg gat tat cta cta ctg ctc ctc ctg gca     411
                 Met Ala Leu Asp Tyr Leu Leu Leu Leu Leu Leu Ala
                 1               5                   10

tcc gca gtg gct gcg atg gaa gaa acg tta atg gac acc aga acg gct     459
Ser Ala Val Ala Ala Met Glu Glu Thr Leu Met Asp Thr Arg Thr Ala
        15                  20                  25

act gca gag ctg ggc tgg acg gcc aat cct gcg tcc ggg tgg gaa gaa     507
Thr Ala Glu Leu Gly Trp Thr Ala Asn Pro Ala Ser Gly Trp Glu Glu
    30                  35                  40

gtc agt ggc tac gat gaa aac ctg aac acc atc cgc acc tac cag gtg     555
Val Ser Gly Tyr Asp Glu Asn Leu Asn Thr Ile Arg Thr Tyr Gln Val
45                  50                  55                  60

tgc aat gtc ttc gag ccc aac cag aac aat tgg ctg ctc acc acc ttc     603
Cys Asn Val Phe Glu Pro Asn Gln Asn Asn Trp Leu Leu Thr Thr Phe
                65                  70                  75

atc aac cgg cgg ggg gcc cat cgc atc tac aca gag atg cgc ttc act     651
Ile Asn Arg Arg Gly Ala His Arg Ile Tyr Thr Glu Met Arg Phe Thr
            80                  85                  90

gtg aga gac tgc agc agc ctc cct aat gtc cca gga tcc tgc aag gag     699
Val Arg Asp Cys Ser Ser Leu Pro Asn Val Pro Gly Ser Cys Lys Glu
        95                  100                 105

acc ttc aac ttg tat tac tat gag act gac tct gtc att gcc acc aag     747
Thr Phe Asn Leu Tyr Tyr Tyr Glu Thr Asp Ser Val Ile Ala Thr Lys
    110                 115                 120

aag tca gcc ttc tgg tct gag gcc ccc tac ctc aaa gta gac acc att     795
Lys Ser Ala Phe Trp Ser Glu Ala Pro Tyr Leu Lys Val Asp Thr Ile
125                 130                 135                 140

gct gca gat gag agc ttc tcc cag gtg gac ttt ggg gga agg ctg atg     843
Ala Ala Asp Glu Ser Phe Ser Gln Val Asp Phe Gly Gly Arg Leu Met
                145                 150                 155

aag gta aac aca gaa gtc agg agc ttt ggg cct ctt act cgg aat ggt     891
Lys Val Asn Thr Glu Val Arg Ser Phe Gly Pro Leu Thr Arg Asn Gly

160                 165                 170

ttt tac ctc gct ttt cag gat tat gga gcc tgt atg tct ctt ctt tct      939
Phe Tyr Leu Ala Phe Gln Asp Tyr Gly Ala Cys Met Ser Leu Leu Ser
        175                 180                 185

gtc cgt gtc ttc ttc aaa aag tgt ccc agc att gtg caa aat ttt gca      987
Val Arg Val Phe Phe Lys Lys Cys Pro Ser Ile Val Gln Asn Phe Ala
    190                 195                 200

gtg ttt cca gag act atg aca ggg gca gag agc aca tct ctg gtg att     1035
Val Phe Pro Glu Thr Met Thr Gly Ala Glu Ser Thr Ser Leu Val Ile
205                 210                 215                 220

gct cgg ggc aca tgc atc ccc aac gca gag gaa gtg gac gtg ccc atc     1083
Ala Arg Gly Thr Cys Ile Pro Asn Ala Glu Glu Val Asp Val Pro Ile
                225                 230                 235

aaa ctc tac tgc aac ggg gat ggg gaa tgg atg gtg cct att ggg cga     1131
Lys Leu Tyr Cys Asn Gly Asp Gly Glu Trp Met Val Pro Ile Gly Arg
            240                 245                 250

tgc acc tgc aag cct ggc tat gag cct gag aac agc gtg gca tgc aag     1179
Cys Thr Cys Lys Pro Gly Tyr Glu Pro Glu Asn Ser Val Ala Cys Lys
        255                 260                 265

gct tgc cct gca ggg aca ttc aag gcc agc cag gaa gct gaa ggc tgc     1227
Ala Cys Pro Ala Gly Thr Phe Lys Ala Ser Gln Glu Ala Glu Gly Cys
    270                 275                 280

tcc cac tgc ccc tcc aac agc cgc tcc cct gca gag gcg tct ccc atc     1275
Ser His Cys Pro Ser Asn Ser Arg Ser Pro Ala Glu Ala Ser Pro Ile
285                 290                 295                 300

tgc acc tgt cgg acc ggt tat tac cga gcg gac ttt gac cct cca gaa     1323
Cys Thr Cys Arg Thr Gly Tyr Tyr Arg Ala Asp Phe Asp Pro Pro Glu
                305                 310                 315

gtg gca tgc act agc gtc cca tca ggt ccc cgc aat gtt atc tcc atc     1371
Val Ala Cys Thr Ser Val Pro Ser Gly Pro Arg Asn Val Ile Ser Ile
            320                 325                 330

gtc aat gag acg tcc atc att ctg gag tgg cac cct cca agg gag aca     1419
Val Asn Glu Thr Ser Ile Ile Leu Glu Trp His Pro Pro Arg Glu Thr
        335                 340                 345

ggt ggg cgg gat gat gtg acc tac aac atc atc tgc aaa aag tgc cgg     1467
Gly Gly Arg Asp Asp Val Thr Tyr Asn Ile Ile Cys Lys Lys Cys Arg
    350                 355                 360

gca gac cgc cgg agc tgc tcc cgc tgt gac gac aat gtg gag ttt gtg     1515
Ala Asp Arg Arg Ser Cys Ser Arg Cys Asp Asp Asn Val Glu Phe Val
365                 370                 375                 380

ccc agg cag ctg ggc ctg acg gag tgc cgc gtc tcc atc agc agc ctg     1563
Pro Arg Gln Leu Gly Leu Thr Glu Cys Arg Val Ser Ile Ser Ser Leu
                385                 390                 395

tgg gcc cac acc ccc tac acc ttt gac atc cag gcc atc aat gga gtc     1611
Trp Ala His Thr Pro Tyr Thr Phe Asp Ile Gln Ala Ile Asn Gly Val
            400                 405                 410

tcc agc aag agt ccc ttc ccc cca cag cac gtc tct gtc aac atc acc     1659

Ser Ser Lys Ser Pro Phe Pro Pro Gln His Val Ser Val Asn Ile Thr
        415                 420                 425

aca aac caa gcc gcc ccc tcc acc gtt ccc atc atg cac caa gtc agt      1707
Thr Asn Gln Ala Ala Pro Ser Thr Val Pro Ile Met His Gln Val Ser
    430                 435                 440

gcc act atg agg agc atc acc ttg tca tgg cca cag ccg gag cag ccc      1755
Ala Thr Met Arg Ser Ile Thr Leu Ser Trp Pro Gln Pro Glu Gln Pro
445                 450                 455                 460

aat ggc atc atc ctg gac tat gag atc cgg tac tat gag aag gaa cac      1803
Asn Gly Ile Ile Leu Asp Tyr Glu Ile Arg Tyr Tyr Glu Lys Glu His
                465                 470                 475

aat gag ttc aac tcc tcc atg gcc agg agt cag acc aac aca gca agg      1851
Asn Glu Phe Asn Ser Ser Met Ala Arg Ser Gln Thr Asn Thr Ala Arg
            480                 485                 490

att gat ggg ctg cgg cct ggc atg gta tat gtg gta cag gtg cgt gcc      1899
Ile Asp Gly Leu Arg Pro Gly Met Val Tyr Val Val Gln Val Arg Ala
        495                 500                 505

cgc act gtt gct ggc tac ggc aag ttc agt ggc aag atg tgc ttc cag      1947
Arg Thr Val Ala Gly Tyr Gly Lys Phe Ser Gly Lys Met Cys Phe Gln
    510                 515                 520

act ctg act gac gat gat tac aag tca gag ctg agg gag cag ctg ccc      1995
Thr Leu Thr Asp Asp Asp Tyr Lys Ser Glu Leu Arg Glu Gln Leu Pro
525                 530                 535                 540

ctg att gct ggc tcg gca gcg gcc ggg gtc gtg ttc gtt gtg tcc ttg      2043
Leu Ile Ala Gly Ser Ala Ala Ala Gly Val Val Phe Val Val Ser Leu
                545                 550                 555

gtg gcc atc tct atc gtc tgt agc agg aaa cgg gct tat agc aaa gag      2091
Val Ala Ile Ser Ile Val Cys Ser Arg Lys Arg Ala Tyr Ser Lys Glu
            560                 565                 570

gct gtg tac agc gat aag ctc cag cat tac agc aca ggc cga ggc tcc      2139
Ala Val Tyr Ser Asp Lys Leu Gln His Tyr Ser Thr Gly Arg Gly Ser
        575                 580                 585

cca ggg atg aag atc tac att gac ccc ttc act tac gag gat ccc aac      2187
Pro Gly Met Lys Ile Tyr Ile Asp Pro Phe Thr Tyr Glu Asp Pro Asn
    590                 595                 600

gaa gct gtc cgg gag ttt gcc aag gag att gat gta tct ttt gtg aaa      2235
Glu Ala Val Arg Glu Phe Ala Lys Glu Ile Asp Val Ser Phe Val Lys
605                 610                 615                 620

att gaa gag gtc atc gga gca ggg gag ttt gga gaa gtg tac aag ggg      2283
Ile Glu Glu Val Ile Gly Ala Gly Glu Phe Gly Glu Val Tyr Lys Gly
                625                 630                 635

cgt ttg aaa ctg cca ggc aag agg gaa atc tac gtg gcc atc aag acc      2331
Arg Leu Lys Leu Pro Gly Lys Arg Glu Ile Tyr Val Ala Ile Lys Thr
            640                 645                 650

ctg aag gca ggg tac tcg gag aag cag cgt cgg gac ttt ctg agt gag      2379
Leu Lys Ala Gly Tyr Ser Glu Lys Gln Arg Arg Asp Phe Leu Ser Glu
        655                 660                 665

gcg agc atc atg ggc cag ttc gac cat cct aac atc att cgc ctg gag 2427
Ala Ser Ile Met Gly Gln Phe Asp His Pro Asn Ile Ile Arg Leu Glu
    670                 675                 680

ggt gtg gtc acc aag agt cgg cct gtc atg atc atc aca gag ttc atg 2475
Gly Val Val Thr Lys Ser Arg Pro Val Met Ile Ile Thr Glu Phe Met
685                 690                 695                 700

gag aat ggt gca ttg gat tct ttc ctc agg caa aat gac ggg cag ttc 2523
Glu Asn Gly Ala Leu Asp Ser Phe Leu Arg Gln Asn Asp Gly Gln Phe
                705                 710                 715

acc gtg atc cag ctt gtg ggt atg ctc agg ggc atc gct gct ggc atg 2571
Thr Val Ile Gln Leu Val Gly Met Leu Arg Gly Ile Ala Ala Gly Met
            720                 725                 730

aag tac ctg gct gag atg aat tat gtg cat cgg gac ctg gct gct agg 2619
Lys Tyr Leu Ala Glu Met Asn Tyr Val His Arg Asp Leu Ala Ala Arg
        735                 740                 745

aac att ctg gtc aac agt aac ctg gtg tgc aag gtg tcc gac ttt ggc 2667
Asn Ile Leu Val Asn Ser Asn Leu Val Cys Lys Val Ser Asp Phe Gly
    750                 755                 760

ctc tcc cgc tac ctc cag gat gac acc tca gat ccc acc tac acc agc 2715
Leu Ser Arg Tyr Leu Gln Asp Asp Thr Ser Asp Pro Thr Tyr Thr Ser
765                 770                 775                 780

tcc ttg gga ggg aag atc cct gtg aga tgg aca gct cca gag gcc atc 2763
Ser Leu Gly Gly Lys Ile Pro Val Arg Trp Thr Ala Pro Glu Ala Ile
                785                 790                 795

gcc tac cgc aag ttc act tca gcc agc gac gtt tgg agc tat ggg atc 2811
Ala Tyr Arg Lys Phe Thr Ser Ala Ser Asp Val Trp Ser Tyr Gly Ile
            800                 805                 810

gtc atg tgg gaa gtc atg tca ttt gga gag aga ccc tat tgg gat atg 2859
Val Met Trp Glu Val Met Ser Phe Gly Glu Arg Pro Tyr Trp Asp Met
        815                 820                 825

tcc aac caa gat gtc atc aat gcc atc gag cag gac tac cgg ctg ccc 2907
Ser Asn Gln Asp Val Ile Asn Ala Ile Glu Gln Asp Tyr Arg Leu Pro
    830                 835                 840

cca ccc atg gac tgt cca gct gct cta cac cag ctc atg ctg gac tgt 2955
Pro Pro Met Asp Cys Pro Ala Ala Leu His Gln Leu Met Leu Asp Cys
845                 850                 855                 860

tgg cag aag gac cgg aac agc cgg ccc cgg ttt gcg gag att gtc aac 3003
Trp Gln Lys Asp Arg Asn Ser Arg Pro Arg Phe Ala Glu Ile Val Asn
                865                 870                 875

acc cta gat aag atg atc cgg aac ccg gca agt ctc aag act gtg gca 3051
Thr Leu Asp Lys Met Ile Arg Asn Pro Ala Ser Leu Lys Thr Val Ala
            880                 885                 890

acc atc acc gcc gtg cct tcc cag ccc ctg ctc gac cgc tcc atc cca 3099
Thr Ile Thr Ala Val Pro Ser Gln Pro Leu Leu Asp Arg Ser Ile Pro
        895                 900                 905

gac ttc acg gcc ttt acc acc gtg gat gac tgg ctc agc gcc atc aaa 3147
Asp Phe Thr Ala Phe Thr Thr Val Asp Asp Trp Leu Ser Ala Ile Lys
    910                 915                 920

atg gtc cag tac agg gac agc ttc ctc act gct ggc ttc acc tcc ctc     3195
Met Val Gln Tyr Arg Asp Ser Phe Leu Thr Ala Gly Phe Thr Ser Leu
925                 930                 935                 940

cag ctg gtc acc cag atg aca tca gaa gac ctc ctg aga ata ggc atc     3243
Gln Leu Val Thr Gln Met Thr Ser Glu Asp Leu Leu Arg Ile Gly Ile
                945                 950                 955

acc ttg gca ggc cat cag aag aag atc ctg aac agc att cat tct atg     3291
Thr Leu Ala Gly His Gln Lys Lys Ile Leu Asn Ser Ile His Ser Met
            960                 965                 970

agg gtc cag ata agt cag tca cca acg gca atg gca tga gaactcttgt     3340
Arg Val Gln Ile Ser Gln Ser Pro Thr Ala Met Ala
        975                 980

ttcttgggga aggagaggag ggaaaaggac cagggtcaag ggggaccaga ggttgaccac     3400

tgtggaatgt actggagaga ctggcttctc agctgaggaa tgcatttcca tcagtgaaga     3460

atcaaccgga cctgttgcta gcaggcaatc tccatttctc agtgacagaa gcatgtttga     3520

gatgccgtgg gaaaccaaat atataataat aaaaatataa aaaggtgatg ttcaacagaa     3580

gtgaagacaa aacaatatgc atcaggagaa caagagtaaa cccagctccc actctcagtg     3640

ggctgcagtt gcccaaccac aggaagaaag ggaaggaggt agagggaaga aacagaagca     3700

gtgttccatt ttcttcctca ccaatgacat tcttttcttt tctcctttcg tactcctccc     3760

tgagagtccc ctcccttctc ccacactcgt ttccctttgc tcatgactcc tgtagggaag     3820

tttcttcaaa caaaacccag ctcctgagtc tccagatgtt gttctgtcag ttgccaaagg     3880

actttgctga ccactgcatg gggatccaac caattcaatt aatgtcttca tattgaagaa     3940

gagatgtacc ttcaattgaa aacctcgttt ttcttttgtt tgcattttct gcaaaaagga     4000

aaaagaaacc acaaattggg gaaaaaaaaa gaagaaaaac ctgtttccgt gtgcaaaagc     4060

acacatatgt atgtctgtgt tataaaatga ctgtgcttgt tcgtaacaga tgcaaacaag     4120

aaagaagaac tgggaagtct ttgtccctag gaaatccaaa ggggctggaa tatggtgttg     4180

gtttggcttt ctggttggcc caatcggcct attggctcaa tgggaagaga ggagagggag     4240

aaaaataaaa tgaaaggaaa aaaaaaagtt tgcaaattca gacaggaaac aggtgagtgg     4300

tttgaattgg atgcagtgtg ggccatcctg gaatgatact gactgattaa ttattcctga     4360

taacatctca agaaaaggag aaggaaagtg tttctggaga atgttctttc acatcactgg     4420

aatctgcaat tcaagaagtg acaagggaga attcttgctt tacctatgga ctggcttaag     4480

ccgtgtggca tccgaggaat gtttcaaatg tgtctgtgtt tctctttaca ttccttgttg     4540

tacctcattg ttcaattcac ttttgtaaat tccacctaac atttaattat tttaaatttc     4600

tccttttacc ttaatctcct tgctaatttt atctgtctaa ttaaaaagag cagaagcatg     4660

tctgggttta cgtaaaaaaa aaaaaaaaaa a                                   4691

<210>  32
<211>  984
<212>  PRT
<213>  Homo sapiens

<400>  32

Met Ala Leu Asp Tyr Leu Leu Leu Leu Leu Leu Ala Ser Ala Val Ala
1               5                   10                  15

Ala Met Glu Glu Thr Leu Met Asp Thr Arg Thr Ala Thr Ala Glu Leu
            20                  25                  30

Gly Trp Thr Ala Asn Pro Ala Ser Gly Trp Glu Glu Val Ser Gly Tyr
        35                  40                  45

Asp Glu Asn Leu Asn Thr Ile Arg Thr Tyr Gln Val Cys Asn Val Phe
    50                  55                  60

Glu Pro Asn Gln Asn Asn Trp Leu Leu Thr Thr Phe Ile Asn Arg Arg
65                  70                  75                  80

Gly Ala His Arg Ile Tyr Thr Glu Met Arg Phe Thr Val Arg Asp Cys
                85                  90                  95

Ser Ser Leu Pro Asn Val Pro Gly Ser Cys Lys Glu Thr Phe Asn Leu
            100                 105                 110

Tyr Tyr Tyr Glu Thr Asp Ser Val Ile Ala Thr Lys Lys Ser Ala Phe
        115                 120                 125

Trp Ser Glu Ala Pro Tyr Leu Lys Val Asp Thr Ile Ala Ala Asp Glu
    130                 135                 140

Ser Phe Ser Gln Val Asp Phe Gly Gly Arg Leu Met Lys Val Asn Thr
145                 150                 155                 160

Glu Val Arg Ser Phe Gly Pro Leu Thr Arg Asn Gly Phe Tyr Leu Ala
                165                 170                 175

Phe Gln Asp Tyr Gly Ala Cys Met Ser Leu Leu Ser Val Arg Val Phe
            180                 185                 190

Phe Lys Lys Cys Pro Ser Ile Val Gln Asn Phe Ala Val Phe Pro Glu
        195                 200                 205

Thr Met Thr Gly Ala Glu Ser Thr Ser Leu Val Ile Ala Arg Gly Thr
    210                 215                 220

Cys Ile Pro Asn Ala Glu Glu Val Asp Val Pro Ile Lys Leu Tyr Cys
225                 230                 235                 240

Asn Gly Asp Gly Glu Trp Met Val Pro Ile Gly Arg Cys Thr Cys Lys
                245                 250                 255

Pro Gly Tyr Glu Pro Glu Asn Ser Val Ala Cys Lys Ala Cys Pro Ala
            260                 265                 270

Gly Thr Phe Lys Ala Ser Gln Glu Ala Glu Gly Cys Ser His Cys Pro
        275                 280                 285

Ser Asn Ser Arg Ser Pro Ala Glu Ala Ser Pro Ile Cys Thr Cys Arg
    290                 295                 300

Thr Gly Tyr Tyr Arg Ala Asp Phe Asp Pro Pro Glu Val Ala Cys Thr
305                 310                 315                 320

Ser Val Pro Ser Gly Pro Arg Asn Val Ile Ser Ile Val Asn Glu Thr
                325                 330                 335

Ser Ile Ile Leu Glu Trp His Pro Pro Arg Glu Thr Gly Gly Arg Asp
            340                 345                 350

Asp Val Thr Tyr Asn Ile Ile Cys Lys Lys Cys Arg Ala Asp Arg Arg
        355                 360                 365

Ser Cys Ser Arg Cys Asp Asp Asn Val Glu Phe Val Pro Arg Gln Leu
    370                 375                 380

Gly Leu Thr Glu Cys Arg Val Ser Ile Ser Ser Leu Trp Ala His Thr
385                 390                 395                 400

Pro Tyr Thr Phe Asp Ile Gln Ala Ile Asn Gly Val Ser Ser Lys Ser
                405                 410                 415

Pro Phe Pro Pro Gln His Val Ser Val Asn Ile Thr Thr Asn Gln Ala
            420                 425                 430

Ala Pro Ser Thr Val Pro Ile Met His Gln Val Ser Ala Thr Met Arg
        435                 440                 445

Ser Ile Thr Leu Ser Trp Pro Gln Pro Glu Gln Pro Asn Gly Ile Ile
    450                 455                 460

Leu Asp Tyr Glu Ile Arg Tyr Tyr Glu Lys Glu His Asn Glu Phe Asn
465                 470                 475                 480

Ser Ser Met Ala Arg Ser Gln Thr Asn Thr Ala Arg Ile Asp Gly Leu
                485                 490                 495

Arg Pro Gly Met Val Tyr Val Val Gln Val Arg Ala Arg Thr Val Ala
            500                 505                 510

Gly Tyr Gly Lys Phe Ser Gly Lys Met Cys Phe Gln Thr Leu Thr Asp
        515                 520                 525

Asp Asp Tyr Lys Ser Glu Leu Arg Glu Gln Leu Pro Leu Ile Ala Gly
    530                 535                 540

Ser Ala Ala Ala Gly Val Val Phe Val Val Ser Leu Val Ala Ile Ser
545                 550                 555                 560

Ile Val Cys Ser Arg Lys Arg Ala Tyr Ser Lys Glu Ala Val Tyr Ser
                565                 570                 575

Asp Lys Leu Gln His Tyr Ser Thr Gly Arg Gly Ser Pro Gly Met Lys
            580                 585                 590

Ile Tyr Ile Asp Pro Phe Thr Tyr Glu Asp Pro Asn Glu Ala Val Arg
        595                 600                 605

Glu Phe Ala Lys Glu Ile Asp Val Ser Phe Val Lys Ile Glu Glu Val
    610                 615                 620

Ile Gly Ala Gly Glu Phe Gly Glu Val Tyr Lys Gly Arg Leu Lys Leu
625                 630                 635                 640

Pro Gly Lys Arg Glu Ile Tyr Val Ala Ile Lys Thr Leu Lys Ala Gly
                645                 650                 655

Tyr Ser Glu Lys Gln Arg Arg Asp Phe Leu Ser Glu Ala Ser Ile Met
            660                 665                 670

Gly Gln Phe Asp His Pro Asn Ile Ile Arg Leu Glu Gly Val Val Thr
        675                 680                 685

Lys Ser Arg Pro Val Met Ile Ile Thr Glu Phe Met Glu Asn Gly Ala
    690                 695                 700

Leu Asp Ser Phe Leu Arg Gln Asn Asp Gly Gln Phe Thr Val Ile Gln
705                 710                 715                 720

Leu Val Gly Met Leu Arg Gly Ile Ala Ala Gly Met Lys Tyr Leu Ala

                725                 730                 735

Glu Met Asn Tyr Val His Arg Asp Leu Ala Ala Arg Asn Ile Leu Val
            740                 745                 750

Asn Ser Asn Leu Val Cys Lys Val Ser Asp Phe Gly Leu Ser Arg Tyr
        755                 760                 765

Leu Gln Asp Asp Thr Ser Asp Pro Thr Tyr Thr Ser Ser Leu Gly Gly
    770                 775                 780

Lys Ile Pro Val Arg Trp Thr Ala Pro Glu Ala Ile Ala Tyr Arg Lys
785                 790                 795                 800

Phe Thr Ser Ala Ser Asp Val Trp Ser Tyr Gly Ile Val Met Trp Glu
                805                 810                 815

Val Met Ser Phe Gly Glu Arg Pro Tyr Trp Asp Met Ser Asn Gln Asp
            820                 825                 830

Val Ile Asn Ala Ile Glu Gln Asp Tyr Arg Leu Pro Pro Pro Met Asp
        835                 840                 845

Cys Pro Ala Ala Leu His Gln Leu Met Leu Asp Cys Trp Gln Lys Asp
    850                 855                 860

Arg Asn Ser Arg Pro Arg Phe Ala Glu Ile Val Asn Thr Leu Asp Lys
865                 870                 875                 880

Met Ile Arg Asn Pro Ala Ser Leu Lys Thr Val Ala Thr Ile Thr Ala
                885                 890                 895

Val Pro Ser Gln Pro Leu Leu Asp Arg Ser Ile Pro Asp Phe Thr Ala
            900                 905                 910

Phe Thr Thr Val Asp Asp Trp Leu Ser Ala Ile Lys Met Val Gln Tyr
        915                 920                 925

Arg Asp Ser Phe Leu Thr Ala Gly Phe Thr Ser Leu Gln Leu Val Thr
    930                 935                 940

Gln Met Thr Ser Glu Asp Leu Leu Arg Ile Gly Ile Thr Leu Ala Gly
945                 950                 955                 960

His Gln Lys Lys Ile Leu Asn Ser Ile His Ser Met Arg Val Gln Ile
                965                 970                 975

Ser Gln Ser Pro Thr Ala Met Ala
            980

<210>  33
<211>  5816
<212>  DNA
<213>  Homo sapiens

<220>
<221>  CDS
<222>  (346)..(4428)

<400>  33
attataacag ccaatgcagc cgccatccac gcacaagcaa agaagcatgt cccatttaaa      60

tacacccacg cagccccagc gcccttagcc gatcggggcg ctcagcccac acgcaccgct     120

gctcggggct tggagatccg cgcaggctgg gctcccggac ccggcggacc gacgcgcgga     180

ggatcgggat ccggcgctgt ggggctgggg tggcgggggg aggctgggcc cggggcctct     240

ggcgcgacac ccgcatgagg acgcgagtga aatagaccaa ggtggaattt ccaagggaaa     300

agcttcgggg tggttttggt ccatttctcc agcgaagaag tagac atg gcg agc gac     357
                                                  Met Ala Ser Asp
                                                  1

tcc ccg gct cga agc ctg gat gaa ata gat ctc tcg gct ctg agg gac       405
Ser Pro Ala Arg Ser Leu Asp Glu Ile Asp Leu Ser Ala Leu Arg Asp
5                   10                  15                  20

ccc gca ggg atc ttt gaa ttg gtg gaa ctt gtt gga aat gga aca tac       453
Pro Ala Gly Ile Phe Glu Leu Val Glu Leu Val Gly Asn Gly Thr Tyr
                25                  30                  35

ggg caa gtt tat aag ggt cgt cat gtc aaa acg ggc cag ctt gca gcc       501
Gly Gln Val Tyr Lys Gly Arg His Val Lys Thr Gly Gln Leu Ala Ala
            40                  45                  50

atc aag gtt atg gat gtc aca ggg gat gaa gag gaa gaa atc aaa caa       549
Ile Lys Val Met Asp Val Thr Gly Asp Glu Glu Glu Glu Ile Lys Gln
        55                  60                  65

gaa att aac atg ttg aag aaa tat tct cat cac cgg aat att gct aca       597
Glu Ile Asn Met Leu Lys Lys Tyr Ser His His Arg Asn Ile Ala Thr
    70                  75                  80

tac tat ggt gct ttt atc aaa aag aac cca cca ggc atg gat gac caa       645
Tyr Tyr Gly Ala Phe Ile Lys Lys Asn Pro Pro Gly Met Asp Asp Gln
85                  90                  95                  100

ctt tgg ttg gtg atg gag ttt tgt ggt gct ggc tct gtc acc gac ctg       693
Leu Trp Leu Val Met Glu Phe Cys Gly Ala Gly Ser Val Thr Asp Leu
                105                 110                 115

atc aag aac aca aaa ggt aac acg ttg aaa gag gag tgg att gca tac       741
Ile Lys Asn Thr Lys Gly Asn Thr Leu Lys Glu Glu Trp Ile Ala Tyr
            120                 125                 130

atc tgc agg gaa atc tta cgg ggg ctg agt cac ctg cac cag cat aaa       789
Ile Cys Arg Glu Ile Leu Arg Gly Leu Ser His Leu His Gln His Lys

135                 140                 145

gtg att cat cga gat att aaa ggg caa aat gtc ttg ctg act gaa aat      837
Val Ile His Arg Asp Ile Lys Gly Gln Asn Val Leu Leu Thr Glu Asn
    150                 155                 160

gca gaa gtt aaa cta gtg gac ttt gga gtc agt gct cag ctt gat cga      885
Ala Glu Val Lys Leu Val Asp Phe Gly Val Ser Ala Gln Leu Asp Arg
165                 170                 175                 180

aca gtg ggc agg agg aat act ttc att gga act ccc tac tgg atg gca      933
Thr Val Gly Arg Arg Asn Thr Phe Ile Gly Thr Pro Tyr Trp Met Ala
                185                 190                 195

cca gaa gtt att gcc tgt gat gaa aac cca gat gcc aca tat gat ttc      981
Pro Glu Val Ile Ala Cys Asp Glu Asn Pro Asp Ala Thr Tyr Asp Phe
            200                 205                 210

aag agt gac ttg tgg tct ttg ggt atc acc gcc att gaa atg gca gaa      1029
Lys Ser Asp Leu Trp Ser Leu Gly Ile Thr Ala Ile Glu Met Ala Glu
        215                 220                 225

ggt gct ccc cct ctc tgt gac atg cac ccc atg aga gct ctc ttc ctc      1077
Gly Ala Pro Pro Leu Cys Asp Met His Pro Met Arg Ala Leu Phe Leu
    230                 235                 240

atc ccc cgg aac cca gcg cct cgg ctg aag tct aag aag tgg tca aaa      1125
Ile Pro Arg Asn Pro Ala Pro Arg Leu Lys Ser Lys Lys Trp Ser Lys
245                 250                 255                 260

aaa ttc cag tca ttt att gag agc tgc ttg gta aag aat cac agc cag      1173
Lys Phe Gln Ser Phe Ile Glu Ser Cys Leu Val Lys Asn His Ser Gln
                265                 270                 275

cga cca gca aca gaa caa ttg atg aag cat cca ttt ata cga gac caa      1221
Arg Pro Ala Thr Glu Gln Leu Met Lys His Pro Phe Ile Arg Asp Gln
            280                 285                 290

cct aat gag cga cag gtc cgc att caa ctc aag gac cat att gat aga      1269
Pro Asn Glu Arg Gln Val Arg Ile Gln Leu Lys Asp His Ile Asp Arg
        295                 300                 305

aca aag aag aag cga gga gaa aaa gat gag aca gag tat gag tac agt      1317
Thr Lys Lys Lys Arg Gly Glu Lys Asp Glu Thr Glu Tyr Glu Tyr Ser
    310                 315                 320

gga agt gag gaa gaa gag gag gag aat gac tca gga gag ccc agc tcc      1365
Gly Ser Glu Glu Glu Glu Glu Glu Asn Asp Ser Gly Glu Pro Ser Ser
325                 330                 335                 340

atc ctg aat ctg cca ggg gag tcg acg ctg cgg agg gac ttt ctg agg      1413
Ile Leu Asn Leu Pro Gly Glu Ser Thr Leu Arg Arg Asp Phe Leu Arg
                345                 350                 355

ctg cag ctg gcc aac aag gag cgt tct gag gcc cta cgg agg cag cag      1461
Leu Gln Leu Ala Asn Lys Glu Arg Ser Glu Ala Leu Arg Arg Gln Gln
            360                 365                 370

ctg gag cag cag cag cgg gag aat gag gag cac aag cgg cag ctg ctg      1509
Leu Glu Gln Gln Gln Arg Glu Asn Glu Glu His Lys Arg Gln Leu Leu
        375                 380                 385

gcc gag cgt cag aag cgc atc gag gag cag aaa gag cag agg cgg cgg      1557

Ala Glu Arg Gln Lys Arg Ile Glu Glu Gln Lys Glu Gln Arg Arg Arg
    390                 395                 400

ctg gag gag caa caa agg cga gag aag gag ctg cgg aag cag cag gag      1605
Leu Glu Glu Gln Gln Arg Arg Glu Lys Glu Leu Arg Lys Gln Gln Glu
405                 410                 415                 420

agg gag cag cgc cgg cac tat gag gag cag atg cgc cgg gag gag gag      1653
Arg Glu Gln Arg Arg His Tyr Glu Glu Gln Met Arg Arg Glu Glu Glu
                425                 430                 435

agg agg cgt gcg gag cat gaa cag gaa tac atc agg cga cag tta gag      1701
Arg Arg Arg Ala Glu His Glu Gln Glu Tyr Ile Arg Arg Gln Leu Glu
            440                 445                 450

gag gag cag aga cag tta gag atc ttg cag cag cag cta ctg cat gaa      1749
Glu Glu Gln Arg Gln Leu Glu Ile Leu Gln Gln Gln Leu Leu His Glu
        455                 460                 465

caa gct cta ctt ctg gaa tat aag cgc aaa caa ttg gaa gaa cag aga      1797
Gln Ala Leu Leu Leu Glu Tyr Lys Arg Lys Gln Leu Glu Glu Gln Arg
    470                 475                 480

caa gca gaa aga ctg cag agg cag cta aag caa gaa aga gac tac tta      1845
Gln Ala Glu Arg Leu Gln Arg Gln Leu Lys Gln Glu Arg Asp Tyr Leu
485                 490                 495                 500

gtt tcc ctt cag cat cag cgg cag gag cag agg cct gtg gag aag aag      1893
Val Ser Leu Gln His Gln Arg Gln Glu Gln Arg Pro Val Glu Lys Lys
                505                 510                 515

cca ctg tac cat tac aaa gaa gga atg agt cct agt gag aag cca gca      1941
Pro Leu Tyr His Tyr Lys Glu Gly Met Ser Pro Ser Glu Lys Pro Ala
            520                 525                 530

tgg gcc aag gag gta gaa gaa cgg tca agg ctc aac cgg caa agt tcc      1989
Trp Ala Lys Glu Val Glu Glu Arg Ser Arg Leu Asn Arg Gln Ser Ser
        535                 540                 545

cct gcc atg cct cac aag gtt gcc aac agg ata tct gac ccc aac ctg      2037
Pro Ala Met Pro His Lys Val Ala Asn Arg Ile Ser Asp Pro Asn Leu
    550                 555                 560

ccc cca agg tcg gag tcc ttc agc att agt gga gtt cag cct gct cga      2085
Pro Pro Arg Ser Glu Ser Phe Ser Ile Ser Gly Val Gln Pro Ala Arg
565                 570                 575                 580

aca ccc ccc atg ctc aga cca gtc gat ccc cag atc cca cat ctg gta      2133
Thr Pro Pro Met Leu Arg Pro Val Asp Pro Gln Ile Pro His Leu Val
                585                 590                 595

gct gta aaa tcc cag gga cct gcc ttg acc gcc tcc cag tca gtg cac      2181
Ala Val Lys Ser Gln Gly Pro Ala Leu Thr Ala Ser Gln Ser Val His
            600                 605                 610

gag cag ccc aca aag ggc ctc tct ggg ttt cag gag gct ctg aac gtg      2229
Glu Gln Pro Thr Lys Gly Leu Ser Gly Phe Gln Glu Ala Leu Asn Val
        615                 620                 625

acc tcc cac cgc gtg gag atg cca cgc cag aac tca gat ccc acc tcg      2277
Thr Ser His Arg Val Glu Met Pro Arg Gln Asn Ser Asp Pro Thr Ser
    630                 635                 640

gaa aat cct cct ctc ccc act cgc att gaa aag ttt gac cga agc tct 2325
Glu Asn Pro Pro Leu Pro Thr Arg Ile Glu Lys Phe Asp Arg Ser Ser
645                 650                 655                 660

tgg tta cga cag gaa gaa gac att cca cca aag gtg cct caa aga aca 2373
Trp Leu Arg Gln Glu Glu Asp Ile Pro Pro Lys Val Pro Gln Arg Thr
                665                 670                 675

act tct ata tcc cca gca tta gcc aga aag aat tct cct ggg aat ggt 2421
Thr Ser Ile Ser Pro Ala Leu Ala Arg Lys Asn Ser Pro Gly Asn Gly
            680                 685                 690

agt gct ctg gga ccc aga cta gga tct caa ccc atc aga gca agc aac 2469
Ser Ala Leu Gly Pro Arg Leu Gly Ser Gln Pro Ile Arg Ala Ser Asn
        695                 700                 705

cct gat ctc cgg aga act gag ccc atc ttg gag agc ccc ttg cag agg 2517
Pro Asp Leu Arg Arg Thr Glu Pro Ile Leu Glu Ser Pro Leu Gln Arg
    710                 715                 720

acc agc agt ggc agt tcc tcc agc tcc agc acc cct agc tcc cag ccc 2565
Thr Ser Ser Gly Ser Ser Ser Ser Ser Ser Thr Pro Ser Ser Gln Pro
725                 730                 735                 740

agc tcc caa gga ggc tcc cag cct gga tca caa gca gga tcc agt gaa 2613
Ser Ser Gln Gly Gly Ser Gln Pro Gly Ser Gln Ala Gly Ser Ser Glu
                745                 750                 755

cgc acc aga gtt cga gcc aac agt aag tca gaa gga tca cct gtg ctc 2661
Arg Thr Arg Val Arg Ala Asn Ser Lys Ser Glu Gly Ser Pro Val Leu
            760                 765                 770

ccc cat gag cct gcc aag gtg aaa cca gaa gaa tcc agg gac att acc 2709
Pro His Glu Pro Ala Lys Val Lys Pro Glu Glu Ser Arg Asp Ile Thr
        775                 780                 785

cgg ccc agt cga cca gct agc tac aaa aaa gct ata gat gag gat ctg 2757
Arg Pro Ser Arg Pro Ala Ser Tyr Lys Lys Ala Ile Asp Glu Asp Leu
    790                 795                 800

acg gca tta gcc aaa gaa cta aga gaa ctc cgg att gaa gaa aca aac 2805
Thr Ala Leu Ala Lys Glu Leu Arg Glu Leu Arg Ile Glu Glu Thr Asn
805                 810                 815                 820

cgc cca atg aag aag gtg act gat tac tcc tcc tcc agt gag gag tca 2853
Arg Pro Met Lys Lys Val Thr Asp Tyr Ser Ser Ser Ser Glu Glu Ser
                825                 830                 835

gaa agt agc gag gaa gag gag gaa gat gga gag agc gag acc cat gat 2901
Glu Ser Ser Glu Glu Glu Glu Glu Asp Gly Glu Ser Glu Thr His Asp
            840                 845                 850

ggg aca gtg gct gtc agc gac ata ccc aga ctg ata cca aca gga gct 2949
Gly Thr Val Ala Val Ser Asp Ile Pro Arg Leu Ile Pro Thr Gly Ala
        855                 860                 865

cca ggc agc aac gag cag tac aat gtg gga atg gtg ggg acg cat ggg 2997
Pro Gly Ser Asn Glu Gln Tyr Asn Val Gly Met Val Gly Thr His Gly
    870                 875                 880

ctg gag acc tct cat gcg gac agt ttc agc ggc agt att tca aga gaa 3045
Leu Glu Thr Ser His Ala Asp Ser Phe Ser Gly Ser Ile Ser Arg Glu
885                 890                 895                 900

gga acc ttg atg att aga gag acg tct gga gag aag aag cga tct ggc 3093
Gly Thr Leu Met Ile Arg Glu Thr Ser Gly Glu Lys Lys Arg Ser Gly
                905                 910                 915

cac agt gac agc aat ggc ttt gct ggc cac atc aac ctc cct gac ctg 3141
His Ser Asp Ser Asn Gly Phe Ala Gly His Ile Asn Leu Pro Asp Leu
            920                 925                 930

gtg cag cag agc cat tct cca gct gga acc ccg act gag gga ctg ggg 3189
Val Gln Gln Ser His Ser Pro Ala Gly Thr Pro Thr Glu Gly Leu Gly
        935                 940                 945

cgc gtc tca acc cat tcc cag gag atg gac tct ggg act gaa tat ggc 3237
Arg Val Ser Thr His Ser Gln Glu Met Asp Ser Gly Thr Glu Tyr Gly
    950                 955                 960

atg ggg agc agc acc aaa gcc tcc ttc acc ccc ttt gtg gac ccc aga 3285
Met Gly Ser Ser Thr Lys Ala Ser Phe Thr Pro Phe Val Asp Pro Arg
965                 970                 975                 980

gta tac cag acg tct ccc act gat gaa gat gaa gag gat gag gaa tca 3333
Val Tyr Gln Thr Ser Pro Thr Asp Glu Asp Glu Glu Asp Glu Glu Ser
                985                 990                 995

tca gcc gca gct ctg ttt act agc gaa ctt ctt agg caa gaa cag 3378
Ser Ala Ala Ala Leu Phe Thr Ser Glu Leu Leu Arg Gln Glu Gln
            1000                1005                1010

gcc aaa ctc aat gaa gca aga aag att tcg gtg gta aat gta aac 3423
Ala Lys Leu Asn Glu Ala Arg Lys Ile Ser Val Val Asn Val Asn
            1015                1020                1025

cca acc aac att cgg cct cat agc gac aca cca gaa atc aga aaa 3468
Pro Thr Asn Ile Arg Pro His Ser Asp Thr Pro Glu Ile Arg Lys
            1030                1035                1040

tac aag aaa cga ttc aac tca gaa ata ctt tgt gca gct ctg tgg 3513
Tyr Lys Lys Arg Phe Asn Ser Glu Ile Leu Cys Ala Ala Leu Trp
            1045                1050                1055

ggt gta aac ctt ctg gtg ggg act gaa aat ggc ctg atg ctt ttg 3558
Gly Val Asn Leu Leu Val Gly Thr Glu Asn Gly Leu Met Leu Leu
            1060                1065                1070

gac cga agt ggg caa ggc aaa gtc tat aat ctg atc aac cgg agg 3603
Asp Arg Ser Gly Gln Gly Lys Val Tyr Asn Leu Ile Asn Arg Arg
            1075                1080                1085

cga ttt cag cag atg gat gtg cta gag gga ctg aat gtc ctt gtg 3648
Arg Phe Gln Gln Met Asp Val Leu Glu Gly Leu Asn Val Leu Val
            1090                1095                1100

aca att tca gga aag aag aat aag cta cga gtt tac tat ctt tca 3693
Thr Ile Ser Gly Lys Lys Asn Lys Leu Arg Val Tyr Tyr Leu Ser
            1105                1110                1115

tgg tta aga aac aga ata cta cat aat gac cca gaa gta gaa aag 3738
Trp Leu Arg Asn Arg Ile Leu His Asn Asp Pro Glu Val Glu Lys
            1120                1125                1130

aaa caa ggc tgg atc act gtt ggg gac ttg gaa ggc tgt ata cat 3783
Lys Gln Gly Trp Ile Thr Val Gly Asp Leu Glu Gly Cys Ile His

1135                1140                1145

tat aaa gtt gtt aaa tat gaa agg atc aaa ttt ttg gtg att gcc        3828
Tyr Lys Val Val Lys Tyr Glu Arg Ile Lys Phe Leu Val Ile Ala
        1150                1155                1160

tta aag aat gct gtg gaa ata tat gct tgg gct cct aaa ccg tat        3873
Leu Lys Asn Ala Val Glu Ile Tyr Ala Trp Ala Pro Lys Pro Tyr
        1165                1170                1175

cat aaa ttc atg gca ttt aag tct ttt gca gat ctc cag cac aag        3918
His Lys Phe Met Ala Phe Lys Ser Phe Ala Asp Leu Gln His Lys
        1180                1185                1190

cct ctg cta gtt gat ctc acg gta gaa gaa ggt caa aga tta aag        3963
Pro Leu Leu Val Asp Leu Thr Val Glu Glu Gly Gln Arg Leu Lys
        1195                1200                1205

gtt att ttt ggt tca cac act ggt ttc cat gta att gat gtt gat        4008
Val Ile Phe Gly Ser His Thr Gly Phe His Val Ile Asp Val Asp
        1210                1215                1220

tca gga aac tct tat gat atc tac ata cca tct cat att cag ggc        4053
Ser Gly Asn Ser Tyr Asp Ile Tyr Ile Pro Ser His Ile Gln Gly
        1225                1230                1235

aat atc act cct cat gct att gtc atc ttg cct aaa aca gat gga        4098
Asn Ile Thr Pro His Ala Ile Val Ile Leu Pro Lys Thr Asp Gly
        1240                1245                1250

atg gaa atg ctt gtt tgc tat gag gat gag ggg gtg tat gta aac        4143
Met Glu Met Leu Val Cys Tyr Glu Asp Glu Gly Val Tyr Val Asn
        1255                1260                1265

acc tat ggc cgg ata act aag gat gtg gtg ctc caa tgg gga gaa        4188
Thr Tyr Gly Arg Ile Thr Lys Asp Val Val Leu Gln Trp Gly Glu
        1270                1275                1280

atg ccc acg tct gtg gcc tac att cat tcc aat cag ata atg ggc        4233
Met Pro Thr Ser Val Ala Tyr Ile His Ser Asn Gln Ile Met Gly
        1285                1290                1295

tgg ggc gag aaa gct att gag atc cgg tca gtg gaa aca gga cat        4278
Trp Gly Glu Lys Ala Ile Glu Ile Arg Ser Val Glu Thr Gly His
        1300                1305                1310

ttg gat gga gta ttt atg cat aag cga gct caa agg tta aag ttt        4323
Leu Asp Gly Val Phe Met His Lys Arg Ala Gln Arg Leu Lys Phe
        1315                1320                1325

cta tgt gaa aga aat gat aag gta ttt ttt gca tcc gtg cga tct        4368
Leu Cys Glu Arg Asn Asp Lys Val Phe Phe Ala Ser Val Arg Ser
        1330                1335                1340

gga gga agt agc caa gtg ttt ttc atg acc ctc aac aga aat tcc        4413
Gly Gly Ser Ser Gln Val Phe Phe Met Thr Leu Asn Arg Asn Ser
        1345                1350                1355

atg atg aac tgg taa cagaagagca cttggcactt atcttcatgg cgttatttct        4468
Met Met Asn Trp
        1360

aatttaaaag aacataactc atgtggactt atgccagtct agaggcagaa tcagaaggct        4528

tggttgaaca tatcgctttc cctttttcct ctccctccgc ccctcccagt acagtccatc 4588

tttcaatgtt gcagcctggt tgagaaggag agaaaaaggt ggcaggaatt tccaggagat 4648

ccccaagaat gctgccttgt ctgtggacaa agatggacca tgtgcccttc ggaattaggg 4708

atagaaacaa atattgtgtg ctcttaacga ttaagctgtg ttatggtggg ttttcaggtt 4768

tttacctttt ttctttaccc ctttactctg caagaatggg gaaagaatgc atactgcgaa 4828

aatgagtctt ttaaattctg tctgcctact agttttaagt atatggtatg ttgtaaaatt 4888

tccaatgatg agagacagca caataaatgt accttatctc cttaggctga aggccataac 4948

tacatagtgg agtaatttaa gaactctctt gccttcacca acccaaaagg ttgctttttg 5008

atagcaactg gctaatgaat ttttaaaaag agaagaaaaa tactagtttt cccctctttt 5068

gggaaataga ttttaaatgg ctaaactact agccttaaaa ctactagtct aataaaatca 5128

actaccactt ttgtgaatct gacaggccac atttttatat ggccctttac agaatggagt 5188

gtgttgaaca ggatactaac gccattgagt tgagctggcc tagcgatgga gggacactct 5248

aacacaactt tccctcagct attatgcaac agatcaggga aaaagatggg atgacagatg 5308

gggtcagaca gaaagagctt ctgggaaaca agcttacata gtctttttta aaatgcacaa 5368

agcctcccag ctaagaggtc acttggtttg ggcttcatta ggactgagac tttgttgagt 5428

tctttctggg acttggagag tggatgatat tcaggctctg aacattccca gcgctctccc 5488

gagggtgcca ctttctcaag atgaaaactg tgactgaaaa aattaataat aaatgtttct 5548

gagctgcctg tgttctccct gtgtgggtga gagaagggac tagactccta agcctgcctc 5608

agatacaaga ggcatcattg gctccaattt tagagaactt gaaagcaagg ctttggacaa 5668

aattttgaga ccctaatcac tttaccttcc tccaaattac ccaacatacg gtaaacaaca 5728

tttgtgcaga agtatgtatg tatttagttc aggttgactt gtgtccttat aaactcttac 5788

tcaaatgatt tgaactttaa aaaaaaaa 5816

<210> 34
<211> 1360
<212> PRT
<213> Homo sapiens

<400> 34
   Met Ala Ser Asp Ser Pro Ala Arg Ser Leu Asp Glu Ile Asp Leu Ser
   1 5 10 15
   Ala Leu Arg Asp Pro Ala Gly Ile Phe Glu Leu Val Glu Leu Val Gly
   20 25 30
   Asn Gly Thr Tyr Gly Gln Val Tyr Lys Gly Arg His Val Lys Thr Gly
   35 40 45
   Gln Leu Ala Ala Ile Lys Val Met Asp Val Thr Gly Asp Glu Glu Glu
       50                  55                  60
   Glu Ile Lys Gln Glu Ile Asn Met Leu Lys Lys Tyr Ser His His Arg
   65                  70                  75                  80
   Asn Ile Ala Thr Tyr Tyr Gly Ala Phe Ile Lys Lys Asn Pro Pro Gly
                   85                  90                  95
   Met Asp Asp Gln Leu Trp Leu Val Met Glu Phe Cys Gly Ala Gly Ser
               100                 105                 110
   Val Thr Asp Leu Ile Lys Asn Thr Lys Gly Asn Thr Leu Lys Glu Glu
           115                 120                 125
   Trp Ile Ala Tyr Ile Cys Arg Glu Ile Leu Arg Gly Leu Ser His Leu
       130                 135                 140
   His Gln His Lys Val Ile His Arg Asp Ile Lys Gly Gln Asn Val Leu
   145                 150                 155                 160
   Leu Thr Glu Asn Ala Glu Val Lys Leu Val Asp Phe Gly Val Ser Ala
                   165                 170                 175
   Gln Leu Asp Arg Thr Val Gly Arg Arg Asn Thr Phe Ile Gly Thr Pro
               180                 185                 190
   Tyr Trp Met Ala Pro Glu Val Ile Ala Cys Asp Glu Asn Pro Asp Ala
           195                 200                 205
   Thr Tyr Asp Phe Lys Ser Asp Leu Trp Ser Leu Gly Ile Thr Ala Ile
       210                 215                 220
   Glu Met Ala Glu Gly Ala Pro Pro Leu Cys Asp Met His Pro Met Arg
   225                 230                 235                 240
   Ala Leu Phe Leu Ile Pro Arg Asn Pro Ala Pro Arg Leu Lys Ser Lys
                   245                 250                 255
   Lys Trp Ser Lys Lys Phe Gln Ser Phe Ile Glu Ser Cys Leu Val Lys
               260                 265                 270
   Asn His Ser Gln Arg Pro Ala Thr Glu Gln Leu Met Lys His Pro Phe
           275                 280                 285
   Ile Arg Asp Gln Pro Asn Glu Arg Gln Val Arg Ile Gln Leu Lys Asp
   290 295 300
   His Ile Asp Arg Thr Lys Lys Lys Arg Gly Glu Lys Asp Glu Thr Glu
   305 310 315 320
   Tyr Glu Tyr Ser Gly Ser Glu Glu Glu Glu Glu Glu Asn Asp Ser Gly
   325 330 335
   Glu Pro Ser Ser Ile Leu Asn Leu Pro Gly Glu Ser Thr Leu Arg Arg
   340 345 350
   Asp Phe Leu Arg Leu Gln Leu Ala Asn Lys Glu Arg Ser Glu Ala Leu
   355 360 365
   Arg Arg Gln Gln Leu Glu Gln Gln Gln Arg Glu Asn Glu Glu His Lys
   370 375 380
   Arg Gln Leu Leu Ala Glu Arg Gln Lys Arg Ile Glu Glu Gln Lys Glu
   385 390 395 400
   Gln Arg Arg Arg Leu Glu Glu Gln Gln Arg Arg Glu Lys Glu Leu Arg
   405 410 415
   Lys Gln Gln Glu Arg Glu Gln Arg Arg His Tyr Glu Glu Gln Met Arg
   420 425 430
   Arg Glu Glu Glu Arg Arg Arg Ala Glu His Glu Gln Glu Tyr Ile Arg
   435 440 445
   Arg Gln Leu Glu Glu Glu Gln Arg Gln Leu Glu Ile Leu Gln Gln Gln
   450 455 460
   Leu Leu His Glu Gln Ala Leu Leu Leu Glu Tyr Lys Arg Lys Gln Leu
   465 470 475 480
   Glu Glu Gln Arg Gln Ala Glu Arg Leu Gln Arg Gln Leu Lys Gln Glu
   485 490 495
   Arg Asp Tyr Leu Val Ser Leu Gln His Gln Arg Gln Glu Gln Arg Pro
   500 505 510
   Val Glu Lys Lys Pro Leu Tyr His Tyr Lys Glu Gly Met Ser Pro Ser
   515 520 525
   Glu Lys Pro Ala Trp Ala Lys Glu Val Glu Glu Arg Ser Arg Leu Asn
   530 535 540
   Arg Gln Ser Ser Pro Ala Met Pro His Lys Val Ala Asn Arg Ile Ser
   545                 550                 555                 560
   Asp Pro Asn Leu Pro Pro Arg Ser Glu Ser Phe Ser Ile Ser Gly Val
                   565                 570                 575
   Gln Pro Ala Arg Thr Pro Pro Met Leu Arg Pro Val Asp Pro Gln Ile
               580                 585                 590
   Pro His Leu Val Ala Val Lys Ser Gln Gly Pro Ala Leu Thr Ala Ser
           595                 600                 605
   Gln Ser Val His Glu Gln Pro Thr Lys Gly Leu Ser Gly Phe Gln Glu
       610                 615                 620
   Ala Leu Asn Val Thr Ser His Arg Val Glu Met Pro Arg Gln Asn Ser
   625                 630                 635                 640
   Asp Pro Thr Ser Glu Asn Pro Pro Leu Pro Thr Arg Ile Glu Lys Phe
                   645                 650                 655
   Asp Arg Ser Ser Trp Leu Arg Gln Glu Glu Asp Ile Pro Pro Lys Val
               660                 665                 670
   Pro Gln Arg Thr Thr Ser Ile Ser Pro Ala Leu Ala Arg Lys Asn Ser
           675                 680                 685
   Pro Gly Asn Gly Ser Ala Leu Gly Pro Arg Leu Gly Ser Gln Pro Ile
       690                 695                 700
   Arg Ala Ser Asn Pro Asp Leu Arg Arg Thr Glu Pro Ile Leu Glu Ser
   705                 710                 715                 720
   Pro Leu Gln Arg Thr Ser Ser Gly Ser Ser Ser Ser Ser Ser Thr Pro
                   725                 730                 735
   Ser Ser Gln Pro Ser Ser Gln Gly Gly Ser Gln Pro Gly Ser Gln Ala
               740                 745                 750
   Gly Ser Ser Glu Arg Thr Arg Val Arg Ala Asn Ser Lys Ser Glu Gly
           755                 760                 765
   Ser Pro Val Leu Pro His Glu Pro Ala Lys Val Lys Pro Glu Glu Ser
       770                 775                 780
   Arg Asp Ile Thr Arg Pro Ser Arg Pro Ala Ser Tyr Lys Lys Ala Ile
   785                 790                 795                 800
   Asp Glu Asp Leu Thr Ala Leu Ala Lys Glu Leu Arg Glu Leu Arg Ile
   805 810 815
   Glu Glu Thr Asn Arg Pro Met Lys Lys Val Thr Asp Tyr Ser Ser Ser
   820 825 830
   Ser Glu Glu Ser Glu Ser Ser Glu Glu Glu Glu Glu Asp Gly Glu Ser
   835 840 845
   Glu Thr His Asp Gly Thr Val Ala Val Ser Asp Ile Pro Arg Leu Ile
   850 855 860
   Pro Thr Gly Ala Pro Gly Ser Asn Glu Gln Tyr Asn Val Gly Met Val
   865 870 875 880
   Gly Thr His Gly Leu Glu Thr Ser His Ala Asp Ser Phe Ser Gly Ser
   885 890 895
   Ile Ser Arg Glu Gly Thr Leu Met Ile Arg Glu Thr Ser Gly Glu Lys
   900 905 910
   Lys Arg Ser Gly His Ser Asp Ser Asn Gly Phe Ala Gly His Ile Asn
   915 920 925
   Leu Pro Asp Leu Val Gln Gln Ser His Ser Pro Ala Gly Thr Pro Thr
   930 935 940
   Glu Gly Leu Gly Arg Val Ser Thr His Ser Gln Glu Met Asp Ser Gly
   945 950 955 960
   Thr Glu Tyr Gly Met Gly Ser Ser Thr Lys Ala Ser Phe Thr Pro Phe
   965 970 975
   Val Asp Pro Arg Val Tyr Gln Thr Ser Pro Thr Asp Glu Asp Glu Glu
   980 985 990
   Asp Glu Glu Ser Ser Ala Ala Ala Leu Phe Thr Ser Glu Leu Leu Arg
   995 1000 1005
   Gln Glu Gln Ala Lys Leu Asn Glu Ala Arg Lys Ile Ser Val Val
   1010 1015 1020
   Asn Val Asn Pro Thr Asn Ile Arg Pro His Ser Asp Thr Pro Glu
   1025 1030 1035
   Ile Arg Lys Tyr Lys Lys Arg Phe Asn Ser Glu Ile Leu Cys Ala
   1040 1045 1050
   Ala Leu Trp Gly Val Asn Leu Leu Val Gly Thr Glu Asn Gly Leu
       1055                1060                1065
   Met Leu Leu Asp Arg Ser Gly Gln Gly Lys Val Tyr Asn Leu Ile
       1070                1075                1080
   Asn Arg Arg Arg Phe Gln Gln Met Asp Val Leu Glu Gly Leu Asn
       1085                1090                1095
   Val Leu Val Thr Ile Ser Gly Lys Lys Asn Lys Leu Arg Val Tyr
       1100                1105                1110
   Tyr Leu Ser Trp Leu Arg Asn Arg Ile Leu His Asn Asp Pro Glu
       1115                1120                1125
   Val Glu Lys Lys Gln Gly Trp Ile Thr Val Gly Asp Leu Glu Gly
       1130                1135                1140
   Cys Ile His Tyr Lys Val Val Lys Tyr Glu Arg Ile Lys Phe Leu
       1145                1150                1155
   Val Ile Ala Leu Lys Asn Ala Val Glu Ile Tyr Ala Trp Ala Pro
       1160                1165                1170
   Lys Pro Tyr His Lys Phe Met Ala Phe Lys Ser Phe Ala Asp Leu
       1175                1180                1185
   Gln His Lys Pro Leu Leu Val Asp Leu Thr Val Glu Glu Gly Gln
       1190                1195                1200
   Arg Leu Lys Val Ile Phe Gly Ser His Thr Gly Phe His Val Ile
       1205                1210                1215
   Asp Val Asp Ser Gly Asn Ser Tyr Asp Ile Tyr Ile Pro Ser His
       1220                1225                1230
   Ile Gln Gly Asn Ile Thr Pro His Ala Ile Val Ile Leu Pro Lys
       1235                1240                1245
   Thr Asp Gly Met Glu Met Leu Val Cys Tyr Glu Asp Glu Gly Val
       1250                1255                1260
   Tyr Val Asn Thr Tyr Gly Arg Ile Thr Lys Asp Val Val Leu Gln
       1265                1270                1275
   Trp Gly Glu Met Pro Thr Ser Val Ala Tyr Ile His Ser Asn Gln
   1280                1285                1290
   Ile Met Gly Trp Gly Glu Lys Ala Ile Glu Ile Arg Ser Val Glu
       1295                1300                1305
   Thr Gly His Leu Asp Gly Val Phe Met His Lys Arg Ala Gln Arg
       1310                1315                1320
   Leu Lys Phe Leu Cys Glu Arg Asn Asp Lys Val Phe Phe Ala Ser
       1325                1330                1335
   Val Arg Ser Gly Gly Ser Ser Gln Val Phe Phe Met Thr Leu Asn
       1340                1345                1350
   Arg Asn Ser Met Met Asn Trp
       1355                1360

<210>  35
<211>  5792
<212>  DNA
<213>  Homo sapiens

<220>
<221>  CDS
<222>  (346)..(4404)

<400>  35
   attataacag ccaatgcagc cgccatccac gcacaagcaa agaagcatgt cccatttaaa      60
   tacacccacg cagccccagc gcccttagcc gatcggggcg ctcagcccac acgcaccgct     120
   gctcggggct tggagatccg cgcaggctgg gctcccggac ccggcggacc gacgcgcgga     180
   ggatcgggat ccggcgctgt ggggctgggg tgggcggggg aggctgggcc cggggcctct     240
   ggcgcgacac ccgcatgagg acgcgagtga aatagaccaa ggtggaattt ccaagggaaa     300
   agcttcgggg tggttttggt ccatttctcc agcgaagaag tagac atg gcg agc gac      357
                                                     Met Ala Ser Asp
                                                     1
   tcc ccg gct cga agc ctg gat gaa ata gat ctc tcg gct ctg agg gac      405
   Ser Pro Ala Arg Ser Leu Asp Glu Ile Asp Leu Ser Ala Leu Arg Asp
   5                   10                  15                  20
   ccc gca ggg atc ttt gaa ttg gtg gaa ctt gtt gga aat gga aca tac      453
   Pro Ala Gly Ile Phe Glu Leu Val Glu Leu Val Gly Asn Gly Thr Tyr
                   25                  30                  35
   ggg caa gtt tat aag ggt cgt cat gtc aaa acg ggc cag ctt gca gcc      501
   Gly Gln Val Tyr Lys Gly Arg His Val Lys Thr Gly Gln Leu Ala Ala
               40                  45                  50
   atc aag gtt atg gat gtc aca ggg gat gaa gag gaa gaa atc aaa caa      549
   Ile Lys Val Met Asp Val Thr Gly Asp Glu Glu Glu Glu Ile Lys Gln
           55                  60                  65
   gaa att aac atg ttg aag aaa tat tct cat cac cgg aat att gct aca 597
   Glu Ile Asn Met Leu Lys Lys Tyr Ser His His Arg Asn Ile Ala Thr
   70 75 80
   tac tat ggt gct ttt atc aaa aag aac cca cca ggc atg gat gac caa 645
   Tyr Tyr Gly Ala Phe Ile Lys Lys Asn Pro Pro Gly Met Asp Asp Gln
   85 90 95 100
   ctt tgg ttg gtg atg gag ttt tgt ggt gct ggc tct gtc acc gac ctg 693
   Leu Trp Leu Val Met Glu Phe Cys Gly Ala Gly Ser Val Thr Asp Leu
   105 110 115
   atc aag aac aca aaa ggt aac acg ttg aaa gag gag tgg att gca tac 741
   Ile Lys Asn Thr Lys Gly Asn Thr Leu Lys Glu Glu Trp Ile Ala Tyr
   120 125 130
   atc tgc agg gaa atc tta cgg ggg ctg agt cac ctg cac cag cat aaa 789
   Ile Cys Arg Glu Ile Leu Arg Gly Leu Ser His Leu His Gln His Lys
   135 140 145
   gtg att cat cga gat att aaa ggg caa aat gtc ttg ctg act gaa aat 837
   Val Ile His Arg Asp Ile Lys Gly Gln Asn Val Leu Leu Thr Glu Asn
   150 155 160
   gca gaa gtt aaa cta gtg gac ttt gga gtc agt gct cag ctt gat cga 885
   Ala Glu Val Lys Leu Val Asp Phe Gly Val Ser Ala Gln Leu Asp Arg
   165 170 175 180
   aca gtg ggc agg agg aat act ttc att gga act ccc tac tgg atg gca 933
   Thr Val Gly Arg Arg Asn Thr Phe Ile Gly Thr Pro Tyr Trp Met Ala
   185 190 195
   cca gaa gtt att gcc tgt gat gaa aac cca gat gcc aca tat gat ttc 981
   Pro Glu Val Ile Ala Cys Asp Glu Asn Pro Asp Ala Thr Tyr Asp Phe
   200 205 210
   aag agt gac ttg tgg tct ttg ggt atc acc gcc att gaa atg gca gaa 1029
   Lys Ser Asp Leu Trp Ser Leu Gly Ile Thr Ala Ile Glu Met Ala Glu
   215 220 225
   ggt gct ccc cct ctc tgt gac atg cac ccc atg aga gct ctc ttc ctc 1077
   Gly Ala Pro Pro Leu Cys Asp Met His Pro Met Arg Ala Leu Phe Leu
   230 235 240
   atc ccc cgg aac cca gcg cct cgg ctg aag tct aag aag tgg tca aaa 1125
   Ile Pro Arg Asn Pro Ala Pro Arg Leu Lys Ser Lys Lys Trp Ser Lys
   245 250 255 260
   aaa ttc cag tca ttt att gag agc tgc ttg gta aag aat cac agc cag 1173
   Lys Phe Gln Ser Phe Ile Glu Ser Cys Leu Val Lys Asn His Ser Gln
   265 270 275
   cga cca gca aca gaa caa ttg atg aag cat cca ttt ata cga gac caa 1221
   Arg Pro Ala Thr Glu Gln Leu Met Lys His Pro Phe Ile Arg Asp Gln
   280 285 290
   cct aat gag cga cag gtc cgc att caa ctc aag gac cat att gat aga 1269
   Pro Asn Glu Arg Gln Val Arg Ile Gln Leu Lys Asp His Ile Asp Arg
   295 300 305
   aca aag aag aag cga gga gaa aaa gat gag aca gag tat gag tac agt 1317
   Thr Lys Lys Lys Arg Gly Glu Lys Asp Glu Thr Glu Tyr Glu Tyr Ser
   310                 315                 320
   gga agt gag gaa gaa gag gag gag aat gac tca gga gag ccc agc tcc      1365
   Gly Ser Glu Glu Glu Glu Glu Glu Asn Asp Ser Gly Glu Pro Ser Ser
   325                 330                 335                 340
   atc ctg aat ctg cca ggg gag tcg acg ctg cgg agg gac ttt ctg agg      1413
   Ile Leu Asn Leu Pro Gly Glu Ser Thr Leu Arg Arg Asp Phe Leu Arg
                   345                 350                 355
   ctg cag ctg gcc aac aag gag cgt tct gag gcc cta cgg agg cag cag      1461
   Leu Gln Leu Ala Asn Lys Glu Arg Ser Glu Ala Leu Arg Arg Gln Gln
               360                 365                 370
   ctg gag cag cag cag cgg gag aat gag gag cac aag cgg cag ctg ctg      1509
   Leu Glu Gln Gln Gln Arg Glu Asn Glu Glu His Lys Arg Gln Leu Leu
           375                 380                 385
   gcc gag cgt cag aag cgc atc gag gag cag aaa gag cag agg cgg cgg      1557
   Ala Glu Arg Gln Lys Arg Ile Glu Glu Gln Lys Glu Gln Arg Arg Arg
       390                 395                 400
   ctg gag gag caa caa agg cga gag aag gag ctg cgg aag cag cag gag      1605
   Leu Glu Glu Gln Gln Arg Arg Glu Lys Glu Leu Arg Lys Gln Gln Glu
   405                 410                 415                 420
   agg gag cag cgc cgg cac tat gag gag cag atg cgc cgg gag gag gag      1653
   Arg Glu Gln Arg Arg His Tyr Glu Glu Gln Met Arg Arg Glu Glu Glu
                   425                 430                 435
   agg agg cgt gcg gag cat gaa cag gaa tac atc agg cga cag tta gag      1701
   Arg Arg Arg Ala Glu His Glu Gln Glu Tyr Ile Arg Arg Gln Leu Glu
               440                 445                 450
   gag gag cag aga cag tta gag atc ttg cag cag cag cta ctg cat gaa      1749
   Glu Glu Gln Arg Gln Leu Glu Ile Leu Gln Gln Gln Leu Leu His Glu
           455                 460                 465
   caa gct cta ctt ctg gaa tat aag cgc aaa caa ttg gaa gaa cag aga      1797
   Gln Ala Leu Leu Leu Glu Tyr Lys Arg Lys Gln Leu Glu Glu Gln Arg
       470                 475                 480
   caa gca gaa aga ctg cag agg cag cta aag caa gaa aga gac tac tta      1845
   Gln Ala Glu Arg Leu Gln Arg Gln Leu Lys Gln Glu Arg Asp Tyr Leu
   485                 490                 495                 500
   gtt tcc ctt cag cat cag cgg cag gag cag agg cct gtg gag aag aag      1893
   Val Ser Leu Gln His Gln Arg Gln Glu Gln Arg Pro Val Glu Lys Lys
                   505                 510                 515
   cca ctg tac cat tac aaa gaa gga atg agt cct agt gag aag cca gca      1941
   Pro Leu Tyr His Tyr Lys Glu Gly Met Ser Pro Ser Glu Lys Pro Ala
               520                 525                 530
   tgg gcc aag gag gta gaa gaa cgg tca agg ctc aac cgg caa agt tcc      1989
   Trp Ala Lys Glu Val Glu Glu Arg Ser Arg Leu Asn Arg Gln Ser Ser
           535                 540                 545
   cct gcc atg cct cac aag gtt gcc aac agg ata tct gac ccc aac ctg      2037
   Pro Ala Met Pro His Lys Val Ala Asn Arg Ile Ser Asp Pro Asn Leu
       550                 555                 560
   ccc cca agg tcg gag tcc ttc agc att agt gga gtt cag cct gct cga      2085
   Pro Pro Arg Ser Glu Ser Phe Ser Ile Ser Gly Val Gln Pro Ala Arg
   565                 570                 575                 580
   aca ccc ccc atg ctc aga cca gtc gat ccc cag atc cca cat ctg gta      2133
   Thr Pro Pro Met Leu Arg Pro Val Asp Pro Gln Ile Pro His Leu Val
                   585                 590                 595
   gct gta aaa tcc cag gga cct gcc ttg acc gcc tcc cag tca gtg cac      2181
   Ala Val Lys Ser Gln Gly Pro Ala Leu Thr Ala Ser Gln Ser Val His
               600                 605                 610
   gag cag ccc aca aag ggc ctc tct ggg ttt cag gag gct ctg aac gtg      2229
   Glu Gln Pro Thr Lys Gly Leu Ser Gly Phe Gln Glu Ala Leu Asn Val
           615                 620                 625
   acc tcc cac cgc gtg gag atg cca cgc cag aac tca gat ccc acc tcg      2277
   Thr Ser His Arg Val Glu Met Pro Arg Gln Asn Ser Asp Pro Thr Ser
       630                 635                 640
   gaa aat cct cct ctc ccc act cgc att gaa aag ttt gac cga agc tct      2325
   Glu Asn Pro Pro Leu Pro Thr Arg Ile Glu Lys Phe Asp Arg Ser Ser
   645                 650                 655                 660
   tgg tta cga cag gaa gaa gac att cca cca aag gtg cct caa aga aca      2373
   Trp Leu Arg Gln Glu Glu Asp Ile Pro Pro Lys Val Pro Gln Arg Thr
                   665                 670                 675
   act tct ata tcc cca gca tta gcc aga aag aat tct cct ggg aat ggt      2421
   Thr Ser Ile Ser Pro Ala Leu Ala Arg Lys Asn Ser Pro Gly Asn Gly
               680                 685                 690
   agt gct ctg gga ccc aga cta gga tct caa ccc atc aga gca agc aac      2469
   Ser Ala Leu Gly Pro Arg Leu Gly Ser Gln Pro Ile Arg Ala Ser Asn
           695                 700                 705
   cct gat ctc cgg aga act gag ccc atc ttg gag agc ccc ttg cag agg      2517
   Pro Asp Leu Arg Arg Thr Glu Pro Ile Leu Glu Ser Pro Leu Gln Arg
       710                 715                 720
   acc agc agt ggc agt tcc tcc agc tcc agc acc cct agc tcc cag ccc      2565
   Thr Ser Ser Gly Ser Ser Ser Ser Ser Ser Thr Pro Ser Ser Gln Pro
   725                 730                 735                 740
   agc tcc caa gga ggc tcc cag cct gga tca caa gca gga tcc agt gaa      2613
   Ser Ser Gln Gly Gly Ser Gln Pro Gly Ser Gln Ala Gly Ser Ser Glu
                   745                 750                 755
   cgc acc aga gtt cga gcc aac agt aag tca gaa gga tca cct gtg ctc      2661
   Arg Thr Arg Val Arg Ala Asn Ser Lys Ser Glu Gly Ser Pro Val Leu
               760                 765                 770
   ccc cat gag cct gcc aag gtg aaa cca gaa gaa tcc agg gac att acc      2709
   Pro His Glu Pro Ala Lys Val Lys Pro Glu Glu Ser Arg Asp Ile Thr
           775                 780                 785
   cgg ccc agt cga cca gct gat ctg acg gca tta gcc aaa gaa cta aga      2757
   Arg Pro Ser Arg Pro Ala Asp Leu Thr Ala Leu Ala Lys Glu Leu Arg
       790                 795                 800
   gaa ctc cgg att gaa gaa aca aac cgc cca atg aag aag gtg act gat      2805
   Glu Leu Arg Ile Glu Glu Thr Asn Arg Pro Met Lys Lys Val Thr Asp
   805                 810                 815                 820
   tac tcc tcc tcc agt gag gag tca gaa agt agc gag gaa gag gag gaa 2853
   Tyr Ser Ser Ser Ser Glu Glu Ser Glu Ser Ser Glu Glu Glu Glu Glu
   825 830 835
   gat gga gag agc gag acc cat gat ggg aca gtg gct gtc agc gac ata 2901
   Asp Gly Glu Ser Glu Thr His Asp Gly Thr Val Ala Val Ser Asp Ile
   840 845 850
   ccc aga ctg ata cca aca gga gct cca ggc agc aac gag cag tac aat 2949
   Pro Arg Leu Ile Pro Thr Gly Ala Pro Gly Ser Asn Glu Gln Tyr Asn
   855 860 865
   gtg gga atg gtg ggg acg cat ggg ctg gag acc tct cat gcg gac agt 2997
   Val Gly Met Val Gly Thr His Gly Leu Glu Thr Ser His Ala Asp Ser
   870 875 880
   ttc agc ggc agt att tca aga gaa gga acc ttg atg att aga gag acg 3045
   Phe Ser Gly Ser Ile Ser Arg Glu Gly Thr Leu Met Ile Arg Glu Thr
   885 890 895 900
   tct gga gag aag aag cga tct ggc cac agt gac agc aat ggc ttt gct 3093
   Ser Gly Glu Lys Lys Arg Ser Gly His Ser Asp Ser Asn Gly Phe Ala
   905 910 915
   ggc cac atc aac ctc cct gac ctg gtg cag cag agc cat tct cca gct 3141
   Gly His Ile Asn Leu Pro Asp Leu Val Gln Gln Ser His Ser Pro Ala
   920 925 930
   gga acc ccg act gag gga ctg ggg cgc gtc tca acc cat tcc cag gag 3189
   Gly Thr Pro Thr Glu Gly Leu Gly Arg Val Ser Thr His Ser Gln Glu
   935 940 945
   atg gac tct ggg act gaa tat ggc atg ggg agc agc acc aaa gcc tcc 3237
   Met Asp Ser Gly Thr Glu Tyr Gly Met Gly Ser Ser Thr Lys Ala Ser
   950 955 960
   ttc acc ccc ttt gtg gac ccc aga gta tac cag acg tct ccc act gat 3285
   Phe Thr Pro Phe Val Asp Pro Arg Val Tyr Gln Thr Ser Pro Thr Asp
   965 970 975 980
   gaa gat gaa gag gat gag gaa tca tca gcc gca gct ctg ttt act agc 3333
   Glu Asp Glu Glu Asp Glu Glu Ser Ser Ala Ala Ala Leu Phe Thr Ser
   985 990 995
   gaa ctt ctt agg caa gaa cag gcc aaa ctc aat gaa gca aga aag 3378
   Glu Leu Leu Arg Gln Glu Gln Ala Lys Leu Asn Glu Ala Arg Lys
   1000 1005 1010
   att tcg gtg gta aat gta aac cca acc aac att cgg cct cat agc 3423
   Ile Ser Val Val Asn Val Asn Pro Thr Asn Ile Arg Pro His Ser
   1015 1020 1025
   gac aca cca gaa atc aga aaa tac aag aaa cga ttc aac tca gaa 3468
   Asp Thr Pro Glu Ile Arg Lys Tyr Lys Lys Arg Phe Asn Ser Glu
   1030 1035 1040
   ata ctt tgt gca gct ctg tgg ggt gta aac ctt ctg gtg ggg act 3513
   Ile Leu Cys Ala Ala Leu Trp Gly Val Asn Leu Leu Val Gly Thr
   1045 1050 1055
   gaa aat ggc ctg atg ctt ttg gac cga agt ggg caa ggc aaa gtc 3558
   Glu Asn Gly Leu Met Leu Leu Asp Arg Ser Gly Gln Gly Lys Val
   1060 1065 1070
   tat aat ctg atc aac cgg agg cga ttt cag cag atg gat gtg cta 3603
   Tyr Asn Leu Ile Asn Arg Arg Arg Phe Gln Gln Met Asp Val Leu
   1075 1080 1085
   gag gga ctg aat gtc ctt gtg aca att tca gga aag aag aat aag 3648
   Glu Gly Leu Asn Val Leu Val Thr Ile Ser Gly Lys Lys Asn Lys
   1090 1095 1100
   cta cga gtt tac tat ctt tca tgg tta aga aac aga ata cta cat 3693
   Leu Arg Val Tyr Tyr Leu Ser Trp Leu Arg Asn Arg Ile Leu His
   1105 1110 1115
   aat gac cca gaa gta gaa aag aaa caa ggc tgg atc act gtt ggg 3738
   Asn Asp Pro Glu Val Glu Lys Lys Gln Gly Trp Ile Thr Val Gly
   1120 1125 1130
   gac ttg gaa ggc tgt ata cat tat aaa gtt gtt aaa tat gaa agg 3783
   Asp Leu Glu Gly Cys Ile His Tyr Lys Val Val Lys Tyr Glu Arg
   1135 1140 1145
   atc aaa ttt ttg gtg att gcc tta aag aat gct gtg gaa ata tat 3828
   Ile Lys Phe Leu Val Ile Ala Leu Lys Asn Ala Val Glu Ile Tyr
   1150 1155 1160
   gct tgg gct cct aaa ccg tat cat aaa ttc atg gca ttt aag tct 3873
   Ala Trp Ala Pro Lys Pro Tyr His Lys Phe Met Ala Phe Lys Ser
   1165 1170 1175
   ttt gca gat ctc cag cac aag cct ctg cta gtt gat ctc acg gta 3918
   Phe Ala Asp Leu Gln His Lys Pro Leu Leu Val Asp Leu Thr Val
   1180 1185 1190
   gaa gaa ggt caa aga tta aag gtt att ttt ggt tca cac act ggt 3963
   Glu Glu Gly Gln Arg Leu Lys Val Ile Phe Gly Ser His Thr Gly
   1195 1200 1205
   ttc cat gta att gat gtt gat tca gga aac tct tat gat atc tac 4008
   Phe His Val Ile Asp Val Asp Ser Gly Asn Ser Tyr Asp Ile Tyr
   1210 1215 1220
   ata cca tct cat att cag ggc aat atc act cct cat gct att gtc 4053
   Ile Pro Ser His Ile Gln Gly Asn Ile Thr Pro His Ala Ile Val
   1225 1230 1235
   atc ttg cct aaa aca gat gga atg gaa atg ctt gtt tgc tat gag 4098
   Ile Leu Pro Lys Thr Asp Gly Met Glu Met Leu Val Cys Tyr Glu
   1240 1245 1250
   gat gag ggg gtg tat gta aac acc tat ggc cgg ata act aag gat 4143
   Asp Glu Gly Val Tyr Val Asn Thr Tyr Gly Arg Ile Thr Lys Asp
   1255 1260 1265
   gtg gtg ctc caa tgg gga gaa atg ccc acg tct gtg gcc tac att 4188
   Val Val Leu Gln Trp Gly Glu Met Pro Thr Ser Val Ala Tyr Ile
   1270 1275 1280
   cat tcc aat cag ata atg ggc tgg ggc gag aaa gct att gag atc 4233
   His Ser Asn Gln Ile Met Gly Trp Gly Glu Lys Ala Ile Glu Ile
   1285 1290 1295
   cgg tca gtg gaa aca gga cat ttg gat gga gta ttt atg cat aag 4278
   Arg Ser Val Glu Thr Gly His Leu Asp Gly Val Phe Met His Lys
   1300                1305                1310
   cga gct caa agg  tta aag ttt cta tgt  gaa aga aat gat aag  gta      4323
   Arg Ala Gln Arg  Leu Lys Phe Leu Cys  Glu Arg Asn Asp Lys  Val
               1315                1320                1325
   ttt ttt gca tcc  gtg cga tct gga gga  agt agc caa gtg ttt  ttc      4368
   Phe Phe Ala Ser  Val Arg Ser Gly Gly  Ser Ser Gln Val Phe  Phe
               1330                1335                1340
   atg acc ctc aac  aga aat tcc atg atg  aac tgg taa cagaagagca        4414
   Met Thr Leu Asn  Arg Asn Ser Met Met  Asn Trp
               1345                1350
   cttggcactt atcttcatgg cgttatttct aatttaaaag aacataactc atgtggactt   4474
   atgccagtct agaggcagaa tcagaaggct tggttgaaca tatcgctttc cctttttcct   4534
   ctccctccgc ccctcccagt acagtccatc tttcaatgtt gcagcctggt tgagaaggag   4594
   agaaaaaggt ggcaggaatt tccaggagat ccccaagaat gctgccttgt ctgtggacaa   4654
   agatggacca tgtgcccttc ggaattaggg atagaaacaa atattgtgtg ctcttaacga   4714
   ttaagctgtg ttatggtggg ttttcaggtt tttacctttt ttctttaccc ctttactctg   4774
   caagaatggg gaaagaatgc atactgcgaa aatgagtctt ttaaattctg tctgcctact   4834
   agttttaagt atatggtatg ttgtaaaatt tccaatgatg agagacagca caataaatgt   4894
   accttatctc cttaggctga aggccataac tacatagtgg agtaatttaa gaactctctt   4954
   gccttcacca acccaaaagg ttgctttttg atagcaactg gctaatgaat ttttaaaaag   5014
   agaagaaaaa tactagtttt cccctctttt gggaaataga ttttaaatgg ctaaactact   5074
   agccttaaaa ctactagtct aataaaatca actaccactt ttgtgaatct gacaggccac   5134
   atttttatat ggccctttac agaatggagt gtgttgaaca ggatactaac gccattgagt   5194
   tgagctggcc tagcgatgga gggacactct aacacaactt tccctcagct attatgcaac   5254
   agatcaggga aaaagatggg atgacagatg gggtcagaca gaaagagctt ctgggaaaca   5314
   agcttacata gtctttttta aaatgcacaa agcctcccag ctaagaggtc acttggtttg   5374
   ggcttcatta ggactgagac tttgttgagt tctttctggg acttggagag tggatgatat   5434
   tcaggctctg aacattccca gcgctctccc gagggtgcca ctttctcaag atgaaaactg   5494
   tgactgaaaa aattaataat aaatgtttct gagctgcctg tgttctccct gtgtgggtga   5554
   gagaagggac tagactccta agcctgcctc agatacaaga ggcatcattg gctccaattt   5614
   tagagaactt gaaagcaagg ctttggacaa aattttgaga ccctaatcac tttaccttcc   5674
   tccaaattac ccaacatacg gtaaacaaca tttgtgcaga agtatgtatg tatttagttc   5734
   aggttgactt gtgtccttat aaactcttac tcaaatgatt tgaactttaa aaaaaaaa      5792

<210>  36
<211>  1352

<212>  PRT
<213>  Homo sapiens

<400>  36

Met Ala Ser Asp Ser Pro Ala Arg Ser Leu Asp Glu Ile Asp Leu Ser
1               5                   10                  15

Ala Leu Arg Asp Pro Ala Gly Ile Phe Glu Leu Val Glu Leu Val Gly
            20                  25                  30

Asn Gly Thr Tyr Gly Gln Val Tyr Lys Gly Arg His Val Lys Thr Gly
        35                  40                  45

Gln Leu Ala Ala Ile Lys Val Met Asp Val Thr Gly Asp Glu Glu Glu
    50                  55                  60

Glu Ile Lys Gln Glu Ile Asn Met Leu Lys Lys Tyr Ser His His Arg
65                  70                  75                  80

Asn Ile Ala Thr Tyr Tyr Gly Ala Phe Ile Lys Lys Asn Pro Pro Gly
                85                  90                  95

Met Asp Asp Gln Leu Trp Leu Val Met Glu Phe Cys Gly Ala Gly Ser
            100                 105                 110

Val Thr Asp Leu Ile Lys Asn Thr Lys Gly Asn Thr Leu Lys Glu Glu
        115                 120                 125

Trp Ile Ala Tyr Ile Cys Arg Glu Ile Leu Arg Gly Leu Ser His Leu
    130                 135                 140

His Gln His Lys Val Ile His Arg Asp Ile Lys Gly Gln Asn Val Leu
145                 150                 155                 160

Leu Thr Glu Asn Ala Glu Val Lys Leu Val Asp Phe Gly Val Ser Ala
                165                 170                 175

Gln Leu Asp Arg Thr Val Gly Arg Arg Asn Thr Phe Ile Gly Thr Pro
            180                 185                 190

Tyr Trp Met Ala Pro Glu Val Ile Ala Cys Asp Glu Asn Pro Asp Ala
        195                 200                 205

Thr Tyr Asp Phe Lys Ser Asp Leu Trp Ser Leu Gly Ile Thr Ala Ile
    210                 215                 220

Glu Met Ala Glu Gly Ala Pro Pro Leu Cys Asp Met His Pro Met Arg
225                 230                 235                 240

Ala Leu Phe Leu Ile Pro Arg Asn Pro Ala Pro Arg Leu Lys Ser Lys
                245                 250                 255

Lys Trp Ser Lys Lys Phe Gln Ser Phe Ile Glu Ser Cys Leu Val Lys
            260                 265                 270

Asn His Ser Gln Arg Pro Ala Thr Glu Gln Leu Met Lys His Pro Phe
        275                 280                 285

Ile Arg Asp Gln Pro Asn Glu Arg Gln Val Arg Ile Gln Leu Lys Asp
    290                 295                 300

His Ile Asp Arg Thr Lys Lys Lys Arg Gly Glu Lys Asp Glu Thr Glu
305                 310                 315                 320

Tyr Glu Tyr Ser Gly Ser Glu Glu Glu Glu Glu Glu Asn Asp Ser Gly
                325                 330                 335

Glu Pro Ser Ser Ile Leu Asn Leu Pro Gly Glu Ser Thr Leu Arg Arg
            340                 345                 350

Asp Phe Leu Arg Leu Gln Leu Ala Asn Lys Glu Arg Ser Glu Ala Leu
        355                 360                 365

Arg Arg Gln Gln Leu Glu Gln Gln Gln Arg Glu Asn Glu Glu His Lys
    370                 375                 380

Arg Gln Leu Leu Ala Glu Arg Gln Lys Arg Ile Glu Glu Gln Lys Glu
385                 390                 395                 400

Gln Arg Arg Arg Leu Glu Glu Gln Gln Arg Arg Glu Lys Glu Leu Arg
                405                 410                 415

Lys Gln Gln Glu Arg Glu Gln Arg Arg His Tyr Glu Glu Gln Met Arg
            420                 425                 430

Arg Glu Glu Glu Arg Arg Arg Ala Glu His Glu Gln Glu Tyr Ile Arg
        435                 440                 445

Arg Gln Leu Glu Glu Glu Gln Arg Gln Leu Glu Ile Leu Gln Gln Gln
    450                 455                 460

Leu Leu His Glu Gln Ala Leu Leu Leu Glu Tyr Lys Arg Lys Gln Leu
465                 470                 475                 480

Glu Glu Gln Arg Gln Ala Glu Arg Leu Gln Arg Gln Leu Lys Gln Glu

485                 490                 495

Arg Asp Tyr Leu Val Ser Leu Gln His Gln Arg Gln Glu Gln Arg Pro
            500                 505                 510

Val Glu Lys Lys Pro Leu Tyr His Tyr Lys Glu Gly Met Ser Pro Ser
        515                 520                 525

Glu Lys Pro Ala Trp Ala Lys Glu Val Glu Glu Arg Ser Arg Leu Asn
    530                 535                 540

Arg Gln Ser Ser Pro Ala Met Pro His Lys Val Ala Asn Arg Ile Ser
545                 550                 555                 560

Asp Pro Asn Leu Pro Pro Arg Ser Glu Ser Phe Ser Ile Ser Gly Val
                565                 570                 575

Gln Pro Ala Arg Thr Pro Pro Met Leu Arg Pro Val Asp Pro Gln Ile
            580                 585                 590

Pro His Leu Val Ala Val Lys Ser Gln Gly Pro Ala Leu Thr Ala Ser
        595                 600                 605

Gln Ser Val His Glu Gln Pro Thr Lys Gly Leu Ser Gly Phe Gln Glu
    610                 615                 620

Ala Leu Asn Val Thr Ser His Arg Val Glu Met Pro Arg Gln Asn Ser
625                 630                 635                 640

Asp Pro Thr Ser Glu Asn Pro Pro Leu Pro Thr Arg Ile Glu Lys Phe
                645                 650                 655

Asp Arg Ser Ser Trp Leu Arg Gln Glu Glu Asp Ile Pro Pro Lys Val
            660                 665                 670

Pro Gln Arg Thr Thr Ser Ile Ser Pro Ala Leu Ala Arg Lys Asn Ser
        675                 680                 685

Pro Gly Asn Gly Ser Ala Leu Gly Pro Arg Leu Gly Ser Gln Pro Ile
    690                 695                 700

Arg Ala Ser Asn Pro Asp Leu Arg Arg Thr Glu Pro Ile Leu Glu Ser
705                 710                 715                 720

Pro Leu Gln Arg Thr Ser Ser Gly Ser Ser Ser Ser Ser Ser Thr Pro
                725                 730                 735

Ser Ser Gln Pro Ser Ser Gln Gly Gly Ser Gln Pro Gly Ser Gln Ala
            740                 745                 750

Gly Ser Ser Glu Arg Thr Arg Val Arg Ala Asn Ser Lys Ser Glu Gly
        755                 760                 765

Ser Pro Val Leu Pro His Glu Pro Ala Lys Val Lys Pro Glu Glu Ser
    770                 775                 780

Arg Asp Ile Thr Arg Pro Ser Arg Pro Ala Asp Leu Thr Ala Leu Ala
785                 790                 795                 800

Lys Glu Leu Arg Glu Leu Arg Ile Glu Glu Thr Asn Arg Pro Met Lys
                805                 810                 815

Lys Val Thr Asp Tyr Ser Ser Ser Ser Glu Glu Ser Glu Ser Ser Glu
            820                 825                 830

Glu Glu Glu Glu Asp Gly Glu Ser Glu Thr His Asp Gly Thr Val Ala
        835                 840                 845

Val Ser Asp Ile Pro Arg Leu Ile Pro Thr Gly Ala Pro Gly Ser Asn
    850                 855                 860

Glu Gln Tyr Asn Val Gly Met Val Gly Thr His Gly Leu Glu Thr Ser
865                 870                 875                 880

His Ala Asp Ser Phe Ser Gly Ser Ile Ser Arg Glu Gly Thr Leu Met
                885                 890                 895

Ile Arg Glu Thr Ser Gly Glu Lys Lys Arg Ser Gly His Ser Asp Ser
            900                 905                 910

Asn Gly Phe Ala Gly His Ile Asn Leu Pro Asp Leu Val Gln Gln Ser
        915                 920                 925

His Ser Pro Ala Gly Thr Pro Thr Glu Gly Leu Gly Arg Val Ser Thr
    930                 935                 940

His Ser Gln Glu Met Asp Ser Gly Thr Glu Tyr Gly Met Gly Ser Ser
945                 950                 955                 960

Thr Lys Ala Ser Phe Thr Pro Phe Val Asp Pro Arg Val Tyr Gln Thr
                965                 970                 975

Ser Pro Thr Asp Glu Asp Glu Glu Asp Glu Glu Ser Ser Ala Ala Ala
            980                 985                 990

Leu Phe Thr Ser Glu Leu Leu Arg Gln Glu Gln Ala Lys Leu Asn Glu
        995                 1000                1005

Ala Arg Lys Ile Ser Val Val Asn Val Asn Pro Thr Asn Ile Arg
    1010                1015                1020

Pro His Ser Asp Thr Pro Glu Ile Arg Lys Tyr Lys Lys Arg Phe
    1025                1030                1035

Asn Ser Glu Ile Leu Cys Ala Ala Leu Trp Gly Val Asn Leu Leu
    1040                1045                1050

Val Gly Thr Glu Asn Gly Leu Met Leu Leu Asp Arg Ser Gly Gln
    1055                1060                1065

Gly Lys Val Tyr Asn Leu Ile Asn Arg Arg Arg Phe Gln Gln Met
    1070                1075                1080

Asp Val Leu Glu Gly Leu Asn Val Leu Val Thr Ile Ser Gly Lys
    1085                1090                1095

Lys Asn Lys Leu Arg Val Tyr Tyr Leu Ser Trp Leu Arg Asn Arg
    1100                1105                1110

Ile Leu His Asn Asp Pro Glu Val Glu Lys Lys Gln Gly Trp Ile
    1115                1120                1125

Thr Val Gly Asp Leu Glu Gly Cys Ile His Tyr Lys Val Val Lys
    1130                1135                1140

Tyr Glu Arg Ile Lys Phe Leu Val Ile Ala Leu Lys Asn Ala Val
    1145                1150                1155

Glu Ile Tyr Ala Trp Ala Pro Lys Pro Tyr His Lys Phe Met Ala
    1160                1165                1170

Phe Lys Ser Phe Ala Asp Leu Gln His Lys Pro Leu Leu Val Asp
    1175                1180                1185

Leu Thr Val Glu Glu Gly Gln Arg Leu Lys Val Ile Phe Gly Ser
    1190                1195                1200

His Thr Gly Phe His Val Ile Asp Val Asp Ser Gly Asn Ser Tyr
    1205                1210                1215

Asp Ile Tyr Ile Pro Ser His Ile Gln Gly Asn Ile Thr Pro His
    1220                1225                1230

Ala Ile Val Ile Leu Pro Lys Thr Asp Gly Met Glu Met Leu Val
    1235                1240                1245

Cys Tyr Glu Asp Glu Gly Val Tyr Val Asn Thr Tyr Gly Arg Ile
    1250                1255                1260

Thr Lys Asp Val Val Leu Gln Trp Gly Glu Met Pro Thr Ser Val
    1265                1270                1275

Ala Tyr Ile His Ser Asn Gln Ile Met Gly Trp Gly Glu Lys Ala
    1280                1285                1290

Ile Glu Ile Arg Ser Val Glu Thr Gly His Leu Asp Gly Val Phe
    1295                1300                1305

Met His Lys Arg Ala Gln Arg Leu Lys Phe Leu Cys Glu Arg Asn
    1310                1315                1320

Asp Lys Val Phe Phe Ala Ser Val Arg Ser Gly Gly Ser Ser Gln
    1325                1330                1335

Val Phe Phe Met Thr Leu Asn Arg Asn Ser Met Met Asn Trp
    1340                1345                1350

<210> 37
<211> 5729
<212> DNA
<213> Homo sapiens

<220>
<221> CDS
<222> (346)..(4341)

<400> 37
attataacag ccaatgcagc cgccatccac gcacaagcaa agaagcatgt cccatttaaa 60

tacacccacg cagccccagc gcccttagcc gatcggggcg ctcagcccac acgcaccgct 120

gctcggggct tggagatccg cgcaggctgg gctcccggac ccggcggacc gacgcgcgga 180

ggatcgggat ccggcgctgt ggggctgggg tgggcggggg aggctgggcc cggggcctct 240

ggcgcgacac ccgcatgagg acgcgagtga aatagaccaa ggtggaattt ccaagggaaa 300

agcttcgggg tggttttggt ccatttctcc agcgaagaag tagac atg gcg agc gac 357
Met Ala Ser Asp
1

tcc ccg gct cga agc ctg gat gaa ata gat ctc tcg gct ctg agg gac 405
Ser Pro Ala Arg Ser Leu Asp Glu Ile Asp Leu Ser Ala Leu Arg Asp
5                   10                  15                  20

ccc gca ggg atc ttt gaa ttg gtg gaa ctt gtt gga aat gga aca tac 453
Pro Ala Gly Ile Phe Glu Leu Val Glu Leu Val Gly Asn Gly Thr Tyr
                25                  30                  35

ggg caa gtt tat aag ggt cgt cat gtc aaa acg ggc cag ctt gca gcc 501
Gly Gln Val Tyr Lys Gly Arg His Val Lys Thr Gly Gln Leu Ala Ala
            40                  45                  50

atc aag gtt atg gat gtc aca ggg gat gaa gag gaa gaa atc aaa caa 549
Ile Lys Val Met Asp Val Thr Gly Asp Glu Glu Glu Glu Ile Lys Gln
        55                  60                  65

gaa att aac atg ttg aag aaa tat tct cat cac cgg aat att gct aca 597
Glu Ile Asn Met Leu Lys Lys Tyr Ser His His Arg Asn Ile Ala Thr
    70                  75                  80

tac tat ggt gct ttt atc aaa aag aac cca cca ggc atg gat gac caa 645
Tyr Tyr Gly Ala Phe Ile Lys Lys Asn Pro Pro Gly Met Asp Asp Gln
85                  90                  95                  100

ctt tgg ttg gtg atg gag ttt tgt ggt gct ggc tct gtc acc gac ctg 693
Leu Trp Leu Val Met Glu Phe Cys Gly Ala Gly Ser Val Thr Asp Leu
                105                 110                 115

atc aag aac aca aaa ggt aac acg ttg aaa gag gag tgg att gca tac 741
Ile Lys Asn Thr Lys Gly Asn Thr Leu Lys Glu Glu Trp Ile Ala Tyr
            120                 125                 130

atc tgc agg gaa atc tta cgg ggg ctg agt cac ctg cac cag cat aaa 789
Ile Cys Arg Glu Ile Leu Arg Gly Leu Ser His Leu His Gln His Lys
        135                 140                 145

gtg att cat cga gat att aaa ggg caa aat gtc ttg ctg act gaa aat 837
Val Ile His Arg Asp Ile Lys Gly Gln Asn Val Leu Leu Thr Glu Asn
    150                 155                 160

gca gaa gtt aaa cta gtg gac ttt gga gtc agt gct cag ctt gat cga 885
Ala Glu Val Lys Leu Val Asp Phe Gly Val Ser Ala Gln Leu Asp Arg
165                 170                 175                 180

aca gtg ggc agg agg aat act ttc att gga act ccc tac tgg atg gca 933
Thr Val Gly Arg Arg Asn Thr Phe Ile Gly Thr Pro Tyr Trp Met Ala
                185                 190                 195

cca gaa gtt att gcc tgt gat gaa aac cca gat gcc aca tat gat ttc 981
Pro Glu Val Ile Ala Cys Asp Glu Asn Pro Asp Ala Thr Tyr Asp Phe
            200                 205                 210

aag agt gac ttg tgg tct ttg ggt atc acc gcc att gaa atg gca gaa 1029
Lys Ser Asp Leu Trp Ser Leu Gly Ile Thr Ala Ile Glu Met Ala Glu
        215                 220                 225

ggt gct ccc cct ctc tgt gac atg cac ccc atg aga gct ctc ttc ctc 1077
Gly Ala Pro Pro Leu Cys Asp Met His Pro Met Arg Ala Leu Phe Leu
    230                 235                 240

atc ccc cgg aac cca gcg cct cgg ctg aag tct aag aag tgg tca aaa 1125
Ile Pro Arg Asn Pro Ala Pro Arg Leu Lys Ser Lys Lys Trp Ser Lys
245                 250                 255                 260

aaa ttc cag tca ttt att gag agc tgc ttg gta aag aat cac agc cag 1173
Lys Phe Gln Ser Phe Ile Glu Ser Cys Leu Val Lys Asn His Ser Gln
                265                 270                 275

cga cca gca aca gaa caa ttg atg aag cat cca ttt ata cga gac caa     1221
Arg Pro Ala Thr Glu Gln Leu Met Lys His Pro Phe Ile Arg Asp Gln
            280                 285                 290

cct aat gag cga cag gtc cgc att caa ctc aag gac cat att gat aga     1269
Pro Asn Glu Arg Gln Val Arg Ile Gln Leu Lys Asp His Ile Asp Arg
        295                 300                 305

aca aag aag aag cga gga gaa aaa gat gag aca gag tat gag tac agt     1317
Thr Lys Lys Lys Arg Gly Glu Lys Asp Glu Thr Glu Tyr Glu Tyr Ser
    310                 315                 320

gga agt gag gaa gaa gag gag gag aat gac tca gga gag ccc agc tcc     1365
Gly Ser Glu Glu Glu Glu Glu Glu Asn Asp Ser Gly Glu Pro Ser Ser
325                 330                 335                 340

atc ctg aat ctg cca ggg gag tcg acg ctg cgg agg gac ttt ctg agg     1413
Ile Leu Asn Leu Pro Gly Glu Ser Thr Leu Arg Arg Asp Phe Leu Arg
                345                 350                 355

ctg cag ctg gcc aac aag gag cgt tct gag gcc cta cgg agg cag cag     1461
Leu Gln Leu Ala Asn Lys Glu Arg Ser Glu Ala Leu Arg Arg Gln Gln
            360                 365                 370

ctg gag cag cag cag cgg gag aat gag gag cac aag cgg cag ctg ctg     1509
Leu Glu Gln Gln Gln Arg Glu Asn Glu Glu His Lys Arg Gln Leu Leu
        375                 380                 385

gcc gag cgt cag aag cgc atc gag gag cag aaa gag cag agg cgg cgg     1557
Ala Glu Arg Gln Lys Arg Ile Glu Glu Gln Lys Glu Gln Arg Arg Arg
    390                 395                 400

ctg gag gag caa caa agg cga gag aag gag ctg cgg aag cag cag gag     1605
Leu Glu Glu Gln Gln Arg Arg Glu Lys Glu Leu Arg Lys Gln Gln Glu
405                 410                 415                 420

agg gag cag cgc cgg cac tat gag gag cag atg cgc cgg gag gag gag     1653
Arg Glu Gln Arg Arg His Tyr Glu Glu Gln Met Arg Arg Glu Glu Glu
                425                 430                 435

agg agg cgt gcg gag cat gaa cag gaa tat aag cgc aaa caa ttg gaa     1701
Arg Arg Arg Ala Glu His Glu Gln Glu Tyr Lys Arg Lys Gln Leu Glu
            440                 445                 450

gaa cag aga caa gca gaa aga ctg cag agg cag cta aag caa gaa aga     1749
Glu Gln Arg Gln Ala Glu Arg Leu Gln Arg Gln Leu Lys Gln Glu Arg
        455                 460                 465

gac tac tta gtt tcc ctt cag cat cag cgg cag gag cag agg cct gtg     1797
Asp Tyr Leu Val Ser Leu Gln His Gln Arg Gln Glu Gln Arg Pro Val
    470                 475                 480

gag aag aag cca ctg tac cat tac aaa gaa gga atg agt cct agt gag     1845
Glu Lys Lys Pro Leu Tyr His Tyr Lys Glu Gly Met Ser Pro Ser Glu
485                 490                 495                 500

aag cca gca tgg gcc aag gag gta gaa gaa cgg tca agg ctc aac cgg     1893
Lys Pro Ala Trp Ala Lys Glu Val Glu Glu Arg Ser Arg Leu Asn Arg
                505                 510                 515

caa agt tcc cct gcc atg cct cac aag gtt gcc aac agg ata tct gac     1941
Gln Ser Ser Pro Ala Met Pro His Lys Val Ala Asn Arg Ile Ser Asp

520                 525                 530

ccc aac ctg ccc cca agg tcg gag tcc ttc agc att agt gga gtt cag        1989
Pro Asn Leu Pro Pro Arg Ser Glu Ser Phe Ser Ile Ser Gly Val Gln
        535                 540                 545

cct gct cga aca ccc ccc atg ctc aga cca gtc gat ccc cag atc cca        2037
Pro Ala Arg Thr Pro Pro Met Leu Arg Pro Val Asp Pro Gln Ile Pro
    550                 555                 560

cat ctg gta gct gta aaa tcc cag gga cct gcc ttg acc gcc tcc cag        2085
His Leu Val Ala Val Lys Ser Gln Gly Pro Ala Leu Thr Ala Ser Gln
565                 570                 575                 580

tca gtg cac gag cag ccc aca aag ggc ctc tct ggg ttt cag gag gct        2133
Ser Val His Glu Gln Pro Thr Lys Gly Leu Ser Gly Phe Gln Glu Ala
                585                 590                 595

ctg aac gtg acc tcc cac cgc gtg gag atg cca cgc cag aac tca gat        2181
Leu Asn Val Thr Ser His Arg Val Glu Met Pro Arg Gln Asn Ser Asp
            600                 605                 610

ccc acc tcg gaa aat cct cct ctc ccc act cgc att gaa aag ttt gac        2229
Pro Thr Ser Glu Asn Pro Pro Leu Pro Thr Arg Ile Glu Lys Phe Asp
        615                 620                 625

cga agc tct tgg tta cga cag gaa gaa gac att cca cca aag gtg cct        2277
Arg Ser Ser Trp Leu Arg Gln Glu Glu Asp Ile Pro Pro Lys Val Pro
    630                 635                 640

caa aga aca act tct ata tcc cca gca tta gcc aga aag aat tct cct        2325
Gln Arg Thr Thr Ser Ile Ser Pro Ala Leu Ala Arg Lys Asn Ser Pro
645                 650                 655                 660

ggg aat ggt agt gct ctg gga ccc aga cta gga tct caa ccc atc aga        2373
Gly Asn Gly Ser Ala Leu Gly Pro Arg Leu Gly Ser Gln Pro Ile Arg
                665                 670                 675

gca agc aac cct gat ctc cgg aga act gag ccc atc ttg gag agc ccc        2421
Ala Ser Asn Pro Asp Leu Arg Arg Thr Glu Pro Ile Leu Glu Ser Pro
            680                 685                 690

ttg cag agg acc agc agt ggc agt tcc tcc agc tcc agc acc cct agc        2469
Leu Gln Arg Thr Ser Ser Gly Ser Ser Ser Ser Ser Ser Thr Pro Ser
        695                 700                 705

tcc cag ccc agc tcc caa gga ggc tcc cag cct gga tca caa gca gga        2517
Ser Gln Pro Ser Ser Gln Gly Gly Ser Gln Pro Gly Ser Gln Ala Gly
    710                 715                 720

tcc agt gaa cgc acc aga gtt cga gcc aac agt aag tca gaa gga tca        2565
Ser Ser Glu Arg Thr Arg Val Arg Ala Asn Ser Lys Ser Glu Gly Ser
725                 730                 735                 740

cct gtg ctc ccc cat gag cct gcc aag gtg aaa cca gaa gaa tcc agg        2613
Pro Val Leu Pro His Glu Pro Ala Lys Val Lys Pro Glu Glu Ser Arg
                745                 750                 755

gac att acc cgg ccc agt cga cca gct agc tac aaa aaa gct ata gat        2661
Asp Ile Thr Arg Pro Ser Arg Pro Ala Ser Tyr Lys Lys Ala Ile Asp
            760                 765                 770

gag gat ctg acg gca tta gcc aaa gaa cta aga gaa ctc cgg att gaa        2709

Glu Asp Leu Thr Ala Leu Ala Lys Glu Leu Arg Glu Leu Arg Ile Glu
        775                 780                 785

gaa aca aac cgc cca atg aag aag gtg act gat tac tcc tcc tcc agt      2757
Glu Thr Asn Arg Pro Met Lys Lys Val Thr Asp Tyr Ser Ser Ser Ser
    790                 795                 800

gag gag tca gaa agt agc gag gaa gag gag gaa gat gga gag agc gag      2805
Glu Glu Ser Glu Ser Ser Glu Glu Glu Glu Glu Asp Gly Glu Ser Glu
805                 810                 815                 820

acc cat gat ggg aca gtg gct gtc agc gac ata ccc aga ctg ata cca      2853
Thr His Asp Gly Thr Val Ala Val Ser Asp Ile Pro Arg Leu Ile Pro
                825                 830                 835

aca gga gct cca ggc agc aac gag cag tac aat gtg gga atg gtg ggg      2901
Thr Gly Ala Pro Gly Ser Asn Glu Gln Tyr Asn Val Gly Met Val Gly
            840                 845                 850

acg cat ggg ctg gag acc tct cat gcg gac agt ttc agc ggc agt att      2949
Thr His Gly Leu Glu Thr Ser His Ala Asp Ser Phe Ser Gly Ser Ile
        855                 860                 865

tca aga gaa gga acc ttg atg att aga gag acg tct gga gag aag aag      2997
Ser Arg Glu Gly Thr Leu Met Ile Arg Glu Thr Ser Gly Glu Lys Lys
    870                 875                 880

cga tct ggc cac agt gac agc aat ggc ttt gct ggc cac atc aac ctc      3045
Arg Ser Gly His Ser Asp Ser Asn Gly Phe Ala Gly His Ile Asn Leu
885                 890                 895                 900

cct gac ctg gtg cag cag agc cat tct cca gct gga acc ccg act gag      3093
Pro Asp Leu Val Gln Gln Ser His Ser Pro Ala Gly Thr Pro Thr Glu
                905                 910                 915

gga ctg ggg cgc gtc tca acc cat tcc cag gag atg gac tct ggg act      3141
Gly Leu Gly Arg Val Ser Thr His Ser Gln Glu Met Asp Ser Gly Thr
            920                 925                 930

gaa tat ggc atg ggg agc agc acc aaa gcc tcc ttc acc ccc ttt gtg      3189
Glu Tyr Gly Met Gly Ser Ser Thr Lys Ala Ser Phe Thr Pro Phe Val
        935                 940                 945

gac ccc aga gta tac cag acg tct ccc act gat gaa gat gaa gag gat      3237
Asp Pro Arg Val Tyr Gln Thr Ser Pro Thr Asp Glu Asp Glu Glu Asp
    950                 955                 960

gag gaa tca tca gcc gca gct ctg ttt act agc gaa ctt ctt agg caa      3285
Glu Glu Ser Ser Ala Ala Ala Leu Phe Thr Ser Glu Leu Leu Arg Gln
965                 970                 975                 980

gaa cag gcc aaa ctc aat gaa gca aga aag att tcg gtg gta aat gta      3333
Glu Gln Ala Lys Leu Asn Glu Ala Arg Lys Ile Ser Val Val Asn Val
                985                 990                 995

aac cca acc aac  att cgg cct cat agc  gac aca cca gaa atc  aga      3378
Asn Pro Thr Asn  Ile Arg Pro His Ser  Asp Thr Pro Glu Ile  Arg
            1000                 1005                 1010

aaa tac aag aaa  cga ttc aac tca gaa  ata ctt tgt gca gct  ctg      3423
Lys Tyr Lys Lys  Arg Phe Asn Ser Glu  Ile Leu Cys Ala Ala  Leu
            1015                 1020                 1025

tgg ggt gta aac ctt ctg gtg ggg act gaa aat ggc ctg atg ctt 3468
Trp Gly Val Asn Leu Leu Val Gly Thr Glu Asn Gly Leu Met Leu
            1030                1035                1040

ttg gac cga agt ggg caa ggc aaa gtc tat aat ctg atc aac cgg 3513
Leu Asp Arg Ser Gly Gln Gly Lys Val Tyr Asn Leu Ile Asn Arg
            1045                1050                1055

agg cga ttt cag cag atg gat gtg cta gag gga ctg aat gtc ctt 3558
Arg Arg Phe Gln Gln Met Asp Val Leu Glu Gly Leu Asn Val Leu
            1060                1065                1070

gtg aca att tca gga aag aag aat aag cta cga gtt tac tat ctt 3603
Val Thr Ile Ser Gly Lys Lys Asn Lys Leu Arg Val Tyr Tyr Leu
            1075                1080                1085

tca tgg tta aga aac aga ata cta cat aat gac cca gaa gta gaa 3648
Ser Trp Leu Arg Asn Arg Ile Leu His Asn Asp Pro Glu Val Glu
            1090                1095                1100

aag aaa caa ggc tgg atc act gtt ggg gac ttg gaa ggc tgt ata 3693
Lys Lys Gln Gly Trp Ile Thr Val Gly Asp Leu Glu Gly Cys Ile
            1105                1110                1115

cat tat aaa gtt gtt aaa tat gaa agg atc aaa ttt ttg gtg att 3738
His Tyr Lys Val Val Lys Tyr Glu Arg Ile Lys Phe Leu Val Ile
            1120                1125                1130

gcc tta aag aat gct gtg gaa ata tat gct tgg gct cct aaa ccg 3783
Ala Leu Lys Asn Ala Val Glu Ile Tyr Ala Trp Ala Pro Lys Pro
            1135                1140                1145

tat cat aaa ttc atg gca ttt aag tct ttt gca gat ctc cag cac 3828
Tyr His Lys Phe Met Ala Phe Lys Ser Phe Ala Asp Leu Gln His
            1150                1155                1160

aag cct ctg cta gtt gat ctc acg gta gaa gaa ggt caa aga tta 3873
Lys Pro Leu Leu Val Asp Leu Thr Val Glu Glu Gly Gln Arg Leu
            1165                1170                1175

aag gtt att ttt ggt tca cac act ggt ttc cat gta att gat gtt 3918
Lys Val Ile Phe Gly Ser His Thr Gly Phe His Val Ile Asp Val
            1180                1185                1190

gat tca gga aac tct tat gat atc tac ata cca tct cat att cag 3963
Asp Ser Gly Asn Ser Tyr Asp Ile Tyr Ile Pro Ser His Ile Gln
            1195                1200                1205

ggc aat atc act cct cat gct att gtc atc ttg cct aaa aca gat 4008
Gly Asn Ile Thr Pro His Ala Ile Val Ile Leu Pro Lys Thr Asp
            1210                1215                1220

gga atg gaa atg ctt gtt tgc tat gag gat gag ggg gtg tat gta 4053
Gly Met Glu Met Leu Val Cys Tyr Glu Asp Glu Gly Val Tyr Val
            1225                1230                1235

aac acc tat ggc cgg ata act aag gat gtg gtg ctc caa tgg gga 4098
Asn Thr Tyr Gly Arg Ile Thr Lys Asp Val Val Leu Gln Trp Gly
            1240                1245                1250

gaa atg ccc acg tct gtg gcc tac att cat tcc aat cag ata atg 4143
Glu Met Pro Thr Ser Val Ala Tyr Ile His Ser Asn Gln Ile Met
            1255                1260                1265

ggc tgg ggc gag aaa gct att gag atc cgg tca gtg gaa aca gga 4188
Gly Trp Gly Glu Lys Ala Ile Glu Ile Arg Ser Val Glu Thr Gly
            1270                1275                1280

cat ttg gat gga gta ttt atg cat aag cga gct caa agg tta aag 4233
His Leu Asp Gly Val Phe Met His Lys Arg Ala Gln Arg Leu Lys
            1285                1290                1295

ttt cta tgt gaa aga aat gat aag gta ttt ttt gca tcc gtg cga 4278
Phe Leu Cys Glu Arg Asn Asp Lys Val Phe Phe Ala Ser Val Arg
            1300                1305                1310

tct gga gga agt agc caa gtg ttt ttc atg acc ctc aac aga aat 4323
Ser Gly Gly Ser Ser Gln Val Phe Phe Met Thr Leu Asn Arg Asn
            1315                1320                1325

tcc atg atg aac tgg taa cagaagagca cttggcactt atcttcatgg 4371
Ser Met Met Asn Trp
            1330

cgttatttct aatttaaaag aacataactc atgtggactt atgccagtct agaggcagaa 4431

tcagaaggct tggttgaaca tatcgctttc cctttttcct ctccctccgc ccctcccagt 4491

acagtccatc tttcaatgtt gcagcctggt tgagaaggag agaaaaaggt ggcaggaatt 4551

tccaggagat ccccaagaat gctgccttgt ctgtggacaa agatggacca tgtgcccttc 4611

ggaattaggg atagaaacaa atattgtgtg ctcttaacga ttaagctgtg ttatggtggg 4671

ttttcaggtt tttacctttt ttctttaccc ctttactctg caagaatggg gaaagaatgc 4731

atactgcgaa aatgagtctt ttaaattctg tctgcctact agttttaagt atatggtatg 4791

ttgtaaaatt tccaatgatg agagacagca caataaatgt accttatctc cttaggctga 4851

aggccataac tacatagtgg agtaatttaa gaactctctt gccttcacca acccaaaagg 4911

ttgctttttg atagcaactg gctaatgaat ttttaaaaag agaagaaaaa tactagtttt 4971

cccctctttt gggaaataga ttttaaatgg ctaaactact agccttaaaa ctactagtct 5031

aataaaatca actaccactt ttgtgaatct gacaggccac atttttatat ggccctttac 5091

agaatggagt gtgttgaaca ggatactaac gccattgagt tgagctggcc tagcgatgga 5151

gggacactct aacacaactt tccctcagct attatgcaac agatcaggga aaaagatggg 5211

atgacagatg gggtcagaca gaaagagctt ctgggaaaca agcttacata gtctttttta 5271

aaatgcacaa agcctcccag ctaagaggtc acttggtttg ggcttcatta ggactgagac 5331

tttgttgagt tctttctggg acttggagag tggatgatat tcaggctctg aacattccca 5391

gcgctctccc gagggtgcca ctttctcaag atgaaaactg tgactgaaaa aattaataat 5451

aaatgtttct gagctgcctg tgttctccct gtgtgggtga gagaagggac tagactccta 5511

agcctgcctc agatacaaga ggcatcattg gctccaattt tagagaactt gaaagcaagg 5571

ctttggacaa aattttgaga ccctaatcac tttaccttcc tccaaattac ccaacatacg 5631

gtaaacaaca tttgtgcaga agtatgtatg tatttagttc aggttgactt gtgtccttat     5691

aaactcttac tcaaatgatt tgaactttaa aaaaaaa                              5729

<210>  38
<211>  1331
<212>  PRT
<213>  Homo sapiens

<400>  38

Met Ala Ser Asp Ser Pro Ala Arg Ser Leu Asp Glu Ile Asp Leu Ser
1               5                   10                  15

Ala Leu Arg Asp Pro Ala Gly Ile Phe Glu Leu Val Glu Leu Val Gly
            20                  25                  30

Asn Gly Thr Tyr Gly Gln Val Tyr Lys Gly Arg His Val Lys Thr Gly
        35                  40                  45

Gln Leu Ala Ala Ile Lys Val Met Asp Val Thr Gly Asp Glu Glu Glu
    50                  55                  60

Glu Ile Lys Gln Glu Ile Asn Met Leu Lys Lys Tyr Ser His His Arg
65                  70                  75                  80

Asn Ile Ala Thr Tyr Tyr Gly Ala Phe Ile Lys Lys Asn Pro Pro Gly
                85                  90                  95

Met Asp Asp Gln Leu Trp Leu Val Met Glu Phe Cys Gly Ala Gly Ser
            100                 105                 110

Val Thr Asp Leu Ile Lys Asn Thr Lys Gly Asn Thr Leu Lys Glu Glu
        115                 120                 125

Trp Ile Ala Tyr Ile Cys Arg Glu Ile Leu Arg Gly Leu Ser His Leu
    130                 135                 140

His Gln His Lys Val Ile His Arg Asp Ile Lys Gly Gln Asn Val Leu
145                 150                 155                 160

Leu Thr Glu Asn Ala Glu Val Lys Leu Val Asp Phe Gly Val Ser Ala
                165                 170                 175

Gln Leu Asp Arg Thr Val Gly Arg Arg Asn Thr Phe Ile Gly Thr Pro
            180                 185                 190

Tyr Trp Met Ala Pro Glu Val Ile Ala Cys Asp Glu Asn Pro Asp Ala
        195                 200                 205

Thr Tyr Asp Phe Lys Ser Asp Leu Trp Ser Leu Gly Ile Thr Ala Ile
    210                 215                 220

Glu Met Ala Glu Gly Ala Pro Pro Leu Cys Asp Met His Pro Met Arg
225                 230                 235                 240

Ala Leu Phe Leu Ile Pro Arg Asn Pro Ala Pro Arg Leu Lys Ser Lys
                245                 250                 255

Lys Trp Ser Lys Lys Phe Gln Ser Phe Ile Glu Ser Cys Leu Val Lys
            260                 265                 270

Asn His Ser Gln Arg Pro Ala Thr Glu Gln Leu Met Lys His Pro Phe
        275                 280                 285

Ile Arg Asp Gln Pro Asn Glu Arg Gln Val Arg Ile Gln Leu Lys Asp
    290                 295                 300

His Ile Asp Arg Thr Lys Lys Lys Arg Gly Glu Lys Asp Glu Thr Glu
305                 310                 315                 320

Tyr Glu Tyr Ser Gly Ser Glu Glu Glu Glu Glu Glu Asn Asp Ser Gly
                325                 330                 335

Glu Pro Ser Ser Ile Leu Asn Leu Pro Gly Glu Ser Thr Leu Arg Arg
            340                 345                 350

Asp Phe Leu Arg Leu Gln Leu Ala Asn Lys Glu Arg Ser Glu Ala Leu
        355                 360                 365

Arg Arg Gln Gln Leu Glu Gln Gln Gln Arg Glu Asn Glu Glu His Lys
    370                 375                 380

Arg Gln Leu Leu Ala Glu Arg Gln Lys Arg Ile Glu Glu Gln Lys Glu
385                 390                 395                 400

Gln Arg Arg Arg Leu Glu Glu Gln Gln Arg Arg Glu Lys Glu Leu Arg
                405                 410                 415

Lys Gln Gln Glu Arg Glu Gln Arg Arg His Tyr Glu Glu Gln Met Arg
            420                 425                 430

Arg Glu Glu Glu Arg Arg Arg Ala Glu His Glu Gln Glu Tyr Lys Arg
        435                 440                 445

Lys Gln Leu Glu Glu Gln Arg Gln Ala Glu Arg Leu Gln Arg Gln Leu
    450                 455                 460

Lys Gln Glu Arg Asp Tyr Leu Val Ser Leu Gln His Gln Arg Gln Glu
465                 470                 475                 480

Gln Arg Pro Val Glu Lys Lys Pro Leu Tyr His Tyr Lys Glu Gly Met
                485                 490                 495

Ser Pro Ser Glu Lys Pro Ala Trp Ala Lys Glu Val Glu Glu Arg Ser
            500                 505                 510

Arg Leu Asn Arg Gln Ser Ser Pro Ala Met Pro His Lys Val Ala Asn
        515                 520                 525

Arg Ile Ser Asp Pro Asn Leu Pro Pro Arg Ser Glu Ser Phe Ser Ile
    530                 535                 540

Ser Gly Val Gln Pro Ala Arg Thr Pro Pro Met Leu Arg Pro Val Asp
545                 550                 555                 560

Pro Gln Ile Pro His Leu Val Ala Val Lys Ser Gln Gly Pro Ala Leu
                565                 570                 575

Thr Ala Ser Gln Ser Val His Glu Gln Pro Thr Lys Gly Leu Ser Gly
            580                 585                 590

Phe Gln Glu Ala Leu Asn Val Thr Ser His Arg Val Glu Met Pro Arg
        595                 600                 605

Gln Asn Ser Asp Pro Thr Ser Glu Asn Pro Pro Leu Pro Thr Arg Ile
    610                 615                 620

Glu Lys Phe Asp Arg Ser Ser Trp Leu Arg Gln Glu Glu Asp Ile Pro
625                 630                 635                 640

Pro Lys Val Pro Gln Arg Thr Thr Ser Ile Ser Pro Ala Leu Ala Arg
                645                 650                 655

Lys Asn Ser Pro Gly Asn Gly Ser Ala Leu Gly Pro Arg Leu Gly Ser
            660                 665                 670

Gln Pro Ile Arg Ala Ser Asn Pro Asp Leu Arg Arg Thr Glu Pro Ile
        675                 680                 685

Leu Glu Ser Pro Leu Gln Arg Thr Ser Ser Gly Ser Ser Ser Ser Ser
    690                 695                 700

Ser Thr Pro Ser Ser Gln Pro Ser Ser Gln Gly Gly Ser Gln Pro Gly

705                 710                 715                 720

Ser Gln Ala Gly Ser Ser Glu Arg Thr Arg Val Arg Ala Asn Ser Lys
                725                 730                 735

Ser Glu Gly Ser Pro Val Leu Pro His Glu Pro Ala Lys Val Lys Pro
            740                 745                 750

Glu Glu Ser Arg Asp Ile Thr Arg Pro Ser Arg Pro Ala Ser Tyr Lys
        755                 760                 765

Lys Ala Ile Asp Glu Asp Leu Thr Ala Leu Ala Lys Glu Leu Arg Glu
    770                 775                 780

Leu Arg Ile Glu Glu Thr Asn Arg Pro Met Lys Lys Val Thr Asp Tyr
785                 790                 795                 800

Ser Ser Ser Ser Glu Glu Ser Glu Ser Ser Glu Glu Glu Glu Glu Asp
                805                 810                 815

Gly Glu Ser Glu Thr His Asp Gly Thr Val Ala Val Ser Asp Ile Pro
            820                 825                 830

Arg Leu Ile Pro Thr Gly Ala Pro Gly Ser Asn Glu Gln Tyr Asn Val
        835                 840                 845

Gly Met Val Gly Thr His Gly Leu Glu Thr Ser His Ala Asp Ser Phe
    850                 855                 860

Ser Gly Ser Ile Ser Arg Glu Gly Thr Leu Met Ile Arg Glu Thr Ser
865                 870                 875                 880

Gly Glu Lys Lys Arg Ser Gly His Ser Asp Ser Asn Gly Phe Ala Gly
                885                 890                 895

His Ile Asn Leu Pro Asp Leu Val Gln Gln Ser His Ser Pro Ala Gly
            900                 905                 910

Thr Pro Thr Glu Gly Leu Gly Arg Val Ser Thr His Ser Gln Glu Met
        915                 920                 925

Asp Ser Gly Thr Glu Tyr Gly Met Gly Ser Ser Thr Lys Ala Ser Phe
    930                 935                 940

Thr Pro Phe Val Asp Pro Arg Val Tyr Gln Thr Ser Pro Thr Asp Glu
945                 950                 955                 960

Asp Glu Glu Asp Glu Glu Ser Ser Ala Ala Ala Leu Phe Thr Ser Glu
                965                 970                 975

Leu Leu Arg Gln Glu Gln Ala Lys Leu Asn Glu Ala Arg Lys Ile Ser
            980                 985                 990

Val Val Asn Val Asn Pro Thr Asn  Ile Arg Pro His Ser  Asp Thr Pro
        995                 1000                 1005

Glu Ile  Arg Lys Tyr Lys Lys  Arg Phe Asn Ser Glu  Ile Leu Cys
    1010                 1015                 1020

Ala Ala  Leu Trp Gly Val Asn  Leu Leu Val Gly Thr  Glu Asn Gly
    1025                 1030                 1035

Leu Met  Leu Leu Asp Arg Ser  Gly Gln Gly Lys Val  Tyr Asn Leu
    1040                 1045                 1050

Ile Asn  Arg Arg Arg Phe Gln  Gln Met Asp Val Leu  Glu Gly Leu
    1055                 1060                 1065

Asn Val  Leu Val Thr Ile Ser  Gly Lys Lys Asn Lys  Leu Arg Val
    1070                 1075                 1080

Tyr Tyr  Leu Ser Trp Leu Arg  Asn Arg Ile Leu His  Asn Asp Pro
    1085                 1090                 1095

Glu Val  Glu Lys Lys Gln Gly  Trp Ile Thr Val Gly  Asp Leu Glu
    1100                 1105                 1110

Gly Cys  Ile His Tyr Lys Val  Val Lys Tyr Glu Arg  Ile Lys Phe
    1115                 1120                 1125

Leu Val  Ile Ala Leu Lys Asn  Ala Val Glu Ile Tyr  Ala Trp Ala
    1130                 1135                 1140

Pro Lys  Pro Tyr His Lys Phe  Met Ala Phe Lys Ser  Phe Ala Asp
    1145                 1150                 1155

Leu Gln  His Lys Pro Leu Leu  Val Asp Leu Thr Val  Glu Glu Gly
    1160                 1165                 1170

Gln Arg  Leu Lys Val Ile Phe  Gly Ser His Thr Gly  Phe His Val
    1175                 1180                 1185

Ile Asp  Val Asp Ser Gly Asn  Ser Tyr Asp Ile Tyr  Ile Pro Ser
    1190                 1195                 1200

His Ile Gln Gly Asn Ile Thr Pro His Ala Ile Val Ile Leu Pro
    1205                1210                1215

Lys Thr Asp Gly Met Glu Met Leu Val Cys Tyr Glu Asp Glu Gly
    1220                1225                1230

Val Tyr Val Asn Thr Tyr Gly Arg Ile Thr Lys Asp Val Val Leu
    1235                1240                1245

Gln Trp Gly Glu Met Pro Thr Ser Val Ala Tyr Ile His Ser Asn
    1250                1255                1260

Gln Ile Met Gly Trp Gly Glu Lys Ala Ile Glu Ile Arg Ser Val
    1265                1270                1275

Glu Thr Gly His Leu Asp Gly Val Phe Met His Lys Arg Ala Gln
    1280                1285                1290

Arg Leu Lys Phe Leu Cys Glu Arg Asn Asp Lys Val Phe Phe Ala
    1295                1300                1305

Ser Val Arg Ser Gly Gly Ser Ser Gln Val Phe Phe Met Thr Leu
    1310                1315                1320

Asn Arg Asn Ser Met Met Asn Trp
    1325                1330

<210> 39
<211> 5705
<212> DNA
<213> Homo sapiens

<220>
<221> CDS
<222> (346)..(4317)

<400> 39
attataacag ccaatgcagc cgccatccac gcacaagcaa agaagcatgt cccatttaaa 60

tacacccacg cagccccagc gcccttagcc gatcggggcg ctcagcccac acgcaccgct 120

gctcggggct tggagatccg cgcaggctgg gctcccggac ccggcggacc gacgcgcgga 180

ggatcgggat ccggcgctgt ggggctgggg tgggcggggg aggctgggcc cggggcctct 240

ggcgcgacac ccgcatgagg acgcgagtga aatagaccaa ggtggaattt ccaagggaaa 300

agcttcgggg tggttttggt ccatttctcc agcgaagaag tagac atg gcg agc gac 357
Met Ala Ser Asp
1

tcc ccg gct cga agc ctg gat gaa ata gat ctc tcg gct ctg agg gac 405

Ser Pro Ala Arg Ser Leu Asp Glu Ile Asp Leu Ser Ala Leu Arg Asp
5                   10                  15                  20

ccc gca ggg atc ttt gaa ttg gtg gaa ctt gtt gga aat gga aca tac     453
Pro Ala Gly Ile Phe Glu Leu Val Glu Leu Val Gly Asn Gly Thr Tyr
                25                  30                  35

ggg caa gtt tat aag ggt cgt cat gtc aaa acg ggc cag ctt gca gcc     501
Gly Gln Val Tyr Lys Gly Arg His Val Lys Thr Gly Gln Leu Ala Ala
            40                  45                  50

atc aag gtt atg gat gtc aca ggg gat gaa gag gaa gaa atc aaa caa     549
Ile Lys Val Met Asp Val Thr Gly Asp Glu Glu Glu Glu Ile Lys Gln
        55                  60                  65

gaa att aac atg ttg aag aaa tat tct cat cac cgg aat att gct aca     597
Glu Ile Asn Met Leu Lys Lys Tyr Ser His His Arg Asn Ile Ala Thr
    70                  75                  80

tac tat ggt gct ttt atc aaa aag aac cca cca ggc atg gat gac caa     645
Tyr Tyr Gly Ala Phe Ile Lys Lys Asn Pro Pro Gly Met Asp Asp Gln
85                  90                  95                  100

ctt tgg ttg gtg atg gag ttt tgt ggt gct ggc tct gtc acc gac ctg     693
Leu Trp Leu Val Met Glu Phe Cys Gly Ala Gly Ser Val Thr Asp Leu
                105                 110                 115

atc aag aac aca aaa ggt aac acg ttg aaa gag gag tgg att gca tac     741
Ile Lys Asn Thr Lys Gly Asn Thr Leu Lys Glu Glu Trp Ile Ala Tyr
            120                 125                 130

atc tgc agg gaa atc tta cgg ggg ctg agt cac ctg cac cag cat aaa     789
Ile Cys Arg Glu Ile Leu Arg Gly Leu Ser His Leu His Gln His Lys
        135                 140                 145

gtg att cat cga gat att aaa ggg caa aat gtc ttg ctg act gaa aat     837
Val Ile His Arg Asp Ile Lys Gly Gln Asn Val Leu Leu Thr Glu Asn
    150                 155                 160

gca gaa gtt aaa cta gtg gac ttt gga gtc agt gct cag ctt gat cga     885
Ala Glu Val Lys Leu Val Asp Phe Gly Val Ser Ala Gln Leu Asp Arg
165                 170                 175                 180

aca gtg ggc agg agg aat act ttc att gga act ccc tac tgg atg gca     933
Thr Val Gly Arg Arg Asn Thr Phe Ile Gly Thr Pro Tyr Trp Met Ala
                185                 190                 195

cca gaa gtt att gcc tgt gat gaa aac cca gat gcc aca tat gat ttc     981
Pro Glu Val Ile Ala Cys Asp Glu Asn Pro Asp Ala Thr Tyr Asp Phe
            200                 205                 210

aag agt gac ttg tgg tct ttg ggt atc acc gcc att gaa atg gca gaa     1029
Lys Ser Asp Leu Trp Ser Leu Gly Ile Thr Ala Ile Glu Met Ala Glu
        215                 220                 225

ggt gct ccc cct ctc tgt gac atg cac ccc atg aga gct ctc ttc ctc     1077
Gly Ala Pro Pro Leu Cys Asp Met His Pro Met Arg Ala Leu Phe Leu
    230                 235                 240

atc ccc cgg aac cca gcg cct cgg ctg aag tct aag aag tgg tca aaa     1125
Ile Pro Arg Asn Pro Ala Pro Arg Leu Lys Ser Lys Lys Trp Ser Lys
245                 250                 255                 260

aaa ttc cag tca ttt att gag agc tgc ttg gta aag aat cac agc cag 1173
Lys Phe Gln Ser Phe Ile Glu Ser Cys Leu Val Lys Asn His Ser Gln
                265                 270                 275

cga cca gca aca gaa caa ttg atg aag cat cca ttt ata cga gac caa 1221
Arg Pro Ala Thr Glu Gln Leu Met Lys His Pro Phe Ile Arg Asp Gln
            280                 285                 290

cct aat gag cga cag gtc cgc att caa ctc aag gac cat att gat aga 1269
Pro Asn Glu Arg Gln Val Arg Ile Gln Leu Lys Asp His Ile Asp Arg
        295                 300                 305

aca aag aag aag cga gga gaa aaa gat gag aca gag tat gag tac agt 1317
Thr Lys Lys Lys Arg Gly Glu Lys Asp Glu Thr Glu Tyr Glu Tyr Ser
    310                 315                 320

gga agt gag gaa gaa gag gag gag aat gac tca gga gag ccc agc tcc 1365
Gly Ser Glu Glu Glu Glu Glu Glu Asn Asp Ser Gly Glu Pro Ser Ser
325                 330                 335                 340

atc ctg aat ctg cca ggg gag tcg acg ctg cgg agg gac ttt ctg agg 1413
Ile Leu Asn Leu Pro Gly Glu Ser Thr Leu Arg Arg Asp Phe Leu Arg
                345                 350                 355

ctg cag ctg gcc aac aag gag cgt tct gag gcc cta cgg agg cag cag 1461
Leu Gln Leu Ala Asn Lys Glu Arg Ser Glu Ala Leu Arg Arg Gln Gln
            360                 365                 370

ctg gag cag cag cag cgg gag aat gag gag cac aag cgg cag ctg ctg 1509
Leu Glu Gln Gln Gln Arg Glu Asn Glu Glu His Lys Arg Gln Leu Leu
        375                 380                 385

gcc gag cgt cag aag cgc atc gag gag cag aaa gag cag agg cgg cgg 1557
Ala Glu Arg Gln Lys Arg Ile Glu Glu Gln Lys Glu Gln Arg Arg Arg
    390                 395                 400

ctg gag gag caa caa agg cga gag aag gag ctg cgg aag cag cag gag 1605
Leu Glu Glu Gln Gln Arg Arg Glu Lys Glu Leu Arg Lys Gln Gln Glu
405                 410                 415                 420

agg gag cag cgc cgg cac tat gag gag cag atg cgc cgg gag gag gag 1653
Arg Glu Gln Arg Arg His Tyr Glu Glu Gln Met Arg Arg Glu Glu Glu
                425                 430                 435

agg agg cgt gcg gag cat gaa cag gaa tat aag cgc aaa caa ttg gaa 1701
Arg Arg Arg Ala Glu His Glu Gln Glu Tyr Lys Arg Lys Gln Leu Glu
            440                 445                 450

gaa cag aga caa gca gaa aga ctg cag agg cag cta aag caa gaa aga 1749
Glu Gln Arg Gln Ala Glu Arg Leu Gln Arg Gln Leu Lys Gln Glu Arg
        455                 460                 465

gac tac tta gtt tcc ctt cag cat cag cgg cag gag cag agg cct gtg 1797
Asp Tyr Leu Val Ser Leu Gln His Gln Arg Gln Glu Gln Arg Pro Val
    470                 475                 480

gag aag aag cca ctg tac cat tac aaa gaa gga atg agt cct agt gag 1845
Glu Lys Lys Pro Leu Tyr His Tyr Lys Glu Gly Met Ser Pro Ser Glu
485                 490                 495                 500

aag cca gca tgg gcc aag gag gta gaa gaa cgg tca agg ctc aac cgg 1893
Lys Pro Ala Trp Ala Lys Glu Val Glu Glu Arg Ser Arg Leu Asn Arg
                505                 510                 515

caa agt tcc cct gcc atg cct cac aag gtt gcc aac agg ata tct gac 1941
Gln Ser Ser Pro Ala Met Pro His Lys Val Ala Asn Arg Ile Ser Asp
            520                 525                 530

ccc aac ctg ccc cca agg tcg gag tcc ttc agc att agt gga gtt cag 1989
Pro Asn Leu Pro Pro Arg Ser Glu Ser Phe Ser Ile Ser Gly Val Gln
        535                 540                 545

cct gct cga aca ccc ccc atg ctc aga cca gtc gat ccc cag atc cca 2037
Pro Ala Arg Thr Pro Pro Met Leu Arg Pro Val Asp Pro Gln Ile Pro
    550                 555                 560

cat ctg gta gct gta aaa tcc cag gga cct gcc ttg acc gcc tcc cag 2085
His Leu Val Ala Val Lys Ser Gln Gly Pro Ala Leu Thr Ala Ser Gln
565                 570                 575                 580

tca gtg cac gag cag ccc aca aag ggc ctc tct ggg ttt cag gag gct 2133
Ser Val His Glu Gln Pro Thr Lys Gly Leu Ser Gly Phe Gln Glu Ala
                585                 590                 595

ctg aac gtg acc tcc cac cgc gtg gag atg cca cgc cag aac tca gat 2181
Leu Asn Val Thr Ser His Arg Val Glu Met Pro Arg Gln Asn Ser Asp
            600                 605                 610

ccc acc tcg gaa aat cct cct ctc ccc act cgc att gaa aag ttt gac 2229
Pro Thr Ser Glu Asn Pro Pro Leu Pro Thr Arg Ile Glu Lys Phe Asp
        615                 620                 625

cga agc tct tgg tta cga cag gaa gaa gac att cca cca aag gtg cct 2277
Arg Ser Ser Trp Leu Arg Gln Glu Glu Asp Ile Pro Pro Lys Val Pro
    630                 635                 640

caa aga aca act tct ata tcc cca gca tta gcc aga aag aat tct cct 2325
Gln Arg Thr Thr Ser Ile Ser Pro Ala Leu Ala Arg Lys Asn Ser Pro
645                 650                 655                 660

ggg aat ggt agt gct ctg gga ccc aga cta gga tct caa ccc atc aga 2373
Gly Asn Gly Ser Ala Leu Gly Pro Arg Leu Gly Ser Gln Pro Ile Arg
                665                 670                 675

gca agc aac cct gat ctc cgg aga act gag ccc atc ttg gag agc ccc 2421
Ala Ser Asn Pro Asp Leu Arg Arg Thr Glu Pro Ile Leu Glu Ser Pro
            680                 685                 690

ttg cag agg acc agc agt ggc agt tcc tcc agc tcc agc acc cct agc 2469
Leu Gln Arg Thr Ser Ser Gly Ser Ser Ser Ser Ser Ser Thr Pro Ser
        695                 700                 705

tcc cag ccc agc tcc caa gga ggc tcc cag cct gga tca caa gca gga 2517
Ser Gln Pro Ser Ser Gln Gly Gly Ser Gln Pro Gly Ser Gln Ala Gly
    710                 715                 720

tcc agt gaa cgc acc aga gtt cga gcc aac agt aag tca gaa gga tca 2565
Ser Ser Glu Arg Thr Arg Val Arg Ala Asn Ser Lys Ser Glu Gly Ser
725                 730                 735                 740

cct gtg ctc ccc cat gag cct gcc aag gtg aaa cca gaa gaa tcc agg 2613
Pro Val Leu Pro His Glu Pro Ala Lys Val Lys Pro Glu Glu Ser Arg
                745                 750                 755

gac att acc cgg ccc agt cga cca gct gat ctg acg gca tta gcc aaa 2661
Asp Ile Thr Arg Pro Ser Arg Pro Ala Asp Leu Thr Ala Leu Ala Lys

760                 765                 770

gaa cta aga gaa ctc cgg att gaa gaa aca aac cgc cca atg aag aag     2709
Glu Leu Arg Glu Leu Arg Ile Glu Glu Thr Asn Arg Pro Met Lys Lys
        775                 780                 785

gtg act gat tac tcc tcc tcc agt gag gag tca gaa agt agc gag gaa     2757
Val Thr Asp Tyr Ser Ser Ser Ser Glu Glu Ser Glu Ser Ser Glu Glu
    790                 795                 800

gag gag gaa gat gga gag agc gag acc cat gat ggg aca gtg gct gtc     2805
Glu Glu Glu Asp Gly Glu Ser Glu Thr His Asp Gly Thr Val Ala Val
805                 810                 815                 820

agc gac ata ccc aga ctg ata cca aca gga gct cca ggc agc aac gag     2853
Ser Asp Ile Pro Arg Leu Ile Pro Thr Gly Ala Pro Gly Ser Asn Glu
                825                 830                 835

cag tac aat gtg gga atg gtg ggg acg cat ggg ctg gag acc tct cat     2901
Gln Tyr Asn Val Gly Met Val Gly Thr His Gly Leu Glu Thr Ser His
            840                 845                 850

gcg gac agt ttc agc ggc agt att tca aga gaa gga acc ttg atg att     2949
Ala Asp Ser Phe Ser Gly Ser Ile Ser Arg Glu Gly Thr Leu Met Ile
        855                 860                 865

aga gag acg tct gga gag aag aag cga tct ggc cac agt gac agc aat     2997
Arg Glu Thr Ser Gly Glu Lys Lys Arg Ser Gly His Ser Asp Ser Asn
    870                 875                 880

ggc ttt gct ggc cac atc aac ctc cct gac ctg gtg cag cag agc cat     3045
Gly Phe Ala Gly His Ile Asn Leu Pro Asp Leu Val Gln Gln Ser His
885                 890                 895                 900

tct cca gct gga acc ccg act gag gga ctg ggg cgc gtc tca acc cat     3093
Ser Pro Ala Gly Thr Pro Thr Glu Gly Leu Gly Arg Val Ser Thr His
                905                 910                 915

tcc cag gag atg gac tct ggg act gaa tat ggc atg ggg agc agc acc     3141
Ser Gln Glu Met Asp Ser Gly Thr Glu Tyr Gly Met Gly Ser Ser Thr
            920                 925                 930

aaa gcc tcc ttc acc ccc ttt gtg gac ccc aga gta tac cag acg tct     3189
Lys Ala Ser Phe Thr Pro Phe Val Asp Pro Arg Val Tyr Gln Thr Ser
        935                 940                 945

ccc act gat gaa gat gaa gag gat gag gaa tca tca gcc gca gct ctg     3237
Pro Thr Asp Glu Asp Glu Glu Asp Glu Glu Ser Ser Ala Ala Ala Leu
    950                 955                 960

ttt act agc gaa ctt ctt agg caa gaa cag gcc aaa ctc aat gaa gca     3285
Phe Thr Ser Glu Leu Leu Arg Gln Glu Gln Ala Lys Leu Asn Glu Ala
965                 970                 975                 980

aga aag att tcg gtg gta aat gta aac cca acc aac att cgg cct cat     3333
Arg Lys Ile Ser Val Val Asn Val Asn Pro Thr Asn Ile Arg Pro His
                985                 990                 995

agc gac aca cca  gaa atc aga aaa tac  aag aaa cga ttc aac  tca     3378
Ser Asp Thr Pro  Glu Ile Arg Lys Tyr  Lys Lys Arg Phe Asn  Ser
            1000                 1005                 1010

gaa ata ctt tgt  gca gct ctg tgg ggt  gta aac ctt ctg gtg  ggg     3423

Glu Ile Leu Cys Ala Ala Leu Trp Gly Val Asn Leu Leu Val Gly
            1015                1020                1025

act gaa aat ggc ctg atg ctt ttg gac cga agt ggg caa ggc aaa 3468
Thr Glu Asn Gly Leu Met Leu Leu Asp Arg Ser Gly Gln Gly Lys
            1030                1035                1040

gtc tat aat ctg atc aac cgg agg cga ttt cag cag atg gat gtg 3513
Val Tyr Asn Leu Ile Asn Arg Arg Arg Phe Gln Gln Met Asp Val
            1045                1050                1055

cta gag gga ctg aat gtc ctt gtg aca att tca gga aag aag aat 3558
Leu Glu Gly Leu Asn Val Leu Val Thr Ile Ser Gly Lys Lys Asn
            1060                1065                1070

aag cta cga gtt tac tat ctt tca tgg tta aga aac aga ata cta 3603
Lys Leu Arg Val Tyr Tyr Leu Ser Trp Leu Arg Asn Arg Ile Leu
            1075                1080                1085

cat aat gac cca gaa gta gaa aag aaa caa ggc tgg atc act gtt 3648
His Asn Asp Pro Glu Val Glu Lys Lys Gln Gly Trp Ile Thr Val
            1090                1095                1100

ggg gac ttg gaa ggc tgt ata cat tat aaa gtt gtt aaa tat gaa 3693
Gly Asp Leu Glu Gly Cys Ile His Tyr Lys Val Val Lys Tyr Glu
            1105                1110                1115

agg atc aaa ttt ttg gtg att gcc tta aag aat gct gtg gaa ata 3738
Arg Ile Lys Phe Leu Val Ile Ala Leu Lys Asn Ala Val Glu Ile
            1120                1125                1130

tat gct tgg gct cct aaa ccg tat cat aaa ttc atg gca ttt aag 3783
Tyr Ala Trp Ala Pro Lys Pro Tyr His Lys Phe Met Ala Phe Lys
            1135                1140                1145

tct ttt gca gat ctc cag cac aag cct ctg cta gtt gat ctc acg 3828
Ser Phe Ala Asp Leu Gln His Lys Pro Leu Leu Val Asp Leu Thr
            1150                1155                1160

gta gaa gaa ggt caa aga tta aag gtt att ttt ggt tca cac act 3873
Val Glu Glu Gly Gln Arg Leu Lys Val Ile Phe Gly Ser His Thr
            1165                1170                1175

ggt ttc cat gta att gat gtt gat tca gga aac tct tat gat atc 3918
Gly Phe His Val Ile Asp Val Asp Ser Gly Asn Ser Tyr Asp Ile
            1180                1185                1190

tac ata cca tct cat att cag ggc aat atc act cct cat gct att 3963
Tyr Ile Pro Ser His Ile Gln Gly Asn Ile Thr Pro His Ala Ile
            1195                1200                1205

gtc atc ttg cct aaa aca gat gga atg gaa atg ctt gtt tgc tat 4008
Val Ile Leu Pro Lys Thr Asp Gly Met Glu Met Leu Val Cys Tyr
            1210                1215                1220

gag gat gag ggg gtg tat gta aac acc tat ggc cgg ata act aag 4053
Glu Asp Glu Gly Val Tyr Val Asn Thr Tyr Gly Arg Ile Thr Lys
            1225                1230                1235

gat gtg gtg ctc caa tgg gga gaa atg ccc acg tct gtg gcc tac 4098
Asp Val Val Leu Gln Trp Gly Glu Met Pro Thr Ser Val Ala Tyr
            1240                1245                1250

att cat tcc aat cag ata atg ggc tgg ggc gag aaa gct att gag 4143
Ile His Ser Asn Gln Ile Met Gly Trp Gly Glu Lys Ala Ile Glu
            1255                1260                1265

atc cgg tca gtg gaa aca gga cat ttg gat gga gta ttt atg cat 4188
Ile Arg Ser Val Glu Thr Gly His Leu Asp Gly Val Phe Met His
            1270                1275                1280

aag cga gct caa agg tta aag ttt cta tgt gaa aga aat gat aag 4233
Lys Arg Ala Gln Arg Leu Lys Phe Leu Cys Glu Arg Asn Asp Lys
            1285                1290                1295

gta ttt ttt gca tcc gtg cga tct gga gga agt agc caa gtg ttt 4278
Val Phe Phe Ala Ser Val Arg Ser Gly Gly Ser Ser Gln Val Phe
            1300                1305                1310

ttc atg acc ctc aac aga aat tcc atg atg aac tgg taa cagaagagca 4327
Phe Met Thr Leu Asn Arg Asn Ser Met Met Asn Trp
            1315                1320

cttggcactt atcttcatgg cgttatttct aatttaaaag aacataactc atgtggactt 4387

atgccagtct agaggcagaa tcagaaggct tggttgaaca tatcgctttc cctttttcct 4447

ctccctccgc ccctcccagt acagtccatc tttcaatgtt gcagcctggt tgagaaggag 4507

agaaaaaggt ggcaggaatt tccaggagat ccccaagaat gctgccttgt ctgtggacaa 4567

agatggacca tgtgcccttc ggaattaggg atagaaacaa atattgtgtg ctcttaacga 4627

ttaagctgtg ttatggtggg ttttcaggtt tttacctttt ttctttaccc ctttactctg 4687

caagaatggg gaaagaatgc atactgcgaa aatgagtctt ttaaattctg tctgcctact 4747

agttttaagt atatggtatg ttgtaaaatt tccaatgatg agagacagca caataaatgt 4807

accttatctc cttaggctga aggccataac tacatagtgg agtaatttaa gaactctctt 4867

gccttcacca acccaaaagg ttgctttttg atagcaactg gctaatgaat ttttaaaaag 4927

agaagaaaaa tactagtttt cccctctttt gggaaataga ttttaaatgg ctaaactact 4987

agccttaaaa ctactagtct aataaaatca actaccactt ttgtgaatct gacaggccac 5047

atttttatat ggccctttac agaatggagt gtgttgaaca ggatactaac gccattgagt 5107

tgagctggcc tagcgatgga gggacactct aacacaactt tccctcagct attatgcaac 5167

agatcaggga aaaagatggg atgacagatg gggtcagaca gaaagagctt ctgggaaaca 5227

agcttacata gtctttttta aaatgcacaa agcctcccag ctaagaggtc acttggtttg 5287

ggcttcatta ggactgagac tttgttgagt tctttctggg acttggagag tggatgatat 5347

tcaggctctg aacattccca gcgctctccc gagggtgcca ctttctcaag atgaaaactg 5407

tgactgaaaa aattaataat aaatgtttct gagctgcctg tgttctccct gtgtgggtga 5467

gagaagggac tagactccta agcctgcctc agatacaaga ggcatcattg gctccaattt 5527

tagagaactt gaaagcaagg ctttggacaa aattttgaga ccctaatcac tttaccttcc 5587

tccaaattac ccaacatacg gtaaacaaca tttgtgcaga agtatgtatg tatttagttc 5647

aggttgactt gtgtccttat aaactcttac tcaaatgatt tgaactttaa aaaaaaa      5705

<210>  40
<211>  1323
<212>  PRT
<213>  Homo sapiens

<400>  40

Met Ala Ser Asp Ser Pro Ala Arg Ser Leu Asp Glu Ile Asp Leu Ser
1               5                   10                  15

Ala Leu Arg Asp Pro Ala Gly Ile Phe Glu Leu Val Glu Leu Val Gly
            20                  25                  30

Asn Gly Thr Tyr Gly Gln Val Tyr Lys Gly Arg His Val Lys Thr Gly
        35                  40                  45

Gln Leu Ala Ala Ile Lys Val Met Asp Val Thr Gly Asp Glu Glu Glu
    50                  55                  60

Glu Ile Lys Gln Glu Ile Asn Met Leu Lys Lys Tyr Ser His His Arg
65                  70                  75                  80

Asn Ile Ala Thr Tyr Tyr Gly Ala Phe Ile Lys Lys Asn Pro Pro Gly
                85                  90                  95

Met Asp Asp Gln Leu Trp Leu Val Met Glu Phe Cys Gly Ala Gly Ser
            100                 105                 110

Val Thr Asp Leu Ile Lys Asn Thr Lys Gly Asn Thr Leu Lys Glu Glu
        115                 120                 125

Trp Ile Ala Tyr Ile Cys Arg Glu Ile Leu Arg Gly Leu Ser His Leu
    130                 135                 140

His Gln His Lys Val Ile His Arg Asp Ile Lys Gly Gln Asn Val Leu
145                 150                 155                 160

Leu Thr Glu Asn Ala Glu Val Lys Leu Val Asp Phe Gly Val Ser Ala
                165                 170                 175

Gln Leu Asp Arg Thr Val Gly Arg Arg Asn Thr Phe Ile Gly Thr Pro
            180                 185                 190

Tyr Trp Met Ala Pro Glu Val Ile Ala Cys Asp Glu Asn Pro Asp Ala
        195                 200                 205

Thr Tyr Asp Phe Lys Ser Asp Leu Trp Ser Leu Gly Ile Thr Ala Ile
    210                 215                 220

Glu Met Ala Glu Gly Ala Pro Pro Leu Cys Asp Met His Pro Met Arg
225                 230                 235                 240

Ala Leu Phe Leu Ile Pro Arg Asn Pro Ala Pro Arg Leu Lys Ser Lys
                245                 250                 255

Lys Trp Ser Lys Lys Phe Gln Ser Phe Ile Glu Ser Cys Leu Val Lys
            260                 265                 270

Asn His Ser Gln Arg Pro Ala Thr Glu Gln Leu Met Lys His Pro Phe
        275                 280                 285

Ile Arg Asp Gln Pro Asn Glu Arg Gln Val Arg Ile Gln Leu Lys Asp
    290                 295                 300

His Ile Asp Arg Thr Lys Lys Lys Arg Gly Glu Lys Asp Glu Thr Glu
305                 310                 315                 320

Tyr Glu Tyr Ser Gly Ser Glu Glu Glu Glu Glu Glu Asn Asp Ser Gly
                325                 330                 335

Glu Pro Ser Ser Ile Leu Asn Leu Pro Gly Glu Ser Thr Leu Arg Arg
            340                 345                 350

Asp Phe Leu Arg Leu Gln Leu Ala Asn Lys Glu Arg Ser Glu Ala Leu
        355                 360                 365

Arg Arg Gln Gln Leu Glu Gln Gln Gln Arg Glu Asn Glu Glu His Lys
    370                 375                 380

Arg Gln Leu Leu Ala Glu Arg Gln Lys Arg Ile Glu Glu Gln Lys Glu
385                 390                 395                 400

Gln Arg Arg Arg Leu Glu Glu Gln Gln Arg Arg Glu Lys Glu Leu Arg
                405                 410                 415

Lys Gln Gln Glu Arg Glu Gln Arg Arg His Tyr Glu Glu Gln Met Arg
            420                 425                 430

Arg Glu Glu Glu Arg Arg Arg Ala Glu His Glu Gln Glu Tyr Lys Arg
        435                 440                 445

Lys Gln Leu Glu Glu Gln Arg Gln Ala Glu Arg Leu Gln Arg Gln Leu
    450                 455                 460

Lys Gln Glu Arg Asp Tyr Leu Val Ser Leu Gln His Gln Arg Gln Glu
465                 470                 475                 480

Gln Arg Pro Val Glu Lys Lys Pro Leu Tyr His Tyr Lys Glu Gly Met
                485                 490                 495

Ser Pro Ser Glu Lys Pro Ala Trp Ala Lys Glu Val Glu Glu Arg Ser
            500                 505                 510

Arg Leu Asn Arg Gln Ser Ser Pro Ala Met Pro His Lys Val Ala Asn
        515                 520                 525

Arg Ile Ser Asp Pro Asn Leu Pro Pro Arg Ser Glu Ser Phe Ser Ile
    530                 535                 540

Ser Gly Val Gln Pro Ala Arg Thr Pro Pro Met Leu Arg Pro Val Asp
545                 550                 555                 560

Pro Gln Ile Pro His Leu Val Ala Val Lys Ser Gln Gly Pro Ala Leu
                565                 570                 575

Thr Ala Ser Gln Ser Val His Glu Gln Pro Thr Lys Gly Leu Ser Gly
            580                 585                 590

Phe Gln Glu Ala Leu Asn Val Thr Ser His Arg Val Glu Met Pro Arg
        595                 600                 605

Gln Asn Ser Asp Pro Thr Ser Glu Asn Pro Pro Leu Pro Thr Arg Ile
    610                 615                 620

Glu Lys Phe Asp Arg Ser Ser Trp Leu Arg Gln Glu Glu Asp Ile Pro
625                 630                 635                 640

Pro Lys Val Pro Gln Arg Thr Thr Ser Ile Ser Pro Ala Leu Ala Arg
                645                 650                 655

Lys Asn Ser Pro Gly Asn Gly Ser Ala Leu Gly Pro Arg Leu Gly Ser
            660                 665                 670

Gln Pro Ile Arg Ala Ser Asn Pro Asp Leu Arg Arg Thr Glu Pro Ile
        675                 680                 685

Leu Glu Ser Pro Leu Gln Arg Thr Ser Ser Gly Ser Ser Ser Ser Ser
    690                 695                 700

Ser Thr Pro Ser Ser Gln Pro Ser Ser Gln Gly Gly Ser Gln Pro Gly
705                 710                 715                 720

Ser Gln Ala Gly Ser Ser Glu Arg Thr Arg Val Arg Ala Asn Ser Lys
                725                 730                 735

Ser Glu Gly Ser Pro Val Leu Pro His Glu Pro Ala Lys Val Lys Pro
            740                 745                 750

Glu Glu Ser Arg Asp Ile Thr Arg Pro Ser Arg Pro Ala Asp Leu Thr
        755                 760                 765

Ala Leu Ala Lys Glu Leu Arg Glu Leu Arg Ile Glu Glu Thr Asn Arg
    770                 775                 780

Pro Met Lys Lys Val Thr Asp Tyr Ser Ser Ser Ser Glu Glu Ser Glu
785                 790                 795                 800

Ser Ser Glu Glu Glu Glu Glu Asp Gly Glu Ser Glu Thr His Asp Gly
                805                 810                 815

Thr Val Ala Val Ser Asp Ile Pro Arg Leu Ile Pro Thr Gly Ala Pro
            820                 825                 830

Gly Ser Asn Glu Gln Tyr Asn Val Gly Met Val Gly Thr His Gly Leu
        835                 840                 845

Glu Thr Ser His Ala Asp Ser Phe Ser Gly Ser Ile Ser Arg Glu Gly
    850                 855                 860

Thr Leu Met Ile Arg Glu Thr Ser Gly Glu Lys Lys Arg Ser Gly His
865                 870                 875                 880

Ser Asp Ser Asn Gly Phe Ala Gly His Ile Asn Leu Pro Asp Leu Val
                885                 890                 895

Gln Gln Ser His Ser Pro Ala Gly Thr Pro Thr Glu Gly Leu Gly Arg
            900                 905                 910

Val Ser Thr His Ser Gln Glu Met Asp Ser Gly Thr Glu Tyr Gly Met
        915                 920                 925

Gly Ser Ser Thr Lys Ala Ser Phe Thr Pro Phe Val Asp Pro Arg Val
    930                 935                 940

Tyr Gln Thr Ser Pro Thr Asp Glu Asp Glu Glu Asp Glu Glu Ser Ser
945                 950                 955                 960

Ala Ala Ala Leu Phe Thr Ser Glu Leu Leu Arg Gln Glu Gln Ala Lys

965                 970                 975

Leu Asn Glu Ala Arg Lys Ile Ser Val Val Asn Val Asn Pro Thr Asn
            980                 985                 990

Ile Arg Pro His Ser Asp Thr Pro Glu Ile Arg Lys Tyr Lys Lys Arg
        995                 1000                1005

Phe Asn Ser Glu Ile Leu Cys Ala Ala Leu Trp Gly Val Asn Leu
    1010                1015                1020

Leu Val Gly Thr Glu Asn Gly Leu Met Leu Leu Asp Arg Ser Gly
    1025                1030                1035

Gln Gly Lys Val Tyr Asn Leu Ile Asn Arg Arg Arg Phe Gln Gln
    1040                1045                1050

Met Asp Val Leu Glu Gly Leu Asn Val Leu Val Thr Ile Ser Gly
    1055                1060                1065

Lys Lys Asn Lys Leu Arg Val Tyr Tyr Leu Ser Trp Leu Arg Asn
    1070                1075                1080

Arg Ile Leu His Asn Asp Pro Glu Val Glu Lys Lys Gln Gly Trp
    1085                1090                1095

Ile Thr Val Gly Asp Leu Glu Gly Cys Ile His Tyr Lys Val Val
    1100                1105                1110

Lys Tyr Glu Arg Ile Lys Phe Leu Val Ile Ala Leu Lys Asn Ala
    1115                1120                1125

Val Glu Ile Tyr Ala Trp Ala Pro Lys Pro Tyr His Lys Phe Met
    1130                1135                1140

Ala Phe Lys Ser Phe Ala Asp Leu Gln His Lys Pro Leu Leu Val
    1145                1150                1155

Asp Leu Thr Val Glu Glu Gly Gln Arg Leu Lys Val Ile Phe Gly
    1160                1165                1170

Ser His Thr Gly Phe His Val Ile Asp Val Asp Ser Gly Asn Ser
    1175                1180                1185

Tyr Asp Ile Tyr Ile Pro Ser His Ile Gln Gly Asn Ile Thr Pro
    1190                1195                1200

His Ala Ile Val Ile Leu Pro Lys Thr Asp Gly Met Glu Met Leu
    1205                1210                1215

Val Cys Tyr Glu Asp Glu Gly Val Tyr Val Asn Thr Tyr Gly Arg
    1220                1225                1230

Ile Thr Lys Asp Val Val Leu Gln Trp Gly Glu Met Pro Thr Ser
    1235                1240                1245

Val Ala Tyr Ile His Ser Asn Gln Ile Met Gly Trp Gly Glu Lys
    1250                1255                1260

Ala Ile Glu Ile Arg Ser Val Glu Thr Gly His Leu Asp Gly Val
    1265                1270                1275

Phe Met His Lys Arg Ala Gln Arg Leu Lys Phe Leu Cys Glu Arg
    1280                1285                1290

Asn Asp Lys Val Phe Phe Ala Ser Val Arg Ser Gly Gly Ser Ser
    1295                1300                1305

Gln Val Phe Phe Met Thr Leu Asn Arg Asn Ser Met Met Asn Trp
    1310                1315                1320

<210>  41
<211>  5651
<212>  DNA
<213>  Homo sapiens

<220>
<221>  CDS
<222>  (346)..(4263)

<400>  41
attataacag ccaatgcagc cgccatccac gcacaagcaa agaagcatgt cccatttaaa      60

tacacccacg cagccccagc gcccttagcc gatcggggcg ctcagcccac acgcaccgct     120

gctcggggct tggagatccg cgcaggctgg gctcccggac ccggcggacc gacgcgcgga     180

ggatcgggat ccggcgctgt ggggctgggg tgggcggggg aggctgggcc cggggcctct     240

ggcgcgacac ccgcatgagg acgcgagtga aatagaccaa ggtggaattt ccaagggaaa      300

agcttcgggg tggttttggt ccatttctcc agcgaagaag tagac atg gcg agc gac     357
                                                  Met Ala Ser Asp
                                                  1

tcc ccg gct cga agc ctg gat gaa ata gat ctc tcg gct ctg agg gac       405
Ser Pro Ala Arg Ser Leu Asp Glu Ile Asp Leu Ser Ala Leu Arg Asp
5                   10                  15                  20

ccc gca ggg atc ttt gaa ttg gtg gaa ctt gtt gga aat gga aca tac       453
Pro Ala Gly Ile Phe Glu Leu Val Glu Leu Val Gly Asn Gly Thr Tyr

                25                  30                  35

ggg caa gtt tat aag ggt cgt cat gtc aaa acg ggc cag ctt gca gcc 501
Gly Gln Val Tyr Lys Gly Arg His Val Lys Thr Gly Gln Leu Ala Ala
            40                  45                  50

atc aag gtt atg gat gtc aca ggg gat gaa gag gaa gaa atc aaa caa 549
Ile Lys Val Met Asp Val Thr Gly Asp Glu Glu Glu Glu Ile Lys Gln
        55                  60                  65

gaa att aac atg ttg aag aaa tat tct cat cac cgg aat att gct aca 597
Glu Ile Asn Met Leu Lys Lys Tyr Ser His His Arg Asn Ile Ala Thr
    70                  75                  80

tac tat ggt gct ttt atc aaa aag aac cca cca ggc atg gat gac caa 645
Tyr Tyr Gly Ala Phe Ile Lys Lys Asn Pro Pro Gly Met Asp Asp Gln
85                  90                  95                  100

ctt tgg ttg gtg atg gag ttt tgt ggt gct ggc tct gtc acc gac ctg 693
Leu Trp Leu Val Met Glu Phe Cys Gly Ala Gly Ser Val Thr Asp Leu
                105                 110                 115

atc aag aac aca aaa ggt aac acg ttg aaa gag gag tgg att gca tac 741
Ile Lys Asn Thr Lys Gly Asn Thr Leu Lys Glu Glu Trp Ile Ala Tyr
            120                 125                 130

atc tgc agg gaa atc tta cgg ggg ctg agt cac ctg cac cag cat aaa 789
Ile Cys Arg Glu Ile Leu Arg Gly Leu Ser His Leu His Gln His Lys
        135                 140                 145

gtg att cat cga gat att aaa ggg caa aat gtc ttg ctg act gaa aat 837
Val Ile His Arg Asp Ile Lys Gly Gln Asn Val Leu Leu Thr Glu Asn
    150                 155                 160

gca gaa gtt aaa cta gtg gac ttt gga gtc agt gct cag ctt gat cga 885
Ala Glu Val Lys Leu Val Asp Phe Gly Val Ser Ala Gln Leu Asp Arg
165                 170                 175                 180

aca gtg ggc agg agg aat act ttc att gga act ccc tac tgg atg gca 933
Thr Val Gly Arg Arg Asn Thr Phe Ile Gly Thr Pro Tyr Trp Met Ala
                185                 190                 195

cca gaa gtt att gcc tgt gat gaa aac cca gat gcc aca tat gat ttc 981
Pro Glu Val Ile Ala Cys Asp Glu Asn Pro Asp Ala Thr Tyr Asp Phe
            200                 205                 210

aag agt gac ttg tgg tct ttg ggt atc acc gcc att gaa atg gca gaa 1029
Lys Ser Asp Leu Trp Ser Leu Gly Ile Thr Ala Ile Glu Met Ala Glu
        215                 220                 225

ggt gct ccc cct ctc tgt gac atg cac ccc atg aga gct ctc ttc ctc 1077
Gly Ala Pro Pro Leu Cys Asp Met His Pro Met Arg Ala Leu Phe Leu
    230                 235                 240

atc ccc cgg aac cca gcg cct cgg ctg aag tct aag aag tgg tca aaa 1125
Ile Pro Arg Asn Pro Ala Pro Arg Leu Lys Ser Lys Lys Trp Ser Lys
245                 250                 255                 260

aaa ttc cag tca ttt att gag agc tgc ttg gta aag aat cac agc cag 1173
Lys Phe Gln Ser Phe Ile Glu Ser Cys Leu Val Lys Asn His Ser Gln
                265                 270                 275

cga cca gca aca gaa caa ttg atg aag cat cca ttt ata cga gac caa 1221

Arg Pro Ala Thr Glu Gln Leu Met Lys His Pro Phe Ile Arg Asp Gln
            280                 285                 290

cct aat gag cga cag gtc cgc att caa ctc aag gac cat att gat aga 1269
Pro Asn Glu Arg Gln Val Arg Ile Gln Leu Lys Asp His Ile Asp Arg
        295                 300                 305

aca aag aag aag cga gga gaa aaa gat gag aca gag tat gag tac agt 1317
Thr Lys Lys Lys Arg Gly Glu Lys Asp Glu Thr Glu Tyr Glu Tyr Ser
    310                 315                 320

gga agt gag gaa gaa gag gag gag aat gac tca gga gag ccc agc tcc 1365
Gly Ser Glu Glu Glu Glu Glu Glu Asn Asp Ser Gly Glu Pro Ser Ser
325                 330                 335                 340

atc ctg aat ctg cca ggg gag tcg acg ctg cgg agg gac ttt ctg agg 1413
Ile Leu Asn Leu Pro Gly Glu Ser Thr Leu Arg Arg Asp Phe Leu Arg
                345                 350                 355

ctg cag ctg gcc aac aag gag cgt tct gag gcc cta cgg agg cag cag 1461
Leu Gln Leu Ala Asn Lys Glu Arg Ser Glu Ala Leu Arg Arg Gln Gln
            360                 365                 370

ctg gag cag cag cag cgg gag aat gag gag cac aag cgg cag ctg ctg 1509
Leu Glu Gln Gln Gln Arg Glu Asn Glu Glu His Lys Arg Gln Leu Leu
        375                 380                 385

gcc gag cgt cag aag cgc atc gag gag cag aaa gag cag agg cgg cgg 1557
Ala Glu Arg Gln Lys Arg Ile Glu Glu Gln Lys Glu Gln Arg Arg Arg
    390                 395                 400

ctg gag gag caa caa agg cga gag aag gag ctg cgg aag cag cag gag 1605
Leu Glu Glu Gln Gln Arg Arg Glu Lys Glu Leu Arg Lys Gln Gln Glu
405                 410                 415                 420

agg gag cag cgc cgg cac tat gag gag cag atg cgc cgg gag gag gag 1653
Arg Glu Gln Arg Arg His Tyr Glu Glu Gln Met Arg Arg Glu Glu Glu
                425                 430                 435

agg agg cgt gcg gag cat gaa cag gaa tac atc agg cga cag tta gag 1701
Arg Arg Arg Ala Glu His Glu Gln Glu Tyr Ile Arg Arg Gln Leu Glu
            440                 445                 450

gag gag cag aga cag tta gag atc ttg cag cag cag cta ctg cat gaa 1749
Glu Glu Gln Arg Gln Leu Glu Ile Leu Gln Gln Gln Leu Leu His Glu
        455                 460                 465

caa gct cta ctt ctg gaa tat aag cgc aaa caa ttg gaa gaa cag aga 1797
Gln Ala Leu Leu Leu Glu Tyr Lys Arg Lys Gln Leu Glu Glu Gln Arg
    470                 475                 480

caa gca gaa aga ctg cag agg cag cta aag caa gaa aga gac tac tta 1845
Gln Ala Glu Arg Leu Gln Arg Gln Leu Lys Gln Glu Arg Asp Tyr Leu
485                 490                 495                 500

gtt tcc ctt cag cat cag cgg cag gag cag agg cct gtg gag aag aag 1893
Val Ser Leu Gln His Gln Arg Gln Glu Gln Arg Pro Val Glu Lys Lys
                505                 510                 515

cca ctg tac cat tac aaa gaa gga atg agt cct agt gag aag cca gca 1941
Pro Leu Tyr His Tyr Lys Glu Gly Met Ser Pro Ser Glu Lys Pro Ala
            520                 525                 530

tgg gcc aag gag atc cca cat ctg gta gct gta aaa tcc cag gga cct     1989
Trp Ala Lys Glu Ile Pro His Leu Val Ala Val Lys Ser Gln Gly Pro
        535                 540                 545

gcc ttg acc gcc tcc cag tca gtg cac gag cag ccc aca aag ggc ctc     2037
Ala Leu Thr Ala Ser Gln Ser Val His Glu Gln Pro Thr Lys Gly Leu
    550                 555                 560

tct ggg ttt cag gag gct ctg aac gtg acc tcc cac cgc gtg gag atg     2085
Ser Gly Phe Gln Glu Ala Leu Asn Val Thr Ser His Arg Val Glu Met
565                 570                 575                 580

cca cgc cag aac tca gat ccc acc tcg gaa aat cct cct ctc ccc act     2133
Pro Arg Gln Asn Ser Asp Pro Thr Ser Glu Asn Pro Pro Leu Pro Thr
                585                 590                 595

cgc att gaa aag ttt gac cga agc tct tgg tta cga cag gaa gaa gac     2181
Arg Ile Glu Lys Phe Asp Arg Ser Ser Trp Leu Arg Gln Glu Glu Asp
            600                 605                 610

att cca cca aag gtg cct caa aga aca act tct ata tcc cca gca tta     2229
Ile Pro Pro Lys Val Pro Gln Arg Thr Thr Ser Ile Ser Pro Ala Leu
        615                 620                 625

gcc aga aag aat tct cct ggg aat ggt agt gct ctg gga ccc aga cta     2277
Ala Arg Lys Asn Ser Pro Gly Asn Gly Ser Ala Leu Gly Pro Arg Leu
    630                 635                 640

gga tct caa ccc atc aga gca agc aac cct gat ctc cgg aga act gag     2325
Gly Ser Gln Pro Ile Arg Ala Ser Asn Pro Asp Leu Arg Arg Thr Glu
645                 650                 655                 660

ccc atc ttg gag agc ccc ttg cag agg acc agc agt ggc agt tcc tcc     2373
Pro Ile Leu Glu Ser Pro Leu Gln Arg Thr Ser Ser Gly Ser Ser Ser
                665                 670                 675

agc tcc agc acc cct agc tcc cag ccc agc tcc caa gga ggc tcc cag     2421
Ser Ser Ser Thr Pro Ser Ser Gln Pro Ser Ser Gln Gly Gly Ser Gln
            680                 685                 690

cct gga tca caa gca gga tcc agt gaa cgc acc aga gtt cga gcc aac     2469
Pro Gly Ser Gln Ala Gly Ser Ser Glu Arg Thr Arg Val Arg Ala Asn
        695                 700                 705

agt aag tca gaa gga tca cct gtg ctc ccc cat gag cct gcc aag gtg     2517
Ser Lys Ser Glu Gly Ser Pro Val Leu Pro His Glu Pro Ala Lys Val
    710                 715                 720

aaa cca gaa gaa tcc agg gac att acc cgg ccc agt cga cca gct agc     2565
Lys Pro Glu Glu Ser Arg Asp Ile Thr Arg Pro Ser Arg Pro Ala Ser
725                 730                 735                 740

tac aaa aaa gct ata gat gag gat ctg acg gca tta gcc aaa gaa cta     2613
Tyr Lys Lys Ala Ile Asp Glu Asp Leu Thr Ala Leu Ala Lys Glu Leu
                745                 750                 755

aga gaa ctc cgg att gaa gaa aca aac cgc cca atg aag aag gtg act     2661
Arg Glu Leu Arg Ile Glu Glu Thr Asn Arg Pro Met Lys Lys Val Thr
            760                 765                 770

gat tac tcc tcc tcc agt gag gag tca gaa agt agc gag gaa gag gag     2709
Asp Tyr Ser Ser Ser Ser Glu Glu Ser Glu Ser Ser Glu Glu Glu Glu
        775                 780                 785

gaa gat gga gag agc gag acc cat gat ggg aca gtg gct gtc agc gac 2757
Glu Asp Gly Glu Ser Glu Thr His Asp Gly Thr Val Ala Val Ser Asp
    790                 795                 800

ata ccc aga ctg ata cca aca gga gct cca ggc agc aac gag cag tac 2805
Ile Pro Arg Leu Ile Pro Thr Gly Ala Pro Gly Ser Asn Glu Gln Tyr
805                 810                 815                 820

aat gtg gga atg gtg ggg acg cat ggg ctg gag acc tct cat gcg gac 2853
Asn Val Gly Met Val Gly Thr His Gly Leu Glu Thr Ser His Ala Asp
                825                 830                 835

agt ttc agc ggc agt att tca aga gaa gga acc ttg atg att aga gag 2901
Ser Phe Ser Gly Ser Ile Ser Arg Glu Gly Thr Leu Met Ile Arg Glu
            840                 845                 850

acg tct gga gag aag aag cga tct ggc cac agt gac agc aat ggc ttt 2949
Thr Ser Gly Glu Lys Lys Arg Ser Gly His Ser Asp Ser Asn Gly Phe
        855                 860                 865

gct ggc cac atc aac ctc cct gac ctg gtg cag cag agc cat tct cca 2997
Ala Gly His Ile Asn Leu Pro Asp Leu Val Gln Gln Ser His Ser Pro
    870                 875                 880

gct gga acc ccg act gag gga ctg ggg cgc gtc tca acc cat tcc cag 3045
Ala Gly Thr Pro Thr Glu Gly Leu Gly Arg Val Ser Thr His Ser Gln
885                 890                 895                 900

gag atg gac tct ggg act gaa tat ggc atg ggg agc agc acc aaa gcc 3093
Glu Met Asp Ser Gly Thr Glu Tyr Gly Met Gly Ser Ser Thr Lys Ala
                905                 910                 915

tcc ttc acc ccc ttt gtg gac ccc aga gta tac cag acg tct ccc act 3141
Ser Phe Thr Pro Phe Val Asp Pro Arg Val Tyr Gln Thr Ser Pro Thr
            920                 925                 930

gat gaa gat gaa gag gat gag gaa tca tca gcc gca gct ctg ttt act 3189
Asp Glu Asp Glu Glu Asp Glu Glu Ser Ser Ala Ala Ala Leu Phe Thr
        935                 940                 945

agc gaa ctt ctt agg caa gaa cag gcc aaa ctc aat gaa gca aga aag 3237
Ser Glu Leu Leu Arg Gln Glu Gln Ala Lys Leu Asn Glu Ala Arg Lys
    950                 955                 960

att tcg gtg gta aat gta aac cca acc aac att cgg cct cat agc gac 3285
Ile Ser Val Val Asn Val Asn Pro Thr Asn Ile Arg Pro His Ser Asp
965                 970                 975                 980

aca cca gaa atc aga aaa tac aag aaa cga ttc aac tca gaa ata ctt 3333
Thr Pro Glu Ile Arg Lys Tyr Lys Lys Arg Phe Asn Ser Glu Ile Leu
                985                 990                 995

tgt gca gct ctg tgg ggt gta aac ctt ctg gtg ggg act gaa aat 3378
Cys Ala Ala Leu Trp Gly Val Asn Leu Leu Val Gly Thr Glu Asn
            1000                1005                1010

ggc ctg atg ctt ttg gac cga agt ggg caa ggc aaa gtc tat aat 3423
Gly Leu Met Leu Leu Asp Arg Ser Gly Gln Gly Lys Val Tyr Asn
            1015                1020                1025

ctg atc aac cgg agg cga ttt cag cag atg gat gtg cta gag gga 3468
Leu Ile Asn Arg Arg Arg Phe Gln Gln Met Asp Val Leu Glu Gly

1030                1035                1040

ctg aat gtc ctt gtg aca att tca gga aag aag aat aag cta cga        3513
Leu Asn Val Leu Val Thr Ile Ser Gly Lys Lys Asn Lys Leu Arg
        1045                1050                1055

gtt tac tat ctt tca tgg tta aga aac aga ata cta cat aat gac        3558
Val Tyr Tyr Leu Ser Trp Leu Arg Asn Arg Ile Leu His Asn Asp
        1060                1065                1070

cca gaa gta gaa aag aaa caa ggc tgg atc act gtt ggg gac ttg        3603
Pro Glu Val Glu Lys Lys Gln Gly Trp Ile Thr Val Gly Asp Leu
        1075                1080                1085

gaa ggc tgt ata cat tat aaa gtt gtt aaa tat gaa agg atc aaa        3648
Glu Gly Cys Ile His Tyr Lys Val Val Lys Tyr Glu Arg Ile Lys
        1090                1095                1100

ttt ttg gtg att gcc tta aag aat gct gtg gaa ata tat gct tgg        3693
Phe Leu Val Ile Ala Leu Lys Asn Ala Val Glu Ile Tyr Ala Trp
        1105                1110                1115

gct cct aaa ccg tat cat aaa ttc atg gca ttt aag tct ttt gca        3738
Ala Pro Lys Pro Tyr His Lys Phe Met Ala Phe Lys Ser Phe Ala
        1120                1125                1130

gat ctc cag cac aag cct ctg cta gtt gat ctc acg gta gaa gaa        3783
Asp Leu Gln His Lys Pro Leu Leu Val Asp Leu Thr Val Glu Glu
        1135                1140                1145

ggt caa aga tta aag gtt att ttt ggt tca cac act ggt ttc cat        3828
Gly Gln Arg Leu Lys Val Ile Phe Gly Ser His Thr Gly Phe His
        1150                1155                1160

gta att gat gtt gat tca gga aac tct tat gat atc tac ata cca        3873
Val Ile Asp Val Asp Ser Gly Asn Ser Tyr Asp Ile Tyr Ile Pro
        1165                1170                1175

tct cat att cag ggc aat atc act cct cat gct att gtc atc ttg        3918
Ser His Ile Gln Gly Asn Ile Thr Pro His Ala Ile Val Ile Leu
        1180                1185                1190

cct aaa aca gat gga atg gaa atg ctt gtt tgc tat gag gat gag        3963
Pro Lys Thr Asp Gly Met Glu Met Leu Val Cys Tyr Glu Asp Glu
        1195                1200                1205

ggg gtg tat gta aac acc tat ggc cgg ata act aag gat gtg gtg        4008
Gly Val Tyr Val Asn Thr Tyr Gly Arg Ile Thr Lys Asp Val Val
        1210                1215                1220

ctc caa tgg gga gaa atg ccc acg tct gtg gcc tac att cat tcc        4053
Leu Gln Trp Gly Glu Met Pro Thr Ser Val Ala Tyr Ile His Ser
        1225                1230                1235

aat cag ata atg ggc tgg ggc gag aaa gct att gag atc cgg tca        4098
Asn Gln Ile Met Gly Trp Gly Glu Lys Ala Ile Glu Ile Arg Ser
        1240                1245                1250

gtg gaa aca gga cat ttg gat gga gta ttt atg cat aag cga gct        4143
Val Glu Thr Gly His Leu Asp Gly Val Phe Met His Lys Arg Ala
        1255                1260                1265

caa agg tta aag ttt cta tgt gaa aga aat gat aag gta ttt ttt        4188

Gln Arg Leu Lys Phe Leu Cys Glu Arg Asn Asp Lys Val Phe Phe
            1270                1275                1280

gca tcc gtg cga tct gga gga agt agc caa gtg ttt ttc atg acc      4233
Ala Ser Val Arg Ser Gly Gly Ser Ser Gln Val Phe Phe Met Thr
            1285                1290                1295

ctc aac aga aat tcc atg atg aac tgg taa cagaagagca cttggcactt    4283
Leu Asn Arg Asn Ser Met Met Asn Trp
            1300                1305

atcttcatgg cgttatttct aatttaaaag aacataactc atgtggactt atgccagtct    4343

agaggcagaa tcagaaggct tggttgaaca tatcgctttc cctttttcct ctccctccgc    4403

ccctcccagt acagtccatc tttcaatgtt gcagcctggt tgagaaggag agaaaaaggt    4463

ggcaggaatt tccaggagat ccccaagaat gctgccttgt ctgtggacaa agatggacca    4523

tgtgcccttc ggaattaggg atagaaacaa atattgtgtg ctcttaacga ttaagctgtg    4583

ttatggtggg ttttcaggtt tttacctttt ttctttaccc ctttactctg caagaatggg    4643

gaaagaatgc atactgcgaa aatgagtctt ttaaattctg tctgcctact agttttaagt    4703

atatggtatg ttgtaaaatt tccaatgatg agagacagca caataaatgt accttatctc    4763

cttaggctga aggccataac tacatagtgg agtaatttaa gaactctctt gccttcacca    4823

acccaaaagg ttgctttttg atagcaactg gctaatgaat tttaaaaaag agaagaaaaa    4883

tactagtttt cccctctttt gggaaataga ttttaaatgg ctaaactact agccttaaaa    4943

ctactagtct aataaaatca actaccactt ttgtgaatct gacaggccac atttttatat    5003

ggccctttac agaatggagt gtgttgaaca ggatactaac gccattgagt tgagctggcc    5063

tagcgatgga gggacactct aacacaactt tccctcagct attatgcaac agatcaggga    5123

aaaagatggg atgacagatg gggtcagaca gaaagagctt ctgggaaaca agcttacata    5183

gtctttttta aaatgcacaa agcctcccag ctaagaggtc acttggtttg ggcttcatta    5243

ggactgagac tttgttgagt tctttctggg acttggagag tggatgatat tcaggctctg    5303

aacattccca gcgctctccc gagggtgcca ctttctcaag atgaaaactg tgactgaaaa    5363

aattaataat aaatgtttct gagctgcctg tgttctccct gtgtgggtga gagaagggac    5423

tagactccta agcctgcctc agatacaaga ggcatcattg gctccaattt tagagaactt    5483

gaaagcaagg ctttggacaa aattttgaga ccctaatcac tttaccttcc tccaaattac    5543

ccaacatacg gtaaacaaca tttgtgcaga agtatgtatg tatttagttc aggttgactt    5603

gtgtccttat aaactcttac tcaaatgatt tgaactttaa aaaaaaaa                5651

<210>  42
<211>  1305
<212>  PRT
<213>  Homo sapiens

<400> 42

Met Ala Ser Asp Ser Pro Ala Arg Ser Leu Asp Glu Ile Asp Leu Ser
1               5                   10                  15

Ala Leu Arg Asp Pro Ala Gly Ile Phe Glu Leu Val Glu Leu Val Gly
            20                  25                  30

Asn Gly Thr Tyr Gly Gln Val Tyr Lys Gly Arg His Val Lys Thr Gly
        35                  40                  45

Gln Leu Ala Ala Ile Lys Val Met Asp Val Thr Gly Asp Glu Glu Glu
    50                  55                  60

Glu Ile Lys Gln Glu Ile Asn Met Leu Lys Lys Tyr Ser His His Arg
65                  70                  75                  80

Asn Ile Ala Thr Tyr Tyr Gly Ala Phe Ile Lys Lys Asn Pro Pro Gly
                85                  90                  95

Met Asp Asp Gln Leu Trp Leu Val Met Glu Phe Cys Gly Ala Gly Ser
            100                 105                 110

Val Thr Asp Leu Ile Lys Asn Thr Lys Gly Asn Thr Leu Lys Glu Glu
        115                 120                 125

Trp Ile Ala Tyr Ile Cys Arg Glu Ile Leu Arg Gly Leu Ser His Leu
    130                 135                 140

His Gln His Lys Val Ile His Arg Asp Ile Lys Gly Gln Asn Val Leu
145                 150                 155                 160

Leu Thr Glu Asn Ala Glu Val Lys Leu Val Asp Phe Gly Val Ser Ala
                165                 170                 175

Gln Leu Asp Arg Thr Val Gly Arg Arg Asn Thr Phe Ile Gly Thr Pro
            180                 185                 190

Tyr Trp Met Ala Pro Glu Val Ile Ala Cys Asp Glu Asn Pro Asp Ala
        195                 200                 205

Thr Tyr Asp Phe Lys Ser Asp Leu Trp Ser Leu Gly Ile Thr Ala Ile
    210                 215                 220

Glu Met Ala Glu Gly Ala Pro Pro Leu Cys Asp Met His Pro Met Arg
225                 230                 235                 240

Ala Leu Phe Leu Ile Pro Arg Asn Pro Ala Pro Arg Leu Lys Ser Lys

245                 250                 255

Lys Trp Ser Lys Lys Phe Gln Ser Phe Ile Glu Ser Cys Leu Val Lys
            260                 265                 270

Asn His Ser Gln Arg Pro Ala Thr Glu Gln Leu Met Lys His Pro Phe
        275                 280                 285

Ile Arg Asp Gln Pro Asn Glu Arg Gln Val Arg Ile Gln Leu Lys Asp
    290                 295                 300

His Ile Asp Arg Thr Lys Lys Lys Arg Gly Glu Lys Asp Glu Thr Glu
305                 310                 315                 320

Tyr Glu Tyr Ser Gly Ser Glu Glu Glu Glu Glu Glu Asn Asp Ser Gly
                325                 330                 335

Glu Pro Ser Ser Ile Leu Asn Leu Pro Gly Glu Ser Thr Leu Arg Arg
            340                 345                 350

Asp Phe Leu Arg Leu Gln Leu Ala Asn Lys Glu Arg Ser Glu Ala Leu
        355                 360                 365

Arg Arg Gln Gln Leu Glu Gln Gln Gln Arg Glu Asn Glu Glu His Lys
    370                 375                 380

Arg Gln Leu Leu Ala Glu Arg Gln Lys Arg Ile Glu Glu Gln Lys Glu
385                 390                 395                 400

Gln Arg Arg Arg Leu Glu Glu Gln Gln Arg Arg Glu Lys Glu Leu Arg
                405                 410                 415

Lys Gln Gln Glu Arg Glu Gln Arg Arg His Tyr Glu Glu Gln Met Arg
            420                 425                 430

Arg Glu Glu Glu Arg Arg Arg Ala Glu His Glu Gln Glu Tyr Ile Arg
        435                 440                 445

Arg Gln Leu Glu Glu Glu Gln Arg Gln Leu Glu Ile Leu Gln Gln Gln
    450                 455                 460

Leu Leu His Glu Gln Ala Leu Leu Leu Glu Tyr Lys Arg Lys Gln Leu
465                 470                 475                 480

Glu Glu Gln Arg Gln Ala Glu Arg Leu Gln Arg Gln Leu Lys Gln Glu
                485                 490                 495

Arg Asp Tyr Leu Val Ser Leu Gln His Gln Arg Gln Glu Gln Arg Pro
        500                 505                 510

Val Glu Lys Lys Pro Leu Tyr His Tyr Lys Glu Gly Met Ser Pro Ser
    515                 520                 525

Glu Lys Pro Ala Trp Ala Lys Glu Ile Pro His Leu Val Ala Val Lys
    530                 535                 540

Ser Gln Gly Pro Ala Leu Thr Ala Ser Gln Ser Val His Glu Gln Pro
545                 550                 555                 560

Thr Lys Gly Leu Ser Gly Phe Gln Glu Ala Leu Asn Val Thr Ser His
                565                 570                 575

Arg Val Glu Met Pro Arg Gln Asn Ser Asp Pro Thr Ser Glu Asn Pro
        580                 585                 590

Pro Leu Pro Thr Arg Ile Glu Lys Phe Asp Arg Ser Ser Trp Leu Arg
    595                 600                 605

Gln Glu Glu Asp Ile Pro Pro Lys Val Pro Gln Arg Thr Thr Ser Ile
    610                 615                 620

Ser Pro Ala Leu Ala Arg Lys Asn Ser Pro Gly Asn Gly Ser Ala Leu
625                 630                 635                 640

Gly Pro Arg Leu Gly Ser Gln Pro Ile Arg Ala Ser Asn Pro Asp Leu
                645                 650                 655

Arg Arg Thr Glu Pro Ile Leu Glu Ser Pro Leu Gln Arg Thr Ser Ser
        660                 665                 670

Gly Ser Ser Ser Ser Ser Ser Thr Pro Ser Ser Gln Pro Ser Ser Gln
    675                 680                 685

Gly Gly Ser Gln Pro Gly Ser Gln Ala Gly Ser Ser Glu Arg Thr Arg
    690                 695                 700

Val Arg Ala Asn Ser Lys Ser Glu Gly Ser Pro Val Leu Pro His Glu
705                 710                 715                 720

Pro Ala Lys Val Lys Pro Glu Glu Ser Arg Asp Ile Thr Arg Pro Ser
                725                 730                 735

Arg Pro Ala Ser Tyr Lys Lys Ala Ile Asp Glu Asp Leu Thr Ala Leu
        740                 745                 750

Ala Lys Glu Leu Arg Glu Leu Arg Ile Glu Glu Thr Asn Arg Pro Met
        755                 760                 765

Lys Lys Val Thr Asp Tyr Ser Ser Ser Ser Glu Glu Ser Glu Ser Ser
    770                 775                 780

Glu Glu Glu Glu Glu Asp Gly Glu Ser Glu Thr His Asp Gly Thr Val
785                 790                 795                 800

Ala Val Ser Asp Ile Pro Arg Leu Ile Pro Thr Gly Ala Pro Gly Ser
                805                 810                 815

Asn Glu Gln Tyr Asn Val Gly Met Val Gly Thr His Gly Leu Glu Thr
            820                 825                 830

Ser His Ala Asp Ser Phe Ser Gly Ser Ile Ser Arg Glu Gly Thr Leu
        835                 840                 845

Met Ile Arg Glu Thr Ser Gly Glu Lys Lys Arg Ser Gly His Ser Asp
    850                 855                 860

Ser Asn Gly Phe Ala Gly His Ile Asn Leu Pro Asp Leu Val Gln Gln
865                 870                 875                 880

Ser His Ser Pro Ala Gly Thr Pro Thr Glu Gly Leu Gly Arg Val Ser
                885                 890                 895

Thr His Ser Gln Glu Met Asp Ser Gly Thr Glu Tyr Gly Met Gly Ser
            900                 905                 910

Ser Thr Lys Ala Ser Phe Thr Pro Phe Val Asp Pro Arg Val Tyr Gln
        915                 920                 925

Thr Ser Pro Thr Asp Glu Asp Glu Glu Asp Glu Glu Ser Ser Ala Ala
    930                 935                 940

Ala Leu Phe Thr Ser Glu Leu Leu Arg Gln Glu Gln Ala Lys Leu Asn
945                 950                 955                 960

Glu Ala Arg Lys Ile Ser Val Val Asn Val Asn Pro Thr Asn Ile Arg
                965                 970                 975

Pro His Ser Asp Thr Pro Glu Ile Arg Lys Tyr Lys Lys Arg Phe Asn
            980                 985                 990

Ser Glu Ile Leu Cys Ala Ala Leu  Trp Gly Val Asn Leu  Leu Val Gly
        995                 1000                1005

Thr Glu Asn Gly Leu Met Leu Leu Asp Arg Ser Gly Gln Gly Lys
    1010                1015                1020

Val Tyr Asn Leu Ile Asn Arg Arg Arg Phe Gln Gln Met Asp Val
    1025                1030                1035

Leu Glu Gly Leu Asn Val Leu Val Thr Ile Ser Gly Lys Lys Asn
    1040                1045                1050

Lys Leu Arg Val Tyr Tyr Leu Ser Trp Leu Arg Asn Arg Ile Leu
    1055                1060                1065

His Asn Asp Pro Glu Val Glu Lys Lys Gln Gly Trp Ile Thr Val
    1070                1075                1080

Gly Asp Leu Glu Gly Cys Ile His Tyr Lys Val Val Lys Tyr Glu
    1085                1090                1095

Arg Ile Lys Phe Leu Val Ile Ala Leu Lys Asn Ala Val Glu Ile
    1100                1105                1110

Tyr Ala Trp Ala Pro Lys Pro Tyr His Lys Phe Met Ala Phe Lys
    1115                1120                1125

Ser Phe Ala Asp Leu Gln His Lys Pro Leu Leu Val Asp Leu Thr
    1130                1135                1140

Val Glu Glu Gly Gln Arg Leu Lys Val Ile Phe Gly Ser His Thr
    1145                1150                1155

Gly Phe His Val Ile Asp Val Asp Ser Gly Asn Ser Tyr Asp Ile
    1160                1165                1170

Tyr Ile Pro Ser His Ile Gln Gly Asn Ile Thr Pro His Ala Ile
    1175                1180                1185

Val Ile Leu Pro Lys Thr Asp Gly Met Glu Met Leu Val Cys Tyr
    1190                1195                1200

Glu Asp Glu Gly Val Tyr Val Asn Thr Tyr Gly Arg Ile Thr Lys
    1205                1210                1215

Asp Val Val Leu Gln Trp Gly Glu Met Pro Thr Ser Val Ala Tyr
    1220                1225                1230

Ile His Ser Asn Gln Ile Met Gly Trp Gly Glu Lys Ala Ile Glu

1235                1240                1245

Ile Arg Ser Val Glu Thr Gly His Leu Asp Gly Val Phe Met His
    1250                1255                1260

Lys Arg Ala Gln Arg Leu Lys Phe Leu Cys Glu Arg Asn Asp Lys
    1265                1270                1275

Val Phe Phe Ala Ser Val Arg Ser Gly Gly Ser Ser Gln Val Phe
    1280                1285                1290

Phe Met Thr Leu Asn Arg Asn Ser Met Met Asn Trp
    1295                1300                1305

<210>  43
<211>  5627
<212>  DNA
<213>  Homo sapiens

<220>
<221>  CDS
<222>  (346)..(4239)

<400>  43
attataacag ccaatgcagc cgccatccac gcacaagcaa agaagcatgt cccatttaaa      60

tacacccacg cagccccagc gcccttagcc gatcggggcg ctcagcccac acgcaccgct     120

gctcggggct tggagatccg cgcaggctgg gctcccggac ccggcggacc gacgcgcgga     180

ggatcgggat ccggcgctgt ggggctgggg tggcgggggg aggctgggcc cggggcctct     240

ggcgcgacac ccgcatgagg acgcgagtga aatagaccaa ggtggaattt ccaagggaaa      300

agcttcgggg tggttttggt ccatttctcc agcgaagaag tagac atg gcg agc gac      357
                                                  Met Ala Ser Asp
                                                  1

tcc ccg gct cga agc ctg gat gaa ata gat ctc tcg gct ctg agg gac      405
Ser Pro Ala Arg Ser Leu Asp Glu Ile Asp Leu Ser Ala Leu Arg Asp
5                   10                  15                  20

ccc gca ggg atc ttt gaa ttg gtg gaa ctt gtt gga aat gga aca tac      453
Pro Ala Gly Ile Phe Glu Leu Val Glu Leu Val Gly Asn Gly Thr Tyr
                25                  30                  35

ggg caa gtt tat aag ggt cgt cat gtc aaa acg ggc cag ctt gca gcc      501
Gly Gln Val Tyr Lys Gly Arg His Val Lys Thr Gly Gln Leu Ala Ala
            40                  45                  50

atc aag gtt atg gat gtc aca ggg gat gaa gag gaa gaa atc aaa caa      549
Ile Lys Val Met Asp Val Thr Gly Asp Glu Glu Glu Glu Ile Lys Gln
        55                  60                  65

gaa att aac atg ttg aag aaa tat tct cat cac cgg aat att gct aca      597
Glu Ile Asn Met Leu Lys Lys Tyr Ser His His Arg Asn Ile Ala Thr
    70                  75                  80

tac tat ggt gct ttt atc aaa aag aac cca cca ggc atg gat gac caa 645
Tyr Tyr Gly Ala Phe Ile Lys Lys Asn Pro Pro Gly Met Asp Asp Gln
85                  90                  95                  100

ctt tgg ttg gtg atg gag ttt tgt ggt gct ggc tct gtc acc gac ctg 693
Leu Trp Leu Val Met Glu Phe Cys Gly Ala Gly Ser Val Thr Asp Leu
                105                 110                 115

atc aag aac aca aaa ggt aac acg ttg aaa gag gag tgg att gca tac 741
Ile Lys Asn Thr Lys Gly Asn Thr Leu Lys Glu Glu Trp Ile Ala Tyr
            120                 125                 130

atc tgc agg gaa atc tta cgg ggg ctg agt cac ctg cac cag cat aaa 789
Ile Cys Arg Glu Ile Leu Arg Gly Leu Ser His Leu His Gln His Lys
        135                 140                 145

gtg att cat cga gat att aaa ggg caa aat gtc ttg ctg act gaa aat 837
Val Ile His Arg Asp Ile Lys Gly Gln Asn Val Leu Leu Thr Glu Asn
    150                 155                 160

gca gaa gtt aaa cta gtg gac ttt gga gtc agt gct cag ctt gat cga 885
Ala Glu Val Lys Leu Val Asp Phe Gly Val Ser Ala Gln Leu Asp Arg
165                 170                 175                 180

aca gtg ggc agg agg aat act ttc att gga act ccc tac tgg atg gca 933
Thr Val Gly Arg Arg Asn Thr Phe Ile Gly Thr Pro Tyr Trp Met Ala
                185                 190                 195

cca gaa gtt att gcc tgt gat gaa aac cca gat gcc aca tat gat ttc 981
Pro Glu Val Ile Ala Cys Asp Glu Asn Pro Asp Ala Thr Tyr Asp Phe
            200                 205                 210

aag agt gac ttg tgg tct ttg ggt atc acc gcc att gaa atg gca gaa 1029
Lys Ser Asp Leu Trp Ser Leu Gly Ile Thr Ala Ile Glu Met Ala Glu
        215                 220                 225

ggt gct ccc cct ctc tgt gac atg cac ccc atg aga gct ctc ttc ctc 1077
Gly Ala Pro Pro Leu Cys Asp Met His Pro Met Arg Ala Leu Phe Leu
    230                 235                 240

atc ccc cgg aac cca gcg cct cgg ctg aag tct aag aag tgg tca aaa 1125
Ile Pro Arg Asn Pro Ala Pro Arg Leu Lys Ser Lys Lys Trp Ser Lys
245                 250                 255                 260

aaa ttc cag tca ttt att gag agc tgc ttg gta aag aat cac agc cag 1173
Lys Phe Gln Ser Phe Ile Glu Ser Cys Leu Val Lys Asn His Ser Gln
                265                 270                 275

cga cca gca aca gaa caa ttg atg aag cat cca ttt ata cga gac caa 1221
Arg Pro Ala Thr Glu Gln Leu Met Lys His Pro Phe Ile Arg Asp Gln
            280                 285                 290

cct aat gag cga cag gtc cgc att caa ctc aag gac cat att gat aga 1269
Pro Asn Glu Arg Gln Val Arg Ile Gln Leu Lys Asp His Ile Asp Arg
        295                 300                 305

aca aag aag aag cga gga gaa aaa gat gag aca gag tat gag tac agt 1317
Thr Lys Lys Lys Arg Gly Glu Lys Asp Glu Thr Glu Tyr Glu Tyr Ser
    310                 315                 320

gga agt gag gaa gaa gag gag gag aat gac tca gga gag ccc agc tcc 1365
Gly Ser Glu Glu Glu Glu Glu Glu Asn Asp Ser Gly Glu Pro Ser Ser

325                 330                 335                 340

atc ctg aat ctg cca ggg gag tcg acg ctg cgg agg gac ttt ctg agg      1413
Ile Leu Asn Leu Pro Gly Glu Ser Thr Leu Arg Arg Asp Phe Leu Arg
                345                 350                 355

ctg cag ctg gcc aac aag gag cgt tct gag gcc cta cgg agg cag cag      1461
Leu Gln Leu Ala Asn Lys Glu Arg Ser Glu Ala Leu Arg Arg Gln Gln
            360                 365                 370

ctg gag cag cag cag cgg gag aat gag gag cac aag cgg cag ctg ctg      1509
Leu Glu Gln Gln Gln Arg Glu Asn Glu Glu His Lys Arg Gln Leu Leu
        375                 380                 385

gcc gag cgt cag aag cgc atc gag gag cag aaa gag cag agg cgg cgg      1557
Ala Glu Arg Gln Lys Arg Ile Glu Glu Gln Lys Glu Gln Arg Arg Arg
    390                 395                 400

ctg gag gag caa caa agg cga gag aag gag ctg cgg aag cag cag gag      1605
Leu Glu Glu Gln Gln Arg Arg Glu Lys Glu Leu Arg Lys Gln Gln Glu
405                 410                 415                 420

agg gag cag cgc cgg cac tat gag gag cag atg cgc cgg gag gag gag      1653
Arg Glu Gln Arg Arg His Tyr Glu Glu Gln Met Arg Arg Glu Glu Glu
                425                 430                 435

agg agg cgt gcg gag cat gaa cag gaa tac atc agg cga cag tta gag      1701
Arg Arg Arg Ala Glu His Glu Gln Glu Tyr Ile Arg Arg Gln Leu Glu
            440                 445                 450

gag gag cag aga cag tta gag atc ttg cag cag cag cta ctg cat gaa      1749
Glu Glu Gln Arg Gln Leu Glu Ile Leu Gln Gln Gln Leu Leu His Glu
        455                 460                 465

caa gct cta ctt ctg gaa tat aag cgc aaa caa ttg gaa gaa cag aga      1797
Gln Ala Leu Leu Leu Glu Tyr Lys Arg Lys Gln Leu Glu Glu Gln Arg
    470                 475                 480

caa gca gaa aga ctg cag agg cag cta aag caa gaa aga gac tac tta      1845
Gln Ala Glu Arg Leu Gln Arg Gln Leu Lys Gln Glu Arg Asp Tyr Leu
485                 490                 495                 500

gtt tcc ctt cag cat cag cgg cag gag cag agg cct gtg gag aag aag      1893
Val Ser Leu Gln His Gln Arg Gln Glu Gln Arg Pro Val Glu Lys Lys
                505                 510                 515

cca ctg tac cat tac aaa gaa gga atg agt cct agt gag aag cca gca      1941
Pro Leu Tyr His Tyr Lys Glu Gly Met Ser Pro Ser Glu Lys Pro Ala
            520                 525                 530

tgg gcc aag gag atc cca cat ctg gta gct gta aaa tcc cag gga cct      1989
Trp Ala Lys Glu Ile Pro His Leu Val Ala Val Lys Ser Gln Gly Pro
        535                 540                 545

gcc ttg acc gcc tcc cag tca gtg cac gag cag ccc aca aag ggc ctc      2037
Ala Leu Thr Ala Ser Gln Ser Val His Glu Gln Pro Thr Lys Gly Leu
    550                 555                 560

tct ggg ttt cag gag gct ctg aac gtg acc tcc cac cgc gtg gag atg      2085
Ser Gly Phe Gln Glu Ala Leu Asn Val Thr Ser His Arg Val Glu Met
565                 570                 575                 580

cca cgc cag aac tca gat ccc acc tcg gaa aat cct cct ctc ccc act      2133

Pro Arg Gln Asn Ser Asp Pro Thr Ser Glu Asn Pro Pro Leu Pro Thr
                585                 590                 595

cgc att gaa aag ttt gac cga agc tct tgg tta cga cag gaa gaa gac      2181
Arg Ile Glu Lys Phe Asp Arg Ser Ser Trp Leu Arg Gln Glu Glu Asp
            600                 605                 610

att cca cca aag gtg cct caa aga aca act tct ata tcc cca gca tta      2229
Ile Pro Pro Lys Val Pro Gln Arg Thr Thr Ser Ile Ser Pro Ala Leu
        615                 620                 625

gcc aga aag aat tct cct ggg aat ggt agt gct ctg gga ccc aga cta      2277
Ala Arg Lys Asn Ser Pro Gly Asn Gly Ser Ala Leu Gly Pro Arg Leu
    630                 635                 640

gga tct caa ccc atc aga gca agc aac cct gat ctc cgg aga act gag      2325
Gly Ser Gln Pro Ile Arg Ala Ser Asn Pro Asp Leu Arg Arg Thr Glu
645                 650                 655                 660

ccc atc ttg gag agc ccc ttg cag agg acc agc agt ggc agt tcc tcc      2373
Pro Ile Leu Glu Ser Pro Leu Gln Arg Thr Ser Ser Gly Ser Ser Ser
                665                 670                 675

agc tcc agc acc cct agc tcc cag ccc agc tcc caa gga ggc tcc cag      2421
Ser Ser Ser Thr Pro Ser Ser Gln Pro Ser Ser Gln Gly Gly Ser Gln
            680                 685                 690

cct gga tca caa gca gga tcc agt gaa cgc acc aga gtt cga gcc aac      2469
Pro Gly Ser Gln Ala Gly Ser Ser Glu Arg Thr Arg Val Arg Ala Asn
        695                 700                 705

agt aag tca gaa gga tca cct gtg ctc ccc cat gag cct gcc aag gtg      2517
Ser Lys Ser Glu Gly Ser Pro Val Leu Pro His Glu Pro Ala Lys Val
    710                 715                 720

aaa cca gaa gaa tcc agg gac att acc cgg ccc agt cga cca gct gat      2565
Lys Pro Glu Glu Ser Arg Asp Ile Thr Arg Pro Ser Arg Pro Ala Asp
725                 730                 735                 740

ctg acg gca tta gcc aaa gaa cta aga gaa ctc cgg att gaa gaa aca      2613
Leu Thr Ala Leu Ala Lys Glu Leu Arg Glu Leu Arg Ile Glu Glu Thr
                745                 750                 755

aac cgc cca atg aag aag gtg act gat tac tcc tcc tcc agt gag gag      2661
Asn Arg Pro Met Lys Lys Val Thr Asp Tyr Ser Ser Ser Ser Glu Glu
            760                 765                 770

tca gaa agt agc gag gaa gag gag gaa gat gga gag agc gag acc cat      2709
Ser Glu Ser Ser Glu Glu Glu Glu Glu Asp Gly Glu Ser Glu Thr His
        775                 780                 785

gat ggg aca gtg gct gtc agc gac ata ccc aga ctg ata cca aca gga      2757
Asp Gly Thr Val Ala Val Ser Asp Ile Pro Arg Leu Ile Pro Thr Gly
    790                 795                 800

gct cca ggc agc aac gag cag tac aat gtg gga atg gtg ggg acg cat      2805
Ala Pro Gly Ser Asn Glu Gln Tyr Asn Val Gly Met Val Gly Thr His
805                 810                 815                 820

ggg ctg gag acc tct cat gcg gac agt ttc agc ggc agt att tca aga      2853
Gly Leu Glu Thr Ser His Ala Asp Ser Phe Ser Gly Ser Ile Ser Arg
                825                 830                 835

gaa gga acc ttg atg att aga gag acg tct gga gag aag aag cga tct 2901
Glu Gly Thr Leu Met Ile Arg Glu Thr Ser Gly Glu Lys Lys Arg Ser
            840                 845                 850

ggc cac agt gac agc aat ggc ttt gct ggc cac atc aac ctc cct gac 2949
Gly His Ser Asp Ser Asn Gly Phe Ala Gly His Ile Asn Leu Pro Asp
        855                 860                 865

ctg gtg cag cag agc cat tct cca gct gga acc ccg act gag gga ctg 2997
Leu Val Gln Gln Ser His Ser Pro Ala Gly Thr Pro Thr Glu Gly Leu
    870                 875                 880

ggg cgc gtc tca acc cat tcc cag gag atg gac tct ggg act gaa tat 3045
Gly Arg Val Ser Thr His Ser Gln Glu Met Asp Ser Gly Thr Glu Tyr
885                 890                 895                 900

ggc atg ggg agc agc acc aaa gcc tcc ttc acc ccc ttt gtg gac ccc 3093
Gly Met Gly Ser Ser Thr Lys Ala Ser Phe Thr Pro Phe Val Asp Pro
                905                 910                 915

aga gta tac cag acg tct ccc act gat gaa gat gaa gag gat gag gaa 3141
Arg Val Tyr Gln Thr Ser Pro Thr Asp Glu Asp Glu Glu Asp Glu Glu
            920                 925                 930

tca tca gcc gca gct ctg ttt act agc gaa ctt ctt agg caa gaa cag 3189
Ser Ser Ala Ala Ala Leu Phe Thr Ser Glu Leu Leu Arg Gln Glu Gln
        935                 940                 945

gcc aaa ctc aat gaa gca aga aag att tcg gtg gta aat gta aac cca 3237
Ala Lys Leu Asn Glu Ala Arg Lys Ile Ser Val Val Asn Val Asn Pro
    950                 955                 960

acc aac att cgg cct cat agc gac aca cca gaa atc aga aaa tac aag 3285
Thr Asn Ile Arg Pro His Ser Asp Thr Pro Glu Ile Arg Lys Tyr Lys
965                 970                 975                 980

aaa cga ttc aac tca gaa ata ctt tgt gca gct ctg tgg ggt gta aac 3333
Lys Arg Phe Asn Ser Glu Ile Leu Cys Ala Ala Leu Trp Gly Val Asn
                985                 990                 995

ctt ctg gtg ggg act gaa aat ggc ctg atg ctt ttg gac cga agt 3378
Leu Leu Val Gly Thr Glu Asn Gly Leu Met Leu Leu Asp Arg Ser
            1000                1005                1010

ggg caa ggc aaa gtc tat aat ctg atc aac cgg agg cga ttt cag 3423
Gly Gln Gly Lys Val Tyr Asn Leu Ile Asn Arg Arg Arg Phe Gln
            1015                1020                1025

cag atg gat gtg cta gag gga ctg aat gtc ctt gtg aca att tca 3468
Gln Met Asp Val Leu Glu Gly Leu Asn Val Leu Val Thr Ile Ser
            1030                1035                1040

gga aag aag aat aag cta cga gtt tac tat ctt tca tgg tta aga 3513
Gly Lys Lys Asn Lys Leu Arg Val Tyr Tyr Leu Ser Trp Leu Arg
            1045                1050                1055

aac aga ata cta cat aat gac cca gaa gta gaa aag aaa caa ggc 3558
Asn Arg Ile Leu His Asn Asp Pro Glu Val Glu Lys Lys Gln Gly
            1060                1065                1070

tgg atc act gtt ggg gac ttg gaa ggc tgt ata cat tat aaa gtt 3603
Trp Ile Thr Val Gly Asp Leu Glu Gly Cys Ile His Tyr Lys Val
            1075                1080                1085

gtt aaa tat gaa agg atc aaa ttt ttg gtg att gcc tta aag aat 3648
Val Lys Tyr Glu Arg Ile Lys Phe Leu Val Ile Ala Leu Lys Asn
            1090                1095                1100

gct gtg gaa ata tat gct tgg gct cct aaa ccg tat cat aaa ttc 3693
Ala Val Glu Ile Tyr Ala Trp Ala Pro Lys Pro Tyr His Lys Phe
            1105                1110                1115

atg gca ttt aag tct ttt gca gat ctc cag cac aag cct ctg cta 3738
Met Ala Phe Lys Ser Phe Ala Asp Leu Gln His Lys Pro Leu Leu
            1120                1125                1130

gtt gat ctc acg gta gaa gaa ggt caa aga tta aag gtt att ttt 3783
Val Asp Leu Thr Val Glu Glu Gly Gln Arg Leu Lys Val Ile Phe
            1135                1140                1145

ggt tca cac act ggt ttc cat gta att gat gtt gat tca gga aac 3828
Gly Ser His Thr Gly Phe His Val Ile Asp Val Asp Ser Gly Asn
            1150                1155                1160

tct tat gat atc tac ata cca tct cat att cag ggc aat atc act 3873
Ser Tyr Asp Ile Tyr Ile Pro Ser His Ile Gln Gly Asn Ile Thr
            1165                1170                1175

cct cat gct att gtc atc ttg cct aaa aca gat gga atg gaa atg 3918
Pro His Ala Ile Val Ile Leu Pro Lys Thr Asp Gly Met Glu Met
            1180                1185                1190

ctt gtt tgc tat gag gat gag ggg gtg tat gta aac acc tat ggc 3963
Leu Val Cys Tyr Glu Asp Glu Gly Val Tyr Val Asn Thr Tyr Gly
            1195                1200                1205

cgg ata act aag gat gtg gtg ctc caa tgg gga gaa atg ccc acg 4008
Arg Ile Thr Lys Asp Val Val Leu Gln Trp Gly Glu Met Pro Thr
            1210                1215                1220

tct gtg gcc tac att cat tcc aat cag ata atg ggc tgg ggc gag 4053
Ser Val Ala Tyr Ile His Ser Asn Gln Ile Met Gly Trp Gly Glu
            1225                1230                1235

aaa gct att gag atc cgg tca gtg gaa aca gga cat ttg gat gga 4098
Lys Ala Ile Glu Ile Arg Ser Val Glu Thr Gly His Leu Asp Gly
            1240                1245                1250

gta ttt atg cat aag cga gct caa agg tta aag ttt cta tgt gaa 4143
Val Phe Met His Lys Arg Ala Gln Arg Leu Lys Phe Leu Cys Glu
            1255                1260                1265

aga aat gat aag gta ttt ttt gca tcc gtg cga tct gga gga agt 4188
Arg Asn Asp Lys Val Phe Phe Ala Ser Val Arg Ser Gly Gly Ser
            1270                1275                1280

agc caa gtg ttt ttc atg acc ctc aac aga aat tcc atg atg aac 4233
Ser Gln Val Phe Phe Met Thr Leu Asn Arg Asn Ser Met Met Asn
            1285                1290                1295

tgg taa cagaagagca cttggcactt atcttcatgg cgttatttct aatttaaaag 4289
Trp

aacataactc atgtggactt atgccagtct agaggcagaa tcagaaggct tggttgaaca 4349

tatcgctttc cctttttcct ctccctccgc ccctcccagt acagtccatc tttcaatgtt 4409

gcagcctggt tgagaaggag agaaaaaggt ggcaggaatt tccaggagat ccccaagaat 4469

gctgccttgt ctgtggacaa agatggacca tgtgcccttc ggaattaggg atagaaacaa 4529

atattgtgtg ctcttaacga ttaagctgtg ttatggtggg ttttcaggtt tttacctttt 4589

ttctttaccc ctttactctg caagaatggg gaaagaatgc atactgcgaa aatgagtctt 4649

ttaaattctg tctgcctact agttttaagt atatggtatg ttgtaaaatt tccaatgatg 4709

agagacagca caataaatgt accttatctc cttaggctga aggccataac tacatagtgg 4769

agtaatttaa gaactctctt gccttcacca acccaaaagg ttgctttttg atagcaactg 4829

gctaatgaat ttttaaaaag agaagaaaaa tactagtttt cccctctttt gggaaataga 4889

ttttaaatgg ctaaactact agccttaaaa ctactagtct aataaaatca actaccactt 4949

ttgtgaatct gacaggccac atttttatat ggccctttac agaatggagt gtgttgaaca 5009

ggatactaac gccattgagt tgagctggcc tagcgatgga gggacactct aacacaactt 5069

tccctcagct attatgcaac agatcaggga aaaagatggg atgacagatg gggtcagaca 5129

gaaagagctt ctgggaaaca agcttacata gtctttttta aaatgcacaa agcctcccag 5189

ctaagaggtc acttggtttg ggcttcatta ggactgagac tttgttgagt tctttctggg 5249

acttggagag tggatgatat tcaggctctg aacattccca gcgctctccc gagggtgcca 5309

ctttctcaag atgaaaactg tgactgaaaa aattaataat aaatgtttct gagctgcctg 5369

tgttctccct gtgtgggtga gagaagggac tagactccta agcctgcctc agatacaaga 5429

ggcatcattg gctccaattt tagagaactt gaaagcaagg ctttggacaa aattttgaga 5489

ccctaatcac tttaccttcc tccaaattac ccaacatacg gtaaacaaca tttgtgcaga 5549

agtatgtatg tatttagttc aggttgactt gtgtccttat aaactcttac tcaaatgatt 5609

tgaactttaa aaaaaaaa 5627

<210> 44
<211> 1297
<212> PRT
<213> Homo sapiens

<400> 44
   Met Ala Ser Asp Ser Pro Ala Arg Ser Leu Asp Glu Ile Asp Leu Ser
   1 5 10 15
   Ala Leu Arg Asp Pro Ala Gly Ile Phe Glu Leu Val Glu Leu Val Gly
   20 25 30
   Asn Gly Thr Tyr Gly Gln Val Tyr Lys Gly Arg His Val Lys Thr Gly
   35 40 45
   Gln Leu Ala Ala Ile Lys Val Met Asp Val Thr Gly Asp Glu Glu Glu
       50                  55                  60
   Glu Ile Lys Gln Glu Ile Asn Met Leu Lys Lys Tyr Ser His His Arg
   65                  70                  75                  80
   Asn Ile Ala Thr Tyr Tyr Gly Ala Phe Ile Lys Lys Asn Pro Pro Gly
                   85                  90                  95
   Met Asp Asp Gln Leu Trp Leu Val Met Glu Phe Cys Gly Ala Gly Ser
               100                 105                 110
   Val Thr Asp Leu Ile Lys Asn Thr Lys Gly Asn Thr Leu Lys Glu Glu
           115                 120                 125
   Trp Ile Ala Tyr Ile Cys Arg Glu Ile Leu Arg Gly Leu Ser His Leu
       130                 135                 140
   His Gln His Lys Val Ile His Arg Asp Ile Lys Gly Gln Asn Val Leu
   145                 150                 155                 160
   Leu Thr Glu Asn Ala Glu Val Lys Leu Val Asp Phe Gly Val Ser Ala
                   165                 170                 175
   Gln Leu Asp Arg Thr Val Gly Arg Arg Asn Thr Phe Ile Gly Thr Pro
               180                 185                 190
   Tyr Trp Met Ala Pro Glu Val Ile Ala Cys Asp Glu Asn Pro Asp Ala
           195                 200                 205
   Thr Tyr Asp Phe Lys Ser Asp Leu Trp Ser Leu Gly Ile Thr Ala Ile
       210                 215                 220
   Glu Met Ala Glu Gly Ala Pro Pro Leu Cys Asp Met His Pro Met Arg
   225                 230                 235                 240
   Ala Leu Phe Leu Ile Pro Arg Asn Pro Ala Pro Arg Leu Lys Ser Lys
                   245                 250                 255
   Lys Trp Ser Lys Lys Phe Gln Ser Phe Ile Glu Ser Cys Leu Val Lys
               260                 265                 270
   Asn His Ser Gln Arg Pro Ala Thr Glu Gln Leu Met Lys His Pro Phe
           275                 280                 285
   Ile Arg Asp Gln Pro Asn Glu Arg Gln Val Arg Ile Gln Leu Lys Asp
       290                 295                 300
   His Ile Asp Arg Thr Lys Lys Lys Arg Gly Glu Lys Asp Glu Thr Glu
   305                 310                 315                 320
   Tyr Glu Tyr Ser Gly Ser Glu Glu Glu Glu Glu Glu Asn Asp Ser Gly
                   325                 330                 335
   Glu Pro Ser Ser Ile Leu Asn Leu Pro Gly Glu Ser Thr Leu Arg Arg
               340                 345                 350
   Asp Phe Leu Arg Leu Gln Leu Ala Asn Lys Glu Arg Ser Glu Ala Leu
           355                 360                 365
   Arg Arg Gln Gln Leu Glu Gln Gln Gln Arg Glu Asn Glu Glu His Lys
       370                 375                 380
   Arg Gln Leu Leu Ala Glu Arg Gln Lys Arg Ile Glu Glu Gln Lys Glu
   385                 390                 395                 400
   Gln Arg Arg Arg Leu Glu Glu Gln Gln Arg Arg Glu Lys Glu Leu Arg
                   405                 410                 415
   Lys Gln Gln Glu Arg Glu Gln Arg Arg His Tyr Glu Glu Gln Met Arg
               420                 425                 430
   Arg Glu Glu Glu Arg Arg Arg Ala Glu His Glu Gln Glu Tyr Ile Arg
           435                 440                 445
   Arg Gln Leu Glu Glu Glu Gln Arg Gln Leu Glu Ile Leu Gln Gln Gln
       450                 455                 460
   Leu Leu His Glu Gln Ala Leu Leu Leu Glu Tyr Lys Arg Lys Gln Leu
   465                 470                 475                 480
   Glu Glu Gln Arg Gln Ala Glu Arg Leu Gln Arg Gln Leu Lys Gln Glu
                   485                 490                 495
   Arg Asp Tyr Leu Val Ser Leu Gln His Gln Arg Gln Glu Gln Arg Pro
               500                 505                 510
   Val Glu Lys Lys Pro Leu Tyr His Tyr Lys Glu Gly Met Ser Pro Ser
           515                 520                 525
   Glu Lys Pro Ala Trp Ala Lys Glu Ile Pro His Leu Val Ala Val Lys
       530                 535                 540
   Ser Gln Gly Pro Ala Leu Thr Ala Ser Gln Ser Val His Glu Gln Pro
   545                 550                 555                 560
   Thr Lys Gly Leu Ser Gly Phe Gln Glu Ala Leu Asn Val Thr Ser His
                   565                 570                 575
   Arg Val Glu Met Pro Arg Gln Asn Ser Asp Pro Thr Ser Glu Asn Pro
               580                 585                 590
   Pro Leu Pro Thr Arg Ile Glu Lys Phe Asp Arg Ser Ser Trp Leu Arg
           595                 600                 605
   Gln Glu Glu Asp Ile Pro Pro Lys Val Pro Gln Arg Thr Thr Ser Ile
       610                 615                 620
   Ser Pro Ala Leu Ala Arg Lys Asn Ser Pro Gly Asn Gly Ser Ala Leu
   625                 630                 635                 640
   Gly Pro Arg Leu Gly Ser Gln Pro Ile Arg Ala Ser Asn Pro Asp Leu
                   645                 650                 655
   Arg Arg Thr Glu Pro Ile Leu Glu Ser Pro Leu Gln Arg Thr Ser Ser
               660                 665                 670
   Gly Ser Ser Ser Ser Ser Ser Thr Pro Ser Ser Gln Pro Ser Ser Gln
           675                 680                 685
   Gly Gly Ser Gln Pro Gly Ser Gln Ala Gly Ser Ser Glu Arg Thr Arg
       690                 695                 700
   Val Arg Ala Asn Ser Lys Ser Glu Gly Ser Pro Val Leu Pro His Glu
   705                 710                 715                 720
   Pro Ala Lys Val Lys Pro Glu Glu Ser Arg Asp Ile Thr Arg Pro Ser
                   725                 730                 735
   Arg Pro Ala Asp Leu Thr Ala Leu Ala Lys Glu Leu Arg Glu Leu Arg
               740                 745                 750
   Ile Glu Glu Thr Asn Arg Pro Met Lys Lys Val Thr Asp Tyr Ser Ser
           755                 760                 765
   Ser Ser Glu Glu Ser Glu Ser Ser Glu Glu Glu Glu Glu Asp Gly Glu
       770                 775                 780
   Ser Glu Thr His Asp Gly Thr Val Ala Val Ser Asp Ile Pro Arg Leu
   785                 790                 795                 800
   Ile Pro Thr Gly Ala Pro Gly Ser Asn Glu Gln Tyr Asn Val Gly Met
                   805                 810                 815
   Val Gly Thr His Gly Leu Glu Thr Ser His Ala Asp Ser Phe Ser Gly
               820                 825                 830
   Ser Ile Ser Arg Glu Gly Thr Leu Met Ile Arg Glu Thr Ser Gly Glu
           835                 840                 845
   Lys Lys Arg Ser Gly His Ser Asp Ser Asn Gly Phe Ala Gly His Ile
       850                 855                 860
   Asn Leu Pro Asp Leu Val Gln Gln Ser His Ser Pro Ala Gly Thr Pro
   865                 870                 875                 880
   Thr Glu Gly Leu Gly Arg Val Ser Thr His Ser Gln Glu Met Asp Ser
                   885                 890                 895
   Gly Thr Glu Tyr Gly Met Gly Ser Ser Thr Lys Ala Ser Phe Thr Pro
               900                 905                 910
   Phe Val Asp Pro Arg Val Tyr Gln Thr Ser Pro Thr Asp Glu Asp Glu
           915                 920                 925
   Glu Asp Glu Glu Ser Ser Ala Ala Ala Leu Phe Thr Ser Glu Leu Leu
       930                 935                 940
   Arg Gln Glu Gln Ala Lys Leu Asn Glu Ala Arg Lys Ile Ser Val Val
   945                 950                 955                 960
   Asn Val Asn Pro Thr Asn Ile Arg Pro His Ser Asp Thr Pro Glu Ile
                   965                 970                 975
   Arg Lys Tyr Lys Lys Arg Phe Asn Ser Glu Ile Leu Cys Ala Ala Leu
               980                 985                 990
   Trp Gly Val Asn Leu Leu Val Gly Thr Glu Asn Gly Leu Met Leu Leu
           995                 1000                1005
   Asp Arg Ser Gly Gln Gly Lys Val Tyr Asn Leu Ile Asn Arg Arg
       1010                1015                1020
   Arg Phe Gln Gln Met Asp Val Leu Glu Gly Leu Asn Val Leu Val
       1025                1030                1035
   Thr Ile Ser Gly Lys Lys Asn Lys Leu Arg Val Tyr Tyr Leu Ser
       1040                1045                1050
   Trp Leu Arg Asn Arg Ile Leu His Asn Asp Pro Glu Val Glu Lys
   1055 1060 1065
   Lys Gln Gly Trp Ile Thr Val Gly Asp Leu Glu Gly Cys Ile His
   1070 1075 1080
   Tyr Lys Val Val Lys Tyr Glu Arg Ile Lys Phe Leu Val Ile Ala
   1085 1090 1095
   Leu Lys Asn Ala Val Glu Ile Tyr Ala Trp Ala Pro Lys Pro Tyr
   1100 1105 1110
   His Lys Phe Met Ala Phe Lys Ser Phe Ala Asp Leu Gln His Lys
   1115 1120 1125
   Pro Leu Leu Val Asp Leu Thr Val Glu Glu Gly Gln Arg Leu Lys
   1130 1135 1140
   Val Ile Phe Gly Ser His Thr Gly Phe His Val Ile Asp Val Asp
   1145 1150 1155
   Ser Gly Asn Ser Tyr Asp Ile Tyr Ile Pro Ser His Ile Gln Gly
   1160 1165 1170
   Asn Ile Thr Pro His Ala Ile Val Ile Leu Pro Lys Thr Asp Gly
   1175 1180 1185
   Met Glu Met Leu Val Cys Tyr Glu Asp Glu Gly Val Tyr Val Asn
   1190 1195 1200
   Thr Tyr Gly Arg Ile Thr Lys Asp Val Val Leu Gln Trp Gly Glu
   1205 1210 1215
   Met Pro Thr Ser Val Ala Tyr Ile His Ser Asn Gln Ile Met Gly
   1220 1225 1230
   Trp Gly Glu Lys Ala Ile Glu Ile Arg Ser Val Glu Thr Gly His
   1235 1240 1245
   Leu Asp Gly Val Phe Met His Lys Arg Ala Gln Arg Leu Lys Phe
   1250 1255 1260
   Leu Cys Glu Arg Asn Asp Lys Val Phe Phe Ala Ser Val Arg Ser
   1265 1270 1275
   Gly Gly Ser Ser Gln Val Phe Phe Met Thr Leu Asn Arg Asn Ser
   1280 1285 1290
   Met Met  Asn Trp
       1295

<210>  45
<211>  5564
<212>  DNA
<213>  Homo sapiens

<220>
<221>  CDS
<222>  (346)..(4176)

<400>  45
attataacag ccaatgcagc cgccatccac gcacaagcaa agaagcatgt cccatttaaa      60

tacacccacg cagccccagc gcccttagcc gatcggggcg ctcagcccac acgcaccgct     120

gctcggggct tggagatccg cgcaggctgg gctcccggac ccggcggacc gacgcgcgga     180

ggatcgggat ccggcgctgt ggggctgggg tggcgggggg aggctgggcc cggggcctct     240

ggcgcgacac ccgcatgagg acgcgagtga aatagaccaa ggtggaattt ccaagggaaa     300

agcttcgggg tggttttggt ccatttctcc agcgaagaag tagac atg gcg agc gac     357
                                                  Met Ala Ser Asp
                                                  1

tcc ccg gct cga agc ctg gat gaa ata gat ctc tcg gct ctg agg gac     405
Ser Pro Ala Arg Ser Leu Asp Glu Ile Asp Leu Ser Ala Leu Arg Asp
5                   10                  15                  20

ccc gca ggg atc ttt gaa ttg gtg gaa ctt gtt gga aat gga aca tac     453
Pro Ala Gly Ile Phe Glu Leu Val Glu Leu Val Gly Asn Gly Thr Tyr
                25                  30                  35

ggg caa gtt tat aag ggt cgt cat gtc aaa acg ggc cag ctt gca gcc     501
Gly Gln Val Tyr Lys Gly Arg His Val Lys Thr Gly Gln Leu Ala Ala
            40                  45                  50

atc aag gtt atg gat gtc aca ggg gat gaa gag gaa gaa atc aaa caa     549
Ile Lys Val Met Asp Val Thr Gly Asp Glu Glu Glu Glu Ile Lys Gln
        55                  60                  65

gaa att aac atg ttg aag aaa tat tct cat cac cgg aat att gct aca     597
Glu Ile Asn Met Leu Lys Lys Tyr Ser His His Arg Asn Ile Ala Thr
    70                  75                  80

tac tat ggt gct ttt atc aaa aag aac cca cca ggc atg gat gac caa     645
Tyr Tyr Gly Ala Phe Ile Lys Lys Asn Pro Pro Gly Met Asp Asp Gln
85                  90                  95                  100

ctt tgg ttg gtg atg gag ttt tgt ggt gct ggc tct gtc acc gac ctg     693
Leu Trp Leu Val Met Glu Phe Cys Gly Ala Gly Ser Val Thr Asp Leu
                105                 110                 115

atc aag aac aca aaa ggt aac acg ttg aaa gag gag tgg att gca tac     741
Ile Lys Asn Thr Lys Gly Asn Thr Leu Lys Glu Glu Trp Ile Ala Tyr
            120                 125                 130

atc tgc agg gaa atc tta cgg ggg ctg agt cac ctg cac cag cat aaa 789
Ile Cys Arg Glu Ile Leu Arg Gly Leu Ser His Leu His Gln His Lys
        135                 140                 145

gtg att cat cga gat att aaa ggg caa aat gtc ttg ctg act gaa aat 837
Val Ile His Arg Asp Ile Lys Gly Gln Asn Val Leu Leu Thr Glu Asn
    150                 155                 160

gca gaa gtt aaa cta gtg gac ttt gga gtc agt gct cag ctt gat cga 885
Ala Glu Val Lys Leu Val Asp Phe Gly Val Ser Ala Gln Leu Asp Arg
165                 170                 175                 180

aca gtg ggc agg agg aat act ttc att gga act ccc tac tgg atg gca 933
Thr Val Gly Arg Arg Asn Thr Phe Ile Gly Thr Pro Tyr Trp Met Ala
                185                 190                 195

cca gaa gtt att gcc tgt gat gaa aac cca gat gcc aca tat gat ttc 981
Pro Glu Val Ile Ala Cys Asp Glu Asn Pro Asp Ala Thr Tyr Asp Phe
            200                 205                 210

aag agt gac ttg tgg tct ttg ggt atc acc gcc att gaa atg gca gaa 1029
Lys Ser Asp Leu Trp Ser Leu Gly Ile Thr Ala Ile Glu Met Ala Glu
        215                 220                 225

ggt gct ccc cct ctc tgt gac atg cac ccc atg aga gct ctc ttc ctc 1077
Gly Ala Pro Pro Leu Cys Asp Met His Pro Met Arg Ala Leu Phe Leu
    230                 235                 240

atc ccc cgg aac cca gcg cct cgg ctg aag tct aag aag tgg tca aaa 1125
Ile Pro Arg Asn Pro Ala Pro Arg Leu Lys Ser Lys Lys Trp Ser Lys
245                 250                 255                 260

aaa ttc cag tca ttt att gag agc tgc ttg gta aag aat cac agc cag 1173
Lys Phe Gln Ser Phe Ile Glu Ser Cys Leu Val Lys Asn His Ser Gln
                265                 270                 275

cga cca gca aca gaa caa ttg atg aag cat cca ttt ata cga gac caa 1221
Arg Pro Ala Thr Glu Gln Leu Met Lys His Pro Phe Ile Arg Asp Gln
            280                 285                 290

cct aat gag cga cag gtc cgc att caa ctc aag gac cat att gat aga 1269
Pro Asn Glu Arg Gln Val Arg Ile Gln Leu Lys Asp His Ile Asp Arg
        295                 300                 305

aca aag aag aag cga gga gaa aaa gat gag aca gag tat gag tac agt 1317
Thr Lys Lys Lys Arg Gly Glu Lys Asp Glu Thr Glu Tyr Glu Tyr Ser
    310                 315                 320

gga agt gag gaa gaa gag gag gag aat gac tca gga gag ccc agc tcc 1365
Gly Ser Glu Glu Glu Glu Glu Glu Asn Asp Ser Gly Glu Pro Ser Ser
325                 330                 335                 340

atc ctg aat ctg cca ggg gag tcg acg ctg cgg agg gac ttt ctg agg 1413
Ile Leu Asn Leu Pro Gly Glu Ser Thr Leu Arg Arg Asp Phe Leu Arg
                345                 350                 355

ctg cag ctg gcc aac aag gag cgt tct gag gcc cta cgg agg cag cag 1461
Leu Gln Leu Ala Asn Lys Glu Arg Ser Glu Ala Leu Arg Arg Gln Gln
            360                 365                 370

ctg gag cag cag cag cgg gag aat gag gag cac aag cgg cag ctg ctg 1509
Leu Glu Gln Gln Gln Arg Glu Asn Glu Glu His Lys Arg Gln Leu Leu
        375                 380                 385

gcc gag cgt cag aag cgc atc gag gag cag aaa gag cag agg cgg cgg 1557
Ala Glu Arg Gln Lys Arg Ile Glu Glu Gln Lys Glu Gln Arg Arg Arg
    390                 395                 400

ctg gag gag caa caa agg cga gag aag gag ctg cgg aag cag cag gag 1605
Leu Glu Glu Gln Gln Arg Arg Glu Lys Glu Leu Arg Lys Gln Gln Glu
405                 410                 415                 420

agg gag cag cgc cgg cac tat gag gag cag atg cgc cgg gag gag gag 1653
Arg Glu Gln Arg Arg His Tyr Glu Glu Gln Met Arg Arg Glu Glu Glu
                425                 430                 435

agg agg cgt gcg gag cat gaa cag gaa tat aag cgc aaa caa ttg gaa 1701
Arg Arg Arg Ala Glu His Glu Gln Glu Tyr Lys Arg Lys Gln Leu Glu
            440                 445                 450

gaa cag aga caa gca gaa aga ctg cag agg cag cta aag caa gaa aga 1749
Glu Gln Arg Gln Ala Glu Arg Leu Gln Arg Gln Leu Lys Gln Glu Arg
        455                 460                 465

gac tac tta gtt tcc ctt cag cat cag cgg cag gag cag agg cct gtg 1797
Asp Tyr Leu Val Ser Leu Gln His Gln Arg Gln Glu Gln Arg Pro Val
    470                 475                 480

gag aag aag cca ctg tac cat tac aaa gaa gga atg agt cct agt gag 1845
Glu Lys Lys Pro Leu Tyr His Tyr Lys Glu Gly Met Ser Pro Ser Glu
485                 490                 495                 500

aag cca gca tgg gcc aag gag atc cca cat ctg gta gct gta aaa tcc 1893
Lys Pro Ala Trp Ala Lys Glu Ile Pro His Leu Val Ala Val Lys Ser
                505                 510                 515

cag gga cct gcc ttg acc gcc tcc cag tca gtg cac gag cag ccc aca 1941
Gln Gly Pro Ala Leu Thr Ala Ser Gln Ser Val His Glu Gln Pro Thr
            520                 525                 530

aag ggc ctc tct ggg ttt cag gag gct ctg aac gtg acc tcc cac cgc 1989
Lys Gly Leu Ser Gly Phe Gln Glu Ala Leu Asn Val Thr Ser His Arg
        535                 540                 545

gtg gag atg cca cgc cag aac tca gat ccc acc tcg gaa aat cct cct 2037
Val Glu Met Pro Arg Gln Asn Ser Asp Pro Thr Ser Glu Asn Pro Pro
    550                 555                 560

ctc ccc act cgc att gaa aag ttt gac cga agc tct tgg tta cga cag 2085
Leu Pro Thr Arg Ile Glu Lys Phe Asp Arg Ser Ser Trp Leu Arg Gln
565                 570                 575                 580

gaa gaa gac att cca cca aag gtg cct caa aga aca act tct ata tcc 2133
Glu Glu Asp Ile Pro Pro Lys Val Pro Gln Arg Thr Thr Ser Ile Ser
                585                 590                 595

cca gca tta gcc aga aag aat tct cct ggg aat ggt agt gct ctg gga 2181
Pro Ala Leu Ala Arg Lys Asn Ser Pro Gly Asn Gly Ser Ala Leu Gly
            600                 605                 610

ccc aga cta gga tct caa ccc atc aga gca agc aac cct gat ctc cgg 2229
Pro Arg Leu Gly Ser Gln Pro Ile Arg Ala Ser Asn Pro Asp Leu Arg
        615                 620                 625

aga act gag ccc atc ttg gag agc ccc ttg cag agg acc agc agt ggc 2277
Arg Thr Glu Pro Ile Leu Glu Ser Pro Leu Gln Arg Thr Ser Ser Gly

630                 635                 640

agt tcc tcc agc tcc agc acc cct agc tcc cag ccc agc tcc caa gga      2325
Ser Ser Ser Ser Ser Ser Thr Pro Ser Ser Gln Pro Ser Ser Gln Gly
645                 650                 655                 660

ggc tcc cag cct gga tca caa gca gga tcc agt gaa cgc acc aga gtt      2373
Gly Ser Gln Pro Gly Ser Gln Ala Gly Ser Ser Glu Arg Thr Arg Val
                665                 670                 675

cga gcc aac agt aag tca gaa gga tca cct gtg ctc ccc cat gag cct      2421
Arg Ala Asn Ser Lys Ser Glu Gly Ser Pro Val Leu Pro His Glu Pro
            680                 685                 690

gcc aag gtg aaa cca gaa gaa tcc agg gac att acc cgg ccc agt cga      2469
Ala Lys Val Lys Pro Glu Glu Ser Arg Asp Ile Thr Arg Pro Ser Arg
        695                 700                 705

cca gct agc tac aaa aaa gct ata gat gag gat ctg acg gca tta gcc      2517
Pro Ala Ser Tyr Lys Lys Ala Ile Asp Glu Asp Leu Thr Ala Leu Ala
    710                 715                 720

aaa gaa cta aga gaa ctc cgg att gaa gaa aca aac cgc cca atg aag      2565
Lys Glu Leu Arg Glu Leu Arg Ile Glu Glu Thr Asn Arg Pro Met Lys
725                 730                 735                 740

aag gtg act gat tac tcc tcc tcc agt gag gag tca gaa agt agc gag      2613
Lys Val Thr Asp Tyr Ser Ser Ser Ser Glu Glu Ser Glu Ser Ser Glu
                745                 750                 755

gaa gag gag gaa gat gga gag agc gag acc cat gat ggg aca gtg gct      2661
Glu Glu Glu Glu Asp Gly Glu Ser Glu Thr His Asp Gly Thr Val Ala
            760                 765                 770

gtc agc gac ata ccc aga ctg ata cca aca gga gct cca ggc agc aac      2709
Val Ser Asp Ile Pro Arg Leu Ile Pro Thr Gly Ala Pro Gly Ser Asn
        775                 780                 785

gag cag tac aat gtg gga atg gtg ggg acg cat ggg ctg gag acc tct      2757
Glu Gln Tyr Asn Val Gly Met Val Gly Thr His Gly Leu Glu Thr Ser
    790                 795                 800

cat gcg gac agt ttc agc ggc agt att tca aga gaa gga acc ttg atg      2805
His Ala Asp Ser Phe Ser Gly Ser Ile Ser Arg Glu Gly Thr Leu Met
805                 810                 815                 820

att aga gag acg tct gga gag aag aag cga tct ggc cac agt gac agc      2853
Ile Arg Glu Thr Ser Gly Glu Lys Lys Arg Ser Gly His Ser Asp Ser
                825                 830                 835

aat ggc ttt gct ggc cac atc aac ctc cct gac ctg gtg cag cag agc      2901
Asn Gly Phe Ala Gly His Ile Asn Leu Pro Asp Leu Val Gln Gln Ser
            840                 845                 850

cat tct cca gct gga acc ccg act gag gga ctg ggg cgc gtc tca acc      2949
His Ser Pro Ala Gly Thr Pro Thr Glu Gly Leu Gly Arg Val Ser Thr
        855                 860                 865

cat tcc cag gag atg gac tct ggg act gaa tat ggc atg ggg agc agc      2997
His Ser Gln Glu Met Asp Ser Gly Thr Glu Tyr Gly Met Gly Ser Ser
    870                 875                 880

acc aaa gcc tcc ttc acc ccc ttt gtg gac ccc aga gta tac cag acg      3045

Thr Lys Ala Ser Phe Thr Pro Phe Val Asp Pro Arg Val Tyr Gln Thr
885                 890                 895                 900

tct ccc act gat gaa gat gaa gag gat gag gaa tca tca gcc gca gct      3093
Ser Pro Thr Asp Glu Asp Glu Glu Asp Glu Glu Ser Ser Ala Ala Ala
                905                 910                 915

ctg ttt act agc gaa ctt ctt agg caa gaa cag gcc aaa ctc aat gaa      3141
Leu Phe Thr Ser Glu Leu Leu Arg Gln Glu Gln Ala Lys Leu Asn Glu
            920                 925                 930

gca aga aag att tcg gtg gta aat gta aac cca acc aac att cgg cct      3189
Ala Arg Lys Ile Ser Val Val Asn Val Asn Pro Thr Asn Ile Arg Pro
        935                 940                 945

cat agc gac aca cca gaa atc aga aaa tac aag aaa cga ttc aac tca      3237
His Ser Asp Thr Pro Glu Ile Arg Lys Tyr Lys Lys Arg Phe Asn Ser
    950                 955                 960

gaa ata ctt tgt gca gct ctg tgg ggt gta aac ctt ctg gtg ggg act      3285
Glu Ile Leu Cys Ala Ala Leu Trp Gly Val Asn Leu Leu Val Gly Thr
965                 970                 975                 980

gaa aat ggc ctg atg ctt ttg gac cga agt ggg caa ggc aaa gtc tat      3333
Glu Asn Gly Leu Met Leu Leu Asp Arg Ser Gly Gln Gly Lys Val Tyr
                985                 990                 995

aat ctg atc aac  cgg agg cga ttt cag  cag atg gat gtg cta  gag      3378
Asn Leu Ile Asn  Arg Arg Arg Phe Gln  Gln Met Asp Val Leu  Glu
            1000                 1005                 1010

gga ctg aat gtc  ctt gtg aca att tca  gga aag aag aat aag  cta      3423
Gly Leu Asn Val  Leu Val Thr Ile Ser  Gly Lys Lys Asn Lys  Leu
            1015                 1020                 1025

cga gtt tac tat  ctt tca tgg tta aga  aac aga ata cta cat  aat      3468
Arg Val Tyr Tyr  Leu Ser Trp Leu Arg  Asn Arg Ile Leu His  Asn
            1030                 1035                 1040

gac cca gaa gta  gaa aag aaa caa ggc  tgg atc act gtt ggg  gac      3513
Asp Pro Glu Val  Glu Lys Lys Gln Gly  Trp Ile Thr Val Gly  Asp
            1045                 1050                 1055

ttg gaa ggc tgt  ata cat tat aaa gtt  gtt aaa tat gaa agg  atc      3558
Leu Glu Gly Cys  Ile His Tyr Lys Val  Val Lys Tyr Glu Arg  Ile
            1060                 1065                 1070

aaa ttt ttg gtg  att gcc tta aag aat  gct gtg gaa ata tat  gct      3603
Lys Phe Leu Val  Ile Ala Leu Lys Asn  Ala Val Glu Ile Tyr  Ala
            1075                 1080                 1085

tgg gct cct aaa  ccg tat cat aaa ttc  atg gca ttt aag tct  ttt      3648
Trp Ala Pro Lys  Pro Tyr His Lys Phe  Met Ala Phe Lys Ser  Phe
            1090                 1095                 1100

gca gat ctc cag  cac aag cct ctg cta  gtt gat ctc acg gta  gaa      3693
Ala Asp Leu Gln  His Lys Pro Leu Leu  Val Asp Leu Thr Val  Glu
            1105                 1110                 1115

gaa ggt caa aga  tta aag gtt att ttt  ggt tca cac act ggt  ttc      3738
Glu Gly Gln Arg  Leu Lys Val Ile Phe  Gly Ser His Thr Gly  Phe
            1120                 1125                 1130

cat gta att gat gtt gat tca gga aac tct tat gat atc tac ata 3783
His Val Ile Asp Val Asp Ser Gly Asn Ser Tyr Asp Ile Tyr Ile
            1135                1140                1145

cca tct cat att cag ggc aat atc act cct cat gct att gtc atc 3828
Pro Ser His Ile Gln Gly Asn Ile Thr Pro His Ala Ile Val Ile
            1150                1155                1160

ttg cct aaa aca gat gga atg gaa atg ctt gtt tgc tat gag gat 3873
Leu Pro Lys Thr Asp Gly Met Glu Met Leu Val Cys Tyr Glu Asp
            1165                1170                1175

gag ggg gtg tat gta aac acc tat ggc cgg ata act aag gat gtg 3918
Glu Gly Val Tyr Val Asn Thr Tyr Gly Arg Ile Thr Lys Asp Val
            1180                1185                1190

gtg ctc caa tgg gga gaa atg ccc acg tct gtg gcc tac att cat 3963
Val Leu Gln Trp Gly Glu Met Pro Thr Ser Val Ala Tyr Ile His
            1195                1200                1205

tcc aat cag ata atg ggc tgg ggc gag aaa gct att gag atc cgg 4008
Ser Asn Gln Ile Met Gly Trp Gly Glu Lys Ala Ile Glu Ile Arg
            1210                1215                1220

tca gtg gaa aca gga cat ttg gat gga gta ttt atg cat aag cga 4053
Ser Val Glu Thr Gly His Leu Asp Gly Val Phe Met His Lys Arg
            1225                1230                1235

gct caa agg tta aag ttt cta tgt gaa aga aat gat aag gta ttt 4098
Ala Gln Arg Leu Lys Phe Leu Cys Glu Arg Asn Asp Lys Val Phe
            1240                1245                1250

ttt gca tcc gtg cga tct gga gga agt agc caa gtg ttt ttc atg 4143
Phe Ala Ser Val Arg Ser Gly Gly Ser Ser Gln Val Phe Phe Met
            1255                1260                1265

acc ctc aac aga aat tcc atg atg aac tgg taa cagaagagca 4186
Thr Leu Asn Arg Asn Ser Met Met Asn Trp
            1270                1275

cttggcactt atcttcatgg cgttatttct aatttaaaag aacataactc atgtggactt 4246

atgccagtct agaggcagaa tcagaaggct tggttgaaca tatcgctttc cctttttcct 4306

ctccctccgc ccctcccagt acagtccatc tttcaatgtt gcagcctggt tgagaaggag 4366

agaaaaaggt ggcaggaatt tccaggagat ccccaagaat gctgccttgt ctgtggacaa 4426

agatggacca tgtgcccttc ggaattaggg atagaaacaa atattgtgtg ctcttaacga 4486

ttaagctgtg ttatggtggg ttttcaggtt tttacctttt ttctttaccc ctttactctg 4546

caagaatggg gaaagaatgc atactgcgaa aatgagtctt ttaaattctg tctgcctact 4606

agttttaagt atatggtatg ttgtaaaatt tccaatgatg agagacagca caataaatgt 4666

accttatctc cttaggctga aggccataac tacatagtgg agtaatttaa gaactctctt 4726

gccttcacca acccaaaagg ttgctttttg atagcaactg gctaatgaat ttttaaaaag 4786

agaagaaaaa tactagtttt cccctctttt gggaaataga ttttaaatgg ctaaactact 4846

agccttaaaa ctactagtct aataaaatca actaccactt ttgtgaatct gacaggccac 4906

atttttatat ggccctttac agaatggagt gtgttgaaca ggatactaac gccattgagt 4966

tgagctggcc tagcgatgga gggacactct aacacaactt tccctcagct attatgcaac 5026

agatcaggga aaaagatggg atgacagatg gggtcagaca gaaagagctt ctgggaaaca 5086

agcttacata gtctttttta aaatgcacaa agcctcccag ctaagaggtc acttggtttg 5146

ggcttcatta ggactgagac tttgttgagt tctttctggg acttggagag tggatgatat 5206

tcaggctctg aacattccca gcgctctccc gagggtgcca ctttctcaag atgaaaactg 5266

tgactgaaaa aattaataat aaatgtttct gagctgcctg tgttctccct gtgtgggtga 5326

gagaagggac tagactccta agcctgcctc agatacaaga ggcatcattg gctccaattt 5386

tagagaactt gaaagcaagg ctttggacaa aattttgaga ccctaatcac tttaccttcc 5446

tccaaattac ccaacatacg gtaaacaaca tttgtgcaga agtatgtatg tatttagttc 5506

aggttgactt gtgtccttat aaactcttac tcaaatgatt tgaactttaa aaaaaaa 5564

<210> 46
<211> 1276
<212> PRT
<213> Homo sapiens

<400> 46
   Met Ala Ser Asp Ser Pro Ala Arg Ser Leu Asp Glu Ile Asp Leu Ser
   1 5 10 15
   Ala Leu Arg Asp Pro Ala Gly Ile Phe Glu Leu Val Glu Leu Val Gly
   20 25 30
   Asn Gly Thr Tyr Gly Gln Val Tyr Lys Gly Arg His Val Lys Thr Gly
   35 40 45
   Gln Leu Ala Ala Ile Lys Val Met Asp Val Thr Gly Asp Glu Glu Glu
   50 55 60
   Glu Ile Lys Gln Glu Ile Asn Met Leu Lys Lys Tyr Ser His His Arg
   65 70 75 80
   Asn Ile Ala Thr Tyr Tyr Gly Ala Phe Ile Lys Lys Asn Pro Pro Gly
   85 90 95
   Met Asp Asp Gln Leu Trp Leu Val Met Glu Phe Cys Gly Ala Gly Ser
   100 105 110
   Val Thr Asp Leu Ile Lys Asn Thr Lys Gly Asn Thr Leu Lys Glu Glu
   115 120 125
   Trp Ile Ala Tyr Ile Cys Arg Glu Ile Leu Arg Gly Leu Ser His Leu
       130                 135                 140
   His Gln His Lys Val Ile His Arg Asp Ile Lys Gly Gln Asn Val Leu
   145                 150                 155                 160
   Leu Thr Glu Asn Ala Glu Val Lys Leu Val Asp Phe Gly Val Ser Ala
                   165                 170                 175
   Gln Leu Asp Arg Thr Val Gly Arg Arg Asn Thr Phe Ile Gly Thr Pro
               180                 185                 190
   Tyr Trp Met Ala Pro Glu Val Ile Ala Cys Asp Glu Asn Pro Asp Ala
           195                 200                 205
   Thr Tyr Asp Phe Lys Ser Asp Leu Trp Ser Leu Gly Ile Thr Ala Ile
       210                 215                 220
   Glu Met Ala Glu Gly Ala Pro Pro Leu Cys Asp Met His Pro Met Arg
   225                 230                 235                 240
   Ala Leu Phe Leu Ile Pro Arg Asn Pro Ala Pro Arg Leu Lys Ser Lys
                   245                 250                 255
   Lys Trp Ser Lys Lys Phe Gln Ser Phe Ile Glu Ser Cys Leu Val Lys
               260                 265                 270
   Asn His Ser Gln Arg Pro Ala Thr Glu Gln Leu Met Lys His Pro Phe
           275                 280                 285
   Ile Arg Asp Gln Pro Asn Glu Arg Gln Val Arg Ile Gln Leu Lys Asp
       290                 295                 300
   His Ile Asp Arg Thr Lys Lys Lys Arg Gly Glu Lys Asp Glu Thr Glu
   305                 310                 315                 320
   Tyr Glu Tyr Ser Gly Ser Glu Glu Glu Glu Glu Glu Asn Asp Ser Gly
                   325                 330                 335
   Glu Pro Ser Ser Ile Leu Asn Leu Pro Gly Glu Ser Thr Leu Arg Arg
               340                 345                 350
   Asp Phe Leu Arg Leu Gln Leu Ala Asn Lys Glu Arg Ser Glu Ala Leu
           355                 360                 365
   Arg Arg Gln Gln Leu Glu Gln Gln Gln Arg Glu Asn Glu Glu His Lys
       370                 375                 380
   Arg Gln Leu Leu Ala Glu Arg Gln Lys Arg Ile Glu Glu Gln Lys Glu
   385                 390                 395                 400
   Gln Arg Arg Arg Leu Glu Glu Gln Gln Arg Arg Glu Lys Glu Leu Arg
                   405                 410                 415
   Lys Gln Gln Glu Arg Glu Gln Arg Arg His Tyr Glu Glu Gln Met Arg
               420                 425                 430
   Arg Glu Glu Glu Arg Arg Arg Ala Glu His Glu Gln Glu Tyr Lys Arg
           435                 440                 445
   Lys Gln Leu Glu Glu Gln Arg Gln Ala Glu Arg Leu Gln Arg Gln Leu
       450                 455                 460
   Lys Gln Glu Arg Asp Tyr Leu Val Ser Leu Gln His Gln Arg Gln Glu
   465                 470                 475                 480
   Gln Arg Pro Val Glu Lys Lys Pro Leu Tyr His Tyr Lys Glu Gly Met
                   485                 490                 495
   Ser Pro Ser Glu Lys Pro Ala Trp Ala Lys Glu Ile Pro His Leu Val
               500                 505                 510
   Ala Val Lys Ser Gln Gly Pro Ala Leu Thr Ala Ser Gln Ser Val His
           515                 520                 525
   Glu Gln Pro Thr Lys Gly Leu Ser Gly Phe Gln Glu Ala Leu Asn Val
       530                 535                 540
   Thr Ser His Arg Val Glu Met Pro Arg Gln Asn Ser Asp Pro Thr Ser
   545                 550                 555                 560
   Glu Asn Pro Pro Leu Pro Thr Arg Ile Glu Lys Phe Asp Arg Ser Ser
                   565                 570                 575
   Trp Leu Arg Gln Glu Glu Asp Ile Pro Pro Lys Val Pro Gln Arg Thr
               580                 585                 590
   Thr Ser Ile Ser Pro Ala Leu Ala Arg Lys Asn Ser Pro Gly Asn Gly
           595                 600                 605
   Ser Ala Leu Gly Pro Arg Leu Gly Ser Gln Pro Ile Arg Ala Ser Asn
       610                 615                 620
   Pro Asp Leu Arg Arg Thr Glu Pro Ile Leu Glu Ser Pro Leu Gln Arg
   625                 630                 635                 640
   Thr Ser Ser Gly Ser Ser Ser Ser Ser Ser Thr Pro Ser Ser Gln Pro
                   645                 650                 655
   Ser Ser Gln Gly Gly Ser Gln Pro Gly Ser Gln Ala Gly Ser Ser Glu
               660                 665                 670
   Arg Thr Arg Val Arg Ala Asn Ser Lys Ser Glu Gly Ser Pro Val Leu
           675                 680                 685
   Pro His Glu Pro Ala Lys Val Lys Pro Glu Glu Ser Arg Asp Ile Thr
       690                 695                 700
   Arg Pro Ser Arg Pro Ala Ser Tyr Lys Lys Ala Ile Asp Glu Asp Leu
   705                 710                 715                 720
   Thr Ala Leu Ala Lys Glu Leu Arg Glu Leu Arg Ile Glu Glu Thr Asn
                   725                 730                 735
   Arg Pro Met Lys Lys Val Thr Asp Tyr Ser Ser Ser Ser Glu Glu Ser
               740                 745                 750
   Glu Ser Ser Glu Glu Glu Glu Glu Asp Gly Glu Ser Glu Thr His Asp
           755                 760                 765
   Gly Thr Val Ala Val Ser Asp Ile Pro Arg Leu Ile Pro Thr Gly Ala
       770                 775                 780
   Pro Gly Ser Asn Glu Gln Tyr Asn Val Gly Met Val Gly Thr His Gly
   785                 790                 795                 800
   Leu Glu Thr Ser His Ala Asp Ser Phe Ser Gly Ser Ile Ser Arg Glu
                   805                 810                 815
   Gly Thr Leu Met Ile Arg Glu Thr Ser Gly Glu Lys Lys Arg Ser Gly
               820                 825                 830
   His Ser Asp Ser Asn Gly Phe Ala Gly His Ile Asn Leu Pro Asp Leu
           835                 840                 845
   Val Gln Gln Ser His Ser Pro Ala Gly Thr Pro Thr Glu Gly Leu Gly
       850                 855                 860
   Arg Val Ser Thr His Ser Gln Glu Met Asp Ser Gly Thr Glu Tyr Gly
   865                 870                 875                 880
   Met Gly Ser Ser Thr Lys Ala Ser Phe Thr Pro Phe Val Asp Pro Arg
   885 890 895
   Val Tyr Gln Thr Ser Pro Thr Asp Glu Asp Glu Glu Asp Glu Glu Ser
   900 905 910
   Ser Ala Ala Ala Leu Phe Thr Ser Glu Leu Leu Arg Gln Glu Gln Ala
   915 920 925
   Lys Leu Asn Glu Ala Arg Lys Ile Ser Val Val Asn Val Asn Pro Thr
   930 935 940
   Asn Ile Arg Pro His Ser Asp Thr Pro Glu Ile Arg Lys Tyr Lys Lys
   945 950 955 960
   Arg Phe Asn Ser Glu Ile Leu Cys Ala Ala Leu Trp Gly Val Asn Leu
   965 970 975
   Leu Val Gly Thr Glu Asn Gly Leu Met Leu Leu Asp Arg Ser Gly Gln
   980 985 990
   Gly Lys Val Tyr Asn Leu Ile Asn Arg Arg Arg Phe Gln Gln Met Asp
   995 1000 1005
   Val Leu Glu Gly Leu Asn Val Leu Val Thr Ile Ser Gly Lys Lys
   1010 1015 1020
   Asn Lys Leu Arg Val Tyr Tyr Leu Ser Trp Leu Arg Asn Arg Ile
   1025 1030 1035
   Leu His Asn Asp Pro Glu Val Glu Lys Lys Gln Gly Trp Ile Thr
   1040 1045 1050
   Val Gly Asp Leu Glu Gly Cys Ile His Tyr Lys Val Val Lys Tyr
   1055 1060 1065
   Glu Arg Ile Lys Phe Leu Val Ile Ala Leu Lys Asn Ala Val Glu
   1070 1075 1080
   Ile Tyr Ala Trp Ala Pro Lys Pro Tyr His Lys Phe Met Ala Phe
   1085 1090 1095
   Lys Ser Phe Ala Asp Leu Gln His Lys Pro Leu Leu Val Asp Leu
   1100 1105 1110
   Thr Val Glu Glu Gly Gln Arg Leu Lys Val Ile Phe Gly Ser His
   1115 1120 1125
   Thr Gly Phe His Val Ile Asp Val Asp Ser Gly Asn Ser Tyr Asp
       1130                1135                1140
   Ile Tyr Ile Pro Ser His Ile Gln Gly Asn Ile Thr Pro His Ala
       1145                1150                1155
   Ile Val Ile Leu Pro Lys Thr Asp Gly Met Glu Met Leu Val Cys
       1160                1165                1170
   Tyr Glu Asp Glu Gly Val Tyr Val Asn Thr Tyr Gly Arg Ile Thr
       1175                1180                1185
   Lys Asp Val Val Leu Gln Trp Gly Glu Met Pro Thr Ser Val Ala
       1190                1195                1200
   Tyr Ile His Ser Asn Gln Ile Met Gly Trp Gly Glu Lys Ala Ile
       1205                1210                1215
   Glu Ile Arg Ser Val Glu Thr Gly His Leu Asp Gly Val Phe Met
       1220                1225                1230
   His Lys Arg Ala Gln Arg Leu Lys Phe Leu Cys Glu Arg Asn Asp
       1235                1240                1245
   Lys Val Phe Phe Ala Ser Val Arg Ser Gly Gly Ser Ser Gln Val
       1250                1255                1260
   Phe Phe Met Thr Leu Asn Arg Asn Ser Met Met Asn Trp
       1265                1270                1275

<210>  47
<211>  5540
<212>  DNA
<213>  Homo sapiens

<220>
<221>  CDS
<222>  (346)..(4152)

<400>  47
attataacag ccaatgcagc cgccatccac gcacaagcaa agaagcatgt cccatttaaa      60

tacacccacg cagccccagc gcccttagcc gatcggggcg ctcagcccac acgcaccgct     120

gctcggggct tggagatccg cgcaggctgg gctcccggac ccggcggacc gacgcgcgga     180

ggatcgggat ccggcgctgt ggggctgggg tggcgggggg aggctgggcc cggggcctct     240

ggcgcgacac ccgcatgagg acgcgagtga aatagaccaa ggtggaattt ccaagggaaa      300

agcttcgggg tggttttggt ccatttctcc agcgaagaag tagac atg gcg agc gac     357
                                                  Met Ala Ser Asp

                                                  1

tcc ccg gct cga agc ctg gat gaa ata gat ctc tcg gct ctg agg gac 405
Ser Pro Ala Arg Ser Leu Asp Glu Ile Asp Leu Ser Ala Leu Arg Asp
5                   10                  15                  20

ccc gca ggg atc ttt gaa ttg gtg gaa ctt gtt gga aat gga aca tac 453
Pro Ala Gly Ile Phe Glu Leu Val Glu Leu Val Gly Asn Gly Thr Tyr
                25                  30                  35

ggg caa gtt tat aag ggt cgt cat gtc aaa acg ggc cag ctt gca gcc 501
Gly Gln Val Tyr Lys Gly Arg His Val Lys Thr Gly Gln Leu Ala Ala
            40                  45                  50

atc aag gtt atg gat gtc aca ggg gat gaa gag gaa gaa atc aaa caa 549
Ile Lys Val Met Asp Val Thr Gly Asp Glu Glu Glu Glu Ile Lys Gln
        55                  60                  65

gaa att aac atg ttg aag aaa tat tct cat cac cgg aat att gct aca 597
Glu Ile Asn Met Leu Lys Lys Tyr Ser His His Arg Asn Ile Ala Thr
    70                  75                  80

tac tat ggt gct ttt atc aaa aag aac cca cca ggc atg gat gac caa 645
Tyr Tyr Gly Ala Phe Ile Lys Lys Asn Pro Pro Gly Met Asp Asp Gln
85                  90                  95                  100

ctt tgg ttg gtg atg gag ttt tgt ggt gct ggc tct gtc acc gac ctg 693
Leu Trp Leu Val Met Glu Phe Cys Gly Ala Gly Ser Val Thr Asp Leu
                105                 110                 115

atc aag aac aca aaa ggt aac acg ttg aaa gag gag tgg att gca tac 741
Ile Lys Asn Thr Lys Gly Asn Thr Leu Lys Glu Glu Trp Ile Ala Tyr
            120                 125                 130

atc tgc agg gaa atc tta cgg ggg ctg agt cac ctg cac cag cat aaa 789
Ile Cys Arg Glu Ile Leu Arg Gly Leu Ser His Leu His Gln His Lys
        135                 140                 145

gtg att cat cga gat att aaa ggg caa aat gtc ttg ctg act gaa aat 837
Val Ile His Arg Asp Ile Lys Gly Gln Asn Val Leu Leu Thr Glu Asn
    150                 155                 160

gca gaa gtt aaa cta gtg gac ttt gga gtc agt gct cag ctt gat cga 885
Ala Glu Val Lys Leu Val Asp Phe Gly Val Ser Ala Gln Leu Asp Arg
165                 170                 175                 180

aca gtg ggc agg agg aat act ttc att gga act ccc tac tgg atg gca 933
Thr Val Gly Arg Arg Asn Thr Phe Ile Gly Thr Pro Tyr Trp Met Ala
                185                 190                 195

cca gaa gtt att gcc tgt gat gaa aac cca gat gcc aca tat gat ttc 981
Pro Glu Val Ile Ala Cys Asp Glu Asn Pro Asp Ala Thr Tyr Asp Phe
            200                 205                 210

aag agt gac ttg tgg tct ttg ggt atc acc gcc att gaa atg gca gaa 1029
Lys Ser Asp Leu Trp Ser Leu Gly Ile Thr Ala Ile Glu Met Ala Glu
        215                 220                 225

ggt gct ccc cct ctc tgt gac atg cac ccc atg aga gct ctc ttc ctc 1077
Gly Ala Pro Pro Leu Cys Asp Met His Pro Met Arg Ala Leu Phe Leu
    230                 235                 240

atc ccc cgg aac cca gcg cct cgg ctg aag tct aag aag tgg tca aaa 1125

Ile Pro Arg Asn Pro Ala Pro Arg Leu Lys Ser Lys Lys Trp Ser Lys
245                 250                 255                 260

aaa ttc cag tca ttt att gag agc tgc ttg gta aag aat cac agc cag      1173
Lys Phe Gln Ser Phe Ile Glu Ser Cys Leu Val Lys Asn His Ser Gln
                265                 270                 275

cga cca gca aca gaa caa ttg atg aag cat cca ttt ata cga gac caa      1221
Arg Pro Ala Thr Glu Gln Leu Met Lys His Pro Phe Ile Arg Asp Gln
            280                 285                 290

cct aat gag cga cag gtc cgc att caa ctc aag gac cat att gat aga      1269
Pro Asn Glu Arg Gln Val Arg Ile Gln Leu Lys Asp His Ile Asp Arg
        295                 300                 305

aca aag aag aag cga gga gaa aaa gat gag aca gag tat gag tac agt      1317
Thr Lys Lys Lys Arg Gly Glu Lys Asp Glu Thr Glu Tyr Glu Tyr Ser
    310                 315                 320

gga agt gag gaa gaa gag gag gag aat gac tca gga gag ccc agc tcc      1365
Gly Ser Glu Glu Glu Glu Glu Glu Asn Asp Ser Gly Glu Pro Ser Ser
325                 330                 335                 340

atc ctg aat ctg cca ggg gag tcg acg ctg cgg agg gac ttt ctg agg      1413
Ile Leu Asn Leu Pro Gly Glu Ser Thr Leu Arg Arg Asp Phe Leu Arg
                345                 350                 355

ctg cag ctg gcc aac aag gag cgt tct gag gcc cta cgg agg cag cag      1461
Leu Gln Leu Ala Asn Lys Glu Arg Ser Glu Ala Leu Arg Arg Gln Gln
            360                 365                 370

ctg gag cag cag cag cgg gag aat gag gag cac aag cgg cag ctg ctg      1509
Leu Glu Gln Gln Gln Arg Glu Asn Glu Glu His Lys Arg Gln Leu Leu
        375                 380                 385

gcc gag cgt cag aag cgc atc gag gag cag aaa gag cag agg cgg cgg      1557
Ala Glu Arg Gln Lys Arg Ile Glu Glu Gln Lys Glu Gln Arg Arg Arg
    390                 395                 400

ctg gag gag caa caa agg cga gag aag gag ctg cgg aag cag cag gag      1605
Leu Glu Glu Gln Gln Arg Arg Glu Lys Glu Leu Arg Lys Gln Gln Glu
405                 410                 415                 420

agg gag cag cgc cgg cac tat gag gag cag atg cgc cgg gag gag gag      1653
Arg Glu Gln Arg Arg His Tyr Glu Glu Gln Met Arg Arg Glu Glu Glu
                425                 430                 435

agg agg cgt gcg gag cat gaa cag gaa tat aag cgc aaa caa ttg gaa      1701
Arg Arg Arg Ala Glu His Glu Gln Glu Tyr Lys Arg Lys Gln Leu Glu
            440                 445                 450

gaa cag aga caa gca gaa aga ctg cag agg cag cta aag caa gaa aga      1749
Glu Gln Arg Gln Ala Glu Arg Leu Gln Arg Gln Leu Lys Gln Glu Arg
        455                 460                 465

gac tac tta gtt tcc ctt cag cat cag cgg cag gag cag agg cct gtg      1797
Asp Tyr Leu Val Ser Leu Gln His Gln Arg Gln Glu Gln Arg Pro Val
    470                 475                 480

gag aag aag cca ctg tac cat tac aaa gaa gga atg agt cct agt gag      1845
Glu Lys Lys Pro Leu Tyr His Tyr Lys Glu Gly Met Ser Pro Ser Glu
485                 490                 495                 500

aag cca gca tgg gcc aag gag atc cca cat ctg gta gct gta aaa tcc 1893
Lys Pro Ala Trp Ala Lys Glu Ile Pro His Leu Val Ala Val Lys Ser
                505                 510                 515

cag gga cct gcc ttg acc gcc tcc cag tca gtg cac gag cag ccc aca 1941
Gln Gly Pro Ala Leu Thr Ala Ser Gln Ser Val His Glu Gln Pro Thr
            520                 525                 530

aag ggc ctc tct ggg ttt cag gag gct ctg aac gtg acc tcc cac cgc 1989
Lys Gly Leu Ser Gly Phe Gln Glu Ala Leu Asn Val Thr Ser His Arg
        535                 540                 545

gtg gag atg cca cgc cag aac tca gat ccc acc tcg gaa aat cct cct 2037
Val Glu Met Pro Arg Gln Asn Ser Asp Pro Thr Ser Glu Asn Pro Pro
    550                 555                 560

ctc ccc act cgc att gaa aag ttt gac cga agc tct tgg tta cga cag 2085
Leu Pro Thr Arg Ile Glu Lys Phe Asp Arg Ser Ser Trp Leu Arg Gln
565                 570                 575                 580

gaa gaa gac att cca cca aag gtg cct caa aga aca act tct ata tcc 2133
Glu Glu Asp Ile Pro Pro Lys Val Pro Gln Arg Thr Thr Ser Ile Ser
                585                 590                 595

cca gca tta gcc aga aag aat tct cct ggg aat ggt agt gct ctg gga 2181
Pro Ala Leu Ala Arg Lys Asn Ser Pro Gly Asn Gly Ser Ala Leu Gly
            600                 605                 610

ccc aga cta gga tct caa ccc atc aga gca agc aac cct gat ctc cgg 2229
Pro Arg Leu Gly Ser Gln Pro Ile Arg Ala Ser Asn Pro Asp Leu Arg
        615                 620                 625

aga act gag ccc atc ttg gag agc ccc ttg cag agg acc agc agt ggc 2277
Arg Thr Glu Pro Ile Leu Glu Ser Pro Leu Gln Arg Thr Ser Ser Gly
    630                 635                 640

agt tcc tcc agc tcc agc acc cct agc tcc cag ccc agc tcc caa gga 2325
Ser Ser Ser Ser Ser Ser Thr Pro Ser Ser Gln Pro Ser Ser Gln Gly
645                 650                 655                 660

ggc tcc cag cct gga tca caa gca gga tcc agt gaa cgc acc aga gtt 2373
Gly Ser Gln Pro Gly Ser Gln Ala Gly Ser Ser Glu Arg Thr Arg Val
                665                 670                 675

cga gcc aac agt aag tca gaa gga tca cct gtg ctc ccc cat gag cct 2421
Arg Ala Asn Ser Lys Ser Glu Gly Ser Pro Val Leu Pro His Glu Pro
            680                 685                 690

gcc aag gtg aaa cca gaa gaa tcc agg gac att acc cgg ccc agt cga 2469
Ala Lys Val Lys Pro Glu Glu Ser Arg Asp Ile Thr Arg Pro Ser Arg
        695                 700                 705

cca gct gat ctg acg gca tta gcc aaa gaa cta aga gaa ctc cgg att 2517
Pro Ala Asp Leu Thr Ala Leu Ala Lys Glu Leu Arg Glu Leu Arg Ile
    710                 715                 720

gaa gaa aca aac cgc cca atg aag aag gtg act gat tac tcc tcc tcc 2565
Glu Glu Thr Asn Arg Pro Met Lys Lys Val Thr Asp Tyr Ser Ser Ser
725                 730                 735                 740

agt gag gag tca gaa agt agc gag gaa gag gag gaa gat gga gag agc 2613
Ser Glu Glu Ser Glu Ser Ser Glu Glu Glu Glu Glu Asp Gly Glu Ser
                745                 750                 755

gag acc cat gat ggg aca gtg gct gtc agc gac ata ccc aga ctg ata 2661
Glu Thr His Asp Gly Thr Val Ala Val Ser Asp Ile Pro Arg Leu Ile
            760                 765                 770

cca aca gga gct cca ggc agc aac gag cag tac aat gtg gga atg gtg 2709
Pro Thr Gly Ala Pro Gly Ser Asn Glu Gln Tyr Asn Val Gly Met Val
        775                 780                 785

ggg acg cat ggg ctg gag acc tct cat gcg gac agt ttc agc ggc agt 2757
Gly Thr His Gly Leu Glu Thr Ser His Ala Asp Ser Phe Ser Gly Ser
    790                 795                 800

att tca aga gaa gga acc ttg atg att aga gag acg tct gga gag aag 2805
Ile Ser Arg Glu Gly Thr Leu Met Ile Arg Glu Thr Ser Gly Glu Lys
805                 810                 815                 820

aag cga tct ggc cac agt gac agc aat ggc ttt gct ggc cac atc aac 2853
Lys Arg Ser Gly His Ser Asp Ser Asn Gly Phe Ala Gly His Ile Asn
                825                 830                 835

ctc cct gac ctg gtg cag cag agc cat tct cca gct gga acc ccg act 2901
Leu Pro Asp Leu Val Gln Gln Ser His Ser Pro Ala Gly Thr Pro Thr
            840                 845                 850

gag gga ctg ggg cgc gtc tca acc cat tcc cag gag atg gac tct ggg 2949
Glu Gly Leu Gly Arg Val Ser Thr His Ser Gln Glu Met Asp Ser Gly
        855                 860                 865

act gaa tat ggc atg ggg agc agc acc aaa gcc tcc ttc acc ccc ttt 2997
Thr Glu Tyr Gly Met Gly Ser Ser Thr Lys Ala Ser Phe Thr Pro Phe
    870                 875                 880

gtg gac ccc aga gta tac cag acg tct ccc act gat gaa gat gaa gag 3045
Val Asp Pro Arg Val Tyr Gln Thr Ser Pro Thr Asp Glu Asp Glu Glu
885                 890                 895                 900

gat gag gaa tca tca gcc gca gct ctg ttt act agc gaa ctt ctt agg 3093
Asp Glu Glu Ser Ser Ala Ala Ala Leu Phe Thr Ser Glu Leu Leu Arg
                905                 910                 915

caa gaa cag gcc aaa ctc aat gaa gca aga aag att tcg gtg gta aat 3141
Gln Glu Gln Ala Lys Leu Asn Glu Ala Arg Lys Ile Ser Val Val Asn
            920                 925                 930

gta aac cca acc aac att cgg cct cat agc gac aca cca gaa atc aga 3189
Val Asn Pro Thr Asn Ile Arg Pro His Ser Asp Thr Pro Glu Ile Arg
        935                 940                 945

aaa tac aag aaa cga ttc aac tca gaa ata ctt tgt gca gct ctg tgg 3237
Lys Tyr Lys Lys Arg Phe Asn Ser Glu Ile Leu Cys Ala Ala Leu Trp
    950                 955                 960

ggt gta aac ctt ctg gtg ggg act gaa aat ggc ctg atg ctt ttg gac 3285
Gly Val Asn Leu Leu Val Gly Thr Glu Asn Gly Leu Met Leu Leu Asp
965                 970                 975                 980

cga agt ggg caa ggc aaa gtc tat aat ctg atc aac cgg agg cga ttt 3333
Arg Ser Gly Gln Gly Lys Val Tyr Asn Leu Ile Asn Arg Arg Arg Phe
                985                 990                 995

cag cag atg gat gtg cta gag gga ctg aat gtc ctt gtg aca att 3378
Gln Gln Met Asp Val Leu Glu Gly Leu Asn Val Leu Val Thr Ile

1000                1005                1010

tca gga aag aag aat aag cta cga gtt tac tat ctt tca tgg tta      3423
Ser Gly Lys Lys Asn Lys Leu Arg Val Tyr Tyr Leu Ser Trp Leu
        1015                1020                1025

aga aac aga ata cta cat aat gac cca gaa gta gaa aag aaa caa      3468
Arg Asn Arg Ile Leu His Asn Asp Pro Glu Val Glu Lys Lys Gln
        1030                1035                1040

ggc tgg atc act gtt ggg gac ttg gaa ggc tgt ata cat tat aaa      3513
Gly Trp Ile Thr Val Gly Asp Leu Glu Gly Cys Ile His Tyr Lys
        1045                1050                1055

gtt gtt aaa tat gaa agg atc aaa ttt ttg gtg att gcc tta aag      3558
Val Val Lys Tyr Glu Arg Ile Lys Phe Leu Val Ile Ala Leu Lys
        1060                1065                1070

aat gct gtg gaa ata tat gct tgg gct cct aaa ccg tat cat aaa      3603
Asn Ala Val Glu Ile Tyr Ala Trp Ala Pro Lys Pro Tyr His Lys
        1075                1080                1085

ttc atg gca ttt aag tct ttt gca gat ctc cag cac aag cct ctg      3648
Phe Met Ala Phe Lys Ser Phe Ala Asp Leu Gln His Lys Pro Leu
        1090                1095                1100

cta gtt gat ctc acg gta gaa gaa ggt caa aga tta aag gtt att      3693
Leu Val Asp Leu Thr Val Glu Glu Gly Gln Arg Leu Lys Val Ile
        1105                1110                1115

ttt ggt tca cac act ggt ttc cat gta att gat gtt gat tca gga      3738
Phe Gly Ser His Thr Gly Phe His Val Ile Asp Val Asp Ser Gly
        1120                1125                1130

aac tct tat gat atc tac ata cca tct cat att cag ggc aat atc      3783
Asn Ser Tyr Asp Ile Tyr Ile Pro Ser His Ile Gln Gly Asn Ile
        1135                1140                1145

act cct cat gct att gtc atc ttg cct aaa aca gat gga atg gaa      3828
Thr Pro His Ala Ile Val Ile Leu Pro Lys Thr Asp Gly Met Glu
        1150                1155                1160

atg ctt gtt tgc tat gag gat gag ggg gtg tat gta aac acc tat      3873
Met Leu Val Cys Tyr Glu Asp Glu Gly Val Tyr Val Asn Thr Tyr
        1165                1170                1175

ggc cgg ata act aag gat gtg gtg ctc caa tgg gga gaa atg ccc      3918
Gly Arg Ile Thr Lys Asp Val Val Leu Gln Trp Gly Glu Met Pro
        1180                1185                1190

acg tct gtg gcc tac att cat tcc aat cag ata atg ggc tgg ggc      3963
Thr Ser Val Ala Tyr Ile His Ser Asn Gln Ile Met Gly Trp Gly
        1195                1200                1205

gag aaa gct att gag atc cgg tca gtg gaa aca gga cat ttg gat      4008
Glu Lys Ala Ile Glu Ile Arg Ser Val Glu Thr Gly His Leu Asp
        1210                1215                1220

gga gta ttt atg cat aag cga gct caa agg tta aag ttt cta tgt      4053
Gly Val Phe Met His Lys Arg Ala Gln Arg Leu Lys Phe Leu Cys
        1225                1230                1235

gaa aga aat gat aag gta ttt ttt gca tcc gtg cga tct gga gga      4098

Glu Arg Asn Asp Lys Val Phe Phe Ala Ser Val Arg Ser Gly Gly
            1240                1245                1250

agt agc caa gtg ttt ttc atg acc ctc aac aga aat tcc atg atg      4143
Ser Ser Gln Val Phe Phe Met Thr Leu Asn Arg Asn Ser Met Met
            1255                1260                1265

aac tgg taa cagaagagca cttggcactt atcttcatgg cgttatttct      4192
Asn Trp

aatttaaaag aacataactc atgtggactt atgccagtct agaggcagaa tcagaaggct      4252

tggttgaaca tatcgctttc cctttttcct ctccctccgc ccctcccagt acagtccatc      4312

tttcaatgtt gcagcctggt tgagaaggag agaaaaaggt ggcaggaatt tccaggagat      4372

ccccaagaat gctgccttgt ctgtggacaa agatggacca tgtgcccttc ggaattaggg      4432

atagaaacaa atattgtgtg ctcttaacga ttaagctgtg ttatggtggg ttttcaggtt      4492

tttacctttt ttctttaccc ctttactctg caagaatggg gaaagaatgc atactgcgaa      4552

aatgagtctt ttaaattctg tctgcctact agttttaagt atatggtatg ttgtaaaatt      4612

tccaatgatg agagacagca caataaatgt accttatctc cttaggctga aggccataac      4672

tacatagtgg agtaatttaa gaactctctt gccttcacca acccaaaagg ttgctttttg      4732

atagcaactg gctaatgaat ttttaaaaag agaagaaaaa tactagtttt cccctctttt      4792

gggaaataga ttttaaatgg ctaaactact agccttaaaa ctactagtct aataaaatca      4852

actaccactt ttgtgaatct gacaggccac atttttatat ggccctttac agaatggagt      4912

gtgttgaaca ggatactaac gccattgagt tgagctggcc tagcgatgga gggacactct      4972

aacacaactt tccctcagct attatgcaac agatcaggga aaaagatggg atgacagatg      5032

gggtcagaca gaaagagctt ctgggaaaca agcttacata gtctttttta aaatgcacaa      5092

agcctcccag ctaagaggtc acttggtttg ggcttcatta ggactgagac tttgttgagt      5152

tctttctggg acttggagag tggatgatat tcaggctctg aacattccca gcgctctccc      5212

gagggtgcca ctttctcaag atgaaaactg tgactgaaaa aattaataat aaatgtttct      5272

gagctgcctg tgttctccct gtgtgggtga gagaagggac tagactccta agcctgcctc      5332

agatacaaga ggcatcattg gctccaattt tagagaactt gaaagcaagg ctttggacaa      5392

aattttgaga ccctaatcac tttaccttcc tccaaattac ccaacatacg gtaaacaaca      5452

tttgtgcaga agtatgtatg tatttagttc aggttgactt gtgtccttat aaactcttac      5512

tcaaatgatt tgaactttaa aaaaaaaa                                    5540

<210>  48
<211>  1268
<212>  PRT
<213>  Homo sapiens

<400> 48

Met Ala Ser Asp Ser Pro Ala Arg Ser Leu Asp Glu Ile Asp Leu Ser
1               5                   10                  15

Ala Leu Arg Asp Pro Ala Gly Ile Phe Glu Leu Val Glu Leu Val Gly
            20                  25                  30

Asn Gly Thr Tyr Gly Gln Val Tyr Lys Gly Arg His Val Lys Thr Gly
        35                  40                  45

Gln Leu Ala Ala Ile Lys Val Met Asp Val Thr Gly Asp Glu Glu Glu
    50                  55                  60

Glu Ile Lys Gln Glu Ile Asn Met Leu Lys Lys Tyr Ser His His Arg
65                  70                  75                  80

Asn Ile Ala Thr Tyr Tyr Gly Ala Phe Ile Lys Lys Asn Pro Pro Gly
                85                  90                  95

Met Asp Asp Gln Leu Trp Leu Val Met Glu Phe Cys Gly Ala Gly Ser
            100                 105                 110

Val Thr Asp Leu Ile Lys Asn Thr Lys Gly Asn Thr Leu Lys Glu Glu
        115                 120                 125

Trp Ile Ala Tyr Ile Cys Arg Glu Ile Leu Arg Gly Leu Ser His Leu
    130                 135                 140

His Gln His Lys Val Ile His Arg Asp Ile Lys Gly Gln Asn Val Leu
145                 150                 155                 160

Leu Thr Glu Asn Ala Glu Val Lys Leu Val Asp Phe Gly Val Ser Ala
                165                 170                 175

Gln Leu Asp Arg Thr Val Gly Arg Arg Asn Thr Phe Ile Gly Thr Pro
            180                 185                 190

Tyr Trp Met Ala Pro Glu Val Ile Ala Cys Asp Glu Asn Pro Asp Ala
        195                 200                 205

Thr Tyr Asp Phe Lys Ser Asp Leu Trp Ser Leu Gly Ile Thr Ala Ile
    210                 215                 220

Glu Met Ala Glu Gly Ala Pro Pro Leu Cys Asp Met His Pro Met Arg
225                 230                 235                 240

Ala Leu Phe Leu Ile Pro Arg Asn Pro Ala Pro Arg Leu Lys Ser Lys

245                 250                 255

Lys Trp Ser Lys Lys Phe Gln Ser Phe Ile Glu Ser Cys Leu Val Lys
            260                 265                 270

Asn His Ser Gln Arg Pro Ala Thr Glu Gln Leu Met Lys His Pro Phe
        275                 280                 285

Ile Arg Asp Gln Pro Asn Glu Arg Gln Val Arg Ile Gln Leu Lys Asp
    290                 295                 300

His Ile Asp Arg Thr Lys Lys Lys Arg Gly Glu Lys Asp Glu Thr Glu
305                 310                 315                 320

Tyr Glu Tyr Ser Gly Ser Glu Glu Glu Glu Glu Glu Asn Asp Ser Gly
                325                 330                 335

Glu Pro Ser Ser Ile Leu Asn Leu Pro Gly Glu Ser Thr Leu Arg Arg
            340                 345                 350

Asp Phe Leu Arg Leu Gln Leu Ala Asn Lys Glu Arg Ser Glu Ala Leu
        355                 360                 365

Arg Arg Gln Gln Leu Glu Gln Gln Gln Arg Glu Asn Glu Glu His Lys
    370                 375                 380

Arg Gln Leu Leu Ala Glu Arg Gln Lys Arg Ile Glu Glu Gln Lys Glu
385                 390                 395                 400

Gln Arg Arg Arg Leu Glu Glu Gln Gln Arg Arg Glu Lys Glu Leu Arg
                405                 410                 415

Lys Gln Gln Glu Arg Glu Gln Arg Arg His Tyr Glu Glu Gln Met Arg
            420                 425                 430

Arg Glu Glu Glu Arg Arg Arg Ala Glu His Glu Gln Glu Tyr Lys Arg
        435                 440                 445

Lys Gln Leu Glu Glu Gln Arg Gln Ala Glu Arg Leu Gln Arg Gln Leu
    450                 455                 460

Lys Gln Glu Arg Asp Tyr Leu Val Ser Leu Gln His Gln Arg Gln Glu
465                 470                 475                 480

Gln Arg Pro Val Glu Lys Lys Pro Leu Tyr His Tyr Lys Glu Gly Met
                485                 490                 495

Ser Pro Ser Glu Lys Pro Ala Trp Ala Lys Glu Ile Pro His Leu Val
        500                 505                 510

Ala Val Lys Ser Gln Gly Pro Ala Leu Thr Ala Ser Gln Ser Val His
    515                 520                 525

Glu Gln Pro Thr Lys Gly Leu Ser Gly Phe Gln Glu Ala Leu Asn Val
    530                 535                 540

Thr Ser His Arg Val Glu Met Pro Arg Gln Asn Ser Asp Pro Thr Ser
545                 550                 555                 560

Glu Asn Pro Pro Leu Pro Thr Arg Ile Glu Lys Phe Asp Arg Ser Ser
                565                 570                 575

Trp Leu Arg Gln Glu Glu Asp Ile Pro Pro Lys Val Pro Gln Arg Thr
        580                 585                 590

Thr Ser Ile Ser Pro Ala Leu Ala Arg Lys Asn Ser Pro Gly Asn Gly
    595                 600                 605

Ser Ala Leu Gly Pro Arg Leu Gly Ser Gln Pro Ile Arg Ala Ser Asn
    610                 615                 620

Pro Asp Leu Arg Arg Thr Glu Pro Ile Leu Glu Ser Pro Leu Gln Arg
625                 630                 635                 640

Thr Ser Ser Gly Ser Ser Ser Ser Ser Ser Thr Pro Ser Ser Gln Pro
                645                 650                 655

Ser Ser Gln Gly Gly Ser Gln Pro Gly Ser Gln Ala Gly Ser Ser Glu
        660                 665                 670

Arg Thr Arg Val Arg Ala Asn Ser Lys Ser Glu Gly Ser Pro Val Leu
    675                 680                 685

Pro His Glu Pro Ala Lys Val Lys Pro Glu Glu Ser Arg Asp Ile Thr
    690                 695                 700

Arg Pro Ser Arg Pro Ala Asp Leu Thr Ala Leu Ala Lys Glu Leu Arg
705                 710                 715                 720

Glu Leu Arg Ile Glu Glu Thr Asn Arg Pro Met Lys Lys Val Thr Asp
                725                 730                 735

Tyr Ser Ser Ser Ser Glu Glu Ser Glu Ser Ser Glu Glu Glu Glu Glu
        740                 745                 750

Asp Gly Glu Ser Glu Thr His Asp Gly Thr Val Ala Val Ser Asp Ile
        755                 760                 765

Pro Arg Leu Ile Pro Thr Gly Ala Pro Gly Ser Asn Glu Gln Tyr Asn
    770                 775                 780

Val Gly Met Val Gly Thr His Gly Leu Glu Thr Ser His Ala Asp Ser
785                 790                 795                 800

Phe Ser Gly Ser Ile Ser Arg Glu Gly Thr Leu Met Ile Arg Glu Thr
                805                 810                 815

Ser Gly Glu Lys Lys Arg Ser Gly His Ser Asp Ser Asn Gly Phe Ala
            820                 825                 830

Gly His Ile Asn Leu Pro Asp Leu Val Gln Gln Ser His Ser Pro Ala
        835                 840                 845

Gly Thr Pro Thr Glu Gly Leu Gly Arg Val Ser Thr His Ser Gln Glu
    850                 855                 860

Met Asp Ser Gly Thr Glu Tyr Gly Met Gly Ser Ser Thr Lys Ala Ser
865                 870                 875                 880

Phe Thr Pro Phe Val Asp Pro Arg Val Tyr Gln Thr Ser Pro Thr Asp
                885                 890                 895

Glu Asp Glu Glu Asp Glu Glu Ser Ser Ala Ala Ala Leu Phe Thr Ser
            900                 905                 910

Glu Leu Leu Arg Gln Glu Gln Ala Lys Leu Asn Glu Ala Arg Lys Ile
        915                 920                 925

Ser Val Val Asn Val Asn Pro Thr Asn Ile Arg Pro His Ser Asp Thr
    930                 935                 940

Pro Glu Ile Arg Lys Tyr Lys Lys Arg Phe Asn Ser Glu Ile Leu Cys
945                 950                 955                 960

Ala Ala Leu Trp Gly Val Asn Leu Leu Val Gly Thr Glu Asn Gly Leu
                965                 970                 975

Met Leu Leu Asp Arg Ser Gly Gln Gly Lys Val Tyr Asn Leu Ile Asn
            980                 985                 990

Arg Arg Arg Phe Gln Gln Met Asp  Val Leu Glu Gly Leu  Asn Val Leu
        995                 1000                1005

Val Thr Ile Ser Gly Lys Lys Asn Lys Leu Arg Val Tyr Tyr Leu
    1010                1015                1020

Ser Trp Leu Arg Asn Arg Ile Leu His Asn Asp Pro Glu Val Glu
    1025                1030                1035

Lys Lys Gln Gly Trp Ile Thr Val Gly Asp Leu Glu Gly Cys Ile
    1040                1045                1050

His Tyr Lys Val Val Lys Tyr Glu Arg Ile Lys Phe Leu Val Ile
    1055                1060                1065

Ala Leu Lys Asn Ala Val Glu Ile Tyr Ala Trp Ala Pro Lys Pro
    1070                1075                1080

Tyr His Lys Phe Met Ala Phe Lys Ser Phe Ala Asp Leu Gln His
    1085                1090                1095

Lys Pro Leu Leu Val Asp Leu Thr Val Glu Glu Gly Gln Arg Leu
    1100                1105                1110

Lys Val Ile Phe Gly Ser His Thr Gly Phe His Val Ile Asp Val
    1115                1120                1125

Asp Ser Gly Asn Ser Tyr Asp Ile Tyr Ile Pro Ser His Ile Gln
    1130                1135                1140

Gly Asn Ile Thr Pro His Ala Ile Val Ile Leu Pro Lys Thr Asp
    1145                1150                1155

Gly Met Glu Met Leu Val Cys Tyr Glu Asp Glu Gly Val Tyr Val
    1160                1165                1170

Asn Thr Tyr Gly Arg Ile Thr Lys Asp Val Val Leu Gln Trp Gly
    1175                1180                1185

Glu Met Pro Thr Ser Val Ala Tyr Ile His Ser Asn Gln Ile Met
    1190                1195                1200

Gly Trp Gly Glu Lys Ala Ile Glu Ile Arg Ser Val Glu Thr Gly
    1205                1210                1215

His Leu Asp Gly Val Phe Met His Lys Arg Ala Gln Arg Leu Lys
    1220                1225                1230

Phe Leu Cys Glu Arg Asn Asp Lys Val Phe Phe Ala Ser Val Arg

1235                1240                1245

Ser Gly Gly Ser Ser Gln Val Phe Phe Met Thr Leu Asn Arg Asn
    1250                1255                1260

Ser Met Met Asn Trp
    1265

<210>  49
<211>  4312
<212>  DNA
<213>  Homo sapiens

<220>
<221>  CDS
<222>  (127)..(4071)

<400>  49
aagcgtgcgc gtgatttggc cctgaacgga cgccgtagcc aagaggttgg gcggatgttg      60

tgagccgggt cgctgcggcc gaggctccgg cccccaggac cactggctgc ccatgagaga     120

tgaagg atg gca tcc aag ggg gcc ggc atg tct ttc tcc cgc aag agc        168
       Met Ala Ser Lys Gly Ala Gly Met Ser Phe Ser Arg Lys Ser
       1               5                   10

tat agg ctg acc tca gat gct gag aaa tcc agg gtc aca ggc att gtg       216
Tyr Arg Leu Thr Ser Asp Ala Glu Lys Ser Arg Val Thr Gly Ile Val
15                  20                  25                  30

cag gag aag ctg ctg aat gac tac ctg aac cgc atc ttt tcc tct tct       264
Gln Glu Lys Leu Leu Asn Asp Tyr Leu Asn Arg Ile Phe Ser Ser Ser
                35                  40                  45

gaa cat gca ccc cca gca gcc acc agc agg aaa ccc ctg aac ttc cag       312
Glu His Ala Pro Pro Ala Ala Thr Ser Arg Lys Pro Leu Asn Phe Gln
            50                  55                  60

aac ctg cca gaa cat ttg gac cag ttg cta cag gtg gac aat gag gag       360
Asn Leu Pro Glu His Leu Asp Gln Leu Leu Gln Val Asp Asn Glu Glu
        65                  70                  75

gag gaa agc cag gga cag gtt gaa ggg cgg ctt ggc cca tcc act gtg       408
Glu Glu Ser Gln Gly Gln Val Glu Gly Arg Leu Gly Pro Ser Thr Val
    80                  85                  90

gtc ctg gac cac aca ggc ggc ttt gag ggg ctt ctc ctg gtg gat gat       456
Val Leu Asp His Thr Gly Gly Phe Glu Gly Leu Leu Leu Val Asp Asp
95                  100                 105                 110

gac ctg ctg ggg gtg att gga cac agc aac ttt ggc acc atc cgc tct       504
Asp Leu Leu Gly Val Ile Gly His Ser Asn Phe Gly Thr Ile Arg Ser
                115                 120                 125

acc aca tgc gtg tac aaa ggg aaa tgg ctc tac gag gtc ctc atc tcc       552
Thr Thr Cys Val Tyr Lys Gly Lys Trp Leu Tyr Glu Val Leu Ile Ser
            130                 135                 140

tcc cag ggg ctc atg cag atc ggc tgg tgc acc atc agc tgc cgc ttc       600

Ser Gln Gly Leu Met Gln Ile Gly Trp Cys Thr Ile Ser Cys Arg Phe
        145                 150                 155

aac cag gag gag ggg gtt gga gat aca cac aac tcc tat gcc tat gat      648
Asn Gln Glu Glu Gly Val Gly Asp Thr His Asn Ser Tyr Ala Tyr Asp
    160                 165                 170

ggc aac cgc gtg cgc aag tgg aat gtg acc aca acg aat tat ggc aag      696
Gly Asn Arg Val Arg Lys Trp Asn Val Thr Thr Thr Asn Tyr Gly Lys
175                 180                 185                 190

gcg tgg gca gcg ggg gac atc gtg agc tgc ctg att gac ctg gat gat      744
Ala Trp Ala Ala Gly Asp Ile Val Ser Cys Leu Ile Asp Leu Asp Asp
                195                 200                 205

ggc act ctg tcc ttc tgc ctg aac ggt gta tca ctg ggc act gcc ttt      792
Gly Thr Leu Ser Phe Cys Leu Asn Gly Val Ser Leu Gly Thr Ala Phe
            210                 215                 220

gag aac ctg tcc agg ggc ctg ggt atg gcc tac ttc cca gcc atc agc      840
Glu Asn Leu Ser Arg Gly Leu Gly Met Ala Tyr Phe Pro Ala Ile Ser
        225                 230                 235

ctc tct ttc aag gag tcc gtg gcc ttc aac ttt ggc agc cgt cct ctg      888
Leu Ser Phe Lys Glu Ser Val Ala Phe Asn Phe Gly Ser Arg Pro Leu
    240                 245                 250

cgc tac cca gtg gca ggc tac cgg ccc ctg cag gac cca ccg agt gct      936
Arg Tyr Pro Val Ala Gly Tyr Arg Pro Leu Gln Asp Pro Pro Ser Ala
255                 260                 265                 270

gac ctg gtg cgg gca cag agg ttg ctg ggc tgc ttc cgg gca gtg ctg      984
Asp Leu Val Arg Ala Gln Arg Leu Leu Gly Cys Phe Arg Ala Val Leu
                275                 280                 285

agt gtg gag ctg gac cct gtg gag ggg cgg ctg ttg gac aag gag agc      1032
Ser Val Glu Leu Asp Pro Val Glu Gly Arg Leu Leu Asp Lys Glu Ser
            290                 295                 300

tcc aag tgg cgg ttg cgg ggc cag ccc acc gtc ctc ctc aca ctg gcc      1080
Ser Lys Trp Arg Leu Arg Gly Gln Pro Thr Val Leu Leu Thr Leu Ala
        305                 310                 315

cac atc ttc cat cac ttt gca ccg ctt ctg cgc aag gtg tat ctg gtg      1128
His Ile Phe His His Phe Ala Pro Leu Leu Arg Lys Val Tyr Leu Val
    320                 325                 330

gag gct gtg ctc atg agc ttc ttg ctg ggc atc gtg gag aag ggc aca      1176
Glu Ala Val Leu Met Ser Phe Leu Leu Gly Ile Val Glu Lys Gly Thr
335                 340                 345                 350

ccc aca cag gca cag tcc gtg gtg cac cag gtc ctg gac ctc ttg tgg      1224
Pro Thr Gln Ala Gln Ser Val Val His Gln Val Leu Asp Leu Leu Trp
                355                 360                 365

ctc ttc atg gag gac tac gag gta caa gat tgc ctc aag cag ttg atg      1272
Leu Phe Met Glu Asp Tyr Glu Val Gln Asp Cys Leu Lys Gln Leu Met
            370                 375                 380

atg tct ctg ctt cgg ctg tac cga ttc tca ccc att gtc cca gac ctg      1320
Met Ser Leu Leu Arg Leu Tyr Arg Phe Ser Pro Ile Val Pro Asp Leu
        385                 390                 395

ggc cta cag atc cat tac ctg cgg ctc act atc gcc atc ctg agg cat 1368
Gly Leu Gln Ile His Tyr Leu Arg Leu Thr Ile Ala Ile Leu Arg His
    400                 405                 410

gag aag tcc cgc aag ttt ctg ctt agc aat gtc ctc ttc gac gtg ctc 1416
Glu Lys Ser Arg Lys Phe Leu Leu Ser Asn Val Leu Phe Asp Val Leu
415                 420                 425                 430

cgc tcc gtc gtc ttc ttt tac atc aag agc ccc ctg cgt gtg gag gag 1464
Arg Ser Val Val Phe Phe Tyr Ile Lys Ser Pro Leu Arg Val Glu Glu
                435                 440                 445

gcc ggc ctg cag gag ctc att ccc acc acc tgg tgg ccc cac tgc tcc 1512
Ala Gly Leu Gln Glu Leu Ile Pro Thr Thr Trp Trp Pro His Cys Ser
            450                 455                 460

agt agg gag ggc aaa gag agc acg gag atg aag gag gag acc gca gag 1560
Ser Arg Glu Gly Lys Glu Ser Thr Glu Met Lys Glu Glu Thr Ala Glu
        465                 470                 475

gag cgg ctg cgg cgg cga gcc tac gaa cgg ggc tgt cag cgg ctc agg 1608
Glu Arg Leu Arg Arg Arg Ala Tyr Glu Arg Gly Cys Gln Arg Leu Arg
    480                 485                 490

aag cgc atc gaa gtg gtg gaa gaa cta cag gtc cag atc ctg aag ctg 1656
Lys Arg Ile Glu Val Val Glu Glu Leu Gln Val Gln Ile Leu Lys Leu
495                 500                 505                 510

ctg ctg gac aat aaa gat gac aat ggg ggt gaa gct tct agg tat atc 1704
Leu Leu Asp Asn Lys Asp Asp Asn Gly Gly Glu Ala Ser Arg Tyr Ile
                515                 520                 525

ttc ctg acc aag ttt cgc aag ttt ctg cag gag aac gcc agt ggc cgg 1752
Phe Leu Thr Lys Phe Arg Lys Phe Leu Gln Glu Asn Ala Ser Gly Arg
            530                 535                 540

ggg aac atg ccc atg ctc tgc ccc cct gag tac atg gtc tgc ttc tta 1800
Gly Asn Met Pro Met Leu Cys Pro Pro Glu Tyr Met Val Cys Phe Leu
        545                 550                 555

cac cgg ctg atc tct gcc ctg cgc tac tat tgg gat gaa tac aag gct 1848
His Arg Leu Ile Ser Ala Leu Arg Tyr Tyr Trp Asp Glu Tyr Lys Ala
    560                 565                 570

tcc aat cct cat gct tcc ttc agt gag gag gcc tac atc ccg ccc cag 1896
Ser Asn Pro His Ala Ser Phe Ser Glu Glu Ala Tyr Ile Pro Pro Gln
575                 580                 585                 590

gtc ttc tat aat ggc aag gtg gac tac ttt gac ctg cag cgc ctg ggg 1944
Val Phe Tyr Asn Gly Lys Val Asp Tyr Phe Asp Leu Gln Arg Leu Gly
                595                 600                 605

ggc ctc ctc tcg cac ctg cgg aag acc ctc aaa gat gac ctt gct tcc 1992
Gly Leu Leu Ser His Leu Arg Lys Thr Leu Lys Asp Asp Leu Ala Ser
            610                 615                 620

aaa gcc aac att gtg atc gac cca ctg gag ctc cag tca acc gcc atg 2040
Lys Ala Asn Ile Val Ile Asp Pro Leu Glu Leu Gln Ser Thr Ala Met
        625                 630                 635

gat gac cta gat gag gat gag gag cca gcc cca gct atg gcc cag cgc 2088
Asp Asp Leu Asp Glu Asp Glu Glu Pro Ala Pro Ala Met Ala Gln Arg
    640                 645                 650

ccc atg cag gcc ctg gct gtt ggg ggg cca ctg ccc ctg ccc cgg ccc     2136
Pro Met Gln Ala Leu Ala Val Gly Gly Pro Leu Pro Leu Pro Arg Pro
655                 660                 665                 670

ggc tgg ctc agt tct cca act ttg ggc cga gcc aac cgc ttc ctc agc     2184
Gly Trp Leu Ser Ser Pro Thr Leu Gly Arg Ala Asn Arg Phe Leu Ser
                675                 680                 685

aca gcg gct gtg agc ctc atg acc cca cgg cgg cct ctg agc acc tcg     2232
Thr Ala Ala Val Ser Leu Met Thr Pro Arg Arg Pro Leu Ser Thr Ser
            690                 695                 700

gag aaa gtg aag gtc cgc acg ctg agc gtg gag cag agg acc cgt gag     2280
Glu Lys Val Lys Val Arg Thr Leu Ser Val Glu Gln Arg Thr Arg Glu
        705                 710                 715

gac att gaa ggc agc cac tgg aat gag ggc ttg ctg ctg ggg cgg ccc     2328
Asp Ile Glu Gly Ser His Trp Asn Glu Gly Leu Leu Leu Gly Arg Pro
    720                 725                 730

ccc gag gag cct gag cag ccc ctc acc gag aac tcg ctg ctg gaa gtc     2376
Pro Glu Glu Pro Glu Gln Pro Leu Thr Glu Asn Ser Leu Leu Glu Val
735                 740                 745                 750

ctg gat ggg gcg gtc atg atg tac aac ctc agc gta cac cag cag ctg     2424
Leu Asp Gly Ala Val Met Met Tyr Asn Leu Ser Val His Gln Gln Leu
                755                 760                 765

ggc aag atg gtg ggt gtc tcc gat gat gtc aat gaa tac gct atg gct     2472
Gly Lys Met Val Gly Val Ser Asp Asp Val Asn Glu Tyr Ala Met Ala
            770                 775                 780

ctg agg gac aca gag gac aag ctc cgc cgg tgc ccc aag agg agg aag     2520
Leu Arg Asp Thr Glu Asp Lys Leu Arg Arg Cys Pro Lys Arg Arg Lys
        785                 790                 795

gac atc ctt gca gag ttg acc aag agc cag aag gtt ttc tca gaa aag     2568
Asp Ile Leu Ala Glu Leu Thr Lys Ser Gln Lys Val Phe Ser Glu Lys
    800                 805                 810

ctg gac cac ctg agc cgc cgt ctt gcc tgg gtc cat gcc act gtc tac     2616
Leu Asp His Leu Ser Arg Arg Leu Ala Trp Val His Ala Thr Val Tyr
815                 820                 825                 830

tcc cag gag aag atg ctg gac atc tac tgg ctg ctg cgc gtc tgc ctg     2664
Ser Gln Glu Lys Met Leu Asp Ile Tyr Trp Leu Leu Arg Val Cys Leu
                835                 840                 845

cgg acc att gag cac ggt gat cgc aca ggg tct ctc ttt gcc ttc atg     2712
Arg Thr Ile Glu His Gly Asp Arg Thr Gly Ser Leu Phe Ala Phe Met
            850                 855                 860

ccc gag ttc tac ctg agc gtg gcc atc aac agc tac agt gct ctc aag     2760
Pro Glu Phe Tyr Leu Ser Val Ala Ile Asn Ser Tyr Ser Ala Leu Lys
        865                 870                 875

aat tac ttt ggt ccc gtg cac agc atg gag gag ctc cca ggc tat gaa     2808
Asn Tyr Phe Gly Pro Val His Ser Met Glu Glu Leu Pro Gly Tyr Glu
    880                 885                 890

gag acc ctg acc cgc ctg gct gcc att ctc gcc aaa cac ttt gcc gac     2856
Glu Thr Leu Thr Arg Leu Ala Ala Ile Leu Ala Lys His Phe Ala Asp

895                 900                 905                 910

gca cgc att gtg ggc act gac atc cga gac tca ctg atg cag gcc ctg      2904
Ala Arg Ile Val Gly Thr Asp Ile Arg Asp Ser Leu Met Gln Ala Leu
                915                 920                 925

gcc agc tac gtg tgc tac cca cac tcc ctg cgg gct gtg gag cga atc      2952
Ala Ser Tyr Val Cys Tyr Pro His Ser Leu Arg Ala Val Glu Arg Ile
            930                 935                 940

ccc gag gag cag cgt atc gcc atg gtg agg aac ctc ctg gcg ccc tat      3000
Pro Glu Glu Gln Arg Ile Ala Met Val Arg Asn Leu Leu Ala Pro Tyr
        945                 950                 955

gag cag cgg ccc tgg gcc cag acc aac tgg atc ctg gtg cgg ctc tgg      3048
Glu Gln Arg Pro Trp Ala Gln Thr Asn Trp Ile Leu Val Arg Leu Trp
    960                 965                 970

agg ggc tgt ggc ttc ggg tac cgc tat aca cgg ctg cca cat ctg ctg      3096
Arg Gly Cys Gly Phe Gly Tyr Arg Tyr Thr Arg Leu Pro His Leu Leu
975                 980                 985                 990

aaa acc aaa ctt gag gac gcc aat ttg ccc  agc ctc cag aag ccc  tgc      3144
Lys Thr Lys Leu Glu Asp Ala Asn Leu Pro  Ser Leu Gln Lys Pro  Cys
                995                 1000                1005

cct tcc acc ctg  ctg cag cag cac atg  gcg gac ctc cta cag  cag      3189
Pro Ser Thr Leu  Leu Gln Gln His Met  Ala Asp Leu Leu Gln  Gln
            1010                1015                1020

ggt cct gat gtg  gca ccc agc ttc ctc  aac agc gtc ctc aat  cag      3234
Gly Pro Asp Val  Ala Pro Ser Phe Leu  Asn Ser Val Leu Asn  Gln
            1025                1030                1035

ctc aac tgg gcc  ttc tct gaa ttc att  ggc atg atc caa gag  atc      3279
Leu Asn Trp Ala  Phe Ser Glu Phe Ile  Gly Met Ile Gln Glu  Ile
            1040                1045                1050

cag cag gct gct  gag cgc ctg gag cgg  aac ttt gtg gac agc  cgg      3324
Gln Gln Ala Ala  Glu Arg Leu Glu Arg  Asn Phe Val Asp Ser  Arg
            1055                1060                1065

cag ctc aag gta  tgt gcc acc tgc ttt  gac ctc tcg gtc agc  ctg      3369
Gln Leu Lys Val  Cys Ala Thr Cys Phe  Asp Leu Ser Val Ser  Leu
            1070                1075                1080

ctg cgt gtc ttg  gag atg act atc aca  ctg gtg cct gag ata  ttc      3414
Leu Arg Val Leu  Glu Met Thr Ile Thr  Leu Val Pro Glu Ile  Phe
            1085                1090                1095

ctt gac tgg acc  cgg cct acc tct gag  atg ctg ctg cgg cgt  ctt      3459
Leu Asp Trp Thr  Arg Pro Thr Ser Glu  Met Leu Leu Arg Arg  Leu
            1100                1105                1110

gca cag ctg cta  aac cag gtg ctg aac  cgg gtg aca gct gag  agg      3504
Ala Gln Leu Leu  Asn Gln Val Leu Asn  Arg Val Thr Ala Glu  Arg
            1115                1120                1125

aac ctg ttt gat  cgt gtg gtc acc cta  cgg ctg cct ggc cta  gag      3549
Asn Leu Phe Asp  Arg Val Val Thr Leu  Arg Leu Pro Gly Leu  Glu
            1130                1135                1140

agc gtg gac cac  tat ccc att ctg gtg  gca gtg acg ggc atc  ctg      3594

Ser Val Asp His Tyr Pro Ile Leu Val Ala Val Thr Gly Ile Leu
            1145                1150                1155

gtg cag ctc ctg gtg cgt ggc cca gcc tca gag aga gag caa gcc 3639
Val Gln Leu Leu Val Arg Gly Pro Ala Ser Glu Arg Glu Gln Ala
            1160                1165                1170

aca tca gtg ctc ctg gca gat ccc tgc ttc cag cta cgc tca ata 3684
Thr Ser Val Leu Leu Ala Asp Pro Cys Phe Gln Leu Arg Ser Ile
            1175                1180                1185

tgc tat ctc ctg gga cag cca gag ccc cca gca cct ggc act gct 3729
Cys Tyr Leu Leu Gly Gln Pro Glu Pro Pro Ala Pro Gly Thr Ala
            1190                1195                1200

ctg cca gcc cct gac cgg aag cgc ttc tcc ctg cag agc tat gcg 3774
Leu Pro Ala Pro Asp Arg Lys Arg Phe Ser Leu Gln Ser Tyr Ala
            1205                1210                1215

gat tat atc agt gcc gat gag ctg gcc caa gtg gaa cag atg ctg 3819
Asp Tyr Ile Ser Ala Asp Glu Leu Ala Gln Val Glu Gln Met Leu
            1220                1225                1230

gcg cac ctg acc tct gca tct gcc cag gca gca gct gcc tcc ctg 3864
Ala His Leu Thr Ser Ala Ser Ala Gln Ala Ala Ala Ala Ser Leu
            1235                1240                1245

ccc acc agt gag gag gac ctc tgc ccc atc tgc tat gcc cac ccc 3909
Pro Thr Ser Glu Glu Asp Leu Cys Pro Ile Cys Tyr Ala His Pro
            1250                1255                1260

atc tct gct gtg ttc cag ccc tgt ggc cac aag tcc tgc aaa gcc 3954
Ile Ser Ala Val Phe Gln Pro Cys Gly His Lys Ser Cys Lys Ala
            1265                1270                1275

tgt atc aac cag cac ctg atg aac aac aag gac tgc ttc ttc tgc 3999
Cys Ile Asn Gln His Leu Met Asn Asn Lys Asp Cys Phe Phe Cys
            1280                1285                1290

aaa acc acc atc gtg tct gta gag gac tgg gag aag gga gcc aat 4044
Lys Thr Thr Ile Val Ser Val Glu Asp Trp Glu Lys Gly Ala Asn
            1295                1300                1305

acg agt act acc tcc tca gct gcc tag ccctcacagc ctgtgccatc 4091
Thr Ser Thr Thr Ser Ser Ala Ala
            1310

ctggaacctc cacctttgaa cccagagcca ggctgggccc tatttatgag ctccctttgc 4151

ccttctcctg tatcccacac caccacatcc aacctccttg cctgcctgta tcctcattgg 4211

tgggagccca gccatggccc taattgtgcc tgagcttgac tttcagtcag ggccacagtg 4271

agcattaaat tattattcca tacaaaaaaa aaaaaaaaaa a 4312

<210> 50
<211> 1314
<212> PRT
<213> Homo sapiens

<400> 50

Met Ala Ser Lys Gly Ala Gly Met Ser Phe Ser Arg Lys Ser Tyr Arg
1               5                   10                  15

Leu Thr Ser Asp Ala Glu Lys Ser Arg Val Thr Gly Ile Val Gln Glu
            20                  25                  30

Lys Leu Leu Asn Asp Tyr Leu Asn Arg Ile Phe Ser Ser Ser Glu His
        35                  40                  45

Ala Pro Pro Ala Ala Thr Ser Arg Lys Pro Leu Asn Phe Gln Asn Leu
    50                  55                  60

Pro Glu His Leu Asp Gln Leu Leu Gln Val Asp Asn Glu Glu Glu Glu
65                  70                  75                  80

Ser Gln Gly Gln Val Glu Gly Arg Leu Gly Pro Ser Thr Val Val Leu
                85                  90                  95

Asp His Thr Gly Gly Phe Glu Gly Leu Leu Leu Val Asp Asp Asp Leu
            100                 105                 110

Leu Gly Val Ile Gly His Ser Asn Phe Gly Thr Ile Arg Ser Thr Thr
        115                 120                 125

Cys Val Tyr Lys Gly Lys Trp Leu Tyr Glu Val Leu Ile Ser Ser Gln
    130                 135                 140

Gly Leu Met Gln Ile Gly Trp Cys Thr Ile Ser Cys Arg Phe Asn Gln
145                 150                 155                 160

Glu Glu Gly Val Gly Asp Thr His Asn Ser Tyr Ala Tyr Asp Gly Asn
                165                 170                 175

Arg Val Arg Lys Trp Asn Val Thr Thr Thr Asn Tyr Gly Lys Ala Trp
            180                 185                 190

Ala Ala Gly Asp Ile Val Ser Cys Leu Ile Asp Leu Asp Asp Gly Thr
        195                 200                 205

Leu Ser Phe Cys Leu Asn Gly Val Ser Leu Gly Thr Ala Phe Glu Asn
    210                 215                 220

Leu Ser Arg Gly Leu Gly Met Ala Tyr Phe Pro Ala Ile Ser Leu Ser
225                 230                 235                 240

Phe Lys Glu Ser Val Ala Phe Asn Phe Gly Ser Arg Pro Leu Arg Tyr
                245                 250                 255

Pro Val Ala Gly Tyr Arg Pro Leu Gln Asp Pro Pro Ser Ala Asp Leu
            260                 265                 270

Val Arg Ala Gln Arg Leu Leu Gly Cys Phe Arg Ala Val Leu Ser Val
        275                 280                 285

Glu Leu Asp Pro Val Glu Gly Arg Leu Leu Asp Lys Glu Ser Ser Lys
    290                 295                 300

Trp Arg Leu Arg Gly Gln Pro Thr Val Leu Leu Thr Leu Ala His Ile
305                 310                 315                 320

Phe His His Phe Ala Pro Leu Leu Arg Lys Val Tyr Leu Val Glu Ala
                325                 330                 335

Val Leu Met Ser Phe Leu Leu Gly Ile Val Glu Lys Gly Thr Pro Thr
            340                 345                 350

Gln Ala Gln Ser Val Val His Gln Val Leu Asp Leu Leu Trp Leu Phe
        355                 360                 365

Met Glu Asp Tyr Glu Val Gln Asp Cys Leu Lys Gln Leu Met Met Ser
    370                 375                 380

Leu Leu Arg Leu Tyr Arg Phe Ser Pro Ile Val Pro Asp Leu Gly Leu
385                 390                 395                 400

Gln Ile His Tyr Leu Arg Leu Thr Ile Ala Ile Leu Arg His Glu Lys
                405                 410                 415

Ser Arg Lys Phe Leu Leu Ser Asn Val Leu Phe Asp Val Leu Arg Ser
            420                 425                 430

Val Val Phe Phe Tyr Ile Lys Ser Pro Leu Arg Val Glu Glu Ala Gly
        435                 440                 445

Leu Gln Glu Leu Ile Pro Thr Thr Trp Trp Pro His Cys Ser Ser Arg
    450                 455                 460

Glu Gly Lys Glu Ser Thr Glu Met Lys Glu Glu Thr Ala Glu Glu Arg
465                 470                 475                 480

Leu Arg Arg Arg Ala Tyr Glu Arg Gly Cys Gln Arg Leu Arg Lys Arg
                485                 490                 495

Ile Glu Val Val Glu Glu Leu Gln Val Gln Ile Leu Lys Leu Leu Leu
            500                 505                 510

Asp Asn Lys Asp Asp Asn Gly Gly Glu Ala Ser Arg Tyr Ile Phe Leu
        515                 520                 525

Thr Lys Phe Arg Lys Phe Leu Gln Glu Asn Ala Ser Gly Arg Gly Asn
    530                 535                 540

Met Pro Met Leu Cys Pro Pro Glu Tyr Met Val Cys Phe Leu His Arg
545                 550                 555                 560

Leu Ile Ser Ala Leu Arg Tyr Tyr Trp Asp Glu Tyr Lys Ala Ser Asn
                565                 570                 575

Pro His Ala Ser Phe Ser Glu Glu Ala Tyr Ile Pro Pro Gln Val Phe
            580                 585                 590

Tyr Asn Gly Lys Val Asp Tyr Phe Asp Leu Gln Arg Leu Gly Gly Leu
        595                 600                 605

Leu Ser His Leu Arg Lys Thr Leu Lys Asp Asp Leu Ala Ser Lys Ala
    610                 615                 620

Asn Ile Val Ile Asp Pro Leu Glu Leu Gln Ser Thr Ala Met Asp Asp
625                 630                 635                 640

Leu Asp Glu Asp Glu Glu Pro Ala Pro Ala Met Ala Gln Arg Pro Met
                645                 650                 655

Gln Ala Leu Ala Val Gly Gly Pro Leu Pro Leu Pro Arg Pro Gly Trp
            660                 665                 670

Leu Ser Ser Pro Thr Leu Gly Arg Ala Asn Arg Phe Leu Ser Thr Ala
        675                 680                 685

Ala Val Ser Leu Met Thr Pro Arg Arg Pro Leu Ser Thr Ser Glu Lys
    690                 695                 700

Val Lys Val Arg Thr Leu Ser Val Glu Gln Arg Thr Arg Glu Asp Ile
705                 710                 715                 720

Glu Gly Ser His Trp Asn Glu Gly Leu Leu Leu Gly Arg Pro Pro Glu
                725                 730                 735

Glu Pro Glu Gln Pro Leu Thr Glu Asn Ser Leu Leu Glu Val Leu Asp
            740                 745                 750

Gly Ala Val Met Met Tyr Asn Leu Ser Val His Gln Gln Leu Gly Lys

755                 760                 765

Met Val Gly Val Ser Asp Asp Val Asn Glu Tyr Ala Met Ala Leu Arg
    770                 775                 780

Asp Thr Glu Asp Lys Leu Arg Arg Cys Pro Lys Arg Arg Lys Asp Ile
785                 790                 795                 800

Leu Ala Glu Leu Thr Lys Ser Gln Lys Val Phe Ser Glu Lys Leu Asp
                805                 810                 815

His Leu Ser Arg Arg Leu Ala Trp Val His Ala Thr Val Tyr Ser Gln
            820                 825                 830

Glu Lys Met Leu Asp Ile Tyr Trp Leu Leu Arg Val Cys Leu Arg Thr
        835                 840                 845

Ile Glu His Gly Asp Arg Thr Gly Ser Leu Phe Ala Phe Met Pro Glu
    850                 855                 860

Phe Tyr Leu Ser Val Ala Ile Asn Ser Tyr Ser Ala Leu Lys Asn Tyr
865                 870                 875                 880

Phe Gly Pro Val His Ser Met Glu Glu Leu Pro Gly Tyr Glu Glu Thr
                885                 890                 895

Leu Thr Arg Leu Ala Ala Ile Leu Ala Lys His Phe Ala Asp Ala Arg
            900                 905                 910

Ile Val Gly Thr Asp Ile Arg Asp Ser Leu Met Gln Ala Leu Ala Ser
        915                 920                 925

Tyr Val Cys Tyr Pro His Ser Leu Arg Ala Val Glu Arg Ile Pro Glu
    930                 935                 940

Glu Gln Arg Ile Ala Met Val Arg Asn Leu Leu Ala Pro Tyr Glu Gln
945                 950                 955                 960

Arg Pro Trp Ala Gln Thr Asn Trp Ile Leu Val Arg Leu Trp Arg Gly
                965                 970                 975

Cys Gly Phe Gly Tyr Arg Tyr Thr Arg Leu Pro His Leu Leu Lys Thr
            980                 985                 990

Lys Leu Glu Asp Ala Asn Leu Pro  Ser Leu Gln Lys Pro  Cys Pro Ser
        995                 1000                1005

Thr Leu Leu Gln Gln His Met Ala Asp Leu Leu Gln Gln Gly Pro
    1010                1015                1020

Asp Val Ala Pro Ser Phe Leu Asn Ser Val Leu Asn Gln Leu Asn
    1025                1030                1035

Trp Ala Phe Ser Glu Phe Ile Gly Met Ile Gln Glu Ile Gln Gln
    1040                1045                1050

Ala Ala Glu Arg Leu Glu Arg Asn Phe Val Asp Ser Arg Gln Leu
    1055                1060                1065

Lys Val Cys Ala Thr Cys Phe Asp Leu Ser Val Ser Leu Leu Arg
    1070                1075                1080

Val Leu Glu Met Thr Ile Thr Leu Val Pro Glu Ile Phe Leu Asp
    1085                1090                1095

Trp Thr Arg Pro Thr Ser Glu Met Leu Leu Arg Arg Leu Ala Gln
    1100                1105                1110

Leu Leu Asn Gln Val Leu Asn Arg Val Thr Ala Glu Arg Asn Leu
    1115                1120                1125

Phe Asp Arg Val Val Thr Leu Arg Leu Pro Gly Leu Glu Ser Val
    1130                1135                1140

Asp His Tyr Pro Ile Leu Val Ala Val Thr Gly Ile Leu Val Gln
    1145                1150                1155

Leu Leu Val Arg Gly Pro Ala Ser Glu Arg Glu Gln Ala Thr Ser
    1160                1165                1170

Val Leu Leu Ala Asp Pro Cys Phe Gln Leu Arg Ser Ile Cys Tyr
    1175                1180                1185

Leu Leu Gly Gln Pro Glu Pro Pro Ala Pro Gly Thr Ala Leu Pro
    1190                1195                1200

Ala Pro Asp Arg Lys Arg Phe Ser Leu Gln Ser Tyr Ala Asp Tyr
    1205                1210                1215

Ile Ser Ala Asp Glu Leu Ala Gln Val Glu Gln Met Leu Ala His
    1220                1225                1230

Leu Thr Ser Ala Ser Ala Gln Ala Ala Ala Ala Ser Leu Pro Thr
    1235                1240                1245

Ser Glu Glu Asp Leu Cys Pro Ile Cys Tyr Ala His Pro Ile Ser
    1250                1255                1260

Ala Val Phe Gln Pro Cys Gly His Lys Ser Cys Lys Ala Cys Ile
    1265                1270                1275

Asn Gln His Leu Met Asn Asn Lys Asp Cys Phe Phe Cys Lys Thr
    1280                1285                1290

Thr Ile Val Ser Val Glu Asp Trp Glu Lys Gly Ala Asn Thr Ser
    1295                1300                1305

Thr Thr Ser Ser Ala Ala
    1310

<210> 51
<211> 6891
<212> DNA
<213> Homo sapiens

<220>
<221> CDS
<222> (165)..(3803)

<400> 51
ggtggcctct gtggccgtcc aggctagcgg cggcccgcag gcggcgggga gaaagactct 60

ctcacctggt cttgcggctg tggccaccgc cggccagggg tgtggagggc gtgctgccgg 120

agacgtccgc cgggctctgc agttccgccg ggggtcgggc agct atg gag ccg cgg 176
Met Glu Pro Arg
1

ccc acg gcg ccc tcc tcc ggc gcc ccg gga ctg gcc ggg gtc ggg gag 224
Pro Thr Ala Pro Ser Ser Gly Ala Pro Gly Leu Ala Gly Val Gly Glu
5                   10                  15                  20

acg ccg tca gcc gct gcg ctg gcc gca gcc agg gtg gaa ctg ccc ggc 272
Thr Pro Ser Ala Ala Ala Leu Ala Ala Ala Arg Val Glu Leu Pro Gly
                25                  30                  35

acg gct gtg ccc tcg gtg ccg gag gat gct gcg ccc gcg agc cgg gac 320
Thr Ala Val Pro Ser Val Pro Glu Asp Ala Ala Pro Ala Ser Arg Asp
            40                  45                  50

ggc ggc ggg gtc cgc gat gag ggc ccc gcg gcg gcc ggg gac ggg ctg 368
Gly Gly Gly Val Arg Asp Glu Gly Pro Ala Ala Ala Gly Asp Gly Leu
        55                  60                  65

ggc aga ccc ttg ggg ccc acc ccg agc cag agc cgt ttc cag gtg gac 416
Gly Arg Pro Leu Gly Pro Thr Pro Ser Gln Ser Arg Phe Gln Val Asp
    70                  75                  80

ctg gtt tcc gag aac gcc ggg cgg gcc gct gct gcg gcg gcg gcg gcg 464
Leu Val Ser Glu Asn Ala Gly Arg Ala Ala Ala Ala Ala Ala Ala Ala
85                  90                  95                  100

gcg gcg gca gcg gcg gcg gct ggt gct ggg gcg ggg gcc aag cag acc     512
Ala Ala Ala Ala Ala Ala Ala Gly Ala Gly Ala Gly Ala Lys Gln Thr
                105                 110                 115

ccc gcg gac ggg gaa gcc agc ggc gag agc gag ccg gct aaa ggc agc     560
Pro Ala Asp Gly Glu Ala Ser Gly Glu Ser Glu Pro Ala Lys Gly Ser
            120                 125                 130

gag gaa gcc aag ggc cgc ttc cgc gtg aac ttc gtg gac cca gct gcc     608
Glu Glu Ala Lys Gly Arg Phe Arg Val Asn Phe Val Asp Pro Ala Ala
        135                 140                 145

tcc tcg tcg gct gaa gac agc ctg tca gat gct gcc ggg gtc gga gtc     656
Ser Ser Ser Ala Glu Asp Ser Leu Ser Asp Ala Ala Gly Val Gly Val
    150                 155                 160

gac ggg ccc aac gtg agc ttc cag aac ggc ggg gac acg gtg ctg agc     704
Asp Gly Pro Asn Val Ser Phe Gln Asn Gly Gly Asp Thr Val Leu Ser
165                 170                 175                 180

gag ggc agc agc ctg cac tcc ggc ggc ggc ggc agt ggg cac cac     752
Glu Gly Ser Ser Leu His Ser Gly Gly Gly Gly Ser Gly His His
                185                 190                 195

cag cac tac tat tat gat acc cac acc aac acc tac tac ctg cgc acc     800
Gln His Tyr Tyr Tyr Asp Thr His Thr Asn Thr Tyr Tyr Leu Arg Thr
            200                 205                 210

ttc ggc cac aac acc atg gac gct gtg ccc agg atc gat cac tac cgg     848
Phe Gly His Asn Thr Met Asp Ala Val Pro Arg Ile Asp His Tyr Arg
        215                 220                 225

cac aca gcc gcg cag ctg ggc gag aag ctg ctc cgg cct agc ctg gcg     896
His Thr Ala Ala Gln Leu Gly Glu Lys Leu Leu Arg Pro Ser Leu Ala
    230                 235                 240

gag ctc cac gac gag ctg gaa aag gaa cct ttt gag gat ggc ttt gca     944
Glu Leu His Asp Glu Leu Glu Lys Glu Pro Phe Glu Asp Gly Phe Ala
245                 250                 255                 260

aat ggg gaa gaa agt act cca acc aga gat gct gtg gtc acg tat act     992
Asn Gly Glu Glu Ser Thr Pro Thr Arg Asp Ala Val Val Thr Tyr Thr
                265                 270                 275

gca gaa agt aaa gga gtc gtg aag ttt ggc tgg atc aag ggt gta tta     1040
Ala Glu Ser Lys Gly Val Val Lys Phe Gly Trp Ile Lys Gly Val Leu
            280                 285                 290

gta cgt tgt atg tta aac att tgg ggt gtg atg ctt ttc att aga ttg     1088
Val Arg Cys Met Leu Asn Ile Trp Gly Val Met Leu Phe Ile Arg Leu
        295                 300                 305

tca tgg att gtg ggt caa gct gga ata ggt cta tca gtc ctt gta ata     1136
Ser Trp Ile Val Gly Gln Ala Gly Ile Gly Leu Ser Val Leu Val Ile
    310                 315                 320

atg atg gcc act gtt gtg aca act atc aca gga ttg tct act tca gca     1184
Met Met Ala Thr Val Val Thr Thr Ile Thr Gly Leu Ser Thr Ser Ala
325                 330                 335                 340

ata gca act aat gga ttt gta aga gga gga gga gca tat tat tta ata     1232
Ile Ala Thr Asn Gly Phe Val Arg Gly Gly Gly Ala Tyr Tyr Leu Ile

345                 350                 355

tct aga agt cta ggg cca gaa ttt ggt ggt gca att ggt cta atc ttc      1280
Ser Arg Ser Leu Gly Pro Glu Phe Gly Gly Ala Ile Gly Leu Ile Phe
            360                 365                 370

gcc ttt gcc aac gct gtt gca gtt gct atg tat gtg gtt gga ttt gca      1328
Ala Phe Ala Asn Ala Val Ala Val Ala Met Tyr Val Val Gly Phe Ala
        375                 380                 385

gaa acc gtg gtg gag ttg ctt aag gaa cat tcc ata ctt atg ata gat      1376
Glu Thr Val Val Glu Leu Leu Lys Glu His Ser Ile Leu Met Ile Asp
    390                 395                 400

gaa atc aat gat atc cga att att gga gcc att aca gtc gtg att ctt      1424
Glu Ile Asn Asp Ile Arg Ile Ile Gly Ala Ile Thr Val Val Ile Leu
405                 410                 415                 420

tta ggt atc tca gta gct gga atg gag tgg gaa gca aaa gct cag att      1472
Leu Gly Ile Ser Val Ala Gly Met Glu Trp Glu Ala Lys Ala Gln Ile
                425                 430                 435

gtt ctt ttg gtg atc cta ctt ctt gct att ggt gat ttc gtc ata gga      1520
Val Leu Leu Val Ile Leu Leu Leu Ala Ile Gly Asp Phe Val Ile Gly
            440                 445                 450

aca ttt atc cca ctg gag agc aag aag cca aaa ggg ttt ttt ggt tat      1568
Thr Phe Ile Pro Leu Glu Ser Lys Lys Pro Lys Gly Phe Phe Gly Tyr
        455                 460                 465

aaa tct gaa ata ttt aat gag aac ttt ggg ccc gat ttt cga gag gaa      1616
Lys Ser Glu Ile Phe Asn Glu Asn Phe Gly Pro Asp Phe Arg Glu Glu
    470                 475                 480

gag act ttc ttt tct gta ttt gcc atc ttt ttt cct gct gca act ggt      1664
Glu Thr Phe Phe Ser Val Phe Ala Ile Phe Phe Pro Ala Ala Thr Gly
485                 490                 495                 500

att ctg gct gga gca aat atc tca ggt gat ctt gca gat cct cag tca      1712
Ile Leu Ala Gly Ala Asn Ile Ser Gly Asp Leu Ala Asp Pro Gln Ser
                505                 510                 515

gcc ata ccc aaa gga aca ctc cta gcc att tta att act aca ttg gtt      1760
Ala Ile Pro Lys Gly Thr Leu Leu Ala Ile Leu Ile Thr Thr Leu Val
            520                 525                 530

tac gta gga att gca gta tct gta ggt tct tgt gtt gtt cga gat gcc      1808
Tyr Val Gly Ile Ala Val Ser Val Gly Ser Cys Val Val Arg Asp Ala
        535                 540                 545

act gga aac gtt aat gac act atc gta aca gag cta aca aac tgt act      1856
Thr Gly Asn Val Asn Asp Thr Ile Val Thr Glu Leu Thr Asn Cys Thr
    550                 555                 560

tct gca gcc tgc aaa tta aac ttt gat ttt tca tct tgt gaa agc agt      1904
Ser Ala Ala Cys Lys Leu Asn Phe Asp Phe Ser Ser Cys Glu Ser Ser
565                 570                 575                 580

cct tgt tcc tat ggc cta atg aac aac ttc cag gta atg agt atg gtg      1952
Pro Cys Ser Tyr Gly Leu Met Asn Asn Phe Gln Val Met Ser Met Val
                585                 590                 595

tca gga ttt aca cca cta att tct gca ggt ata ttt tca gcc act ctt      2000

Ser Gly Phe Thr Pro Leu Ile Ser Ala Gly Ile Phe Ser Ala Thr Leu
            600                 605                 610

tct tca gca tta gca tcc cta gtg agt gct ccc aaa ata ttt cag gct      2048
Ser Ser Ala Leu Ala Ser Leu Val Ser Ala Pro Lys Ile Phe Gln Ala
        615                 620                 625

cta tgt aag gac aac atc tac cca gct ttc cag atg ttt gct aaa ggt      2096
Leu Cys Lys Asp Asn Ile Tyr Pro Ala Phe Gln Met Phe Ala Lys Gly
    630                 635                 640

tat ggg aaa aat aat gaa cct ctt cgt ggc tac atc tta aca ttc tta      2144
Tyr Gly Lys Asn Asn Glu Pro Leu Arg Gly Tyr Ile Leu Thr Phe Leu
645                 650                 655                 660

att gca ctt gga ttc atc tta att gct gaa ctg aat gtt att gca cca      2192
Ile Ala Leu Gly Phe Ile Leu Ile Ala Glu Leu Asn Val Ile Ala Pro
                665                 670                 675

att atc tca aac ttc ttc ctt gca tca tat gca ttg atc aat ttt tca      2240
Ile Ile Ser Asn Phe Phe Leu Ala Ser Tyr Ala Leu Ile Asn Phe Ser
            680                 685                 690

gta ttc cat gca tca ctt gca aaa tct cca gga tgg cgt cct gca ttc      2288
Val Phe His Ala Ser Leu Ala Lys Ser Pro Gly Trp Arg Pro Ala Phe
        695                 700                 705

aaa tac tac aac atg tgg ata tca ctt ctt gga gca att ctt tgt tgc      2336
Lys Tyr Tyr Asn Met Trp Ile Ser Leu Leu Gly Ala Ile Leu Cys Cys
    710                 715                 720

ata gta atg ttc gtc att aac tgg tgg gct gca ttg cta aca tat gtg      2384
Ile Val Met Phe Val Ile Asn Trp Trp Ala Ala Leu Leu Thr Tyr Val
725                 730                 735                 740

ata gtc ctt ggg ctg tat att tat gtt acc tac aaa aaa cca gat gtg      2432
Ile Val Leu Gly Leu Tyr Ile Tyr Val Thr Tyr Lys Lys Pro Asp Val
                745                 750                 755

aat tgg gga tcc tct aca caa gcc ctg act tac ctg aat gca ctg cag      2480
Asn Trp Gly Ser Ser Thr Gln Ala Leu Thr Tyr Leu Asn Ala Leu Gln
            760                 765                 770

cat tca att cgt ctt tct gga gtg gaa gac cac gtg aaa aac ttt agg      2528
His Ser Ile Arg Leu Ser Gly Val Glu Asp His Val Lys Asn Phe Arg
        775                 780                 785

cca cag tgt ctt gtt atg aca ggt gct cca aac tca cgt cca gct tta      2576
Pro Gln Cys Leu Val Met Thr Gly Ala Pro Asn Ser Arg Pro Ala Leu
    790                 795                 800

ctt cat ctt gtt cat gat ttc aca aaa aat gtt ggt ttg atg atc tgt      2624
Leu His Leu Val His Asp Phe Thr Lys Asn Val Gly Leu Met Ile Cys
805                 810                 815                 820

ggc cat gta cat atg ggt cct cga aga caa gcc atg aaa gag atg tcc      2672
Gly His Val His Met Gly Pro Arg Arg Gln Ala Met Lys Glu Met Ser
                825                 830                 835

atc gat caa gcc aaa tat cag cga tgg ctt att aag aac aaa atg aag      2720
Ile Asp Gln Ala Lys Tyr Gln Arg Trp Leu Ile Lys Asn Lys Met Lys
            840                 845                 850

gca ttt tat gct cca gta cat gca gat gac ttg aga gaa ggt gca cag 2768
Ala Phe Tyr Ala Pro Val His Ala Asp Asp Leu Arg Glu Gly Ala Gln
            855                 860                 865

tat ttg atg cag gct gct ggt ctt ggt cgt atg aag cca aac aca ctt 2816
Tyr Leu Met Gln Ala Ala Gly Leu Gly Arg Met Lys Pro Asn Thr Leu
        870                 875                 880

gtc ctt gga ttt aag aaa gat tgg ttg caa gca gat atg agg gat gtg 2864
Val Leu Gly Phe Lys Lys Asp Trp Leu Gln Ala Asp Met Arg Asp Val
885 890 895 900

gat atg tat ata aac tta ttt cat gat gct ttt gac ata caa tat gga 2912
Asp Met Tyr Ile Asn Leu Phe His Asp Ala Phe Asp Ile Gln Tyr Gly
905 910 915

gta gtg gtt att cgc cta aaa gaa ggt ctg gat ata tct cat ctt caa 2960
Val Val Val Ile Arg Leu Lys Glu Gly Leu Asp Ile Ser His Leu Gln
                920                 925                 930

gga caa gaa gaa tta ttg tca tca caa gag aaa tct cct ggc acc aag 3008
Gly Gln Glu Glu Leu Leu Ser Ser Gln Glu Lys Ser Pro Gly Thr Lys
            935                 940                 945

gat gtg gta gta agt gtg gaa tat agt aaa aag tcc gat tta gat act 3056
Asp Val Val Val Ser Val Glu Tyr Ser Lys Lys Ser Asp Leu Asp Thr
        950                 955                 960

tcc aaa cca ctc agt gaa aaa cca att aca cac aaa gtt gag gaa gag 3104
Ser Lys Pro Leu Ser Glu Lys Pro Ile Thr His Lys Val Glu Glu Glu
965 970 975 980

gat ggc aag act gca act caa cca ctg ttg aaa aaa gaa tcc aaa ggc 3152
Asp Gly Lys Thr Ala Thr Gln Pro Leu Leu Lys Lys Glu Ser Lys Gly
985 990 995

cct att gtg cct tta aat gta gct gac caa aag ctt ctt gaa gct 3197
Pro Ile Val Pro Leu Asn Val Ala Asp Gln Lys Leu Leu Glu Ala
                1000                1005                1010

agt aca cag ttt cag aaa aaa caa gga aag aat act att gat gtc 3242
Ser Thr Gln Phe Gln Lys Lys Gln Gly Lys Asn Thr Ile Asp Val
                1015                1020                1025

tgg tgg ctt ttt gat gat gga ggt ttg acc tta ttg ata cct tac 3287
Trp Trp Leu Phe Asp Asp Gly Gly Leu Thr Leu Leu Ile Pro Tyr
                1030                1035                1040

ctt ctg acg acc aag aaa aaa tgg aaa gac tgt aag atc aga gta 3332
Leu Leu Thr Thr Lys Lys Lys Trp Lys Asp Cys Lys Ile Arg Val
                1045                1050                1055

ttc att ggt gga aag ata aac aga ata gac cat gac cgg aga gcg 3377
Phe Ile Gly Gly Lys Ile Asn Arg Ile Asp His Asp Arg Arg Ala
                1060                1065                1070

atg gct act ttg ctt agc aag ttc cgg ata gac ttt tct gat atc 3422
Met Ala Thr Leu Leu Ser Lys Phe Arg Ile Asp Phe Ser Asp Ile
                1075                1080                1085

atg gtt cta gga gat atc aat acc aaa cca aag aaa gaa aat att 3467
Met Val Leu Gly Asp Ile Asn Thr Lys Pro Lys Lys Glu Asn Ile
                1090                1095                1100

ata gct ttt gag gaa atc att gag cca tac aga ctt cat gaa gat 3512
Ile Ala Phe Glu Glu Ile Ile Glu Pro Tyr Arg Leu His Glu Asp
                1105                1110                1115

gat aaa gag caa gat att gca gat aaa atg aaa gaa gat gaa cca 3557
Asp Lys Glu Gln Asp Ile Ala Asp Lys Met Lys Glu Asp Glu Pro
                1120                1125                1130

tgg cga ata aca gat aat gag ctt gaa ctt tat aag acc aag aca 3602
Trp Arg Ile Thr Asp Asn Glu Leu Glu Leu Tyr Lys Thr Lys Thr
                1135                1140                1145

tac cgg cag atc agg tta aat gag tta tta aag gaa cat tca agc 3647
Tyr Arg Gln Ile Arg Leu Asn Glu Leu Leu Lys Glu His Ser Ser
                1150                1155                1160

aca gct aat att att gtc atg agt ctc cca gtt gca cga aaa ggt 3692
Thr Ala Asn Ile Ile Val Met Ser Leu Pro Val Ala Arg Lys Gly
                1165                1170                1175

gct gtg tct agt gct ctc tac atg gca tgg tta gaa gct cta tct 3737
Ala Val Ser Ser Ala Leu Tyr Met Ala Trp Leu Glu Ala Leu Ser
                1180                1185                1190

aag gac cta cca cca atc ctc cta gtt cgt ggg aat cat cag agt 3782
Lys Asp Leu Pro Pro Ile Leu Leu Val Arg Gly Asn His Gln Ser
                1195                1200                1205

gtc ctt acc ttc tat tca taa atgttctata cagtggacag ccctccagaa 3833
Val Leu Thr Phe Tyr Ser
                1210

tggtacttca gtgcctagtg tagtaactga aatcttcaat gacacattaa catcacaatg 3893

gcgaatggtg acttttcttt cacgatttca ttaatttgaa agcacacagg aaagttgctc 3953

cattgataac gtgtatggag acttcggttt tagtcaattc catatctcaa tcttaatggt 4013

gattcttctc tgttgaactg aagtttgtga gagtagtttt cctttgctac ttgaatagca 4073

ataaaagcgt gttaactttt tgattgatga aagaagtaca aaaagccttt agccttgagg 4133

tgccttctga aattaaccaa atttcatcca tatatcctct tttataaact tatagaatgt 4193

caaactttgc cttcaactgt ttttatttct agtctcttcc actttaaaac aaaatgaaca 4253

ctgcttgtct tcttccattg accatttagt gttgagtact gtatgtgttt tgttaattct 4313

ataaaggtat ctgttagata ttaaaggtga gaattagggc aggttaatca aaaatgggga 4373

aggggaaatg gtaaccaaaa agtaacccca tggtaaggtt tatatgagta tatgtgaata 4433

tagagctagg aaaaaaagcc cccccaaata cctttttaac ccctctgatt ggctattatt 4493

actatattta ttattattta ttgaaacctt agggaagatt gaagattcat cccatacttc 4553

tatataccat gcttaaaaat cacgtcattc tttaaacaaa aatactcaag atcatttata 4613

tttatttgga gagaaaactg tcctaattta gaatttccct caaatctgag ggacttttaa 4673

gaaatgctaa cagatttttc tggaggaaat ttagacaaaa caatgtcatt tagtagaata 4733

tttcagtatt taagtggaat ttcagtatac tgtactatcc tttataagtc attaaaataa 4793

tgtttcatca aatggttaaa tggaccactg gtttcttaga gaaatgtttt taggcttaat 4853

tcattcaatt gtcaagtaca cttagtctta atacactcag gtttgaacag attattctga 4913

atattaaaat ttaatccatt cttaatattt taaaactttt gttaagaaaa actgccagtt 4973

tgtgcttttg aaatgtctgt tttgacatca tagtctagta aaattttgac agtgcatatg 5033

tactgttact aaaagcttta tatgaaatta ttaatgtgaa gtttttcatt tataattcaa 5093

ggaaggattt cctgaaaaca tttcaaggga tttatgtcta catatttgtg tgtgtgtgtg 5153

tatatatatg taatatgcat acacagatgc atatgtgtat atataatgaa atttatgttg 5213

ctggtatttt gcattttaaa gtgatcaaga ttcattaggc aaactttggt ttaagtaaac 5273

atatgttcaa aatcagatta acagatacag gtttcataga gaacaaaggt gatcatttga 5333

agggcatgct gtaatttcac acaattttcc agttcaaaaa tggagaatac ttcgcctaaa 5393

atactgttaa gtgggttaat tgatacaagt ttctgtggtg gaaaatttat gcaggtttcc 5453

acgaatcctt tttttttttt tttttttttt ttgagacgga gtcttgctct gttgccacgc 5513

tggaatgcag taacgtgatc ttggctcact gcgacctcca cctccccagt tcaagcgatt 5573

ctcctgcctc agcctcccta gtagctggga ctacgggtgc acgccaccat gcccagctaa 5633

tttttgtatt ttgagtagag acagggtttc accgtgttgg ctaggatggt gtctatctct 5693

tgaccttgtg atccacccgc ctcagcctcc cagagtgctg ggattacagg tgcgagccac 5753

tgcgcctggc tggttttcat gaatcttgat agacatctat aacgttatta ttttcagtgg 5813

tgtgcagcat ttttgcttca tgagtatgac ctaggtatag agatctgata acttgaattc 5873

agaatattaa gaaaatgaag taactgattt tctaaaaaaa aaaaaaaaaa aaatttctac 5933

attataactc acagcattgt tccattgcag gttttgcaat gtttgggggt aaagacagta 5993

gaaatattat tcagtaaaca ataatgtgtg aacttttaag atggataata gggcatggac 6053

tgagtgctgc tatcttgaaa tgtgcacagg tacacttacc tttttttttt ttttttttaa 6113

gtttttccca ttcaggaaaa caacattgtg atctgtacta caggaaccaa atgtcatgcg 6173

tcatacatgt gggtataaag tacataaaat atatctaact attcataatg tggggtgggt 6233

aatactgtct gtgaaataat gtaagaagct tttcacttaa aaaaaatgca ttactttcac 6293

ttaacactag acaccaggtc gaaaattttc aaggttatag tacttatttc aacaattctt 6353

agagatgcta gctagtgttg aagctaaaaa tagctttatt tatgctgaat tgtgattttt 6413

ttatgccaaa ttttttttag ttctaatcat tgatgatagc ttggaaataa ataattatgc 6473

catggcattt gacagttcat tattcctata agaattaaat tgagtttaga gagaatggtg 6533

gtgttgagct gattattaac agttactgaa atcaaatatt tatttgttac attattccat 6593

ttgtatttta ggtttccttt tacattcttt ttatatgcat tctgacatta catatttttt 6653

aagactatgg aaataattta aagatttaag ctctggtgga tgattatctg ctaagtaagt 6713

ctgaaaatgt aatattttga taatactgta atatacctgt cacacaaatg cttttctaat 6773

gttttaacct tgagtattgc agttgctgct ttgtacagag gttactgcaa taaaggaagt 6833

ggattcatta aacctattta atgtccaaaa aaaaaaaaaa aaaaaaaaaa aaaaaaa 6891

<210> 52
<211> 1212
<212> PRT
<213> Homo sapiens

<400> 52

<210> 53
<211> 6843
<212> DNA
<213> Homo sapiens

<220>
<221> CDS
<222> (165)..(3755)

<400> 53
   ggtggcctct gtggccgtcc aggctagcgg cggcccgcag gcggcgggga gaaagactct      60
   ctcacctggt cttgcggctg tggccaccgc cggccagggg tgtggagggc gtgctgccgg     120
   agacgtccgc cgggctctgc agttccgccg ggggtcgggc agct atg gag ccg cgg      176
                                                    Met Glu Pro Arg
                                                    1
   ccc acg gcg ccc tcc tcc ggc gcc ccg gga ctg gcc ggg gtc ggg gag      224
   Pro Thr Ala Pro Ser Ser Gly Ala Pro Gly Leu Ala Gly Val Gly Glu
   5                   10                  15                  20
   acg ccg tca gcc gct gcg ctg gcc gca gcc agg gtg gaa ctg ccc ggc      272
   Thr Pro Ser Ala Ala Ala Leu Ala Ala Ala Arg Val Glu Leu Pro Gly
                   25                  30                  35
   acg gct gtg ccc tcg gtg ccg gag gat gct gcg ccc gcg agc cgg gac      320
   Thr Ala Val Pro Ser Val Pro Glu Asp Ala Ala Pro Ala Ser Arg Asp
               40                  45                  50
   ggc ggc ggg gtc cgc gat gag ggc ccc gcg gcg gcc ggg gac ggg ctg      368
   Gly Gly Gly Val Arg Asp Glu Gly Pro Ala Ala Ala Gly Asp Gly Leu
           55                  60                  65
   ggc aga ccc ttg ggg ccc acc ccg agc cag agc cgt ttc cag gtg gac      416
   Gly Arg Pro Leu Gly Pro Thr Pro Ser Gln Ser Arg Phe Gln Val Asp
       70                  75                  80
   ctg gtt tcc gag aac gcc ggg cgg gcc gct gct gcg gcg gcg gcg gcg      464
   Leu Val Ser Glu Asn Ala Gly Arg Ala Ala Ala Ala Ala Ala Ala Ala
   85                  90                  95                  100
   gcg gcg gca gcg gcg gcg gct ggt gct ggg gcg ggg gcc aag cag acc      512
   Ala Ala Ala Ala Ala Ala Ala Gly Ala Gly Ala Gly Ala Lys Gln Thr
                   105                 110                 115
   ccc gcg gac ggg gaa gcc agc ggc gag agc gag ccg gct aaa ggc agc      560
   Pro Ala Asp Gly Glu Ala Ser Gly Glu Ser Glu Pro Ala Lys Gly Ser
               120                 125                 130
   gag gaa gcc aag ggc cgc ttc cgc gtg aac ttc gtg gac cca gct gcc      608
   Glu Glu Ala Lys Gly Arg Phe Arg Val Asn Phe Val Asp Pro Ala Ala
   135                 140                 145
   tcc tcg tcg gct gaa gac agc ctg tca gat gct gcc ggg gtc gga gtc      656
   Ser Ser Ser Ala Glu Asp Ser Leu Ser Asp Ala Ala Gly Val Gly Val
       150                 155                 160
   gac ggg ccc aac gtg agc ttc cag aac ggc ggg gac acg gtg ctg agc      704
   Asp Gly Pro Asn Val Ser Phe Gln Asn Gly Gly Asp Thr Val Leu Ser
   165                 170                 175                 180
   gag ggc agc agc ctg cac tcc ggc ggc ggc ggc ggc agt ggg cac cac        752
   Glu Gly Ser Ser Leu His Ser Gly Gly Gly Gly Gly Ser Gly His His
                   185                 190                 195
   cag cac tac tat tat gat acc cac acc aac acc tac tac ctg cgc acc      800
   Gln His Tyr Tyr Tyr Asp Thr His Thr Asn Thr Tyr Tyr Leu Arg Thr
               200                 205                 210
   ttc ggc cac aac acc atg gac gct gtg ccc agg atc gat cac tac cgg      848
   Phe Gly His Asn Thr Met Asp Ala Val Pro Arg Ile Asp His Tyr Arg
           215                 220                 225
   cac aca gcc gcg cag ctg ggc gag aag ctg ctc cgg cct agc ctg gcg      896
   His Thr Ala Ala Gln Leu Gly Glu Lys Leu Leu Arg Pro Ser Leu Ala
       230                 235                 240
   gag ctc cac gac gag ctg gaa aag gaa cct ttt gag gat ggc ttt gca      944
   Glu Leu His Asp Glu Leu Glu Lys Glu Pro Phe Glu Asp Gly Phe Ala
   245                 250                 255                 260
   aat ggg gaa gaa agt act cca acc aga gat gct gtg gtc acg tat act      992
   Asn Gly Glu Glu Ser Thr Pro Thr Arg Asp Ala Val Val Thr Tyr Thr
                   265                 270                 275
   gca gaa agt aaa gga gtc gtg aag ttt ggc tgg atc aag ggt gta tta      1040
   Ala Glu Ser Lys Gly Val Val Lys Phe Gly Trp Ile Lys Gly Val Leu
               280                 285                 290
   gta cgt tgt atg tta aac att tgg ggt gtg atg ctt ttc att aga ttg      1088
   Val Arg Cys Met Leu Asn Ile Trp Gly Val Met Leu Phe Ile Arg Leu
           295                 300                 305
   tca tgg att gtg ggt caa gct gga ata ggt cta tca gtc ctt gta ata      1136
   Ser Trp Ile Val Gly Gln Ala Gly Ile Gly Leu Ser Val Leu Val Ile
       310                 315                 320
   atg atg gcc act gtt gtg aca act atc aca gga ttg tct act tca gca      1184
   Met Met Ala Thr Val Val Thr Thr Ile Thr Gly Leu Ser Thr Ser Ala
   325                 330                 335                 340
   ata gca act aat gga ttt gta aga gga gga gga gca tat tat tta ata      1232
   Ile Ala Thr Asn Gly Phe Val Arg Gly Gly Gly Ala Tyr Tyr Leu Ile
                   345                 350                 355
   tct aga agt cta ggg cca gaa ttt ggt ggt gca att ggt cta atc ttc      1280
   Ser Arg Ser Leu Gly Pro Glu Phe Gly Gly Ala Ile Gly Leu Ile Phe
               360                 365                 370
   gcc ttt gcc aac gct gtt gca gtt gct atg tat gtg gtt gga ttt gca      1328
   Ala Phe Ala Asn Ala Val Ala Val Ala Met Tyr Val Val Gly Phe Ala
           375                 380                 385
   gaa acc gtg gtg gag ttg ctt aag gaa cat tcc ata ctt atg ata gat      1376
   Glu Thr Val Val Glu Leu Leu Lys Glu His Ser Ile Leu Met Ile Asp
       390                 395                 400
   gaa atc aat gat atc cga att att gga gcc att aca gtc gtg att ctt      1424
   Glu Ile Asn Asp Ile Arg Ile Ile Gly Ala Ile Thr Val Val Ile Leu
   405                 410                 415                 420
   tta ggt atc tca gta gct gga atg gag tgg gaa gca aaa gct cag att      1472
   Leu Gly Ile Ser Val Ala Gly Met Glu Trp Glu Ala Lys Ala Gln Ile
                   425                 430                 435
   gtt ctt ttg gtg atc cta ctt ctt gct att ggt gat ttc gtc ata gga      1520
   Val Leu Leu Val Ile Leu Leu Leu Ala Ile Gly Asp Phe Val Ile Gly
               440                 445                 450
   aca ttt atc cca ctg gag agc aag aag cca aaa ggg ttt ttt ggt tat      1568
   Thr Phe Ile Pro Leu Glu Ser Lys Lys Pro Lys Gly Phe Phe Gly Tyr
           455                 460                 465
   aaa tct gaa ata ttt aat gag aac ttt ggg ccc gat ttt cga gag gaa      1616
   Lys Ser Glu Ile Phe Asn Glu Asn Phe Gly Pro Asp Phe Arg Glu Glu
       470                 475                 480
   gag act ttc ttt tct gta ttt gcc atc ttt ttt cct gct gca act ggt      1664
   Glu Thr Phe Phe Ser Val Phe Ala Ile Phe Phe Pro Ala Ala Thr Gly
   485                 490                 495                 500
   att ctg gct gga gca aat atc tca ggt gat ctt gca gat cct cag tca      1712
   Ile Leu Ala Gly Ala Asn Ile Ser Gly Asp Leu Ala Asp Pro Gln Ser
                   505                 510                 515
   gcc ata ccc aaa gga aca ctc cta gcc att tta att act aca ttg gtt      1760
   Ala Ile Pro Lys Gly Thr Leu Leu Ala Ile Leu Ile Thr Thr Leu Val
               520                 525                 530
   tac gta gga att gca gta tct gta ggt tct tgt gtt gtt cga gat gcc      1808
   Tyr Val Gly Ile Ala Val Ser Val Gly Ser Cys Val Val Arg Asp Ala
           535                 540                 545
   act gga aac gtt aat gac act atc gta aca gag cta aca aac tgt act      1856
   Thr Gly Asn Val Asn Asp Thr Ile Val Thr Glu Leu Thr Asn Cys Thr
       550                 555                 560
   tct gca gcc tgc aaa tta aac ttt gat ttt tca tct tgt gaa agc agt      1904
   Ser Ala Ala Cys Lys Leu Asn Phe Asp Phe Ser Ser Cys Glu Ser Ser
   565                 570                 575                 580
   cct tgt tcc tat ggc cta atg aac aac ttc cag gta atg agt atg gtg      1952
   Pro Cys Ser Tyr Gly Leu Met Asn Asn Phe Gln Val Met Ser Met Val
                   585                 590                 595
   tca gga ttt aca cca cta att tct gca ggt ata ttt tca gcc act ctt      2000
   Ser Gly Phe Thr Pro Leu Ile Ser Ala Gly Ile Phe Ser Ala Thr Leu
               600                 605                 610
   tct tca gca tta gca tcc cta gtg agt gct ccc aaa ata ttt cag gct      2048
   Ser Ser Ala Leu Ala Ser Leu Val Ser Ala Pro Lys Ile Phe Gln Ala
           615                 620                 625
   cta tgt aag gac aac atc tac cca gct ttc cag atg ttt gct aaa ggt      2096
   Leu Cys Lys Asp Asn Ile Tyr Pro Ala Phe Gln Met Phe Ala Lys Gly
       630                 635                 640
   tat ggg aaa aat aat gaa cct ctt cgt ggc tac atc tta aca ttc tta 2144
   Tyr Gly Lys Asn Asn Glu Pro Leu Arg Gly Tyr Ile Leu Thr Phe Leu
   645 650 655 660
   att gca ctt gga ttc atc tta att gct gaa ctg aat gtt att gca cca 2192
   Ile Ala Leu Gly Phe Ile Leu Ile Ala Glu Leu Asn Val Ile Ala Pro
   665 670 675
   att atc tca aac ttc ttc ctt gca tca tat gca ttg atc aat ttt tca 2240
   Ile Ile Ser Asn Phe Phe Leu Ala Ser Tyr Ala Leu Ile Asn Phe Ser
   680 685 690
   gta ttc cat gca tca ctt gca aaa tct cca gga tgg cgt cct gca ttc 2288
   Val Phe His Ala Ser Leu Ala Lys Ser Pro Gly Trp Arg Pro Ala Phe
   695 700 705
   aaa tac tac aac atg tgg ata tca ctt ctt gga gca att ctt tgt tgc 2336
   Lys Tyr Tyr Asn Met Trp Ile Ser Leu Leu Gly Ala Ile Leu Cys Cys
   710 715 720
   ata gta atg ttc gtc att aac tgg tgg gct gca ttg cta aca tat gtg 2384
   Ile Val Met Phe Val Ile Asn Trp Trp Ala Ala Leu Leu Thr Tyr Val
   725 730 735 740
   ata gtc ctt ggg ctg tat att tat gtt acc tac aaa aaa cca gat gtg 2432
   Ile Val Leu Gly Leu Tyr Ile Tyr Val Thr Tyr Lys Lys Pro Asp Val
   745 750 755
   aat tgg gga tcc tct aca caa gcc ctg act tac ctg aat gca ctg cag 2480
   Asn Trp Gly Ser Ser Thr Gln Ala Leu Thr Tyr Leu Asn Ala Leu Gln
   760 765 770
   cat tca att cgt ctt tct gga gtg gaa gac cac gtg aaa aac ttt agg 2528
   His Ser Ile Arg Leu Ser Gly Val Glu Asp His Val Lys Asn Phe Arg
   775 780 785
   cca cag tgt ctt gtt atg aca ggt gct cca aac tca cgt cca gct tta 2576
   Pro Gln Cys Leu Val Met Thr Gly Ala Pro Asn Ser Arg Pro Ala Leu
   790 795 800
   ctt cat ctt gtt cat gat ttc aca aaa aat gtt ggt ttg atg atc tgt 2624
   Leu His Leu Val His Asp Phe Thr Lys Asn Val Gly Leu Met Ile Cys
   805 810 815 820
   ggc cat gta cat atg ggt cct cga aga caa gcc atg aaa gag atg tcc 2672
   Gly His Val His Met Gly Pro Arg Arg Gln Ala Met Lys Glu Met Ser
   825 830 835
   atc gat caa gcc aaa tat cag cga tgg ctt att aag aac aaa atg aag 2720
   Ile Asp Gln Ala Lys Tyr Gln Arg Trp Leu Ile Lys Asn Lys Met Lys
   840 845 850
   gca ttt tat gct cca gta cat gca gat gac ttg aga gaa ggt gca cag 2768
   Ala Phe Tyr Ala Pro Val His Ala Asp Asp Leu Arg Glu Gly Ala Gln
   855 860 865
   tat ttg atg cag gct gct ggt ctt ggt cgt atg aag cca aac aca ctt 2816
   Tyr Leu Met Gln Ala Ala Gly Leu Gly Arg Met Lys Pro Asn Thr Leu
   870 875 880
   gtc ctt gga ttt aag aaa gat tgg ttg caa gca gat atg agg gat gtg 2864
   Val Leu Gly Phe Lys Lys Asp Trp Leu Gln Ala Asp Met Arg Asp Val
   885 890 895 900
   gat atg tat ata aac tta ttt cat gat gct ttt gac ata caa tat gga 2912
   Asp Met Tyr Ile Asn Leu Phe His Asp Ala Phe Asp Ile Gln Tyr Gly
   905 910 915
   gta gtg gtt att cgc cta aaa gaa ggt ctg gat ata tct cat ctt caa 2960
   Val Val Val Ile Arg Leu Lys Glu Gly Leu Asp Ile Ser His Leu Gln
   920 925 930
   gga caa gaa gaa tta ttg tca tca caa gag aaa tct cct ggc acc aag 3008
   Gly Gln Glu Glu Leu Leu Ser Ser Gln Glu Lys Ser Pro Gly Thr Lys
   935 940 945
   gat gtg gta gta agt gtg gaa tat agt aaa aag tcc gat tta gat act 3056
   Asp Val Val Val Ser Val Glu Tyr Ser Lys Lys Ser Asp Leu Asp Thr
   950 955 960
   tcc aaa cca ctc agt gaa aaa cca att aca cac aaa gaa tcc aaa ggc 3104
   Ser Lys Pro Leu Ser Glu Lys Pro Ile Thr His Lys Glu Ser Lys Gly
   965 970 975 980
   cct att gtg cct tta aat gta gct gac caa aag ctt ctt gaa gct agt 3152
   Pro Ile Val Pro Leu Asn Val Ala Asp Gln Lys Leu Leu Glu Ala Ser
   985 990 995
   aca cag ttt cag aaa aaa caa gga aag aat act att gat gtc tgg 3197
   Thr Gln Phe Gln Lys Lys Gln Gly Lys Asn Thr Ile Asp Val Trp
   1000 1005 1010
   tgg ctt ttt gat gat gga ggt ttg acc tta ttg ata cct tac ctt 3242
   Trp Leu Phe Asp Asp Gly Gly Leu Thr Leu Leu Ile Pro Tyr Leu
   1015 1020 1025
   ctg acg acc aag aaa aaa tgg aaa gac tgt aag atc aga gta ttc 3287
   Leu Thr Thr Lys Lys Lys Trp Lys Asp Cys Lys Ile Arg Val Phe
   1030 1035 1040
   att ggt gga aag ata aac aga ata gac cat gac cgg aga gcg atg 3332
   Ile Gly Gly Lys Ile Asn Arg Ile Asp His Asp Arg Arg Ala Met
   1045 1050 1055
   gct act ttg ctt agc aag ttc cgg ata gac ttt tct gat atc atg 3377
   Ala Thr Leu Leu Ser Lys Phe Arg Ile Asp Phe Ser Asp Ile Met
   1060 1065 1070
   gtt cta gga gat atc aat acc aaa cca aag aaa gaa aat att ata 3422
   Val Leu Gly Asp Ile Asn Thr Lys Pro Lys Lys Glu Asn Ile Ile
   1075 1080 1085
   gct ttt gag gaa atc att gag cca tac aga ctt cat gaa gat gat 3467
   Ala Phe Glu Glu Ile Ile Glu Pro Tyr Arg Leu His Glu Asp Asp
   1090 1095 1100
   aaa gag caa gat att gca gat aaa atg aaa gaa gat gaa cca tgg 3512
   Lys Glu Gln Asp Ile Ala Asp Lys Met Lys Glu Asp Glu Pro Trp
   1105 1110 1115
   cga ata aca gat aat gag ctt gaa ctt tat aag acc aag aca tac 3557
   Arg Ile Thr Asp Asn Glu Leu Glu Leu Tyr Lys Thr Lys Thr Tyr
   1120 1125 1130
   cgg cag atc agg tta aat gag tta tta aag gaa cat tca agc aca 3602
   Arg Gln Ile Arg Leu Asn Glu Leu Leu Lys Glu His Ser Ser Thr
   1135                1140                1145
   gct aat att att gtc atg agt ctc cca gtt gca cga aaa ggt gct      3647
   Ala Asn Ile Ile Val Met Ser Leu Pro Val Ala Arg Lys Gly Ala
               1150                1155                1160
   gtg tct agt gct ctc tac atg gca tgg tta gaa gct cta tct aag      3692
   Val Ser Ser Ala Leu Tyr Met Ala Trp Leu Glu Ala Leu Ser Lys
               1165                1170                1175
   gac cta cca cca atc ctc cta gtt cgt ggg aat cat cag agt gtc      3737
   Asp Leu Pro Pro Ile Leu Leu Val Arg Gly Asn His Gln Ser Val
               1180                1185                1190
   ctt acc ttc tat tca taa atgttctata cagtggacag ccctccagaa         3785
   Leu Thr Phe Tyr Ser
               1195
   tggtacttca gtgcctagtg tagtaactga aatcttcaat gacacattaa catcacaatg    3845
   gcgaatggtg acttttcttt cacgatttca ttaatttgaa agcacacagg aaagttgctc    3905
   cattgataac gtgtatggag acttcggttt tagtcaattc catatctcaa tcttaatggt    3965
   gattcttctc tgttgaactg aagtttgtga gagtagtttt cctttgctac ttgaatagca    4025
   ataaaagcgt gttaactttt tgattgatga aagaagtaca aaaagccttt agccttgagg    4085
   tgccttctga aattaaccaa atttcatcca tatatcctct tttataaact tatagaatgt    4145
   caaactttgc cttcaactgt ttttatttct agtctcttcc actttaaaac aaaatgaaca    4205
   ctgcttgtct tcttccattg accatttagt gttgagtact gtatgtgttt tgttaattct    4265
   ataaaggtat ctgttagata ttaaaggtga gaattagggc aggttaatca aaaatgggga    4325
   aggggaaatg gtaaccaaaa agtaacccca tggtaaggtt tatatgagta tatgtgaata    4385
   tagagctagg aaaaaaagcc cccccaaata cctttttaac ccctctgatt ggctattatt    4445
   actatattta ttattattta ttgaaacctt agggaagatt gaagattcat cccatacttc    4505
   tatataccat gcttaaaaat cacgtcattc tttaaacaaa aatactcaag atcatttata    4565
   tttatttgga gagaaaactg tcctaattta gaatttccct caaatctgag ggacttttaa    4625
   gaaatgctaa cagatttttc tggaggaaat ttagacaaaa caatgtcatt tagtagaata    4685
   tttcagtatt taagtggaat ttcagtatac tgtactatcc tttataagtc attaaaataa    4745
   tgtttcatca aatggttaaa tggaccactg gtttcttaga gaaatgtttt taggcttaat    4805
   tcattcaatt gtcaagtaca cttagtctta atacactcag gtttgaacag attattctga    4865
   atattaaaat ttaatccatt cttaatattt taaaactttt gttaagaaaa actgccagtt    4925
   tgtgcttttg aaatgtctgt tttgacatca tagtctagta aaattttgac agtgcatatg    4985
   tactgttact aaaagcttta tatgaaatta ttaatgtgaa gtttttcatt tataattcaa    5045
   ggaaggattt cctgaaaaca tttcaaggga tttatgtcta catatttgtg tgtgtgtgtg    5105
   tatatatatg taatatgcat acacagatgc atatgtgtat atataatgaa atttatgttg    5165
   ctggtatttt gcattttaaa gtgatcaaga ttcattaggc aaactttggt ttaagtaaac 5225
   atatgttcaa aatcagatta acagatacag gtttcataga gaacaaaggt gatcatttga 5285
   agggcatgct gtaatttcac acaattttcc agttcaaaaa tggagaatac ttcgcctaaa 5345
   atactgttaa gtgggttaat tgatacaagt ttctgtggtg gaaaatttat gcaggttttc 5405
   acgaatcctt tttttttttt tttttttttt ttgagacgga gtcttgctct gttgccacgc 5465
   tggaatgcag taacgtgatc ttggctcact gcgacctcca cctccccagt tcaagcgatt 5525
   ctcctgcctc agcctcccta gtagctggga ctacgggtgc acgccaccat gcccagctaa 5585
   tttttgtatt ttgagtagag acagggtttc accgtgttgg ctaggatggt gtctatctct 5645
   tgaccttgtg atccacccgc ctcagcctcc cagagtgctg ggattacagg tgcgagccac 5705
   tgcgcctggc tggttttcat gaatcttgat agacatctat aacgttatta ttttcagtgg 5765
   tgtgcagcat ttttgcttca tgagtatgac ctaggtatag agatctgata acttgaattc 5825
   agaatattaa gaaaatgaag taactgattt tctaaaaaaa aaaaaaaaaa aaatttctac 5885
   attataactc acagcattgt tccattgcag gttttgcaat gtttgggggt aaagacagta 5945
   gaaatattat tcagtaaaca ataatgtgtg aacttttaag atggataata gggcatggac 6005
   tgagtgctgc tatcttgaaa tgtgcacagg tacacttacc tttttttttt ttttttttaa 6065
   gtttttccca ttcaggaaaa caacattgtg atctgtacta caggaaccaa atgtcatgcg 6125
   tcatacatgt gggtataaag tacataaaat atatctaact attcataatg tggggtgggt 6185
   aatactgtct gtgaaataat gtaagaagct tttcacttaa aaaaaatgca ttactttcac 6245
   ttaacactag acaccaggtc gaaaattttc aaggttatag tacttatttc aacaattctt 6305
   agagatgcta gctagtgttg aagctaaaaa tagctttatt tatgctgaat tgtgattttt 6365
   ttatgccaaa ttttttttag ttctaatcat tgatgatagc ttggaaataa ataattatgc 6425
   catggcattt gacagttcat tattcctata agaattaaat tgagtttaga gagaatggtg 6485
   gtgttgagct gattattaac agttactgaa atcaaatatt tatttgttac attattccat 6545
   ttgtatttta ggtttccttt tacattcttt ttatatgcat tctgacatta catatttttt 6605
   aagactatgg aaataattta aagatttaag ctctggtgga tgattatctg ctaagtaagt 6665
   ctgaaaatgt aatattttga taatactgta atatacctgt cacacaaatg cttttctaat 6725
   gttttaacct tgagtattgc agttgctgct ttgtacagag gttactgcaa taaaggaagt 6785
   ggattcatta aacctattta atgtccaaaa aaaaaaaaaa aaaaaaaaaa aaaaaaaa 6843

<210> 54
<211> 1196
<212> PRT
<213> Homo sapiens

<400>  54

Met Glu Pro Arg Pro Thr Ala Pro Ser Ser Gly Ala Pro Gly Leu Ala
1               5                   10                  15

Gly Val Gly Glu Thr Pro Ser Ala Ala Ala Leu Ala Ala Ala Arg Val
            20                  25                  30

Glu Leu Pro Gly Thr Ala Val Pro Ser Val Pro Glu Asp Ala Ala Pro
        35                  40                  45

Ala Ser Arg Asp Gly Gly Gly Val Arg Asp Glu Gly Pro Ala Ala Ala
    50                  55                  60

Gly Asp Gly Leu Gly Arg Pro Leu Gly Pro Thr Pro Ser Gln Ser Arg
65                  70                  75                  80

Phe Gln Val Asp Leu Val Ser Glu Asn Ala Gly Arg Ala Ala Ala Ala
                85                  90                  95

Ala Ala Ala Ala Ala Ala Ala Ala Ala Ala Ala Gly Ala Gly Ala Gly
            100                 105                 110

Ala Lys Gln Thr Pro Ala Asp Gly Glu Ala Ser Gly Glu Ser Glu Pro
        115                 120                 125

Ala Lys Gly Ser Glu Glu Ala Lys Gly Arg Phe Arg Val Asn Phe Val
    130                 135                 140

Asp Pro Ala Ala Ser Ser Ser Ala Glu Asp Ser Leu Ser Asp Ala Ala
145                 150                 155                 160

Gly Val Gly Val Asp Gly Pro Asn Val Ser Phe Gln Asn Gly Gly Asp
                165                 170                 175

Thr Val Leu Ser Glu Gly Ser Ser Leu His Ser Gly Gly Gly Gly Gly
            180                 185                 190

Ser Gly His His Gln His Tyr Tyr Tyr Asp Thr His Thr Asn Thr Tyr
        195                 200                 205

Tyr Leu Arg Thr Phe Gly His Asn Thr Met Asp Ala Val Pro Arg Ile
    210                 215                 220

Asp His Tyr Arg His Thr Ala Ala Gln Leu Gly Glu Lys Leu Leu Arg
225                 230                 235                 240

Pro Ser Leu Ala Glu Leu His Asp Glu Leu Glu Lys Glu Pro Phe Glu

245                 250                 255

Asp Gly Phe Ala Asn Gly Glu Glu Ser Thr Pro Thr Arg Asp Ala Val
            260                 265                 270

Val Thr Tyr Thr Ala Glu Ser Lys Gly Val Val Lys Phe Gly Trp Ile
        275                 280                 285

Lys Gly Val Leu Val Arg Cys Met Leu Asn Ile Trp Gly Val Met Leu
    290                 295                 300

Phe Ile Arg Leu Ser Trp Ile Val Gly Gln Ala Gly Ile Gly Leu Ser
305                 310                 315                 320

Val Leu Val Ile Met Met Ala Thr Val Val Thr Thr Ile Thr Gly Leu
                325                 330                 335

Ser Thr Ser Ala Ile Ala Thr Asn Gly Phe Val Arg Gly Gly Gly Ala
            340                 345                 350

Tyr Tyr Leu Ile Ser Arg Ser Leu Gly Pro Glu Phe Gly Gly Ala Ile
        355                 360                 365

Gly Leu Ile Phe Ala Phe Ala Asn Ala Val Ala Val Ala Met Tyr Val
    370                 375                 380

Val Gly Phe Ala Glu Thr Val Val Glu Leu Leu Lys Glu His Ser Ile
385                 390                 395                 400

Leu Met Ile Asp Glu Ile Asn Asp Ile Arg Ile Ile Gly Ala Ile Thr
                405                 410                 415

Val Val Ile Leu Leu Gly Ile Ser Val Ala Gly Met Glu Trp Glu Ala
            420                 425                 430

Lys Ala Gln Ile Val Leu Leu Val Ile Leu Leu Leu Ala Ile Gly Asp
        435                 440                 445

Phe Val Ile Gly Thr Phe Ile Pro Leu Glu Ser Lys Lys Pro Lys Gly
    450                 455                 460

Phe Phe Gly Tyr Lys Ser Glu Ile Phe Asn Glu Asn Phe Gly Pro Asp
465                 470                 475                 480

Phe Arg Glu Glu Glu Thr Phe Phe Ser Val Phe Ala Ile Phe Phe Pro
                485                 490                 495

Ala Ala Thr Gly Ile Leu Ala Gly Ala Asn Ile Ser Gly Asp Leu Ala
        500                 505                 510

Asp Pro Gln Ser Ala Ile Pro Lys Gly Thr Leu Leu Ala Ile Leu Ile
    515                 520                 525

Thr Thr Leu Val Tyr Val Gly Ile Ala Val Ser Val Gly Ser Cys Val
    530                 535                 540

Val Arg Asp Ala Thr Gly Asn Val Asn Asp Thr Ile Val Thr Glu Leu
545                 550                 555                 560

Thr Asn Cys Thr Ser Ala Ala Cys Lys Leu Asn Phe Asp Phe Ser Ser
                565                 570                 575

Cys Glu Ser Ser Pro Cys Ser Tyr Gly Leu Met Asn Asn Phe Gln Val
        580                 585                 590

Met Ser Met Val Ser Gly Phe Thr Pro Leu Ile Ser Ala Gly Ile Phe
    595                 600                 605

Ser Ala Thr Leu Ser Ser Ala Leu Ala Ser Leu Val Ser Ala Pro Lys
    610                 615                 620

Ile Phe Gln Ala Leu Cys Lys Asp Asn Ile Tyr Pro Ala Phe Gln Met
625                 630                 635                 640

Phe Ala Lys Gly Tyr Gly Lys Asn Asn Glu Pro Leu Arg Gly Tyr Ile
                645                 650                 655

Leu Thr Phe Leu Ile Ala Leu Gly Phe Ile Leu Ile Ala Glu Leu Asn
        660                 665                 670

Val Ile Ala Pro Ile Ile Ser Asn Phe Phe Leu Ala Ser Tyr Ala Leu
    675                 680                 685

Ile Asn Phe Ser Val Phe His Ala Ser Leu Ala Lys Ser Pro Gly Trp
    690                 695                 700

Arg Pro Ala Phe Lys Tyr Tyr Asn Met Trp Ile Ser Leu Leu Gly Ala
705                 710                 715                 720

Ile Leu Cys Cys Ile Val Met Phe Val Ile Asn Trp Trp Ala Ala Leu
                725                 730                 735

Leu Thr Tyr Val Ile Val Leu Gly Leu Tyr Ile Tyr Val Thr Tyr Lys
        740                 745                 750

Lys Pro Asp Val Asn Trp Gly Ser Ser Thr Gln Ala Leu Thr Tyr Leu
        755                 760                 765

Asn Ala Leu Gln His Ser Ile Arg Leu Ser Gly Val Glu Asp His Val
    770                 775                 780

Lys Asn Phe Arg Pro Gln Cys Leu Val Met Thr Gly Ala Pro Asn Ser
785                 790                 795                 800

Arg Pro Ala Leu Leu His Leu Val His Asp Phe Thr Lys Asn Val Gly
                805                 810                 815

Leu Met Ile Cys Gly His Val His Met Gly Pro Arg Arg Gln Ala Met
            820                 825                 830

Lys Glu Met Ser Ile Asp Gln Ala Lys Tyr Gln Arg Trp Leu Ile Lys
        835                 840                 845

Asn Lys Met Lys Ala Phe Tyr Ala Pro Val His Ala Asp Asp Leu Arg
    850                 855                 860

Glu Gly Ala Gln Tyr Leu Met Gln Ala Ala Gly Leu Gly Arg Met Lys
865                 870                 875                 880

Pro Asn Thr Leu Val Leu Gly Phe Lys Lys Asp Trp Leu Gln Ala Asp
                885                 890                 895

Met Arg Asp Val Asp Met Tyr Ile Asn Leu Phe His Asp Ala Phe Asp
            900                 905                 910

Ile Gln Tyr Gly Val Val Val Ile Arg Leu Lys Glu Gly Leu Asp Ile
        915                 920                 925

Ser His Leu Gln Gly Gln Glu Glu Leu Leu Ser Ser Gln Glu Lys Ser
    930                 935                 940

Pro Gly Thr Lys Asp Val Val Val Ser Val Glu Tyr Ser Lys Lys Ser
945                 950                 955                 960

Asp Leu Asp Thr Ser Lys Pro Leu Ser Glu Lys Pro Ile Thr His Lys
                965                 970                 975

Glu Ser Lys Gly Pro Ile Val Pro Leu Asn Val Ala Asp Gln Lys Leu
            980                 985                 990

Leu Glu Ala Ser Thr Gln Phe Gln  Lys Lys Gln Gly Lys  Asn Thr Ile
        995                 1000                1005

Asp Val Trp Trp Leu Phe Asp Asp Gly Gly Leu Thr Leu Leu Ile
    1010                1015                1020

Pro Tyr Leu Leu Thr Thr Lys Lys Lys Trp Lys Asp Cys Lys Ile
    1025                1030                1035

Arg Val Phe Ile Gly Gly Lys Ile Asn Arg Ile Asp His Asp Arg
    1040                1045                1050

Arg Ala Met Ala Thr Leu Leu Ser Lys Phe Arg Ile Asp Phe Ser
    1055                1060                1065

Asp Ile Met Val Leu Gly Asp Ile Asn Thr Lys Pro Lys Lys Glu
    1070                1075                1080

Asn Ile Ile Ala Phe Glu Glu Ile Ile Glu Pro Tyr Arg Leu His
    1085                1090                1095

Glu Asp Asp Lys Glu Gln Asp Ile Ala Asp Lys Met Lys Glu Asp
    1100                1105                1110

Glu Pro Trp Arg Ile Thr Asp Asn Glu Leu Glu Leu Tyr Lys Thr
    1115                1120                1125

Lys Thr Tyr Arg Gln Ile Arg Leu Asn Glu Leu Leu Lys Glu His
    1130                1135                1140

Ser Ser Thr Ala Asn Ile Ile Val Met Ser Leu Pro Val Ala Arg
    1145                1150                1155

Lys Gly Ala Val Ser Ser Ala Leu Tyr Met Ala Trp Leu Glu Ala
    1160                1165                1170

Leu Ser Lys Asp Leu Pro Pro Ile Leu Leu Val Arg Gly Asn His
    1175                1180                1185

Gln Ser Val Leu Thr Phe Tyr Ser
    1190                1195

<210>  55
<211>  3939
<212>  DNA
<213>  Homo sapiens

<220>
<221>  CDS
<222>  (231)..(2783)

<400> 55
   atcctcctcc aggtcctggc gcacagggtg ggagcgctgc gctgcgccgc gctgcgcatc 60
   gcggcccgct tgccgcctgc cccctgccct agctgggcca cctccccggg ctgccggtgg 120
   agggctaaga ggcgctaacg ttacgctgtt tccggttttc cagcgggctc tgtttcccct 180
   cccaaggcgg cggcggctga gcggcggagc cccccaaatg gcctggccag atg cgg 236
   Met Arg
   1
   cag gtt tgc tgc tca gcg ctg ccg ccg ccg cca ctg gag aag ggt cgg 284
   Gln Val Cys Cys Ser Ala Leu Pro Pro Pro Pro Leu Glu Lys Gly Arg
   5 10 15
   tgc agc agc tac agc gac agc agc agc agc agc agc gag agg agc agc 332
   Cys Ser Ser Tyr Ser Asp Ser Ser Ser Ser Ser Ser Glu Arg Ser Ser
   20 25 30
   agc agc agc agc agc agc agc gag agc ggc agc agc agc agg agc agc 380
   Ser Ser Ser Ser Ser Ser Ser Glu Ser Gly Ser Ser Ser Arg Ser Ser
   35 40 45 50
   agc aac aac agc agc atc tct cgt ccc gct gcg ccc cca gag ccg cgg 428
   Ser Asn Asn Ser Ser Ile Ser Arg Pro Ala Ala Pro Pro Glu Pro Arg
   55 60 65
   ccg cag caa cag ccg cag ccc cgc agc ccc gca gcc cgg aga gcc gcc 476
   Pro Gln Gln Gln Pro Gln Pro Arg Ser Pro Ala Ala Arg Arg Ala Ala
   70 75 80
   gcc cgt tcg cga gcc gca gcc gcc ggc ggc atg agg cgc gac ccg gcc 524
   Ala Arg Ser Arg Ala Ala Ala Ala Gly Gly Met Arg Arg Asp Pro Ala
   85 90 95
   ccc ggc ttc tcc atg ctg ctc ttc ggt gtg tcg ctc gcc tgc tac tcg 572
   Pro Gly Phe Ser Met Leu Leu Phe Gly Val Ser Leu Ala Cys Tyr Ser
   100 105 110
   ccc agc ctc aag tca gtg cag gac cag gcg tac aag gca ccc gtg gtg 620
   Pro Ser Leu Lys Ser Val Gln Asp Gln Ala Tyr Lys Ala Pro Val Val
   115 120 125 130
   gtg gag ggc aag gta cag ggg ctg gtc cca gcc ggc ggc tcc agc tcc 668
   Val Glu Gly Lys Val Gln Gly Leu Val Pro Ala Gly Gly Ser Ser Ser
   135 140 145
   aac agc acc cga gag ccg ccc gcc tcg ggt cgg gtg gcg ttg gta aag 716
   Asn Ser Thr Arg Glu Pro Pro Ala Ser Gly Arg Val Ala Leu Val Lys
   150 155 160
   gtg ctg gac aag tgg ccg ctc cgg agc ggg ggg ctg cag cgc gag cag 764
   Val Leu Asp Lys Trp Pro Leu Arg Ser Gly Gly Leu Gln Arg Glu Gln
   165 170 175
   gtg atc agc gtg ggc tcc tgt gtg ccg ctc gaa agg aac cag cgc tac 812
   Val Ile Ser Val Gly Ser Cys Val Pro Leu Glu Arg Asn Gln Arg Tyr
   180 185 190
   atc ttt ttc ctg gag ccc acg gaa cag ccc tta gtc ttt aag acg gcc 860
   Ile Phe Phe Leu Glu Pro Thr Glu Gln Pro Leu Val Phe Lys Thr Ala
   195 200 205 210
   ttt gcc ccc ctc gat acc aac ggc aaa aat ctc aag aaa gag gtg ggc 908
   Phe Ala Pro Leu Asp Thr Asn Gly Lys Asn Leu Lys Lys Glu Val Gly
   215 220 225
   aag atc ctg tgc act gac tgc gcc acc cgg ccc aag ttg aag aag atg 956
   Lys Ile Leu Cys Thr Asp Cys Ala Thr Arg Pro Lys Leu Lys Lys Met
   230 235 240
   aag agc cag acg gga cag gtg ggt gag aag caa tcg ctg aag tgt gag 1004
   Lys Ser Gln Thr Gly Gln Val Gly Glu Lys Gln Ser Leu Lys Cys Glu
   245 250 255
   gca gca gcc ggt aat ccc cag cct tcc tac cgt tgg ttc aag gat ggc 1052
   Ala Ala Ala Gly Asn Pro Gln Pro Ser Tyr Arg Trp Phe Lys Asp Gly
   260 265 270
   aag gag ctc aac cgc agc cga gac att cgc atc aaa tat ggc aac ggc 1100
   Lys Glu Leu Asn Arg Ser Arg Asp Ile Arg Ile Lys Tyr Gly Asn Gly
   275 280 285 290
   aga aag aac tca cga cta cag ttc aac aag gtg aag gtg gag gac gct 1148
   Arg Lys Asn Ser Arg Leu Gln Phe Asn Lys Val Lys Val Glu Asp Ala
   295 300 305
   ggg gag tat gtc tgc gag gcc gag aac atc ctg ggg aag gac acc gtc 1196
   Gly Glu Tyr Val Cys Glu Ala Glu Asn Ile Leu Gly Lys Asp Thr Val
   310 315 320
   cgg ggc cgg ctt tac gtc aac agc gtg agc acc acc ctg tca tcc tgg 1244
   Arg Gly Arg Leu Tyr Val Asn Ser Val Ser Thr Thr Leu Ser Ser Trp
   325 330 335
   tcg ggg cac gcc cgg aag tgc aac gag aca gcc aag tcc tat tgc gtc 1292
   Ser Gly His Ala Arg Lys Cys Asn Glu Thr Ala Lys Ser Tyr Cys Val
   340 345 350
   aat gga ggc gtc tgc tac tac atc gag ggc atc aac cag ctc tcc tgc 1340
   Asn Gly Gly Val Cys Tyr Tyr Ile Glu Gly Ile Asn Gln Leu Ser Cys
   355 360 365 370
   aaa tgt cca aat gga ttc ttc gga cag aga tgt ttg gag aaa ctg cct 1388
   Lys Cys Pro Asn Gly Phe Phe Gly Gln Arg Cys Leu Glu Lys Leu Pro
   375 380 385
   ttg cga ttg tac atg cca gat cct aag caa aaa gcc gag gag ctg tac 1436
   Leu Arg Leu Tyr Met Pro Asp Pro Lys Gln Lys Ala Glu Glu Leu Tyr
   390 395 400
   cag aag agg gtc ctg acc atc acg ggc atc tgc gtg gct ctg ctg gtc 1484
   Gln Lys Arg Val Leu Thr Ile Thr Gly Ile Cys Val Ala Leu Leu Val
   405 410 415
   gtg ggc atc gtc tgt gtg gtg gcc tac tgc aag acc aaa aaa cag cgg 1532
   Val Gly Ile Val Cys Val Val Ala Tyr Cys Lys Thr Lys Lys Gln Arg
   420 425 430
   aag cag atg cac aac cac ctc cgg cag aac atg tgc ccg gcc cat cag 1580
   Lys Gln Met His Asn His Leu Arg Gln Asn Met Cys Pro Ala His Gln
   435 440 445 450
   aac cgg agc ttg gcc aat ggg ccc agc cac ccc cgg ctg gac cca gag 1628
   Asn Arg Ser Leu Ala Asn Gly Pro Ser His Pro Arg Leu Asp Pro Glu
   455                 460                 465
   gag atc cag atg gca gat tat att tcc aag aac gtg cca gcc aca gac      1676
   Glu Ile Gln Met Ala Asp Tyr Ile Ser Lys Asn Val Pro Ala Thr Asp
               470                 475                 480
   cat gtc atc agg aga gaa act gag acc acc ttc tct ggg agc cac tcc      1724
   His Val Ile Arg Arg Glu Thr Glu Thr Thr Phe Ser Gly Ser His Ser
           485                 490                 495
   tgt tct cct tct cac cac tgc tcc aca gcc aca ccc acc tcc agc cac      1772
   Cys Ser Pro Ser His His Cys Ser Thr Ala Thr Pro Thr Ser Ser His
       500                 505                 510
   aga cac gag agc cac acg tgg agc ctg gaa cgt tct gag agc ctg act      1820
   Arg His Glu Ser His Thr Trp Ser Leu Glu Arg Ser Glu Ser Leu Thr
   515                 520                 525                 530
   tct gac tcc cag tcg ggg atc atg cta tca tca gtg ggt acc agc aaa      1868
   Ser Asp Ser Gln Ser Gly Ile Met Leu Ser Ser Val Gly Thr Ser Lys
                   535                 540                 545
   tgc aac agc cca gca tgt gtg gag gcc cgg gca agg cgg gca gca gcc      1916
   Cys Asn Ser Pro Ala Cys Val Glu Ala Arg Ala Arg Arg Ala Ala Ala
               550                 555                 560
   tac aac ctg gag gag cgg cgc agg gcc acc gcg cca ccc tat cac gat      1964
   Tyr Asn Leu Glu Glu Arg Arg Arg Ala Thr Ala Pro Pro Tyr His Asp
           565                 570                 575
   tcc gtg gac tcc ctt cgc gac tcc cca cac agc gag agg tac gtg tcg      2012
   Ser Val Asp Ser Leu Arg Asp Ser Pro His Ser Glu Arg Tyr Val Ser
       580                 585                 590
   gcc ctg acc acg ccc gcg cgc ctc tcg ccc gtg gac ttc cac tac tcg      2060
   Ala Leu Thr Thr Pro Ala Arg Leu Ser Pro Val Asp Phe His Tyr Ser
   595                 600                 605                 610
   ctg gcc acg cag gtg cca act ttc gag atc acg tcc ccc aac tcg gcg      2108
   Leu Ala Thr Gln Val Pro Thr Phe Glu Ile Thr Ser Pro Asn Ser Ala
                   615                 620                 625
   cac gcc gtg tcg ctg ccg ccg gcg gcg ccc atc agt tac cgc ctg gcc      2156
   His Ala Val Ser Leu Pro Pro Ala Ala Pro Ile Ser Tyr Arg Leu Ala
               630                 635                 640
   gag cag cag ccg tta ctg cgg cac ccg gcg ccc ccc ggc ccg gga ccc      2204
   Glu Gln Gln Pro Leu Leu Arg His Pro Ala Pro Pro Gly Pro Gly Pro
           645                 650                 655
   gga ccc ggg ccc ggg ccc ggg ccc ggc gca gac atg cag cgc agc tat      2252
   Gly Pro Gly Pro Gly Pro Gly Pro Gly Ala Asp Met Gln Arg Ser Tyr
       660                 665                 670
   gac agc tac tat tac ccc gcg gcg ggg ccc gga ccg cgg cgc ggg acc      2300
   Asp Ser Tyr Tyr Tyr Pro Ala Ala Gly Pro Gly Pro Arg Arg Gly Thr
   675                 680                 685                 690
   tgc gcg ctc ggc ggc agc ctg ggc agc ctg cct gcc agc ccc ttc cgc      2348
   Cys Ala Leu Gly Gly Ser Leu Gly Ser Leu Pro Ala Ser Pro Phe Arg
                   695                 700                 705
   atc ccc gag gac gac gag tac gag acc acg cag gag tgc gcg ccc ccg      2396
   Ile Pro Glu Asp Asp Glu Tyr Glu Thr Thr Gln Glu Cys Ala Pro Pro
               710                 715                 720
   ccg ccg ccg cgg ccg cgc gcg cgc ggt gcg tcc cgc agg acg tcg gcg      2444
   Pro Pro Pro Arg Pro Arg Ala Arg Gly Ala Ser Arg Arg Thr Ser Ala
           725                 730                 735
   ggg ccc cgg cgc tgg cgc cgc tcg cgc ctc aac ggg ctg gcg gcg cag      2492
   Gly Pro Arg Arg Trp Arg Arg Ser Arg Leu Asn Gly Leu Ala Ala Gln
       740                 745                 750
   cgc gca cgg gcg gcg agg gac tcg ctg tcg ctg agc agc ggc tcg ggc      2540
   Arg Ala Arg Ala Ala Arg Asp Ser Leu Ser Leu Ser Ser Gly Ser Gly
   755                 760                 765                 770
   ggc ggc tca gcc tcg gcg tcg gac gac gac gcg gac gac gcg gac ggg      2588
   Gly Gly Ser Ala Ser Ala Ser Asp Asp Asp Ala Asp Asp Ala Asp Gly
                   775                 780                 785
   gcg ctg gcg gcc gag agc aca cct ttc ctg ggc ctg cgt ggg gcg cac      2636
   Ala Leu Ala Ala Glu Ser Thr Pro Phe Leu Gly Leu Arg Gly Ala His
               790                 795                 800
   gac gcg ctg cgc tcg gac tcg ccg cca ctg tgc ccg gcg gcc gac agc      2684
   Asp Ala Leu Arg Ser Asp Ser Pro Pro Leu Cys Pro Ala Ala Asp Ser
           805                 810                 815
   agg act tac tac tca ctg gac agc cac agc acg cgg gcc agc agc aga      2732
   Arg Thr Tyr Tyr Ser Leu Asp Ser His Ser Thr Arg Ala Ser Ser Arg
       820                 825                 830
   cac agc cgc ggg ccg ccc ccg cgg gcc aag cag gac tcg gcg cca ctc      2780
   His Ser Arg Gly Pro Pro Pro Arg Ala Lys Gln Asp Ser Ala Pro Leu
   835                 840                 845                 850
   tag ggccccgccg cgcgcccctc cgccccgccc gccccactat ctttaaggag      2833
   accagagacc gcctactgga gagaaaggag gaaaaaagaa ataaaaatat ttttattttc      2893
   tataaaagga aaaaagtata acaaaatgtt ttattttcat tttagcaaaa attgtcttat      2953
   aatactagct aacggcaaag gcgtttttat agggaaacta tttatatgta acatcctgat      3013
   ttacagcttc ggaaaaaaaa aagaaacaac aaaaaaaaaa gagagatggg ccaatttttt      3073
   gactctttaa tagaaaccta ttttgtggtg ccttttgctg tatgctcatc tggggctccg      3133
   ggggacaggt ctgggttgtg gggtgctgag agtgacagct gggagagaag aggtgcgggt      3193
   tctgttgggg gcgagggtgg ggggcggtgg ggggttggcc ctaaggaaga actgaggacg      3253
   gaagtagctt tgctcccagg caagagctga agttcccgac ccctgggagg agagggccag      3313
   gaccctaggc ttggagctgg agctggggta acttccaggg ggagataaag acacagctac      3373
   ccgaatgggg aagccctggc tactcacagc tgttaaagga gggggggcag agaaacgttt      3433
   ccccaagagc ctgagcccag ggaggtggca gctctgccag gggcccaatg ttggcggctc      3493
   ctgtccccca cagcctccag gacgcccctc cgtcctctgc agcaccttcg tttacaggtc      3553
   gtcttttcta ttttacacct gcatgtcctt tgcatttcag attctttaga ttggatgcat      3613
   ggtcacgctg ggaccgagaa gagccactcg acagtgtatt ccccttttag caataaagta 3673
   acaccatttc cttctcacag gctgcctcct agcccccaac ccacctatga catccactcg 3733
   tctccagtcc tgccctccca cccccaaaaa ctgggtccac tgctaatttg tcaaaaaggt 3793
   aattgtttaa aaaaaaaaaa aatcccaaac agaaacagat gggaatcctg tcctgaaatg 3853
   tctgaacacc tgactgttga cacttgaaca tttttatatt ataaataaaa tcagaaataa 3913
   ctcctgttaa aaaaaaaaaa aaaaaa 3939

<210> 56
<211> 850
<212> PRT
<213> Homo sapiens

<400> 56
   Met Arg Gln Val Cys Cys Ser Ala Leu Pro Pro Pro Pro Leu Glu Lys
   1 5 10 15
   Gly Arg Cys Ser Ser Tyr Ser Asp Ser Ser Ser Ser Ser Ser Glu Arg
   20 25 30
   Ser Ser Ser Ser Ser Ser Ser Ser Ser Glu Ser Gly Ser Ser Ser Arg
   35 40 45
   Ser Ser Ser Asn Asn Ser Ser Ile Ser Arg Pro Ala Ala Pro Pro Glu
   50 55 60
   Pro Arg Pro Gln Gln Gln Pro Gln Pro Arg Ser Pro Ala Ala Arg Arg
   65 70 75 80
   Ala Ala Ala Arg Ser Arg Ala Ala Ala Ala Gly Gly Met Arg Arg Asp
   85 90 95
   Pro Ala Pro Gly Phe Ser Met Leu Leu Phe Gly Val Ser Leu Ala Cys
   100 105 110
   Tyr Ser Pro Ser Leu Lys Ser Val Gln Asp Gln Ala Tyr Lys Ala Pro
   115 120 125
   Val Val Val Glu Gly Lys Val Gln Gly Leu Val Pro Ala Gly Gly Ser
   130 135 140
   Ser Ser Asn Ser Thr Arg Glu Pro Pro Ala Ser Gly Arg Val Ala Leu
   145 150 155 160
   Val Lys Val Leu Asp Lys Trp Pro Leu Arg Ser Gly Gly Leu Gln Arg
   165 170 175
   Glu Gln Val Ile Ser Val Gly Ser Cys Val Pro Leu Glu Arg Asn Gln
               180                 185                 190
   Arg Tyr Ile Phe Phe Leu Glu Pro Thr Glu Gln Pro Leu Val Phe Lys
           195                 200                 205
   Thr Ala Phe Ala Pro Leu Asp Thr Asn Gly Lys Asn Leu Lys Lys Glu
       210                 215                 220
   Val Gly Lys Ile Leu Cys Thr Asp Cys Ala Thr Arg Pro Lys Leu Lys
   225                 230                 235                 240
   Lys Met Lys Ser Gln Thr Gly Gln Val Gly Glu Lys Gln Ser Leu Lys
                   245                 250                 255
   Cys Glu Ala Ala Ala Gly Asn Pro Gln Pro Ser Tyr Arg Trp Phe Lys
               260                 265                 270
   Asp Gly Lys Glu Leu Asn Arg Ser Arg Asp Ile Arg Ile Lys Tyr Gly
           275                 280                 285
   Asn Gly Arg Lys Asn Ser Arg Leu Gln Phe Asn Lys Val Lys Val Glu
       290                 295                 300
   Asp Ala Gly Glu Tyr Val Cys Glu Ala Glu Asn Ile Leu Gly Lys Asp
   305                 310                 315                 320
   Thr Val Arg Gly Arg Leu Tyr Val Asn Ser Val Ser Thr Thr Leu Ser
                   325                 330                 335
   Ser Trp Ser Gly His Ala Arg Lys Cys Asn Glu Thr Ala Lys Ser Tyr
               340                 345                 350
   Cys Val Asn Gly Gly Val Cys Tyr Tyr Ile Glu Gly Ile Asn Gln Leu
           355                 360                 365
   Ser Cys Lys Cys Pro Asn Gly Phe Phe Gly Gln Arg Cys Leu Glu Lys
       370                 375                 380
   Leu Pro Leu Arg Leu Tyr Met Pro Asp Pro Lys Gln Lys Ala Glu Glu
   385                 390                 395                 400
   Leu Tyr Gln Lys Arg Val Leu Thr Ile Thr Gly Ile Cys Val Ala Leu
                   405                 410                 415
   Leu Val Val Gly Ile Val Cys Val Val Ala Tyr Cys Lys Thr Lys Lys
               420                 425                 430
   Gln Arg Lys Gln Met His Asn His Leu Arg Gln Asn Met Cys Pro Ala
           435                 440                 445
   His Gln Asn Arg Ser Leu Ala Asn Gly Pro Ser His Pro Arg Leu Asp
       450                 455                 460
   Pro Glu Glu Ile Gln Met Ala Asp Tyr Ile Ser Lys Asn Val Pro Ala
   465                 470                 475                 480
   Thr Asp His Val Ile Arg Arg Glu Thr Glu Thr Thr Phe Ser Gly Ser
                   485                 490                 495
   His Ser Cys Ser Pro Ser His His Cys Ser Thr Ala Thr Pro Thr Ser
               500                 505                 510
   Ser His Arg His Glu Ser His Thr Trp Ser Leu Glu Arg Ser Glu Ser
           515                 520                 525
   Leu Thr Ser Asp Ser Gln Ser Gly Ile Met Leu Ser Ser Val Gly Thr
       530                 535                 540
   Ser Lys Cys Asn Ser Pro Ala Cys Val Glu Ala Arg Ala Arg Arg Ala
   545                 550                 555                 560
   Ala Ala Tyr Asn Leu Glu Glu Arg Arg Arg Ala Thr Ala Pro Pro Tyr
                   565                 570                 575
   His Asp Ser Val Asp Ser Leu Arg Asp Ser Pro His Ser Glu Arg Tyr
               580                 585                 590
   Val Ser Ala Leu Thr Thr Pro Ala Arg Leu Ser Pro Val Asp Phe His
           595                 600                 605
   Tyr Ser Leu Ala Thr Gln Val Pro Thr Phe Glu Ile Thr Ser Pro Asn
       610                 615                 620
   Ser Ala His Ala Val Ser Leu Pro Pro Ala Ala Pro Ile Ser Tyr Arg
   625                 630                 635                 640
   Leu Ala Glu Gln Gln Pro Leu Leu Arg His Pro Ala Pro Pro Gly Pro
                   645                 650                 655
   Gly Pro Gly Pro Gly Pro Gly Pro Gly Pro Gly Ala Asp Met Gln Arg
               660                 665                 670
   Ser Tyr Asp Ser Tyr Tyr Tyr Pro Ala Ala Gly Pro Gly Pro Arg Arg
   675                 680                 685
   Gly Thr Cys Ala Leu Gly Gly Ser Leu Gly Ser Leu Pro Ala Ser Pro
       690                 695                 700
   Phe Arg Ile Pro Glu Asp Asp Glu Tyr Glu Thr Thr Gln Glu Cys Ala
   705                 710                 715                 720
   Pro Pro Pro Pro Pro Arg Pro Arg Ala Arg Gly Ala Ser Arg Arg Thr
                   725                 730                 735
   Ser Ala Gly Pro Arg Arg Trp Arg Arg Ser Arg Leu Asn Gly Leu Ala
               740                 745                 750
   Ala Gln Arg Ala Arg Ala Ala Arg Asp Ser Leu Ser Leu Ser Ser Gly
           755                 760                 765
   Ser Gly Gly Gly Ser Ala Ser Ala Ser Asp Asp Asp Ala Asp Asp Ala
       770                 775                 780
   Asp Gly Ala Leu Ala Ala Glu Ser Thr Pro Phe Leu Gly Leu Arg Gly
   785                 790                 795                 800
   Ala His Asp Ala Leu Arg Ser Asp Ser Pro Pro Leu Cys Pro Ala Ala
                   805                 810                 815
   Asp Ser Arg Thr Tyr Tyr Ser Leu Asp Ser His Ser Thr Arg Ala Ser
               820                 825                 830
   Ser Arg His Ser Arg Gly Pro Pro Pro Arg Ala Lys Gln Asp Ser Ala
           835                 840                 845
   Pro Leu
       850

<210>  57
<211>  3921
<212>  DNA
<213>  Homo sapiens

<220>
<221>  CDS
<222>  (231)..(2765)

<400>  57
atcctcctcc aggtcctggc gcacagggtg ggagcgctgc gctgcgccgc gctgcgcatc      60

gcggcccgct tgccgcctgc cccctgccct agctgggcca cctccccggg ctgccggtgg     120

agggctaaga ggcgctaacg ttacgctgtt tccggttttc cagcgggctc tgtttcccct     180

cccaaggcgg cggcggctga gcggcggagc cccccaaatg gcctggccag atg cgg 236
Met Arg
1

cag gtt tgc tgc tca gcg ctg ccg ccg ccg cca ctg gag aag ggt cgg 284
Gln Val Cys Cys Ser Ala Leu Pro Pro Pro Pro Leu Glu Lys Gly Arg
        5                   10                  15

tgc agc agc tac agc gac agc agc agc agc agc agc gag agg agc agc 332
Cys Ser Ser Tyr Ser Asp Ser Ser Ser Ser Ser Ser Glu Arg Ser Ser
    20                  25                  30

agc agc agc agc agc agc agc gag agc ggc agc agc agc agg agc agc 380
Ser Ser Ser Ser Ser Ser Ser Glu Ser Gly Ser Ser Ser Arg Ser Ser
35                  40                  45                  50

agc aac aac agc agc atc tct cgt ccc gct gcg ccc cca gag ccg cgg 428
Ser Asn Asn Ser Ser Ile Ser Arg Pro Ala Ala Pro Pro Glu Pro Arg
                55                  60                  65

ccg cag caa cag ccg cag ccc cgc agc ccc gca gcc cgg aga gcc gcc 476
Pro Gln Gln Gln Pro Gln Pro Arg Ser Pro Ala Ala Arg Arg Ala Ala
            70                  75                  80

gcc cgt tcg cga gcc gca gcc gcc ggc ggc atg agg cgc gac ccg gcc 524
Ala Arg Ser Arg Ala Ala Ala Ala Gly Gly Met Arg Arg Asp Pro Ala
        85                  90                  95

ccc ggc ttc tcc atg ctg ctc ttc ggt gtg tcg ctc gcc tgc tac tcg 572
Pro Gly Phe Ser Met Leu Leu Phe Gly Val Ser Leu Ala Cys Tyr Ser
    100                 105                 110

ccc agc ctc aag tca gtg cag gac cag gcg tac aag gca ccc gtg gtg 620
Pro Ser Leu Lys Ser Val Gln Asp Gln Ala Tyr Lys Ala Pro Val Val
115                 120                 125                 130

gtg gag ggc aag gta cag ggg ctg gtc cca gcc ggc ggc tcc agc tcc 668
Val Glu Gly Lys Val Gln Gly Leu Val Pro Ala Gly Gly Ser Ser Ser
                135                 140                 145

aac agc acc cga gag ccg ccc gcc tcg ggt cgg gtg gcg ttg gta aag 716
Asn Ser Thr Arg Glu Pro Pro Ala Ser Gly Arg Val Ala Leu Val Lys
            150                 155                 160

gtg ctg gac aag tgg ccg ctc cgg agc ggg ggg ctg cag cgc gag cag 764
Val Leu Asp Lys Trp Pro Leu Arg Ser Gly Gly Leu Gln Arg Glu Gln
        165                 170                 175

gtg atc agc gtg ggc tcc tgt gtg ccg ctc gaa agg aac cag cgc tac 812
Val Ile Ser Val Gly Ser Cys Val Pro Leu Glu Arg Asn Gln Arg Tyr
    180                 185                 190

atc ttt ttc ctg gag ccc acg gaa cag ccc tta gtc ttt aag acg gcc 860
Ile Phe Phe Leu Glu Pro Thr Glu Gln Pro Leu Val Phe Lys Thr Ala
195                 200                 205                 210

ttt gcc ccc ctc gat acc aac ggc aaa aat ctc aag aaa gag gtg ggc 908
Phe Ala Pro Leu Asp Thr Asn Gly Lys Asn Leu Lys Lys Glu Val Gly
                215                 220                 225

aag atc ctg tgc act gac tgc gcc acc cgg ccc aag ttg aag aag atg 956
Lys Ile Leu Cys Thr Asp Cys Ala Thr Arg Pro Lys Leu Lys Lys Met

230                 235                 240

aag agc cag acg gga cag gtg ggt gag aag caa tcg ctg aag tgt gag      1004
Lys Ser Gln Thr Gly Gln Val Gly Glu Lys Gln Ser Leu Lys Cys Glu
        245                 250                 255

gca gca gcc ggt aat ccc cag cct tcc tac cgt tgg ttc aag gat ggc      1052
Ala Ala Ala Gly Asn Pro Gln Pro Ser Tyr Arg Trp Phe Lys Asp Gly
    260                 265                 270

aag gag ctc aac cgc agc cga gac att cgc atc aaa tat ggc aac ggc      1100
Lys Glu Leu Asn Arg Ser Arg Asp Ile Arg Ile Lys Tyr Gly Asn Gly
275                 280                 285                 290

aga aag aac tca cga cta cag ttc aac aag gtg aag gtg gag gac gct      1148
Arg Lys Asn Ser Arg Leu Gln Phe Asn Lys Val Lys Val Glu Asp Ala
                295                 300                 305

ggg gag tat gtc tgc gag gcc gag aac atc ctg ggg aag gac acc gtc      1196
Gly Glu Tyr Val Cys Glu Ala Glu Asn Ile Leu Gly Lys Asp Thr Val
            310                 315                 320

cgg ggc cgg ctt tac gtc aac agc gtg agc acc acc ctg tca tcc tgg      1244
Arg Gly Arg Leu Tyr Val Asn Ser Val Ser Thr Thr Leu Ser Ser Trp
        325                 330                 335

tcg ggg cac gcc cgg aag tgc aac gag aca gcc aag tcc tat tgc gtc      1292
Ser Gly His Ala Arg Lys Cys Asn Glu Thr Ala Lys Ser Tyr Cys Val
    340                 345                 350

aat gga ggc gtc tgc tac tac atc gag ggc atc aac cag ctc tcc tgc      1340
Asn Gly Gly Val Cys Tyr Tyr Ile Glu Gly Ile Asn Gln Leu Ser Cys
355                 360                 365                 370

aag tgt cct gtg gga tac acc ggg gac agg tgt cag cag ttc gca atg      1388
Lys Cys Pro Val Gly Tyr Thr Gly Asp Arg Cys Gln Gln Phe Ala Met
                375                 380                 385

gtc aac ttc tcc aaa gcc gag gag ctg tac cag aag agg gtc ctg acc      1436
Val Asn Phe Ser Lys Ala Glu Glu Leu Tyr Gln Lys Arg Val Leu Thr
            390                 395                 400

atc acg ggc atc tgc gtg gct ctg ctg gtc gtg ggc atc gtc tgt gtg      1484
Ile Thr Gly Ile Cys Val Ala Leu Leu Val Val Gly Ile Val Cys Val
        405                 410                 415

gtg gcc tac tgc aag acc aaa aaa cag cgg aag cag atg cac aac cac      1532
Val Ala Tyr Cys Lys Thr Lys Lys Gln Arg Lys Gln Met His Asn His
    420                 425                 430

ctc cgg cag aac atg tgc ccg gcc cat cag aac cgg agc ttg gcc aat      1580
Leu Arg Gln Asn Met Cys Pro Ala His Gln Asn Arg Ser Leu Ala Asn
435                 440                 445                 450

ggg ccc agc cac ccc cgg ctg gac cca gag gag atc cag atg gca gat      1628
Gly Pro Ser His Pro Arg Leu Asp Pro Glu Glu Ile Gln Met Ala Asp
                455                 460                 465

tat att tcc aag aac gtg cca gcc aca gac cat gtc atc agg aga gaa      1676
Tyr Ile Ser Lys Asn Val Pro Ala Thr Asp His Val Ile Arg Arg Glu
            470                 475                 480

act gag acc acc ttc tct ggg agc cac tcc tgt tct cct tct cac cac      1724

Thr Glu Thr Thr Phe Ser Gly Ser His Ser Cys Ser Pro Ser His His
        485                 490                 495

tgc tcc aca gcc aca ccc acc tcc agc cac aga cac gag agc cac acg      1772
Cys Ser Thr Ala Thr Pro Thr Ser Ser His Arg His Glu Ser His Thr
    500                 505                 510

tgg agc ctg gaa cgt tct gag agc ctg act tct gac tcc cag tcg ggg      1820
Trp Ser Leu Glu Arg Ser Glu Ser Leu Thr Ser Asp Ser Gln Ser Gly
515                 520                 525                 530

atc atg cta tca tca gtg ggt acc agc aaa tgc aac agc cca gca tgt      1868
Ile Met Leu Ser Ser Val Gly Thr Ser Lys Cys Asn Ser Pro Ala Cys
                535                 540                 545

gtg gag gcc cgg gca agg cgg gca gca gcc tac aac ctg gag gag cgg      1916
Val Glu Ala Arg Ala Arg Arg Ala Ala Ala Tyr Asn Leu Glu Glu Arg
            550                 555                 560

cgc agg gcc acc gcg cca ccc tat cac gat tcc gtg gac tcc ctt cgc      1964
Arg Arg Ala Thr Ala Pro Pro Tyr His Asp Ser Val Asp Ser Leu Arg
        565                 570                 575

gac tcc cca cac agc gag agg tac gtg tcg gcc ctg acc acg ccc gcg      2012
Asp Ser Pro His Ser Glu Arg Tyr Val Ser Ala Leu Thr Thr Pro Ala
    580                 585                 590

cgc ctc tcg ccc gtg gac ttc cac tac tcg ctg gcc acg cag gtg cca      2060
Arg Leu Ser Pro Val Asp Phe His Tyr Ser Leu Ala Thr Gln Val Pro
595                 600                 605                 610

act ttc gag atc acg tcc ccc aac tcg gcg cac gcc gtg tcg ctg ccg      2108
Thr Phe Glu Ile Thr Ser Pro Asn Ser Ala His Ala Val Ser Leu Pro
                615                 620                 625

ccg gcg gcg ccc atc agt tac cgc ctg gcc gag cag cag ccg tta ctg      2156
Pro Ala Ala Pro Ile Ser Tyr Arg Leu Ala Glu Gln Gln Pro Leu Leu
            630                 635                 640

cgg cac ccg gcg ccc ccc ggc ccg gga ccc gga ccc ggg ccc ggg ccc      2204
Arg His Pro Ala Pro Pro Gly Pro Gly Pro Gly Pro Gly Pro Gly Pro
        645                 650                 655

ggg ccc ggc gca gac atg cag cgc agc tat gac agc tac tat tac ccc      2252
Gly Pro Gly Ala Asp Met Gln Arg Ser Tyr Asp Ser Tyr Tyr Tyr Pro
    660                 665                 670

gcg gcg ggg ccc gga ccg cgg cgc ggg acc tgc gcg ctc ggc ggc agc      2300
Ala Ala Gly Pro Gly Pro Arg Arg Gly Thr Cys Ala Leu Gly Gly Ser
675                 680                 685                 690

ctg ggc agc ctg cct gcc agc ccc ttc cgc atc ccc gag gac gac gag      2348
Leu Gly Ser Leu Pro Ala Ser Pro Phe Arg Ile Pro Glu Asp Asp Glu
                695                 700                 705

tac gag acc acg cag gag tgc gcg ccc ccg ccg ccg ccg cgg ccg cgc      2396
Tyr Glu Thr Thr Gln Glu Cys Ala Pro Pro Pro Pro Pro Arg Pro Arg
            710                 715                 720

gcg cgc ggt gcg tcc cgc agg acg tcg gcg ggg ccc cgg cgc tgg cgc      2444
Ala Arg Gly Ala Ser Arg Arg Thr Ser Ala Gly Pro Arg Arg Trp Arg
        725                 730                 735

cgc tcg cgc ctc aac ggg ctg gcg gcg cag cgc gca cgg gcg gcg agg     2492
Arg Ser Arg Leu Asn Gly Leu Ala Ala Gln Arg Ala Arg Ala Ala Arg
    740                 745                 750

gac tcg ctg tcg ctg agc agc ggc tcg ggc ggc ggc tca gcc tcg gcg     2540
Asp Ser Leu Ser Leu Ser Ser Gly Ser Gly Gly Gly Ser Ala Ser Ala
755                 760                 765                 770

tcg gac gac gac gcg gac gac gcg gac ggg gcg ctg gcg gcc gag agc     2588
Ser Asp Asp Asp Ala Asp Asp Ala Asp Gly Ala Leu Ala Ala Glu Ser
                775                 780                 785

aca cct ttc ctg ggc ctg cgt ggg gcg cac gac gcg ctg cgc tcg gac     2636
Thr Pro Phe Leu Gly Leu Arg Gly Ala His Asp Ala Leu Arg Ser Asp
            790                 795                 800

tcg ccg cca ctg tgc ccg gcg gcc gac agc agg act tac tac tca ctg     2684
Ser Pro Pro Leu Cys Pro Ala Ala Asp Ser Arg Thr Tyr Tyr Ser Leu
        805                 810                 815

gac agc cac agc acg cgg gcc agc agc aga cac agc cgc ggg ccg ccc     2732
Asp Ser His Ser Thr Arg Ala Ser Ser Arg His Ser Arg Gly Pro Pro
    820                 825                 830

ccg cgg gcc aag cag gac tcg gcg cca ctc tag ggccccgccg cgcgcccctc     2785
Pro Arg Ala Lys Gln Asp Ser Ala Pro Leu
835                 840

cgccccgccc gccccactat ctttaaggag accagagacc gcctactgga gagaaaggag     2845

gaaaaaagaa ataaaaatat ttttattttc tataaaagga aaaaagtata acaaaatgtt     2905

ttattttcat tttagcaaaa attgtcttat aatactagct aacggcaaag gcgtttttat     2965

agggaaacta tttatatgta acatcctgat ttacagcttc ggaaaaaaaa aagaaacaac     3025

aaaaaaaaaa gagagatggg ccaatttttt gactctttaa tagaaaccta ttttgtggtg     3085

ccttttgctg tatgctcatc tggggctccg ggggacaggt ctgggttgtg gggtgctgag     3145

agtgacagct gggagagaag aggtgcgggt tctgttgggg gcgagggtgg ggggcggtgg     3205

ggggttggcc ctaaggaaga actgaggacg gaagtagctt tgctcccagg caagagctga     3265

agttcccgac ccctgggagg agagggccag gaccctaggc ttggagctgg agctggggta     3325

acttccaggg ggagataaag acacagctac ccgaatgggg aagccctggc tactcacagc     3385

tgttaaagga gggggggcag agaaacgttt ccccaagagc ctgagcccag ggaggtggca     3445

gctctgccag gggcccaatg ttggcggctc ctgtccccca cagcctccag gacgcccctc     3505

cgtcctctgc agcaccttcg tttacaggtc gtcttttcta ttttacacct gcatgtcctt     3565

tgcatttcag attctttaga ttggatgcat ggtcacgctg ggaccgagaa gagccactcg     3625

acagtgtatt ccccttttag caataaagta acaccatttc cttctcacag gctgcctcct     3685

agcccccaac ccacctatga catccactcg tctccagtcc tgccctccca cccccaaaaa     3745

ctgggtccac tgctaatttg tcaaaaaggt aattgtttaa aaaaaaaaaa aatcccaaac     3805

agaaacagat gggaatcctg tcctgaaatg tctgaacacc tgactgttga cacttgaaca     3865

tttttatatt ataaataaaa tcagaaataa ctcctgttaa aaaaaaaaaa aaaaaa 3921

<210> 58
<211> 844
<212> PRT
<213> Homo sapiens

<400> 58

Met Arg Gln Val Cys Cys Ser Ala Leu Pro Pro Pro Pro Leu Glu Lys
1               5                   10                  15

Gly Arg Cys Ser Ser Tyr Ser Asp Ser Ser Ser Ser Ser Ser Glu Arg
            20                  25                  30

Ser Ser Ser Ser Ser Ser Ser Ser Ser Glu Ser Gly Ser Ser Ser Arg
        35                  40                  45

Ser Ser Ser Asn Asn Ser Ser Ile Ser Arg Pro Ala Ala Pro Pro Glu
    50                  55                  60

Pro Arg Pro Gln Gln Gln Pro Gln Pro Arg Ser Pro Ala Ala Arg Arg
65                  70                  75                  80

Ala Ala Ala Arg Ser Arg Ala Ala Ala Ala Gly Gly Met Arg Arg Asp
                85                  90                  95

Pro Ala Pro Gly Phe Ser Met Leu Leu Phe Gly Val Ser Leu Ala Cys
            100                 105                 110

Tyr Ser Pro Ser Leu Lys Ser Val Gln Asp Gln Ala Tyr Lys Ala Pro
        115                 120                 125

Val Val Val Glu Gly Lys Val Gln Gly Leu Val Pro Ala Gly Gly Ser
    130                 135                 140

Ser Ser Asn Ser Thr Arg Glu Pro Pro Ala Ser Gly Arg Val Ala Leu
145                 150                 155                 160

Val Lys Val Leu Asp Lys Trp Pro Leu Arg Ser Gly Gly Leu Gln Arg
                165                 170                 175

Glu Gln Val Ile Ser Val Gly Ser Cys Val Pro Leu Glu Arg Asn Gln
            180                 185                 190

Arg Tyr Ile Phe Phe Leu Glu Pro Thr Glu Gln Pro Leu Val Phe Lys
        195                 200                 205

Thr Ala Phe Ala Pro Leu Asp Thr Asn Gly Lys Asn Leu Lys Lys Glu
    210                 215                 220

Val Gly Lys Ile Leu Cys Thr Asp Cys Ala Thr Arg Pro Lys Leu Lys
225                 230                 235                 240

Lys Met Lys Ser Gln Thr Gly Gln Val Gly Glu Lys Gln Ser Leu Lys
                245                 250                 255

Cys Glu Ala Ala Ala Gly Asn Pro Gln Pro Ser Tyr Arg Trp Phe Lys
            260                 265                 270

Asp Gly Lys Glu Leu Asn Arg Ser Arg Asp Ile Arg Ile Lys Tyr Gly
        275                 280                 285

Asn Gly Arg Lys Asn Ser Arg Leu Gln Phe Asn Lys Val Lys Val Glu
    290                 295                 300

Asp Ala Gly Glu Tyr Val Cys Glu Ala Glu Asn Ile Leu Gly Lys Asp
305                 310                 315                 320

Thr Val Arg Gly Arg Leu Tyr Val Asn Ser Val Ser Thr Thr Leu Ser
                325                 330                 335

Ser Trp Ser Gly His Ala Arg Lys Cys Asn Glu Thr Ala Lys Ser Tyr
            340                 345                 350

Cys Val Asn Gly Gly Val Cys Tyr Tyr Ile Glu Gly Ile Asn Gln Leu
        355                 360                 365

Ser Cys Lys Cys Pro Val Gly Tyr Thr Gly Asp Arg Cys Gln Gln Phe
    370                 375                 380

Ala Met Val Asn Phe Ser Lys Ala Glu Glu Leu Tyr Gln Lys Arg Val
385                 390                 395                 400

Leu Thr Ile Thr Gly Ile Cys Val Ala Leu Leu Val Val Gly Ile Val
                405                 410                 415

Cys Val Val Ala Tyr Cys Lys Thr Lys Lys Gln Arg Lys Gln Met His
            420                 425                 430

Asn His Leu Arg Gln Asn Met Cys Pro Ala His Gln Asn Arg Ser Leu
        435                 440                 445

Ala Asn Gly Pro Ser His Pro Arg Leu Asp Pro Glu Glu Ile Gln Met
    450                 455                 460

Ala Asp Tyr Ile Ser Lys Asn Val Pro Ala Thr Asp His Val Ile Arg
465                 470                 475                 480

Arg Glu Thr Glu Thr Thr Phe Ser Gly Ser His Ser Cys Ser Pro Ser
                485                 490                 495

His His Cys Ser Thr Ala Thr Pro Thr Ser Ser His Arg His Glu Ser
            500                 505                 510

His Thr Trp Ser Leu Glu Arg Ser Glu Ser Leu Thr Ser Asp Ser Gln
        515                 520                 525

Ser Gly Ile Met Leu Ser Ser Val Gly Thr Ser Lys Cys Asn Ser Pro
    530                 535                 540

Ala Cys Val Glu Ala Arg Ala Arg Arg Ala Ala Ala Tyr Asn Leu Glu
545                 550                 555                 560

Glu Arg Arg Arg Ala Thr Ala Pro Pro Tyr His Asp Ser Val Asp Ser
                565                 570                 575

Leu Arg Asp Ser Pro His Ser Glu Arg Tyr Val Ser Ala Leu Thr Thr
            580                 585                 590

Pro Ala Arg Leu Ser Pro Val Asp Phe His Tyr Ser Leu Ala Thr Gln
        595                 600                 605

Val Pro Thr Phe Glu Ile Thr Ser Pro Asn Ser Ala His Ala Val Ser
    610                 615                 620

Leu Pro Pro Ala Ala Pro Ile Ser Tyr Arg Leu Ala Glu Gln Gln Pro
625                 630                 635                 640

Leu Leu Arg His Pro Ala Pro Pro Gly Pro Gly Pro Gly Pro Gly Pro
                645                 650                 655

Gly Pro Gly Pro Gly Ala Asp Met Gln Arg Ser Tyr Asp Ser Tyr Tyr
            660                 665                 670

Tyr Pro Ala Ala Gly Pro Gly Pro Arg Arg Gly Thr Cys Ala Leu Gly
        675                 680                 685

Gly Ser Leu Gly Ser Leu Pro Ala Ser Pro Phe Arg Ile Pro Glu Asp
    690                 695                 700

Asp Glu Tyr Glu Thr Thr Gln Glu Cys Ala Pro Pro Pro Pro Pro Arg
705                 710                 715                 720

Pro Arg Ala Arg Gly Ala Ser Arg Arg Thr Ser Ala Gly Pro Arg Arg
                725                 730                 735

Trp Arg Arg Ser Arg Leu Asn Gly Leu Ala Ala Gln Arg Ala Arg Ala
            740                 745                 750

Ala Arg Asp Ser Leu Ser Leu Ser Ser Gly Ser Gly Gly Gly Ser Ala
        755                 760                 765

Ser Ala Ser Asp Asp Asp Ala Asp Asp Ala Asp Gly Ala Leu Ala Ala
    770                 775                 780

Glu Ser Thr Pro Phe Leu Gly Leu Arg Gly Ala His Asp Ala Leu Arg
785                 790                 795                 800

Ser Asp Ser Pro Pro Leu Cys Pro Ala Ala Asp Ser Arg Thr Tyr Tyr
                805                 810                 815

Ser Leu Asp Ser His Ser Thr Arg Ala Ser Ser Arg His Ser Arg Gly
            820                 825                 830

Pro Pro Pro Arg Ala Lys Gln Asp Ser Ala Pro Leu
        835                 840

<210>  59
<211>  3963
<212>  DNA
<213>  Homo sapiens

<220>
<221>  CDS
<222>  (231)..(2807)

<400>  59
   atcctcctcc aggtcctggc gcacagggtg ggagcgctgc gctgcgccgc gctgcgcatc      60
   gcggcccgct tgccgcctgc cccctgccct agctgggcca cctccccggg ctgccggtgg     120
   agggctaaga ggcgctaacg ttacgctgtt tccggttttc cagcgggctc tgtttcccct     180
   cccaaggcgg cggcggctga gcggcggagc cccccaaatg gcctggccag atg cgg         236
                                                         Met Arg
                                                         1
   cag gtt tgc tgc tca gcg ctg ccg ccg ccg cca ctg gag aag ggt cgg       284
   Gln Val Cys Cys Ser Ala Leu Pro Pro Pro Pro Leu Glu Lys Gly Arg
           5                   10                  15
   tgc agc agc tac agc gac agc agc agc agc agc agc gag agg agc agc       332
   Cys Ser Ser Tyr Ser Asp Ser Ser Ser Ser Ser Ser Glu Arg Ser Ser
       20                  25                  30
   agc agc agc agc agc agc agc gag agc ggc agc agc agc agg agc agc 380
   Ser Ser Ser Ser Ser Ser Ser Glu Ser Gly Ser Ser Ser Arg Ser Ser
   35 40 45 50
   agc aac aac agc agc atc tct cgt ccc gct gcg ccc cca gag ccg cgg 428
   Ser Asn Asn Ser Ser Ile Ser Arg Pro Ala Ala Pro Pro Glu Pro Arg
   55 60 65
   ccg cag caa cag ccg cag ccc cgc agc ccc gca gcc cgg aga gcc gcc 476
   Pro Gln Gln Gln Pro Gln Pro Arg Ser Pro Ala Ala Arg Arg Ala Ala
   70 75 80
   gcc cgt tcg cga gcc gca gcc gcc ggc ggc atg agg cgc gac ccg gcc 524
   Ala Arg Ser Arg Ala Ala Ala Ala Gly Gly Met Arg Arg Asp Pro Ala
   85 90 95
   ccc ggc ttc tcc atg ctg ctc ttc ggt gtg tcg ctc gcc tgc tac tcg 572
   Pro Gly Phe Ser Met Leu Leu Phe Gly Val Ser Leu Ala Cys Tyr Ser
   100 105 110
   ccc agc ctc aag tca gtg cag gac cag gcg tac aag gca ccc gtg gtg 620
   Pro Ser Leu Lys Ser Val Gln Asp Gln Ala Tyr Lys Ala Pro Val Val
   115 120 125 130
   gtg gag ggc aag gta cag ggg ctg gtc cca gcc ggc ggc tcc agc tcc 668
   Val Glu Gly Lys Val Gln Gly Leu Val Pro Ala Gly Gly Ser Ser Ser
   135 140 145
   aac agc acc cga gag ccg ccc gcc tcg ggt cgg gtg gcg ttg gta aag 716
   Asn Ser Thr Arg Glu Pro Pro Ala Ser Gly Arg Val Ala Leu Val Lys
   150 155 160
   gtg ctg gac aag tgg ccg ctc cgg agc ggg ggg ctg cag cgc gag cag 764
   Val Leu Asp Lys Trp Pro Leu Arg Ser Gly Gly Leu Gln Arg Glu Gln
   165 170 175
   gtg atc agc gtg ggc tcc tgt gtg ccg ctc gaa agg aac cag cgc tac 812
   Val Ile Ser Val Gly Ser Cys Val Pro Leu Glu Arg Asn Gln Arg Tyr
   180 185 190
   atc ttt ttc ctg gag ccc acg gaa cag ccc tta gtc ttt aag acg gcc 860
   Ile Phe Phe Leu Glu Pro Thr Glu Gln Pro Leu Val Phe Lys Thr Ala
   195 200 205 210
   ttt gcc ccc ctc gat acc aac ggc aaa aat ctc aag aaa gag gtg ggc 908
   Phe Ala Pro Leu Asp Thr Asn Gly Lys Asn Leu Lys Lys Glu Val Gly
   215 220 225
   aag atc ctg tgc act gac tgc gcc acc cgg ccc aag ttg aag aag atg 956
   Lys Ile Leu Cys Thr Asp Cys Ala Thr Arg Pro Lys Leu Lys Lys Met
   230 235 240
   aag agc cag acg gga cag gtg ggt gag aag caa tcg ctg aag tgt gag 1004
   Lys Ser Gln Thr Gly Gln Val Gly Glu Lys Gln Ser Leu Lys Cys Glu
   245 250 255
   gca gca gcc ggt aat ccc cag cct tcc tac cgt tgg ttc aag gat ggc 1052
   Ala Ala Ala Gly Asn Pro Gln Pro Ser Tyr Arg Trp Phe Lys Asp Gly
   260 265 270
   aag gag ctc aac cgc agc cga gac att cgc atc aaa tat ggc aac ggc 1100
   Lys Glu Leu Asn Arg Ser Arg Asp Ile Arg Ile Lys Tyr Gly Asn Gly
   275 280 285 290
   aga aag aac tca cga cta cag ttc aac aag gtg aag gtg gag gac gct     1148
   Arg Lys Asn Ser Arg Leu Gln Phe Asn Lys Val Lys Val Glu Asp Ala
               295                 300                 305
   ggg gag tat gtc tgc gag gcc gag aac atc ctg ggg aag gac acc gtc     1196
   Gly Glu Tyr Val Cys Glu Ala Glu Asn Ile Leu Gly Lys Asp Thr Val
           310                 315                 320
   cgg ggc cgg ctt tac gtc aac agc gtg agc acc acc ctg tca tcc tgg     1244
   Arg Gly Arg Leu Tyr Val Asn Ser Val Ser Thr Thr Leu Ser Ser Trp
       325                 330                 335
   tcg ggg cac gcc cgg aag tgc aac gag aca gcc aag tcc tat tgc gtc     1292
   Ser Gly His Ala Arg Lys Cys Asn Glu Thr Ala Lys Ser Tyr Cys Val
       340                 345                 350
   aat gga ggc gtc tgc tac tac atc gag ggc atc aac cag ctc tcc tgc     1340
   Asn Gly Gly Val Cys Tyr Tyr Ile Glu Gly Ile Asn Gln Leu Ser Cys
   355                 360                 365                 370
   aaa tgt cca aat gga ttc ttc gga cag aga tgt ttg gag aaa ctg cct     1388
   Lys Cys Pro Asn Gly Phe Phe Gly Gln Arg Cys Leu Glu Lys Leu Pro
               375                 380                 385
   ttg cga ttg tac atg cca gat cct aag caa aag cac ctt gga ttt gaa     1436
   Leu Arg Leu Tyr Met Pro Asp Pro Lys Gln Lys His Leu Gly Phe Glu
           390                 395                 400
   tta aag gaa gcc gag gag ctg tac cag aag agg gtc ctg acc atc acg     1484
   Leu Lys Glu Ala Glu Glu Leu Tyr Gln Lys Arg Val Leu Thr Ile Thr
       405                 410                 415
   ggc atc tgc gtg gct ctg ctg gtc gtg ggc atc gtc tgt gtg gtg gcc     1532
   Gly Ile Cys Val Ala Leu Leu Val Val Gly Ile Val Cys Val Val Ala
       420                 425                 430
   tac tgc aag acc aaa aaa cag cgg aag cag atg cac aac cac ctc cgg     1580
   Tyr Cys Lys Thr Lys Lys Gln Arg Lys Gln Met His Asn His Leu Arg
   435                 440                 445                 450
   cag aac atg tgc ccg gcc cat cag aac cgg agc ttg gcc aat ggg ccc     1628
   Gln Asn Met Cys Pro Ala His Gln Asn Arg Ser Leu Ala Asn Gly Pro
               455                 460                 465
   agc cac ccc cgg ctg gac cca gag gag atc cag atg gca gat tat att     1676
   Ser His Pro Arg Leu Asp Pro Glu Glu Ile Gln Met Ala Asp Tyr Ile
           470                 475                 480
   tcc aag aac gtg cca gcc aca gac cat gtc atc agg aga gaa act gag     1724
   Ser Lys Asn Val Pro Ala Thr Asp His Val Ile Arg Arg Glu Thr Glu
       485                 490                 495
   acc acc ttc tct ggg agc cac tcc tgt tct cct tct cac cac tgc tcc     1772
   Thr Thr Phe Ser Gly Ser His Ser Cys Ser Pro Ser His His Cys Ser
       500                 505                 510
   aca gcc aca ccc acc tcc agc cac aga cac gag agc cac acg tgg agc     1820
   Thr Ala Thr Pro Thr Ser Ser His Arg His Glu Ser His Thr Trp Ser
   515                 520                 525                 530
   ctg gaa cgt tct gag agc ctg act tct gac tcc cag tcg ggg atc atg     1868
   Leu Glu Arg Ser Glu Ser Leu Thr Ser Asp Ser Gln Ser Gly Ile Met
   535                 540                 545
   cta tca tca gtg ggt acc agc aaa tgc aac agc cca gca tgt gtg gag        1916
   Leu Ser Ser Val Gly Thr Ser Lys Cys Asn Ser Pro Ala Cys Val Glu
               550                 555                 560
   gcc cgg gca agg cgg gca gca gcc tac aac ctg gag gag cgg cgc agg        1964
   Ala Arg Ala Arg Arg Ala Ala Ala Tyr Asn Leu Glu Glu Arg Arg Arg
           565                 570                 575
   gcc acc gcg cca ccc tat cac gat tcc gtg gac tcc ctt cgc gac tcc        2012
   Ala Thr Ala Pro Pro Tyr His Asp Ser Val Asp Ser Leu Arg Asp Ser
       580                 585                 590
   cca cac agc gag agg tac gtg tcg gcc ctg acc acg ccc gcg cgc ctc        2060
   Pro His Ser Glu Arg Tyr Val Ser Ala Leu Thr Thr Pro Ala Arg Leu
   595                 600                 605                 610
   tcg ccc gtg gac ttc cac tac tcg ctg gcc acg cag gtg cca act ttc        2108
   Ser Pro Val Asp Phe His Tyr Ser Leu Ala Thr Gln Val Pro Thr Phe
                   615                 620                 625
   gag atc acg tcc ccc aac tcg gcg cac gcc gtg tcg ctg ccg ccg gcg        2156
   Glu Ile Thr Ser Pro Asn Ser Ala His Ala Val Ser Leu Pro Pro Ala
               630                 635                 640
   gcg ccc atc agt tac cgc ctg gcc gag cag cag ccg tta ctg cgg cac        2204
   Ala Pro Ile Ser Tyr Arg Leu Ala Glu Gln Gln Pro Leu Leu Arg His
           645                 650                 655
   ccg gcg ccc ccc ggc ccg gga ccc gga ccc ggg ccc ggg ccc ggg ccc        2252
   Pro Ala Pro Pro Gly Pro Gly Pro Gly Pro Gly Pro Gly Pro Gly Pro
       660                 665                 670
   ggc gca gac atg cag cgc agc tat gac agc tac tat tac ccc gcg gcg        2300
   Gly Ala Asp Met Gln Arg Ser Tyr Asp Ser Tyr Tyr Tyr Pro Ala Ala
   675                 680                 685                 690
   ggg ccc gga ccg cgg cgc ggg acc tgc gcg ctc ggc ggc agc ctg ggc        2348
   Gly Pro Gly Pro Arg Arg Gly Thr Cys Ala Leu Gly Gly Ser Leu Gly
                   695                 700                 705
   agc ctg cct gcc agc ccc ttc cgc atc ccc gag gac gac gag tac gag        2396
   Ser Leu Pro Ala Ser Pro Phe Arg Ile Pro Glu Asp Asp Glu Tyr Glu
               710                 715                 720
   acc acg cag gag tgc gcg ccc ccg ccg ccg ccg cgg ccg cgc gcg cgc        2444
   Thr Thr Gln Glu Cys Ala Pro Pro Pro Pro Pro Arg Pro Arg Ala Arg
           725                 730                 735
   ggt gcg tcc cgc agg acg tcg gcg ggg ccc cgg cgc tgg cgc cgc tcg        2492
   Gly Ala Ser Arg Arg Thr Ser Ala Gly Pro Arg Arg Trp Arg Arg Ser
       740                 745                 750
   cgc ctc aac ggg ctg gcg gcg cag cgc gca cgg gcg gcg agg gac tcg        2540
   Arg Leu Asn Gly Leu Ala Ala Gln Arg Ala Arg Ala Ala Arg Asp Ser
   755                 760                 765                 770
   ctg tcg ctg agc agc ggc tcg ggc ggc ggc tca gcc tcg gcg tcg gac        2588
   Leu Ser Leu Ser Ser Gly Ser Gly Gly Gly Ser Ala Ser Ala Ser Asp
                   775                 780                 785
   gac gac gcg gac gac gcg gac ggg gcg ctg gcg gcc gag agc aca cct        2636
   Asp Asp Ala Asp Asp Ala Asp Gly Ala Leu Ala Ala Glu Ser Thr Pro
               790                 795                 800
   ttc ctg ggc ctg cgt ggg gcg cac gac gcg ctg cgc tcg gac tcg ccg      2684
   Phe Leu Gly Leu Arg Gly Ala His Asp Ala Leu Arg Ser Asp Ser Pro
           805                 810                 815
   cca ctg tgc ccg gcg gcc gac agc agg act tac tac tca ctg gac agc      2732
   Pro Leu Cys Pro Ala Ala Asp Ser Arg Thr Tyr Tyr Ser Leu Asp Ser
       820                 825                 830
   cac agc acg cgg gcc agc agc aga cac agc cgc ggg ccg ccc ccg cgg      2780
   His Ser Thr Arg Ala Ser Ser Arg His Ser Arg Gly Pro Pro Pro Arg
   835                 840                 845                 850
   gcc aag cag gac tcg gcg cca ctc tag ggccccgccg cgcgcccctc            2827
   Ala Lys Gln Asp Ser Ala Pro Leu
                   855
   cgccccgccc gccccactat ctttaaggag accagagacc gcctactgga gagaaaggag    2887
   gaaaaaagaa ataaaaatat ttttattttc tataaaagga aaaaagtata acaaaatgtt    2947
   ttattttcat tttagcaaaa attgtcttat aatactagct aacggcaaag gcgtttttat    3007
   agggaaacta tttatatgta acatcctgat ttacagcttc ggaaaaaaaa aagaaacaac    3067
   aaaaaaaaaa gagagatggg ccaatttttt gactctttaa tagaaaccta ttttgtggtg    3127
   ccttttgctg tatgctcatc tggggctccg ggggacaggt ctgggttgtg gggtgctgag     3187
   agtgacagct gggagagaag aggtgcgggt tctgttgggg gcgagggtgg ggggcggtgg    3247
   ggggttggcc ctaaggaaga actgaggacg gaagtagctt tgctcccagg caagagctga    3307
   agttcccgac ccctgggagg agagggccag gaccctaggc ttggagctgg agctggggta    3367
   acttccaggg ggagataaag acacagctac ccgaatgggg aagccctggc tactcacagc    3427
   tgttaaagga gggggggcag agaaacgttt ccccaagagc ctgagcccag ggaggtggca    3487
   gctctgccag gggcccaatg ttggcggctc ctgtccccca cagcctccag gacgcccctc   3547
   cgtcctctgc agcaccttcg tttacaggtc gtcttttcta ttttacacct gcatgtcctt    3607
   tgcatttcag attctttaga ttggatgcat ggtcacgctg ggaccgagaa gagccactcg    3667
   acagtgtatt ccccttttag caataaagta acaccatttc cttctcacag gctgcctcct     3727
   agcccccaac ccacctatga catccactcg tctccagtcc tgccctccca cccccaaaaa    3787
   ctgggtccac tgctaatttg tcaaaaaggt aattgtttaa aaaaaaaaaa aatcccaaac    3847
   agaaacagat gggaatcctg tcctgaaatg tctgaacacc tgactgttga cacttgaaca    3907
   tttttatatt ataaataaaa tcagaaataa ctcctgttaa aaaaaaaaaa aaaaaa        3963

<210>  60
<211>  858
<212>  PRT
<213>  Homo sapiens

<400> 60

Met Arg Gln Val Cys Cys Ser Ala Leu Pro Pro Pro Pro Leu Glu Lys
1               5                   10                  15

Gly Arg Cys Ser Ser Tyr Ser Asp Ser Ser Ser Ser Ser Ser Glu Arg
            20                  25                  30

Ser Ser Ser Ser Ser Ser Ser Ser Ser Glu Ser Gly Ser Ser Ser Arg
        35                  40                  45

Ser Ser Ser Asn Asn Ser Ser Ile Ser Arg Pro Ala Ala Pro Pro Glu
    50                  55                  60

Pro Arg Pro Gln Gln Gln Pro Gln Pro Arg Ser Pro Ala Ala Arg Arg
65                  70                  75                  80

Ala Ala Ala Arg Ser Arg Ala Ala Ala Ala Gly Gly Met Arg Arg Asp
                85                  90                  95

Pro Ala Pro Gly Phe Ser Met Leu Leu Phe Gly Val Ser Leu Ala Cys
            100                 105                 110

Tyr Ser Pro Ser Leu Lys Ser Val Gln Asp Gln Ala Tyr Lys Ala Pro
        115                 120                 125

Val Val Val Glu Gly Lys Val Gln Gly Leu Val Pro Ala Gly Gly Ser
    130                 135                 140

Ser Ser Asn Ser Thr Arg Glu Pro Pro Ala Ser Gly Arg Val Ala Leu
145                 150                 155                 160

Val Lys Val Leu Asp Lys Trp Pro Leu Arg Ser Gly Gly Leu Gln Arg
                165                 170                 175

Glu Gln Val Ile Ser Val Gly Ser Cys Val Pro Leu Glu Arg Asn Gln
            180                 185                 190

Arg Tyr Ile Phe Phe Leu Glu Pro Thr Glu Gln Pro Leu Val Phe Lys
        195                 200                 205

Thr Ala Phe Ala Pro Leu Asp Thr Asn Gly Lys Asn Leu Lys Lys Glu
    210                 215                 220

Val Gly Lys Ile Leu Cys Thr Asp Cys Ala Thr Arg Pro Lys Leu Lys
225                 230                 235                 240

Lys Met Lys Ser Gln Thr Gly Gln Val Gly Glu Lys Gln Ser Leu Lys

245                 250                 255

Cys Glu Ala Ala Ala Gly Asn Pro Gln Pro Ser Tyr Arg Trp Phe Lys
            260                 265                 270

Asp Gly Lys Glu Leu Asn Arg Ser Arg Asp Ile Arg Ile Lys Tyr Gly
        275                 280                 285

Asn Gly Arg Lys Asn Ser Arg Leu Gln Phe Asn Lys Val Lys Val Glu
    290                 295                 300

Asp Ala Gly Glu Tyr Val Cys Glu Ala Glu Asn Ile Leu Gly Lys Asp
305                 310                 315                 320

Thr Val Arg Gly Arg Leu Tyr Val Asn Ser Val Ser Thr Thr Leu Ser
                325                 330                 335

Ser Trp Ser Gly His Ala Arg Lys Cys Asn Glu Thr Ala Lys Ser Tyr
            340                 345                 350

Cys Val Asn Gly Gly Val Cys Tyr Tyr Ile Glu Gly Ile Asn Gln Leu
        355                 360                 365

Ser Cys Lys Cys Pro Asn Gly Phe Phe Gly Gln Arg Cys Leu Glu Lys
    370                 375                 380

Leu Pro Leu Arg Leu Tyr Met Pro Asp Pro Lys Gln Lys His Leu Gly
385                 390                 395                 400

Phe Glu Leu Lys Glu Ala Glu Glu Leu Tyr Gln Lys Arg Val Leu Thr
                405                 410                 415

Ile Thr Gly Ile Cys Val Ala Leu Leu Val Val Gly Ile Val Cys Val
            420                 425                 430

Val Ala Tyr Cys Lys Thr Lys Lys Gln Arg Lys Gln Met His Asn His
        435                 440                 445

Leu Arg Gln Asn Met Cys Pro Ala His Gln Asn Arg Ser Leu Ala Asn
    450                 455                 460

Gly Pro Ser His Pro Arg Leu Asp Pro Glu Glu Ile Gln Met Ala Asp
465                 470                 475                 480

Tyr Ile Ser Lys Asn Val Pro Ala Thr Asp His Val Ile Arg Arg Glu
                485                 490                 495

Thr Glu Thr Thr Phe Ser Gly Ser His Ser Cys Ser Pro Ser His His
        500                 505                 510

Cys Ser Thr Ala Thr Pro Thr Ser Ser His Arg His Glu Ser His Thr
    515                 520                 525

Trp Ser Leu Glu Arg Ser Glu Ser Leu Thr Ser Asp Ser Gln Ser Gly
    530                 535                 540

Ile Met Leu Ser Ser Val Gly Thr Ser Lys Cys Asn Ser Pro Ala Cys
545                 550                 555                 560

Val Glu Ala Arg Ala Arg Arg Ala Ala Ala Tyr Asn Leu Glu Glu Arg
                565                 570                 575

Arg Arg Ala Thr Ala Pro Pro Tyr His Asp Ser Val Asp Ser Leu Arg
        580                 585                 590

Asp Ser Pro His Ser Glu Arg Tyr Val Ser Ala Leu Thr Thr Pro Ala
    595                 600                 605

Arg Leu Ser Pro Val Asp Phe His Tyr Ser Leu Ala Thr Gln Val Pro
    610                 615                 620

Thr Phe Glu Ile Thr Ser Pro Asn Ser Ala His Ala Val Ser Leu Pro
625                 630                 635                 640

Pro Ala Ala Pro Ile Ser Tyr Arg Leu Ala Glu Gln Gln Pro Leu Leu
                645                 650                 655

Arg His Pro Ala Pro Pro Gly Pro Gly Pro Gly Pro Gly Pro Gly Pro
        660                 665                 670

Gly Pro Gly Ala Asp Met Gln Arg Ser Tyr Asp Ser Tyr Tyr Tyr Pro
    675                 680                 685

Ala Ala Gly Pro Gly Pro Arg Arg Gly Thr Cys Ala Leu Gly Gly Ser
    690                 695                 700

Leu Gly Ser Leu Pro Ala Ser Pro Phe Arg Ile Pro Glu Asp Asp Glu
705                 710                 715                 720

Tyr Glu Thr Thr Gln Glu Cys Ala Pro Pro Pro Pro Pro Arg Pro Arg
                725                 730                 735

Ala Arg Gly Ala Ser Arg Arg Thr Ser Ala Gly Pro Arg Arg Trp Arg
        740                 745                 750

Arg Ser Arg Leu Asn Gly Leu Ala Ala Gln Arg Ala Arg Ala Ala Arg
        755                 760                 765

Asp Ser Leu Ser Leu Ser Ser Gly Ser Gly Gly Gly Ser Ala Ser Ala
    770                 775                 780

Ser Asp Asp Asp Ala Asp Asp Ala Asp Gly Ala Leu Ala Ala Glu Ser
785                 790                 795                 800

Thr Pro Phe Leu Gly Leu Arg Gly Ala His Asp Ala Leu Arg Ser Asp
                805                 810                 815

Ser Pro Pro Leu Cys Pro Ala Ala Asp Ser Arg Thr Tyr Tyr Ser Leu
            820                 825                 830

Asp Ser His Ser Thr Arg Ala Ser Ser Arg His Ser Arg Gly Pro Pro
        835                 840                 845

Pro Arg Ala Lys Gln Asp Ser Ala Pro Leu
    850                 855

<210>  61
<211>  3945
<212>  DNA
<213>  Homo sapiens

<220>
<221>  CDS
<222>  (231)..(2789)

<400>  61
atcctcctcc aggtcctggc gcacagggtg ggagcgctgc gctgcgccgc gctgcgcatc      60

gcggcccgct tgccgcctgc cccctgccct agctgggcca cctccccggg ctgccggtgg     120

agggctaaga ggcgctaacg ttacgctgtt tccggttttc cagcgggctc tgtttcccct     180

cccaaggcgg cggcggctga gcggcggagc cccccaaatg gcctggccag atg cgg        236
                                                       Met Arg
                                                       1

cag gtt tgc tgc tca gcg ctg ccg ccg ccg cca ctg gag aag ggt cgg       284
Gln Val Cys Cys Ser Ala Leu Pro Pro Pro Pro Leu Glu Lys Gly Arg
        5                   10                  15

tgc agc agc tac agc gac agc agc agc agc agc agc gag agg agc agc       332
Cys Ser Ser Tyr Ser Asp Ser Ser Ser Ser Ser Ser Glu Arg Ser Ser
    20                  25                  30

agc agc agc agc agc agc agc gag agc ggc agc agc agc agg agc agc       380
Ser Ser Ser Ser Ser Ser Ser Glu Ser Gly Ser Ser Ser Arg Ser Ser
35                  40                  45                  50

agc aac aac agc agc atc tct cgt ccc gct gcg ccc cca gag ccg cgg       428

Ser Asn Asn Ser Ser Ile Ser Arg Pro Ala Ala Pro Pro Glu Pro Arg
                55                  60                  65

ccg cag caa cag ccg cag ccc cgc agc ccc gca gcc cgg aga gcc gcc      476
Pro Gln Gln Gln Pro Gln Pro Arg Ser Pro Ala Ala Arg Arg Ala Ala
            70                  75                  80

gcc cgt tcg cga gcc gca gcc gcc ggc ggc atg agg cgc gac ccg gcc      524
Ala Arg Ser Arg Ala Ala Ala Ala Gly Gly Met Arg Arg Asp Pro Ala
        85                  90                  95

ccc ggc ttc tcc atg ctg ctc ttc ggt gtg tcg ctc gcc tgc tac tcg      572
Pro Gly Phe Ser Met Leu Leu Phe Gly Val Ser Leu Ala Cys Tyr Ser
    100                 105                 110

ccc agc ctc aag tca gtg cag gac cag gcg tac aag gca ccc gtg gtg      620
Pro Ser Leu Lys Ser Val Gln Asp Gln Ala Tyr Lys Ala Pro Val Val
115                 120                 125                 130

gtg gag ggc aag gta cag ggg ctg gtc cca gcc ggc ggc tcc agc tcc      668
Val Glu Gly Lys Val Gln Gly Leu Val Pro Ala Gly Gly Ser Ser Ser
                135                 140                 145

aac agc acc cga gag ccg ccc gcc tcg ggt cgg gtg gcg ttg gta aag      716
Asn Ser Thr Arg Glu Pro Pro Ala Ser Gly Arg Val Ala Leu Val Lys
            150                 155                 160

gtg ctg gac aag tgg ccg ctc cgg agc ggg ggg ctg cag cgc gag cag      764
Val Leu Asp Lys Trp Pro Leu Arg Ser Gly Gly Leu Gln Arg Glu Gln
        165                 170                 175

gtg atc agc gtg ggc tcc tgt gtg ccg ctc gaa agg aac cag cgc tac      812
Val Ile Ser Val Gly Ser Cys Val Pro Leu Glu Arg Asn Gln Arg Tyr
    180                 185                 190

atc ttt ttc ctg gag ccc acg gaa cag ccc tta gtc ttt aag acg gcc      860
Ile Phe Phe Leu Glu Pro Thr Glu Gln Pro Leu Val Phe Lys Thr Ala
195                 200                 205                 210

ttt gcc ccc ctc gat acc aac ggc aaa aat ctc aag aaa gag gtg ggc      908
Phe Ala Pro Leu Asp Thr Asn Gly Lys Asn Leu Lys Lys Glu Val Gly
                215                 220                 225

aag atc ctg tgc act gac tgc gcc acc cgg ccc aag ttg aag aag atg      956
Lys Ile Leu Cys Thr Asp Cys Ala Thr Arg Pro Lys Leu Lys Lys Met
            230                 235                 240

aag agc cag acg gga cag gtg ggt gag aag caa tcg ctg aag tgt gag     1004
Lys Ser Gln Thr Gly Gln Val Gly Glu Lys Gln Ser Leu Lys Cys Glu
        245                 250                 255

gca gca gcc ggt aat ccc cag cct tcc tac cgt tgg ttc aag gat ggc     1052
Ala Ala Ala Gly Asn Pro Gln Pro Ser Tyr Arg Trp Phe Lys Asp Gly
    260                 265                 270

aag gag ctc aac cgc agc cga gac att cgc atc aaa tat ggc aac ggc     1100
Lys Glu Leu Asn Arg Ser Arg Asp Ile Arg Ile Lys Tyr Gly Asn Gly
275                 280                 285                 290

aga aag aac tca cga cta cag ttc aac aag gtg aag gtg gag gac gct     1148
Arg Lys Asn Ser Arg Leu Gln Phe Asn Lys Val Lys Val Glu Asp Ala
                295                 300                 305

ggg gag tat gtc tgc gag gcc gag aac atc ctg ggg aag gac acc gtc 1196
Gly Glu Tyr Val Cys Glu Ala Glu Asn Ile Leu Gly Lys Asp Thr Val
            310                 315                 320

cgg ggc cgg ctt tac gtc aac agc gtg agc acc acc ctg tca tcc tgg 1244
Arg Gly Arg Leu Tyr Val Asn Ser Val Ser Thr Thr Leu Ser Ser Trp
        325                 330                 335

tcg ggg cac gcc cgg aag tgc aac gag aca gcc aag tcc tat tgc gtc 1292
Ser Gly His Ala Arg Lys Cys Asn Glu Thr Ala Lys Ser Tyr Cys Val
    340                 345                 350

aat gga ggc gtc tgc tac tac atc gag ggc atc aac cag ctc tcc tgc 1340
Asn Gly Gly Val Cys Tyr Tyr Ile Glu Gly Ile Asn Gln Leu Ser Cys
355                 360                 365                 370

aag tgt cct gtg gga tac acc ggg gac agg tgt cag cag ttc gca atg 1388
Lys Cys Pro Val Gly Tyr Thr Gly Asp Arg Cys Gln Gln Phe Ala Met
                375                 380                 385

gtc aac ttc tcc aag cac ctt gga ttt gaa tta aag gaa gcc gag gag 1436
Val Asn Phe Ser Lys His Leu Gly Phe Glu Leu Lys Glu Ala Glu Glu
            390                 395                 400

ctg tac cag aag agg gtc ctg acc atc acg ggc atc tgc gtg gct ctg 1484
Leu Tyr Gln Lys Arg Val Leu Thr Ile Thr Gly Ile Cys Val Ala Leu
        405                 410                 415

ctg gtc gtg ggc atc gtc tgt gtg gtg gcc tac tgc aag acc aaa aaa 1532
Leu Val Val Gly Ile Val Cys Val Val Ala Tyr Cys Lys Thr Lys Lys
    420                 425                 430

cag cgg aag cag atg cac aac cac ctc cgg cag aac atg tgc ccg gcc 1580
Gln Arg Lys Gln Met His Asn His Leu Arg Gln Asn Met Cys Pro Ala
435                 440                 445                 450

cat cag aac cgg agc ttg gcc aat ggg ccc agc cac ccc cgg ctg gac 1628
His Gln Asn Arg Ser Leu Ala Asn Gly Pro Ser His Pro Arg Leu Asp
                455                 460                 465

cca gag gag atc cag atg gca gat tat att tcc aag aac gtg cca gcc 1676
Pro Glu Glu Ile Gln Met Ala Asp Tyr Ile Ser Lys Asn Val Pro Ala
            470                 475                 480

aca gac cat gtc atc agg aga gaa act gag acc acc ttc tct ggg agc 1724
Thr Asp His Val Ile Arg Arg Glu Thr Glu Thr Thr Phe Ser Gly Ser
        485                 490                 495

cac tcc tgt tct cct tct cac cac tgc tcc aca gcc aca ccc acc tcc 1772
His Ser Cys Ser Pro Ser His His Cys Ser Thr Ala Thr Pro Thr Ser
    500                 505                 510

agc cac aga cac gag agc cac acg tgg agc ctg gaa cgt tct gag agc 1820
Ser His Arg His Glu Ser His Thr Trp Ser Leu Glu Arg Ser Glu Ser
515                 520                 525                 530

ctg act tct gac tcc cag tcg ggg atc atg cta tca tca gtg ggt acc 1868
Leu Thr Ser Asp Ser Gln Ser Gly Ile Met Leu Ser Ser Val Gly Thr
                535                 540                 545

agc aaa tgc aac agc cca gca tgt gtg gag gcc cgg gca agg cgg gca 1916
Ser Lys Cys Asn Ser Pro Ala Cys Val Glu Ala Arg Ala Arg Arg Ala
            550                 555                 560

gca gcc tac aac ctg gag gag cgg cgc agg gcc acc gcg cca ccc tat 1964
Ala Ala Tyr Asn Leu Glu Glu Arg Arg Arg Ala Thr Ala Pro Pro Tyr
        565                 570                 575

cac gat tcc gtg gac tcc ctt cgc gac tcc cca cac agc gag agg tac 2012
His Asp Ser Val Asp Ser Leu Arg Asp Ser Pro His Ser Glu Arg Tyr
    580                 585                 590

gtg tcg gcc ctg acc acg ccc gcg cgc ctc tcg ccc gtg gac ttc cac 2060
Val Ser Ala Leu Thr Thr Pro Ala Arg Leu Ser Pro Val Asp Phe His
595                 600                 605                 610

tac tcg ctg gcc acg cag gtg cca act ttc gag atc acg tcc ccc aac 2108
Tyr Ser Leu Ala Thr Gln Val Pro Thr Phe Glu Ile Thr Ser Pro Asn
                615                 620                 625

tcg gcg cac gcc gtg tcg ctg ccg ccg gcg gcg ccc atc agt tac cgc 2156
Ser Ala His Ala Val Ser Leu Pro Pro Ala Ala Pro Ile Ser Tyr Arg
            630                 635                 640

ctg gcc gag cag cag ccg tta ctg cgg cac ccg gcg ccc ccc ggc ccg 2204
Leu Ala Glu Gln Gln Pro Leu Leu Arg His Pro Ala Pro Pro Gly Pro
        645                 650                 655

gga ccc gga ccc ggg ccc ggg ccc ggg ccc ggc gca gac atg cag cgc 2252
Gly Pro Gly Pro Gly Pro Gly Pro Gly Pro Gly Ala Asp Met Gln Arg
    660                 665                 670

agc tat gac agc tac tat tac ccc gcg gcg ggg ccc gga ccg cgg cgc 2300
Ser Tyr Asp Ser Tyr Tyr Tyr Pro Ala Ala Gly Pro Gly Pro Arg Arg
675                 680                 685                 690

ggg acc tgc gcg ctc ggc ggc agc ctg ggc agc ctg cct gcc agc ccc 2348
Gly Thr Cys Ala Leu Gly Gly Ser Leu Gly Ser Leu Pro Ala Ser Pro
                695                 700                 705

ttc cgc atc ccc gag gac gac gag tac gag acc acg cag gag tgc gcg 2396
Phe Arg Ile Pro Glu Asp Asp Glu Tyr Glu Thr Thr Gln Glu Cys Ala
            710                 715                 720

ccc ccg ccg ccg ccg cgg ccg cgc gcg cgc ggt gcg tcc cgc agg acg 2444
Pro Pro Pro Pro Pro Arg Pro Arg Ala Arg Gly Ala Ser Arg Arg Thr
        725                 730                 735

tcg gcg ggg ccc cgg cgc tgg cgc cgc tcg cgc ctc aac ggg ctg gcg 2492
Ser Ala Gly Pro Arg Arg Trp Arg Arg Ser Arg Leu Asn Gly Leu Ala
    740                 745                 750

gcg cag cgc gca cgg gcg gcg agg gac tcg ctg tcg ctg agc agc ggc 2540
Ala Gln Arg Ala Arg Ala Ala Arg Asp Ser Leu Ser Leu Ser Ser Gly
755                 760                 765                 770

tcg ggc ggc ggc tca gcc tcg gcg tcg gac gac gac gcg gac gac gcg 2588
Ser Gly Gly Gly Ser Ala Ser Ala Ser Asp Asp Asp Ala Asp Asp Ala
                775                 780                 785

gac ggg gcg ctg gcg gcc gag agc aca cct ttc ctg ggc ctg cgt ggg 2636
Asp Gly Ala Leu Ala Ala Glu Ser Thr Pro Phe Leu Gly Leu Arg Gly
            790                 795                 800

gcg cac gac gcg ctg cgc tcg gac tcg ccg cca ctg tgc ccg gcg gcc 2684
Ala His Asp Ala Leu Arg Ser Asp Ser Pro Pro Leu Cys Pro Ala Ala

805                 810                 815

gac agc agg act tac tac tca ctg gac agc cac agc acg cgg gcc agc      2732
Asp Ser Arg Thr Tyr Tyr Ser Leu Asp Ser His Ser Thr Arg Ala Ser
    820                 825                 830

agc aga cac agc cgc ggg ccg ccc ccg cgg gcc aag cag gac tcg gcg      2780
Ser Arg His Ser Arg Gly Pro Pro Pro Arg Ala Lys Gln Asp Ser Ala
835                 840                 845                 850

cca ctc tag ggccccgccg cgcgcccctc cgccccgccc gccccactat              2829
Pro Leu

ctttaaggag accagagacc gcctactgga gagaaaggag gaaaaaagaa ataaaaatat    2889

ttttattttc tataaaagga aaaaagtata acaaaatgtt ttattttcat tttagcaaaa    2949

attgtcttat aatactagct aacggcaaag gcgtttttat agggaaacta tttatatgta    3009

acatcctgat ttacagcttc ggaaaaaaaa aagaaacaac aaaaaaaaaa gagagatggg    3069

ccaatttttt gactctttaa tagaaaccta ttttgtggtg ccttttgctg tatgctcatc    3129

tggggctccg ggggacaggt ctgggttgtg gggtgctgag agtgacagct gggagagaag     3189

aggtgcgggt tctgttgggg gcgagggtgg ggggcggtgg ggggttggcc ctaaggaaga    3249

actgaggacg gaagtagctt tgctcccagg caagagctga agttcccgac ccctgggagg    3309

agagggccag gaccctaggc ttggagctgg agctggggta acttccaggg ggagataaag    3369

acacagctac ccgaatgggg aagccctggc tactcacagc tgttaaagga gggggggcag    3429

agaaacgttt ccccaagagc ctgagcccag ggaggtggca gctctgccag gggcccaatg    3489

ttggcggctc ctgtccccca cagcctccag gacgcccctc cgtcctctgc agcaccttcg    3549

tttacaggtc gtcttttcta ttttacacct gcatgtcctt tgcatttcag attctttaga    3609

ttggatgcat ggtcacgctg ggaccgagaa gagccactcg acagtgtatt ccccttttag     3669

caataaagta acaccatttc cttctcacag gctgcctcct agcccccaac ccacctatga    3729

catccactcg tctccagtcc tgccctccca cccccaaaaa ctgggtccac tgctaatttg    3789

tcaaaaaggt aattgtttaa aaaaaaaaaa aatcccaaac agaaacagat gggaatcctg    3849

tcctgaaatg tctgaacacc tgactgttga cacttgaaca tttttatatt ataaataaaa    3909

tcagaaataa ctcctgttaa aaaaaaaaaa aaaaaa                              3945

<210>  62
<211>  852
<212>  PRT
<213>  Homo sapiens

<400>  62
   Met Arg Gln Val Cys Cys Ser Ala Leu Pro Pro Pro Pro Leu Glu Lys
   1               5                   10                  15
   Gly Arg Cys Ser Ser Tyr Ser Asp Ser Ser Ser Ser Ser Ser Glu Arg
               20                  25                  30
   Ser Ser Ser Ser Ser Ser Ser Ser Ser Glu Ser Gly Ser Ser Ser Arg
           35                  40                  45
   Ser Ser Ser Asn Asn Ser Ser Ile Ser Arg Pro Ala Ala Pro Pro Glu
       50                  55                  60
   Pro Arg Pro Gln Gln Gln Pro Gln Pro Arg Ser Pro Ala Ala Arg Arg
   65                  70                  75                  80
   Ala Ala Ala Arg Ser Arg Ala Ala Ala Ala Gly Gly Met Arg Arg Asp
                   85                  90                  95
   Pro Ala Pro Gly Phe Ser Met Leu Leu Phe Gly Val Ser Leu Ala Cys
               100                 105                 110
   Tyr Ser Pro Ser Leu Lys Ser Val Gln Asp Gln Ala Tyr Lys Ala Pro
           115                 120                 125
   Val Val Val Glu Gly Lys Val Gln Gly Leu Val Pro Ala Gly Gly Ser
       130                 135                 140
   Ser Ser Asn Ser Thr Arg Glu Pro Pro Ala Ser Gly Arg Val Ala Leu
   145                 150                 155                 160
   Val Lys Val Leu Asp Lys Trp Pro Leu Arg Ser Gly Gly Leu Gln Arg
                   165                 170                 175
   Glu Gln Val Ile Ser Val Gly Ser Cys Val Pro Leu Glu Arg Asn Gln
               180                 185                 190
   Arg Tyr Ile Phe Phe Leu Glu Pro Thr Glu Gln Pro Leu Val Phe Lys
           195                 200                 205
   Thr Ala Phe Ala Pro Leu Asp Thr Asn Gly Lys Asn Leu Lys Lys Glu
       210                 215                 220
   Val Gly Lys Ile Leu Cys Thr Asp Cys Ala Thr Arg Pro Lys Leu Lys
   225                 230                 235                 240
   Lys Met Lys Ser Gln Thr Gly Gln Val Gly Glu Lys Gln Ser Leu Lys
                   245                 250                 255
   Cys Glu Ala Ala Ala Gly Asn Pro Gln Pro Ser Tyr Arg Trp Phe Lys
               260                 265                 270
   Asp Gly Lys Glu Leu Asn Arg Ser Arg Asp Ile Arg Ile Lys Tyr Gly
           275                 280                 285
   Asn Gly Arg Lys Asn Ser Arg Leu Gln Phe Asn Lys Val Lys Val Glu
       290                 295                 300
   Asp Ala Gly Glu Tyr Val Cys Glu Ala Glu Asn Ile Leu Gly Lys Asp
   305                 310                 315                 320
   Thr Val Arg Gly Arg Leu Tyr Val Asn Ser Val Ser Thr Thr Leu Ser
                   325                 330                 335
   Ser Trp Ser Gly His Ala Arg Lys Cys Asn Glu Thr Ala Lys Ser Tyr
               340                 345                 350
   Cys Val Asn Gly Gly Val Cys Tyr Tyr Ile Glu Gly Ile Asn Gln Leu
           355                 360                 365
   Ser Cys Lys Cys Pro Val Gly Tyr Thr Gly Asp Arg Cys Gln Gln Phe
       370                 375                 380
   Ala Met Val Asn Phe Ser Lys His Leu Gly Phe Glu Leu Lys Glu Ala
   385                 390                 395                 400
   Glu Glu Leu Tyr Gln Lys Arg Val Leu Thr Ile Thr Gly Ile Cys Val
                   405                 410                 415
   Ala Leu Leu Val Val Gly Ile Val Cys Val Val Ala Tyr Cys Lys Thr
               420                 425                 430
   Lys Lys Gln Arg Lys Gln Met His Asn His Leu Arg Gln Asn Met Cys
           435                 440                 445
   Pro Ala His Gln Asn Arg Ser Leu Ala Asn Gly Pro Ser His Pro Arg
       450                 455                 460
   Leu Asp Pro Glu Glu Ile Gln Met Ala Asp Tyr Ile Ser Lys Asn Val
   465                 470                 475                 480
   Pro Ala Thr Asp His Val Ile Arg Arg Glu Thr Glu Thr Thr Phe Ser
                   485                 490                 495
   Gly Ser His Ser Cys Ser Pro Ser His His Cys Ser Thr Ala Thr Pro
               500                 505                 510
   Thr Ser Ser His Arg His Glu Ser His Thr Trp Ser Leu Glu Arg Ser
   515                 520                 525
   Glu Ser Leu Thr Ser Asp Ser Gln Ser Gly Ile Met Leu Ser Ser Val
       530                 535                 540
   Gly Thr Ser Lys Cys Asn Ser Pro Ala Cys Val Glu Ala Arg Ala Arg
   545                 550                 555                 560
   Arg Ala Ala Ala Tyr Asn Leu Glu Glu Arg Arg Arg Ala Thr Ala Pro
                   565                 570                 575
   Pro Tyr His Asp Ser Val Asp Ser Leu Arg Asp Ser Pro His Ser Glu
               580                 585                 590
   Arg Tyr Val Ser Ala Leu Thr Thr Pro Ala Arg Leu Ser Pro Val Asp
           595                 600                 605
   Phe His Tyr Ser Leu Ala Thr Gln Val Pro Thr Phe Glu Ile Thr Ser
       610                 615                 620
   Pro Asn Ser Ala His Ala Val Ser Leu Pro Pro Ala Ala Pro Ile Ser
   625                 630                 635                 640
   Tyr Arg Leu Ala Glu Gln Gln Pro Leu Leu Arg His Pro Ala Pro Pro
                   645                 650                 655
   Gly Pro Gly Pro Gly Pro Gly Pro Gly Pro Gly Pro Gly Ala Asp Met
               660                 665                 670
   Gln Arg Ser Tyr Asp Ser Tyr Tyr Tyr Pro Ala Ala Gly Pro Gly Pro
           675                 680                 685
   Arg Arg Gly Thr Cys Ala Leu Gly Gly Ser Leu Gly Ser Leu Pro Ala
       690                 695                 700
   Ser Pro Phe Arg Ile Pro Glu Asp Asp Glu Tyr Glu Thr Thr Gln Glu
   705                 710                 715                 720
   Cys Ala Pro Pro Pro Pro Pro Arg Pro Arg Ala Arg Gly Ala Ser Arg
                   725                 730                 735
   Arg Thr Ser Ala Gly Pro Arg Arg Trp Arg Arg Ser Arg Leu Asn Gly
               740                 745                 750
   Leu Ala Ala Gln Arg Ala Arg Ala Ala Arg Asp Ser Leu Ser Leu Ser
           755                 760                 765
   Ser Gly Ser Gly Gly Gly Ser Ala Ser Ala Ser Asp Asp Asp Ala Asp
       770                 775                 780
   Asp Ala Asp Gly Ala Leu Ala Ala Glu Ser Thr Pro Phe Leu Gly Leu
   785                 790                 795                 800
   Arg Gly Ala His Asp Ala Leu Arg Ser Asp Ser Pro Pro Leu Cys Pro
                   805                 810                 815
   Ala Ala Asp Ser Arg Thr Tyr Tyr Ser Leu Asp Ser His Ser Thr Arg
               820                 825                 830
   Ala Ser Ser Arg His Ser Arg Gly Pro Pro Pro Arg Ala Lys Gln Asp
           835                 840                 845
   Ser Ala Pro Leu
       850

<210>  63
<211>  3741
<212>  DNA
<213>  Homo sapiens

<220>
<221>  CDS
<222>  (231)..(2585)

<400>  63
atcctcctcc aggtcctggc gcacagggtg ggagcgctgc gctgcgccgc gctgcgcatc      60

gcggcccgct tgccgcctgc cccctgccct agctgggcca cctccccggg ctgccggtgg     120

agggctaaga ggcgctaacg ttacgctgtt tccggttttc cagcgggctc tgtttcccct     180

cccaaggcgg cggcggctga gcggcggagc cccccaaatg gcctggccag atg cgg        236
                                                       Met Arg
                                                       1

cag gtt tgc tgc tca gcg ctg ccg ccg ccg cca ctg gag aag ggt cgg       284
Gln Val Cys Cys Ser Ala Leu Pro Pro Pro Pro Leu Glu Lys Gly Arg
        5                   10                  15

tgc agc agc tac agc gac agc agc agc agc agc agc gag agg agc agc       332
Cys Ser Ser Tyr Ser Asp Ser Ser Ser Ser Ser Ser Glu Arg Ser Ser
    20                  25                  30

agc agc agc agc agc agc agc gag agc ggc agc agc agc agg agc agc       380
Ser Ser Ser Ser Ser Ser Ser Glu Ser Gly Ser Ser Ser Arg Ser Ser
35                  40                  45                  50

agc aac aac agc agc atc tct cgt ccc gct gcg ccc cca gag ccg cgg       428
Ser Asn Asn Ser Ser Ile Ser Arg Pro Ala Ala Pro Pro Glu Pro Arg
                55                  60                  65

ccg cag caa cag ccg cag ccc cgc agc ccc gca gcc cgg aga gcc gcc       476
Pro Gln Gln Gln Pro Gln Pro Arg Ser Pro Ala Ala Arg Arg Ala Ala

70                  75                  80

gcc cgt tcg cga gcc gca gcc gcc ggc ggc atg agg cgc gac ccg gcc      524
Ala Arg Ser Arg Ala Ala Ala Ala Gly Gly Met Arg Arg Asp Pro Ala
        85                  90                  95

ccc ggc ttc tcc atg ctg ctc ttc ggt gtg tcg ctc gcc tgc tac tcg      572
Pro Gly Phe Ser Met Leu Leu Phe Gly Val Ser Leu Ala Cys Tyr Ser
    100                 105                 110

ccc agc ctc aag tca gtg cag gac cag gcg tac aag gca ccc gtg gtg      620
Pro Ser Leu Lys Ser Val Gln Asp Gln Ala Tyr Lys Ala Pro Val Val
115                 120                 125                 130

gtg gag ggc aag gta cag ggg ctg gtc cca gcc ggc ggc tcc agc tcc      668
Val Glu Gly Lys Val Gln Gly Leu Val Pro Ala Gly Gly Ser Ser Ser
                135                 140                 145

aac agc acc cga gag ccg ccc gcc tcg ggt cgg gtg gcg ttg gta aag      716
Asn Ser Thr Arg Glu Pro Pro Ala Ser Gly Arg Val Ala Leu Val Lys
            150                 155                 160

gtg ctg gac aag tgg ccg ctc cgg agc ggg ggg ctg cag cgc gag cag      764
Val Leu Asp Lys Trp Pro Leu Arg Ser Gly Gly Leu Gln Arg Glu Gln
        165                 170                 175

gtg atc agc gtg ggc tcc tgt gtg ccg ctc gaa agg aac cag cgc tac      812
Val Ile Ser Val Gly Ser Cys Val Pro Leu Glu Arg Asn Gln Arg Tyr
    180                 185                 190

atc ttt ttc ctg gag ccc acg gaa cag ccc tta gtc ttt aag acg gcc      860
Ile Phe Phe Leu Glu Pro Thr Glu Gln Pro Leu Val Phe Lys Thr Ala
195                 200                 205                 210

ttt gcc ccc ctc gat acc aac ggc aaa aat ctc aag aaa gag gtg ggc      908
Phe Ala Pro Leu Asp Thr Asn Gly Lys Asn Leu Lys Lys Glu Val Gly
                215                 220                 225

aag atc ctg tgc act gac tgc gcc acc cgg ccc aag ttg aag aag atg      956
Lys Ile Leu Cys Thr Asp Cys Ala Thr Arg Pro Lys Leu Lys Lys Met
            230                 235                 240

aag agc cag acg gga cag gtg ggt gag aag caa tcg ctg aag tgt gag      1004
Lys Ser Gln Thr Gly Gln Val Gly Glu Lys Gln Ser Leu Lys Cys Glu
        245                 250                 255

gca gca gcc ggt aat ccc cag cct tcc tac cgt tgg ttc aag gat ggc      1052
Ala Ala Ala Gly Asn Pro Gln Pro Ser Tyr Arg Trp Phe Lys Asp Gly
    260                 265                 270

aag gag ctc aac cgc agc cga gac att cgc atc aaa tat ggc aac ggc      1100
Lys Glu Leu Asn Arg Ser Arg Asp Ile Arg Ile Lys Tyr Gly Asn Gly
275                 280                 285                 290

aga aag aac tca cga cta cag ttc aac aag gtg aag gtg gag gac gct      1148
Arg Lys Asn Ser Arg Leu Gln Phe Asn Lys Val Lys Val Glu Asp Ala
                295                 300                 305

ggg gag tat gtc tgc gag gcc gag aac atc ctg ggg aag gac acc gtc      1196
Gly Glu Tyr Val Cys Glu Ala Glu Asn Ile Leu Gly Lys Asp Thr Val
            310                 315                 320

cgg ggc cgg ctt tac gtc aac agc gaa gcc gag gag ctg tac cag aag      1244

Arg Gly Arg Leu Tyr Val Asn Ser Glu Ala Glu Glu Leu Tyr Gln Lys
        325                 330                 335

agg gtc ctg acc atc acg ggc atc tgc gtg gct ctg ctg gtc gtg ggc      1292
Arg Val Leu Thr Ile Thr Gly Ile Cys Val Ala Leu Leu Val Val Gly
    340                 345                 350

atc gtc tgt gtg gtg gcc tac tgc aag acc aaa aaa cag cgg aag cag      1340
Ile Val Cys Val Val Ala Tyr Cys Lys Thr Lys Lys Gln Arg Lys Gln
355                 360                 365                 370

atg cac aac cac ctc cgg cag aac atg tgc ccg gcc cat cag aac cgg      1388
Met His Asn His Leu Arg Gln Asn Met Cys Pro Ala His Gln Asn Arg
                375                 380                 385

agc ttg gcc aat ggg ccc agc cac ccc cgg ctg gac cca gag gag atc      1436
Ser Leu Ala Asn Gly Pro Ser His Pro Arg Leu Asp Pro Glu Glu Ile
            390                 395                 400

cag atg gca gat tat att tcc aag aac gtg cca gcc aca gac cat gtc      1484
Gln Met Ala Asp Tyr Ile Ser Lys Asn Val Pro Ala Thr Asp His Val
        405                 410                 415

atc agg aga gaa act gag acc acc ttc tct ggg agc cac tcc tgt tct      1532
Ile Arg Arg Glu Thr Glu Thr Thr Phe Ser Gly Ser His Ser Cys Ser
    420                 425                 430

cct tct cac cac tgc tcc aca gcc aca ccc acc tcc agc cac aga cac      1580
Pro Ser His His Cys Ser Thr Ala Thr Pro Thr Ser Ser His Arg His
435                 440                 445                 450

gag agc cac acg tgg agc ctg gaa cgt tct gag agc ctg act tct gac      1628
Glu Ser His Thr Trp Ser Leu Glu Arg Ser Glu Ser Leu Thr Ser Asp
                455                 460                 465

tcc cag tcg ggg atc atg cta tca tca gtg ggt acc agc aaa tgc aac      1676
Ser Gln Ser Gly Ile Met Leu Ser Ser Val Gly Thr Ser Lys Cys Asn
            470                 475                 480

agc cca gca tgt gtg gag gcc cgg gca agg cgg gca gca gcc tac aac      1724
Ser Pro Ala Cys Val Glu Ala Arg Ala Arg Arg Ala Ala Ala Tyr Asn
        485                 490                 495

ctg gag gag cgg cgc agg gcc acc gcg cca ccc tat cac gat tcc gtg      1772
Leu Glu Glu Arg Arg Arg Ala Thr Ala Pro Pro Tyr His Asp Ser Val
    500                 505                 510

gac tcc ctt cgc gac tcc cca cac agc gag agg tac gtg tcg gcc ctg      1820
Asp Ser Leu Arg Asp Ser Pro His Ser Glu Arg Tyr Val Ser Ala Leu
515                 520                 525                 530

acc acg ccc gcg cgc ctc tcg ccc gtg gac ttc cac tac tcg ctg gcc      1868
Thr Thr Pro Ala Arg Leu Ser Pro Val Asp Phe His Tyr Ser Leu Ala
                535                 540                 545

acg cag gtg cca act ttc gag atc acg tcc ccc aac tcg gcg cac gcc      1916
Thr Gln Val Pro Thr Phe Glu Ile Thr Ser Pro Asn Ser Ala His Ala
            550                 555                 560

gtg tcg ctg ccg ccg gcg gcg ccc atc agt tac cgc ctg gcc gag cag      1964
Val Ser Leu Pro Pro Ala Ala Pro Ile Ser Tyr Arg Leu Ala Glu Gln
        565                 570                 575

cag ccg tta ctg cgg cac ccg gcg ccc ccc ggc ccg gga ccc gga ccc     2012
Gln Pro Leu Leu Arg His Pro Ala Pro Pro Gly Pro Gly Pro Gly Pro
    580                 585                 590

ggg ccc ggg ccc ggg ccc ggc gca gac atg cag cgc agc tat gac agc     2060
Gly Pro Gly Pro Gly Pro Gly Ala Asp Met Gln Arg Ser Tyr Asp Ser
595                 600                 605                 610

tac tat tac ccc gcg gcg ggg ccc gga ccg cgg cgc ggg acc tgc gcg     2108
Tyr Tyr Tyr Pro Ala Ala Gly Pro Gly Pro Arg Arg Gly Thr Cys Ala
                615                 620                 625

ctc ggc ggc agc ctg ggc agc ctg cct gcc agc ccc ttc cgc atc ccc     2156
Leu Gly Gly Ser Leu Gly Ser Leu Pro Ala Ser Pro Phe Arg Ile Pro
            630                 635                 640

gag gac gac gag tac gag acc acg cag gag tgc gcg ccc ccg ccg ccg     2204
Glu Asp Asp Glu Tyr Glu Thr Thr Gln Glu Cys Ala Pro Pro Pro Pro
        645                 650                 655

ccg cgg ccg cgc gcg cgc ggt gcg tcc cgc agg acg tcg gcg ggg ccc     2252
Pro Arg Pro Arg Ala Arg Gly Ala Ser Arg Arg Thr Ser Ala Gly Pro
    660                 665                 670

cgg cgc tgg cgc cgc tcg cgc ctc aac ggg ctg gcg gcg cag cgc gca     2300
Arg Arg Trp Arg Arg Ser Arg Leu Asn Gly Leu Ala Ala Gln Arg Ala
675                 680                 685                 690

cgg gcg gcg agg gac tcg ctg tcg ctg agc agc ggc tcg ggc ggc ggc     2348
Arg Ala Ala Arg Asp Ser Leu Ser Leu Ser Ser Gly Ser Gly Gly Gly
                695                 700                 705

tca gcc tcg gcg tcg gac gac gac gcg gac gac gcg gac ggg gcg ctg     2396
Ser Ala Ser Ala Ser Asp Asp Asp Ala Asp Asp Ala Asp Gly Ala Leu
            710                 715                 720

gcg gcc gag agc aca cct ttc ctg ggc ctg cgt ggg gcg cac gac gcg     2444
Ala Ala Glu Ser Thr Pro Phe Leu Gly Leu Arg Gly Ala His Asp Ala
        725                 730                 735

ctg cgc tcg gac tcg ccg cca ctg tgc ccg gcg gcc gac agc agg act     2492
Leu Arg Ser Asp Ser Pro Pro Leu Cys Pro Ala Ala Asp Ser Arg Thr
    740                 745                 750

tac tac tca ctg gac agc cac agc acg cgg gcc agc agc aga cac agc     2540
Tyr Tyr Ser Leu Asp Ser His Ser Thr Arg Ala Ser Ser Arg His Ser
755                 760                 765                 770

cgc ggg ccg ccc ccg cgg gcc aag cag gac tcg gcg cca ctc tag         2585
Arg Gly Pro Pro Pro Arg Ala Lys Gln Asp Ser Ala Pro Leu
                775                 780

ggccccgccg cgcgcccctc cgccccgccc gccccactat ctttaaggag accagagacc     2645

gcctactgga gagaaaggag gaaaaaagaa ataaaaatat ttttattttc tataaaagga     2705

aaaaagtata acaaaatgtt ttattttcat tttagcaaaa attgtcttat aatactagct     2765

aacggcaaag gcgtttttat agggaaacta tttatatgta acatcctgat ttacagcttc     2825

ggaaaaaaaa aagaaacaac aaaaaaaaaa gagagatggg ccaatttttt gactctttaa     2885

tagaaaccta ttttgtggtg ccttttgctg tatgctcatc tggggctccg ggggacaggt     2945

ctgggttgtg gggtgctgag agtgacagct gggagagaag aggtgcgggt tctgttgggg 3005

gcgagggtgg ggggcggtgg ggggttggcc ctaaggaaga actgaggacg gaagtagctt 3065

tgctcccagg caagagctga agttcccgac ccctgggagg agagggccag gaccctaggc 3125

ttggagctgg agctggggta acttccaggg ggagataaag acacagctac ccgaatgggg 3185

aagccctggc tactcacagc tgttaaagga gggggggcag agaaacgttt ccccaagagc 3245

ctgagcccag ggaggtggca gctctgccag gggcccaatg ttggcggctc ctgtccccca 3305

cagcctccag gacgcccctc cgtcctctgc agcaccttcg tttacaggtc gtcttttcta 3365

ttttacacct gcatgtcctt tgcatttcag attctttaga ttggatgcat ggtcacgctg 3425

ggaccgagaa gagccactcg acagtgtatt ccccttttag caataaagta acaccatttc 3485

cttctcacag gctgcctcct agcccccaac ccacctatga catccactcg tctccagtcc 3545

tgccctccca cccccaaaaa ctgggtccac tgctaatttg tcaaaaaggt aattgtttaa 3605

aaaaaaaaaa aatcccaaac agaaacagat gggaatcctg tcctgaaatg tctgaacacc 3665

tgactgttga cacttgaaca tttttatatt ataaataaaa tcagaaataa ctcctgttaa 3725

aaaaaaaaaa aaaaaa 3741

<210> 64
<211> 784
<212> PRT
<213> Homo sapiens

<400> 64
   Met Arg Gln Val Cys Cys Ser Ala Leu Pro Pro Pro Pro Leu Glu Lys
   1               5                   10                  15
   Gly Arg Cys Ser Ser Tyr Ser Asp Ser Ser Ser Ser Ser Ser Glu Arg
               20                  25                  30
   Ser Ser Ser Ser Ser Ser Ser Ser Ser Glu Ser Gly Ser Ser Ser Arg
           35                  40                  45
   Ser Ser Ser Asn Asn Ser Ser Ile Ser Arg Pro Ala Ala Pro Pro Glu
       50                  55                  60
   Pro Arg Pro Gln Gln Gln Pro Gln Pro Arg Ser Pro Ala Ala Arg Arg
   65                  70                  75                  80
   Ala Ala Ala Arg Ser Arg Ala Ala Ala Ala Gly Gly Met Arg Arg Asp
                   85                  90                  95
   Pro Ala Pro Gly Phe Ser Met Leu Leu Phe Gly Val Ser Leu Ala Cys
               100                 105                 110
   Tyr Ser Pro Ser Leu Lys Ser Val Gln Asp Gln Ala Tyr Lys Ala Pro
           115                 120                 125
   Val Val Val Glu Gly Lys Val Gln Gly Leu Val Pro Ala Gly Gly Ser
       130                 135                 140
   Ser Ser Asn Ser Thr Arg Glu Pro Pro Ala Ser Gly Arg Val Ala Leu
   145                 150                 155                 160
   Val Lys Val Leu Asp Lys Trp Pro Leu Arg Ser Gly Gly Leu Gln Arg
                   165                 170                 175
   Glu Gln Val Ile Ser Val Gly Ser Cys Val Pro Leu Glu Arg Asn Gln
               180                 185                 190
   Arg Tyr Ile Phe Phe Leu Glu Pro Thr Glu Gln Pro Leu Val Phe Lys
           195                 200                 205
   Thr Ala Phe Ala Pro Leu Asp Thr Asn Gly Lys Asn Leu Lys Lys Glu
       210                 215                 220
   Val Gly Lys Ile Leu Cys Thr Asp Cys Ala Thr Arg Pro Lys Leu Lys
   225                 230                 235                 240
   Lys Met Lys Ser Gln Thr Gly Gln Val Gly Glu Lys Gln Ser Leu Lys
                   245                 250                 255
   Cys Glu Ala Ala Ala Gly Asn Pro Gln Pro Ser Tyr Arg Trp Phe Lys
               260                 265                 270
   Asp Gly Lys Glu Leu Asn Arg Ser Arg Asp Ile Arg Ile Lys Tyr Gly
           275                 280                 285
   Asn Gly Arg Lys Asn Ser Arg Leu Gln Phe Asn Lys Val Lys Val Glu
       290                 295                 300
   Asp Ala Gly Glu Tyr Val Cys Glu Ala Glu Asn Ile Leu Gly Lys Asp
   305                 310                 315                 320
   Thr Val Arg Gly Arg Leu Tyr Val Asn Ser Glu Ala Glu Glu Leu Tyr
                   325                 330                 335
   Gln Lys Arg Val Leu Thr Ile Thr Gly Ile Cys Val Ala Leu Leu Val
               340                 345                 350
   Val Gly Ile Val Cys Val Val Ala Tyr Cys Lys Thr Lys Lys Gln Arg
   355 360 365
   Lys Gln Met His Asn His Leu Arg Gln Asn Met Cys Pro Ala His Gln
   370 375 380
   Asn Arg Ser Leu Ala Asn Gly Pro Ser His Pro Arg Leu Asp Pro Glu
   385 390 395 400
   Glu Ile Gln Met Ala Asp Tyr Ile Ser Lys Asn Val Pro Ala Thr Asp
   405 410 415
   His Val Ile Arg Arg Glu Thr Glu Thr Thr Phe Ser Gly Ser His Ser
   420 425 430
   Cys Ser Pro Ser His His Cys Ser Thr Ala Thr Pro Thr Ser Ser His
   435 440 445
   Arg His Glu Ser His Thr Trp Ser Leu Glu Arg Ser Glu Ser Leu Thr
   450 455 460
   Ser Asp Ser Gln Ser Gly Ile Met Leu Ser Ser Val Gly Thr Ser Lys
   465 470 475 480
   Cys Asn Ser Pro Ala Cys Val Glu Ala Arg Ala Arg Arg Ala Ala Ala
   485 490 495
   Tyr Asn Leu Glu Glu Arg Arg Arg Ala Thr Ala Pro Pro Tyr His Asp
   500 505 510
   Ser Val Asp Ser Leu Arg Asp Ser Pro His Ser Glu Arg Tyr Val Ser
   515 520 525
   Ala Leu Thr Thr Pro Ala Arg Leu Ser Pro Val Asp Phe His Tyr Ser
   530 535 540
   Leu Ala Thr Gln Val Pro Thr Phe Glu Ile Thr Ser Pro Asn Ser Ala
   545 550 555 560
   His Ala Val Ser Leu Pro Pro Ala Ala Pro Ile Ser Tyr Arg Leu Ala
   565 570 575
   Glu Gln Gln Pro Leu Leu Arg His Pro Ala Pro Pro Gly Pro Gly Pro
   580 585 590
   Gly Pro Gly Pro Gly Pro Gly Pro Gly Ala Asp Met Gln Arg Ser Tyr
   595 600 605
   Asp Ser Tyr Tyr Tyr Pro Ala Ala Gly Pro Gly Pro Arg Arg Gly Thr
       610                 615                 620
   Cys Ala Leu Gly Gly Ser Leu Gly Ser Leu Pro Ala Ser Pro Phe Arg
   625                 630                 635                 640
   Ile Pro Glu Asp Asp Glu Tyr Glu Thr Thr Gln Glu Cys Ala Pro Pro
                   645                 650                 655
   Pro Pro Pro Arg Pro Arg Ala Arg Gly Ala Ser Arg Arg Thr Ser Ala
               660                 665                 670
   Gly Pro Arg Arg Trp Arg Arg Ser Arg Leu Asn Gly Leu Ala Ala Gln
           675                 680                 685
   Arg Ala Arg Ala Ala Arg Asp Ser Leu Ser Leu Ser Ser Gly Ser Gly
       690                 695                 700
   Gly Gly Ser Ala Ser Ala Ser Asp Asp Asp Ala Asp Asp Ala Asp Gly
   705                 710                 715                 720
   Ala Leu Ala Ala Glu Ser Thr Pro Phe Leu Gly Leu Arg Gly Ala His
                   725                 730                 735
   Asp Ala Leu Arg Ser Asp Ser Pro Pro Leu Cys Pro Ala Ala Asp Ser
               740                 745                 750
   Arg Thr Tyr Tyr Ser Leu Asp Ser His Ser Thr Arg Ala Ser Ser Arg
           755                 760                 765
   His Ser Arg Gly Pro Pro Pro Arg Ala Lys Gln Asp Ser Ala Pro Leu
       770                 775                 780

<210>  65
<211>  24
<212>  DNA
<213>  Artificial Sequence

<220>
<223>  ATG3 sense primer

<400>  65
cactggaagt ggctgagtac ctga                                              24

<210>  66
<211>  20
<212>  DNA
<213>  Artificial Sequence

<220>
<223>  EPHB1 antisense primer

<400>  66
gccgcagccc atcaatcctt                                                    20


<210>  67
<211>  24
<212>  DNA
<213>  Artificial Sequence

<220>
<223>  TNIK sense primer

<400>  67
ggtcagtgga aacaggacat ttgg                                               24


<210>  68
<211>  22
<212>  DNA
<213>  Artificial Sequence

<220>
<223>  RNF123 antisense primer

<400>  68
acgccttgcc ataattcgtt gt                                                 22


<210>  69
<211>  25
<212>  DNA
<213>  Artificial Sequence

<220>
<223>  SLC12A2 sense primer

<400>  69
ggtcaagctg gaataggtct atcag                                              25


<210>  70
<211>  20
<212>  DNA
<213>  Artificial Sequence

<220>
<223>  NRG2 antisense primer

<400>  70
tgcctcacac ttcagcgatt                                                    20


<210>  71
<211>  150
<212>  DNA
<213>  Homo sapiens

<400>  71
cactggaagt ggctgagtac ctgaccccgg tcctcaagga atcaaagttt aaggaaacag      60

gtgtaattac cccagaagag gaacacaatg agttcaactc ctccatggcc aggagtcaga     120

ccaacacagc aaggattgat gggctgcggc                                    150

<210>  72
<211>  318
<212>  DNA
<213>  Homo sapiens

<400>  72
ggtcagtgga aacaggacat ttggatggag tatttatgca taagcgagct caaaggttaa      60

agtttctatg tgaaagaaat gataaggtga ttggacacag caactttggc accatccgct     120

ctaccacatg cgtgtacaaa gggaaatggc tctacgaggt cctcatctcc tcccaggggc     180

tcatgcagat cggctggtgc accatcagct gccgcttcaa ccaggaggag ggggttggag     240

atacacacaa ctcctatgcc tatgatggca accgcgtgcg caagtggaat gtgaccacaa     300

cgaattatgg caaggcgt                                                   318

<210>  73
<211>  189
<212>  DNA
<213>  Homo sapiens

<400>  73
ggtcaagctg gaataggtct atcagtcctt gtaataatga tggccactgt tgtgacaact      60

atcacaggat tgtctacttc agcaatagca actaatggat ttgtaagagg agccacccgg     120

cccaagttga agaagatgaa gagccagacg ggacaggtgg gtgagaagca atcgctgaag     180

tgtgaggca                                                             189